# EUROPEAN PATENT APPLICATION

(11) **EP 4 455 159 A1**
(43) Date of publication of application: **30.10.2024**
(21) Application number: 23169886.1
(22) Date of filing: 25.04.2023
(51) Int. Cl.: C07K 14/575, A61K 47/00, A61K 51/00, A61P 3/10, A61P 35/00, G01N 33/68, A61K 38/00

(54) **GASTRIC INHIBITORY PEPTIDE RECEPTOR LIGANDS WITH BIO-DISTRIBUTION MODIFIER**

(71) Applicant: 3B Pharmaceuticals GmbH, 12489 Berlin (DE)
(72) Inventor: The designation of the inventor has not yet been filed
(74) Representative: Bohmann, Armin K.

(57) **Abstract**

The present invention is related to a compound comprising a cyclic peptide of formula (Ia) 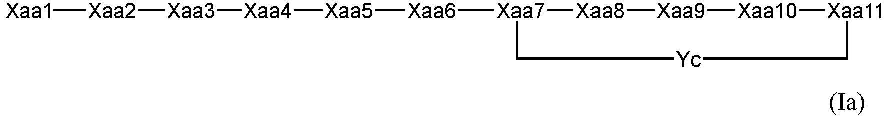 or a cyclic peptide of formula (Ib) 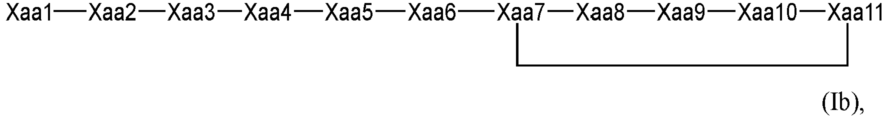 each comprising an N-terminal modification group A comprising a bio-distribution modifier, wherein the N-terminal modification group A is attached to Xaa1, and
each comprising a C-terminal group C-term comprising a bio-distribution modifier, wherein the C-terminal group C-term is attached to Xaa11.

## Description

### FIELD OF THE INVENTION

The present invention is related to a chemical compound; a peptide; a Gastric Inhibitory Peptide Receptor (GIPR) binding compound; a Gastric Inhibitory Peptide Receptor (GIPR) binding peptide; a composition comprising the compound; a composition comprising the Gastric Inhibitory Peptide Receptor (GIPR) binding compound; a composition comprising the peptide; a composition comprising the Gastric Inhibitory Peptide Receptor (GIPR) peptide; the compound, the Gastric Inhibitory Peptide Receptor (GIPR) binding compound, the peptide, the Gastric Inhibitory Peptide Receptor (GIPR) peptide and the compositions, respectively, for use in a method for the diagnosis of a disease; the compound, the Gastric Inhibitory Peptide Receptor (GIPR) binding compound and the compositions, respectively, for use in a method for the treatment of a disease; the compound, the Gastric Inhibitory Peptide Receptor (GIPR) binding compound, the peptide, Gastric Inhibitory Peptide Receptor (GIPR) peptide and the compositions, respectively, for use in a method of diagnosis and treatment of a disease which is also referred to as "thera(g)nosis" or "thera(g)nostics"; the compound, the Gastric Inhibitory Peptide Receptor (GIPR) binding compound, the peptide, the Gastric Inhibitory Peptide Receptor (GIPR) binding peptide, and the compositions, respectively, for use in a method for delivering a radionuclide to a Gastric Inhibitory Peptide Receptor (GIPR) to a cell, preferably a GIPR overexpressing tumor cell or pancreatic beta cell; a method for the diagnosis of a disease using the compound, Gastric Inhibitory Peptide Receptor (GIPR) binding compound, the peptide, the Gastric Inhibitory Peptide Receptor (GIPR) binding peptide and the compositions, respectively; a method for the treatment of a disease using the compound, the Gastric Inhibitory Peptide Receptor (GIPR) binding compound, the peptide, the Gastric Inhibitory Peptide Receptor (GIPR) binding peptide and the compositions, respectively; a method for the diagnosis and treatment of a disease which is also referred to as "thera(g)nosis" or "thera(g)nostics", using the compound, the Gastric Inhibitory Peptide Receptor (GIPR) binding compound, the peptide, the binding peptide and the compositions, respectively; a method for the delivery of a radionuclide to a Gastric Inhibitory Peptide Receptor (GIPR) expressing tissue using the compound, the Gastric Inhibitory Peptide Receptor (GIPR) binding compound, the peptide, the Gastric Inhibitory Peptide Receptor (GIPR) binding peptide and the compositions, respectively.

### BACKGROUND

The human gastric inhibitory polypeptide (GIP) is a 42 amino acid incretin hormone (H-Tyr-Ala-Glu-Gly-Thr-Phe-Ile-Ser-Asp-Tyr-Ser-Ile-Ala-Met-Asp-Lys-Ile-His-Gln-Gln-Asn-Phe-Val-Asn-Trp-Leu-Leu-Ala-Gln-Lys-Gly-Lys-Lys-Asn-Asp-Trp-Lys-His-Asn-Ile-Thr-Gln-OH) (SEQ ID NO: 2) secreted from the enteroendocrine K-cells, which forces diet-related insulin secretion via the GIPR signaling cascade (Cho et al., Vitam Horm, 2010, 84, 111-150). It was first discovered during cholecystokinin studies by John Brown and Raymond Pederson in the 1970s (Brown et al., Scand J Gastroenterol, 1970, 5, 537-541).

The GIPR is a 7-transmembrane spanning class B1 G-protein coupled receptor (GPCR) (Fredriksson et al., Mol Pharmacol, 2003, 63, 1256-1272), which is expressed in the gastrointestinal tract and the pancreatic islet cells (Usdin et al., Endocrinology, 1993, 133, 2861-2870). The extra-cellular N-terminal domain recognizes the ligand on its middle or C-terminal part, followed by the N-terminal part docking into the transmembrane domain of the receptor (Parthier et al., Proc Natl Acad Sci U S A, 2007, 104, 13942-13947). Receptor conformation induces transcription of the pro-insulin gene and insulin release by activating the adenylyl cyclase pathway that increases intracellular cyclic adenosine 3*'* 5'-monophasphate (cAMP) which is associated with elevated Ca²⁺ influx (Ding et al., Diabetes, 1997, 46, 615-621). Also, proliferative and anti-apoptotic effects were triggered by activation of the mitogen-activated protein kinase (MAPK) pathway and inhibition of caspase 3 (Khan et al., Peptides, 2020, 125, 170201). Both GIP(1-42)NH₂ and GIP(1-30)NH₂ are GIPR agonists and substrates for the dipeptidyl peptidase-4 (DPP4) resulting in potent GIPR inhibitors (GIP(3-42)NH₂; GIP(3-30) NH₂). While N-terminal truncations lead to decreased receptor activation and antagonism (Hansen et al., Br J Pharmacol, 2016, 173, 826-838), C-terminal modifications have only minor impact on stability or tissue distribution.

Recently, the GIPR came into the focus for peptide receptor radionuclide therapy (PRRT). In contrast to non-cancerous tissues, the GIPR is highly expressed in several subgroups of neuroendocrine neoplasms (Waser et al., J Clin Endocrinol Metab, 2012, 97, 482-488).

In 2012, a study showing a high GIP Receptor expression in gastroenteropancreatic and bronchial neuroendocrine tumors was published (Waser et al., J Clin Endocrinol Metab, 2012, 97, 482-488). The authors claimed GIPR represents a novel molecular target for clinical applications such as *in vivo* scintigraphy and targeted radiotherapy. WO 2012/168464 described a method for imaging and therapy of endocrine gastroenteropancreatic tumors and bronchial and thyroid neuroendocrine tumors by targeting GIPR. The receptor's incidence and density in human neuroendocrine tumors and non-neoplastic tissues was analyzed by autoradiography. Of these tumors, functional pancreatic neuroendocrine tumors, including insulinomas, gastrinomas, glucagonomas and vipomas, as well as non-functional pancreatic NETs and ileal NETs, presented highest receptor expression. In non-neoplastic tissues the highest expression was found in islets of the human pancreas. Academic groups published studies in which radiolabelled GIP analogs were used for nuclear medicine imaging applications (Gourni et al., J Nucl Med, 2014, 55, 976-982; Willekens et al., Sci Rep, 2018, 8, 2948). As proof of concept these studies have been successful, however, tumor uptake of the labeled peptides was around 20x lower when compared to kidney uptake.

### DETAILED DESCRIPTION OF THE INVENTION

The problem underlying the present invention is the provision of a compound which is suitable as a diagnostic agent and/or a therapeutic agent, particularly if conjugated to a diagnostically and/or therapeutically active radionuclide.

A further problem underlying the present invention is the provision of a compound which is suitable as a diagnostic agent and/or a therapeutic agent, particularly if conjugated to a diagnostically and/or therapeutically active radionuclide, having a pEC₅₀ of equal to or greater than 8.0 and/or a pIC₅₀ of equal to or greater than 7.5 for Gastric Inhibitory Peptide Receptor (GIPR).

A further problem underlying the present invention is the provision of a compound which is suitable as a diagnostic agent and/or a therapeutic agent, particularly if conjugated to a diagnostically and/or therapeutically active radionuclide, in the diagnosis and/or therapy of a disease where the diseased cells and/or diseased tissues express Gastric Inhibitory Peptide Receptor (GIPR). A still further problem underlying the instant invention is the provision of a compound which is suitable for delivering a diagnostically and/or therapeutically effective radionuclide to a diseased cell and/or diseased tissue, respectively, and more particularly a GIPR-expressing diseased cell and/or diseased tissue, preferably the diseased tissue comprises cancer or tumor cells.

Also, a problem underlying the present invention is the provision of a method for the diagnosis of a disease, of a method for the treatment and/or prevention of a disease, and a method for the combined diagnosis and treatment of a disease; preferably such disease is a disease involving GIPR-expressing cells and/or tissues, more particularly a GIPR-expressing diseased cell and/or diseased tissue, preferably the diseased tissue comprises or contains cancer or tumor cells or pancreatic beta cells.

A still further problem underlying the present invention is the provision of a method for the identification of a subject, wherein the subject is likely to respond or likely not to respond to a treatment of a disease, a method for the selection of a subject from a group of subjects, wherein the subject is likely to respond or likely not to respond to a treatment of a disease; preferably, the disease is cancer, more preferably the disease is a neuroendocrine tumor.

Also, a problem underlying the present invention is the provision of a pharmaceutical composition containing a compound having the characteristics as outlined above. Furthermore, a problem underlying the present invention is the provision of a kit which is suitable for use in any of the above methods.

These and other problems are solved by the subject matter of the attached independent claims; preferred embodiments may be taken from the attached dependent claims.

These and other problems are also solved by the subject matter of the following Embodiments.

Embodiment 1. A compound comprising a cyclic peptide of formula (Ia) 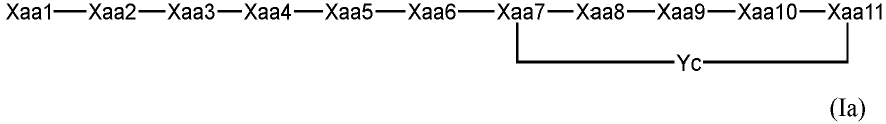
or a cyclic peptide of formula (Ib) 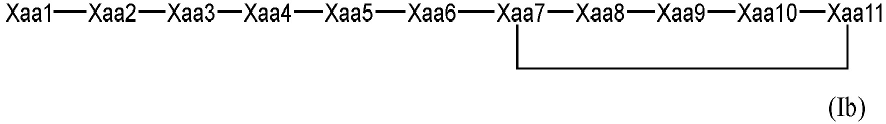
each comprising an N-terminal modification group A attached to Xaa1,
and each comprising a C-terminal group C-term attached to Xaa11,
wherein
   the peptide sequence is drawn from left to right in N- to C-terminal direction,
   the N-terminal modification group A comprises a Z group, wherein a linker is optionally interspersed between the Z group and Xaa1, wherein the Z group comprises a bio-distribution modifier,
   Xaa1 is a residue of an α-amino acid comprising a side chain, wherein the side chain comprises an acidic or polar group,
   wherein the carbonyl group of Xaa1 is covalently attached to the α-nitrogen atom of Xaa2,
   Xaa2 is a residue of an α-amino acid comprising a side chain, wherein the side chain comprises a heteroaryl ring or an aryl ring, preferably Xaa2 is a residue of an α-amino acid with a side chain comprising a heteroaryl ring,
   wherein the carbonyl group of Xaa2 is covalently attached to the α-nitrogen atom of Xaa3,
   Xaa3 is a residue of an α-amino acid comprising a side chain, wherein the side chain comprises a heteroaryl ring or an aryl ring, preferably Xaa3 is a residue of an α-amino acid comprising a side chain, wherein the side chain comprises a heteroaryl ring,
   wherein the carbonyl group of Xaa3 is covalently attached to the α-nitrogen atom of Xaa4,
   Xaa4 is a residue of an α-amino acid optionally comprising a Z group or is a residue of an N-alkylated α-amino acid optionally comprising a Z group, wherein the Z group is a chelator or a bio-distribution modifier,
   wherein the carbonyl group of Xaa4 is covalently attached to the α-nitrogen atom of Xaa5,
   Xaa5 is a residue of an α-amino acid optionally comprising a Z group, wherein the Z group is a chelator or a bio-distribution modifier,
   wherein the carbonyl group of Xaa5 is covalently attached to the α-nitrogen atom of Xaa6,
   Xaa6 is a residue of an α-amino acid comprising a side chain, wherein the side chain comprises a branched aliphatic group,
   wherein the carbonyl group of Xaa6 is covalently attached to the α-nitrogen atom of Xaa7,
   Xaa7 is a residue of an α-amino acid comprising a side chain, wherein the side chain comprises a nucleophilic group, wherein the nucleophilic group comprises a heteroatom, wherein the heteroatom is selected from the group comprising a sulfur atom and a nitrogen atom,
   wherein the carbonyl group of Xaa7 is covalently attached to the α-nitrogen atom of Xaa8,
   Xaa8 is a residue of an α-amino acid optionally comprising a Z group, wherein the Z group is a chelator or a bio-distribution modifier,
   wherein the carbonyl group of Xaa8 is covalently attached to the α-nitrogen atom of Xaa9,
   Xaa9 is a residue of an α-amino acid,
   wherein the carbonyl group of Xaa9 is covalently attached to the α-nitrogen atom of Xaa10,
   Xaa10 is a residue of an α-amino acid comprising a side chain, wherein the side chain comprises a branched aliphatic group or an unsubstituted aromatic ring,
   wherein the carbonyl group of Xaa10 is covalently attached to the α-nitrogen atom of Xaa11,
   Xaa11 is a residue of an α-amino acid comprising a side chain, wherein the side chain comprises a nucleophilic group, wherein the nucleophilic group comprises a heteroatom, wherein the heteroatom is selected from the group comprising a sulfur atom and a nitrogen atom,
   the C-terminal modification C-term comprises a Z group, wherein a linker is optionally interspersed between the Xaa11 and the Z group, wherein the Z group comprises a bio-distribution modifier,
   wherein Xaa7 and Xaa11 are indirectly linked or directly covalently linked to each other forming a macrocyclic ring,
   wherein if Xaa7 and Xaa11 are indirectly linked forming a cyclic peptide of formula (Ia), the heteroatom of Xaa7 and the heteroatom of Xaa11 are linked through a ring interspersed between the heteroatom of Xaa7 and the heteroatom of Xaa1 1 forming Yc, wherein the ring is a heteroaryl ring, an aryl ring optionally comprising a Z group or a heterocyclic ring, wherein the heteroatom is each and individually selected from the group consisting of a sulfur atom and a nitrogen atom, wherein if the heteroatom of Xaa7 is a sulfur atom, a thioether bond covalently links Xaa7 to the ring, if the heteroatom of Xaa7 is a nitrogen atom, an amine bond or an amide bond covalently links Xaa7 to the ring, if the heteroatom of Xaa11 is a sulfur atom, a thioether bond covalently links Xaa11 to the ring, and if the heteroatom of Xaa11 is a nitrogen atom, an amine bond or an amide bond covalently links Xaa11 to the ring,
or wherein if Xaa7 and Xaa11 are directly covalently linked forming a cyclic peptide of formula (Ib), the heteroatom of Xaa7 is a sulfur atom and the heteroatom of Xaa11 is a sulfur atom forming a disulfide bond,
and wherein each and any of the bio-distribution modifiers of the N-terminal modification group A, Xaa4, Xaa5 and Xaa8 comprises a residue of an aliphatic carboxylic acid,
   wherein to an ω-carbon atom of the aliphatic carboxylic acid a nitrogen atom, a triazolyl moiety, a hydroxymethyl-triazolyl moiety or an urea moiety is covalently attached,
      wherein to the nitrogen atom, to the triazolyl moiety, to the hydroxymethyl triazolyl moiety or to the urea moiety
      (a) 1 or 2 first level alkyl groups are covalently attached, wherein each and any first level alkyl group individually and independently comprises 2 or more hydroxyl groups, wherein each and any hydroxyl group is a primary hydroxyl group,
   or (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group
      wherein, if the bio-distribution modifier is comprised by the N-terminal modification group A,
         (a) the carbonyl group of the aliphatic carboxylic acid of the bio-distribution modifier is covalently attached to the α-nitrogen atom of Xaa1,
      or (b) if a linker comprising an amino group and a carbonyl group is interspersed between the bio-distribution modifier and Xaa1, the carbonyl group of the aliphatic carboxylic acid of the bio-distribution modifier is covalently attached to the amino group of the linker and the carbonyl group of the linker is covalently attached to the α-nitrogen atom of Xaa1,
   or wherein, if the bio-distribution modifier is comprised by Xaa4 comprising an amino group in the side chain, the carbonyl group of the aliphatic carboxylic acid of the bio-distribution modifier is covalently attached to the amino group of the side chain of Xaa4,
   or wherein, if the bio-distribution modifier is comprised by Xaa5 comprising an amino group in the side chain, the carbonyl group of the aliphatic carboxylic acid of the bio-distribution modifier is covalently attached to the amino group of the side chain of Xaa5,
   or wherein, if the bio-distribution modifier is comprised by Xaa8 comprising an amino group in the side chain, the carbonyl group of the aliphatic carboxylic acid of the bio-distribution modifier is covalently attached to the amino group of the side chain of Xaa8, and
wherein the bio-distribution modifier comprised by the C-terminal modification group C-term comprises a residue of an α-amino acid with an aliphatic side chain, wherein to an ω-carbon atom of the side chain of the α-amino acid a nitrogen atom, a triazolyl moiety, a hydroxymethyl-triazolyl moiety or an urea moiety is covalently attached,
   wherein to the nitrogen atom, to the triazolyl moiety, to the hydroxymethyl triazolyl moiety or to the urea moiety
   (a) 1 or 2 first level alkyl groups are covalently attached, wherein each and any first level alkyl group individually and independently comprises 2 or more hydroxyl groups, wherein each and any hydroxyl group is a primary hydroxyl group,
   or (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
and wherein
   (a) the carbonyl group of Xaa11 is covalently attached to the α-nitrogen atom of the α-amino acid of the bio-distribution modifier,
   or (b) if a linker comprising an amino group and a carbonyl group is interspersed between the Xaa11 and the bio-distribution modifier, the carbonyl group of Xaa11 is covalently attached to the amino group of the linker and the carbonyl group of the linker is covalently attached to the α-nitrogen atom of the α-amino acid,
   and wherein to the carbonyl group of the α-amino acid of the bio-distribution modifier an amino group is covalently attached thereby forming an amide.

Embodiment 2. The compound of Embodiment 1,
wherein
Xaa1 is a residue of an α-amino acid comprising a side chain, wherein the side chain comprises an acidic or polar group, preferably Xaa1 is a residue of an α-amino acid of formulae (IIa), (IIb) or (IIc), more preferably Xaa1 is a residue of an α-amino acid of formula (IIa), 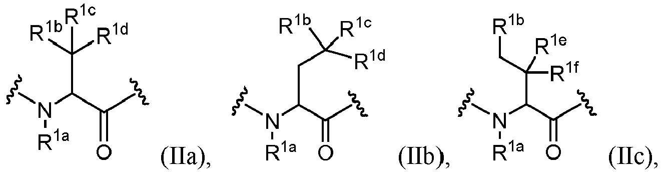
wherein
   the carbonyl group of the aliphatic carboxylic acid comprised by the bio-distribution modifier comprised by the N-terminal modification group A is covalently attached to the α-nitrogen atom of Xaa1, or if a linker comprising an amino group and a carbonyl group is interspersed between the bio-distribution modifier comprised by the N-terminal modification group A and Xaa1, the carbonyl group of the linker is covalently attached to the α-nitrogen atom of Xaa1,
      - R^{1a}: is selected from the group consisting of H and (C₁-C₄)alkyl,
      - R^{1b}: is selected from the group consisting of CO₂H, SO₃H, OPO₃H₂, CONH₂ and OH,
      - R^{1c}: is selected from the group consisting of H, OH and (C₁-C₂)alkyl under the proviso that R^{1b} is selected from the group consisting of CO₂H, SO₃H and CONH₂, or is selected from the group consisting of H and (C₁-C₂)alkyl under the proviso that R^{1b} is selected from the group consisting of OH and OPO₃H₂,
      - R^{1d}: is selected from the group consisting of H and (C₁-C₂)alkyl, preferably if R^{1c} is (C₁-C₂)alkyl, R^{1d} is the same (C₁-C₂)alkyl,
      - R^{1e}: is selected from the group consisting of H, OH and (C₁-C₂)alkyl,
      - R^{1f}: is selected from the group consisting of H and (C₁-C₂)alkyl, preferably if R^{1e} is (C₁-C₂)alkyl, R^{1f} is the same (C₁-C₂)alkyl,
   and the carbonyl group of Xaa1 is covalently attached to the α-nitrogen atom of Xaa2,
Xaa2 is (a) a residue of an α-amino acid comprising a side chain, wherein the side chain comprises a heteroaryl ring or an aryl ring, preferably Xaa2 is a residue of an α-amino acid of formulae (IIIa) or (IIIb), more preferably a residue of an α-amino acid of formula (IIIa), 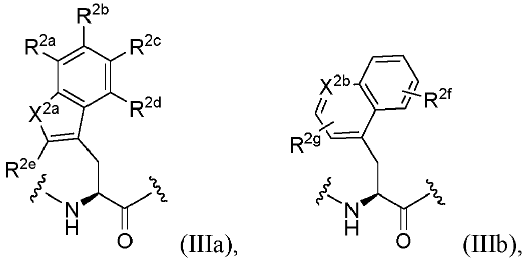
wherein
   - X^{2a}: is selected from the group consisting of NH, NCH₃ and S, preferably selected from the group consisting of NH and S,
   - X^{2b}: is selected from the group consisting of N, C-H, C-F and C-CH₃, preferably selected from the group consisting of N and C-H,
   - R^{2a}: is selected from the group consisting of H, a halogen, methyl and OCH₃, preferably R^{2a} is selected from the group consisting of H and F,
   - R^{2b}: is selected from the group consisting of H, a halogen, methyl and OCH₃, preferably the halogen is selected from the group consisting of Cl and Br, more preferably R^{2b} is selected from the group consisting of H, Cl and Br,
   - R^{2c}: is selected from the group consisting of H, OH, a halogen, methyl and OCH₃, preferably the halogen is selected from the group consisting of F, Cl and Br, more preferably R^{2c} is selected from the group consisting of H, F and OH,
   - R^{2d}: is selected from the group consisting of H, a halogen, methyl and OCH₃, wherein the halogen is selected from the group consisting of F, Cl and Br, preferably R^{2d} is H,
   - R^{2e}: is selected from the group consisting of H, a halogen and methyl, preferably the halogen is Cl, more preferably R^{2e} is H,
   - R^{2f}: is selected from the group consisting of H and OCH₃, preferably R^{2f} is H,
   - R^{2g}: is selected from the group consisting of H, OH, methyl and OCH₃, preferably R^{2g} is H,
or (b), under the proviso that Xaa3 is of formulae (IVa) or (IVb), preferably under the proviso that Xaa3 is of formula (IVa), Xaa2 is a residue of an α-amino acid comprising a side chain, wherein the side chain comprises an aryl ring, preferably Xaa2 is a residue of an α-amino acid of formula (IIIc) 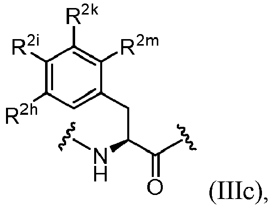
wherein
   - R^{2h}: is selected from the group consisting of H, methyl, CF₃, OH, OCH₃, OCF₃, CONH₂ and a halogen, preferably the halogen is selected from the group consisting of Cl and Br, more preferably R^{2h} is H, Cl or Br,
   - R²ⁱ: is selected from the group consisting of H, methyl, C(CH₃)₃, CF₃, OH, OCH₃, OCH₂CH₃, OCF₃, CONH₂, CO₂H and a halogen, preferably the halogen is selected from the group consisting of Cl and Br, more preferably R²ⁱ is selected from the group consisting of H, Cl and Br,
   - R^{2k}: is selected from the group consisting of H, methyl, CF₃, OH, OCH₃, OCF₃, CONH₂ and a halogen, preferably the halogen is selected from the group consisting of Cl and Br, more preferably R^{2k} is selected from the group consisting of H, Cl and Br,
   - R^{2m}: is selected from the group consisting of H, methyl, CF₃, OH, OCH₃, OCF₃, CONH₂ and a halogen, preferably the halogen is selected from the group consisting of Cl and Br, more preferably R^{2m} is selected from the group consisting of H, Cl and Br,
and the carbonyl group of Xaa2 is covalently attached to the α-nitrogen atom of Xaa3,
Xaa3 is (a) a residue of an α-amino acid comprising a side chain, wherein the side chain comprises a heteroaryl ring or an aryl ring, preferably Xaa3 is a residue of an α-amino acid of formulae (IVa) or (IVb), more preferably Xaa3 is a residue of an α-amino acid of formula (IVa), 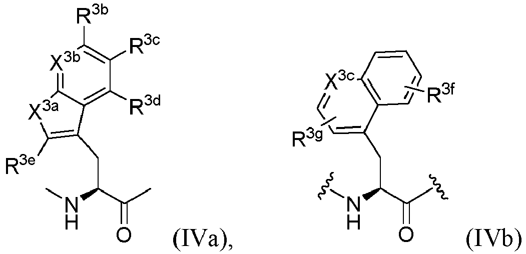
wherein
   - X^{3a}: is selected from the group consisting of NH, NCH₃ and S, preferably X^{3a} is selected from the group consisting of NH and S,
   - X^{3b}: is selected from the group consisting of C-H, C-F, C-Cl, C-Br, C-CH₃, C-OCH₃ and N, preferably X^{3b} is selected from the group consisting of C-H and C-F,
   - X^{3c}: is selected from the group consisting of C-H and N, preferably X^{3c} is C-H,
   - R^{3b}: is selected from the group consisting of H, a halogen, methyl and OCH₃, preferably the halogen is selected from the group consisting of F and Cl, more preferably R^{3b} is selected from the group consisting of H and F,
   - R^{3c}: is selected from the group consisting of H, a halogen, methyl and OCH₃, preferably the halogen is selected from the group consisting of Cl, Br and F, more preferably R^{3c} is selected from the group consisting of Cl and Br,
   - R^{3d}: is selected from the group consisting of H, a halogen, methyl and OCH₃, preferably the halogen is F,
   - R^{3e}: is selected from the group consisting of H, a halogen and methyl, preferably the halogen is Cl,
   - R^{3f}: is selected from the group consisting of H and OCH₃,
   - R^{3g}: is selected from the group consisting of H, F, OH, methyl and OCH₃,
or (b) under the proviso that Xaa2 is an amino acid residue of formula (IIIa), Xaa3 is a residue of an α-amino acid comprising a side chain, wherein the side chain comprises an aryl ring, preferably Xaa3 is a residue of an α-amino acid of formulae (IVc) or (IVd) 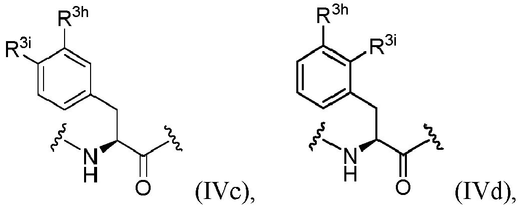
wherein
   - R^{3h}: is selected from the group consisting of a halogen, OH, methyl, CF₃, OCH₃, OCF₃ and CONH₂, preferably the halogen is selected from the group consisting of Cl and Br,
   - R³ⁱ: is selected from the group consisting of a halogen, OH, methyl, CF₃, OCH₃, OCF₃ and CONH₂, preferably the halogen is selected from the group consisting of Cl and Br,
and the carbonyl group of Xaa3 is covalently attached to the α-nitrogen atom of Xaa4,
Xaa4 is (a) a residue of a cyclic non-aromatic α-amino acid, preferably Xaa4 is a residue of an α-amino acid of formula (Va) 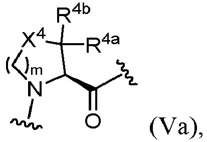
wherein
   - R^{4a}: is selected from the group consisting of H, OH, methyl and CF₃,
   - R^{4b}: is selected from the group consisting of H and methyl, preferably R^{4b} is methyl if R^{4a} is methyl,
   - X⁴: is selected from the group consisting of CHR^{4c}, S and O,
   wherein R^{4c} is selected from the group consisting of NH₂, OH, H, NHR^{4d}, methyl and F, preferably R^{4c} is NH₂ or OH,
   wherein R^{4d} is selected from the group consisting of Ac and a Z group,
   - m: is 1 or 2, preferably m is 1,
or (b) a residue of an N-alkylated α-amino acid, preferably Xaa4 is a residue of an N-alkylated α-amino acid of formula (Vb) 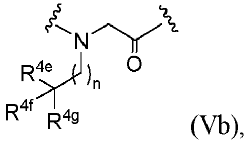
wherein
   - n: is 0, 1, 2, 3, 4, or 5, preferably n is 0, 1 or 3,
   - R^{4e}: is, if n is 0, selected from the group consisting of H, methyl, an aryl ring, COOH, CONH₂ and C(=O)R^{4h},
   or is, if n is 1, 2, 3, 4 or 5, selected from the group consisting of H, methyl, an aryl ring, OH, NH₂, COOH, CONH₂ and NHR^{4h},
   - R^{4f}: is selected from the group consisting of H and (C₁-C₂)alkyl,
   - R^{4g}: is selected from the group consisting of H and methyl,
   - R^{4h}: is a Z group, preferably the Z group is a bio-distribution modifier,
or (c) Xaa4 is a residue of a bicyclic non-aromatic α-amino acid, preferably Xaa4 is a residue of a bicyclic non-aromatic α-amino acid of formula (Vc) 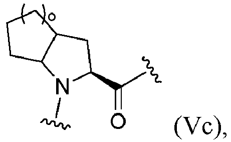
wherein
   - o: is 1 or 2,
and the carbonyl group of Xaa4 is covalently attached to the α-nitrogen atom of Xaa5,
Xaa5 is (a) a residue of an α-amino acid optionally comprising a Z group, wherein the α-nitrogen atom of the α-amino acid is optionally substituted by (C₁-C₄)alkyl or (b) a cyclic α-amino acid, and the carbonyl group of Xaa5 is covalently attached to the α-nitrogen atom of Xaa6,
Xaa6 is a residue of an α-amino acid comprising a side chain, wherein the side chain comprises a branched aliphatic group, preferably Xaa6 is a residue of an α-amino acid of formula (VIIa) 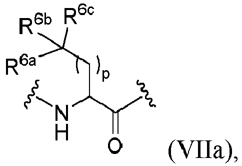
wherein
   - p: is 1, 0 or 2, preferably p is 1,
   - R^{6a}: is (C₁-C₂)alkyl,
   - R^{6b}: is methyl,
   - R^{6c}: is H, if p is 0,
   or is selected from the group consisting of H and methyl, if p is 1 or 2,
and the carbonyl group of Xaa6 is covalently attached to the α-nitrogen atom of Xaa7,
Xaa7 is a residue of an α-amino acid comprising a side chain, wherein the side chain comprises a nucleophilic group, wherein the nucleophilic group comprises a heteroatom, wherein the heteroatom is selected from the group comprising a sulfur atom and a nitrogen atom, preferably Xaa7 is a residue of an α-amino acid of formula (Villa) 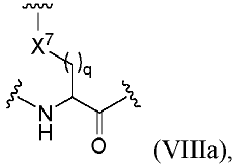
wherein
   - X⁷: comprises the heteroatom and is selected from the group consisting of-S- and -NH-,
   - q: is 1 or 2, preferably q is 1,
and the carbonyl group of Xaa7 is covalently attached to the α-nitrogen atom of Xaa8,
Xaa8 is (a) a residue of an aliphatic cyclic α-amino acid, of an aliphatic heterocyclic α-amino acid or of an aliphatic cyclic α-amino acid comprising an annulated aromatic ring, preferably Xaa8 is a residue of an α-amino acid of formula (IXa) or (IXb), more preferably Xaa8 is a residue of an α-amino acid of formula (IXa), 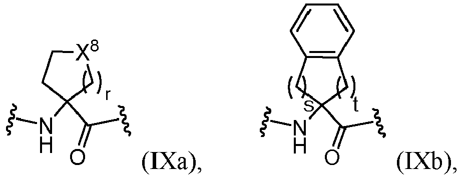
wherein
   - X⁸: is selected from the group consisting of NR^{8a}, O, NH, and CH₂,
   wherein R^{8a} is a Z group or R^{8a} is acetyl,
   wherein if R^{8a} is a Z group, the Z group preferably is a chelator optionally comprising a linker,
   - r: is 2 or 1, preferably r is 2,
   - s: is 1 or 2, preferably s is 1,
   - t: is 1 or 2, preferably t is 1 if s is 1, and t is 2 if s is 2,
or (b) a residue of an α-amino acid or an α-alkyl-α-amino acid, preferably Xaa8 is a residue of an α-amino acid of formula (IXc), 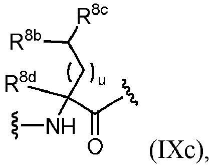
wherein
   - R^{8b}: is selected from the group consisting of H, OH, NH₂, NHR^{8e}, (C₁-C₂)alkyl, COOH, CONH₂, an aryl ring and a heteroaryl ring, preferably the aryl ring is phenyl and the heteroaryl ring is 3-indoyl, wherein R^{8e} is a Z group, preferably the Z group is a chelator optionally comprising a linker,
   - R^{8c}: is selected from the group consisting of H and methyl, if R^{8b} is selected from the group consisting of OH, NH₂ and NHR^{8e},
   or is selected from the group consisting of H, methyl and OH, if R^{8b} is selected from the group consisting of H, (C₁-C₂)alkyl, COOH, CONH₂, an aryl ring and a heteroaryl ring,
   - R^{8d}: is selected from the group consisting of H and (C₁-C₂)alkyl,
   - u: is 0, 1, 2, 3, 4, or 5, preferably u is 0, 1 or 3,
and the carbonyl group of Xaa8 is covalently attached to the α-nitrogen atom of Xaa9,
Xaa9 is a residue of an acyclic α-amino acid, wherein the acyclic α-amino acid is in L-configuration and the carbonyl group of Xaa9 is covalently attached to the α-nitrogen atom of Xaa10,
Xaa10 is a residue of an α-amino acid comprising a side chain, wherein the side chain comprises a branched aliphatic group or an unsubstituted aromatic ring, preferably Xaa10 is a residue of an α-amino acid of formula (XIa), 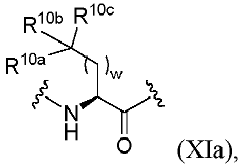
wherein
   - R^{10a}: is selected from the group consisting of (C₁-C₂)alkyl and phenyl,
   - R^{10b}: is selected from the group consisting of methyl and H,
   - R^{10c}: is selected from the group consisting of H and methyl,
   - w: is 0 or 1,
and the carbonyl group of Xaa10 is covalently attached to the α-nitrogen atom of Xaa11,
Xaa11 is a residue of an α-amino acid comprising a side chain, wherein the side chain comprises a nucleophilic group, wherein the nucleophilic group comprises a heteroatom, wherein the heteroatom is selected from the group comprising a sulfur atom and a nitrogen atom, preferably Xaa11 is an α-amino acid of formula (XIIa), 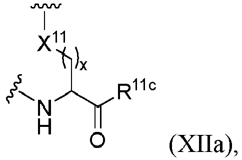
wherein
   - X¹¹: comprises the heteroatom and is selected from the group consisting of -S- and -NH-,
   - R^{11c}: is the C-terminal group C-term,
   - x: is 1 or 2,
and wherein a linker is optionally interspersed between the bio-distribution modifier comprised by the N-terminal modification group A and Xaa1, wherein the bio-distribution modifier comprises a residue of an aliphatic carboxylic acid,
   wherein to the ω-carbon atom of the aliphatic carboxylic acid a nitrogen atom, a triazolyl moiety, a hydroxymethyl-triazolyl moiety or an urea moiety is covalently attached,
      wherein to the nitrogen atom, to the triazolyl moiety, to the hydroxymethyl triazolyl moiety or to the urea moiety
      (a) 1 or 2 first level alkyl groups are covalently attached, wherein each and any first level alkyl group individually and independently comprises 2 or more hydroxyl groups, wherein each and any hydroxyl group is a primary hydroxyl group,
   or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
   preferably the bio-distribution modifier is a structure of formula (A-I), 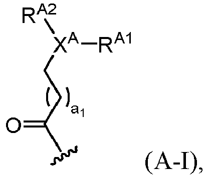
   wherein
      - X^{A}: comprises a nitrogen atom, a triazolyl moiety, a hydroxymethyl-triazolyl moiety or an urea moiety,
      - a₁: is 0, 1, 2, 3, 4, 5, or 6, if X^{A} is a triazolyl moiety, preferably a₁ is 2,
      if X^{A} is selected from the group comprising a nitrogen atom, a hydroxymethyl-triazolyl moiety and an urea moiety, preferably a₁ is 4,
      - R^{A1}: is a first level alkyl group of a structure of formula (A-II), 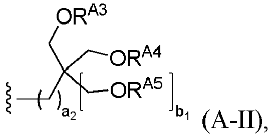
      wherein
      - a₂: is 0 or 1,
      - b₁: is 0 or 1,

      wherein, if b₁ is 0,
         R^{A3} and R^{A4} are individually and independently a second level alkyl group of a structure of formula (A-III),
      or R^{A3} and R^{A4} are each H,
      or wherein, if b₁ is 1,
      each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), 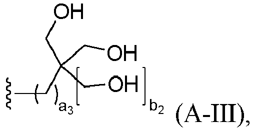 wherein
      - a₃: is 0 or 1, and
      - b₂: is 0 or 1,
      and

      - R^{A2}: is absent, if X^{A} is selected from the group comprising a triazolyl moiety, a hydroxmethyl-triazolyl moiety and an urea moiety,
      - or R^{A2}: is H or a first level alkyl group of a structure of formula (A-II), if X^{A} is a nitrogen atom, wherein
      a₂ is 0 or 1,
      b₁ is 0 or 1,
      wherein if b₁ is 0,
      R^{A3} and R^{A4} are individually and independently a second level alkyl group of a structure of formula (A-III)
      or R^{A3} and R^{A4} are each H,
      or wherein if b₁ is 1,
      each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), wherein in formula (A-III)
      - as: is 0 or 1, and
      - b₂: is 0 or 1,
and wherein the carbonyl group of the structure (A-I) is covalently attached to the α-nitrogen atom of Xaa1,
   or if a linker, comprising an amino group and a carbonyl group, is interspersed between the bio-distribution modifier comprised by the N-terminal modification group A and Xaa1, the carbonyl group of the structure (A-I) is covalently attached to the amino group of the linker,
and wherein a linker is optionally interspersed between Xaa11 and the bio-distribution modifier comprised by the C-terminal modification group C-term,
   wherein the bio-distribution modifier comprises a residue of an α-amino acid with an aliphatic side chain,
   wherein to an ω-carbon atom of the aliphatic side chain of the α-amino acid a nitrogen atom, a triazolyl moiety, a hydroxymethyl-triazolyl moiety or an urea moiety is covalently attached,
      wherein to the nitrogen atom, to the triazolyl moiety, to the hydroxymethyl triazolyl moiety or to the urea moiety
      (a) 1 or 2 first level alkyl groups are covalently attached, wherein each and any first level alkyl group individually and independently comprises 2 or more hydroxyl groups, wherein each and any hydroxyl group is a primary hydroxyl group,
   or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
      and wherein to the carbonyl group attached to the α-carbon atom of the α-amino acid an amino group is covalently attached thereby forming an amide,
   preferably the bio-distribution modifier is a structure of formula (C-I), 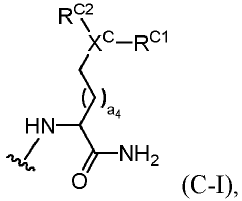
   wherein
      - X^{C}: comprises a nitrogen atom, a triazolyl moiety, a hydroxymethyl-triazolyl moiety and an urea moiety,
      - a₄: is 0, 1, 2, 3, or 4,

      if X^{C} is a triazolyl moiety, preferably a₄ is 1 or 2, more preferably a₄ is 1 if X^{C} is selected from the group comprising a nitrogen atom, a hydroxymethyl-triazolyl moiety and an urea moiety, preferably a₄ is 3,
         R^{C1} is a first level alkyl group of a structure of formula (C-II), 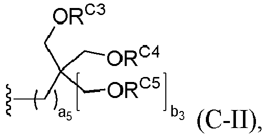
      wherein
         - as: is 0 or 1,
         - b₃: is 0 or 1,

         wherein, if b₃ is 0,
         R^{C3} and R^{C4} are individually and independently a second level alkyl group of a structure of formula (C-III)
         or R^{C3} and R^{C4} are each H,
            or wherein, if b₃ is 1, each and any of R^{C3}, R^{C4} and R^{C5} is selected from a second level alkyl group of a structure of formula (C-III) and H, preferably each and any of R^{C3}, R^{C4} and R^{C5} is a second level alkyl group of a structure of formula (C-III), 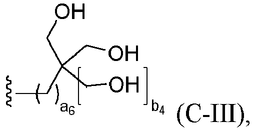 wherein
               - a₆: is 0 or 1, and
               - b₄: is 0 or 1

               - and R^{C2}: is absent, if X^{C} is selected from the group comprising a triazolyl moiety, a hydroxmethyl-triazolyl moiety and an urea moiety,
               - or R^{C2}: is H or is a first level alkyl group of a structure of formula (C-II), if X^{C} is a nitrogen atom, wherein
               as is 0 or 1,
               b₃ is 0 or 1,
            wherein, if b₃ is 0,
               R^{C3} and R^{C4} are individually and independently a second level alkyl group of a structure of formula (C-III)
               or R^{C3} and R^{C4} are H,
            or wherein, if b₃ is 1,
               each and any of R^{C3}, R^{C4} and R^{C5} is individually and independently selected from a second level alkyl group of a structure of formula (C-III) and H, preferably each and any of R^{C3}, R^{C4} and R^{C5} is individually and independently a second level alkyl group of a structure of formula (C-III), wherein in formula (C-III)
               - a₆: is 0 or 1, and
               - b₄: is 0 or 1,
         and wherein the α-nitrogen atom of the structure (C-I) is covalently attached to the carbonyl group of Xaa11, or if a linker comprising a carbonyl group and an amino group is interspersed between Xaa11 and the bio-distribution modifier comprised by the C-terminal modification group C-term, the α-nitrogen atom of the structure (C-I) is covalently attached to the carbonyl group of the linker, more preferably a linker is interspersed between Xaa11 and the bio-distribution comprised by the C-terminal modification group C-term.

Embodiment 3. The compound of any one of Embodiments 1 and 2,
wherein
Xaa7 is a residue of formula (VIIIa) 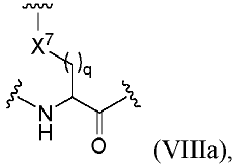 wherein
   - X⁷: is -S- or -NH-,
   - q: is 1 or 2, preferably q is 1,
Yc is a structure of formula (XIIIa) 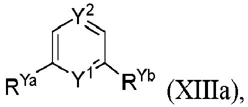 wherein
   - R^{Ya}: is -CH₂-,
   - R^{Yb}: is -CH₂-,
   - Y¹: is selected from the group consisting of N and CH,
   - Y²: is selected from the group consisting of CH and N,
and Xaa11 is a residue of formula (XIIa) 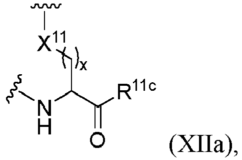 wherein
   - X¹¹: is -S- or -NH-,
   - R^{11c}: is the C-terminal group C-term,
   - x: is 1 or 2, preferably x is 1,
preferably the compound comprises a cyclic peptide of formula (Ic), 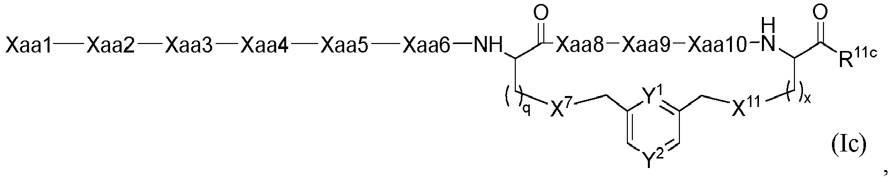
more preferably the compound comprises a cyclic peptide of formula (Ic),
wherein X⁷ is -S-, X¹¹ is -S-, Y¹ is N or CH, Y² is CH, q is 1, and x is 1,
most preferably the compound comprises a cyclic peptide of formula (Ic),
wherein X⁷ is -S-, X¹¹ is -S-, Y¹ is N, Y² is CH, q is 1, and x is 1.

Embodiment 4. The compound of Embodiment 3, wherein X⁷ is -S-, X¹¹ is -S-, Y¹ is N or CH, Y² is CH, q is 1 or 2, preferably q is 1 and, x is 1 or 2, preferably x is 1.

Embodiment 5. The compound of any one of Embodiments 3 and 4, wherein X⁷ is -S-, X¹¹ is -S-, Y¹ is N, Y² is CH, q is 1, and x is 1.

Embodiment 6. The compound of Embodiment 3, wherein Xaa7 is a residue of an α-amino acid selected from the group consisting of Cys, cys, Hcy and Dap, wherein Xaa11 is a residue selected from the group consisting of Cys, cys, Hcy and Dap, and wherein Yc is selected from the group consisting of 2Lut, 3MeBn and 3Lut.

Embodiment 7. The compound of any one of Embodiments 3, 4 and 6, wherein Xaa7 is a Cys or Hey residue, wherein Xaa11 is a Cys or Hey residue, and wherein Yc is selected from the group consisting of 2Lut and 3MeBn, preferably is 2Lut.

Embodiment 8. The compound of any one of Embodiments 3 to 7, preferably the compound of Embodiment 7, wherein Xaa7 is a Cys residue, wherein Xaa11 is a Cys residue, and wherein Yc is 2Lut.

Embodiment 9. The compound of any one of Embodiments 3 and 6, wherein X⁷ is -NH-, X¹¹ is -S-, Y¹ is N or CH, preferably Y¹ is N, Y² is CH, q is 1 or 2, preferably q is 1, and x is 1 or 2, preferably x is 1.

Embodiment 10. The compound of any one of Embodiments 3 and 9, preferably the compound of Embodiment 9, wherein Xaa7 is a Dap residue, wherein Xaa11 is a residue selected from the group consisting of Cys, cys, Hcy, and wherein Yc is selected from the group consisting of 2Lut and 3MeBn, preferably Yc is 2Lut.

Embodiment 11. The compound of any one of Embodiments 3 and 6, wherein X⁷ is -S-, X¹¹ is -NH-, Y¹ is N or CH, preferably Y¹ is N, Y² is CH, q is 1 or 2, preferably q is 1 and, x is 1

Embodiment 12. The compound of any one of Embodiments 3, 6 and 11, preferably the compound of Embodiment 11, wherein Xaa7 is a Cys or Hey residue, preferably Xaa7 is a Cys residue, wherein Xaa11 is a Dap residue, and wherein Yc is selected from 2Lut and 3MeBn, preferably Yc is 2Lut.

Embodiment 13. The compound of any one of Embodiments 3 and 6, wherein X⁷ is -NH-, X¹¹ is -NH-, Y¹ is N or CH, preferably Y¹ is N, Y² is CH, q is 1 or 2, preferably q is 1, and x is 1 or 2, preferably x is 1.

Embodiment 14. The compound of any one of Embodiments 3, 6 and 13, preferably the compound of Embodiment 13, wherein Xaa7 is a Dap residue, wherein Xaa11 is a Dap residue, and wherein Yc is is selected from the group consisting of 2Lut and 3MeBn, preferably Yc is 2Lut.

Embodiment 15. The compound of any one of Embodiments 1 and 2,
wherein
Xaa7 is a residue of formula (VIIIa) 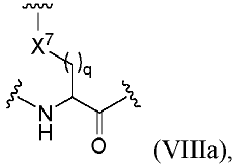 wherein
   - X⁷: is -S- or -NH-,
   - q: is 1 or 2, preferably q is 1,
Yc is a structure of formula (XIIIh) 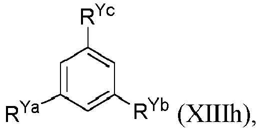 wherein
   - R^{Ya}: is -CH₂-,
   - R^{Yb}: is -CH₂-, and
   - R^{Yc}: is -CH₂-R^{Yd}, and
   - R^{Yd}: is a structure of formulae (XIIIc), (XIIId) or (XIIIe), 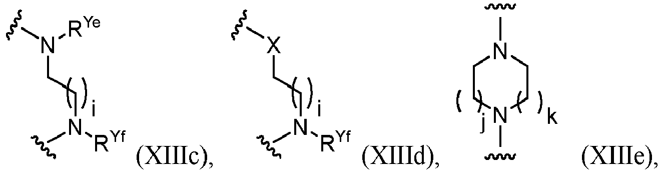
   wherein
   - R^{Ye} and R^{Yf}: are each and independently selected from the group consisting of H and (C₁-C₄)alkyl,
   - i: is each and independently 1, 2, 3, 4, 5 or 6, preferably i is 1 or 2,
   - j and k: are each and independently 1, 2 or 3, and

   - X: is O or S, preferably X is S,
   wherein
   in formulae (XIIIc) and (XIIIe)
      one of the two nitrogen atoms is covalently attached to -CH₂- of R^{Yc}
   and in formula (XIIId)
      -X- is covalently attached to -CH₂- of R^{Yc}
   while to the remaining nitrogen atom optionally a Z group is covalently attached, preferably the Z group comprises a chelator and optionally a linker,
and Xaa11 is a residue of formula (XIIa) 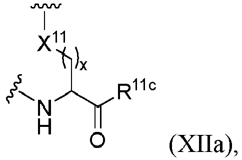 wherein
   - X¹¹: is -S- or -NH-,
   - R^{11c}: is the C-terminal group C-term,
   - x: is 1 or 2, preferably x is 1,
preferably the compound comprises a cyclic peptide of formula (Ii), 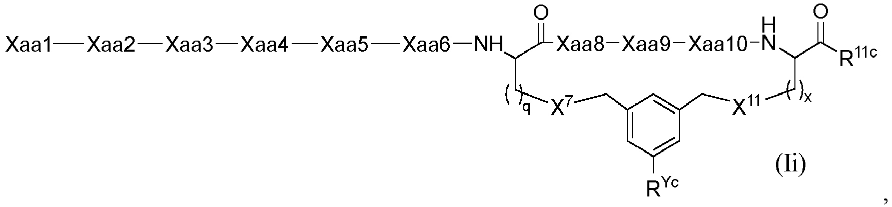
more preferably the compound comprises a cyclic peptide of formula (Ii),
   wherein
   - X⁷: is -S- and X¹¹ is -S-,
   - R^{Yc}: is -CH₂-R^{Yd}, and
   - R^{Yd}: is a structure of formulae (XIIId) or (XIIIe),
   wherein R^{Yf} is H, i is 1, j is 1, and k is 1,
   wherein in formula (XIIId) -X- is S and is attached to -CH₂- of R^{Yc}, and in formula (XIIIe) one of the two nitrogen atoms is attached to -CH₂- of RYC,
   while to the remaining nitrogen atom in either (XIIId) or (XIIIe) optionally a Z group is covalently attached, preferably the Z group comprises a chelator and optionally a linker,
   - q: is 1, and
   - x: is 1,
most preferably the compound comprises a cyclic peptide of formula (Ii), wherein R^{Yd} is a structure of formula (XIIId), wherein -X- is S and R^{Yf} is H, and i is 1.

Embodiment 16. The compound of Embodiment 15, wherein Xaa7 is a Cys residue, wherein Xaa11 is a Cys residue, and wherein Yc is tMeBn(DOTA-AET) or tMeBn(DOTA-PP).

Embodiment 17. The compound of any one of Embodiments 1 to 2,
wherein
Xaa7 is a residue of formula (VIIIa) 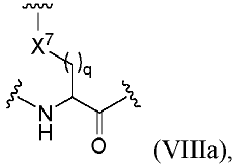 wherein
   - X⁷: is -S- or -NH-,
   - q: is 1 or 2, preferably q is 1,
Yc is a structure of formula (XIIIb) 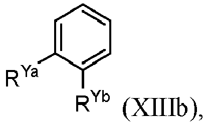 wherein
   - R^{Ya}: is -CH₂-,
   - R^{Yb}: is -CH₂-,
and Xaa11 is a residue of formula (XIIa) 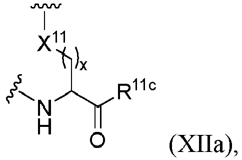 wherein
   - X¹¹: is -S- or -NH-,
   - R^{11c}: is the C-terminal group C-term,
   - x: is 1 or 2, preferably x is 1,
preferably the compound comprises a cyclic peptide of formula (Id), 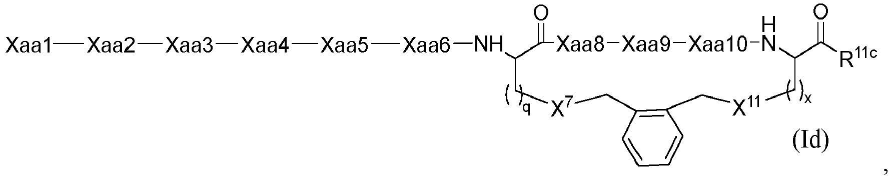
more preferably the compound comprises a cyclic peptide of formula (Id),
   wherein X⁷ is -S-, X¹¹ is -S-, q is 1, and x is 1.

Embodiment 18. The compound of Embodiment 17, wherein X⁷ is -S-, X¹¹ is -S-, q is 1 or 2, preferably q is 1, and x is 1 or 2, preferably x is 1.

Embodiment 19. The compound of any one of Embodiments 17 and 18, preferably the compound of Embodiment 18, wherein Xaa7 is a Cys or Hey residue, wherein Xaa11 is a Cys or Hey residue, and wherein Yc is 2MeBn.

Embodiment 20. The compound of Embodiment 17, wherein X⁷ is -NH-, X¹¹ is -S-, q is 1 or 2, preferably q is 1, and x is 1 or 2, preferably x is 1.

Embodiment 21. The compound of any one of Embodiments 17 and 20, preferably the compound of Embodiment 20, wherein Xaa7 is a Dap residue, wherein Xaa11 is a Cys or Hey residue, and wherein Yc is 2MeBn.

Embodiment 22. The compound of Embodiment 17, wherein X⁷ is -S-, X¹¹ is -NH-, q is 1 or 2, preferably q is 1, and x is 1 or 2, preferably x is 1.

Embodiment 23. The compound of any one of Embodiments 17 and 22, preferably the compound of Embodiment 22, wherein Xaa7 is a Cys or Hey residue, wherein Xaa11 is a Dap residue, and wherein Yc is 2MeBn.

Embodiment 24. The compound of Embodiment 17, wherein X⁷ is -NH-, X¹¹ is -NH-, q is 1 or 2, preferably q is 1, and x is 1 or 2, preferably x is 1.

Embodiment 25. The compound of any one of Embodiments 17 and 24, preferably the compound of Embodiment 24, wherein Xaa7 is a Dap residue, wherein Xaa11 is a Dap residue, and wherein Yc is 2MeBn.

Embodiment 26. The compound of any one of Embodiments 1 and 2,
wherein
Xaa7 is a residue of formula (VIIIb) 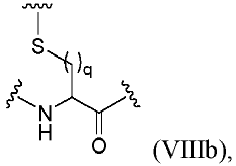 wherein
   - q: is 1 or 2, preferably q is 1,
Yc is a structure of formula (XIIIg) 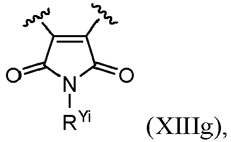 wherein
   - R^{Yi}: is selected from the group consisting of H and (C₁-C₆)alkyl,
and Xaa11 is a residue of formula (XIIb) 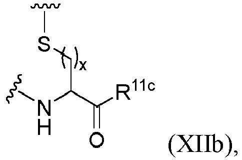 wherein
   - R^{11c}: is the C-terminal group C-term,
   - x: is 1 or 2, preferably x is 1,
preferably the compound comprises a cyclic peptide of formula (Ig), 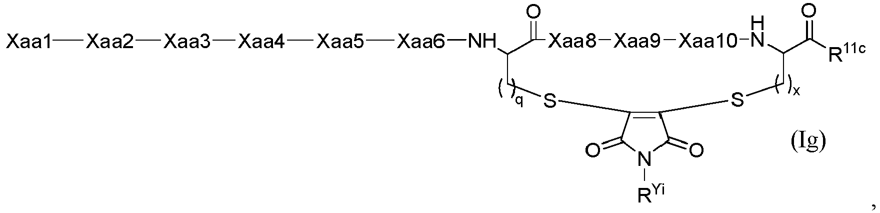
more preferably the compound comprises a cyclic peptide of formula (Ig),
   wherein q is 1, and x is 1 or 2.

Embodiment 27. The compound of Embodiment 26, wherein Xaa7 is a Cys residue, wherein Xaa7 is selected from the group comprising Cys and Hcy, and wherein Yc is mli.

Embodiment 28. The compound of any one of Embodiments 1 and 2,
wherein
Yc is a structure of formula (XIIIf) 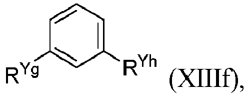 wherein
   - R^{Yg}: is -C(=O)-,
   - R^{Yh}: is -CH₂-,
Xaa7 is a residue of formula (VIIIa) 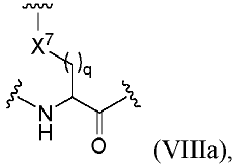 wherein
   - X⁷: is -S- or -NH- and is covalently attached to R^{Yh},
   - q: is 1 or 2, preferably q is 1,
and Xaa11 is a residue of formula (XIIa) 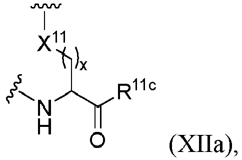 wherein
   - X¹¹: is -NH- and is covalently attached to R^{Yg},
   - R^{11c}: is the C-terminal group C-term,
   - x: is 1 or 2, preferably x is 1,
preferably the compound comprises a cyclic peptide of formula (If), 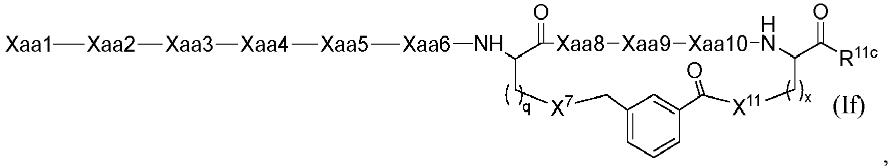
more preferably the compound comprises a cyclic peptide of formula (If),
   wherein X⁷ is -S-, q is 1, and x is 1.

Embodiment 29. The compound of Embodiment 28, wherein X⁷ is -S-, q is 1 or 2, preferably q is 1, and x is 1 or 2, preferably x is 1.

Embodiment 30. The compound of any one of Embodiments 28 and 29, preferably the compound of Embodiment 29, wherein Xaa7 is a Cys or Hey residue, wherein Xaa11 is a Dap residue, and wherein Yc is 3CbBn.

Embodiment 31. The compound of Embodiment 28, wherein X⁷ is -NH-, q is 1 or 2, preferably q is 1, and x is 1 or 2, preferably x is 1.

Embodiment 32. The compound of Embodiment 28, wherein Xaa7 is a Dap residue, wherein Xaa11 is a Dap residue, and wherein Yc is 3CbBn.

Embodiment 33. The compound of any one of Embodiments 1 and 2,
wherein
Yc is a structure of formula (XIIIf) 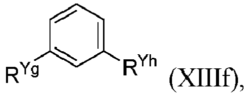 wherein
   - R^{Yg}: is -C(=O)-,
   - R^{Yh}: is -CH₂-,
Xaa7 is a residue of formula (VIIIa) 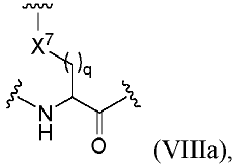 wherein
   - X⁷: is -NH- and is covalently attached to R^{Yg},
   - q: is 1 or 2, preferably q is 1,
and Xaa11 is a residue of formula (XIIa) 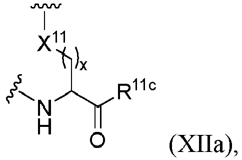 wherein
   - X¹¹: is -S- or -NH- and is covalently attached to R^{Yh},
   - R^{11c}: is the C-terminal group C-term,
   - x: is 1 or 2, preferably x is 1,
preferably the compound comprises a cyclic peptide of formula (Ie), 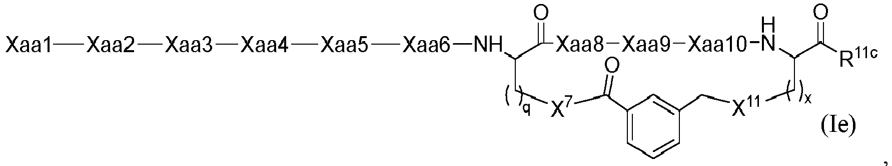
more preferably the compound comprises a cyclic peptide of formula (Ie),
   wherein X¹¹ is -S-, q is 1, and x is 1.

Embodiment 34. The compound of Embodiment 33, wherein X¹¹ is -S-, q is 1 or 2, preferably q is 1, and x is 1 or 2, preferably x is 1.

Embodiment 35. The compound of any one of Embodiments 33 and 34, preferably the compound of Embodiment 34, wherein Xaa7 is a Dap residue, wherein Xaa11 is Cys or Hey residue, and wherein Yc is 3MeBz.

Embodiment 36. The compound of Embodiment 33, wherein X¹¹ is -NH-, q is 1 or 2, preferably q is 1, and x is 1 or 2, preferably x is 1.

Embodiment 37. The compound of any one of Embodiments 33 and 36, preferably the compound of Embodiment 36, wherein Xaa7 is a Dap residue, wherein Xaa11 is a Dap residue, and wherein Yc is 3MeBz.

Embodiment 38. The compound of any one of Embodiments 1 and 2,
wherein
the compound comprises a cyclic peptide of formula (Ib),
Xaa7 is a residue of formula (VIIIc) 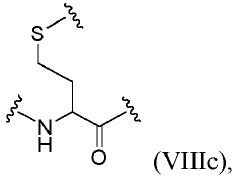
and Xaa11 is a residue of formula (XIIc) 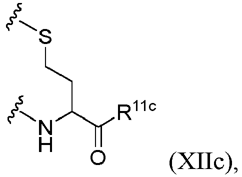 wherein
   - R^{11c}: is the C-terminal group C-term,
preferably the compound comprises a cyclic peptide of formula (Ih),

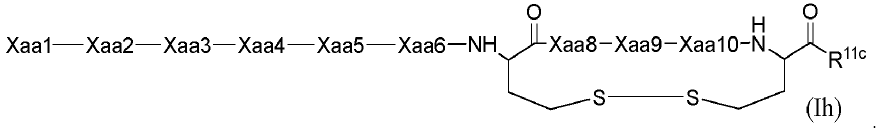

Embodiment 39. The compound of Embodiment 38, wherein Xaa7 is a Hcy residue and Xaa11 is a Hcy residue.

Embodiment 40. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38 and 39, wherein Xaa1 is a residue of an α-amino acid comprising a side chain, wherein the side chain comprises an acidic or polar group within the side chain, preferably Xaa1 is a residue of an α-amino acid of formulae (IId) or (IIe), more preferably Xaa1 is a residue of an α-amino acid formula (IId), 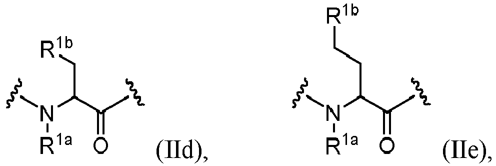 wherein
- R^{1a}: is selected from the group consisting of H and methyl,
- R^{1b}: is selected from the group consisting of CO₂H, CONH₂, OH, SO₃H and OPO₃H₂,
and wherein the carbonyl group of the aliphatic carboxylic acid comprised by the bio-distribution modifier comprised by the N-terminal modification group A is covalently attached to the α-nitrogen atom Xaa1, or if a linker comprising an amino group and a carbonyl group is interspersed between the bio-distribution modifier comprised by the N-terminal modification group A and Xaa1, a carbonyl group of the linker is covalently attached to the α-nitrogen atom of Xaa1.

Embodiment 41. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 and 40, wherein Xaa1 is a residue of an α-L-amino acid comprising a side chain, wherein the side chain comprises an acidic or polar group within the side chain, Xaa1 is a residue of an α-L-amino acid of formulae (IIf) or (IIg), preferably Xaa1 is a residue of an α-L-amino acid of formula (IIf), 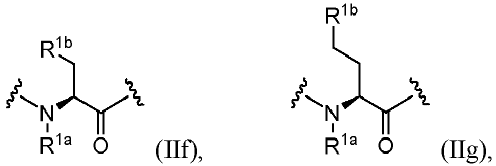 wherein
- R^{1a}: is selected from the group consisting of H and methyl,
- R^{1b}: is selected from the group consisting of CO₂H, CONH₂, OH, SO₃H and OPO₃H₂,
and wherein the carbonyl group of the aliphatic carboxylic acid comprised by the bio-distribution modifier comprised by the N-terminal modification group A is covalently attached to the α-nitrogen atom Xaa1, or if a linker comprising an amino group and a carbonyl group is interspersed between the bio-distribution modifier comprised by the N-terminal modification group A and Xaa1, a carbonyl group of the linker is covalently attached to the α-nitrogen atom of Xaa1.

Embodiment 42. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 and 41, preferably the compound of Embodiment 41, wherein Xaa1 is a residue of an α-L-amino acid comprising a side chain, wherein the side chain comprises an acidic or polar group within the side chain, Xaa1 is a residue of an α-L-amino acid of formulae (IIf) or (IIg), preferably Xaa1 is a residue of an α-L-amino acid of formula (IIf),
wherein
- R^{1a}: is H,
- R^{1b}: is selected from the group consisting of CO₂H, CONH₂, OH and SO₃H,
and wherein the carbonyl group of the aliphatic carboxylic acid comprised by the bio-distribution modifier comprised by the N-terminal modification group A is covalently attached to the α-nitrogen atom Xaa1.

Embodiment 43. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41 and 42, preferably the compound of Embodiment 42, wherein Xaa1 is a residue of an α-L-amino acid comprising a side chain, wherein the side chain comprises an acidic or polar within the side chain, Xaa1 is a residue of an α-L-amino acid of formulae (IIf) or (IIg), preferably Xaa1 is a residue of an α-L-amino acid of formula (IIf),
wherein
- R^{1a}: is H,
- R^{1b}: is CO₂H or CONH₂,
and wherein the carbonyl group of the aliphatic carboxylic acid comprised by the bio-distribution modifier comprised by the N-terminal modification group A is covalently attached to the α-nitrogen atom Xaa1.

Embodiment 44. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 and 41, wherein Xaa1 is a residue of an α-L-amino acid selected from the group consisting of Asp, Asn, Glu, Gln, Hse, Cya, Pse, Nmd and Ser.

Embodiment 45. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42 and 44, preferably the compound of Embodiment 44, wherein Xaa1 is a residue of an α-L-amino acid selected from the group consisting of Asp, Asn, Glu, Gln, Hse, Cya, and Ser.

Embodiment 46. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44 and 45, preferably the compound of any one of Embodiments 44 and 45, wherein Xaa1 is a residue of an α-L-amino acid selected from the group consisting of Asp, Asn, Glu, and Gln.

Embodiment 47. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45 and 46, preferably the compound of any one of Embodiments 44 to 46, wherein Xaa1 is an Asp residue.

Embodiment 48. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46 and 47, wherein Xaa2 is a residue of an α-L-amino acid of formula (IIId), 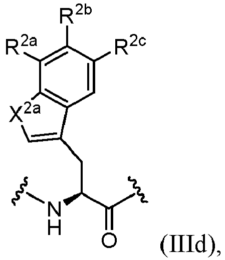 wherein
- X^{2a}: is NH or S, preferably is NH,
- R^{2a}: is H or F,
- R^{2b}: is selected from the group consisting of H, F and Cl, and
- R^{2c}: is selected from the group consisting of H, F, Cl, Br and OH.

Embodiment 49. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 and 48, preferably the compound of Embodiment 48, wherein Xaa2 is a residue of an α-L-amino acid of formula (IIId),
wherein
- X^{2a}: is NH,
- R^{2a}: is H or F,
- R^{2b}: is H,
- R^{2c}: is H, F or OH.

Embodiment 50. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 and 48, wherein Xaa2 is a residue of an α-L-amino acid selected from the group consisting of Trp, Hyw, 5Fw, 6Clw, 7Fw, Bta, 5Clw, 5Brw and 6Fw.

Embodiment 51. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 and 50, preferably the compound of Embodiment 50, wherein Xaa2 is a residue of an α-L-amino acid selected from the group consisting of Trp, Hyw, 5Fw, 6Clw, 7Fw, 5Clw, 5Brw and 6Fw.

Embodiment 52. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 and 51, preferably the compound of any one of Embodiments 50 and 51, wherein Xaa2 is a residue of an α-L-amino acid selected from the group consisting of Trp, Hyw, 5Fw and 7Fw.

Embodiment 53. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51 and 52, preferably the compound of any one of Embodiment 50, 51 and 52, wherein Xaa2 is a Trp residue.

Embodiment 54. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46 and 47, wherein Xaa2 is 1Ni.

Embodiment 55. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46 and 47, wherein under the proviso that Xaa3 is of formulae (IVa) or (IVb), preferably under the proviso that Xaa3 is of formula (IVa), Xaa2 is a residue of an α-L-amino acid of formula (IIIc) 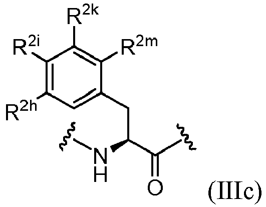 wherein
- R^{2h}: is H or Cl,
- R²ⁱ: is H or Cl,
- R^{2k}: is H or Cl,
- R^{2m}: is H or Cl.

Embodiment 56. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 and 55, preferably the compound of Embodiment 55, wherein under the proviso that Xaa3 is of formula (IVa) or (IVb), preferably under the proviso that Xaa3 is of formula (IVa), Xaa2 is a residue of an α-L-amino acid selected from the group consisting of Phe, Ocf, Mcf, Pcf, Eaa, Egm, Egn and Egp.

Embodiment 57. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 55 and 56, preferably the compound of any one of Embodiments 55 and 56, wherein under the proviso that Xaa3 is of formulae (IVa) or (IVb), preferably under the proviso that Xaa3 is of formula (IVa), Xaa2 is a residue of an α-L-amino acid selected from the group consisting of Mcf and Egp.

Embodiment 58. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56 and 57, wherein Xaa3 is a residue of an amino acid of formula (IVe) or (IVf), 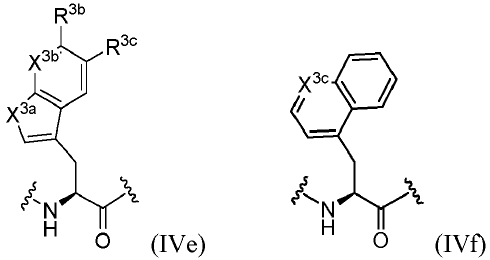 wherein
- R^{3b}: is H or F,
- R^{3c}: is selected from the group consisting of Cl, Br, H and F,
- X^{3a}: is NH or S,
- X^{3b}: is selected from the group consisting of C-H, C-F and N, and
- X^{3c}: is C-H or N, preferably X^{3c} is C-H.

Embodiment 59. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57 and 58, preferably the compound of Embodiment 58, wherein Xaa3 is a residue of an amino acid of formula (IVg) 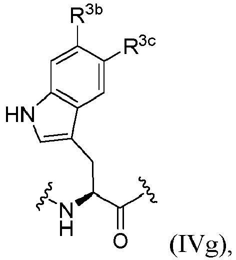 wherein
- R^{3b}: is H or F, and
- R^{3e}: is selected from the group consisting of Cl, Br, and H.

Embodiment 60. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56 and 57, wherein Xaa3 is a residue of an α-L-amino acid selected from the group consisting of 5Clw, 5Brw, Trp, 1Ni, Bta, 5Fw, 6Fw, 7Fw, 6Clw and 7Nw.

Embodiment 61. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58 and 60, preferably the compound of any one of Embodiments 58 and 60, wherein Xaa3 is a residue of an α-L-amino acid selected from the group consisting of 5Clw, 5Brw, Trp, 1Ni, Bta, 5Fw, 6Fw and 7Fw.

Embodiment 62. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 and 61, preferably the compound of any one of Embodiments 58, 59, 60 and 61, wherein Xaa3 is a residue of an α-L-amino acid selected from the group consisting of 5Clw and 5Brw.

Embodiment 63. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61 and 62, preferably the compound of any one of Embodiments 58, 59, 60, 61 and 62, wherein Xaa3 is a 5Clw residue.

Embodiment 64. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53 and 54, wherein under the proviso that Xaa2 is of formulae (IIIa) or (IIIb), preferably under the proviso that Xaa2 is of formula (IIIa), Xaa3 is a residue of an α-L-amino acid acid of formulae (IVc) or (IVd) 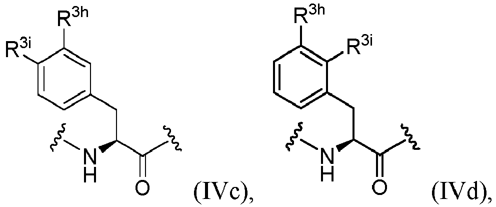 wherein R³ⁱ and R^{3h} each and independently is Cl.

Embodiment 65. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 and 64, preferably the compound of Embodiment 64, wherein under the proviso that Xaa2 is of formulae (IIIa) or (IIIb), preferably under the proviso that Xaa2 is of formula (IIIa), Xaa3 is Eaa or Egp.

Embodiment 66. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 and 65, wherein Xaa4 is a residue of an α-L-amino acid of formula (Va) 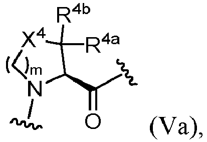 wherein
- m: is 1 or 2, preferably m is 1,
- R^{4a}: is selected from the group consisting of H, OH and methyl,
- R^{4b}: is either H or methyl, preferably R^{4b} is methyl if R^{4a} is methyl,
- X⁴: is selected from the group consisting of CHR^{4c}, CH₂, S and O,
wherein R^{4c} is selected from the group consisting of NHR^{4d}, NH₂, H, OH, methyl and F,
wherein R^{4d} is Ac or is a Z group, preferably the Z group is a chelator optionally comprising a linker, more preferably the chelator is DOTA.

Embodiment 67. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65 and 66, preferably the compound of Embodiment 66, wherein Xaa4 is a residue of an α-L-amino acid of formula (Vd) 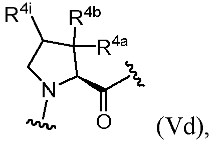 wherein
- R^{4a}: is selected from the group consisting of H, OH and methyl,
- R^{4b}: is H or methyl, preferably R^{4b} is methyl, if R^{4a} is methyl,
- R⁴ⁱ: is selected from the group consisting of NH₂, OH, NHR^{4d}, H and F,
wherein R^{4d} is H, Ac, or is a Z group, if the Z group is a chelator comprising
an optional linker, preferably the chelator is DOTA.

Embodiment 68. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66 and 67, preferably the compound of any one of Embodiments 66 and 67, wherein Xaa4 is a residue of an α-L-amino acid of formula (Ve) 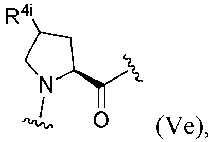 wherein
- R⁴ⁱ: is selected from the group consisting of NH₂, OH and H.

Embodiment 69. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65 and 66, preferably the compound of Embodiment 66, wherein Xaa4 is a residue of an α-L-amino acid selected from the group consisting of Tap, Hyp, Pro, 4Ap, 4Ap(Ac), Tap(DOTA), 4Tfp, H3p, Eaz, Oxa, Dtc and Pip.

Embodiment 70. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66 and 69, preferably the compound of any one of Embodiments 66 and 69, wherein Xaa4 is a residue of an α-L-amino acid selected from the group consisting of Tap, Hyp, Pro, 4Ap, 4Ap(Ac), Tap(DOTA), 4Tfp, H3p, Eaz, Oxa and Dtc.

Embodiment 71. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 69 and 70, preferably the compound of any one of Embodiments 66, 67, 69 and 70, wherein Xaa4 is a residue of an α-L-amino acid selected from the group consisting of Tap, Hyp, Pro, 4Ap, 4Ap(Ac), Tap(DOTA), 4Tfp and H3p.

Embodiment 72. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, preferably the compound of any one of Embodiments 66, 67, 68, 69, 70 and 71, wherein Xaa4 is a residue of an α-L-amino acid selected from the group consisting of Tap, Hyp, Pro and 4Ap.

Embodiment 73. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71 and 72, preferably the compound of any one of Embodiments 66, 67, 68, 69, 70, 71 and 72, wherein Xaa4 is a Tap residue.

Embodiment 74. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71 and 72, preferably the compound of any one of Embodiments 66, 67, 68, 69, 70, 71 and 72, wherein Xaa4 is a Hyp residue.

Embodiment 75. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71 and 72, preferably the compound of any one of Embodiments 66, 67, 68, 69, 70, 71 and 72, wherein Xaa4 is a Pro residue.

Embodiment 76. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 and 65, wherein Xaa4 is a residue of an N-alkylated α-amino acid of formula (Vf) 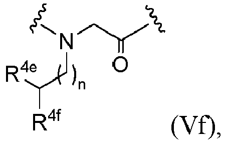 wherein
- n: is 0, 1 or 3,
- R^{4e}: if n = 0, is selected from the group consisting of H and phenyl,
or if n = 1, is selected from the group consisting of methyl, CONH₂ and COOH,
or if n = 3, is selected from the group consisting of NH₂ and NHR^{4h}, wherein R^{4h} is a Z group, preferably the Z group is a bio-distribution modifier,
- R^{4f}: is selected from the group consisting of H and methyl.

Embodiment 77. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65 and 76, preferably the compound of Embodiment 76, wherein Xaa4 is a residue of an N-alkylated α-amino acid selected from the group consisting of Nmg, Nlys, Nleu, Nphe and Nglu.

Embodiment 78. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 and 65, wherein Xaa4 is an Oic residue.

Embodiment 79. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 and 78, wherein Xaa5 is a residue of an α-amino acid optionally comprising a Z group, wherein the Z group is a chelator or a bio-distribution modifier, wherein the α-nitrogen atom of the α-amino acid is optionally substituted by a methyl group.

Embodiment 80. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78 and 79, preferably the compound of Embodiment 79, wherein Xaa5 is a residue of an α-amino acid of formulae (VIa) or (IVb), preferably Xaa5 is a residue of an α-amino acid of formula (VIa), 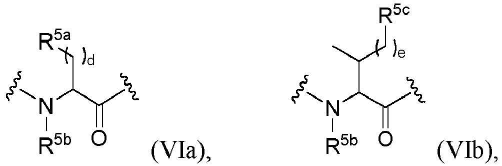 wherein
- R^{5a}: is selected from the group comprising H, COOH, SO₃H, OPO₃H, CONH₂, OH, NH₂, NHR^{5d}, NHC(=NH)NH₂, (C₁-C₄)alkyl , an aryl ring and a heteroaryl ring,
wherein R^{5d} is a Z group, preferably the Z group is a bio-distribution modifier, and wherein preferably the aryl ring is phenyl,
and wherein preferably the heteroaryl ring is 3-indoyl or 4-imidazoyl,
preferably R^{5a} is COOH,
- R^{5b}: is selected from the group consisting of H and methyl, preferably R^{5b} is H,
- d: is 1, 2, 3, 4, or 5, preferably d is 2,
- R^{5c}: is selected from the group consisting of H and OH,
- e: is 1 or 2.

Embodiment 81. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 and 80, preferably the compound of any one of Embodiments 79 and 80, wherein Xaa5 is a residue of an α-amino acid of formula (VIa),
wherein
- R^{5a}: is selected from the group comprising COOH, CONH₂, OH, NH₂, NHC(=NH)NH₂ and a heteroaryl ring,
wherein preferably the heteroaryl ring is 3-indoyl or 4-imidazoyl,
preferably R^{5a} is COOH,
- d: is 1, 2, 3, 4, or 5, preferably d is 2,
- R^{5b}: is H.

Embodiment 82. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80 and 81, preferably the compound of any one of Embodiments 79, 80 and to 81, wherein Xaa5 is a residue of an α-amino acid of formula (VIa),
wherein
- R^{5a}: is selected from the group comprising COOH, CONH₂ and OH, preferably R^{5a} is COOH,
- d: is 1, or 2, preferably d is 2.

Embodiment 83. The compound of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81 and 82, preferably the compound of any one of Embodiments 80, 81 and 82, more preferably the compound of Embodiment 82, wherein the stereo configuration of Xaa5 is the L-configuration or is the D-configuration, preferably the stereo configuration of Xaa5 is the L-configuration.

Embodiment 84. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79 and 80, preferably the compound of any one of Embodiments 79 and 80, wherein Xaa5 is a residue of an α-amino acid selected from the group consisting of Glu, Gln, Asp, Asn, Ser, Hse, Pse, Nme, glu, Ala, Trp, Lys, Arg, His, Hly, Har, Val and Phe.

Embodiment 85. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 83 and 84, preferably the compound of any one of Embodiments 79, 80, 81, 83 and 84, wherein Xaa5 is a residue of an α-L-amino acid selected from the group consisting of Glu, Gln, Asp, Asn, Ser, Hse, Trp, Lys, Arg, His, Hly, and Har.

Embodiment 86. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84 and 85, preferably the compound of any one of Embodiments 79, 80, 81, 82, 83, 84 and 85, wherein Xaa5 is a residue of an α-L-amino acid selected from the group consisting of Glu, Gln, Asp, Asn, Ser, and Hse.

Embodiment 87. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85 and 86, preferably the compound of any one of Embodiments 79, 80, 81, 82, 83, 84, 85 and 86, wherein Xaa5 is a Glu residue.

Embodiment 88. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 and 78, wherein Xaa5 is a residue of a cyclic α-L-amino acid, preferably Xaa5 is a Pro residue.

Embodiment 89. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87 and 88, wherein Xaa6 is a residue of an α-amino acid of formula (VIIa) 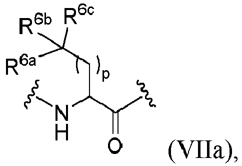 wherein
- p: is 1 or 0, preferably p is 1,
- R^{6a}: is (C₁-C₂)alkyl,
- R^{6b}: is methyl,
- R^{6c}: is H if p is 0, and is H or methyl, if p is 1.

Embodiment 90. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88 and 89, preferably the compound of Embodiment 89, wherein the stereo configuration of Xaa6 is the L-configuration or is the D-configuration, preferably the stereo configuration of Xaa6 is the D-configuration.

Embodiment 91. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89 and 90, preferably the compound of any one of Embodiments 89 and 90, wherein Xaa6 is a residue of an α-amino acid selected from the group consisting of leu, Leu, Npg, Val, Ile and Hle.

Embodiment 92. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 and 91, preferably the compound of any one of Embodiments 89, 90 and 91, wherein Xaa6 is a residue of an α-amino acid selected from the group consisting of leu, Leu, Npg, Val and Ile.

Embodiment 93. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91 and 92, preferably the compound of any one of Embodiments 89, 90, 91 and 92, wherein Xaa6 is residue of a leu residue.

Embodiment 94. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92 and 93, preferably the compound of any one of Embodiments 83 and 90, wherein the stereo configuration of Xaa5 is the L-configuration and the stereo configuration of Xaa6 is the D-configuration, or the stereo configuration of Xaa5 is the D-configuration and the stereo configuration of Xaa6 is the L-configuration, preferably the stereo configuration of Xaa5 is the L-configuration and the stereo configuration of Xaa6 is the D-configuration.

Embodiment 95. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93 and 94, wherein Xaa8 is a residue of an aliphatic cyclic α-amino acid or a residue of an aliphatic heterocyclic α-amino acid, preferably Xaa8 is a residue of a cyclic α-amino acid of formula (IXa), 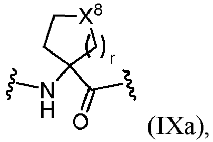 wherein
- r: is 2 or 1, preferably r is 2,
- X⁸: is selected from the group consisting of NR^{8a}, O, NH, and CH₂, wherein R^{8a} is a Z group or R^{8a} is acetyl, preferably R^{8a} is a Z group, the Z group is a chelator optionally comprising a linker, wherein more preferably the chelator is DOTA.

Embodiment 96. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 and 95, preferably the compound of Embodiment 95, wherein Xaa8 is a residue of an aliphatic heterocyclic α-amino acid of formula (IXd) 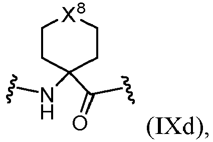 wherein
- X⁸: is selected from the group consisting of NR^{8a}, O, and NH,
wherein R^{8a} is a Z group, wherein preferably the Z group is a chelator optionally comprising a linker, wherein more preferably the Z group is a chelator without an optional linker, wherein most preferably the chelator is DOTA.

Embodiment 97. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95 and 96, preferably the compound of any one of Embodiments 95 and 96, wherein Xaa8 is a residue of an aliphatic heterocyclic α-amino acid of formula (IXd), wherein
- X⁸: is NR^{8a} or O,
wherein R^{8a} is a Z group, wherein the Z group is a chelator, wherein preferably the chelator is DOTA.

Embodiment 98. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 and 95, preferably the compound of Embodiment 95, wherein Xaa8 is a residue a cyclic α-amino acid selected from the group consisting of Apc(R^{8a}), Ape, Thp, Egz, and Eca, wherein R^{8a} is a Z group, wherein if the Z group is a chelator optionally comprising a linker, preferably the chelator is DOTA, or R^{8a} is acetyl.

Embodiment 99. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96 and 98, preferably the compound of any one of Embodiments 95, 96 and 98, wherein Xaa8 is a residue of a cyclic α-amino acid residue selected from the group consisting of Apc(R^{8a}), Apc, and Thp, wherein R^{8a} is a Z group, wherein if the Z group is a chelator optionally comprising a linker, preferably the chelator is DOTA, or R^{8a} is acetyl.

Embodiment 100. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 and 99, preferably the compound of any one of Embodiments 95, 96, 97, 98 and 99, wherein Xaa8 is Apc(R^{8a}), wherein R^{8a} is a Z group, wherein the Z group is a chelator optionally comprising a linker, wherein the chelator
a) preferably is selected from the group consisting of DOTA, DOTAGA, DOTAM, DOTP, NOTA, NODAGA, PSC, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
b) more preferably is selected from the group consisting of DOTA, DOTAGA, DOTAM, NOTA, NODAGA, PSC, NODA-MPAA, NOPO, HBED, DTPA, CHX-A"-DTPA, CB-TE2A, Macropa, PCTA, N4, and analogs thereof,
   and
c) most preferably is selected from the group consisting of DOTA, DOTAGA, DOTAM, NOTA, NODAGA, PSC, NOPO, Macropa, PCTA and analogs thereof.

Embodiment 101. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100, preferably the compound of any one of Embodiments 95, 96, 97, 98, 99 and 100, more preferably the compound of Embodiment 100, wherein the chelator is DOTA.

Embodiment 102. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93 and 94, wherein Xaa8 is an Aic residue.

Embodiment 103. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93 and 94, wherein Xaa8 is a residue of an α-amino acid selected from the group consisting of Lys, Lys(R^{8e}), lys(R^{8e}), Amk(R^{8e}), Dab(R^{8e}) and dab(R^{8e}), wherein R^{8e} is a Z group, preferably the Z group is a chelator optionally comprising a linker.

Embodiment 104. The compound of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94 and 103, preferably the compound of Embodiment 103, wherein Xaa8 is a residue of an α-amino acid selected from the group consisting of Lys(R^{8e}), lys(R^{8e}), Amk(R^{8e}), Dab(R^{8e}) and dab(R^{8e}), wherein R^{8e} is a Z group, wherein the Z group is a chelator optionally comprising a linker, wherein the chelator
a) preferably is selected from the group consisting of DOTA, DOTAGA, DOTAM, DOTP, NOTA, NODAGA, PSC, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
b) more preferably is selected from the group consisting of DOTA, DOTAGA, DOTAM, NOTA, NODAGA, PSC, NODA-MPAA, NOPO, HBED, DTPA, CHX-A"-DTPA, CB-TE2A, Macropa, PCTA, N4, and analogs thereof,
   and
c) most preferably is selected from the group consisting of DOTA, DOTAGA, DOTAM, NOTA, NODAGA, PSC, NOPO, Macropa, PCTA and analogs thereof.

Embodiment 105. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 103 and 104, preferably the compound of any one of Embodiments 103 and 104, more preferably the compound of Embodiment 104, wherein Xaa8 is an Amk(R^{8e}) residue, wherein R^{8e} is a Z group, wherein the Z group is a chelator optionally comprising a linker, wherein the chelator
a) preferably is selected from the group consisting of DOTA, DOTAGA, DOTAM, DOTP, NOTA, NODAGA, PSC, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
b) more preferably is selected from the group consisting of DOTA, DOTAGA, DOTAM, NOTA, NODAGA, PSC, NODA-MPAA, NOPO, HBED, DTPA, CHX-A"-DTPA, CB-TE2A, Macropa, PCTA, N4, and analogs thereof,
   and
c) most preferably is selected from the group consisting of DOTA, DOTAGA, DOTAM, NOTA, NODAGA, PSC, NOPO, Macropa, PCTA and analogs thereof.

Embodiment 106. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93 and 94, wherein Xaa8 is a residue of an α-amino acid residue selected from the group consisting of Aib, Deg, Ams, ala, Ala, Gln, Thr, Trp, Phe and Glu.

Embodiment 107. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105 and 106, wherein Xaa9 is a residue of an amino acid of formula (Xa) 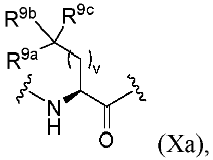 wherein
- R^{9a}: is selected from the group consisting of (C₁-C₄)alkyl, COOH, CONH₂, OH, NH₂, NHC(=NH)NH₂, an aryl ring and a heteroaryl ring, preferably the aryl ring is phenyl and the heteroaryl ring is 3-indoyl, and cyclo-hexyl,
- R^{9b}: is methyl or H,
- R^{9c}: is H or methyl, and
- v: is 0, 1, 2, preferably v is 1.

Embodiment 108. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106 and 107, preferably the compound of Embodiment 107, wherein Xaa9 is a residue of an amino acid of formula (Xa), wherein
- R^{9a}: is selected from the group consisting of (C₁-C₂)alkyl, COOH, OH, NHC(=NH)NH₂, an aryl ring and a heteroaryl ring, preferably the aryl ring is phenyl and the heteroaryl ring is 3-indoyl, and cyclo-hexyl,
- R^{9b}: is methyl or H,
- R^{9c}: is H, and
- v: is 0, 1, 2, preferably v is 1.

Embodiment 109. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106 and 107, preferably the compound of Embodiment 107, wherein Xaa9 is a residue of an α-L-amino acid selected from the group consisting of Leu, Hle, Ile, Arg, Trp, Glu, Phe, Ser, Cha, Npg and Tle.

Embodiment 110. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108 and 109, preferably the compound of Embodiments 107, 108 and 109, more preferably the compound of Embodiment 109, wherein Xaa9 is a residue of an α-L-amino acid selected from the group consisting of Leu, Hle, Ile, Arg, Trp, Glu, Phe and Ser.

Embodiment 111. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109 and 110, preferably the compound of Embodiments 107, 108, 109 and 110, more preferably the compound of Embodiment 110, wherein Xaa9 is a Leu residue.

Embodiment 112. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110 and 111, wherein Xaa10 is a residue of an amino acid of formula (XIa) 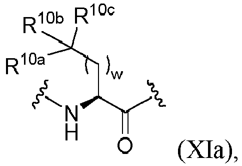 wherein
- R^{10a}: is selected from the group consisting of (C₁-C₂)alkyl and phenyl, preferably R^{10a} is (C₁-C₂)alkyl, more preferably (C₁-C₂)alkyl is ethyl,
- R^{10b}: is selected from the group consisting of methyl and H, preferably R^{10b} is methyl,
- R^{10c}: is selected from the group consisting of H and methyl, R^{10C} is H, if w is 1, preferably R^{10c} is H, and
- w: is 0 or 1, preferably w is 0.

Embodiment 113. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111 and 112, preferably the compound of Embodiment 112, wherein Xaa10 is a residue of an amino acid of formula (XIa), wherein
- R^{10a}: is selected from (C₁-C₂)alkyl, preferably is ethyl,
- R^{10b}: is selected from the group consisting of methyl and H, preferably R^{10b} is methyl,
- R^{10c}: is selected from the group consisting of H and methyl, R^{10C} is H, if w is 1, preferably R^{10c} is H, and
- w: is 0 or 1, preferably w is 0.

Embodiment 114. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111 and 112, preferably the compound of Embodiment 112, wherein Xaa10 is a residue of an α-L-amino acid selected from the group consisting of Ile, Leu, Tle, Val, and Phe.

Embodiment 115. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113 and 114, preferably the compound of any one of Embodiments 112, 113 and 114, wherein Xaa10 is a residue of an α-L-amino acid selected from the group consisting of Ile, Leu and Tle.

Embodiment 116. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114 and 115, preferably the compound of any one of Embodiments 112, 113, 114 and 115, wherein Xaa10 is an Ile residue.

Embodiment 117. The compound of any one of Embodiments 1, 2, 3, 4, 5, 6, 7 and 8, wherein
- Xaa1: is Asp, Asn, Glu, Gln or Cya,
- Xaa2: is Trp, Hyw, 5Fw, 6Clw or 7Fw,
- Xaa3: is 5Clw, 5Brw, 5Fw, Trp, 1Ni, Bta, 6Fw or Trp,
- Xaa4: is Tap, Hyp, 4Tfp, Pro, H3p, Eaz, Oxa, Dtc, Nmg, Nleu, Nlys, Nphe or Nglu,
- Xaa5: is Glu, glu, Nme, Gln, Asp, Asn or Hse,
- Xaa6: is leu, Leu, Npg, Val or Ile,
- Xaa7: is Cys or Hcy, Dap
- Xaa8: is Apc(DOTA), Thp, Apc, Aib, Deg, Ams, Amk(DOTA), Egz, Eca, or ala,
- Xaa9: is Leu, Hle, Ile, Phe, Glu, Arg, Ser or Trp,
- Xaa10: is Ile, Leu, Val or Tle,
- Xaa11: is Cys, Hcy, Dap and
- Yc: is 3MeBn or 3Lut.

Embodiment 118. The compound of any one of Embodiments 1, 2, 3, 4, 5, 6, 7 and 8, wherein
- Xaa1: is Asp or Glu,
- Xaa2: is Trp or Hyw,
- Xaa3: is 5Clw or 5Brw,
- Xaa4: is Tap, Hyp, or Pro,
- Xaa5: is Glu,
- Xaa6: is leu or Leu,
- Xaa7: is Cys,
- Xaa8: is Apc(DOTA) or Amk(DOTA),
- Xaa9: is Leu,
- Xaa10: is Ile
- Xaa11: is Cys and
- Yc: is 3Lut.

Embodiment 119. The compound of any one of Embodiments 1, 2, 3, 4, 5, 6, 7 and 8, wherein
- Xaa1: is Asp,
- Xaa2: is Trp,
- Xaa3: is 5Clw,
- Xaa4: is Tap or Hyp,
- Xaa5: is Glu,
- Xaa6: is leu,
- Xaa7: is Cys,
- Xaa8: is Apc(DOTA) or Amk(DOTA),
- Xaa9: is Leu,
- Xaa10: is Ile
- Xaa11: is Cys and
- Yc: is 3Lut.

Embodiment 120. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118 and 119, wherein a linker comprising an amino group and a carbonyl group is optionally interspersed between the bio-distribution modifier comprised by the N-terminal modification group A and Xaa1, wherein the bio-distribution modifier comprises a residue of an aliphatic carboxylic acid,
wherein to an ω-carbon atom of the aliphatic carboxylic acid a nitrogen atom, a triazolyl moiety, a hydroxymethyl-triazolyl moiety or an urea moiety is covalently attached,
   wherein to the nitrogen atom, to the triazolyl moiety, to the hydroxymethyl triazolyl moiety or to the urea moiety
      (a) 1 or 2 first level alkyl groups are covalently attached, wherein each and any first level alkyl group individually and independently comprises 2 or more hydroxyl groups, wherein each and any hydroxyl group is a primary hydroxyl group,
   or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
preferably the bio-distribution modifier is a structure of formula (A-I), 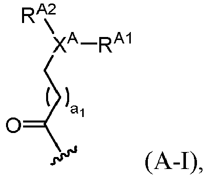
wherein
   - X^{A}: comprises a nitrogen atom, a triazolyl moiety, a hydroxymethyl-triazolyl moiety or an urea moiety,
   - a₁: is 0, 1, 2, 3, 4, 5, or 6, if X^{A} is a triazolyl moiety, preferably a₁ is 2, if X^{A} is selected from the group comprising a nitrogen atom, a hydroxymethyl-triazolyl moiety and an urea moiety, preferably a₁ is 4,
   - R^{A1}: is a first level alkyl group of a structure of formula (A-II), 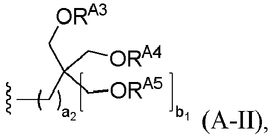
   wherein
   - a₂: is 0 or 1,
   - b₁: is 0 or 1,

   wherein, if b₁ is 0,
      R^{A3} and R^{A4} are individually and independently a second level alkyl group of a structure of formula (A-III),
   or R^{A3} and R^{A4} are each H,
   or wherein, if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), 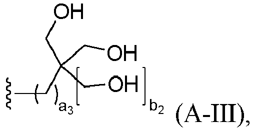
   wherein
      - a₃: is 0 or 1, and
      - b₂: is 0 or 1,
and wherein
   - R^{A2}: is absent, if X^{A} is selected from the group comprising a triazolyl moiety, a hydroxmethyl-triazolyl moiety and an urea moiety,
   - or R^{A2}: is H or a first level alkyl group of a structure of formula (A-II), if X^{A} is a nitrogen atom, wherein
   a₂ is 0 or 1,
   b₁ is 0 or 1,

   wherein if b₁ is 0,
      R^{A3} and R^{A4} are individually and independently a second level alkyl group of a structure of formula (A-III)
   or R^{A3} and R^{A4} are H,
   or wherein if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), wherein in formula (A-III)
   - as: is 0 or 1, and
   - b₂: is 0 or 1,
and wherein the carbonyl group of the structure (A-I) is covalently attached to the α-nitrogen atom of Xaa1,
or if a linker comprising an amino group and a carbonyl group, is interspersed between the bio-distribution modifier comprised by the N-terminal modification group A and Xaa1, the carbonyl group of the structure (A-I) is covalently attached to the amino group of the linker.

Embodiment 121. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119 and 120, preferably the compound of Embodiment 120, wherein a linker comprising an amino group and a carbonyl group is optionally interspersed between the bio-distribution modifier comprised by the N-terminal modification group A and Xaa1, wherein the bio-distribution modifier comprises a residue of an aliphatic carboxylic acid, wherein to an ω-carbon atom of the aliphatic carboxylic acid a nitrogen atom is covalently attached, wherein to the nitrogen atom
(a) 1 or 2 first level alkyl groups are covalently attached, wherein each and any first level alkyl group individually and independently comprises 2 or more hydroxyl groups, wherein each and any hydroxyl group is a primary hydroxyl group,

or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
preferably the bio-distribution modifier is a structure of formula (A-IV), 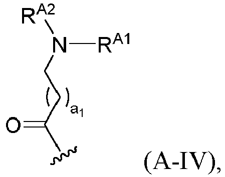
wherein
   - a₁: is 0, 1, 2, 3, 4, 5, or 6, preferably a₁ is 4,
   - R^{A1}: is a first level alkyl group of a structure of formula (A-II), 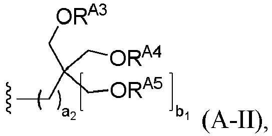
   wherein
   - a₂: is 0 or 1,
   - b₁: is 0 or 1,

   wherein, if b₁ is 0,
      R^{A3} and R^{A4} are individually and independently second level alkyl groups of a structure of formula (A-III),
   or R^{A3} and R^{A4} are each H,
   or wherein, if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is H, 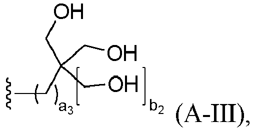
   wherein
      - a₃: is 0 or 1, and
      - b₂: is 0 or 1,
and wherein
   - R^{A2}: is H or a first level alkyl group of a structure of formula (A-II),
   a₂ is 0 or 1,
   b₁ is 0 or 1,

   wherein if b₁ is 0,
      R^{A3} and R^{A4} are individually and independently a second level alkyl group of a structure of formula (A-III), wherein in formula (A-III) as is 0 or 1, and b₂ is 0 or 1,
   or R^{A3} and R^{A4} are each H,
   or wherein if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is H, wherein in formula (A-III)
   - a₃: is 0 or 1, and
   - b₂: is 0 or 1.

Embodiment 122. The compound of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120 and 121, preferably the compound of any one of Embodiments 120 and 121, more preferably the compound of Embodiment 121, wherein the bio-distribution modifier comprised by the N-terminal modification group A comprises a residue of an aliphatic carboxylic acid, wherein to an ω-carbon atom of the aliphatic carboxylic acid a nitrogen atom is covalently attached, wherein to the nitrogen atom 1 or 2 first level alkyl groups are covalently attached, wherein each and any first level alkyl group individually and independently comprises two hydroxyl groups, wherein each and any hydroxyl group is a primary hydroxyl group, wherein preferably the bio-distribution modifier is a structure of formula (A-VII), 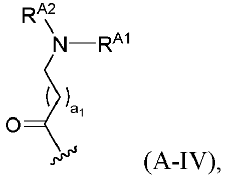 wherein
- a₁: is 0, 1, 2, 3, 4, 5, or 6, preferably a₁ is 4,
- R^{A1}: is a first level alkyl group of a structure of formula (A-V), 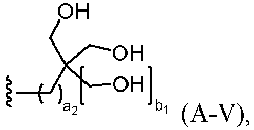
wherein a₂ is 0 or 1 and b₁ is 0 or 1,
and wherein
- R^{A2}: is a first level alkyl group of a structure of formula (A-V), wherein a₂ is 0 or 1 and b₁ is 0 or 1,
- or R^{A2}: is H.

Embodiment 123. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121 and 122, preferably the compound of any one of Embodiment 120, 121 and to 122, more preferably the compound of Embodiment 122, wherein R^{A1} is a first level alkyl group of structure of formula (A-V), wherein a₂ is 0 or 1, preferably a₂ is 0, and b₁ is 0 or 1, and wherein R^{A2} is H.

Embodiment 124. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122 and 123, preferably the compound of any one of Embodiments 120, 121, 122 and to 123, more preferably the compound of Embodiment 123, wherein the bio-distribution modifier comprised by the N-terminal modification group A is HAC:Ahx.

Embodiment 125. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121 and 122, preferably the compound of any one of Embodiments 120, 121 and 122, more preferably the compound of Embodiment 122, wherein R^{A1} is a first level alkyl group of a structure of formula (A-V), wherein a₂ is 0 or 1, preferably a₂ is 1, and b₁ is 0 or 1, and wherein R^{A2} is a first level alkyl group of structure of formula (A-V), wherein a₂ is 0 or 1, preferably a₂ is 1, and b₁ is 0 or 1.

Embodiment 126. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122 and 125, preferably the compound of any one of Embodiments 120 to 122 and 125, more preferably the compound of Embodiments 125, wherein R^{A1} is a first level alkyl group of structure of formula (A-V), wherein a₂ is 0 or 1, preferably a₂ is 1, and b₁ is 0 or 1, and wherein R^{A2} is a first level alkyl group of structure of formula (A-V), wherein a₂ is 0 or 1, preferably a₂ is 1, and b₁ is 0 or 1 and wherein a₁ in R^{A1} and a₁ in R^{A2} are identical and b₁ in R^{A1} and b₁ in R^{A2} are identical.

Embodiment 127. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 125 and 126, preferably the compound of any one of Embodiments 120, 121 and 122, 125 and 126, more preferably the compound of Embodiment 126, wherein the bio-distribution modifier comprised by the N-terminal modification group A is selected from the group comprising HPD2:Ahx, PEO2:Ahx.

Embodiment 128. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120 and 121, preferably the compound of any one of Embodiment 120 and 121, more preferably the compound of Embodiment 121, wherein a linker comprising an amino group and a carbonyl group is optionally interspersed between the bio-distribution modifier comprised by the N-terminal modification group A and Xaa1, wherein the bio-distribution modifier comprises a residue of an aliphatic carboxylic acid, wherein to an ω-carbon atom of the aliphatic carboxylic acid a nitrogen atom is covalently attached, wherein to the nitrogen atom 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
preferably the bio-distribution modifier is a structure of formula (A-IV), 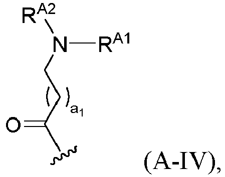
wherein
   - a₁: is 0, 1, 2, 3, 4, 5, or 6, preferably a₁ is 4,
   - R^{A1}: is a first level alkyl group of a structure of formula (A-II), 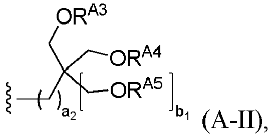
   wherein
   - a₂: is 0 or 1,
   - b₁: is 0 or 1,
   wherein, if b₁ is 0,
   R^{A3} and R^{A4} are individually and independently a second level alkyl group of a structure of formula (A-III),
   or wherein, if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), 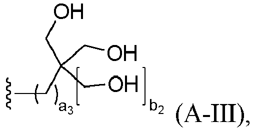
   wherein
      - a₃: is 0 or 1, and
      - b₂: is 0 or 1,
   preferably b₁ is 0,
and wherein
   - R^{A2}: is H or a first level alkyl group of a structure of formula (A-II), wherein
   a₂ is 0 or 1,
   b₁ is 0 or 1,
   wherein if b₁ is 0,
   R^{A3} and R^{A4} are individually and independently a second level alkyl group of a structure of formula (A-III), wherein in formula (A-III) as is 0 or 1, and b₂ is 0 or 1,
   or wherein if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), wherein in formula (A-III) as is 0 or 1, and b₂ is 0 or 1,
   preferably b₁ is 0.

Embodiment 129. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121 and 128, preferably the compound of any one of Embodiments 120, 121 and 128, more preferably the compound of Embodiment 128, wherein the bio-distribution modifier comprised by the N-terminal modification group A is a structure of formula (A-IV), 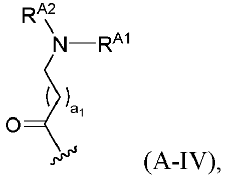 wherein
- a₁: is 0, 1, 2, 3, 4, 5, or 6, preferably a₁ is 4,
- R^{A1}: is a first level alkyl group of a structure of formula (A-VI), 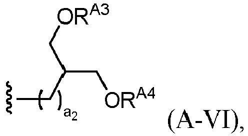
wherein a₂ is 0 or 1, and wherein
R^{A3} and R^{A4} are individually and independently a second level alkyl group of a structure of formula (A-VII), 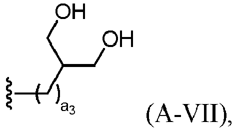 wherein a₃ is 0 or 1,
and wherein
- R^{A2}: is a first level alkyl group of a structure of formula (A-VI), wherein a₂ is 0 and wherein,
R^{A3} and R^{A4} are individually and independently a second level alkyl group of a structure of formula (A-VII), wherein in formula (A-VII) as is 0 or 1,
- or R^{A2}: is H.

Embodiment 130. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 128 and 129, preferably the compound of any one of Embodiments 120, 121, 128 and 129, more preferably the compound of Embodiment 129, wherein R^{A1} is a first level alkyl group of structure of formula (A-VI), wherein a₂ is 0 or 1, preferably a₂ is 0, and wherein as in R^{A3} and as in R^{A4} are identical and wherein R^{A2} is H.

Embodiment 131. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 128, 129 and 130, preferably the compound of any one of Embodiments 120, 121, 128, 129 and 130, more preferably the compound of Embodiment 130, wherein the bio-distribution modifier comprised by the N-terminal modification group A is selected from the group comprising HAC2:HAC:Ahx and HPD2:HAC:Ahx.

Embodiment 132. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121 and 128, preferably the compound of any one of Embodiments 120, 121 and 128, more preferably the compound of Embodiment 128,
wherein R^{A1} is a first level alkyl group of a structure of formula (A-VI),
wherein a₂ is 0 or 1, preferably a₂ is 1,
and wherein R^{A2} is a first level alkyl group of a structure of formula (A-VI),
   wherein a₂ is 0 or 1, preferably a₂ is 1,
   preferably a₂ in R^{A1} and a₂ in R^{A2} are identical,
and wherein preferably
   as in R^{A3} of R^{A1},
   and as in R^{A4} of R^{A1,}
   and as in R^{A3} of R^{A2}
   and as in R^{A4} of R^{A2} are identical.

Embodiment 133. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 128 and 132, preferably the compound of any one of Embodiments 120, 121, 128 and 132, more preferably the compound of Embodiment 132, wherein the bio-distribution modifier comprised by the N-terminal modification group A is selected from the group comprising HAC4:HPD2:Ahx, HPD4:HPD2:Ahx.

Embodiment 134. The compound of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119 and 120, preferably the compound of any one of Embodiment 120, wherein a linker comprising an amino group and a carbonyl group is optionally interspersed between the bio-distribution modifier comprised by the N-terminal modification group A and Xaa1, wherein the bio-distribution modifier comprises a residue of an aliphatic carboxylic acid,
wherein to an ω-carbon atom of the aliphatic carboxylic acid a triazolyl moiety is covalently attached, wherein to the triazolyl moiety
   (a) one first level alkyl groups is covalently attached, wherein the first level alkyl group comprises 2 or more hydroxyl groups, wherein each and any hydroxyl group is a primary hydroxyl group,
   or, (b) one first level alkyl groups is covalently attached, wherein the first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein the first level alkyl group comprises 0, 1 or 2 hydroxyl groups, wherein each and any hydroxyl group is a primary hydroxyl group,
preferably the bio-distribution modifier is a structure of formula (A-VIII), 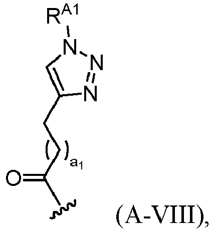 wherein
   - a₁: is 0, 1, 2, 3, 4, 5, or 6, preferably a₁ is 2,
   - R^{A1}: is a first level alkyl group of a structure of formula (A-II), 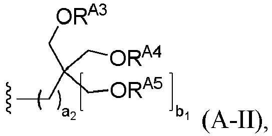
   wherein
   - a₂: is 0 or 1,
   - b₁: is 0 or 1,

   wherein, if b₁ is 0,
      R^{A3} and R^{A4} are individually and independently a second level alkyl group of a structure of formula (A-III),
   or R^{A3} and R^{A4} are each H,
   or wherein, if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), 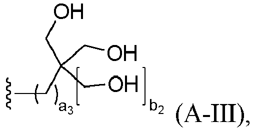
   wherein
      - a₃: is 0 or 1, and
      - b₂: is 0 or 1.

Embodiment 135. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120 and 134, preferably the compound of any one of Embodiments 120 and 134, more preferably the compound of Embodiment 134, wherein to the triazolyl moiety one first level alkyl groups is covalently attached, wherein the first level alkyl group comprises 2 or more hydroxyl groups, wherein each and any hydroxyl group is a primary hydroxyl group, wherein preferably the bio-distribution modifier is a structure of formula (A-VIII), 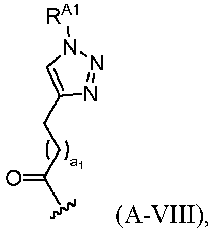 wherein
- a₁: is 0, 1, 2, 3, 4, 5, or 6, preferably a₁ is 2,
- R^{A1}: is a first level alkyl group of a structure of formula (A-V), 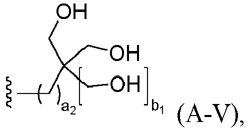
wherein a₂ is 0 or 1 and b₁ is 0 or 1.

Embodiment 136. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 134 and 135, preferably the compound of any one of Embodiments 120, 134 and 135, more preferably the compound of Embodiment 135, wherein the bio-distribution modifier comprised by the N-terminal modification group A is selected from the group comprising HPD:Hyx, PEO:Hyx, TRIS:Hyx and HAC:Hyx, preferably is selected from the group comprising HPD:Hyx, PEO:Hyx and TRIS:Hyx.

Embodiment 137. The compound of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120 and 134, preferably the compound of any one of Embodiments 120 and 134, more preferably the compound of Embodiment 134, wherein to the triazolyl moiety one first level alkyl groups is covalently attached, wherein the first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
preferably the bio-distribution modifier is a structure of formula (A-VIII), 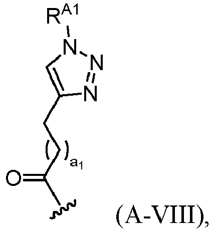
wherein
   - a₁: is 0, 1, 2, 3, 4, 5, or 6, preferably a₁ is 2,
   - R^{A1}: is a first level alkyl group of a structure of formula (A-II), 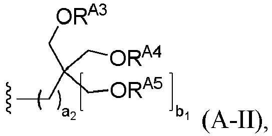
   wherein
   - a₂: is 0 or 1,
   - b₁: is 0 or 1,
   wherein, if b₁ is 0,
   R^{A3} and R^{A4} are individually and independently a second level alkyl group of a structure of formula (A-III),
   or wherein, if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-VII), 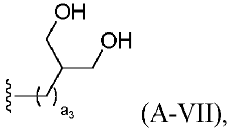
   wherein
      - a₃: is 0 or 1.

Embodiment 138. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 134 and 137, preferably the compound of any one of Embodiments 120, 134 and 137, more preferably the compound of Embodiment 137, wherein if b₁ is 0, as in R^{A3} and as in R^{A4} are identical and b₁ in R^{A3} and b₁ in R^{A4} are identical.

Embodiment 139. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 134, 137 and 138, preferably the compound of any one of Embodiments 120, 134, 137 and 138, more preferably the compound of Embodiment 138, wherein the bio-distribution modifier comprised by the N-terminal modification group A is selected from the group comprising HAC2:HPD:Hyx, HPD2:HPD:Hyx, HAC2:HAC:Hyx, HPD2:HAC:Hyx, preferably comprising HAC2:HPD:Hyx and HPD2:HPD:Hyx .

Embodiment 140. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 134 and 137, preferably the compound of any one of Embodiments 120, 134 and 137, more preferably the compound of Embodiment 137, wherein b₁ is 1 and each and any of R^{A3}, R^{A4} and R^{A5} is a second level alkyl group of a structure of formula (A-VII), wherein as in R^{A3}, as in R^{A4} and as in R^{A5} are identical.

Embodiment 141. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 134, 137 and 140, preferably the compound of any one of Embodiments 120, 134, 137 and 140, more preferably the compound of Embodiment 140, wherein the bio-distribution modifier comprises by the N-terminal modification group A
is selected from the group comprising HAC3:TRIS:Hyx, HPD3:PEO:Hyx, HPD3:TRIS:Hyx, and HAC3:PEO:Hyx .

Embodiment 142. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 134 and 137, preferably the compound of any one of Embodiments 120, 134 and 137, more preferably the compound of Embodiment 137, wherein b₁ is 1 and R^{A3} and R^{A4} are a second level alkyl group of a structure of formula (A-VII) and R^{A5} is H, wherein as in R^{A3} and as in R^{A4} are identical.

Embodiment 143. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 134, 137 and 142, preferably the compound of any one of Embodiments 120, 134, 137 and 142, more preferably the compound of Embodiment 142, wherein the bio-distribution modifier comprised by the N-terminal modification group A is selected from the group comprising HPD2:PEO:Hyx, HAC2:PEO:Hyx, HPD2:TRIS:Hyx and HAC2:TRIS:Hyx.

Embodiment 144. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 134 and 137, preferably the compound of any one of Embodiments 120, 134 and 137, more preferably the compound of Embodiment 137, wherein b₁ is 1 and R^{A3} is a second level alkyl group of a structure of formula (A-III) and R^{A4} and R^{A5} are each H.

Embodiment 145. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 134, 137 and 144, preferably the compound of any one of Embodiments 120, 134, 137 and 144, more preferably the compound of Embodiment 144, wherein the bio-distribution modifier comprised by the N-terminal modification group A is selected from the group comprising HPD:PEO:Hyx, HAC:PEO:Hyx, HPD:TRIS:Hyx and HAC:TRIS:Hyx.

Embodiment 146. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119 and 120, preferably the compound of Embodiment 120, wherein a linker comprising an amino group and a carbonyl group is optionally interspersed between the bio-distribution modifier comprised by the N-terminal modification group A and Xaa1, wherein the bio-distribution modifier comprises a residue of an aliphatic carboxylic acid,
wherein to an ω-carbon atom of the aliphatic carboxylic acid, a hydroxymethyl-triazolyl moiety is covalently attached,
   wherein to the hydroxymethyl triazolyl moiety
   one first level alkyl group is covalently attached, wherein the first level alkyl group is covalently attached via an ether linkage to two second level alkyl groups, wherein each and any second level alkyl group individually and independently comprises 2 or more hydroxyl groups, wherein each and any hydroxyl group is a primary hydroxyl group,
preferably the bio-distribution modifier is a structure of formula (A-IX), 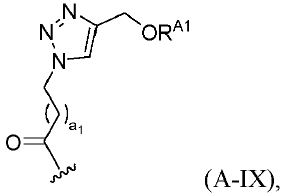
wherein
   - a₁: is 0, 1, 2, 3, 4, 5, or 6, preferably a₁ is 4,
   - R^{A1}: is a first level alkyl group of a structure of formula (A-VI), 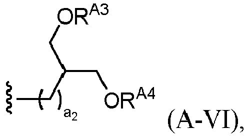
   wherein
   - a₂: is 0 or 1,
   and R^{A3} and R^{A4} are individually and independently a second level alkyl group of a structure of formula (A-VII), 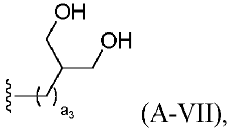 wherein
   - a₃: is 0 or 1.

Embodiment 147. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120 and 146, preferably the compound of any one of Embodiments 120 and 146, more preferably the compound of Embodiment 146, wherein as in R^{A3} and as in R^{A4} are identical.

Embodiment 148. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 146 and 147, preferably the compound of any one of Embodiments 120, 146 and 147, more preferably the compound of Embodiment 147, wherein the bio-distribution modifier comprised by the N-terminal modification group A is selected from the group comprising HAC2:HAC:Tzx, HAC2:HPD:Tzx, HPD2:HPD:Tzx, HPD2:HAC:Tzx.

Embodiment 149. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119 and 120, preferably the compound of Embodiment 120, wherein a linker comprising an amino group and an carbonyl group is optionally interspersed between the bio-distribution modifier comprised by the N-terminal modification group A and Xaa1, wherein the bio-distribution modifier comprises a residue of an aliphatic carboxylic acid, wherein to an ω-carbon atom of the aliphatic carboxylic acid an urea moiety is covalently attached, wherein to the urea moiety
(a) one first level alkyl group is covalently attached, wherein the first level alkyl group comprises 2 or more hydroxyl groups, wherein each and any hydroxyl group is a primary hydroxyl group,
or, (b) one first level alkyl group is covalently attached, wherein the first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein the first level alkyl group comprises 0, 1 or 2 hydroxyl groups, wherein each and any hydroxyl group is a primary hydroxyl group,
preferably the bio-distribution modifier is a structure of formula (A-X), 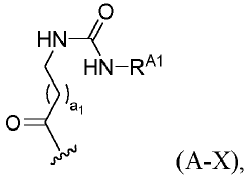 wherein
   - a₁: is 0, 1, 2, 3, 4, 5, or 6, preferably a₁ is 4,
   - R^{A1}: is a first level alkyl group of a structure of formula (A-II), 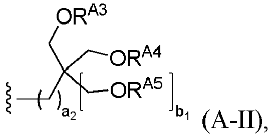
   wherein
   - a₂: is 0 or 1,
   - b₁: is 0 or 1,

   wherein, if b₁ is 0,
      R^{A3} and R^{A4} are individually and independently a second level alkyl group of a structure of formula (A-III),
   or R^{A3} and R^{A4} are each H,
   or wherein, if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), 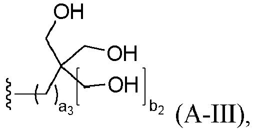
   wherein
      - a₃: is 0 or 1, and
      - b₂: is 0 or 1,
and wherein the carbonyl group of the structure (A-X) is covalently attached to the α-nitrogen atom of Xaa1,
or if a linker comprising an amino group and a carbonyl group is interspersed between the bio-distribution modifier comprised by the N-terminal modification group A and Xaa1, the carbonyl group of the structure (A-X) is covalently attached to the amino group of the linker.

Embodiment 150. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120 and 149, preferably the compound of any one of Embodiments 120 and 149, more preferably the compound of Embodiment 149, wherein to the urea moiety one first level alkyl groups is covalently attached, wherein the first level alkyl group comprises 2 or more hydroxyl groups, wherein each and any hydroxyl group is a primary hydroxyl group, wherein preferably the bio-distribution modifier is a structure of formula (A-X), 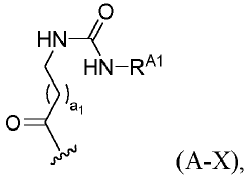 wherein
- a₁: is 0, 1, 2, 3, 4, 5, or 6, preferably a₁ is 4,
- R^{A1}: is a first level alkyl group of a structure of formula (A-V), 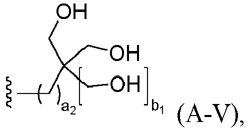
wherein a₂ is 0 or 1 and b₁ is 0 or 1.

Embodiment 151. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 149 and 150, preferably the compound of any one of Embodiments 120, 149 and 150, more preferably the compound of Embodiment 150, wherein the bio-distribution modifier comprised by the N-terminal modification group A is selected from the group comprising HPD:Aur, PEO:Aur, TRIS:Aur and HAC:Aur, preferably is selected from the group comprising PEO:Aur and TRIS:Aur.

Embodiment 152. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120 and 149, preferably the compound of any one of Embodiments 120 and 149, more preferably the compound of Embodiment 149, wherein to the urea moiety one first level alkyl group is covalently attached, wherein the first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein the first level alkyl group comprises 0, 1 or 2 hydroxyl groups, wherein each and any hydroxyl group is a primary hydroxyl group,
preferably the bio-distribution modifier is a structure of formula (A-X), 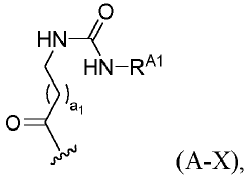
wherein
   - a₁: is 0, 1, 2, 3, 4, 5, or 6, preferably a₁ is 2,
   - R^{A1}: is a first level alkyl group of a structure of formula (A-II), 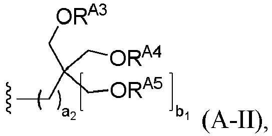
   wherein
   - a₂: is 0 or 1,
   - b₁: is 0 or 1,
   wherein, if b₁ is 0,
   R^{A3} and R^{A4} are individually and independently a second level alkyl group of a structure of formula (A-III),
   or wherein, if b₁ is 1,
   each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), 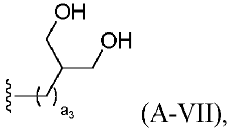
   wherein a₃ is 0 or 1.

Embodiment 153. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 149 and 152, preferably the compound of any one of Embodiments 120, 149 and 152, more preferably the compound of Embodiment 152, wherein if b₁ is 0, as in R^{A3} and as in R^{A4} are identical.

Embodiment 154. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 149, 152 and 153, preferably the compound of any one of Embodiments 120, 149, 152 and 153, more preferably the compound of Embodiment 153, wherein the bio-distribution modifier comprised by the N-terminal modification group A is selected from the group comprising HAC2:HPD:Aur, HPD2:HPD:Aur, HAC2:HAC:Aur, HPD2:HAC:Aur, preferably is selected from the group comprising HAC2:HAC:Aur and HPD2:HAC:Aur.

Embodiment 155. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 149 and 152, preferably the compound of any one of Embodiments 120, 149 and 152, more preferably the compound of Embodiment 152, wherein b₁ is 1 and each and any of R^{A3}, R^{A4} and R^{A5} is a second level alkyl group of a structure of formula (A-III), wherein as in R^{A3}, as in R^{A4} and as in R^{A5} are identical.

Embodiment 156. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 149, 152 and 155, preferably the compound of any one of Embodiments 120, 149, 152 and 155, more preferably the compound of Embodiment 155, wherein the bio-distribution modifier comprised by the N-terminal modification group A is selected from the group comprising HAC3:PEO:Aur, HAC3:TRIS:Aur, HPD3:PEO:Aur, HPD3:TRIS:Aur.

Embodiment 157. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 149 and 152, preferably the compound of any one of Embodiments 120, 149 and 152, more preferably the compound of Embodiment 152, wherein b₁ is 1 and R^{A3} and R^{A4} are a second level alkyl group of a structure of formula (A-VII) and R^{A5} is H, wherein as in R^{A3} and as in R^{A4} are identical.

Embodiment 158. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 149, 152 and 157, preferably the compound of any one of Embodiments 120, 149, 152 and 157, more preferably the compound of Embodiment 157, wherein the bio-distribution modifier comprised by the N-terminal modification group A is selected from the group comprising HAC2:PEO:Aur, HAC2:TRIS:Aur, HPD2:PEO:Aur and HPD2:TRIS:Aur.

Embodiment 159. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 149 and 152, preferably the compound of any one of Embodiments 120, 149 and 152, more preferably the compound of Embodiment 152, wherein b₁ is 1 and R^{A3} is a second level alkyl group of a structure of formula (A-III) and R^{A4} and R^{A5} are H.

Embodiment 160. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 149, 152 and 159, preferably the compound of any one of Embodiments 120, 149, 152 and 159, more preferably the compound of Embodiment 159, wherein the bio-distribution modifier comprised by the N-terminal modification group A is selected from the group comprising HAC:PEO:Aur, HAC:TRIS:Aur, HPD:PEO:Aur and HPD:TRIS:Aur.

Embodiment 161. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159 and 160, wherein a linker comprising an amino group and a carbonyl group is optionally interspersed between Xaa11 and the bio-distribution modifier comprised by the C-terminal modification group C-term,
wherein the bio-distribution modifier comprises a residue of an α-amino acid with an aliphatic side chain,
wherein to an ω-carbon atom of the aliphatic side chain of the α-amino acid a nitrogen atom, a triazolyl moiety, a hydroxymethyl-triazolyl moiety or an urea moiety is covalently attached,
   wherein to the nitrogen atom, to the triazolyl moiety, to the hydroxymethyl triazolyl moiety or to the urea moiety
   (a) 1 or 2 first level alkyl groups are covalently attached, wherein each and any first level alkyl group individually and independently comprises 2 or more hydroxyl groups, wherein each and any hydroxyl group is a primary hydroxyl group,
or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
and wherein to the carbonyl group attached to the α-carbon atom of the α-amino acid an amino group is covalently attached thereby forming an amide,
preferably the bio-distribution modifier is a structure of formula (C-I), 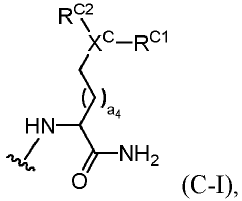
wherein
   - X^{C}: comprises a nitrogen atom, a triazolyl moiety, a hydroxymethyl-triazolyl moiety and an urea moiety,
   - a₄: is 0, 1, 2, 3, or 4,
   if X^{C} is a triazolyl moiety, preferably a₄ is 1 or 2, more preferably a₄ is 1,
   if X^{C} is selected from the group comprising a nitrogen atom, a hydroxymethyl-triazolyl moiety and an urea moiety, preferably a₄ is 3,
      R^{C1} is a first level alkyl group of a structure of formula (C-II), 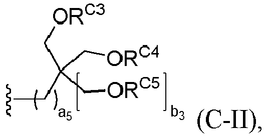 wherein
         as is 0 or 1,
         b₃ is 0 or 1,
      wherein, if b₃ is 0,
      R^{C3} and R^{C4} are individually and independently a second level alkyl group of a structure of formula (C-III), or R^{C3} and R^{C4} are H,
   or wherein, if b₃ is 1, each and any of R^{C3}, R^{C4} and R^{C5} is selected from a second level alkyl group of a structure of formula (C-III) and H, preferably each and any of R^{C3}, R^{C4} and R^{C5} is individually and independently a second level alkyl group of a structure of formula (C-III), 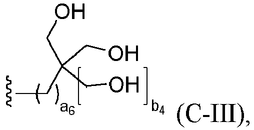 wherein
      a₆ is 0 or 1, and
      b₄ is 0 or 1
   - and R^{C2}: is absent, if X^{C} is selected from the group comprising a triazolyl moiety, a hydroxmethyl-triazolyl moiety and an urea moiety,
   - or R^{C2}: is H or is a first level alkyl group of a structure of formula (C-II), if X^{C} is a nitrogen atom, wherein
   a₅ is 0 or 1,
   b₃ is 0 or 1,
   wherein, if b₃ is 0,
   R^{C3} and R^{C4} are individually and independently a second level alkyl group of a structure of formula (C-III), or R^{C3} and R^{C4} are H,
      or wherein, if b₃ is 1,
   each and any of R^{C3}, R^{C4} and R^{C5} is individually and independently selected from a second level alkyl group of a structure of formula (C-III) and H, preferably each and any of R^{C3}, R^{C4} and R^{C5} is individually and independently a second level alkyl group of a structure of formula (C-III), wherein in formula (C-III)
   a₆ is 0 or 1, and
   b₄ is 0 or 1,
and wherein the α-nitrogen atom of the structure (C-I) is covalently attached to the carbonyl group of Xaa11, or if a linker comprising an amino group and a carbonyl group is interspersed between Xaa11 and the bio-distribution modifier comprised by the C-terminal modification group C-term, the α-nitrogen atom of the structure (C-I) is covalently attached to the carbonyl group of the linker, more preferably the linker is interspersed between Xaa11 and the bio-distribution comprised by the C-terminal modification group C-term.

Embodiment 162. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160 and 161, preferably the compound of Embodiment 161, wherein a linker comprising an amino group and a carbonyl group is optionally interspersed between Xaa11 and the bio-distribution modifier comprised by the C-terminal modification group C-term, wherein the bio-distribution modifier comprises a residue of an α-amino acid with an aliphatic side chain, wherein to an ω-carbon atom of the aliphatic side chain of the α-amino acid a nitrogen atom is covalently attached,
wherein to the nitrogen atom,
(a) 1 or 2 first level alkyl groups are covalently attached, wherein each and any first level alkyl group individually and independently comprises 2 or more hydroxyl groups, wherein each and any hydroxyl group is a primary hydroxyl group,

or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
   and wherein to the carbonyl group attached to the α-carbon atom of the α-amino acid an amino group is covalently attached thereby forming an amide,
preferably the bio-distribution modifier is a structure of formula (C-IV), 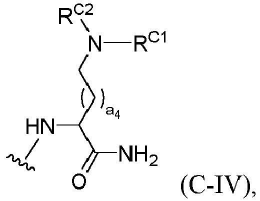 wherein
   - a₄: is 0, 1, 2, 3, or 4, preferably a₄ is 3,
   - R^{C1}: is a first level alkyl group of a structure of formula (C-II), 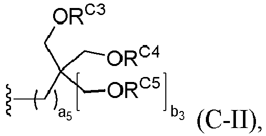 wherein
   a₅ is 0 or 1,
   b₃ is 0 or 1,

   wherein, if b₃ is 0,
   R^{C3} and R^{C4} are individually and independently a second level alkyl group of a structure of formula (C-III), or R^{C3} and R^{C4} are each H,
      or wherein, if b₃ is 1, each and any of R^{C3}, R^{C4} and R^{C5} is individually and independently selected from a second level alkyl group of a structure of formula (C-III) and H, preferably each and any of R^{C3}, R^{C4} and R^{C5} is individually and independently a second level alkyl group of a structure of formula (C-III), 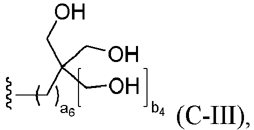 wherein
      - a₆: is 0 or 1, and
      - b₄: is 0 or 1
   and wherein
   - R^{C2}: is H or is a first level alkyl group of a structure of formula (C-II), wherein
   a₅ is 0 or 1,
   b₃ is 0 or 1,

   wherein, if b₃ is 0,
   R^{C3} and R^{C4} are individually and independently a second level alkyl group of a structure of formula (C-III), or R^{C3} and R^{C4} are H,
      or wherein, if b₃ is 1,
   each and any of R^{C3}, R^{C4} and R^{C5} is individually and independently selected from a second level alkyl group of a structure of formula (C-III) and H, preferably each and any of R^{C3}, R^{C4} and R^{C5} is individually and independently a second level alkyl group of a structure of formula (C-III), wherein in formula (C-III)

   - a₆: is 0 or 1, and
   - b₄: is 0 or 1,
and wherein the α-nitrogen atom of the structure (C-I) is covalently attached to the carbonyl group of Xaa11, or if a linker comprising an amino group and a carbonyl group is interspersed between Xaa11 and the bio-distribution modifier comprised by the C-terminal modification group C-term, the α-nitrogen atom of the structure (C-I) is covalently attached to the carbonyl group of the linker, more preferably the linker is interspersed between Xaa11 and the bio-distribution comprised by the C-terminal modification group C-term, preferably the linker is selected from the group comprising APAc and Ttds.

Embodiment 163. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161 and 162, preferably the compound of any one of Embodiments 161 and 162, more preferably the compound of Embodiment 162, wherein the bio-distribution modifier comprised by the C-terminal modification group C-term comprises a residue of an α-amino acid with an aliphatic side chain, wherein to an ω-carbon atom of the aliphatic side chain of the α-amino acid a nitrogen atom is covalently attached, wherein to the nitrogen atom, 1 or 2 first level alkyl groups are covalently attached, wherein each and any first level alkyl group individually and independently comprises 2 or more hydroxyl groups, wherein each and any hydroxyl group is a primary hydroxyl group, wherein preferably the bio-distribution modifier is a structure of formula (C-IV), 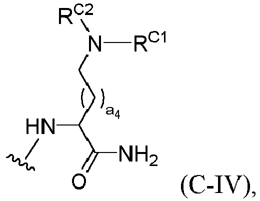 wherein
- a₄: is 0, 1, 2, 3, or 4, preferably a₄ is 3,
- R^{C1}: is a first level alkyl group of a structure of formula (C-V), 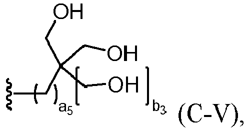
wherein as is 0 or 1 and b₃ is 0 or 1,
and wherein
- R^{C2}: is a first level alkyl group of a structure of formula (C-V), wherein as is 0 or 1 and b₃ is 0 or 1,
- or R^{A2}: is H.

Embodiment 164. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162 and 163, preferably the compound of any one of Embodiments 161, 162 and 163, more preferably the compound of Embodiment, wherein R^{C1} is a first level alkyl group of structure of formula (C-V), wherein as is 0 or 1, preferably as is 0, and b₃ is 0, and wherein R^{C2} is H.

Embodiment 165. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163 and 164, preferably the compound of any one of Embodiment 161, 162, 163 and 164, more preferably the compound of Embodiment 164, wherein the bio-distribution modifier comprised by the C-terminal modification group C-term is lys:HAC.

Embodiment 166. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162 and 163, preferably the compound of any one of Embodiments 161, 162 and 163, more preferably the compound of Embodiment 163, wherein R^{C1} is a first level alkyl group of a structure of formula (C-V), wherein as is 0 or 1, preferably as is 1, and b₃ is 0 or 1, and wherein R^{C2} is a first level alkyl group of structure of formula (C-V), wherein as is 0 or 1, preferably as is 1, and b₃ is 0 or 1.

Embodiment 167. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163 and 166, preferably the compound of any one of Embodiments 161, 162 and 163 and 166, more preferably the compound of Embodiment 166,
wherein R^{C1} is a first level alkyl group of structure of formula (C-V),
wherein a₅ is 0 or 1, preferably a₅ is 1, and b₃ is 0 or 1,
and wherein R^{C2} is a first level alkyl group of structure of formula (C-V),
   wherein a₅ is 0 or 1, preferably a₅ is 1, and b₃ is 0 or 1,
and wherein a₅ in R^{C1} and a₅ in R^{C2} are identical,
   and b₃ in R^{C1} and b₃ in R^{C2} are identical.

Embodiment 168. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 166 and 167, preferably the compound of any one of Embodiments 161, 162 and 163, 166 and 167, more preferably the compound of Embodiment 167, wherein the bio-distribution modifier comprised by the C-terminal modification group C-term is selected from the group comprising lys:HPD2 and lys:PEO2.

Embodiment 169. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161 and 162, preferably the compound of any one of Embodiments 161 and 162, more preferably the compound of Embodiment 162, wherein a linker is optionally interspersed between Xaa11 and the bio-distribution modifier comprised by the C-terminal modification group C-term,
wherein the bio-distribution modifier comprises a residue of an α-amino acid with an aliphatic side chain, wherein to an ω-carbon atom of the aliphatic side chain of the α-amino acid a nitrogen atom is covalently attached,
wherein to the nitrogen atom, 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,

and wherein to the carbonyl group attached to the α-carbon atom of the α-amino acid an amino group is covalently attached thereby forming an amide,
preferably the bio-distribution modifier is a structure of formula (C-IV), 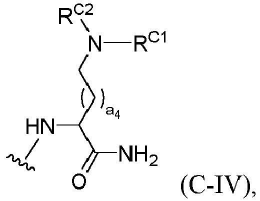 wherein
   - a₄: is 0, 1, 2, 3, or 4, preferably a₄ is 3,
   - R^{C1}: is a first level alkyl group of a structure of formula (C-II), 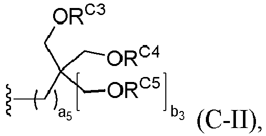
   wherein
   - a₅: is 0 or 1,
   - b₃: is 0 or 1,

   wherein, if b₃ is 0,
   R^{C3} and R^{C4} are individually and independently a second level alkyl group of a structure of formula (C-III), or R^{C3} and R^{C4} are each H,
      or wherein, if b₃ is 1, each and any of R^{C3}, R^{C4} and R^{C5} is selected from a second level alkyl group of a structure of formula (C-III) and H, preferably each and any of R^{C3}, R^{C4} and R^{C5} is a second level alkyl group of a structure of formula (C-III), 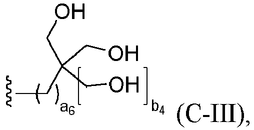 wherein
      - a₆: is 0 or 1, and
      - b₄: is 0 or 1, preferably b₄ is 0
   and wherein
   - R^{C2}: is H or is a first level alkyl group of a structure of formula (C-II), wherein
   a₅ is 0 or 1,
   b₃ is 0 or 1,

   wherein, if b₃ is 0,
   R^{C3} and R^{C4} are individually and independently a second level alkyl group of a structure of formula (C-III), or R^{C3} and R^{C4} are each H,
      or wherein, if b₃ is 1,
   each and any of R^{C3}, R^{C4} and R^{C5} is individually and independently selected from a second level alkyl group of a structure of formula (C-III) and H, preferably each and any of R^{C3}, R^{C4} and R^{C5} is individually and independently a second level alkyl group of a structure of formula (C-III), wherein in formula (C-III)
      - a₆: is 0 or 1, and
      - b₄: is 0 or 1.

Embodiment 170. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162 and 169, preferably the compound of any one of Embodiments 161, 162 and 169, more preferably the compound of Embodiment 169, wherein the bio-distribution modifier comprised by the C-terminal modification group C-term is a structure of formula (C-IV), 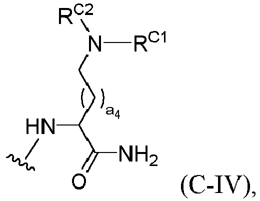 wherein
- a₄: is 0, 1, 2, 3, 4, 5, or 6, preferably a₄ is 3,
- R^{C1}: is a first level alkyl group of a structure of formula (C-VI), 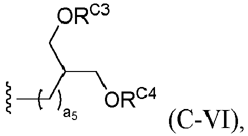
wherein as is 0 or 1, and wherein
R^{C3} and R^{C4} are individually and independently a second level alkyl group of a structure of formula (C-VII), 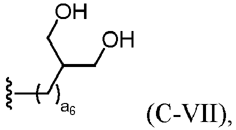
wherein a₆ is 0 or 1,
   and wherein
   - R^{C2}: is a first level alkyl group of a structure of formula (C-VI), wherein as is 0 and wherein,
   - R^{C3} and R^{C4}: are individually and independently a second level alkyl group of a structure of formula (C-VII), wherein in formula (C-VII) a₆ is 0 or 1,
   - or R^{C2}: is H.

Embodiment 171. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 169 and 170, preferably the compound of any one of Embodiments 161, 162, 169 and 170, more preferably the compound of Embodiment 170, wherein R^{C1} is a first level alkyl group of structure of formula (C-VI), wherein as is 0 or 1, preferably as is 0, and wherein a₆ in R^{C3} and a₆ in R^{C4} are identical and wherein R^{C2} is H.

Embodiment 172. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162 and 169 to 171, preferably the compound of any one of Embodiments 161, 162 and 169 to 171, more preferably the compound of Embodiment 171, wherein the bio-distribution modifier comprised by the C-terminal modification group C-term is selected from the group comprising lys:HAC:HAC2 and lys:HAC:HPD2.

Embodiment 173. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162 and 169, preferably the compound of any one of Embodiments 161, 162 and 169, more preferably the compound of Embodiment 169, wherein
wherein R^{C1} is a first level alkyl group of a structure of formula (C-VI),
wherein a₅ is 0 or 1, preferably a₅ is 1,
and wherein R^{C2} is a first level alkyl group of a structure of formula (C-VI),
   wherein a₅ is 0 or 1, preferably a₅ is 1,
   preferably a₅ in R^{C1} and a₅ in R^{C2} are identical,
and wherein preferably a₆ in R^{C3} and a₆ in R^{C4} of R^{C1} and a₆ in R^{C3} and a₆ in R^{C4} of R^{C2} are identical.

Embodiment 174. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 169 and 173, preferably the compound of any one of Embodiments 161, 162, 169 and 173, more preferably the compound of Embodiment 173, wherein the bio-distribution modifier comprised by the C-terminal modification group C-term is selected from the group comprising lys:HPD2:HAC4 and lys:HPD2:HPD4.

Embodiment 175. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160 and 161, preferably the compound of Embodiment 161, wherein a linker comprising an amino group and a carbonyl group is optionally interspersed between Xaa11 and the bio-distribution modifier comprised by the C-terminal modification group C-term,
wherein the bio-distribution modifier comprises a residue of an α-amino acid with an aliphatic side chain,
   wherein to an ω-carbon atom of the aliphatic side chain of the α-amino acid a triazolyl moiety is covalently attached, wherein to the triazolyl moiety,
      (a) one first level alkyl group is covalently attached, wherein the first level alkyl group comprises 2 or more hydroxyl groups, wherein each and any hydroxyl group is a primary hydroxyl group,
   or, (b) one first level alkyl groups is covalently attached, wherein the first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein the first level alkyl group comprises 0, 1 or 2 hydroxyl groups, wherein each and any hydroxyl group is a primary hydroxyl group,
and wherein to the carbonyl group attached to the α-carbon atom of the α-amino acid an amino group is covalently attached thereby forming an amide,
preferably the bio-distribution modifier is a structure of formula (C-VIII), 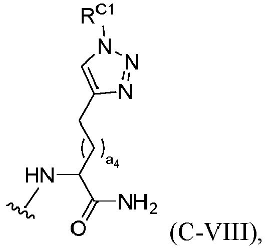
wherein
   - a₄: is 0, 1, 2, 3, or 4, more preferably a₄ is 1
   - R^{C1}: is a first level alkyl group of a structure of formula (C-II), 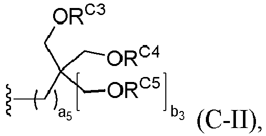 wherein
   a₅ is 0 or 1,
   b₃ is 0 or 1,

   wherein, if b₃ is 0,
   R^{C3} and R^{C4} are individually and independently a second level alkyl group of a structure of formula (C-III), or R^{C3} and R^{C4} are each H,
      or wherein, if b₃ is 1, each and any of R^{C3}, R^{C4} and R^{C5} is individually and independently selected from a second level alkyl group of a structure of formula (C-III) and H, preferably each and any of R^{C3}, R^{C4} and R^{C5} is individually and independently a second level alkyl group of a structure of formula (C-III), 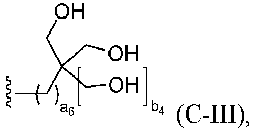 wherein
      - a₆: is 0 or 1, and
      - b₄: is 0 or 1.

Embodiment 176. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161 and 175, preferably the compound of any one of Embodiments 161 and 175, more preferably the compound of Embodiment 175, wherein to the triazolyl moiety one first level alkyl groups is covalently attached, wherein the first level alkyl group comprises 2 or more hydroxyl groups, wherein each and any hydroxyl group is a primary hydroxyl group, wherein the preferably the bio-distribution modifier is a structure of formula (C-VIII), 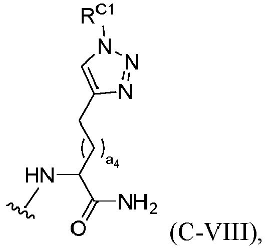 wherein
- a₄: is 0, 1, 2, 3, or 4, more preferably a₄ is 1
- R^{C1}: is a first level alkyl group of a structure of formula (C-V), 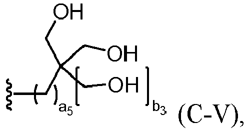
wherein a₅ is 0 or 1 and b₃ is 0 or 1.

Embodiment 177. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 161, 175 and 176, preferably the compound of any one of Embodiments 161, 175 and 176, more preferably the compound of Embodiment 176, wherein the bio-distribution modifier comprised by the C-terminal modification group C-term is selected from the group comprising prx:HPD, prx:PEO, prx:TRIS and prx:HAC, preferably is selected from the group comprising prx:HPD, prx:PEO and prx:TRIS.

Embodiment 178. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161 and 175, preferably the compound of any one of Embodiments 161 and 175, more preferably the compound of Embodiment 175, wherein to the triazolyl moiety one first level alkyl groups is covalently attached, wherein the first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
preferably the bio-distribution modifier is a structure of formula (C-VIII), 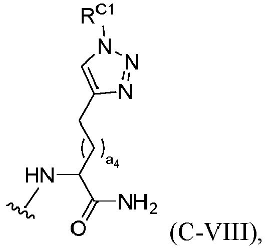
wherein
   - a₄: is 0, 1, 2, 3, or 4, more preferably a₄ is 1
   - R^{C1}: is a first level alkyl group of a structure of formula (C-II), 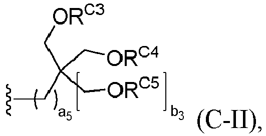
   wherein
   - a₅: is 0 or 1,
   - b₃: is 0 or 1,
   wherein, if b₃ is 0,
   R^{C3} and R^{C4} are individually and independently a second level alkyl group of a structure of formula (C-III),
      or wherein, if b₃ is 1,
   each and any of R^{C3}, R^{C4} and R^{C5} is individually and independently selected from a second level alkyl group of a structure of formula (C-VII) and H, preferably each and any of R^{C3}, R^{C4} and R^{C5} is individually and independently a second level alkyl group of a structure of formula (C-VII), 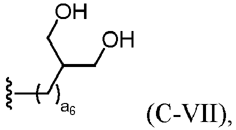 wherein a₆ is 0 or 1.

Embodiment 179. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 175 and 178, preferably the compound of any one of Embodiments 161, 175 and 178, more preferably the compound of Embodiment 178, wherein if b₃ is 0, and a₆ in R^{C3} and a₆ in R^{C4} are identical.

Embodiment 180. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 175, 178 and 179, preferably the compound of any one of Embodiments 161, 175, 178 and 179, more preferably the compound of Embodiment 179, wherein the bio-distribution modifier comprised by the C-terminal modification group C-term is selected from the group comprising prx:HPD:HAC2, prx:HPD:HPD2, prx:HAC:HAC2 and prx:HAC:HPD2, preferably is selected from the group comprising prx:HPD:HAC2 and prx:HPD:HPD2.

Embodiment 181. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 175 and 178, preferably the compound of any one of Embodiments 161, 175 and 178, more preferably the compound of Embodiment 178,
wherein if b₃ is 1 and each and any of R^{C3}, R^{C4} and R^{C5} is a second level alkyl group of a structure of formula (C-VII), wherein a₆ in R^{C3}, a₆ in R^{C4} and a₆ in R^{C5} are identical.

Embodiment 182. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 175, 178 and 181, preferably the compound of any one of Embodiments 161, 175, 178 and 181, more preferably the compound of Embodiment 181, wherein the bio-distribution modifier comprised by the C-terminal modification group C-term is selected from the group comprising prx:PEO:HPD3, prx:TRIS:HPD3, prx:TRIS:HAC3 and prx:PEO:HAC3.

Embodiment 183. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 161, 175 and 178, preferably the compound of any one of Embodiments 161, 175 and 178, more preferably the compound of Embodiment 178,
wherein if b₃ is 1 and R^{C3} and R^{C4} are a second level alkyl group of a structure of formula (C-VII) and R^{C5} is H, and a₆ in R^{C3} and a₆ in R^{C4} are identical.

Embodiment 184. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 175, 178 and 183, preferably the compound of any one of Embodiments 161, 175, 178 and 183, more preferably the compound of Embodiment 183, wherein the bio-distribution modifier comprised by the C-terminal modification group C-term is selected from the group comprising prx:PEO:HPD2, prx:PEO:HAC2, prx:TRIS:HPD2 and prx:TRIS:HAC2.

Embodiment 185. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 175 and 178, preferably the compound of any one of Embodiments 161, 175 and 178, more preferably the compound of Embodiment 178,
wherein if b₃ is 1 and R^{C3} is a second level alkyl group of a structure of formula (C-VII), R^{C4} and R^{C5} are each H.

Embodiment 186. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 175, 178 and 185, preferably the compound of any one of Embodiment 161, 175, 178 and 185, more preferably the compound of Embodiment 185, wherein the bio-distribution modifier comprised by the C-terminal modification group C-term is selected from the group comprising prx:PEO:HPD, prx:PEO:HAC, prx:TRIS:HPD and prx:TRIS:HAC.

Embodiment 187. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, preferably the compound of Embodiment 161, wherein a linker optionally comprising an amino group and a carbonyl group is optionally interspersed between Xaa11 and the bio-distribution modifier comprised by the C-terminal modification group C-term,
wherein the bio-distribution modifier comprises a residue of an α-amino acid with an aliphatic side chain,
wherein to an ω-carbon atom of the aliphatic side chain of the α-amino acid a hydroxymethyl-triazolyl moiety is covalently attached,
   wherein to the hydroxymethyl triazolyl moiety
   one first level alkyl group is covalently attached, wherein the first level alkyl group is covalently attached via an ether linkage to two second level alkyl groups, wherein each and any second level alkyl group individually and independently comprises 2 or more hydroxyl groups, wherein each and any hydroxyl group is a primary hydroxyl group,
and wherein to the carbonyl group attached to the α-carbon atom of the α-amino acid an amino group is covalently attached thereby forming an amide,
preferably the bio-distribution modifier is a structure of formula (C-IX), 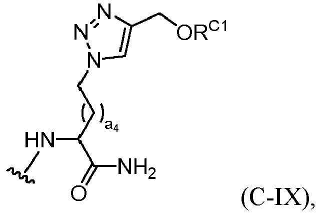
wherein
   - a₄: is 0, 1, 2, 3, or 4, preferably a₄ is 3,
   - R^{C1}: is a first level alkyl group of a structure of formula (C-VI), 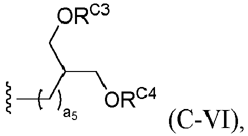 wherein
   as is 0 or 1,
   R^{C3} and R^{C4} are individually and independently a second level alkyl group of a structure of formula (C-VII), 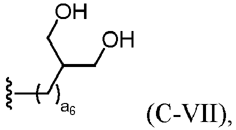
   wherein a₆ is 0 or 1.

Embodiment 188. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161 and 187, preferably the compound of any one of Embodiments 161 and 187, more preferably the compound of Embodiment 187, wherein a₆ in R^{C3} and a₆ in R^{C4} are identical.

Embodiment 189. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 187 and 188, preferably the compound of any one of Embodiments 161, 187 and 188, more preferably the compound of Embodiment 188, wherein the bio-distribution modifier comprised by the C-terminal modification group C-term is selected from the group comprising tzk:HAC:HAC2, tzk:HPD:HAC2, tzk:HPD:HPD2, and tzk:HAC:HPD2.

Embodiment 190. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160 and 161, preferably the compound of Embodiment 161, wherein a linker comprising an amino group and a carbonyl group is optionally interspersed between Xaa11 and the bio-distribution modifier comprised by the C-terminal modification group C-term,
wherein the bio-distribution modifier comprises a residue of an α-amino acid with an aliphatic side chain,
wherein to an ω-carbon atom of the aliphatic side chain of the α-amino acid an urea moiety is covalently attached,
   wherein to the urea moiety
      (a) one first level alkyl group is covalently attached, wherein the first level alkyl group comprises 2 or more hydroxyl groups, wherein each and any hydroxyl group is a primary hydroxyl group,
   or, (b) one first level alkyl group is covalently attached, wherein the first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein the first level alkyl group comprises 0, 1 or 2 hydroxyl groups, wherein each and any hydroxyl group is a primary hydroxyl group,
and wherein to the carbonyl group attached to the α-carbon atom of the α-amino acid an amino group is covalently attached thereby forming an amide,
preferably the bio-distribution modifier is a structure of formula (C-X), 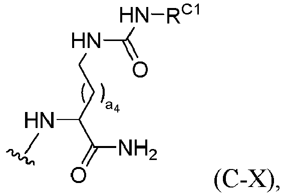
wherein
   - a₄: is 0, 1, 2, 3, or 4, preferably a₄ is 3,
   - R^{C1}: is a first level alkyl group of a structure of formula (C-II), 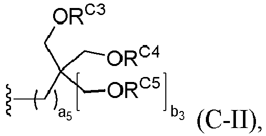 wherein
   as is 0 or 1,
   b₃ is 0 or 1,

   wherein, if b₃ is 0,
      R^{C3} and R^{C4} are individually and independently a second level alkyl group of a structure of formula (C-III), or R^{C3} and R^{C4} are H,
   or wherein, if b₃ is 1, each and any of R^{C3}, R^{C4} and R^{C5} is individually and independently selected from a second level alkyl group of a structure of formula (C-III) and H, preferably each and any of R^{C3}, R^{C4} and R^{C5} is individually and independently a second level alkyl group of a structure of formula (C-III), 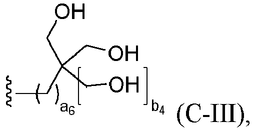 wherein
      - a₆: is 0 or 1, and
      - b₄: is 0 or 1
and wherein the α-nitrogen atom of the structure (C-I) is covalently attached to the carbonyl group of Xaa11, or if a linker comprising an amino group and a carbonyl group is interspersed between Xaa11 and the bio-distribution modifier comprised by the C-terminal modification group C-term, the α-nitrogen atom of the structure (C-I) is covalently attached to the carbonyl group of the linker, more preferably the linker is interspersed between Xaa11 and the bio-distribution comprised by the C-terminal modification group C-term.

Embodiment 191. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161 and 190, preferably the compound of any one of Embodiments 161 and 190, more preferably the compound of Embodiment 190, wherein to the urea moiety one first level alkyl group is covalently attached, wherein the first level alkyl group comprises 2 or more hydroxyl groups, wherein each and any hydroxyl group is a primary hydroxyl group, wherein the preferably the bio-distribution modifier is a structure of formula (C-X), 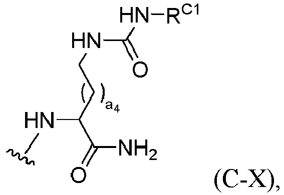 wherein
- a₄: is 0, 1, 2, 3, or 4, preferably a₄ is 3,
- R^{C1}: is a first level alkyl group of a structure of formula (C-V), 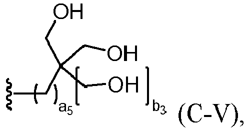
wherein as is 0 or 1 and bs is 0 or 1.

Embodiment 192. The compound of any one of Embodiments 1 to 161, 190 and 191, preferably the compound of any one of Embodiments 161, 190 and 191, more preferably the compound of Embodiment 191, wherein the bio-distribution modifier comprised by the C-terminal modification group C-term is selected from the group comprising kur:HPD, kur:PEO, kur:TRIS and kur:HAC.

Embodiment 193. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161 and 190, preferably the compound of any one of Embodiments 161 and 190, more preferably the compound of Embodiment 190, wherein to the urea moiety one first level alkyl group is covalently attached, wherein the first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein the first level alkyl group comprises 0, 1 or 2 hydroxyl groups, wherein each and any hydroxyl group is a primary hydroxyl group, preferably the bio-distribution modifier is a structure of formula (C-X), 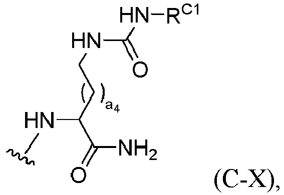 wherein
- a₄: is 0, 1, 2, 3, or 4, preferably a₄ is 3,
- R^{C1}: is a first level alkyl group of a structure of formula (C-II), 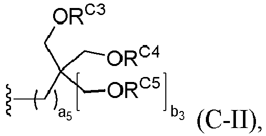 wherein
as is 0 or 1,
b₃ is 0 or 1,

wherein, if b₃ is 0,
R^{C3} and R^{C4} are individually and independently a second level alkyl group of a structure of formula (C-VII),
   or wherein, if b₃ is 1, each and any of R^{C3}, R^{C4} and R^{C5} is individually and independently selected from a second level alkyl group of a structure of formula (C-VII) and H, preferably each and any of R^{C3}, R^{C4} and R^{C5} individually and independently is a second level alkyl group of a structure of formula (C-VII), 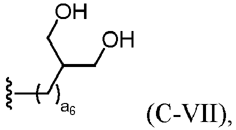
wherein a₆ is 0 or 1.

Embodiment 194. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 190 and 193, preferably the compound of any one of Embodiments 161, 190 and 193, more preferably the compound of Embodiment 193, wherein if b₃ is 0, a₆ in R^{C3} and a₆ in R^{C4} are identical.

Embodiment 195. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 190, 193 and 194, preferably the compound of any one of Embodiments 161, 190, 193 and 194, more preferably the compound of Embodiment 194, wherein the bio-distribution modifier comprised by the C-terminal modification group C-term is selected from the group comprising kur:HPD:HAC2, kur:HPD:HPD2, kur:HAC:HAC2, and kur:HAC:HPD2, preferably is selected from the group comprising kur:HAC:HAC2 and kur:HAC:HPD2.

Embodiment 196. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 190 and 193, preferably the compound of any one of Embodiments 161, 190 and 193, more preferably the compound of Embodiment 193,
wherein if b₃ is 1 and each and any of R^{C3}, R^{C4} and R^{C5} is a second level alkyl group of a structure of formula (C-VII) and a₆ in R^{C3}, a₆ in R^{C4} and a₆ in R^{C5} are identical.

Embodiment 197. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 190, 193 and 196, preferably the compound of any one of Embodiments 161, 190, 193 and 196, more preferably the compound of Embodiment 196, wherein the bio-distribution modifier comprised by the C-terminal modification group C-term is selected from the group comprising kur:PEO:HAC3, kur:TRIS:HAC3, kur:PEO:HPD3 and kur:TRIS:HPD3.

Embodiment 198. The compound of any one of Embodiments 1 to 161, 190 and 193, preferably the compound of any one of Embodiments 161, 190 and 193, more preferably the compound of Embodiment 193,
wherein if b₃ is 1 and R^{C3} and R^{C4} are a second level alkyl group of a structure of formula (C-VII) and R^{C5} is H, and a₆ in R^{C3} and a₆ in R^{C4} are identical.

Embodiment 199. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 190, 193 and 198, preferably the compound of any one of Embodiments 161, 190, 193 and 198, more preferably the compound of Embodiment 198, wherein the bio-distribution modifier comprised by the C-terminal modification group C-term is selected from the group comprising kur:PEO:HAC2, kur:TRIS:HAC2, kur:PEO:HPD2 and kur:TRIS:HPD2.

Embodiment 200. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 190 and 193, preferably the compound of any one of Embodiments 161, 190 and 193, more preferably the compound of Embodiment 193,
wherein bs is 1 and R^{C3} is a second level alkyl group of a structure of formula (C-VII) and R^{C4} and R^{C5} are each H.

Embodiment 201. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 190, 193 and 200, preferably the compound of any one of Embodiments 161, 190, 193 and 200, more preferably the compound of Embodiment 200, wherein the bio-distribution modifier comprised by the C-terminal modification group C-term is selected from the group comprising kur:PEO:HAC, kur:TRIS:HAC, kur:PEO:HPD and kur:TRIS:HPD.

Embodiment 202. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200 and 201, wherein the bio-distribution modifier comprised by the N-terminal modification group A
is selected from the group comprising HPD2:Ahx, HPD3:PEO:Hyx, PEO:Aur, PEO:Hyx, PEO2:Ahx, TRIS:Hyx, HPD3:TRIS:Hyx, HAC3:TRIS:Hyx, HAC3:PEO:Hyx, HAC3:PEO:Aur, HAC3:TRIS:Aur, HPD3:PEO:Aur, HPD3:TRIS:Aur, TRIS:Aur, HAC2:HPD:Aur, HAC2:HPD:Hyx, HAC4:HPD2:Ahx, HPD2:HPD:Aur, HPD2:HPD:Hyx, HPD4:HPD2:Ahx, HAC2:HAC:Aur, HPD2:HAC:Aur, HAC2:HAC:Ahx, HAC2:HAC:Tzx, HAC2:HPD:Tzx, HPD:Aur, HPD:Hyx, HPD2:HAC:Ahx, HPD2:HPD:Tzx, HAC:Aur, HAC2:PEO:Hyx, HPD2:TRIS:Hyx, HAC2:TRIS:Hyx, HAC2:PEO:Aur, HAC2:TRIS:Aur, HPD2:PEO:Aur, HPD2:TRIS:Aur, HPD2:HAC:Tzx, HPD2:PEO:Hyx, HAC2:HAC:Hyx, HPD2:HAC:Hyx, HPD:PEO:Hyx, HAC:PEO:Hyx, HPD:TRIS:Hyx, HAC:TRIS:Hyx, HAC:PEO:Aur, HAC:TRIS:Aur, HPD:PEO:Aur, HPD:TRIS:Aur, HAC:Ahx and HAC:Hyx,
preferably is selected from the group comprising HPD2:Ahx, HPD3:PEO:Hyx, PEO:Aur, PEO:Hyx, PEO2:Ahx, TRIS:Hyx, HPD3:TRIS:Hyx, HAC3:TRIS:Hyx, HAC3:PEO:Hyx, HAC3:PEO:Aur, HAC3:TRIS:Aur, HPD3:PEO:Aur, HPD3:TRIS:Aur, TRIS:Aur, HAC2:HPD:Aur, HAC2:HPD:Hyx, HAC4:HPD2:Ahx, HPD2:HPD:Aur, HPD2:HPD:Hyx, HPD4:HPD2:Ahx, HAC2:HAC:Aur, HPD2:HAC:Aur, HAC2:HAC:Ahx, HAC2:HAC:Tzx, HAC2:HPD:Tzx, HPD:Aur, HPD:Hyx, HPD2:HAC:Ahx, HPD2:HPD:Tzx, HAC:Aur, HAC2:PEO:Hyx, HPD2:TRIS:Hyx, HAC2:TRIS:Hyx, HAC2:PEO:Aur, HAC2:TRIS:Aur, HPD2:PEO:Aur, HPD2:TRIS:Aur and HPD2:HAC:Tzx,
more preferably is selected from the group comprising HPD2:Ahx, HPD3:PEO:Hyx, PEO:Aur, PEO:Hyx, PEO2:Ahx, TRIS:Hyx, HPD3:TRIS:Hyx, HAC3:TRIS:Hyx, HAC3:PEO:Hyx, HAC3:PEO:Aur, HAC3:TRIS:Aur, HPD3:PEO:Aur, HPD3:TRIS:Aur, TRIS:Aur, HAC2:HPD:Aur, HAC2:HPD:Hyx, HAC4:HPD2:Ahx, HPD2:HPD:Aur, HPD2:HPD:Hyx, HPD4:HPD2:Ahx, HAC2:HAC:Aur and HPD2:HAC:Aur,
and most preferably is selected from the group comprising HPD2:Ahx, HPD3:PEO:Hyx, PEO:Aur, PEO:Hyx, PEO2:Ahx, TRIS:Hyx, HPD3:TRIS:Hyx, HAC3:TRIS:Hyx, HAC3:PEO:Hyx, HAC3:PEO:Aur, HAC3:TRIS:Aur, HPD3:PEO:Aur, HPD3:TRIS:Aur and TRIS:Aur.

Embodiment 203. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201 and 202, preferably the compound of Embodiment 202, wherein the bio-distribution modifier comprised by the N-terminal modification group
is selected from the group comprising HPD2:Ahx, HPD3:PEO:Hyx, PEO:Aur, PEO:Hyx, PEO2:Ahx, TRIS:Hyx, HPD3:TRIS:Hyx, HAC3:TRIS:Hyx, HAC3:PEO:Hyx, HAC3:PEO:Aur, HAC3:TRIS:Aur, HPD3:PEO:Aur, HPD3:TRIS:Aur, TRIS:Aur, HAC2:HPD:Aur, HAC2:HPD:Hyx, HAC4:HPD2:Ahx, HPD2:HPD:Aur, HPD2:HPD:Hyx, HPD4:HPD2:Ahx, HAC2:HAC:Aur and HPD2:HAC:Aur
and preferably is selected from the group comprising HPD2:Ahx, HPD3:PEO:Hyx, PEO:Aur, PEO:Hyx, PEO2:Ahx, TRIS:Hyx, HPD3:TRIS:Hyx, HAC3:TRIS:Hyx, HAC3:PEO:Hyx, HAC3:PEO:Aur, HAC3:TRIS:Aur, HPD3:PEO:Aur, HPD3:TRIS:Aur and TRIS:Aur.

Embodiment 204. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202 and 203, preferably the compound of any one of Embodiments 202 and 203, more preferably the compound of Embodiment 203, wherein the bio-distribution modifier comprised by the N-terminal modification group is selected from the group comprising HPD2:Ahx, HPD3:PEO:Hyx, PEO:Aur, PEO:Hyx, PEO2:Ahx, TRIS:Hyx, HPD3:TRIS:Hyx, HAC3:TRIS:Hyx, HAC3:PEO:Hyx, HAC3:PEO:Aur, HAC3:TRIS:Aur, HPD3:PEO:Aur, HPD3:TRIS:Aur and TRIS:Aur.

Embodiment 205. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203 and 204, wherein the bio-distribution modifier comprised by the C-terminal modification group C-term
is selected from the group comprising lys:HPD2, prx:PEO:HPD3, kur:PEO, prx:PEO, lys:PEO2, prx:TRIS, prx:TRIS:HPD3, prx:TRIS:HAC3, prx:PEO:HAC3, kur:PEO:HAC3, kur:TRIS:HAC3, kur:PEO:HPD3, kur:TRIS:HPD3, kur:TRIS, kur:HPD:HAC2, prx:HPD:HAC2, lys:HPD2:HAC4, kur:HPD:HPD2, prx:HPD:HPD2, lys:HPD2:HPD4, kur:HAC:HAC2, kur:HAC:HPD2, lys:HAC:HAC2, tzk:HAC:HAC2, tzk:HPD:HAC2, kur:HPD, prx:HPD, lys:HAC:HPD2, tzk:HPD:HPD2, kur:HAC, prx:PEO:HAC2, prx:TRIS:HPD2, prx:TRIS:HAC2, kur:PEO:HAC2, kur:TRIS:HAC2, kur:PEO:HPD2, kur:TRIS:HPD2, tzk:HAC:HPD2, prx:PEO:HPD2, prx:HAC:HAC2, prx:HAC:HPD2, prx:PEO:HPD, prx:PEO:HAC, prx:TRIS:HPD, prx:TRIS:HAC, kur:PEO:HAC, kur:TRIS:HAC, kur:PEO:HPD, kur:TRIS:HPD, lys:HAC and prx:HAC,
preferably is selected from the group comprising lys:HPD2, prx:PEO:HPD3, kur:PEO, prx:PEO, lys:PEO2, prx:TRIS, prx:TRIS:HPD3, prx:TRIS:HAC3, prx:PEO:HAC3, kur:PEO:HAC3, kur:TRIS:HAC3, kur:PEO:HPD3, kur:TRIS:HPD3, kur:TRIS, kur:HPD:HAC2, prx:HPD:HAC2, lys:HPD2:HAC4, kur:HPD:HPD2, prx:HPD:HPD2, lys:HPD2:HPD4, kur:HAC:HAC2, kur:HAC:HPD2, lys:HAC:HAC2, tzk:HAC:HAC2, tzk:HPD:HAC2, kur:HPD, prx:HPD, lys:HAC:HPD2, tzk:HPD:HPD2, kur:HAC, prx:PEO:HAC2, prx:TRIS:HPD2, prx:TRIS:HAC2, kur:PEO:HAC2, kur:TRIS:HAC2, kur:PEO:HPD2, kur:TRIS:HPD2 and tzk:HAC:HPD2,
more preferably is selected from the group comprising lys:HPD2, prx:PEO:HPD3, kur:PEO, prx:PEO, lys:PEO2, prx:TRIS, prx:TRIS:HPD3, prx:TRIS:HAC3, prx:PEO:HAC3, kur:PEO:HAC3, kur:TRIS:HAC3, kur:PEO:HPD3, kur:TRIS:HPD3, kur:TRIS, kur:HPD:HAC2, prx:HPD:HAC2, lys:HPD2:HAC4, kur:HPD:HPD2, prx:HPD:HPD2, lys:HPD2:HPD4, kur:HAC:HAC2 and kur:HAC:HPD2,
and most preferably is selected from the group comprising lys:HPD2, prx:PEO:HPD3, kur:PEO, prx:PEO, lys:PEO2, prx:TRIS, prx:TRIS:HPD3, prx:TRIS:HAC3, prx:PEO:HAC3, kur:PEO:HAC3, kur:TRIS:HAC3, kur:PEO:HPD3, kur:TRIS:HPD3 and kur:TRIS.

Embodiment 206. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204 and 205, preferably the compound of Embodiment 205, wherein the bio-distribution modifier comprised by the C-terminal modification group C-term
is selected from the group comprising lys:HPD2, prx:PEO:HPD3, kur:PEO, prx:PEO, lys:PEO2, prx:TRIS, prx:TRIS:HPD3, prx:TRIS:HAC3, prx:PEO:HAC3, kur:PEO:HAC3, kur:TRIS:HAC3, kur:PEO:HPD3, kur:TRIS:HPD3, kur:TRIS, kur:HPD:HAC2, prx:HPD:HAC2, lys:HPD2:HAC4, kur:HPD:HPD2, prx:HPD:HPD2, lys:HPD2:HPD4, kur:HAC:HAC2 and kur:HAC:HPD2,
and preferably is selected from the group comprising lys:HPD2, prx:PEO:HPD3, kur:PEO, prx:PEO, lys:PEO2, prx:TRIS, prx:TRIS:HPD3, prx:TRIS:HAC3, prx:PEO:HAC3, kur:PEO:HAC3, kur:TRIS:HAC3, kur:PEO:HPD3, kur:TRIS:HPD3 and kur:TRIS.

Embodiment 207. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205 and 206, preferably the compound of any one of Embodiments 205 and 206, more preferably the compound of Embodiment 206, wherein the bio-distribution modifier comprised by the C-terminal modification group C-term is selected from the group comprising lys:HPD2, prx:PEO:HPD3, kur:PEO, prx:PEO, lys:PEO2, prx:TRIS, prx:TRIS:HPD3, prx:TRIS:HAC3, prx:PEO:HAC3, kur:PEO:HAC3, kur:TRIS:HAC3, kur:PEO:HPD3, kur:TRIS:HPD3 and kur:TRIS.

Embodiment 208. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 127, 133, 136, 139, 141, 151, 154, 156, 168, 174, 177, 180, 182, 192, 195 and 197, preferably the compound of any one of Embodiment 127, 133, 136, 139, 141, 151, 154, 156, 168, 174, 177, 180, 182, 192, 195 and 197,
wherein
if the bio-distribution modifier comprised by the N-terminal modification group A is HAC2:HPD:Aur, the bio-distribution modifier comprised by the C-terminal modification group C-term is kur:HPD:HAC2,
or if the bio-distribution modifier comprised by the N-terminal modification group A is HAC2:HPD:Hyx, the bio-distribution modifier comprised by the C-terminal modification group C-term is prx:HPD:HAC2,
or if the bio-distribution modifier comprised by the N-terminal modification group A is HAC4:HPD2:Ahx, the bio-distribution modifier comprised by the C-terminal modification group C-term is lys:HPD2:HAC4,
or if the bio-distribution modifier comprised by the N-terminal modification group A is HPD2:HPD:Aur, the bio-distribution modifier comprised by the C-terminal modification group C-term is kur:HPD:HPD2,
or if the bio-distribution modifier comprised by the N-terminal modification group A is HPD2:HPD:Hyx, the bio-distribution modifier comprised by the C-terminal modification group C-term is prx:HPD:HPD2,
or if the bio-distribution modifier comprised by the N-terminal modification group A is HPD4:HPD2:Ahx, the bio-distribution modifier comprised by the C-terminal modification group C-term is lys:HPD2:HPD4,
or if the bio-distribution modifier comprised by the N-terminal modification group A is HAC2:HAC:Aur, the bio-distribution modifier comprised by the C-terminal modification group C-term is kur:HAC:HAC2,
or if the bio-distribution modifier comprised by the N-terminal modification group A is HPD2:HAC:Aur, the bio-distribution modifier comprised by the C-terminal modification group C-term is kur:HAC:HPD2,
preferably
if the bio-distribution modifier comprised by the N-terminal modification group A is HPD2:Ahx, the bio-distribution modifier comprised by the C-terminal modification group C-term is lys:HPD2,
or if the bio-distribution modifier comprised by the N-terminal modification group A is HPD3:PEO:Hyx, the bio-distribution modifier comprised by the C-terminal modification group C-term is prx:PEO:HPD3,
or if the bio-distribution modifier comprised by the N-terminal modification group A is PEO:Aur, the bio-distribution modifier comprised by the C-terminal modification group C-term is kur:PEO,
or if the bio-distribution modifier comprised by the N-terminal modification group A is PEO:Hyx, the bio-distribution modifier comprised by the C-terminal modification group C-term is prx:PEO,
or if the bio-distribution modifier comprised by the N-terminal modification group A is PEO2:Ahx, the bio-distribution modifier comprised by the C-terminal modification group C-term is lys:PEO2,
or if the bio-distribution modifier comprised by the N-terminal modification group A is TRIS:Hyx, the bio-distribution modifier comprised by the C-terminal modification group C-term is prx:TRIS,
or if the bio-distribution modifier comprised by the N-terminal modification group A is HPD3:TRIS:Hyx, the bio-distribution modifier comprised by the C-terminal modification group C-term is prx:TRIS:HPD3,
or if the bio-distribution modifier comprised by the N-terminal modification group A is HAC3:TRIS:Hyx, the bio-distribution modifier comprised by the C-terminal modification group C-term is prx:TRIS:HAC3,
or if the bio-distribution modifier comprised by the N-terminal modification group A is HAC3:PEO:Hyx, the bio-distribution modifier comprised by the C-terminal modification group C-term is prx:PEO:HAC3,
or if the bio-distribution modifier comprised by the N-terminal modification group A is HPD2:PEO:Hyx, the bio-distribution modifier comprised by the C-terminal modification group C-term is prx:PEO:HPD2,
or if the bio-distribution modifier comprised by the N-terminal modification group A is HAC3:PEO:Aur, the bio-distribution modifier comprised by the C-terminal modification group C-term is kur:PEO:HAC3,
or if the bio-distribution modifier comprised by the N-terminal modification group A is HAC3:TRIS:Aur, the bio-distribution modifier comprised by the C-terminal modification group C-term is kur:TRIS:HAC3,
or if the bio-distribution modifier comprised by the N-terminal modification f group A is HPD3:PEO:Aur, the bio-distribution modifier comprised by the C-terminal modification group C-term is kur:PEO:HPD3,
or if the bio-distribution modifier comprised by the N-terminal modification f group A is HPD3TRIS: Aur, the bio-distribution modifier comprised by the C-terminal modification group C-term is kur:TRIS:HPD3,
or if the bio-distribution modifier comprised by the N-terminal modification f group A is TRIS:Aur, the bio-distribution modifier comprised by the C-terminal modification group C-term is kur:TRIS.

Embodiment 209. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 127, 136, 141, 151, 156, 168, 177, 182, 192 and 197, preferably the compound of any one of Embodiments 127, 136, 141, 151, 156, 168, 177, 182, 192 and 197, wherein
if the bio-distribution modifier comprised by the N-terminal modification group A is HPD2:Ahx, the bio-distribution modifier comprised by the C-terminal modification group C-term is lys:HPD2,
or if the bio-distribution modifier comprised by the N-terminal modification group A is HPD3:PEO:Hyx, the bio-distribution modifier comprised by the C-terminal modification group C-term is prx:PEO:HPD3,
or if the bio-distribution modifier comprised by the N-terminal modification group A is PEO:Aur, the bio-distribution modifier comprised by the C-terminal modification group C-term is kur:PEO,
or if the bio-distribution modifier comprised by the N-terminal modification group A is PEO:Hyx, the bio-distribution modifier comprised by the C-terminal modification group C-term is prx:PEO,
or if the bio-distribution modifier comprised by the N-terminal modification group A is PEO2:Ahx, the bio-distribution modifier comprised by the C-terminal modification group C-term is lys:PEO2,
or if the bio-distribution modifier comprised by the N-terminal modification group A is TRIS:Hyx, the bio-distribution modifier comprised by the C-terminal modification group C-term is prx:TRIS,
or if the bio-distribution modifier comprised by the N-terminal modification group A is HPD3:TRIS:Hyx, the bio-distribution modifier comprised by the C-terminal modification group C-term is prx:TRIS:HPD3,
or if the bio-distribution modifier comprised by the N-terminal modification group A is HAC3:TRIS:Hyx, the bio-distribution modifier comprised by the C-terminal modification group C-term is prx:TRIS:HAC3,
or if the bio-distribution modifier comprised by the N-terminal modification group A is HAC3:PEO:Hyx, the bio-distribution modifier comprised by the C-terminal modification group C-term is prx:PEO:HAC3,
or if the bio-distribution modifier comprised by the N-terminal modification group A is HPD2:PEO:Hyx, the bio-distribution modifier comprised by the C-terminal modification group C-term is prx:PEO:HPD2,
or if the bio-distribution modifier comprised by the N-terminal modification group A is HAC3:PEO:Aur, the bio-distribution modifier comprised by the C-terminal modification group C-term is kur:PEO:HAC3,
or if the bio-distribution modifier comprised by the N-terminal modification group A is HAC3:TRIS:Aur, the bio-distribution modifier comprised by the C-terminal modification group C-term is kur:TRIS:HAC3,
or if the bio-distribution modifier comprised by the N-terminal modification group A is HPD3:PEO:Aur, the bio-distribution modifier comprised by the C-terminal modification group C-term is kur:PEO:HPD3,
or if the bio-distribution modifier comprised by the N-terminal modification group A is HPD3TRIS: Aur, the bio-distribution modifier comprised by the C-terminal modification group C-term is kur:TRIS:HPD3,
or if the bio-distribution modifier comprised by the N-terminal modification group A is TRIS:Aur, the bio-distribution modifier comprised by the C-terminal modification group C-term is kur:TRIS.

Embodiment 210. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208 and 209, wherein the compound comprises three or more Z groups.

Embodiment 211. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, preferably the compound of Embodiment 210, wherein
the first of the three or more Z groups is the Z group comprised by the N-terminal modification group A, wherein the Z group comprises the bio-distribution modifier optionally comprising a linker,
and the second of the three or more Z groups is the Z group comprised by the C-terminal modification group C-term, wherein the Z group comprises the bio-distribution modifier optionally comprising a linker,
and the third of the three or more Z groups
   a) is covalently attached to or is part of substituent R^{4d}, under the proviso that Xaa4 is a structure of formula (Va), (Vd) or (Ve),
   b) is covalently attached to or is part of substituent R^{4h}, under the proviso that Xaa4 is a structure of formula (Vb) or (Vf),
   c) is covalently attached to or is part of substituent R^{5d}, under the proviso that Xaa5 is a structure of formula (VIa),
   d) is covalently attached to or is part of substituent R^{8a}, under the proviso that Xaa8 is a structure of formula (IXa) or (IXd),
   e) is covalently attached to or is part of substituent R^{8e}, under the proviso that Xaa8 is a structure of formula (IXc),
      or
   f) is covalently attached to or is part of substituent R^{Yd}, under the proviso that Yc is a structure of formula (XIIIh), comprising a residue R^{Yc}, wherein to R^{Yc} a residue R^{Yd} is covalently attached, wherein R^{Yd} is a structure of formula (XIIIc), (XIIId) or (XIIIe).

Embodiment 212. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210 and 211, preferably the compound of any one of Embodiments 210 and 211, more preferably the compound of Embodiment 211, wherein the third of the three or more Z groups is
a) is covalently attached to or is part of substituent R^{4d}, under the proviso that Xaa4 is a structure of formula (Va), (Vd) or (Ve),
b) is covalently attached to or is part of substituent R^{4h}, under the proviso that Xaa4 is a structure of formula (Vb) or (Vf),
c) is covalently attached to or is part of substituent R^{8a}, under the proviso that Xaa8 is a structure of formula (IXa) or (IXd),
   or
d) is covalently attached to or is part of substituent R^{8e}, under the proviso that Xaa8 is a structure of formula (IXc).

Embodiment 213. The compound of any one of Embodiment 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208 and 209, preferably the compound of Embodiments 208 and 209, more preferably the compound of Embodiment 209, wherein the compound comprises three Z groups.

Embodiment 214. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209 and 213, preferably the compound of any one of Embodiments 208, 209 and 213, more preferably the compound of any one of Embodiments 209 and 213, most preferably the compound of Embodiment 213, wherein
the first of the three Z groups is the Z group comprised by the N-terminal modification group A, wherein the Z group comprises the bio-distribution modifier optionally comprising a linker,
and the second of the three Z groups is the Z group comprised by the C-terminal modification group C-term, wherein the Z group comprises the bio-distribution modifier optionally comprising a linker,
and the third of the three Z groups is a chelator optionally comprising a linker,
preferably each and any linker comprises an amino group and a carbonyl group.

Embodiment 215. The compound of any one of Embodiments , 213 and 214, preferably the compound of any one of Embodiments 208, 209, 213 and 214, more preferably the compound of any one of Embodiments 209, 213 and 214, most preferably the compound of Embodiment 214, wherein the chelator is covalently attached to or is part of substituent R^{8a}, under the proviso that Xaa8 is a structure of formula (IXa) or (IXd).

Embodiment 216. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209 and 213 to 215, preferably the compound of any one of Embodiments 208, 209 and 213 to 215, more preferably the compound of any one of Embodiments 209 and 213 to 215, most preferably the compound of Embodiment 215, wherein Xaa8 is Apc(R^{8a}), wherein R^{8a} is a chelator
a) preferably selected from the group consisting of DOTA, DOTAGA, DOTAM, DOTP, NOTA, NODAGA, PSC, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
b) more preferably selected from the group consisting of DOTA, DOTAGA, DOTAM, NOTA, NODAGA, PSC, NODA-MPAA, NOPO, HBED, DTPA, CHX-A"-DTPA, CB-TE2A, Macropa, PCTA, N4, and analogs thereof,
   and
c) most preferably selected from the group consisting of DOTA, DOTAGA, DOTAM, NOTA, NODAGA, PSC, NOPO, Macropa, PCTA and analogs thereof.

Embodiment 217. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209 and 213 to 216, preferably the compound of any one of Embodiments 208, 209 and 213 to 216, more preferably the compound of any one of Embodiments 209 and 213 to 216, most preferably the compound of Embodiment 216, wherein Xaa8 is Apc(R^{8a}), wherein R^{8a} is a chelator, wherein the chelator is DOTA.

Embodiment 218. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 213 and 214, preferably the compound of any one of Embodiments 208, 209, 213 and 214, more preferably the compound of any one of Embodiments 209,213 and 214, most preferably the compound of Embodiment 214, wherein the chelator is covalently attached to or is part of substituent R^{8e}, under the proviso that Xaa8 is a structure of formula (IXc).

Embodiment 219. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 213, 214 and 218, preferably the compound of any one of Embodiments 208, 209, 213, 214 and 218, more preferably the compound of any one of Embodiments 209, 213, 214 and 218, most preferably the compound of Embodiment 218, wherein Xaa8 is Amk(R^{8e}), wherein R^{8e} is a chelator
a) preferably selected from the group consisting of DOTA, DOTAGA, DOTAM, DOTP, NOTA, NODAGA, PSC, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
b) more preferably selected from the group consisting of DOTA, DOTAGA, DOTAM, NOTA, NODAGA, PSC, NODA-MPAA, NOPO, HBED, DTPA, CHX-A"-DTPA, CB-TE2A, Macropa, PCTA, N4, and analogs thereof,
   and
c) most preferably selected from the group consisting of DOTA, DOTAGA, DOTAM, NOTA, NODAGA, PSC, NOPO, Macropa, PCTA and analogs thereof.

Embodiment 220. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 213, 214, 218 and 219, preferably the compound of any one of Embodiments 208, 209, 213, 214, 218 and 219, more preferably the compound of any one of Embodiments 209, 213, 214, 218 and 219, most preferably the compound of Embodiment 219, wherein Xaa8 is Amk(R^{8e}), wherein R^{8e} is a chelator, wherein the chelator is DOTA.

Embodiment 221. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219 and 220,
wherein the compound is selected from the group consisting of compound HPD2:Ahx-Asp-Trp-5Clw-Tap-Glu-leu-[Cys(2Lut)-Apc(DOTA)-Leu-Ile-Cys]-APAc-lys:HPD2 (GIP-1001) of the following formula 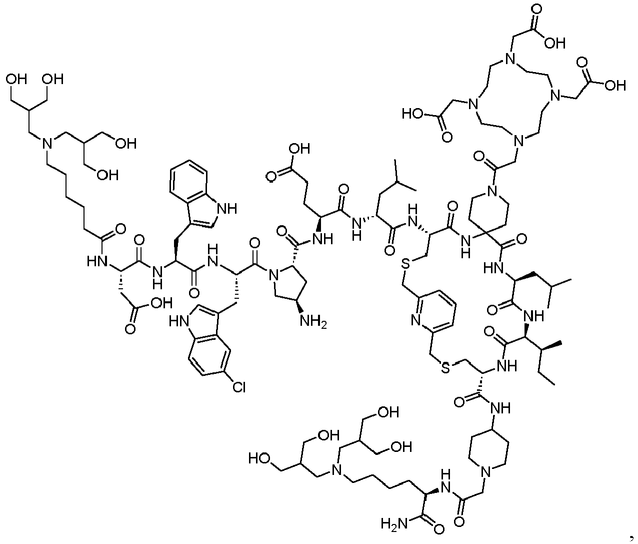
compound HPD3:PEO:Hyx-Asp-Trp-5Clw-Tap-Glu-leu-[Cys(2Lut)-Apc(DOTA)-Leu-Ile-Cys]-APAc-prx:PEO:HPD3 (GIP-1002) of the following formula 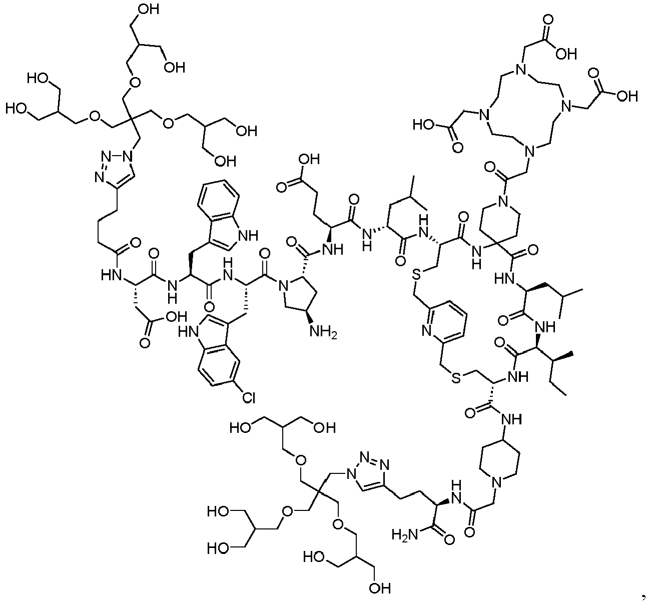
compound PEO:Aur-Asp-Trp-5Clw-Tap-Glu-leu-[Cys(2Lut)-Apc(DOTA)-Leu-Ile-Cys]-APAc-kur:PEO (GIP-1003) of the following formula 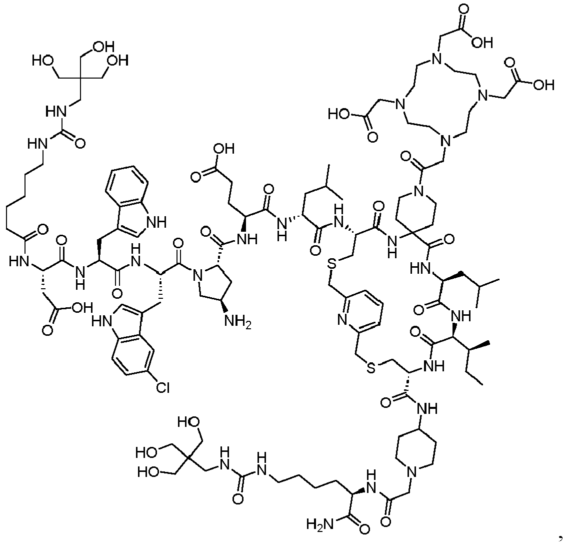
compound PEO:Hyx-Asp-Trp-5Clw-Tap-Glu-leu-[Cys(2Lut)-Apc(DOTA)-Leu-Ile-Cys]-APAc-prx:PEO (GIP-1004) of the following formula 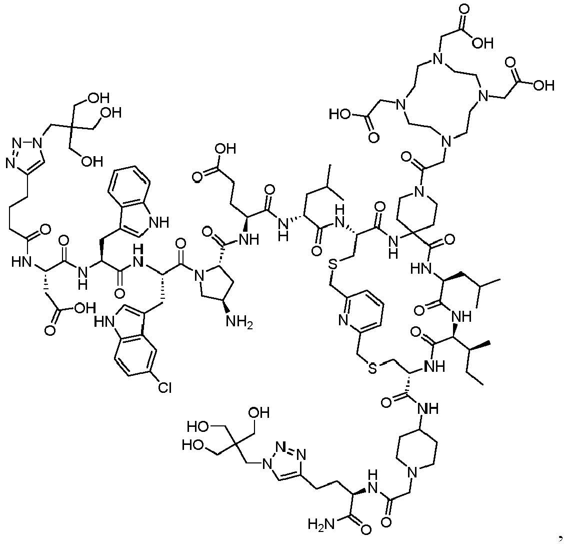
compound PEO2:Ahx-Asp-Trp-5Clw-Tap-Glu-leu-[Cys(2Lut)-Apc(DOTA)-Leu-Ile-Cys]-APAc-lys:PEO2 (GIP-1005) of the following formula 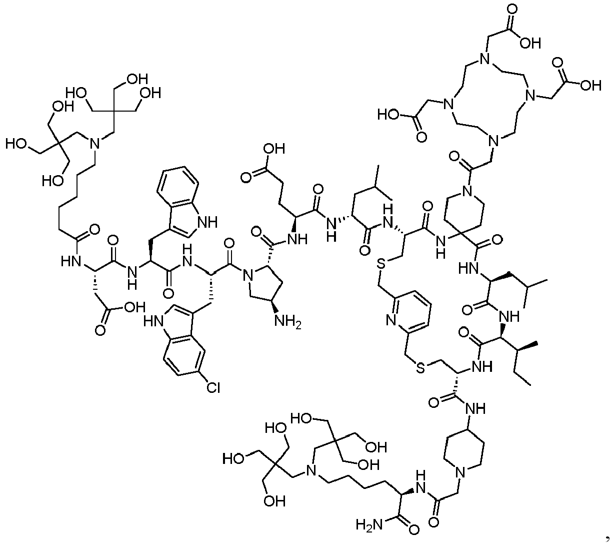
compound TRIS:Hyx-Asp-Trp-5Clw-Tap-Glu-leu-[Cys(2Lut)-Apc(DOTA)-Leu-Ile-Cys]-APAc-prx:TRIS (GIP-1006) of the following formula 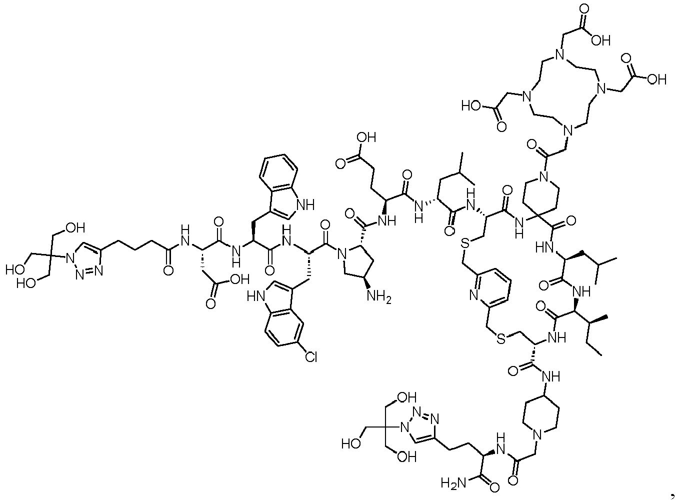
compound HPD3:TRIS:Hyx-Asp-Trp-5Clw-Tap-Glu-leu-[Cys(2Lut)-Apc(DOTA)-Leu-Ile-Cys]-APAc-prx:TRIS:HPD3 (GIP-1007) of the following formula 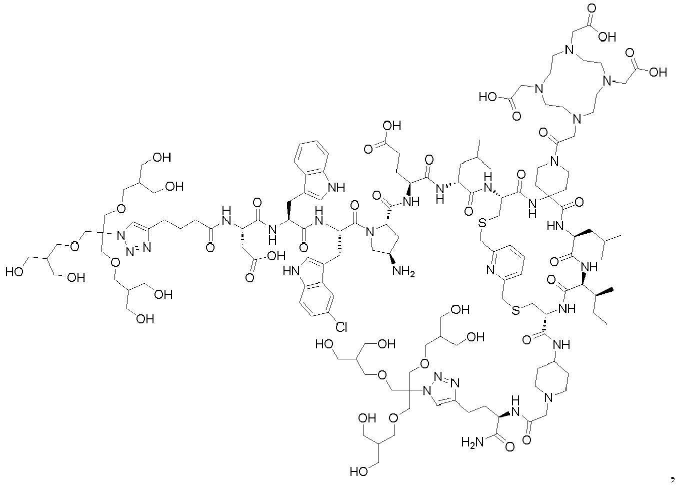
compound HAC3:TRIS:Hyx-Asp-Trp-5Clw-Tap-Glu-leu-[Cys(2Lut)-Apc(DOTA)-Leu-Ile-Cys]-APAc-prx:TRIS:HAC3 (GIP-1008) of the following formula 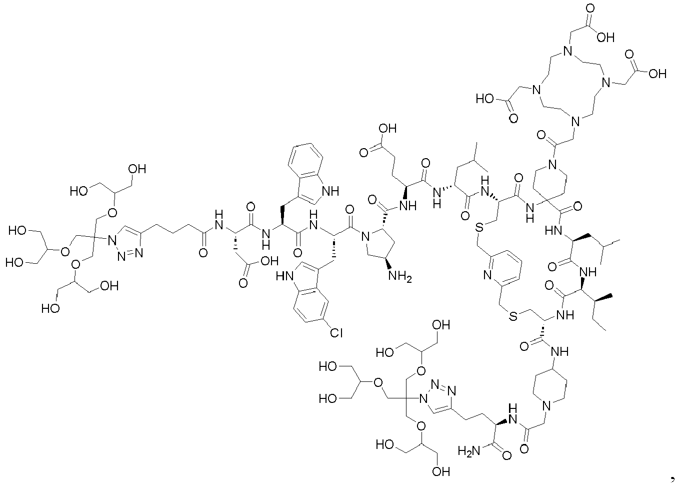
compound HAC3:PEO:Hyx-Asp-Trp-5Clw-Tap-Glu-leu-[Cys(2Lut)-Apc(DOTA)-Leu-Ile-Cys]-APAc-prx:PEO:HAC3 (GIP-1009) of the following formula 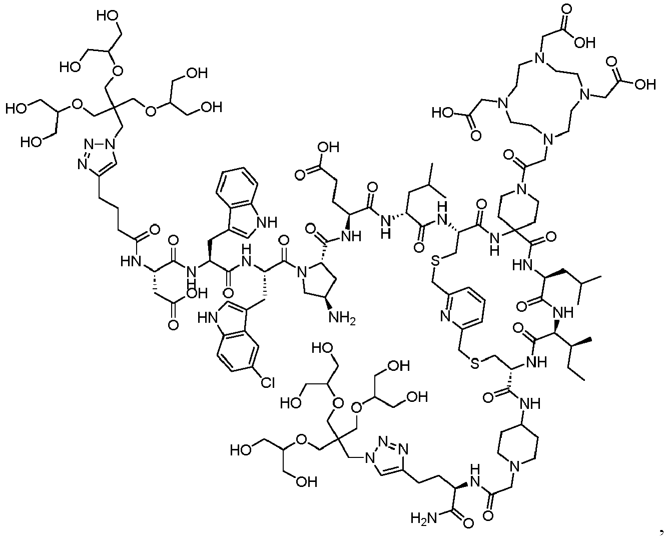
compound HAC3:PEO:Aur-Asp-Trp-5Clw-Tap-Glu-leu-[Cys(2Lut)-Apc(DOTA)-Leu-Ile-Cys]-APAc-kur:PEO:HAC3 (GIP-1010) of the following formula 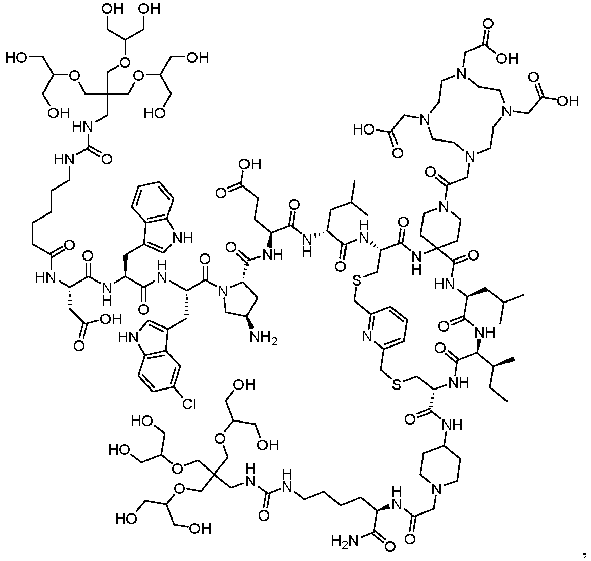
compound HAC3:TRIS:Aur-Asp-Trp-SClw-Tap-Glu-leu-[Cys(2Lut)-Apc(DOTA)-Leu-Ile-Cys]-APAc-kur:TRIS:HAC3 (GIP-1011) of the following formula 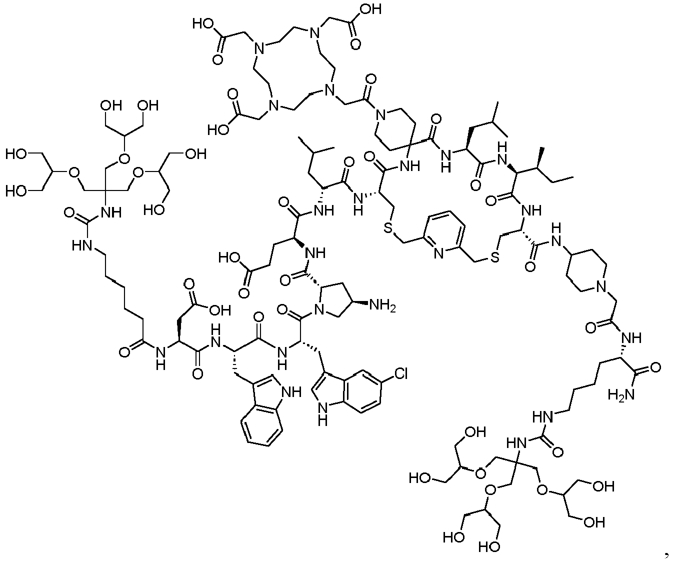
compound HPD3:PEO:Aur-Asp-Trp-5Clw-Tap-Glu-leu-[Cys(2Lut)-Apc(DOTA)-Leu-Ile-Cys]-APAc-kur:PEO:HPD3 (GIP-1012) of the following formula 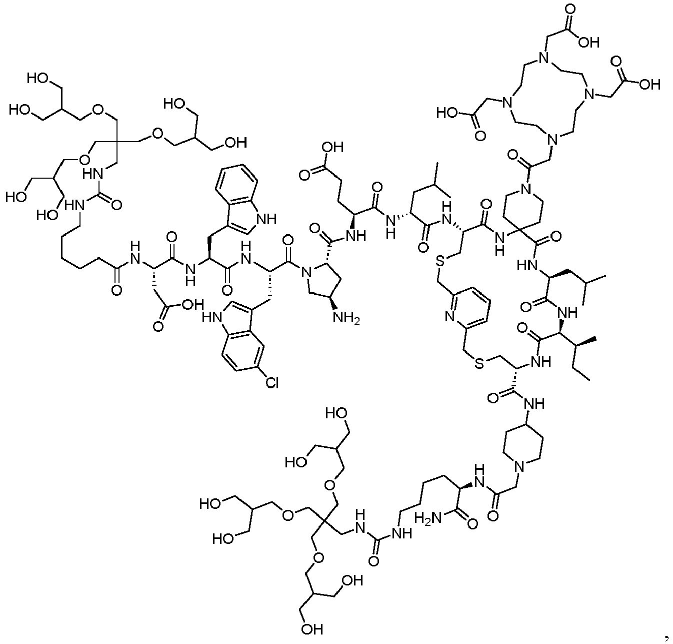
compound HPD3:TRIS:Aur-Asp-Trp-5Clw-Tap-Glu-leu-[Cys(2Lut)-Apc(DOTA)-Leu-Ile-Cys]-APAc-kur:TRIS:HPD3 (GIP-1013) of the following formula 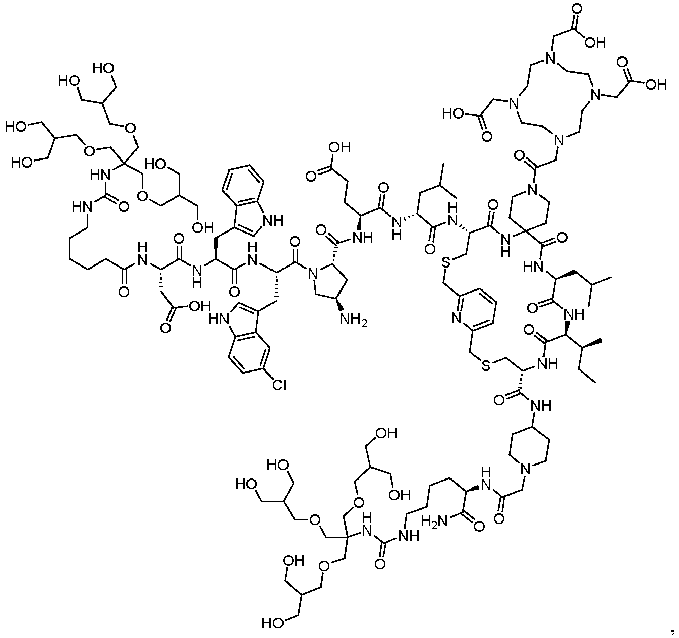
compound TRIS:Aur-Asp-Trp-5Clw-Tap-Glu-leu-[Cys(2Lut)-Apc(DOTA)-Leu-Ile-Cys]-APAc-kur:TRIS (GIP-1014) of the following formula 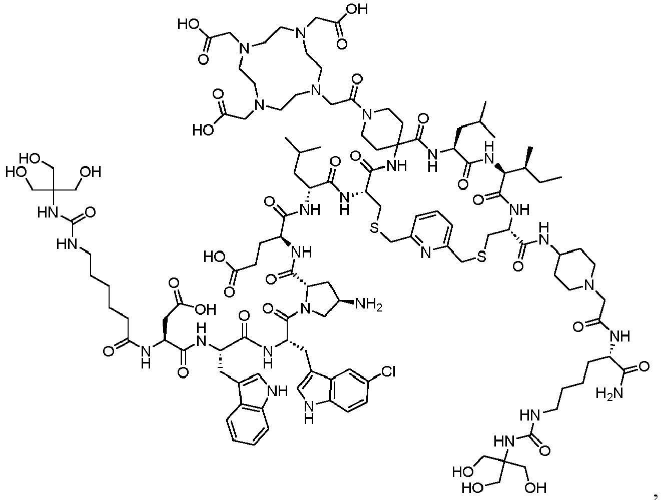
compound HPD2:Ahx-Asp-Trp-5Clw-Tap-Glu-leu-[Cys(2Lut)-Amk(DOTA)-Leu-Ile-Cys]-APAc-lys:HPD2 (GIP-1015) of the following formula 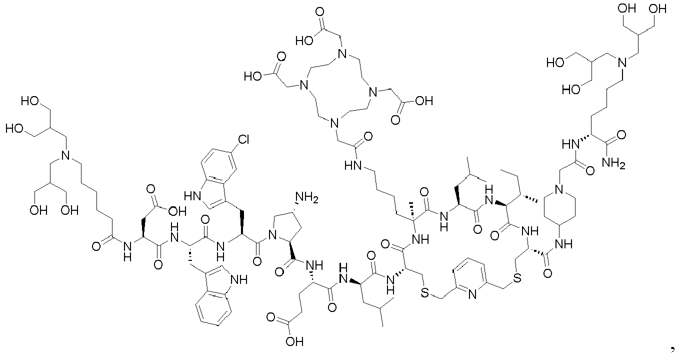
compound HPD3:PEO:Hyx-Asp-Trp-5Clw-Tap-Glu-leu-[Cys(2Lut)-Amk(DOTA)-Leu-Ile-Cys]-APAc-prx:PEO:HPD3 (GIP-1016) of the following formula 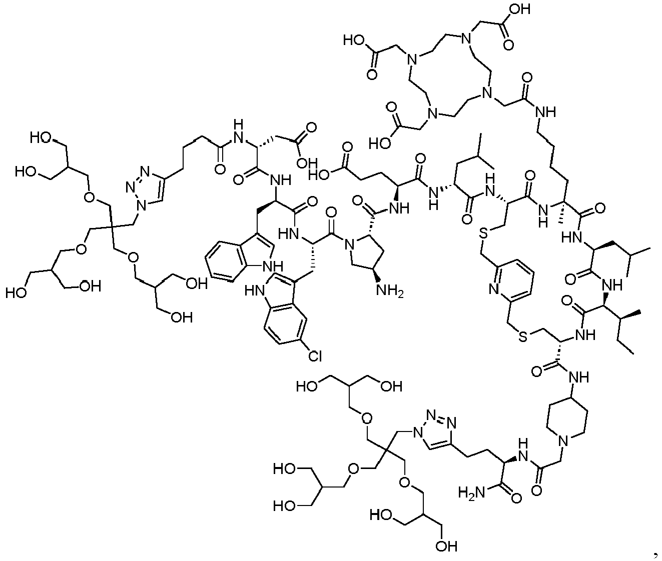
compound PEO:Aur-Asp-Trp-5Clw-Tap-Glu-leu-[Cys(2Lut)-Amk(DOTA)-Leu-Ile-Cys]-APAc-kur:PEO (GIP-1017) of the following formula 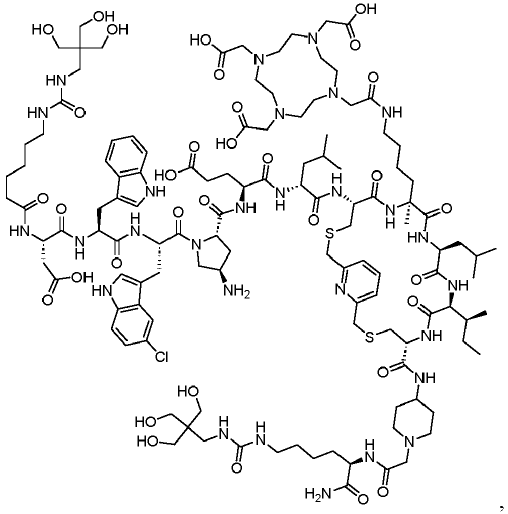
compound PEO:Hyx-Asp-Trp-5Clw-Tap-Glu-leu-[Cys(2Lut)-Amk(DOTA)-Leu-Ile-Cys]-APAc-prx:PEO (GIP-1018) of the following formula 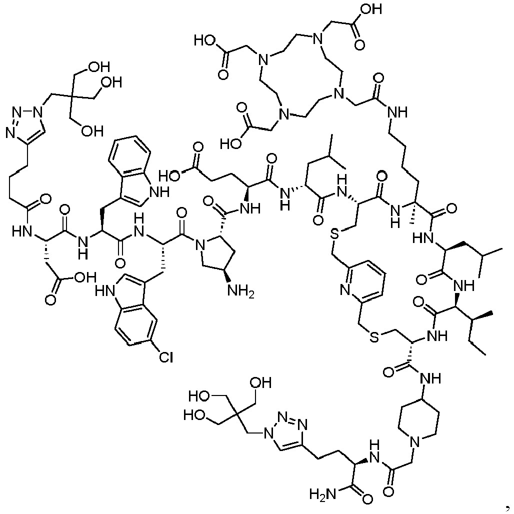
compound PEO2:Ahx-Asp-Trp-5Clw-Tap-Glu-leu-[Cys(2Lut)-Amk(DOTA)-Leu-Ile-Cys]-APAc-lys:PEO2 (GIP-1019) of the following formula 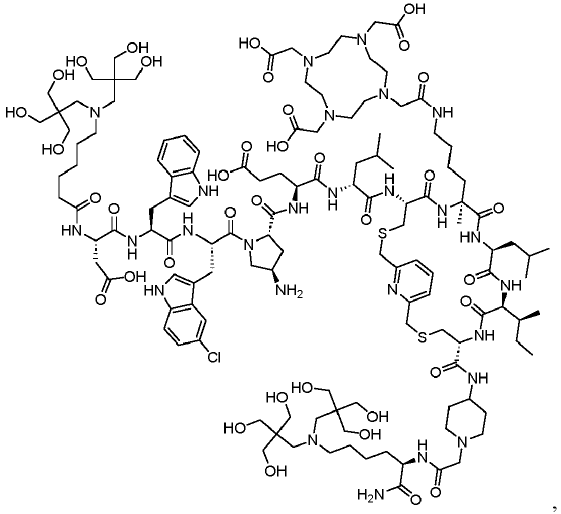
compound TRIS:Hyx-Asp-Trp-5Clw-Tap-Glu-leu-[Cys(2Lut)-Amk(DOTA)-Leu-Ile-Cys]-APAc-prx:TRIS (GIP-1020) of the following formula 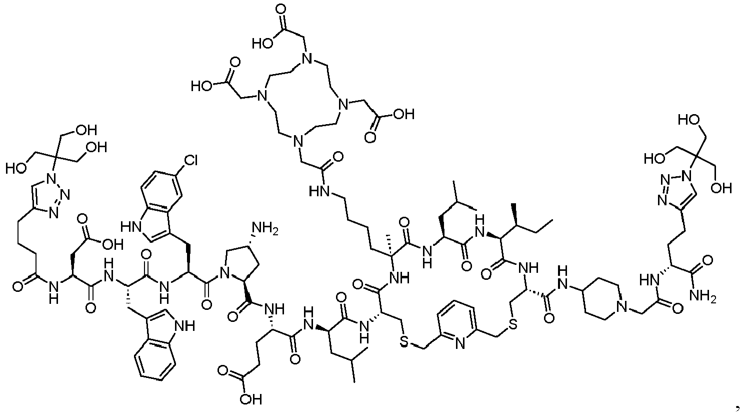
compound HPD3:TRIS:Hyx-Asp-Trp-5Clw-Tap-Glu-leu-[Cys(2Lut)-Amk(DOTA)-Leu-Ile-Cys]-APAc-prx:TRIS:HPD3 (GIP-1021) of the following formula 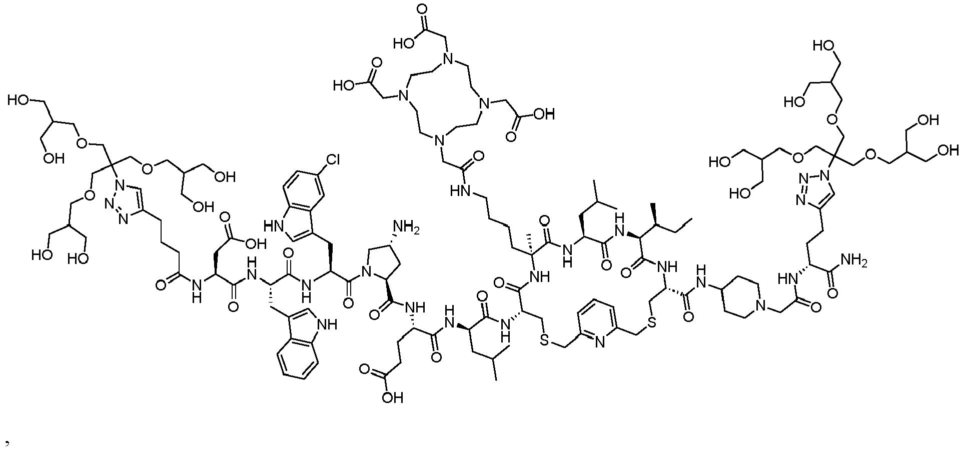
compound HAC3:TRIS:Hyx-Asp-Trp-5Clw-Tap-Glu-leu-[Cys(2Lut)-Amk(DOTA)-Leu-Ile-Cys]-APAc-prx:TRIS:HAC3 (GIP-1022) of the following formula 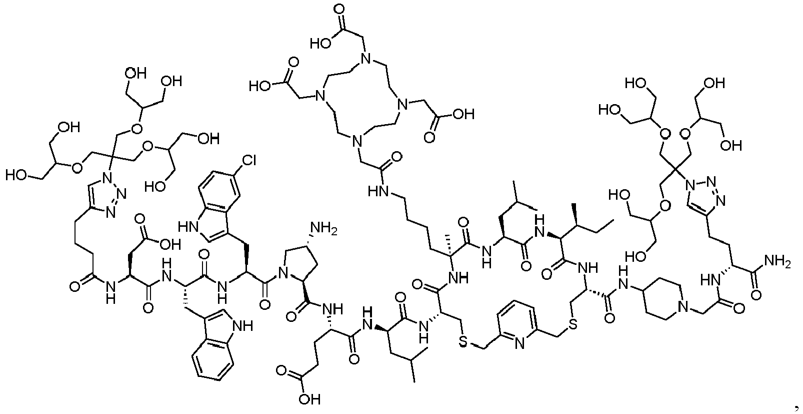
compound HAC3:PEO:Hyx-Asp-Trp-5Clw-Tap-Glu-leu-[Cys(2Lut)-Amk(DOTA)-Leu-Ile-Cys]-APAc-prx:PEO:HAC3 (GIP-1023) of the following formula 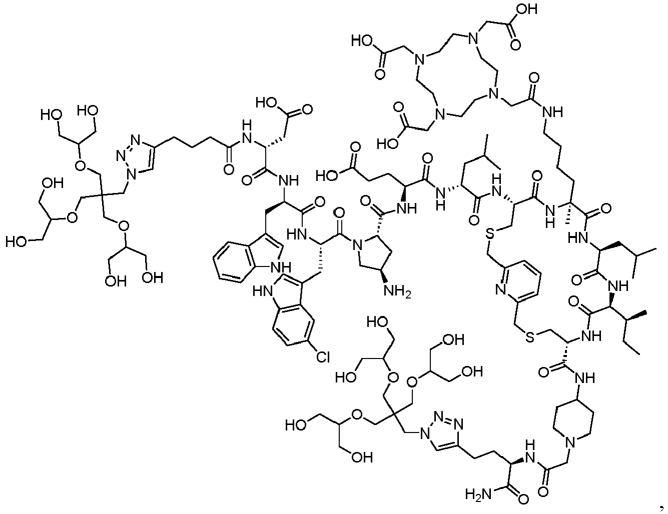
compound HAC3:PEO:Aur-Asp-Trp-5Clw-Tap-Glu-leu-[Cys(2Lut)-Amk(DOTA)-Leu-Ile-Cys]-APAc-kur:PEO:HAC3 (GIP-1024) of the following formula 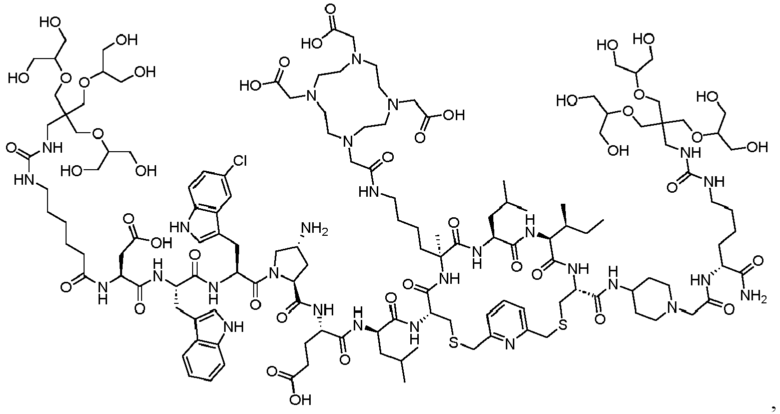
compound HAC3:TRIS:Aur-Asp-Trp-SClw-Tap-Glu-leu-[Cys(2Lut)-Amk(DOTA)-Leu-Ile-Cys]-APAc-kur:TRIS:HAC3 (GIP-1025) of the following formula 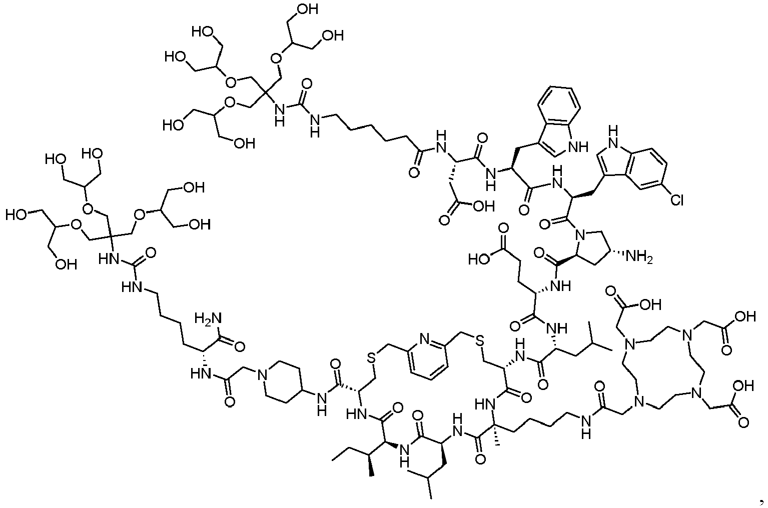
compound HPD3:PEO:Aur-Asp-Trp-5Clw-Tap-Glu-leu-[Cys(2Lut)-Amk(DOTA)-Leu-Ile-Cys]-APAc-kur:PEO:HPD3 (GIP-1026) of the following formula 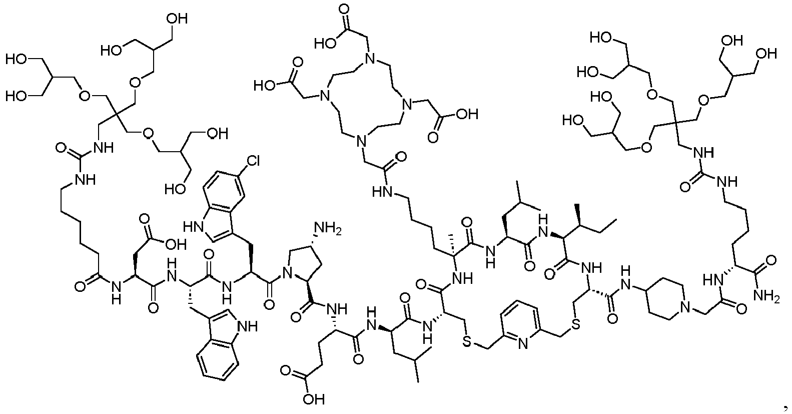
compound HPD3:TRIS:Aur-Asp-Trp-5Clw-Tap-Glu-leu-[Cys(2Lut)-Amk(DOTA)-Leu-Ile-Cys]-APAc-kur:TRIS:HPD3 (GIP-1027) of the following formula 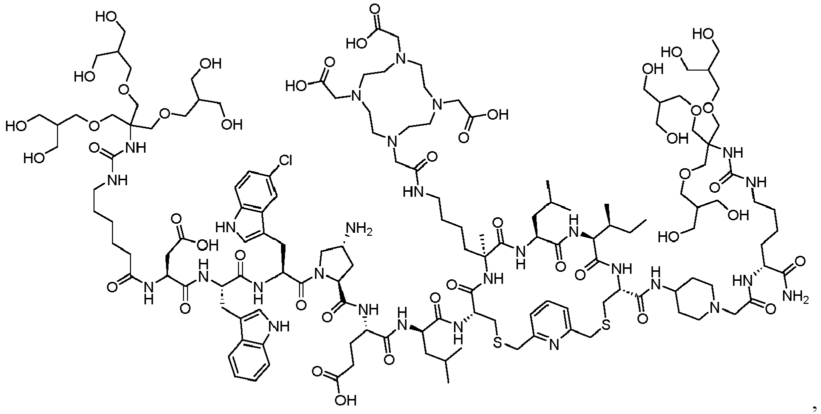
compound TRIS:Aur-Asp-Trp-5Clw-Tap-Glu-leu-[Cys(2Lut)-Amk(DOTA)-Leu-Ile-Cys]-APAc-kur:TRIS (GIP-1028) of the following formula 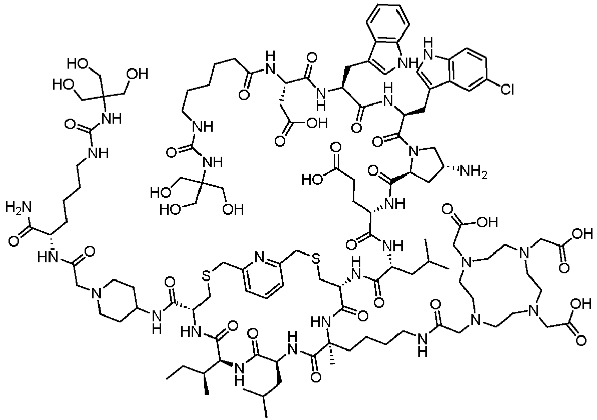
compound HPD2:Ahx-Asp-Trp-5Clw-Hyp-Glu-leu-[Cys(2Lut)-Apc(DOTA)-Leu-Ile-Cys]-APAc-lys:HPD2 (GIP-1029) of the following formula 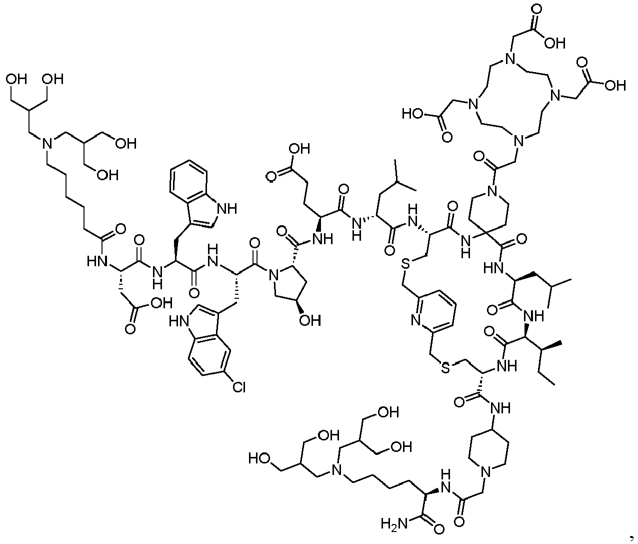
compound HPD3:PEO:Hyx-Asp-Trp-5Clw-Hyp-Glu-leu-[Cys(2Lut)-Apc(DOTA)-Leu-Ile-Cys]-APAc-prx:PEO:HPD3 (GIP-1030) of the following formula 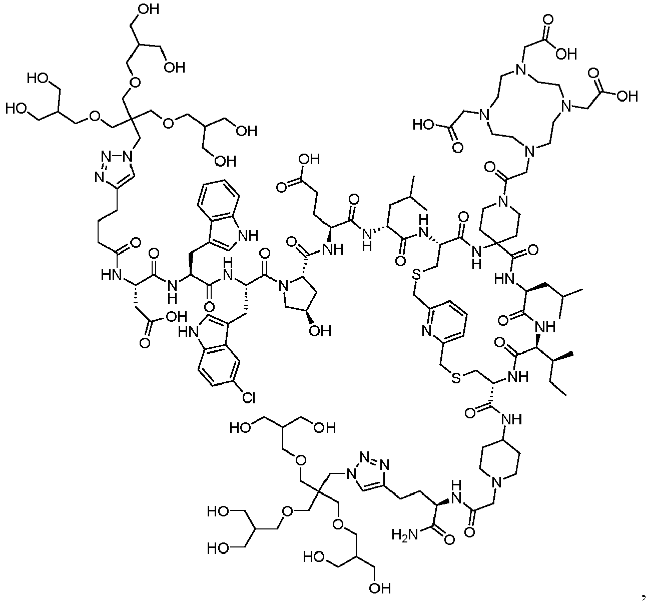
compound PEO:Aur-Asp-Trp-5Clw-Hyp-Glu-leu-[Cys(2Lut)-Apc(DOTA)-Leu-Ile-Cys]-APAc-kur:PEO (GIP-1031) of the following formula 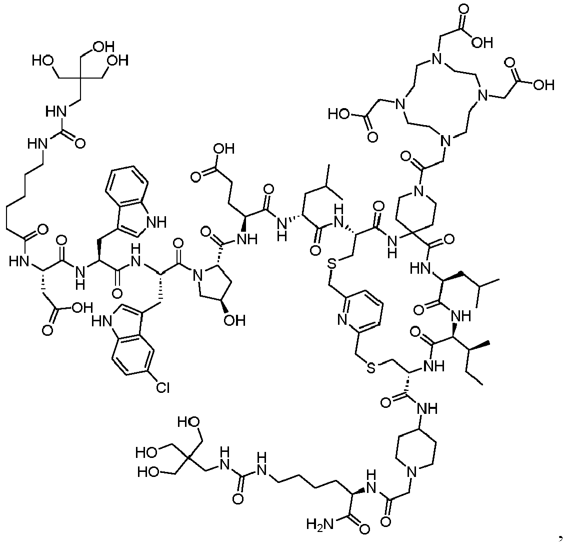
compound PEO:Hyx-Asp-Trp-5Clw-Hyp-Glu-leu-[Cys(2Lut)-Apc(DOTA)-Leu-Ile-Cys]-APAc-prx:PEO (GIP-1032) of the following formula 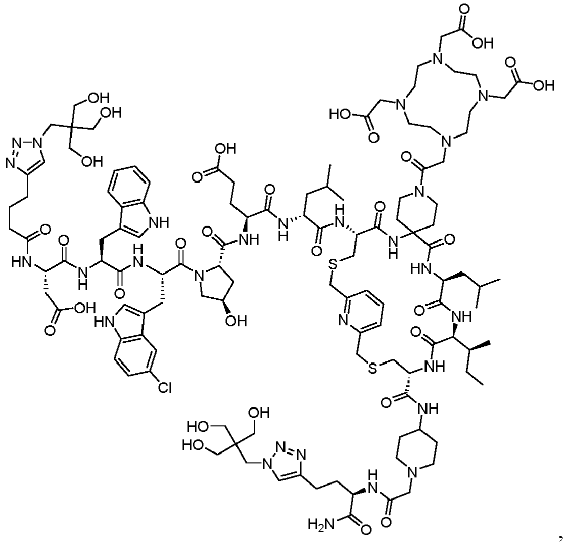
compound PEO2:Ahx-Asp-Trp-5Clw-Hyp-Glu-leu-[Cys(2Lut)-Apc(DOTA)-Leu-Ile-Cys]-APAc-lys:PEO2 (GIP-1033) of the following formula 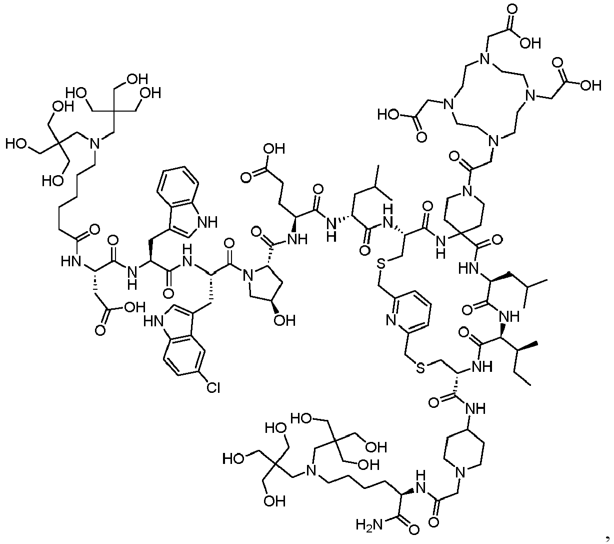
compound TRIS:Hyx-Asp-Trp-5Clw-Hyp-Glu-leu-[Cys(2Lut)-Apc(DOTA)-Leu-Ile-Cys]-APAc-prx:TRIS (GIP-1034) of the following formula 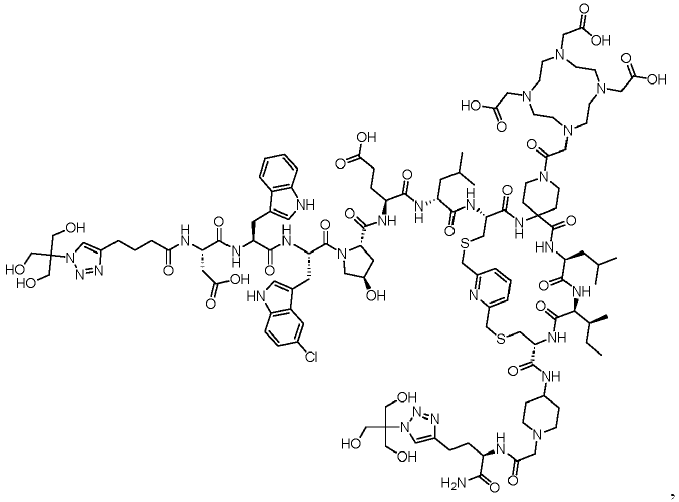
compound HPD3:TRIS:Hyx-Asp-Trp-5Clw-Hyp-Glu-leu-[Cys(2Lut)-Apc(DOTA)-Leu-Ile-Cys]-APAc-prx:TRIS:HPD3 (GIP-1035) of the following formula 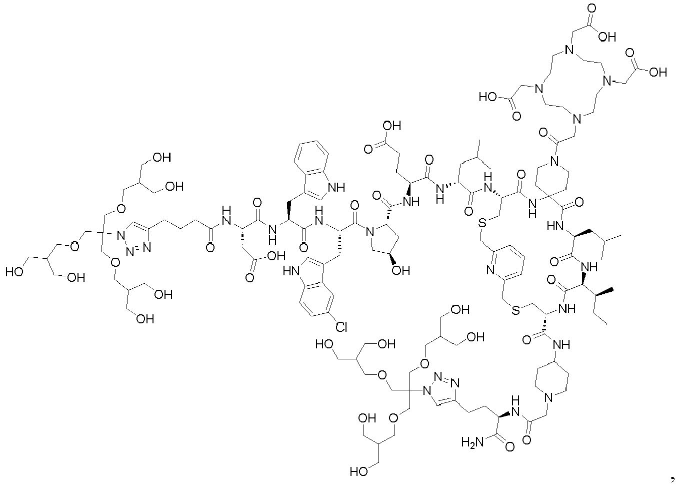
compound HAC3:TRIS:Hyx-Asp-Trp-5Clw-Hyp-Glu-leu-[Cys(2Lut)-Apc(DOTA)-Leu-Ile-Cys]-APAc-prx:TRIS:HAC3 (GIP-1036) of the following formula 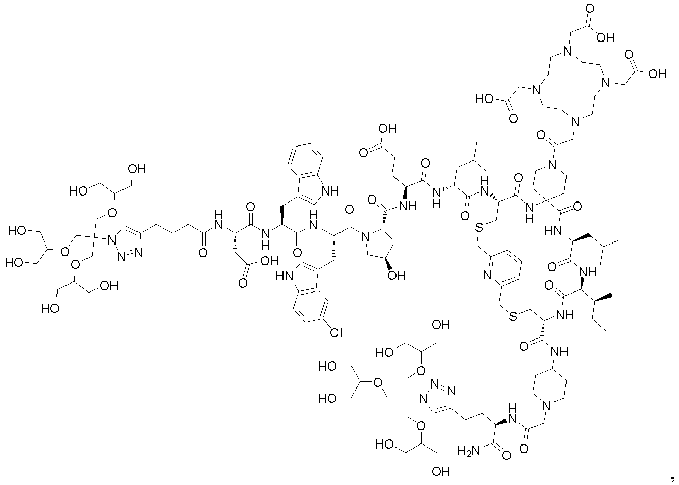
compound HAC3:PEO:Hyx-Asp-Trp-5Clw-Hyp-Glu-leu-[Cys(2Lut)-Apc(DOTA)-Leu-Ile-Cys]-APAc-prx:PEO:HAC3 (GIP-1037) of the following formula 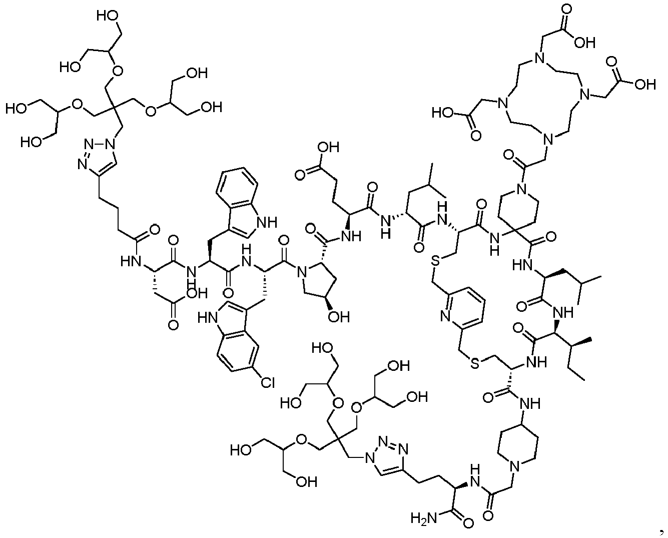
compound HAC3:PEO:Aur-Asp-Trp-5Clw-Hyp-Glu-leu-[Cys(2Lut)-Apc(DOTA)-Leu-Ile-Cys]-APAc-kur:PEO:HAC3 (GIP-1038) of the following formula 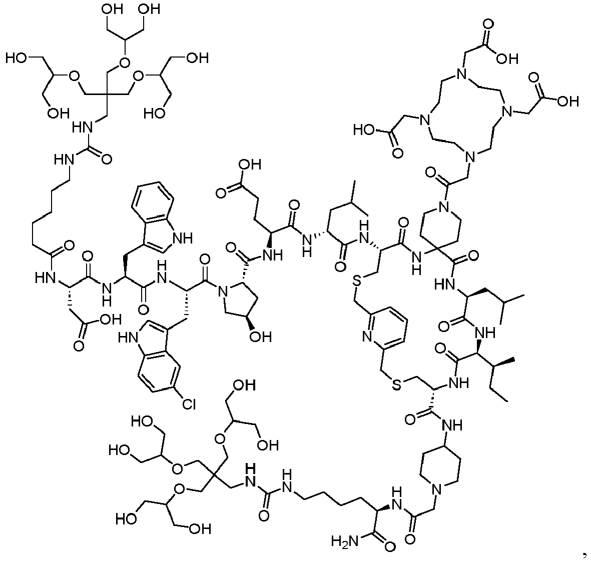
compound HAC3:TRIS:Aur-Asp-Trp-5Clw-Hyp-Glu-leu-[Cys(2Lut)-Apc(DOTA)-Leu-Ile-Cys]-APAc-kur:TRIS:HAC3 (GIP-1039) of the following formula 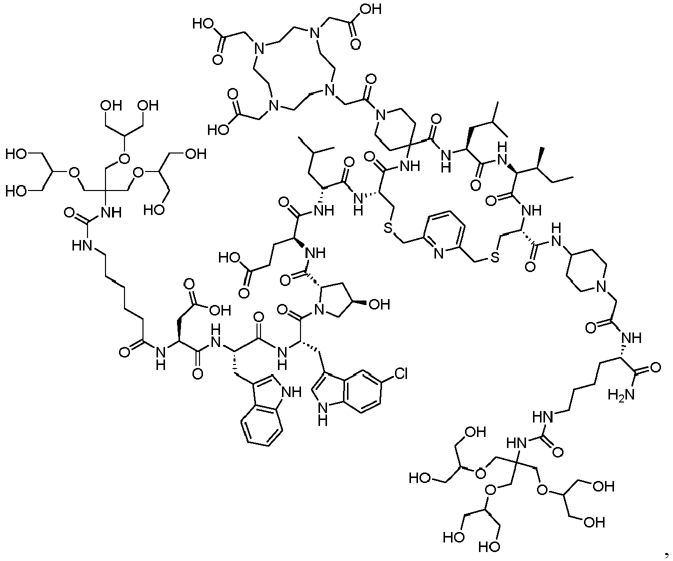
compound HPD3:PEO:Aur-Asp-Trp-5Clw-Hyp-Glu-leu-[Cys(2Lut)-Apc(DOTA)-Leu-Ile-Cys]-APAc-kur:PEO:HPD3 (GIP-1040) of the following formula 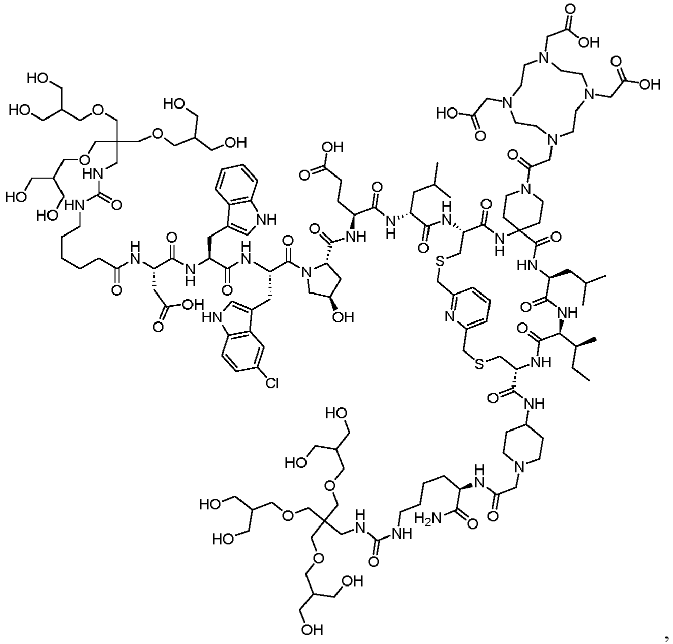
compound HPD3:TRIS:Aur-Asp-Trp-5Clw-Hyp-Glu-leu-[Cys(2Lut)-Apc(DOTA)-Leu-Ile-Cys]-APAc-kur:TRIS:HPD3 (GIP-1041) of the following formula 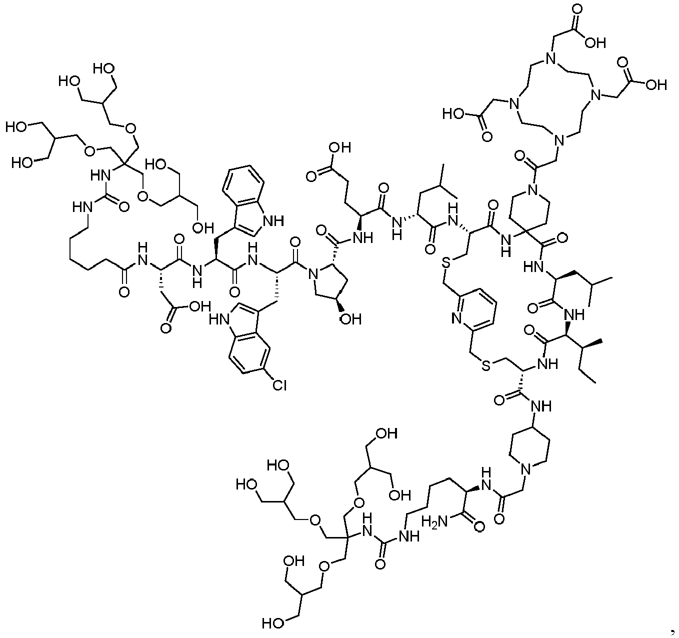
compound TRIS:Aur-Asp-Trp-5Clw-Hyp-Glu-leu-[Cys(2Lut)-Apc(DOTA)-Leu-Ile-Cys]-APAc-kur:TRIS (GIP-1042) of the following formula 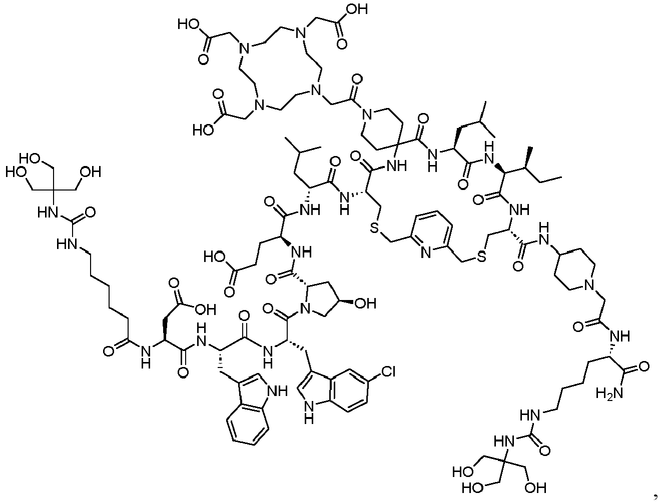
compound HPD2:Ahx-Asp-Trp-5Clw-Hyp-Glu-leu-[Cys(2Lut)-Amk(DOTA)-Leu-Ile-Cys]-APAc-lys:HPD2 (GIP-1043) of the following formula 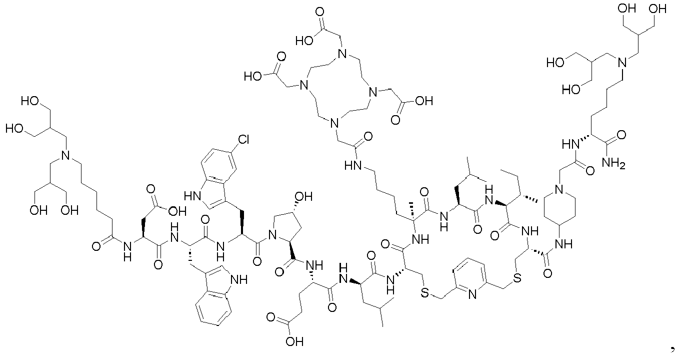
compound HPD3:PEO:Hyx-Asp-Trp-5Clw-Hyp-Glu-leu-[Cys(2Lut)-Amk(DOTA)-Leu-Ile-Cys]-APAc-prx:PEO:HPD3 (GIP-1044) of the following formula 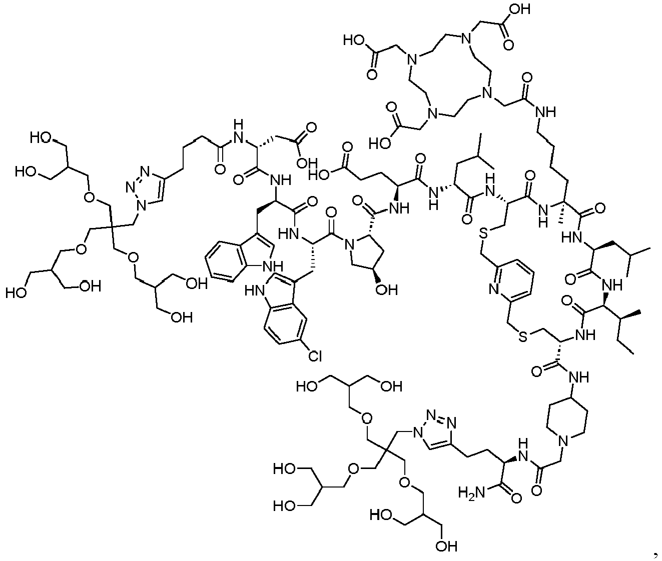
compound PEO:Aur-Asp-Trp-5Clw-Hyp-Glu-leu-[Cys(2Lut)-Amk(DOTA)-Leu-Ile-Cys]-APAc-kur:PEO (GIP-1045) of the following formula 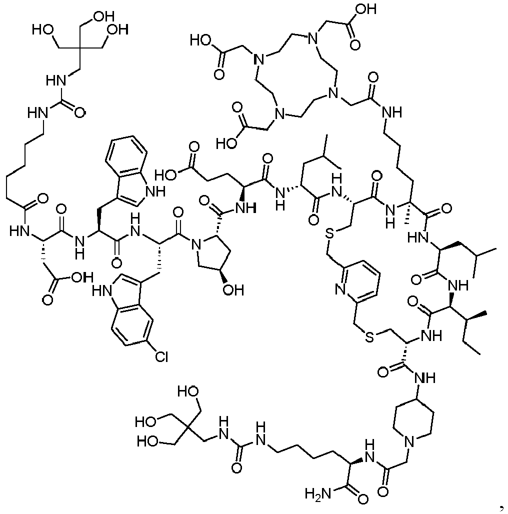
compound PEO:Hyx-Asp-Trp-5Clw-Hyp-Glu-leu-[Cys(2Lut)-Amk(DOTA)-Leu-Ile-Cys]-APAc-prx:PEO (GIP-1046) of the following formula 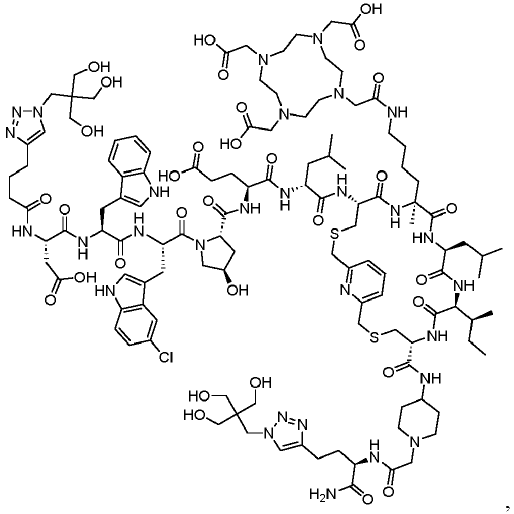
compound PEO2:Ahx-Asp-Trp-5Clw-Hyp-Glu-leu-[Cys(2Lut)-Amk(DOTA)-Leu-Ile-Cys]-APAc-lys:PEO2 (GIP-1047) of the following formula 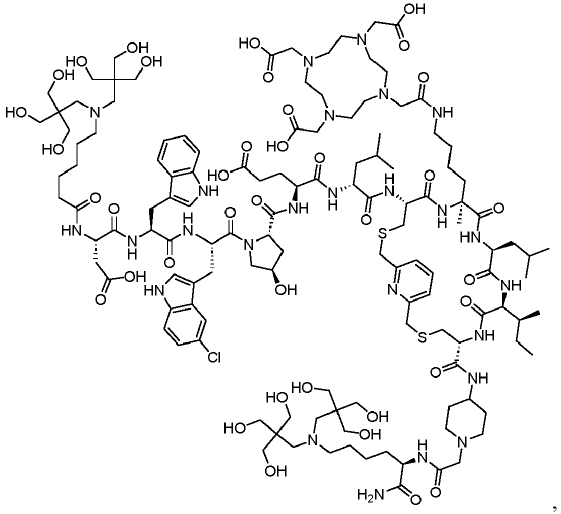
compound TRIS:Hyx-Asp-Trp-5Clw-Hyp-Glu-leu-[Cys(2Lut)-Amk(DOTA)-Leu-Ile-Cys]-APAc-prx:TRIS (GIP-1048) of the following formula 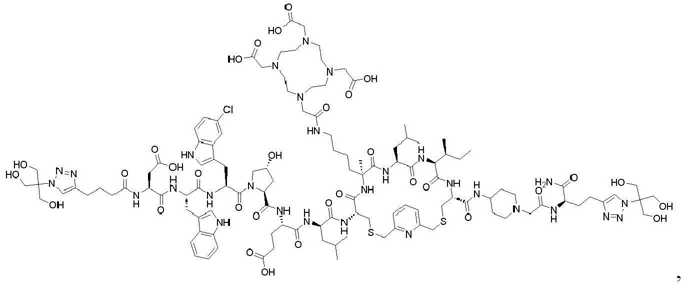
compound HPD3:TRIS:Hyx-Asp-Trp-5Clw-Hyp-Glu-leu-[Cys(2Lut)-Amk(DOTA)-Leu-Ile-Cys]-APAc-prx:TRIS:HPD3 (GIP-1049) of the following formula 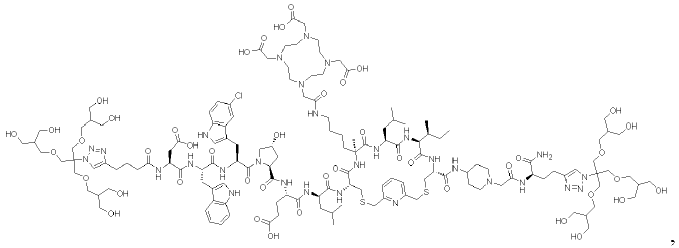
compound HAC3:TRIS:Hyx-Asp-Trp-5Clw-Hyp-Glu-leu-[Cys(2Lut)-Amk(DOTA)-Leu-Ile-Cys]-APAc-prx:TRIS:HAC3 (GIP-1050) of the following formula 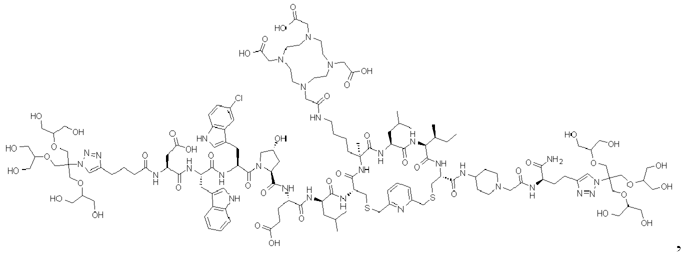
compound HAC3:PEO:Hyx-Asp-Trp-5Clw-Hyp-Glu-leu-[Cys(2Lut)-Amk(DOTA)-Leu-Ile-Cys]-APAc-prx:PEO:HAC3 (GIP-1051) of the following formula 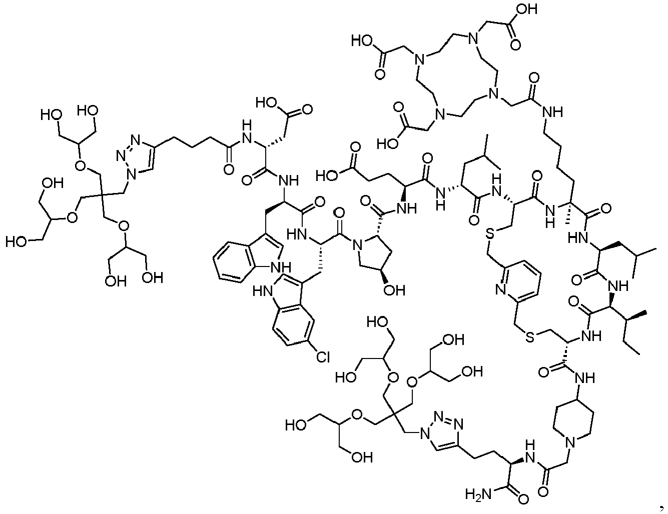
compound HAC3:PEO:Aur-Asp-Trp-5Clw-Hyp-Glu-leu-[Cys(2Lut)-Amk(DOTA)-Leu-Ile-Cys]-APAc-kur:PEO:HAC3 (GIP-1052) of the following formula 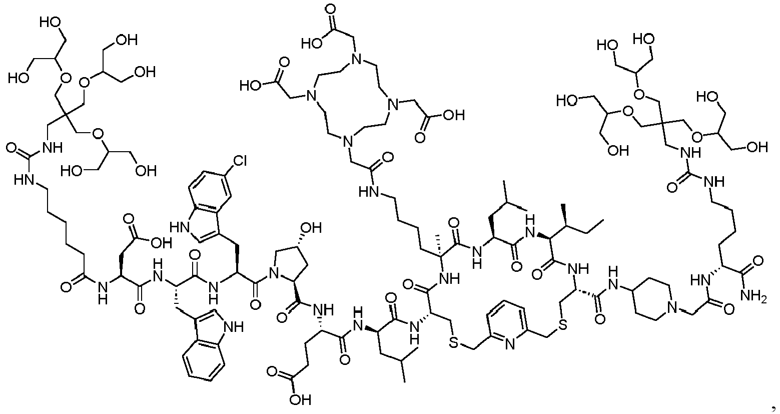
compound HAC3:TRIS:Aur-Asp-Trp-5Clw-Hyp-Glu-leu-[Cys(2Lut)-Amk(DOTA)-Leu-Ile-Cys]-APAc-kur:TRIS:HAC3 (GIP-1053) of the following formula 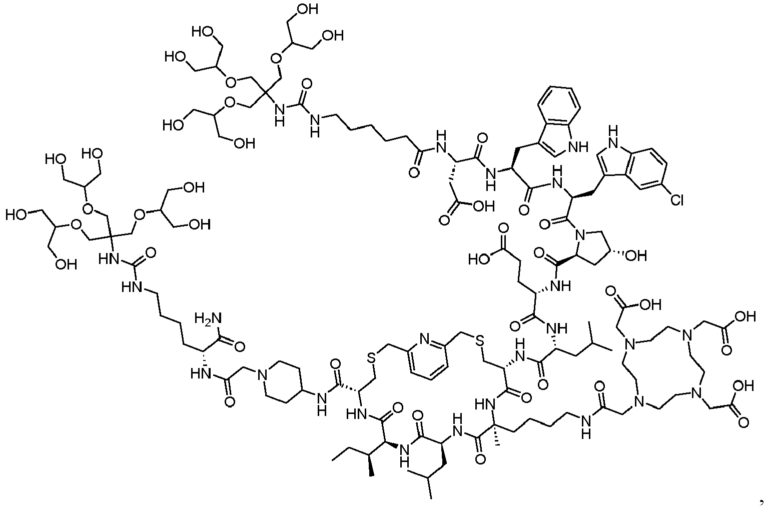
compound HPD3:PEO:Aur-Asp-Trp-5Clw-Hyp-Glu-leu-[Cys(2Lut)-Amk(DOTA)-Leu-Ile-Cys]-APAc-kur:PEO:HPD3 (GIP-1054) of the following formula 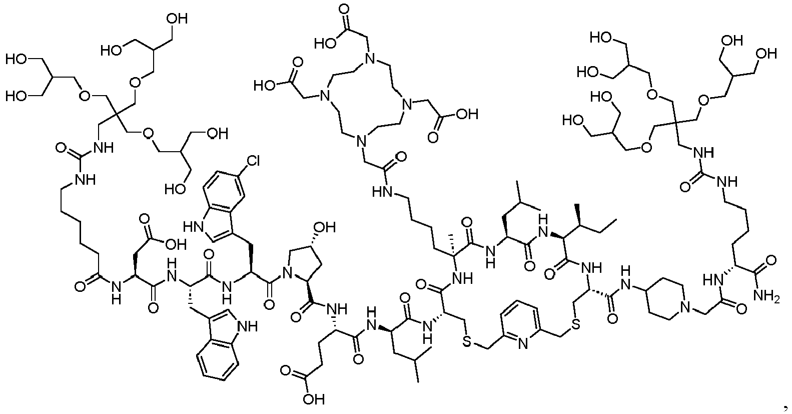
compound HPD3:TRIS:Aur-Asp-Trp-SClw-Hyp-Glu-leu-[Cys(2Lut)-Amk(DOTA)-Leu-Ile-Cys]-APAc-kur:TRIS:HPD3 (GIP-1055) of the following formula 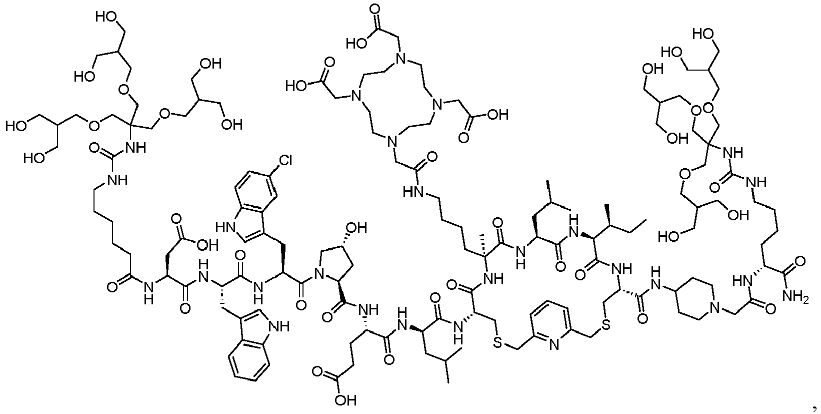
compound TRIS:Aur-Asp-Trp-5Clw-Hyp-Glu-leu-[Cys(2Lut)-Amk(DOTA)-Leu-Ile-Cys]-APAc-kur:TRIS (GIP-1056) of the following formula 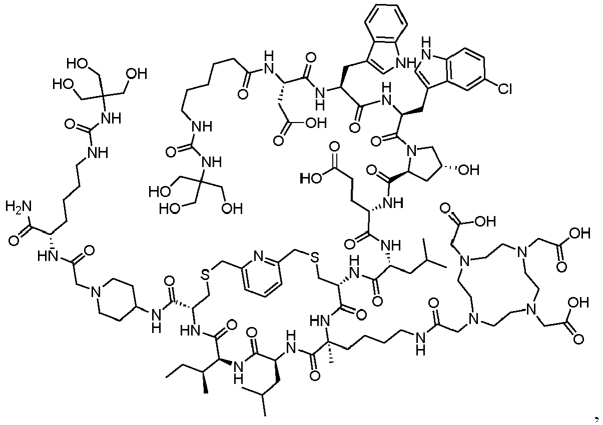
compound HAC2:HPD:Aur-Asp-Trp-5Clw-Tap-Glu-leu-[Cys(2Lut)-Apc(DOTA)-Leu-Ile-Cys]-APAc-kur:HPD:HAC2 (GIP-1057) of the following formula 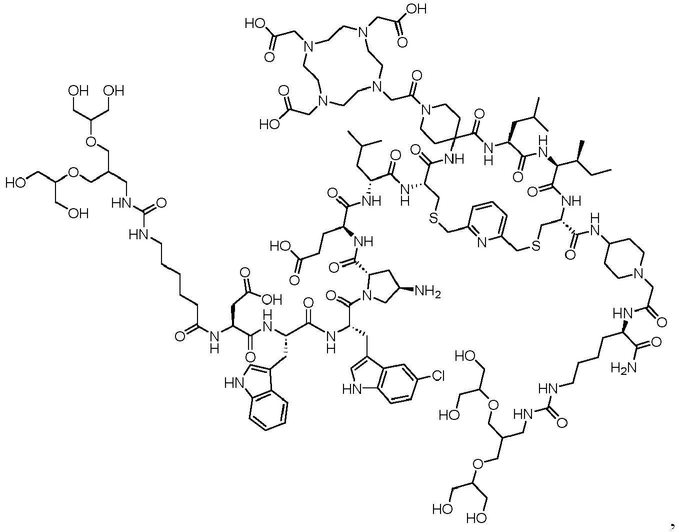
compound HAC2:HPD:Hyx-Asp-Trp-5Clw-Tap-Glu-leu-[Cys(2Lut)-Apc(DOTA)-Leu-Ile-Cys]-APAc-prx:HPD:HAC2 (GIP-1058) of the following formula 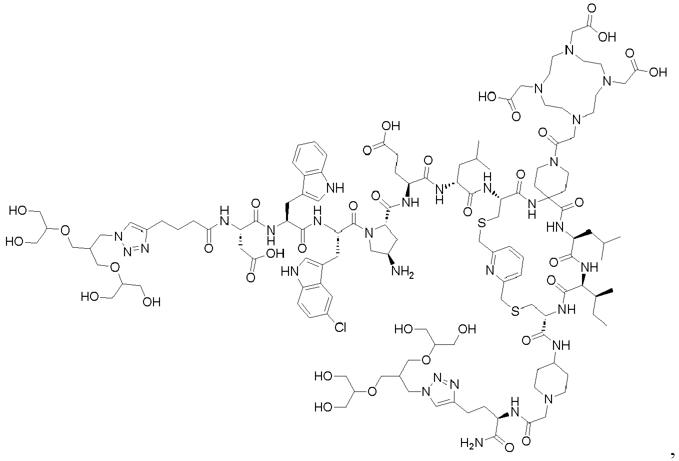
compound HAC4:HPD2:Ahx-Asp-Trp-5Clw-Tap-Glu-leu-[Cys(2Lut)-Apc(DOTA)-Leu-Ile-Cys]-APAc-lys:HPD2:HAC4 (GIP-1059) of the following formula 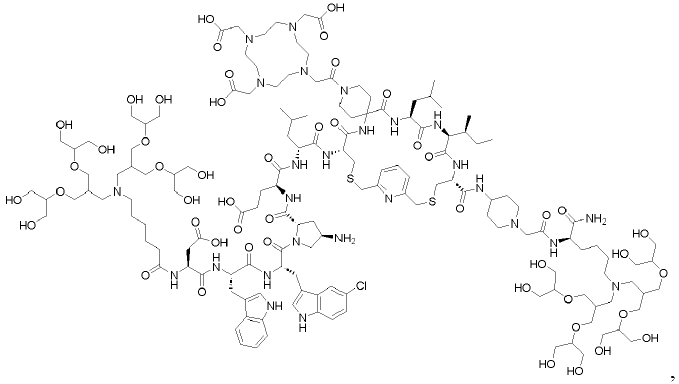
compound HPD2:HPD:Aur-Asp-Trp-5Clw-Tap-Glu-leu-[Cys(2Lut)-Apc(DOTA)-Leu-Ile-Cys]-APAc-kur:HPD:HPD2 (GIP-1060) of the following formula 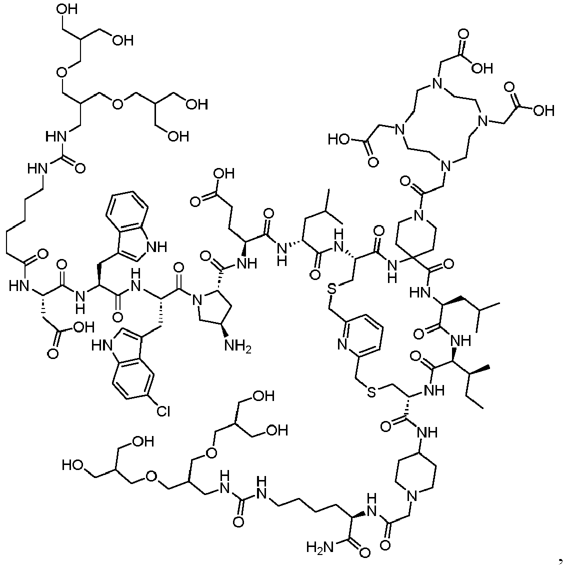
compound HPD2:HPD:Hyx-Asp-Trp-5Clw-Tap-Glu-leu-[Cys(2Lut)-Apc(DOTA)-Leu-Ile-Cys]-APAc-prx:HPD:HPD2 (GIP-1061) of the following formula 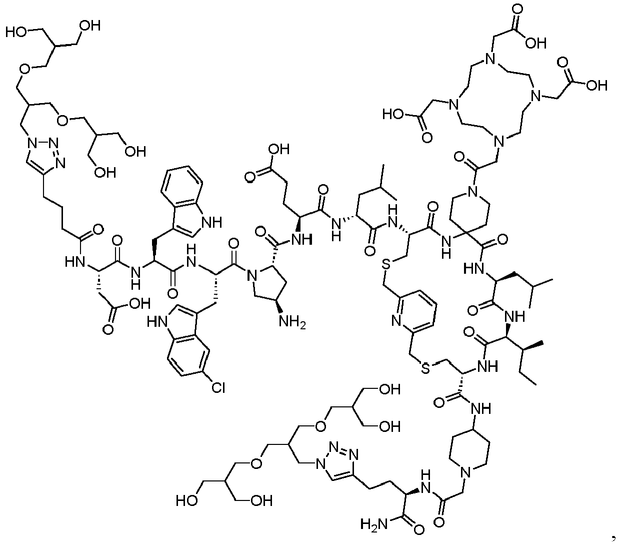
compound HPD4:HPD2:Ahx-Asp-Trp-5Clw-Tap-Glu-leu-[Cys(2Lut)-Apc(DOTA)-Leu-Ile-Cys]-APAc-lys:HPD2:HPD4 (GIP-1062) of the following formula 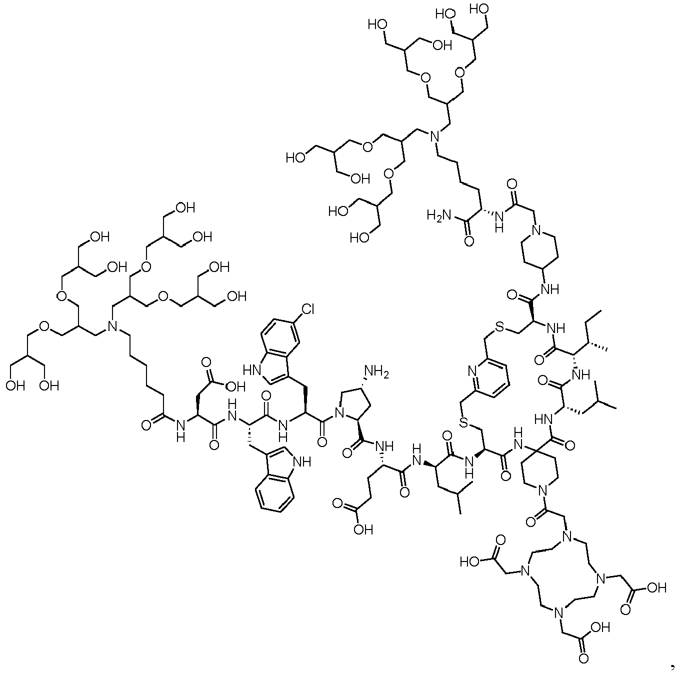
compound HAC2:HAC:Aur-Asp-Trp-5Clw-Tap-Glu-leu-[Cys(2Lut)-Apc(DOTA)-Leu-Ile-Cys]-APAc-kur:HAC:HAC2 (GIP-1063) of the following formula 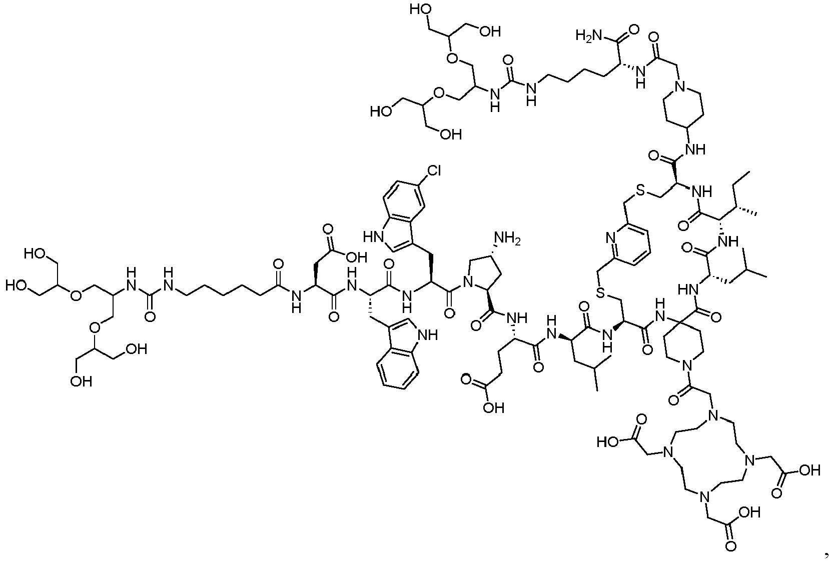
compound HPD2:HAC:Aur-Asp-Trp-5Clw-Tap-Glu-leu-[Cys(2Lut)-Apc(DOTA)-Leu-Ile-Cys]-APAc-kur:HAC:HPD2 (GIP-1064) of the following formula 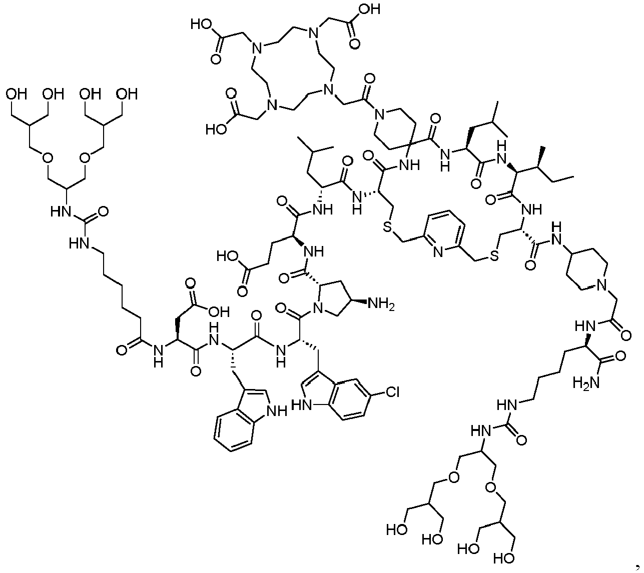
compound HAC2:HPD:Aur-Asp-Trp-5Clw-Tap-Glu-leu-[Cys(2Lut)-Amk(DOTA)-Leu-Ile-Cys]-APAc-kur:HPD:HAC2 (GIP-1065) of the following formula 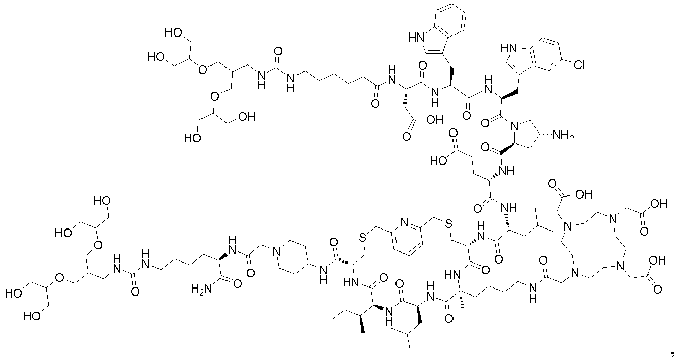
compound HAC2:HPD:Hyx-Asp-Trp-5Clw-Tap-Glu-leu-[Cys(2Lut)-Amk(DOTA)-Leu-Ile-Cys]-APAc-prx:HPD:HAC2 (GIP-1066) of the following formula 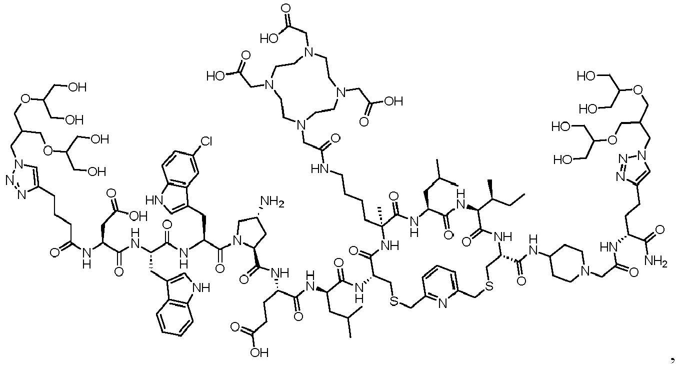
compound HAC4:HPD2:Ahx-Asp-Trp-5Clw-Tap-Glu-leu-[Cys(2Lut)-Amk(DOTA)-Leu-Ile-Cys]-APAc-lys:HPD2:HAC4 (GIP-1067) of the following formula 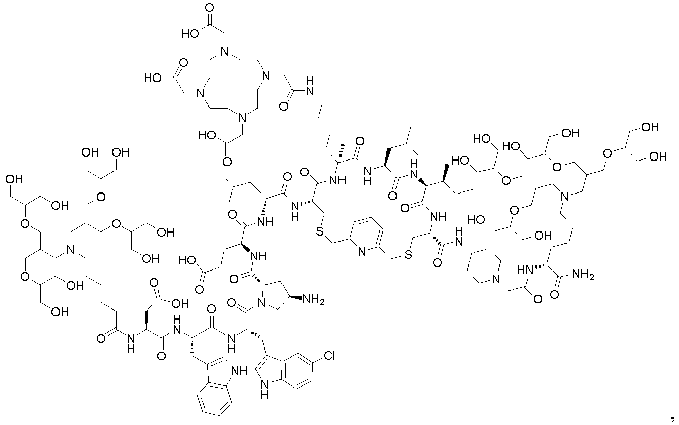
compound HPD2:HPD:Aur-Asp-Trp-5Clw-Tap-Glu-leu-[Cys(2Lut)-Amk(DOTA)-Leu-Ile-Cys]-APAc-kur:HPD:HPD2 (GIP-1068) of the following formula 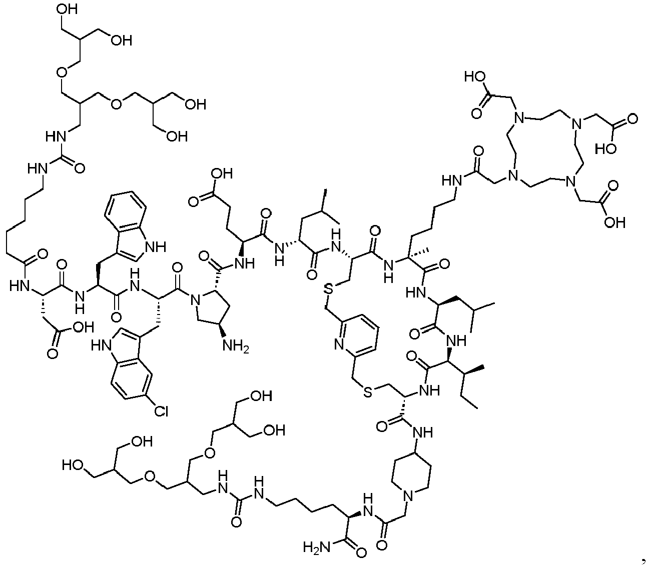
compound HPD2:HPD:Hyx-Asp-Trp-5Clw-Tap-Glu-leu-[Cys(2Lut)-Amk(DOTA)-Leu-Ile-Cys]-APAc-prx:HPD:HPD2 (GIP-1069) of the following formula 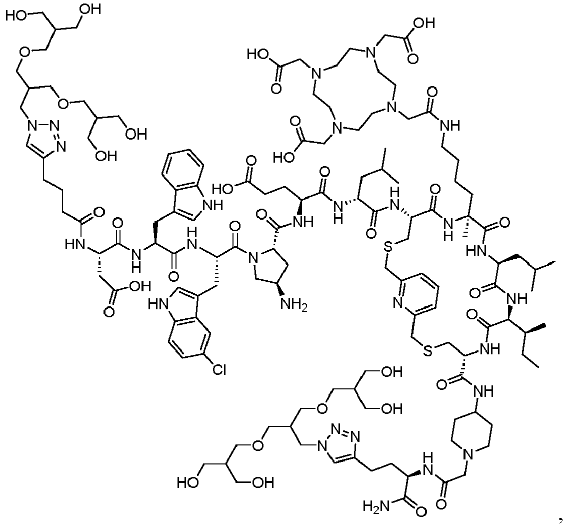
compound HPD4:HPD2:Ahx-Asp-Trp-5Clw-Tap-Glu-leu-[Cys(2Lut)-Amk(DOTA)-Leu-Ile-Cys]-APAc-lys:HPD2:HPD4 (GIP-1070) of the following formula 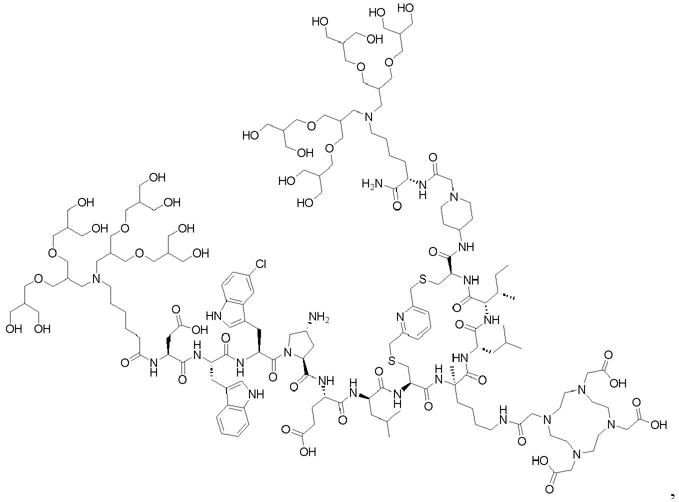
compound HAC2:HAC:Aur-Asp-Trp-5Clw-Tap-Glu-leu-[Cys(2Lut)-Amk(DOTA)-Leu-Ile-Cys]-APAc-kur:HAC:HAC2 (GIP-1071) of the following formula 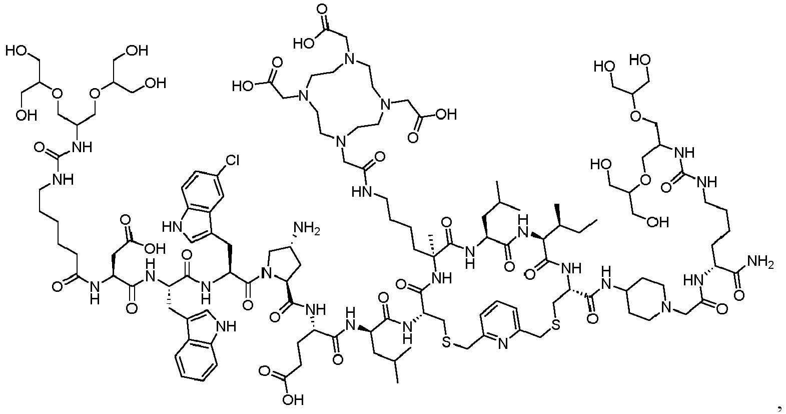
compound HPD2:HAC:Aur-Asp-Trp-5Clw-Tap-Glu-leu-[Cys(2Lut)-Amk(DOTA)-Leu-Ile-Cys]-APAc-kur:HAC:HPD2 (GIP-1072) of the following formula 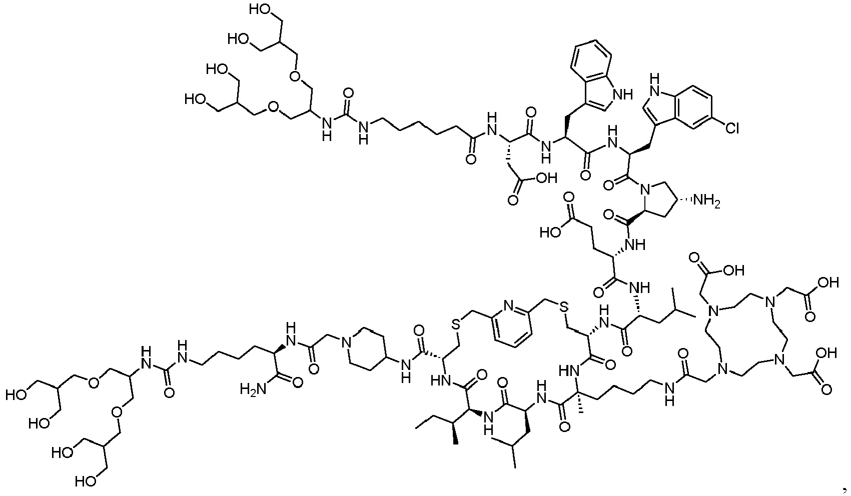
compound HAC2:HPD:Aur-Asp-Trp-5Clw-Hyp-Glu-leu-[Cys(2Lut)-Apc(DOTA)-Leu-Ile-Cys]-APAc-kur:HPD:HAC2 (GIP-1073) of the following formula 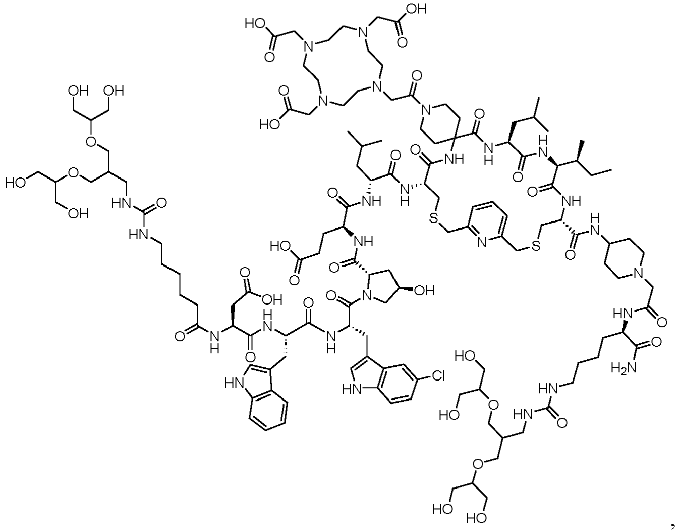
compound HAC2:HPD:Hyx-Asp-Trp-5Clw-Hyp-Glu-leu-[Cys(2Lut)-Apc(DOTA)-Leu-Ile-Cys]-APAc-prx:HPD:HAC2 (GIP-1074) of the following formula 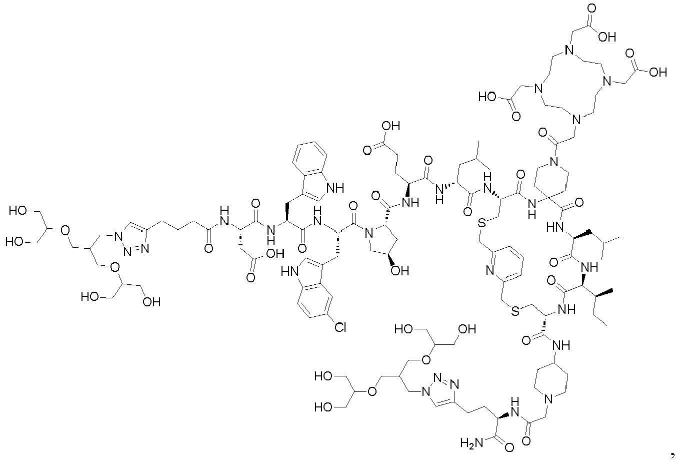
compound HAC4:HPD2:Ahx-Asp-Trp-5Clw-Hyp-Glu-leu-[Cys(2Lut)-Apc(DOTA)-Leu-Ile-Cys]-APAc-lys:HPD2:HAC4 (GIP-1075) of the following formula 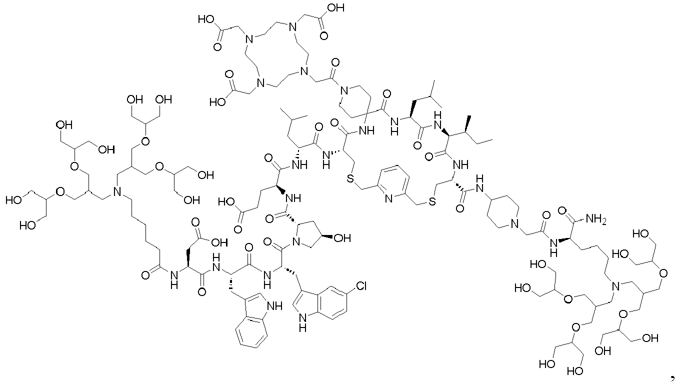
compound HPD2:HPD:Aur-Asp-Trp-5Clw-Hyp-Glu-leu-[Cys(2Lut)-Apc(DOTA)-Leu-Ile-Cys]-APAc-kur:HPD:HPD2 (GIP-1076) of the following formula 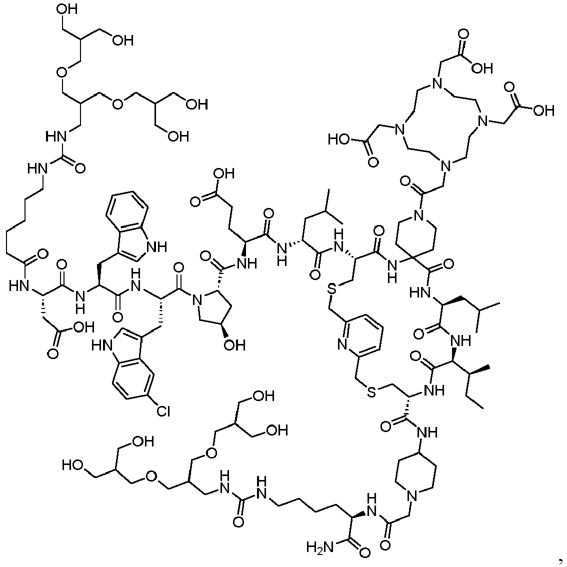
compound HPD2:HPD:Hyx-Asp-Trp-5Clw-Hyp-Glu-leu-[Cys(2Lut)-Apc(DOTA)-Leu-Ile-Cys]-APAc-prx:HPD:HPD2 (GIP-1077) of the following formula 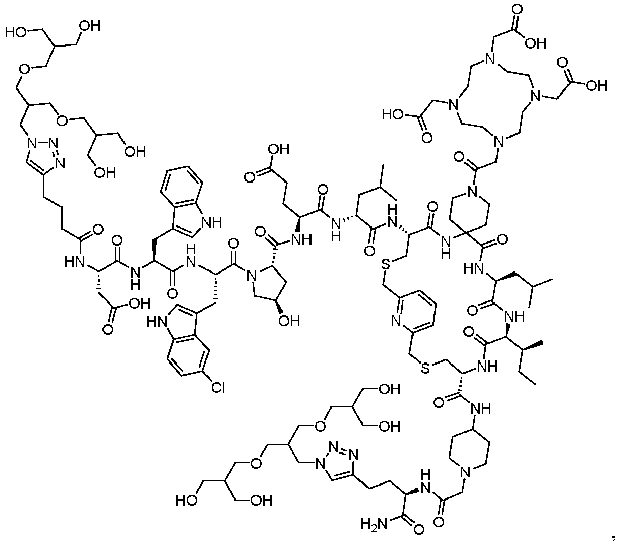
compound HPD4:HPD2:Ahx-Asp-Trp-5Clw-Hyp-Glu-leu-[Cys(2Lut)-Apc(DOTA)-Leu-Ile-Cys]-APAc-lys:HPD2:HPD4 (GIP-1078) of the following formula 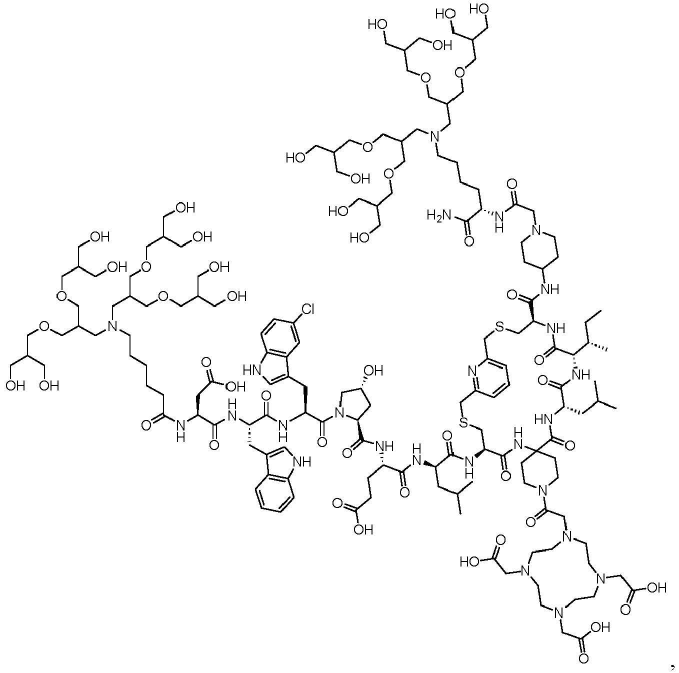
compound HAC2:HAC:Aur-Asp-Trp-5Clw-Hyp-Glu-leu-[Cys(2Lut)-Apc(DOTA)-Leu-Ile-Cys]-APAc-kur:HAC:HAC2 (GIP-1079) of the following formula 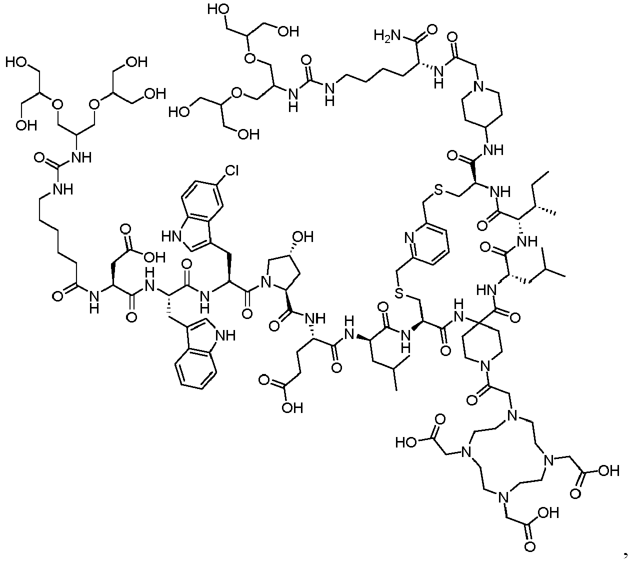
compound HPD2:HAC:Aur-Asp-Trp-5Clw-Hyp-Glu-leu-[Cys(2Lut)-Apc(DOTA)-Leu-Ile-Cys]-APAc-kur:HAC:HPD2 (GIP-1080) of the following formula 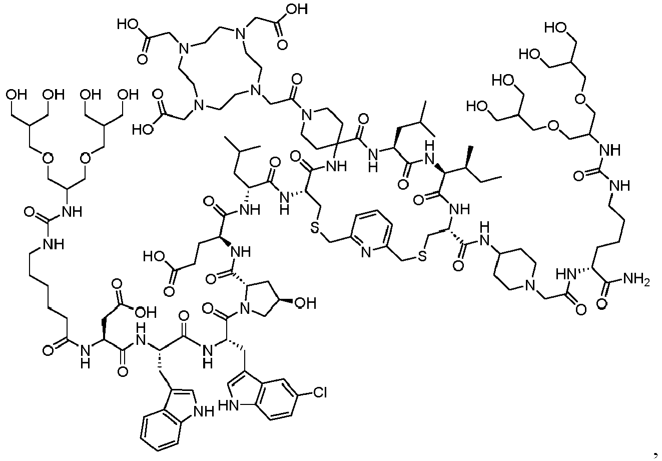
compound HAC2:HPD:Aur-Asp-Trp-5Clw-Hyp-Glu-leu-[Cys(2Lut)-Amk(DOTA)-Leu-Ile-Cys]-APAc-kur:HPD:HAC2 (GIP-1081) of the following formula 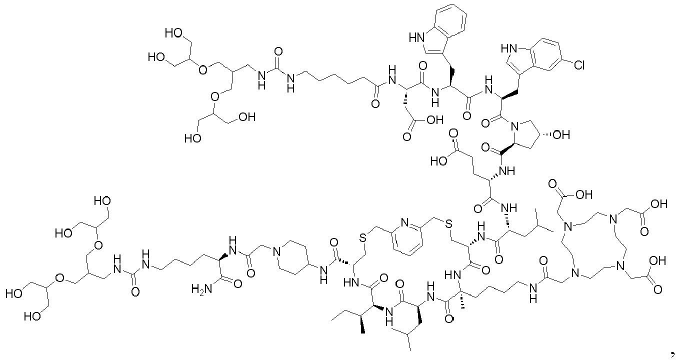
compound HAC2:HPD:Hyx-Asp-Trp-5Clw-Hyp-Glu-leu-[Cys(2Lut)-Amk(DOTA)-Leu-Ile-Cys]-APAc-prx:HPD:HAC2 (GIP-1082) of the following formula 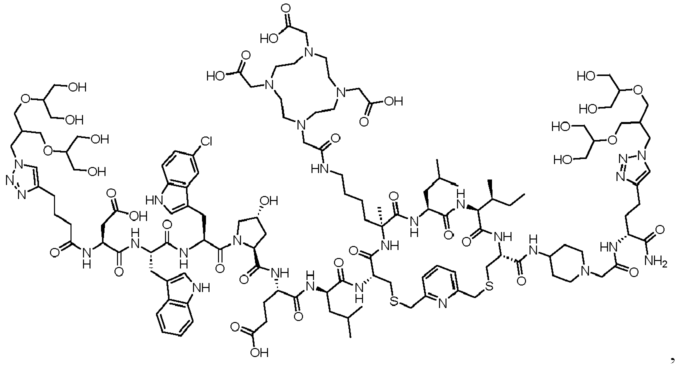
compound HAC4:HPD2:Ahx-Asp-Trp-5Clw-Hyp-Glu-leu-[Cys(2Lut)-Amk(DOTA)-Leu-Ile-Cys]-APAc-lys:HPD2:HAC4 (GIP-1083) of the following formula 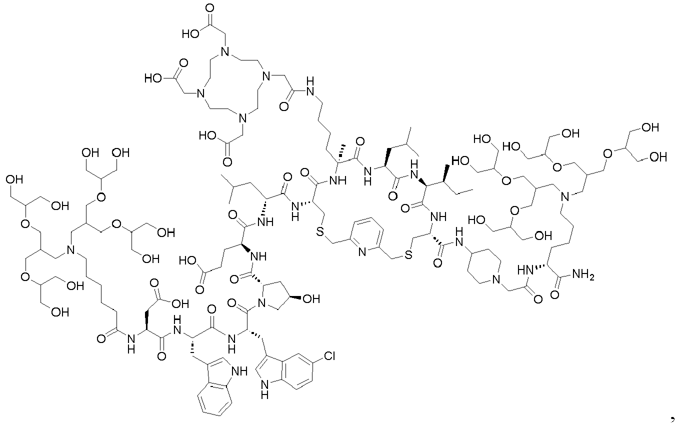
compound HPD2:HPD:Aur-Asp-Trp-5Clw-Hyp-Glu-leu-[Cys(2Lut)-Amk(DOTA)-Leu-Ile-Cys]-APAc-kur:HPD:HPD2 (GIP-1084) of the following formula 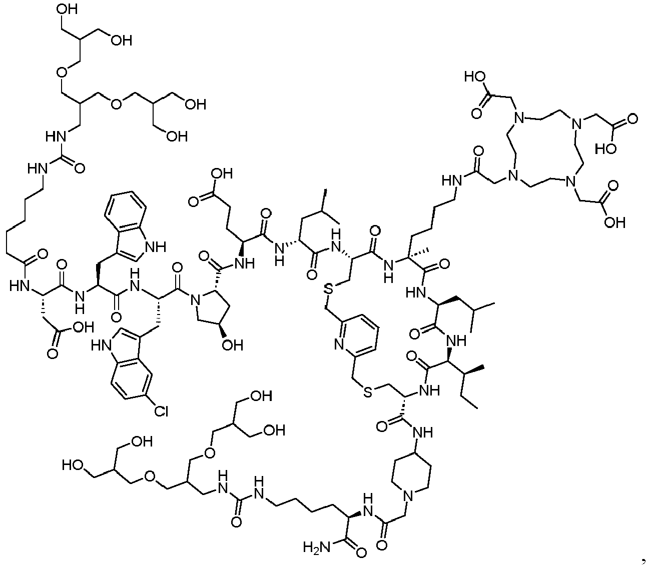
compound HPD2:HPD:Hyx-Asp-Trp-5Clw-Hyp-Glu-leu-[Cys(2Lut)-Amk(DOTA)-Leu-Ile-Cys]-APAc-prx:HPD:HPD2 (GIP-1085) of the following formula 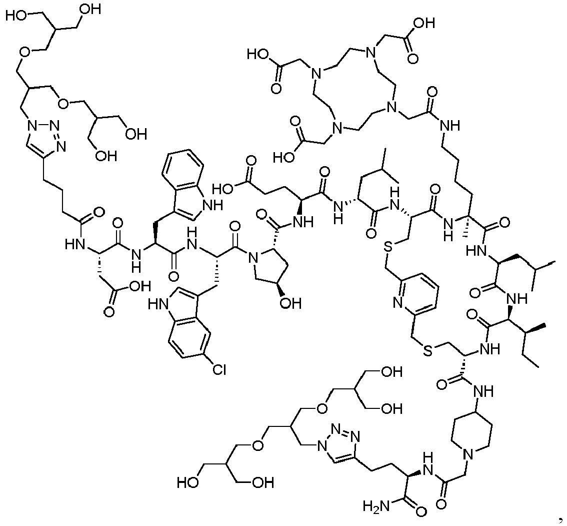
compound HPD4:HPD2:Ahx-Asp-Trp-5Clw-Hyp-Glu-leu-[Cys(2Lut)-Amk(DOTA)-Leu-Ile-Cys]-APAc-lys:HPD2:HPD4 (GIP-1086) of the following formula 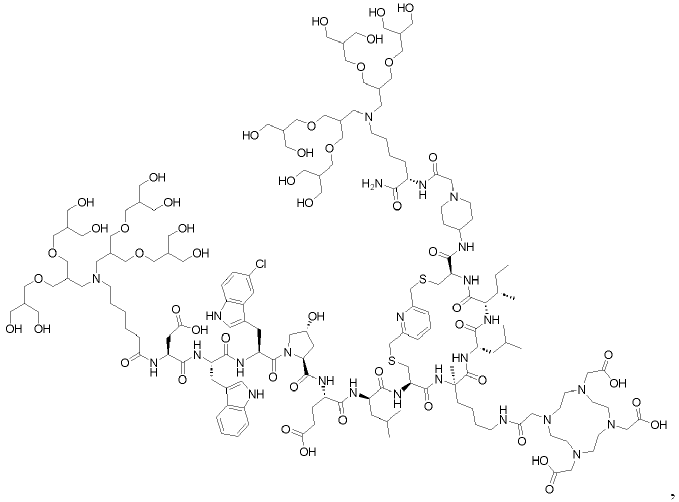
compound HAC2:HAC:Aur-Asp-Trp-5Clw-Hyp-Glu-leu-[Cys(2Lut)-Amk(DOTA)-Leu-Ile-Cys]-APAc-kur:HAC:HAC2 (GIP-1087) of the following formula 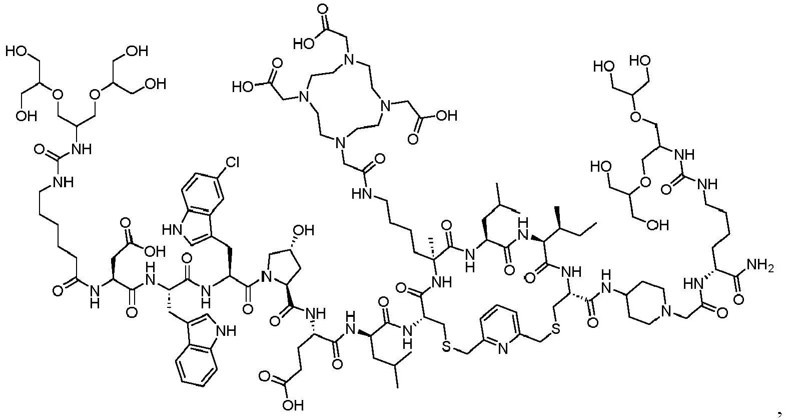
compound HPD2:HAC:Aur-Asp-Trp-5Clw-Hyp-Glu-leu-[Cys(2Lut)-Amk(DOTA)-Leu-Ile-Cys]-APAc-kur:HAC:HPD2 (GIP-1088) of the following formula 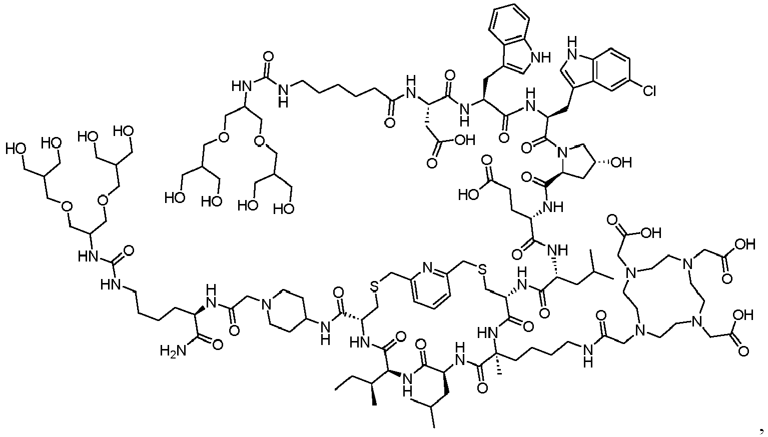
compound HAC2:HAC:Ahx-Asp-Trp-5Clw-Tap-Glu-leu-[Cys(2Lut)-Apc(DOTA)-Leu-Ile-Cys]-APAc-lys:HAC:HAC2 (GIP-1089) of the following formula 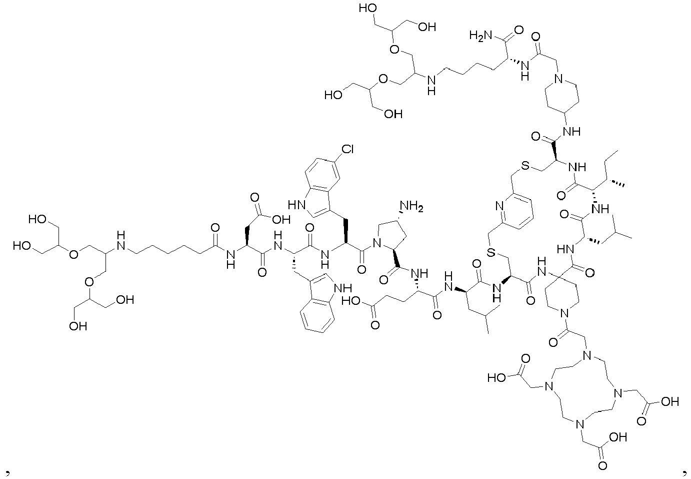
compound HAC2 :HAC: Tzx-Asp-Trp-5 Clw-Tap-Glu-leu-[Cys(2Lut)-Apc(DOTA)-Leu-Ile-Cys] - APAc-tzk:HAC:HAC2 (GIP-1090) of the following formula 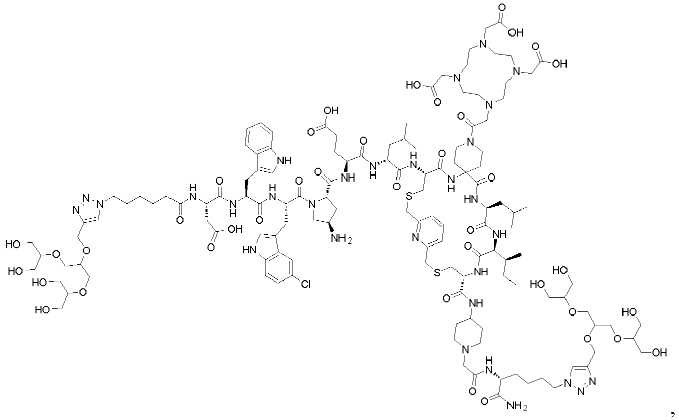
compound HAC2:HPD:Tzx-Asp-Trp-5Clw-Tap-Glu-leu-[Cys(2Lut)-Apc(DOTA)-Leu-Ile-Cys]-APAc-tzk:HPD:HAC2 (GIP-1091) of the following formula 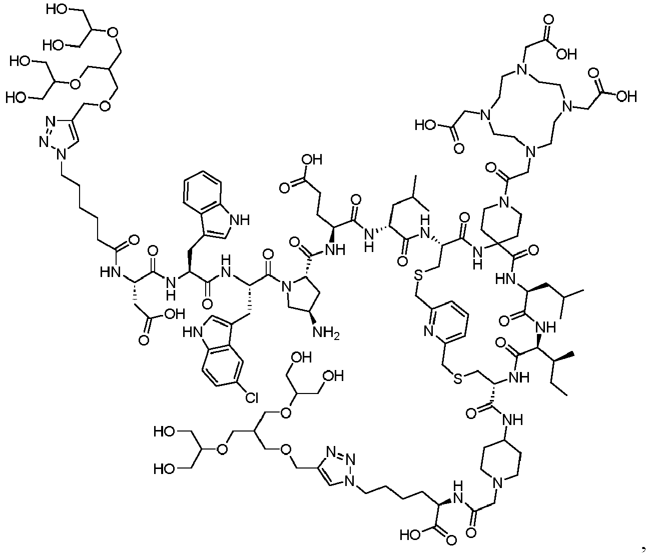
compound HPD:Aur-Asp-Trp-5Clw-Tap-Glu-leu-[Cys(2Lut)-Apc(DOTA)-Leu-Ile-Cys]-APAc-kur:HPD (GIP-1092) of the following formula 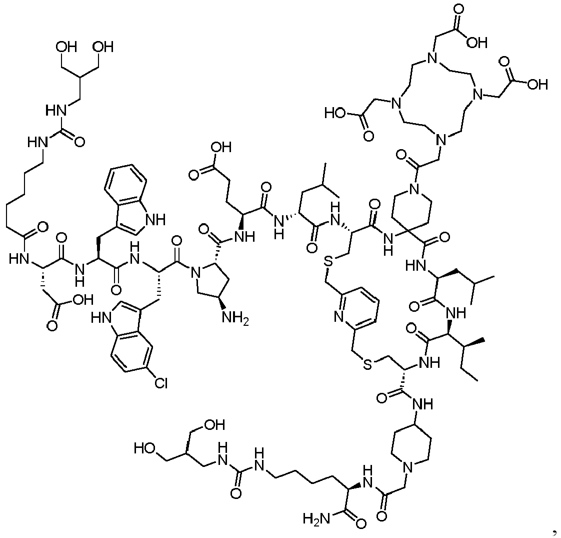
compound HPD:Hyx-Asp-Trp-5Clw-Tap-Glu-leu-[Cys(2Lut)-Apc(DOTA)-Leu-Ile-Cys]-APAc-prx:HPD (GIP-1093) of the following formula 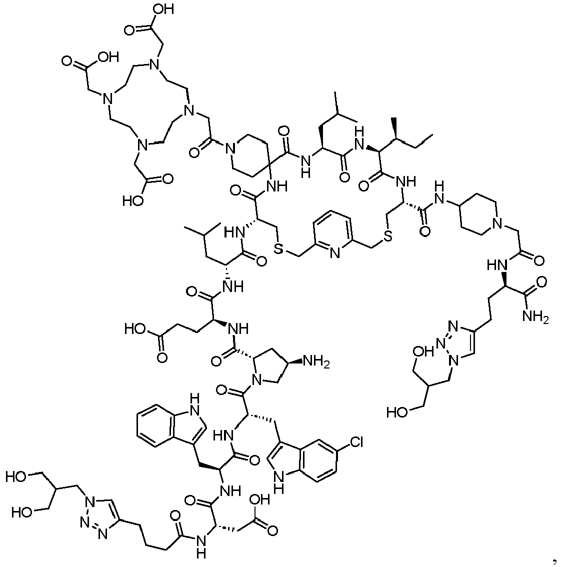
compound HPD2:HAC:Ahx-Asp-Trp-5Clw-Tap-Glu-leu-[Cys(2Lut)-Apc(DOTA)-Leu-Ile-Cys]-APAc-lys:HAC:HPD2 (GIP-1094) of the following formula 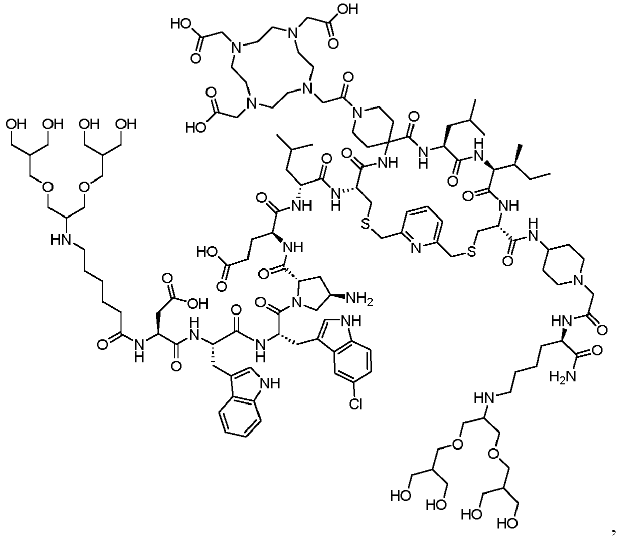
compound HPD2:HPD:Tzx-Asp-Trp-5Clw-Tap-Glu-leu-[Cys(2Lut)-Apc(DOTA)-Leu-Ile-Cys]-APAc-tzk:HPD:HPD2 (GIP-1095) of the following formula 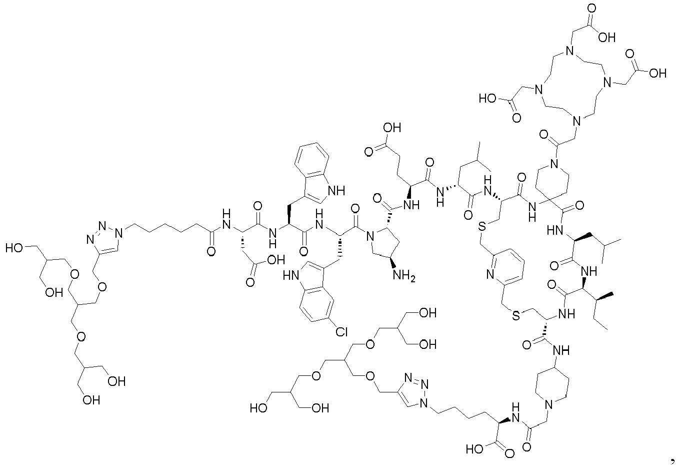
compound HAC:Aur-Asp-Trp-5Clw-Tap-Glu-leu-[Cys(2Lut)-Apc(DOTA)-Leu-Ile-Cys]-APAc-kur:HAC (GIP-1096) of the following formula 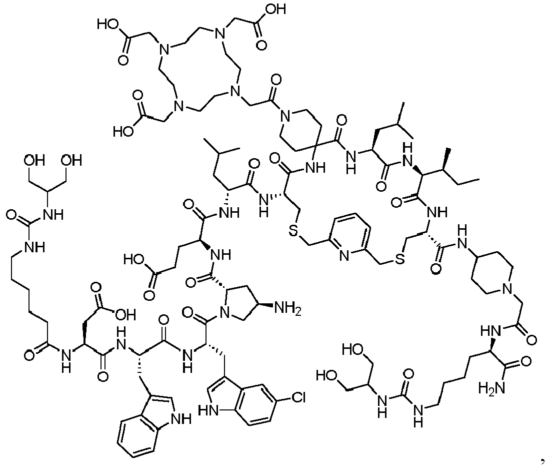
compound HPD2:HAC:Tzx-Asp-Trp-5Clw-Tap-Glu-leu-[Cys(2Lut)-Apc(DOTA)-Leu-Ile-Cys]-APAc-tzk:HAC:HPD2 (GIP-1097) of the following formula 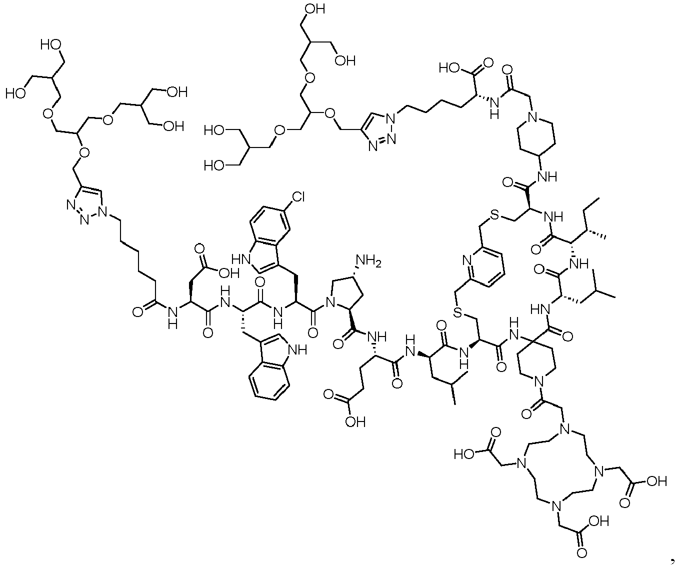
compound HAC2:HAC:Ahx-Asp-Trp-5Clw-Tap-Glu-leu-[Cys(2Lut)-Amk(DOTA)-Leu-Ile-Cys]-APAc-lys:HAC:HAC2 (GIP-1098) of the following formula 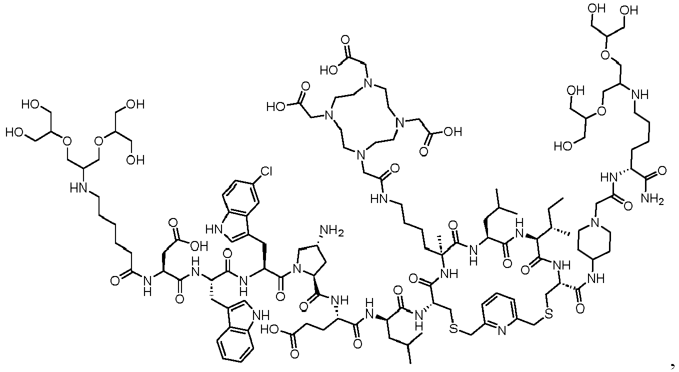
compound HAC2:HAC:Tzx-Asp-Trp-5Clw-Tap-Glu-leu-[Cys(2Lut)-Amk(DOTA)-Leu-Ile-Cys]-APAc-tzk:HAC:HAC2 (GIP-1099) of the following formula 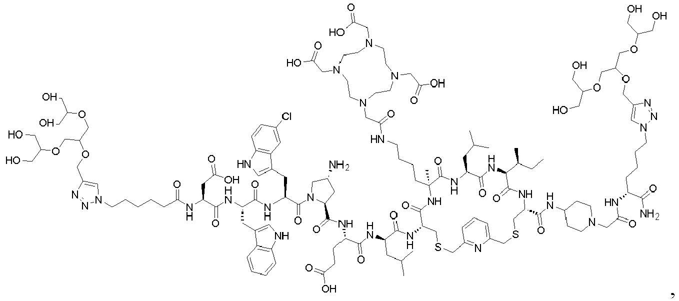
compound HAC2:HPD:Tzx-Asp-Trp-5Clw-Tap-Glu-leu-[Cys(2Lut)-Amk(DOTA)-Leu-Ile-Cys]-APAc-tzk:HPD:HAC2 (GIP-1100) of the following formula 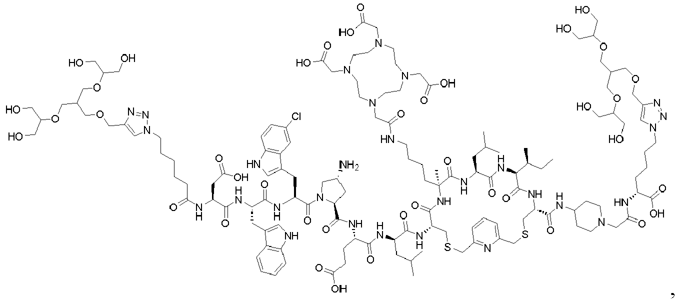
compound HPD:Aur-Asp-Trp-5Clw-Tap-Glu-leu-[Cys(2Lut)-Amk(DOTA)-Leu-Ile-Cys]-APAc-kur:HPD (GIP-1101) of the following formula 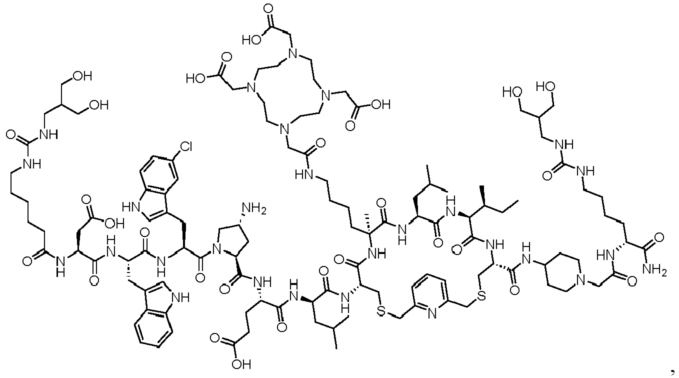
compound HPD:Hyx-Asp-Trp-5Clw-Tap-Glu-leu-[Cys(2Lut)-Amk(DOTA)-Leu-Ile-Cys]-APAc-prx:HPD (GIP-1102) of the following formula 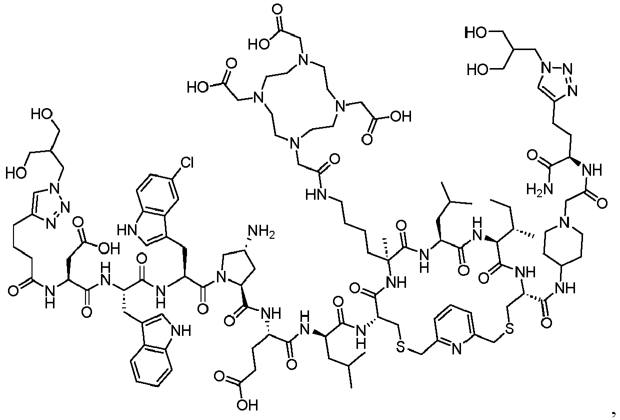
compound HPD2:HAC:Ahx-Asp-Trp-5Clw-Tap-Glu-leu-[Cys(2Lut)-Amk(DOTA)-Leu-Ile-Cys]-APAc-lys:HAC:HPD2 (GIP-1103) of the following formula 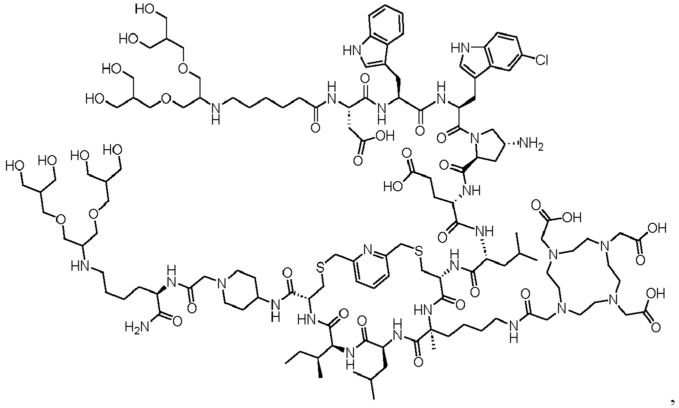
compound HPD2:HPD:Tzx-Asp-Trp-5Clw-Tap-Glu-leu-[Cys(2Lut)-Amk(DOTA)-Leu-Ile-Cys]-APAc-tzk:HPD:HPD2 (GIP-1104) of the following formula 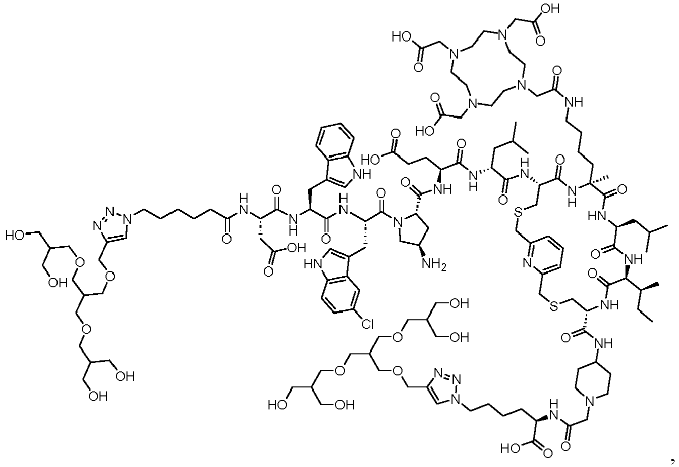
compound HAC:Aur-Asp-Trp-5Clw-Tap-Glu-leu-[Cys(2Lut)-Amk(DOTA)-Leu-Ile-Cys]-APAc-kur:HAC (GIP-1105) of the following formula 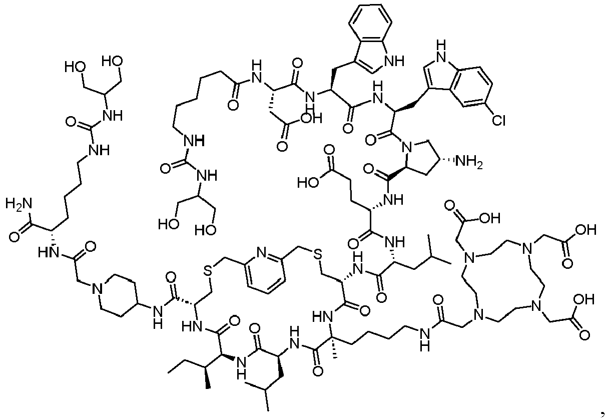
compound HPD2:HAC:Tzx-Asp-Trp-5Clw-Tap-Glu-leu-[Cys(2Lut)-Amk(DOTA)-Leu-Ile-Cys]-APAc-tzk:HAC:HPD2 (GIP-1106) of the following formula 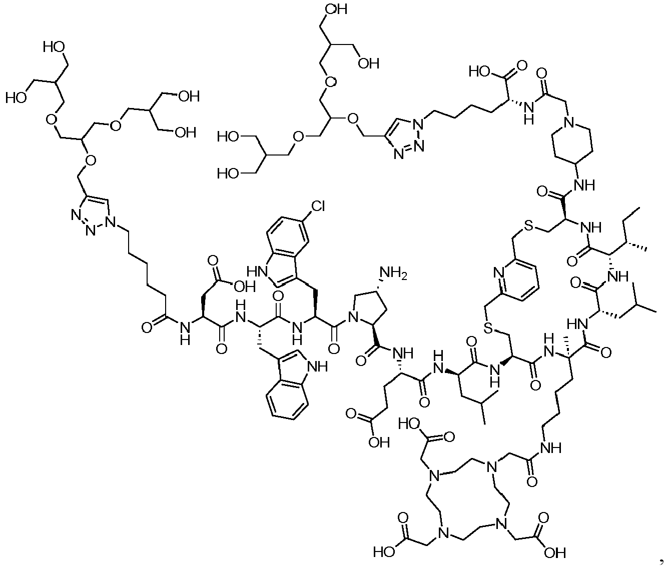
compound HAC2:HAC:Ahx-Asp-Trp-5Clw-Hyp-Glu-leu-[Cys(2Lut)-Apc(DOTA)-Leu-Ile-Cys]-APAc-lys:HAC:HAC2 (GIP-1107) of the following formula 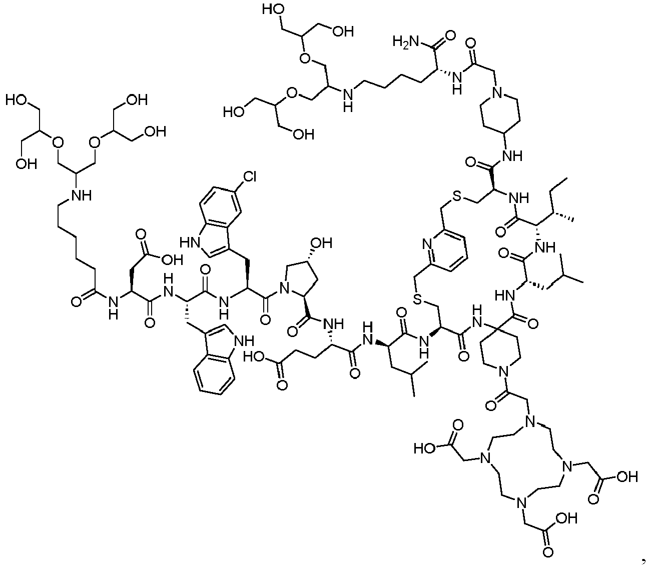
compound HAC2:HAC:Tzx-Asp-Trp-5Clw-Hyp-Glu-leu-[Cys(2Lut)-Apc(DOTA)-Leu-Ile-Cys]-APAc-tzk:HAC:HAC2 (GIP-1108) of the following formula 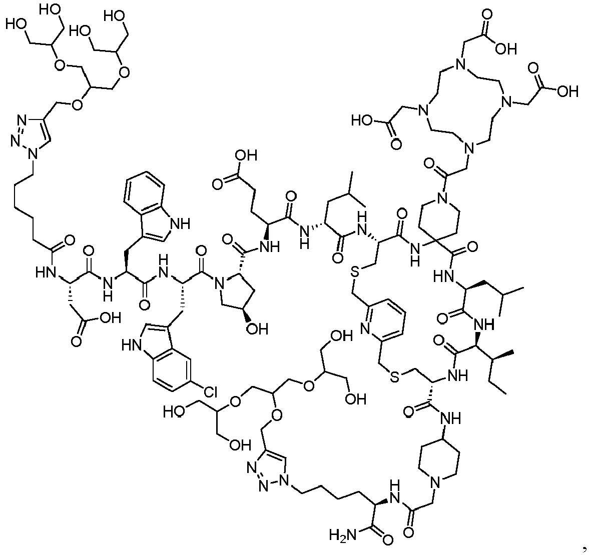
compound HAC2:HPD:Tzx-Asp-Trp-5Clw-Hyp-Glu-leu-[Cys(2Lut)-Apc(DOTA)-Leu-Ile-Cys]-APAc-tzk:HPD:HAC2 (GIP-1109) of the following formula 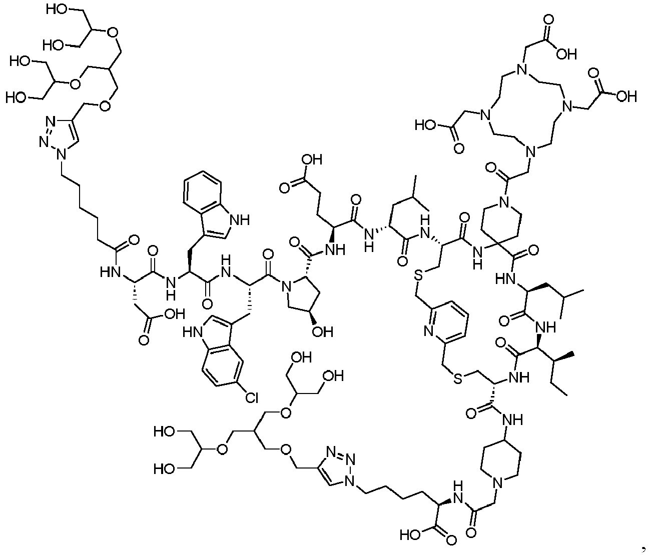
compound HPD:Aur-Asp-Trp-5Clw-Hyp-Glu-leu-[Cys(2Lut)-Apc(DOTA)-Leu-Ile-Cys]-APAc-kur:HPD (GIP-1110) of the following formula 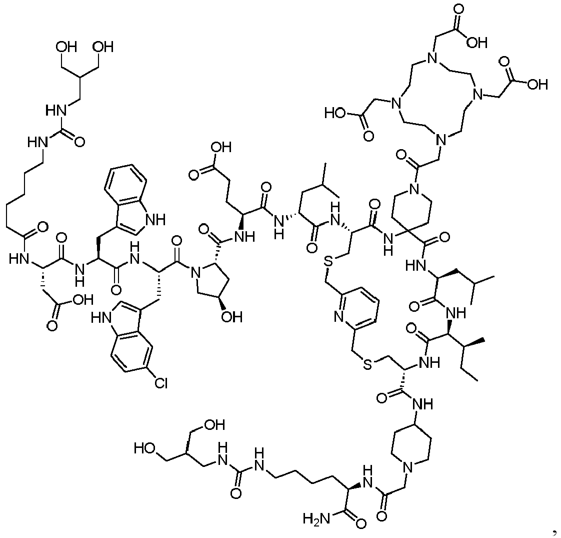
compound HPD:Hyx-Asp-Trp-5Clw-Hyp-Glu-leu-[Cys(2Lut)-Apc(DOTA)-Leu-Ile-Cys]-APAc-prx:HPD (GIP-1111) of the following formula 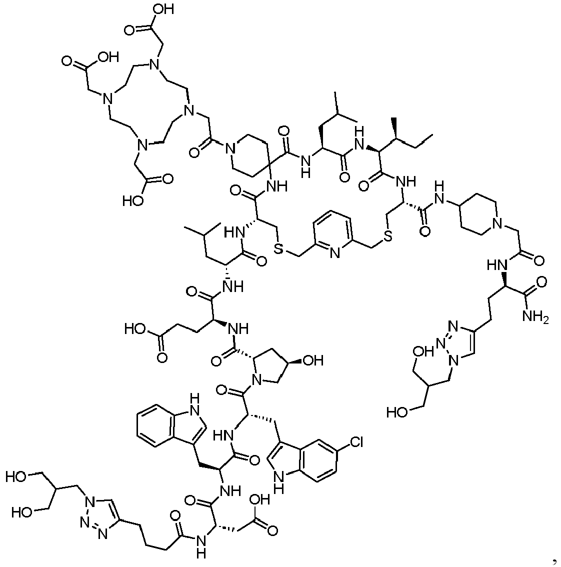
compound HPD2:HAC:Ahx-Asp-Trp-5Clw-Hyp-Glu-leu-[Cys(2Lut)-Apc(DOTA)-Leu-Ile-Cys]-APAc-lys:HAC:HPD2 (GIP-1112) of the following formula 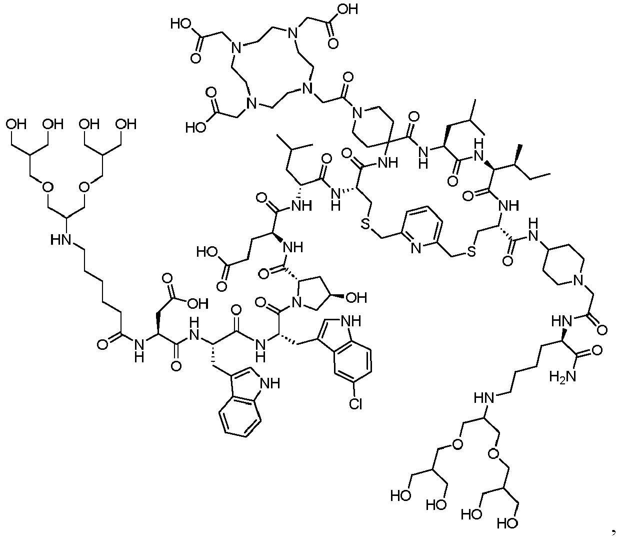
compound HPD2:HPD:Tzx-Asp-Trp-5Clw-Hyp-Glu-leu-[Cys(2Lut)-Apc(DOTA)-Leu-Ile-Cys]-APAc-tzk:HPD:HPD2 (GIP-1113) of the following formula 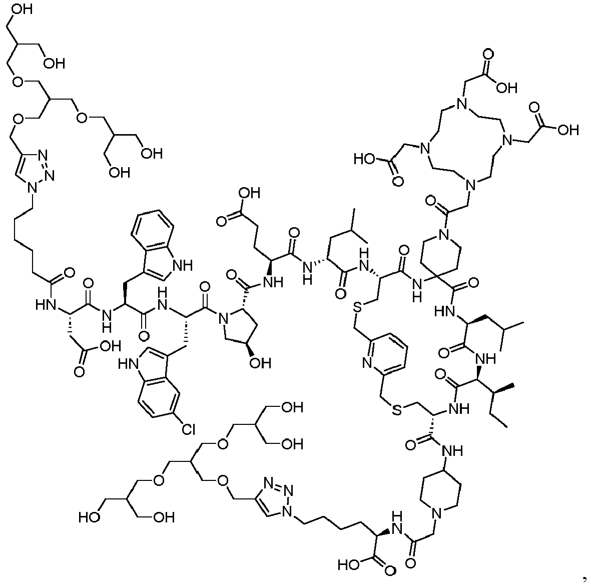
compound HAC:Aur-Asp-Trp-5Clw-Hyp-Glu-leu-[Cys(2Lut)-Apc(DOTA)-Leu-Ile-Cys]-APAc-kur:HAC (GIP-1114) of the following formula 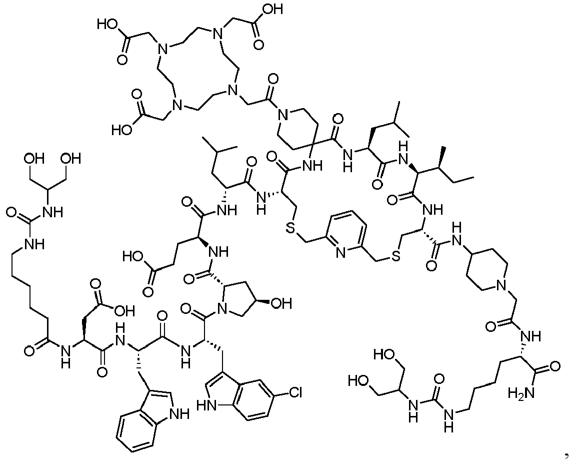
compound HPD2:HAC:Tzx-Asp-Trp-5Clw-Hyp-Glu-leu-[Cys(2Lut)-Apc(DOTA)-Leu-Ile-Cys]-APAc-tzk:HAC:HPD2 (GIP-1115) of the following formula 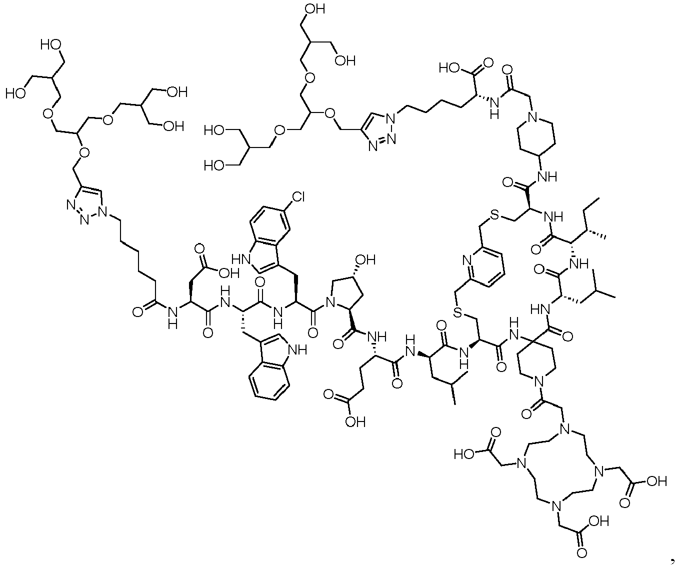
compound HAC2:HAC:Ahx-Asp-Trp-5Clw-Hyp-Glu-leu-[Cys(2Lut)-Amk(DOTA)-Leu-Ile-Cys]-APAc-lys:HAC:HAC2 (GIP-1116) of the following formula 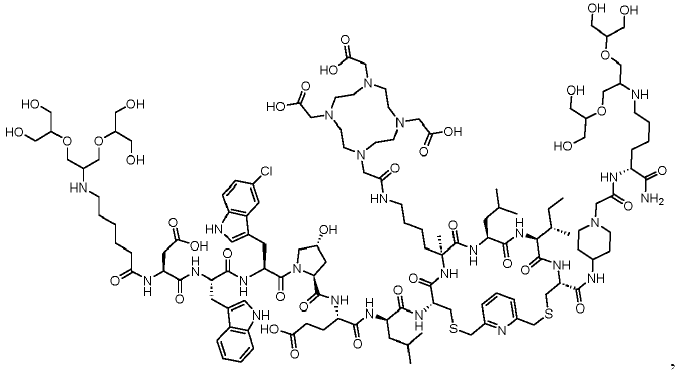
compound HAC2:HAC:Tzx-Asp-Trp-5Clw-Hyp-Glu-leu-[Cys(2Lut)-Amk(DOTA)-Leu-Ile-Cys]-APAc-tzk:HAC:HAC2 (GIP-1117) of the following formula 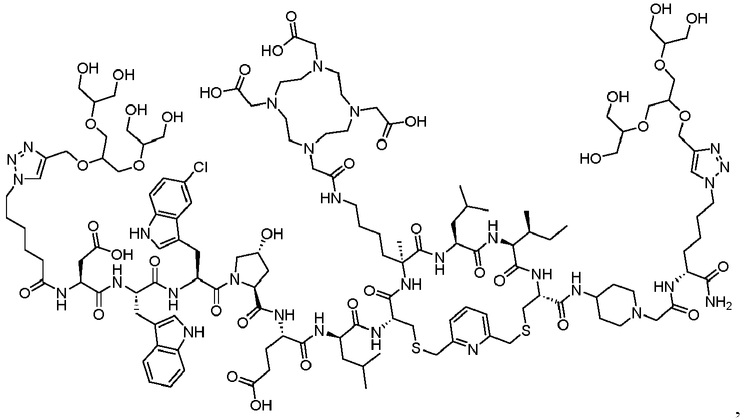
compound HAC2:HPD:Tzx-Asp-Trp-5Clw-Hyp-Glu-leu-[Cys(2Lut)-Amk(DOTA)-Leu-Ile-Cys]-APAc-tzk:HPD:HAC2 (GIP-1118) of the following formula 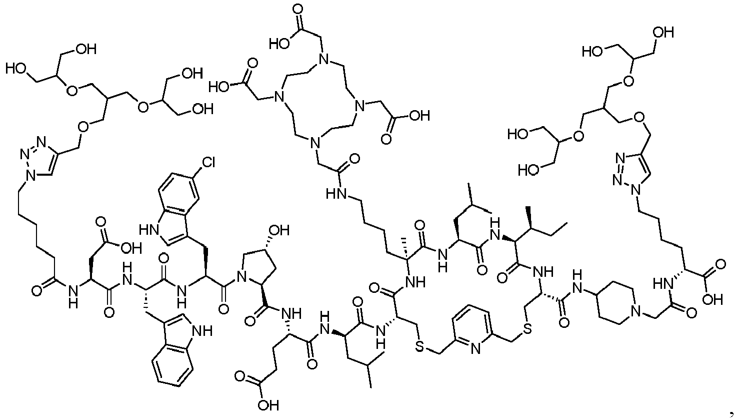
compound HPD:Aur-Asp-Trp-5Clw-Hyp-Glu-leu-[Cys(2Lut)-Amk(DOTA)-Leu-Ile-Cys]-APAc-kur:HPD (GIP-1119) of the following formula 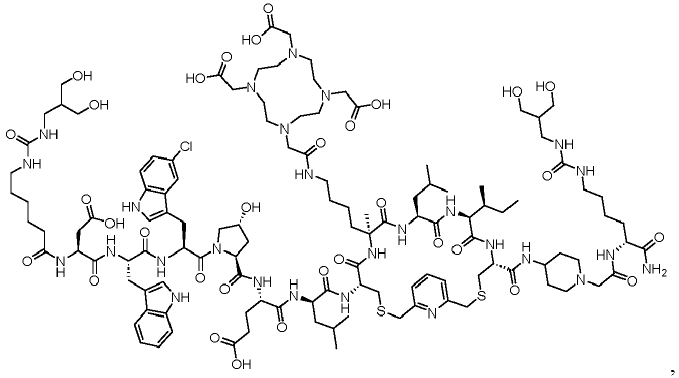
compound HPD:Hyx-Asp-Trp-5Clw-Hyp-Glu-leu-[Cys(2Lut)-Amk(DOTA)-Leu-Ile-Cys]-APAc-prx:HPD (GIP-1120) of the following formula 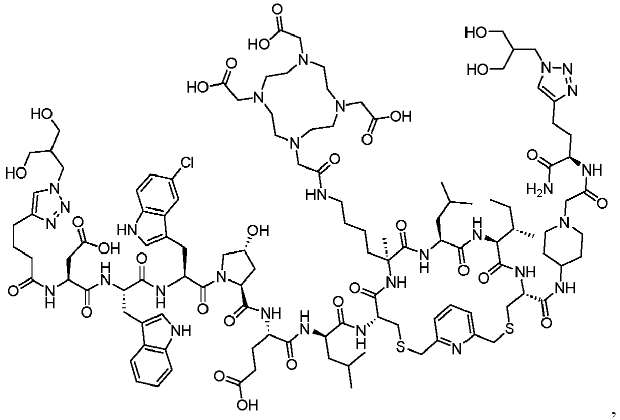
compound HPD2:HAC:Ahx-Asp-Trp-5Clw-Hyp-Glu-leu-[Cys(2Lut)-Amk(DOTA)-Leu-Ile-Cys]-APAc-lys:HAC:HPD2 (GIP-1121) of the following formula 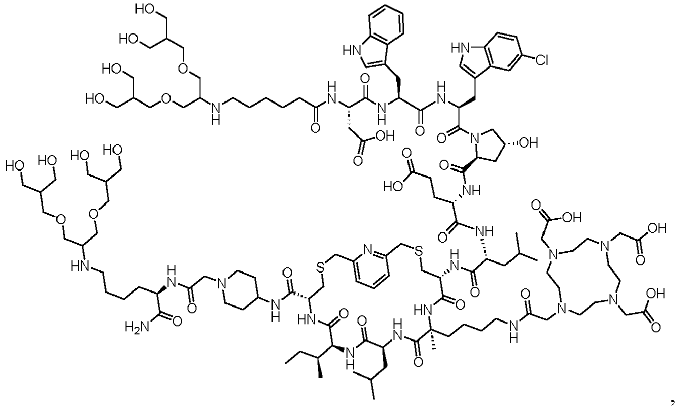
compound HPD2:HPD:Tzx-Asp-Trp-5Clw-Hyp-Glu-leu-[Cys(2Lut)-Amk(DOTA)-Leu-Ile-Cys]-APAc-tzk:HPD:HPD2 (GIP-1122) of the following formula 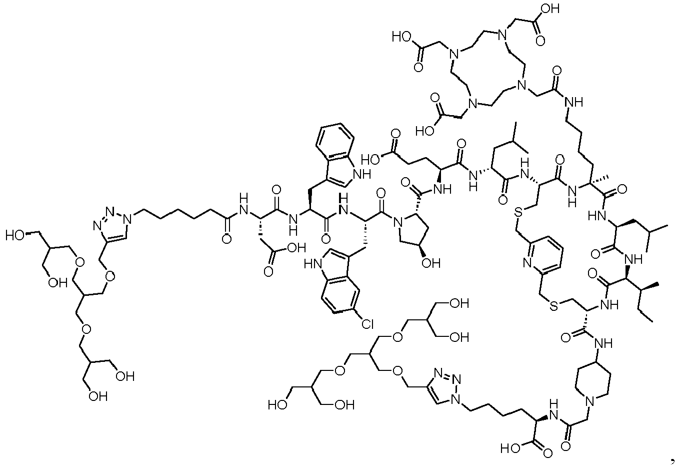
compound HAC:Aur-Asp-Trp-5Clw-Hyp-Glu-leu-[Cys(2Lut)-Amk(DOTA)-Leu-Ile-Cys]-APAc-kur:HAC (GIP-1123) of the following formula 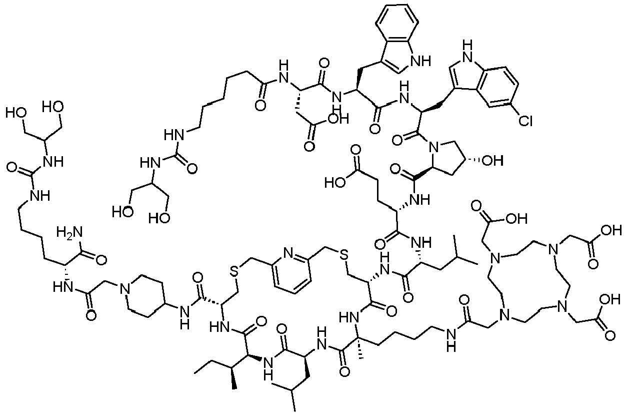
compound HPD2:HAC:Tzx-Asp-Trp-5Clw-Hyp-Glu-leu-[Cys(2Lut)-Amk(DOTA)-Leu-Ile-Cys]-APAc-tzk:HAC:HPD2 (GIP-1124) of the following formula 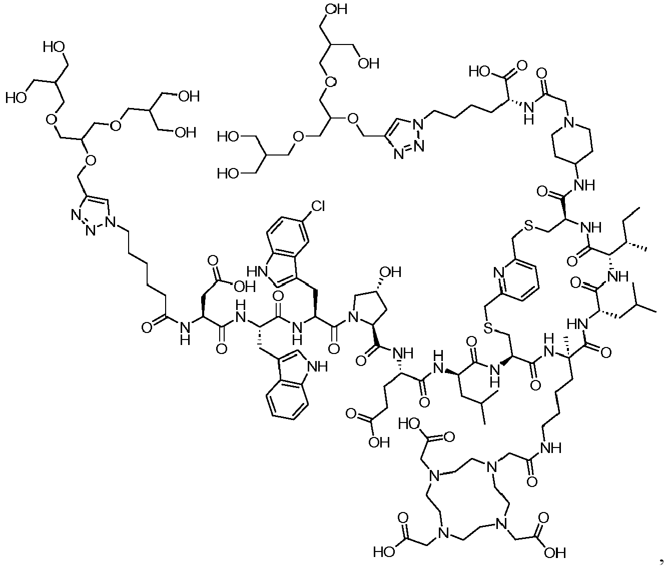
compound HAC:Ahx-Asp-Trp-5Clw-Tap-Glu-leu-[Cys(2Lut)-Apc(DOTA)-Leu-Ile-Cys]-APAc-lys:HAC (GIP-1125) of the following formula 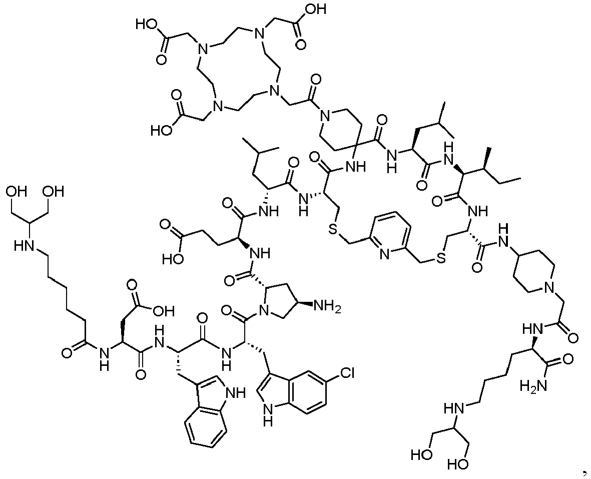
compound HAC:Hyx-Asp-Trp-5Clw-Tap-Glu-leu-[Cys(2Lut)-Apc(DOTA)-Leu-Ile-Cys]-APAc-prx:HAC (GIP-1126) of the following formula 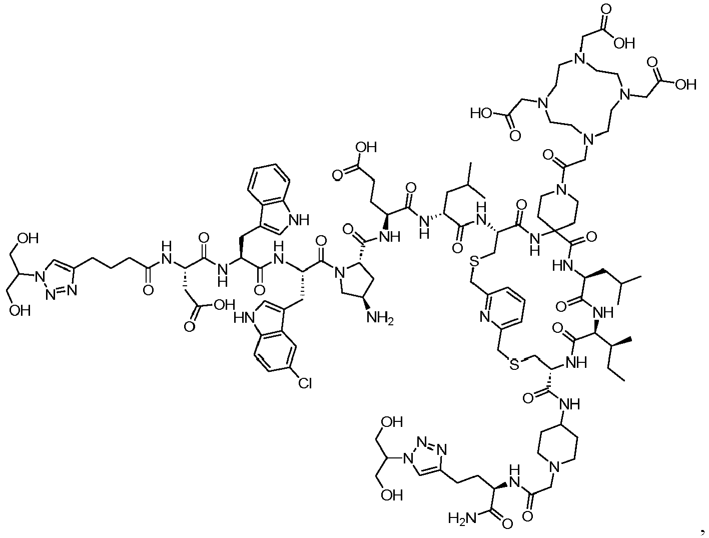
compound HAC:Ahx-Asp-Trp-5Clw-Tap-Glu-leu-[Cys(2Lut)-Amk(DOTA)-Leu-Ile-Cys]-APAc-lys:HAC (GIP-1127) of the following formula 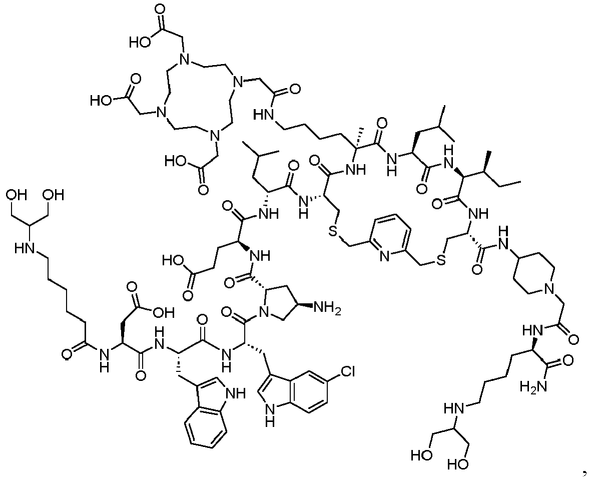
compound HAC:Hyx-Asp-Trp-5Clw-Tap-Glu-leu-[Cys(2Lut)-Amk(DOTA)-Leu-Ile-Cys]-APAc-prx:HAC (GIP-1128) of the following formula 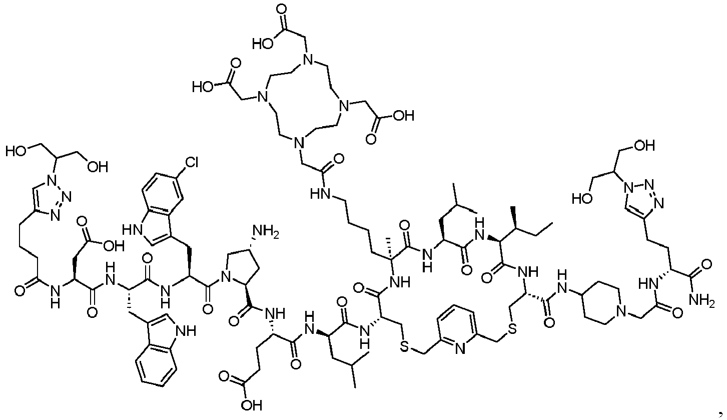
compound HAC:Ahx-Asp-Trp-5Clw-Hyp-Glu-leu-[Cys(2Lut)-Apc(DOTA)-Leu-Ile-Cys]-APAc-lys:HAC (GIP-1129) of the following formula 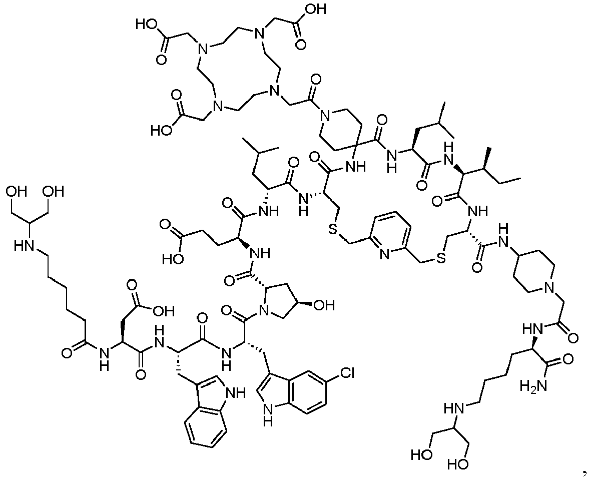
compound HAC:Hyx-Asp-Trp-5Clw-Hyp-Glu-leu-[Cys(2Lut)-Apc(DOTA)-Leu-Ile-Cys]-APAc-prx:HAC (GIP-1130) of the following formula 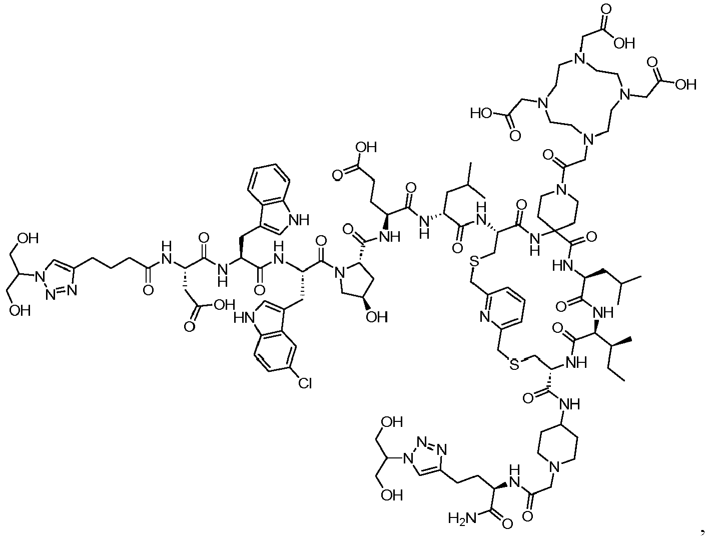
compound HAC:Ahx-Asp-Trp-5Clw-Hyp-Glu-leu-[Cys(2Lut)-Amk(DOTA)-Leu-Ile-Cys]-APAc-lys:HAC (GIP-1131) of the following formula 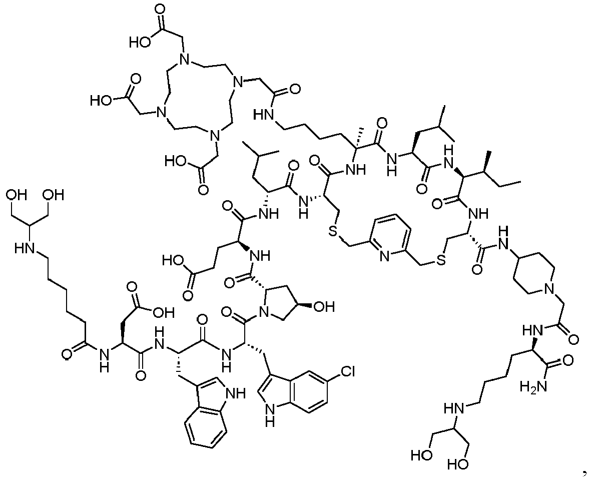
and compound HAC:Hyx-Asp-Trp-5Clw-Hyp-Glu-leu-[Cys(2Lut)-Amk(DOTA)-Leu-Ile-Cys]-APAc-prx:HAC (GIP-1132) of the following formula

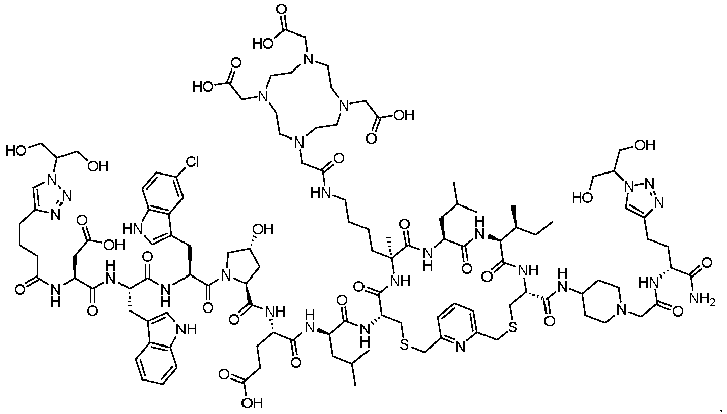

Embodiment 222. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220 and 221, preferably the compound of Embodiment 221, wherein the compound
is selected from the group consisting of GIP-1001 to GIP-1132,
preferably is selected from the group consisting of GIP-1001 to GIP-1124,
more preferably is selected from the group consisting of GIP-1001 to GIP-1088
and most preferably is selected from the group consisting of GIP-1001 to GIP-1056.

Embodiment 223. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221 and 222, preferably the compound of any one of Embodiments 221 and 222, more preferably the compound of Embodiment 222, wherein the compound
is selected from the group consisting of GIP-1001 to GIP-1056,
preferably is selected from the group consisting of GIP-1001 to GIP-1042,
more preferably is selected from the group consisting of GIP-1001 to GIP-1028
and most preferably is selected from the group consisting of GIP-1001 to GIP-1014.

Embodiment 224. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221 and 222, preferably the compound of any one of Embodiments 221 and 222, more preferably the compound of Embodiment 222, wherein the compound
is selected from the group consisting of GIP-1057 to GIP-1088,
preferably is selected from the group consisting of GIP-1057 to GIP-1080,
more preferably is selected from the group consisting of GIP-1057 to GIP-1072
and most preferably is selected from the group consisting of GIP-1057 to GIP-1064.

Embodiment 225. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223 and 224, wherein any S-atom, which can be oxidized, preferably any sulfur atom of a thioether group, is present as -S-, -S(O)- or -S(O₂)- or a mixture thereof.

Embodiment 226. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224 and 225, wherein the compound comprises a diagnostically active radionuclide, wherein the radionuclide
is a radionuclide used for diagnosis,
preferably is selected from the group comprising ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ¹⁷⁷Lu, ²⁰¹Tl, ²⁰³Pb, ¹⁸F, ⁷⁶Br, ⁷⁷Br, ¹²³I, ¹²⁴I, and ¹²⁵I,
more preferably is selected from the group comprising ⁴³Sc, ⁴⁴Sc, ⁶⁴Cu, ⁶⁷Ga, ⁶⁸Ga,
⁸⁶Y, ⁸⁹Zr, ¹¹¹In, ¹⁵²Tb, ¹⁵⁵Tb, and ²⁰³Pb,
   and
most preferably is selected from the group comprising ⁶⁴Cu, ⁶⁸Ga, ¹¹¹In and ²⁰³Pb.

Embodiment 227. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224 and 225, wherein the compound comprises a therapeutically active radionuclide, wherein the radionuclide
is a radionuclide used for therapy,
preferably is selected from the group comprising ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu , ¹⁸⁶Re, ¹⁸⁸Re, ²¹²Pb, ²¹³Bi, ²²³Ra, ²²⁵Ac, ²²⁶Th, ²²⁷Th, ¹³¹I, and ²¹¹At,
more preferably is selected from the group comprising ⁴⁷Sc, ⁶⁷Cu, ⁹⁰Y, ¹⁷⁷Lu, ²¹²Pb, ²¹³Bi, ²²⁵Ac, and ²²⁷Th,
and most preferably is selected from the group comprising ⁹⁰Y, ¹⁷⁷Lu, ²¹²Pb, ²²⁵Ac, and ²²⁷Th.

Embodiment 228. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225 and 226, preferably the compound of Embodiment 226, for use in a method of diagnosing a disease.

Embodiment 229. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227, preferably the compound of Embodiment 227, for use in a method for the treatment of a disease.

Embodiment 230. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227, for use in a method for the identification of a subject, wherein the subject is likely to respond or likely not to respond to a treatment of a disease, wherein the method for the identification of a subject comprises carrying out a method of diagnosing a disease using a compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227.

Embodiment 231. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227, for use in a method for the selection of a subject from a group of subjects, wherein the subject is likely to respond or likely not to respond to a treatment of a disease, wherein the method for the selection of a subject from a group of subjects comprises carrying out a method of diagnosing a disease using a compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227.

Embodiment 232. The compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227, for use in a method for the stratification of a group of subjects into subjects which are likely to respond to a treatment of a disease, and into subjects which are not likely to respond to a treatment of a disease, wherein the method for the stratification of a group of subjects comprises carrying out a method of diagnosing a disease using a compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227.

Embodiment 233. The compound for use of any one of Embodiments 228, 229, 230, 231 and 232, wherein the disease is cancer.

Embodiment 234. The compound for use of Embodiment 233, wherein the cancer is expressing Gastric Inhibitory Peptide Receptor (GIPR), preferably, the cancer is overexpressing GIPR.

Embodiment 235. The compound for use of any one of Embodiments 233 and 234, wherein the cancer is a neuroendocrine cancer or a neuroendocrine neoplasm.

Embodiment 236. The compound for use of any one of Embodiment 233, 234 and 235, wherein the cancer is selected from the group consisting of medullary thyroid cancer, gastrointestinal neuroendocrine neoplasms, ileal neuroendocrine neoplasms, pancreatic neuroendocrine neoplasms, nonfunctioning neuroendocrine neoplasms, Insulinomas, Gastrinomas, Glucagonomas, VIPomas, Somatostatinoma, ACTHoma, lung neuroendocrine neoplasms, typical carcinoid tumors, atypical carcinoid tumors, small cell carcinoma of the lung, large cell carcinoma of the lung, thymic neuroendocrine tumors, Merkel cell carcinoma, Pheochromocytoma of the adrenal gland, adrenal cancer, parathyroid cancer, paraganglioma, pituitary gland tumors, neuroendocrine tumors of the ovaries and neuroendocrine tumors of the testicles.

Embodiment 237. The compound for use of any one of Embodiments 228, 229, 230, 231 and 232, wherein the disease is a metabolic disease.

Embodiment 238. The compound for use of Embodiment 237, wherein a diseased cell and/or a diseased tissue, preferably a diseased cell and/or a diseased tissue of the metabolic disease expresses GIPR.

Embodiment 239. The compound for use of Embodiment 238, wherein the diseased cell and/or the diseased tissue overexpresses GIPR.

Embodiment 240. The compound for use of any one of Embodiments 238 and 239, wherein the diseased cell is a pancreatic cell.

Embodiment 241. The compound for use of any one of Embodiments 237, 238, 239 and 240, wherein the metabolic disease is selected from the group consisting of type 2 diabetes, type 1 diabetes, metabolic syndrome, insulin resistance, dyslipidemia, impaired fasting glucose, and impaired glucose tolerance.

Embodiment 242. A composition comprising a compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227 and a pharmaceutically acceptable excipient.

Embodiment 243. The composition of Embodiment 242, wherein the composition is a pharmaceutical composition.

Embodiment 244. A kit comprising a compound of any one of Embodiments 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226 and 227 and one or more optional excipient(s) and optionally one or more device(s).

Embodiment 245. The kit of Embodiment 244, wherein the device(s) is/are selected from the group comprising a labeling device, a purification device, a handling device, a radioprotection device, an analytical device or an administration device.

In accordance with the present invention, the compound of the present invention as subject to Embodiment 1 will also be referred to herein as the first aspect of the present invention.

In accordance with the present invention, the compound of the present invention according to the first aspect of the present invention, including any embodiment thereof, for use in a method of diagnosing a disease as subject to Embodiment 228 will also be referred to herein as the second aspect of the present invention.

In accordance with the present invention, the compound of the present invention according to the first aspect of the present invention, including any embodiment thereof, for use in a method for the treatment of a disease as subject to Embodiment 229 will also be referred to herein as the third aspect of the present invention.

In accordance with the present invention, the compound of the present invention according to the first aspect including any embodiment thereof, for use in a method for the identification of a subject, wherein the subject is likely to respond or likely not to respond to a treatment of a disease, wherein the method for the identification of a subject comprises carrying out a method of diagnosing a disease using a compound according to the first aspect of the present invention, including any embodiment thereof, as subject to Embodiment 230 will also be referred to herein as the fourth aspect of the present invention.

In accordance with the present invention, the compound of the present invention according to the first aspect including any embodiment thereof, for use in a method for the selection of a subject from a group of subjects, wherein the subject is likely to respond or likely not to respond to a treatment of a disease, wherein the method for the selection of a subject from a group of subjects comprises carrying out a method of diagnosing a disease using a compound according to the first aspect of the present invention, including any embodiment thereof, as subject to Embodiment 231 will also be referred to herein as the fifth aspect of the present invention.

In accordance with the present invention, the compound of the present invention according to the first aspect including any embodiment thereof, for use in a method for the stratification of a group of subjects into subjects which are likely to respond to a treatment of a disease, and into subjects which are not likely to respond to a treatment of a disease, wherein the method for the stratification of a group of subjects comprises carrying out a method of diagnosing a disease using a compound according to the first aspect of the present invention, including any embodiment thereof, as subject to Embodiment 232will also be referred to herein as the sixth aspect of the present invention.

In accordance with the present invention, the composition as subject to Embodiment 242 comprising a compound according to the first aspect of the present invention, including any embodiment thereof, and a pharmaceutically acceptable excipient will also be referred to herein as the seventh aspect of the present invention.

In accordance with the present invention, the kit as subject to Embodiment 242 comprising a compound according to the first aspect of the present invention, including any embodiment thereof, and one or more optional excipient(s) and optionally one or more device(s) will also be referred to herein as the eighth aspect of the present invention.

According to the present invention, each and any embodiment of the compound of the first aspect is also an embodiment of the peptide of the second aspect, and vice versa.

The present inventors have surprisingly found that the compounds of the invention show a high affinity to Gastric Inhibitory Peptide Receptor (GIPR). Furthermore, the present inventors have surprisingly found that compounds of the invention show other characteristics, which predestine the compounds of the invention for use in the diagnosis and therapy of diseases involving Gastric Inhibitory Peptide Receptor (GIPR). Such other characteristics comprise high stability in plasma and selectivity for Gastric Inhibitory Peptide Receptor (GIPR). Another surprising characteristic of the compounds of the invention is a low retention in normal, i.e. non-diseased tissues.

According to the present invention, the compounds of the invention may comprise a bio-distribution modifier comprised by an N-terminal modification group A and a bio-distribution modifier comprised by a C-terminal modification group C-term, which is/are either directly or by means of a linker attached. More preferably, the chelator is attached to Xaa8.

In an embodiment and as preferably used herein, a diagnostically active compound is a compound, which is suitable for or useful in the diagnosis of a disease.

In an embodiment and as preferably used herein, a diagnostic agent or a diagnostically active agent is a compound, which is suitable for or useful in the diagnosis of a disease.

In an embodiment and as preferably used herein, a therapeutically active compound is a compound, which is suitable for or useful in the treatment of a disease.

In an embodiment and as preferably used herein, a therapeutic agent or a therapeutically active agent is a compound, which is suitable for or useful in the treatment of a disease.

In an embodiment and as preferably used herein, a theragnostically active compound is a compound, which is suitable for or useful in both the diagnosis and therapy of a disease.

In an embodiment and as preferably used herein, a theragnostic agent or a theragnostically active agent is a compound, which is suitable for or useful in both the diagnosis and therapy of a disease.

In an embodiment and as preferably used herein, theragnostics is a method for the combined diagnosis and therapy of a disease; preferably, the combined diagnostically and therapeutically active compounds used in theragnostics are radiolabeled.

In an embodiment and as preferably used herein, treatment of a disease is treatment and/or prevention of a disease.

In an embodiment and as preferably used herein, pEC50 is determined in a FACS binding assay, wherein the FACS binding assay is as described in the example part.

In an embodiment and as preferably used herein, pIC50 is determined in a FACS binding assay, wherein the FACS binding assay is as described in the example part.

In an embodiment and as preferably used herein, a disease involving GIPR is a disease, wherein cells including but not limited to tumor cells expressing or pancreatic beta cells, preferably in an upregulated manner, GIPR and tissue either expressing GIPR, preferably in an upregulated manner respectively, are either a or the cause for the disease and/or the symptoms of the disease, or are part of the pathology underlying the disease. A preferred GIPR-expressing cell is a tumor cell. In an embodiment of the disease, preferably when used in connection with the treatment, treating and/or therapy of the disease, affecting the cells, the tissue and pathology, respectively, results in cure, treatment or amelioration of the disease and/or the symptoms of the disease. In an embodiment of the disease, preferably when used in connection with the diagnosis and/or diagnosing of the disease, labeling of the GIPR-expressing cells and/or of the GIPR-expressing tissue allows discriminating or distinguishing said cells and/or said tissue from healthy or GIPR-non-expressing cells and/or healthy or GIPR-non-expressing tissue. More preferably such discrimination or distinction forms the basis for said diagnosis and diagnosing, respectively. In an embodiment thereof, labeling means the interaction of a detectable label either directly or indirectly with the GIPR-expressing cells and/or with the GIPR-expressing tissue or tissue containing such GIPR-expressing cells; more preferably such interaction involves or is based on the interaction of the label or a compound bearing such label with GIPR.

In an embodiment and as preferably used herein, a target cell is a cell, which is expressing GIPR and is a or the cause for a disease and/or the symptoms of a disease, or is part of the pathology underlying a disease.

In an embodiment and as preferably used herein, a non-target cell is a cell, which is either not expressing GIPR and/or is not a or the cause for a disease and/or the symptoms of a disease, or is part of the pathology underlying a disease.

In an embodiment and as preferably used herein, a neoplasm is an abnormal new growth of cells. The cells in a neoplasm grow more rapidly than normal cells and will continue to grow if not treated. A neoplasm may be benign or malignant.

In an embodiment and as preferably used herein, a tumor is a mass lesion that may be benign or malignant.

In an embodiment and as preferably used herein, a cancer is a malignant neoplasm.

In an embodiment and as preferably used herein, a cell and/or tissue expressing GIPR is overexpressing GIPR. More preferably, GIPR is overexpressed if the extent of expression is one observed in a cell and/or tissue of a person diagnosed to suffer from the disease, preferably the disease being cancer and metabolic disease as disclosed herein.

Accordingly, the compounds of the invention are thus particularly suitable for and useful in the diagnosis and treatment, respectively, of these diseases. Insofar, the above indications are indications, which can be treated by the compound of the invention. It will be understood by the person skilled in the art that also metastases and metastases of the above indications in particular can be treated and diagnosed by the compound of the invention and the methods of diagnosis and methods of treatment making use of the compound of the invention.

It is also within the present invention that the compound of the invention is used or is for use in a method for the treatment of a disease as disclosed herein. Such method, preferably, comprises the step of administering to a subject in need thereof a therapeutically effective amount of the compound of the invention. Such method includes, but is not limited to, curative or adjuvant cancer treatment. It is used as palliative treatment, wherein cure is not possible and the aim is for local disease control or symptomatic relief or as therapeutic treatment, wherein the therapy has survival benefit and it can be curative.

The method for the treatment of a disease as disclosed herein, includes the treatment of the disease disclosed herein, including tumors and cancer as well as metabolic diseases, and may be used either as the primary therapy or as second, third, fourth or last line therapy. It is also within the present invention to combine the compound of the invention with further therapeutic approaches. It is well known to the person skilled in the art that the precise treatment intent including curative, adjuvant, neoadjuvant, therapeutic, or palliative treatment intent will depend on the tumor type, location, and stage, as well as the general health of the patient.

Without wishing to be bound by any theory, the therapeutic effect of the compounds of present invention is based on the delivery of a radionuclide to a diseased GIPR expressing cell or structure, which is destroyed by the radiation emitted by the radionuclide. Preferably, GIPR is overexpressed by such cell or structure.

Without wishing to be bound by any theory, the therapeutic use of the compounds of the invention arises from the binding of said compounds to GIPR expressing cells, cancer cells in particular, wherein said cells are killed by the radiation emitted by the radionuclide. It will also be appreciated by a person skilled in the art that GIPR is a pan-neuroendocrine-tumor target. Because of this, any respective cancer and tumor can be treated and diagnosed, respectively.

In an embodiment of the present invention, the disease is selected from the group comprising a cancer and tumor, respectively, expressing GIPR, preferably over-expressing GIPR. In a preferred embodiment, the cancer is a neuroendocrine cancer or neuroendocrine tumor, preferably each and any expressing or overexpressing GIPR.

In a further embodiment, the diseases is selected from the group comprising medullary thyroid cancer, gastrointestinal neuroendocrine neoplasms, ileal neuroendocrine neoplasms, pancreatic neuroendocrine neoplasms, nonfunctioning neuroendocrine neoplasms, Insulinomas, Gastrinomas, Glucagonomas, VIPomas, Somatostatinoma, ACTHoma, lung neuroendocrine neoplasms, typical carcinoid tumors, atypical carcinoid tumors, small cell carcinoma of the lung, large cell carcinoma of the lung, thymic neuroendocrine tumors, Merkel cell carcinoma, Pheochromocytoma of the adrenal gland, adrenal cancer, parathyroid cancer, paraganglioma, pituitary gland tumors, neuroendocrine tumors of the ovaries and neuroendocrine tumors of the testicles.

In a further embodiment, the disease is a metabolic disease. Preferably, the metabolic disease is one where GIPR is expressed, more preferably where GIPR is overexpressed in pancreatic beta cells. In a still further embodiment, the disease is selected from the group comprising metabolic diseases and disorders, preferably type 2 diabetes, type 1 diabetes, metabolic syndrome, insulin resistance, dyslipidemia, impaired fasting glucose, and impaired glucose tolerance.

In an embodiment, the compounds of the present invention are used to detect cells and tissues overexpressing the GIPR, whereby such detection is achieved by conjugating a detectable label to the compounds of the invention, preferably a detectable radionuclide. In a preferred embodiment, the cells and tissues detected are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g., neoplasms, tumors, and cancers).

In another embodiment, the compounds of the present invention are used to treat cells and tissues overexpressing the GIPR. In a preferred embodiment, the cells and tissues treated are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g. neoplasms, tumors, and cancers) and the therapeutic activity is achieved by conjugating therapeutically active effector to the compounds of the present invention, preferably a therapeutically active radionuclide.

An effective amount is a dosage of the compound sufficient to provide a therapeutically or medically desirable result or effect in the subject to which the compound is administered. The effective amount will vary with the particular condition being treated, the age and physical condition of the subject being treated, the severity of the condition, the duration of the treatment, the nature of the concurrent or combination therapy (if any), the specific route of administration and like factors within the knowledge and expertise of the health practitioner. For example, in connection with methods directed towards treating subjects having a condition characterized by abnormal cell proliferation, an effective amount to inhibit proliferation would be an amount sufficient to reduce or halt altogether the abnormal cell proliferation so as to slow or halt the development of or the progression of a cell mass such as, for example, a tumor. As used in the embodiments, "inhibit" embraces all of the foregoing.

In other embodiments, a therapeutically effective amount will be an amount necessary to extend the dormancy of micrometastases or to stabilize any residual primary tumor cells following surgical or drug therapy.

In a preferred embodiment, the compound of the present invention is for use in the treatment and/or prevention of a disease, whereby such treatment is targeted radionuclide therapy. Targeted radionuclide therapy is a form of radiation therapy (also called radiotherapy) using molecules labeled with a radionuclide to deliver a toxic level of radiation to sites of disease. Targeted radionuclide therapy may be applied systemically or locally. In contrast, in external beam radiation therapy a source outside of the body is producing a high-energy beam, which is then focused at sites of disease, passing through the skin into the body. It is as well distinguished from internal radiation therapy (brachytherapy), where a radioactive implant is placed at or near the site of disease.

Preferably, radionuclide therapy makes use of or is based on different forms of radiation emitted by a radionuclide. Such radiation can, for example, be any one of alpha (α), beta (β) or gamma (γ) radiation caused by the emission of photons, emission of electrons including but not limited to β⁻-particles and Auger-electrons, emission of protons, emission of neutrons, emission of positrons or emission of α-particles. Depending on the kind of particle or radiation emitted by said radionuclide, radionuclide therapy can, for example, be distinguished as β-particle radionuclide therapy, α-particle radionuclide therapy or Auger electron radionuclide therapy. All of these forms of radionuclide therapy are encompassed by the present invention, and all of these forms of radionuclide therapy can be realized by the compound of the invention, preferably under the proviso that the radionuclide attached to the compound of the invention, more preferably as an effector, is providing for this kind of radiation.

Radionuclide therapy preferably works by damaging the DNA of cells. The damage is caused by a β-particle, α-particle, or Auger electron directly or indirectly ionizing the atoms which make up the DNA chain. Indirect ionization happens as a result of the ionization of water, forming free radicals, notably hydroxyl radicals, which then damage the DNA.

In the most common forms of radionuclide therapy, most of the radiation effect is through free radicals. Because cells have mechanisms for repairing DNA damage, breaking the DNA on both strands proves to be the most significant technique in modifying cell characteristics. Because cancer cells generally are undifferentiated and stem cell-like, they reproduce more, and have a diminished ability to repair sub-lethal damage compared to most healthy differentiated cells. The DNA damage is inherited through cell division, accumulating damage to the cancer cells, causing them to die or reproduce more slowly.

Oxygen is a potent radiosensitizer, increasing the effectiveness of a given dose of radiation by forming DNA-damaging free radicals. Therefore, use of high-pressure oxygen tanks, blood substitutes that carry increased oxygen, hypoxic cell radiosensitizers such as misonidazole and metronidazole, and hypoxic cytotoxins, such as tirapazamine may be applied.

The total radioactive dose may be fractionated, i.e. spread out over time in one or more treatments for several important reasons. Fractionation allows normal cells time to recover, while tumor cells are generally less efficient in repair between fractions. Fractionation also allows tumor cells that were in a relatively radio-resistant phase of the cell cycle during one treatment to cycle into a sensitive phase of the cycle before the next fraction is given.

It is generally known that different cancers respond differently to radiation therapy. The response of a cancer to radiation is described by its radiosensitivity. Highly radiosensitive cancer cells are rapidly killed by modest doses of radiation. These include leukemias, most lymphomas, and germ cell tumors.

Radionuclide therapy is in itself painless. Many low-dose palliative treatments cause minimal or no side effects. Treatment to higher doses may cause varying side effects during treatment (acute side effects), in the months or years following treatment (long-term side effects), or after re-treatment (cumulative side effects). The nature, severity, and longevity of side effects depends on the organs that receive the radiation, the treatment itself (type of radionuclide, dose, fractionation, concurrent chemotherapy), and the patient.

It is within the present inventions that the method for the treatment of a disease of the invention may realize each and any of the above strategies, which are as such known in the art, and which insofar constitute further embodiments of the invention.

It is also within the present invention that the compound of the invention is used in a method for the diagnosis of a disease as disclosed herein. Such method, preferably, comprises the step of administering to a subject in need thereof a diagnostically effective amount of the compound of the invention.

In accordance with the present invention, an imaging method is selected from the group consisting of scintigraphy, Single Photon Emission Computed Tomography (SPECT) and Positron Emission Tomography (PET).

Scintigraphy is a form of diagnostic test or method used in nuclear medicine, wherein radiopharmaceuticals are internalized by cells, tissues and/or organs, preferably internalized *in vivo,* and radiation emitted by said internalized radiopharmaceuticals is captured by external detectors (gamma cameras) to form and display two-dimensional images. In contrast thereto, SPECT and PET forms and displays three-dimensional images. Because of this, SPECT and PET are classified as separate techniques to scintigraphy, although they also use gamma cameras to detect internal radiation. Scintigraphy is unlike a diagnostic X-ray where external radiation is passed through the body to form an image.

Single Photon Emission Tomography (SPECT) scans are a type of nuclear imaging technique using gamma rays. They are very similar to conventional nuclear medicine planar imaging using a gamma camera. Before the SPECT scan, the patient is injected with a radiolabeled compound emitting gamma rays that can be detected by the scanner. A computer collects the information from the gamma camera and translates this into two-dimensional cross-sections. These cross-sections can be added back together to form a three-dimensional image of an organ or a tissue. SPECT involves detection of gamma rays emitted singly, and sequentially, by the radionuclide provided by the radiolabeled compound. To acquire SPECT images, the gamma camera is rotated around the patient. Projections are acquired at defined points during the rotation, typically every 3 - 6 degrees. In most cases, a full 360 degree rotation is used to obtain an optimal reconstruction. The time taken to obtain each projection is also variable, but 15-20 seconds is typical. This gives a total scan time of 15 - 20 minutes. Multi-headed gamma cameras are faster. Since SPECT acquisition is very similar to planar gamma camera imaging, the same radiopharmaceuticals may be used.

Positron Emitting Tomography (PET) is a non-invasive, diagnostic imaging technique for measuring the biochemical, physiological and pathophysiological processes within the human body. PET is unique since it is able to produce images of the body's basic biochemistry or functions. Traditional diagnostic techniques, such as X-rays, CT scans, or MRI, produce images of the body's anatomy or structure. The premise with these techniques is that any changes in structure or anatomy associated with a disease can be seen. Biochemical and physiological processes are also altered by a disease, and may occur before any gross changes in anatomy. PET is an imaging technique that can visualize some of these early biochemical and physiological changes. PET scanners rely on radiation emitted from the patient to create the images. Each patient is given a minute amount of a radioactive compound that either closely resembles a natural substance used by the body or binds specifically to a receptor or molecular structure. As the radioisotope undergoes positron emission decay (also known as positive beta decay), it emits a positron, the antiparticle counterpart of an electron. After traveling up to a few millimeters, the positron encounters an electron and annihilates, producing a pair of annihilation (gamma) photons moving in opposite directions. These are detected when they reach a scintillation material in the scanning device, creating a burst of light, which is detected by photomultiplier tubes or silicon avalanche photodiodes. The technique depends on simultaneous or coincident detection of the pair of photons. Photons that do not arrive in pairs, i.e., within a few nanoseconds, are ignored. All coincidences are forwarded to the image processing unit where the final image data is produced using image reconstruction procedures.

SPECT/CT and PET/CT is the combination of SPECT and PET with computed tomography (CT). The key benefits of combining these modalities are improving the reader's confidence and accuracy. With traditional PET and SPECT, the limited number of photons emitted from the area of abnormality produces a very low-level background that makes it difficult to anatomically localize the area. Adding CT helps determine the location of the abnormal area from an anatomic perspective and categorize the likelihood that this represents a disease.

It is within the present inventions that the method for the diagnosis of a disease of the invention may realize each and any of the above strategies which are as such known in the art, and which insofar constitute further embodiments of the invention.

In an embodiment and as preferably used herein, a linkage is an attachment of two atoms of two independent moieties. A preferred linkage is a chemical bond or a plurality of chemical bonds. More preferably a chemical bond is a covalent bond or a plurality of chemical bonds. Most preferably the linkage is a covalent bond or a coordinate bond. As preferably used herein, an embodiment of a coordinate bond is a bond or group of bonds as realized when a metal is bound by a chelator. Depending on the type of atoms linked and their atomic environment different types of linkages are created. These types of linkage are defined by the type of atom arrangements created by the linkage.

For instance, the linking of a moiety comprising an amine with a moiety comprising a carboxylic acid leads to a linkage named amide (which is also referred to as amide linkage, -CO-N-, -N-CO-). It will be acknowledged by a person skilled in the art that this and the following examples of creating linkages are only prototypical examples and are by no means limiting the scope of the instant application. It will be acknowledged by a person in the art that the linking of a moiety comprising an isothiocyanate with a moiety comprising an amine leads to thiourea (which is also referred to as a thiourea linkage, -N-C(=S)-N-), and linking of a moiety comprising a C atom with a moiety comprising a thiol-group (-C-SH) leads to thioether (which is also referred to as a thioether linkage, -C-S-C-). A non-limiting list of linkages as preferably used in connection with the chelator and linker of the invention and their characteristic type of atom arrangement is presented Table 1.

**Table 1:**

| **Linkage** | **Characteristic atom arrangement** | **Linkage** | **Characteristic atom arrangement** |
|---|---|---|---|
| Amide | 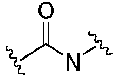 | Ether | 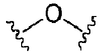 |
| Sulfonamide | 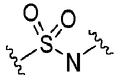 | Ester | 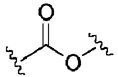 |
| Urea | 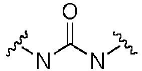 | Carbamate | 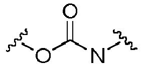 |
| Thioether | 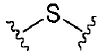 | Thiourea | 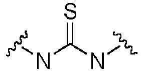 |
| Disulfide | 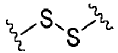 | Triazole | 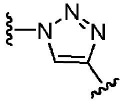 |

Examples of reactive groups which, in some embodiments of the invention, are used in the formation of linkages between the chelator and linker, directly between the chelator and the compound of the invention, the bio-distribution modifier and linker or the bio-distribution modifier and the compound of the invention are summarized in Table 2. It will, however, be understood by a person skilled in the art that neither the linkages which may be realized in embodiments for the formation of the conjugates of the invention are limited to the ones of Table 2 nor the reactive groups forming such linkages.

**Table 2:**

| **first reactive group** | **second reactive group** | **(type of) linkage** |
|---|---|---|
| amino | carboxylic acid | amide |
| amino | activated carboxylic acid | amide |
| amino | sulfonyl halide | sulfonamide |
| amino | isocyanate | urea |
| amino | p-nitrophenylcarbamate | urea |
| sulfhydryl | Michael acceptor (e.g. Maleimide) | thioether |
| bromo | sulfhydryl | thioether |
| isothiocyanate | amino | thiourea |
| hydroxyl | carboxylic acid | ester |
| azide | alkyne | triazole |
| sulfhydryl | sulfhydryl | disulfide |
| sulfhydryl | 2-Pyridine-di sulfide | disulfide |
| carbonate ester | amino | carbamate |
| chloroformate | amino | carbamate |
| bromo | hydroxyl | ether |

The following are reactive groups and functionalities which are utilized or amenable of forming linkages between moieties or structures as used in embodiments of the conjugate of the invention: Primary or secondary amino, carboxylic acid, activated carboxylic acid, chloro, bromo, iodo, sulfhydryl, hydroxyl, sulfonic acid, activated sulfonic acid, sulfonic acid esters like mesylate or tosylate, Michael acceptors, strained alkenes like *trans* cyclooctene, isocyanate, isothiocyanate, azide, alkyne and tetrazine.

As preferably used herein, the term "activated carboxylic acid" refers to a carboxylic acid group with the general formula -CO-X, wherein X is a leaving group. For example, activated forms of a carboxylic acid group may include, but are not limited to, acyl chlorides, symmetrical or unsymmetrical anhydrides, and esters. In some embodiments, the activated carboxylic acid group is an ester with pentafluorophenol, nitrophenol, benzotriazole, azabenzotriazole, thiophenol or N-hydroxysuccinimide (NHS) as leaving group.

In an embodiment and as preferably used herein the term "mediating a linkage" means that a linkage or a type of linkage is established, preferably a linkage between two moieties. In a preferred embodiment the linkage and the type of linkage is as defined herein.

To the extent it is referred in the instant application to a range indicated by a lower integer and a higher integer such as, for example, 1-4, such range is a representation of the lower integer, the higher integer and any integer between the lower integer and the higher integer. Insofar, the range is actually an individualized disclosure of said integer. In said example, the range of 1-4 thus means 1, 2, 3 and 4.

In an embodiment and as preferably used herein, (C₁-C₂)alkyl means each and individually any of methyl and ethyl.

In an embodiment and as preferably used herein, the expression alkyl refers each and individually to a saturated, straight-chain or branched hydrocarbon group and is usually accompanied by a qualifier which specifies the number of carbon atoms it may contain. For example the expression (C₁-C₃)alkyl means each and individually any of methyl, ethyl, n-propyl and isopropyl.

In an embodiment and as preferably used herein, (C₁-C₃)alkyl means each and individually any of methyl, ethyl, n-propyl and isopropyl.

In an embodiment and as preferably used herein, (C₁-C₄)alkyl refers to a saturated or unsaturated, straight-chain or branched hydrocarbon group having from 1 to 4 carbon atoms. Representative examples of (C₁-C₄)alkyl include, but are not limited to, any of methyl, ethyl, n-propyl, isopropyl, n-butyl, isobutyl, sec-butyl and tert-butyl.

In an embodiment and as preferably used herein, (C₃-C₅)alkyl refers to a saturated or unsaturated, straight-chain or branched hydrocarbon group having from 3 to 5 carbon atoms. Representative examples of (C₃-C₅)alkyl include, but are not limited to, any of n-propyl, isopropyl, n-butyl, isobutyl, *sec*-butyl, *tert*-butyl, n-pentyl, 2-pentyl, 2-methyl-butyl, 3-methyl-butyl, 3-pentyl, 3-methyl-but-2-yl, 2-methyl-but-2-yl and 2,2-dimethylpropyl. In a more preferred embodiment and as preferably used herein, (C₃-C₅)alkyl refers to isopropyl, isobutyl, *tert*-butyl and 2,2-dimethylpropyl.

In an embodiment and as preferably used herein, (C₁-C₆)alkyl refers to a saturated or unsaturated, straight-chain or branched hydrocarbon group having from 1 to 6 carbon atoms. Representative examples of (C₁-C₆)alkyl include, but are not limited to, any of methyl, ethyl, n-propyl, isopropyl, n-butyl, isobutyl, sec-butyl, *tert*-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl and 3,3-dimethyl-but-2-yl. A (C₁-C₆)alkyl group can be unsubstituted or substituted with one or more groups, including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, (C₁-C₈)alkyl refers to a saturated or unsaturated, straight-chain or branched hydrocarbon group having from 1 to 8 carbon atoms. Representative (C₁-C₈)alkyl groups include, but are not limited to, any of methyl, ethyl, n-propyl, isopropyl, n-butyl, isobutyl, sec-butyl, *tert*-butyl, n-pentyl, 2-pentyl, 2-methyl-butyl, 3-methyl-butyl, 3-pentyl, 3-methyl-but-2-yl, 2-methyl-but-2-yl, 2,2-dimethylpropyl, n-hexyl, 2-hexyl, 2-methyl-pentyl, 3-methyl-pentyl, 4-methyl-pentyl, 3-hexyl, 2-ethyl-butyl, 2-methyl-pent-2-yl, 2,2-dimethyl-butyl, 3,3-dimethyl-butyl, 3-methyl-pent-2-yl, 4-methyl-pent-2-yl, 2,3-dimethyl-butyl, 3-methyl-pent-3-yl, 2-methyl-pent-3-yl, 2,3-dimethyl-but-2-yl, 3,3-dimethyl-but-2-yl, n-heptyl, 2-heptyl, 2-methyl-hexyl, 3-methyl-hexyl, 4-methyl-hexyl, 5-methyl-hexyl, 3-heptyl, 2-ethyl-pentyl, 3-ethyl-pentyl, 4-heptyl, 2-methyl-hex-2-yl, 2,2-dimetyhl-pentyl, 3,3-dimetyhl-pentyl, 4,4-dimetyhl-pentyl, 3-methyl-hex-2-yl, 4-methyl-hex-2-yl, 5-methyl-hex-2-yl, 2,3-dimethyl-pentyl, 2,4-dimethyl-pentyl, 3,4-dimethyl-pentyl, 3-methyl-hex-3-yl, 2-ethyl-2-methyl-butyl, 4-methyl-hex-3-yl, 5-methyl-hex-3-yl, 2-ethyl-3-methyl-butyl, 2,3-dimethyl-pent-2-yl, 2,4-dimethyl-pent-2-yl, 3,3-dimethyl-pent-2-yl, 4,4-dimethyl-pent-2-yl, 2,2,3-trimethyl-butyl, 2,3,3-trimethyl-butyl, 2,3,3-trimethyl-but-2-yl, n-octyl, 2-octyl, 2-methyl-heptyl, 3-methyl-heptyl, 4-methyl-heptyl, 5-methyl-heptyl, 6-methyl-heptyl, 3-octyl, 2-ethyl-hexyl, 3-ethyl-hexyl, 4-ethyl-hexyl, 4-octyl, 2-propyl-pentyl, 2-methyl-hept-2-yl, 2,2-dimethyl-hexyl, 3,3-dimethyl-hexyl, 4,4-dimethyl-hexyl, 5,5-dimethyl-hexyl, 3-methyl-hept-2-yl, 4-methyl-hept-2-yl, 5-methyl-hept-2-yl, 6-methyl-hept-2-yl, 2,3-dimethyl-hex-1-yl, 2,4-dimethyl-hex-1-yl, 2,5-dimethyl-hex-1-yl, 3,4-dimethyl-hex-1-yl, 3,5-dimethyl-hex-1-yl, 3,5-dimethyl-hex-1-yl, 3-methyl-hept-3-yl, 2-ethyl-2-methyl-1-yl, 3-ethyl-3-methyl-1-yl, 4-methyl-hept-3-yl, 5-methyl-hept-3-yl, 6-methyl-hept-3-yl, 2-ethyl-3-methyl-pentyl, 2-ethyl-4-methyl-pentyl, 3-ethyl-4-methyl-pentyl, 2,3-dimethyl-hex-2-yl, 2,4-dimethyl-hex-2-yl, 2,5-dimethyl-hex-2-yl, 3,3-dimethyl-hex-2-yl, 3,4-dimethyl-hex-2-yl, 3,5-dimethyl-hex-2-yl, 4,4-dimethyl-hex-2-yl, 4,5-dimethyl-hex-2-yl, 5,5-dimethyl-hex-2-yl, 2,2,3-trimethyl-pentyl, 2,2,4-trimethyl-pentyl, 2,3,3-trimethyl-pentyl, 2,3,4-trimethyl-pentyl, 2,4,4-trimethyl-pentyl, 3,3,4-trimethyl-pentyl, 3,4,4-trimethyl-pentyl, 2,3,3-trimethyl-pent-2-yl, 2,3,4-trimethyl-pent-2-yl, 2,4,4-trimethyl-pent-2-yl, 3,4,4-trimethyl-pent-2-yl, 2,2,3,3-tetramethyl-butyl, 3,4-dimethyl-hex-3-yl, 3,5-dimethyl-hex-3-yl, 4,4-dimethyl-hex-3-yl, 4,5-dimethyl-hex-3-yl, 5,5-dimethyl-hex-3-yl, 3-ethyl-3-methyl-pent-2-yl, 3-ethyl-4-methyl-pent-2-yl, 3-ethyl-hex-3-yl, 2,2-diethyl-butyl, 3-ethyl-3-methyl-pentyl, 4-ethyl-hex-3-yl, 5-methyl-hept-3-yl, 2-ethyl-3-methyl-pentyl, 4-methyl-hept-4-yl, 3-methyl-hept-4-yl, 2-methyl-hept-4-yl, 3-ethyl-hex-2-yl, 2-ethyl-2-methyl-pentyl, 2-isopropyl-pentyl, 2,2-dimethyl-hex-3-yl, 2,2,4-trimethyl-pent-3-yl and 2-ethyl-3-methyl-pentyl. A (C₁-C₈)alkyl group can be unsubstituted or substituted with one or more groups, including, but not limited to, (C₁-C₈)alkyl, -O-[(C₁-C₈)alkyl], -aryl, -CO-R', -O-CO-R', -CO-OR', -CO-NH₂, -CO-NHR',CO-NR'₂, -NH-CO-R', -SO₂-R', -SO-R', -OH, -halogen, -N₃, -NH₂, -NHR', -NR'₂ and -CN; where each R' is independently selected from -(C₁-C₈)alkyl and aryl.

In an embodiment and as preferably used herein, "aryl" or "aryl ring" refers to a carbocyclic aromatic group, wherein the carbocyclic aromatic group is selected from the group consisting of aromatic groups with a single ring and aromatic groups with two or more fused rings, preferably two rings. Examples of carbocyclic aromatic groups with a single benzene ring include, but are not limited to, phenyl, and examples of carbocyclic aromatic groups with two or more fused benzene rings include, but are not limited to naphthyl and anthracenyl. A carbocyclic aromatic group can be unsubstituted or substituted with one or more groups including, but not limited to, -(C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃,NH₂, -NHR', -NR'₂ and -CN; where each R' is independently selected from -(C₁-C₈)alkyl and aryl. In a more preferred embodiment the carbocyclic aromatic group comprises two substitutents of the one or more groups, wherein the first of said two groups is attached to a first ring atom of the carbocyclic aromatic group. The second of said two groups is attached either to a second ring atom of the carbocyclic aromatic group directly adjacent to said first ring atom or to a second ring atom of the carbocyclic aromatic group, wherein the second ring atom is separated from the first ring atom by a further ring atom.

In an embodiment and as preferably used herein, "heteroaryl" or "heteroaryl ring" refers to a heterocyclic aromatic group, wherein the heterocyclic aromatic group is selected from the group consisting of heterocyclic aromatic groups with a single ring and heterocyclic aromatic group with two or more fused rings, preferably two rings. Examples of heterocyclic aromatic groups with a single ring include, but are not limited to, furanyl, thiophenyl, pyrroyl, pyrazoyl, oxazoyl, thiazoyl, imidazoyl, pyridinyl pyrimidinyl and examples of heterocyclic aromatic groups with two or more fused rings include, but are not limited to benzothiophenyl, benzofuranyl, indoyl and quinolinyl. A heterocyclic aromatic group can be unsubstituted or substituted with one or more groups including, but not limited to, -(C₁-C₈)alkyl, -O-[(C₁-C₈)alkyl], -aryl, -COR', -O-CO-R', -CO-OR', -CO-NH₂, -CO-NHR', -CO-NR'₂, -NH-CO-R', -SO₂-R', -SO-R',OH, -halogen, -N₃, -NH₂, -NHR', -NR'₂ and -CN, wherein each R' is independently selected from -(C₁-C₈)alkyl and aryl. In a more preferred embodiment the heterocyclic aromatic group comprises two substitutents of the one or more groups, wherein the first of said two groups is attached to a first ring atom of the heterocyclic aromatic group. The second of said two groups is attached either to a second ring atom of the heterocyclic aromatic group directly adjacent to said first ring atom or to a second ring atom of the heterocyclic aromatic group, wherein the second ring atom is separated from the first ring atom by a further ring atom.

In an embodiment and as preferably used herein, "aromatic resiude" or/and "aromatic ring" refers to an aryl as defined herein.

In an embodiment and as preferably used herein, "aromatic side chain" refers to a side chain wherein the side chain comprises an "aromatic ring" as defined herein.

In an embodiment and as preferably used herein, "bicyclic aromatic ring" refers to an aryl as defined herein, wherein the carbocyclic aromatic group of the aryl is selected from the group of aromatic groups with two or more fused rings, preferably two rings.

In an embodiment and as preferably used herein, "bicyclic aromatic side chain" refers to a side chain wherein the side chain comprises a "bicyclic aromatic ring" as defined herein.

In an embodiment and as preferably used herein, arylene refers to an aryl group which has two covalent bonds and can be in the *ortho, meta,* or *para* configurations as shown in the following structures: 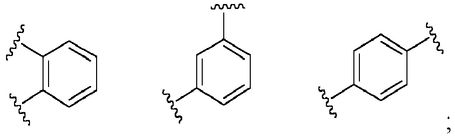 in which the phenyl group can be unsubstituted or substituted with four groups, including, but not limited to, (C₁-C₈)alkyl, -O-[(C₁-C₈)alkyl], -aryl, -CO-R', -O-CO-R', -CO-OR', -CO-NH₂, -CO-NHR', -CO-NR'₂, -NH-CO-R', -SO₂-R', -SO-R', -OH, -halogen, -N₃, -NH₂, -NHR',-NR'₂ and -CN; where each R' is independently selected from -(C₁-C₈)alkyl and aryl.

In an embodiment and as preferably used herein, atoms with unspecified atomic mass numbers in any structural formula or in any passage of the instant specification including the claims are either of unspecified isotopic composition, naturally occurring mixtures of isotopes or individual isotopes. This applies in particular to carbon, oxygen, nitrogen, sulfur, phosphorus, halogens and metal atoms, including but not limited to C, O, N, S, F, P, Cl, Br, At, Sc, Cr, Mn, Co, Fe, Cu, Ga, Sr, Zr, Y, Mo, Tc, Ru, Rh, Pd, Pt, Ag, In, Sb, Sn, Te, I, Pr, Pm, Dy, Sm, Gd, Tb, Ho, Dy, Er, Yb, Tm, Lu, Sn, Re, Rd, Os, Ir, Au, Pb, Bi, Po, Fr, Ra, Ac, Th and Fm.

In an embodiment and as preferably used herein, a chelator is a compound which is capable of forming a chelate, whereby a chelate is a compound, preferably a cyclic compound where a metal or a moiety having an electron gap or a lone pair of electrons participates in the formation of the ring. More preferably, a chelator is a kind of compound where a single ligand occupies more than one coordination site at a central atom.

In an embodiment and as preferably used herein, a bio-distribution modifier is a compound the function of which can be the attenuation of unintended or undesired bio-distribution of the compound of the invention by altering physio-chemical properties of the compound of the invention.

In an embodiment and as preferably used herein, "Z group" refers to (a) a chelator optionally comprising a linker, or (b) a bio-distribution modifier optionally comprising a linker. The group and position, respectively, of the compound of the invention where a Z group may be covalently attached, is also referred to herein as Z group attachment point.

In accordance with the present invention, Z group attachment points are in particular the following ones: the N-terminal modification group A, which comprises one Z group; Xaa4 comprising one Z group; Xaa5 comprising one Z group; Xaa8 comprising one Z group; Yc comprising one Z group; and the C-terminal modification group C-term, which comprises one Z-group.

More preferred Z group attachment points are the N-terminal modification group A comprising one Z-group; Xaa8 comprising one Z group; and the C-terminal modification group C-term comprising one Z-group.

As will be appreciated by a person skilled in the art, the chemical nature of the chelator, of the bio-distribution modifier, of the linker of the chelator and of the linker of the bio-distribution modifier define the kind of amino acid residue to be used for attaching any one of them to the compound of the invention. Typically, the chelator, the bio-distribution modifier, the linker of the chelator and the linker of the bio-distribution comprise a chemical group suitable for reacting with the amino acid residue, whereby such chemical group is selected form the group comprising a carboxylic acid group and an amino group.

In an embodiment and as preferably used herein, "amino acid residue" refers to all atoms of an amino acid, which remain in a peptide chain after the combination of said amino acid with another amino acid, an N-terminal modification group A, a C-terminal modification group C-term or a Z group either to either the α-nitrogen atom of said amino acid or the carbonyl group of said amino acid.

In an embodiment and as preferably used herein, "L-configuration" and "D-configuration" refer to the stereo configuration of the α-carbon atom of an α-amino acid or a residue of an α-amino acid.

In an embodiment and as preferably used herein, "side chain" refers to all atoms of an amino acid residue that are not comprised in the "main chain" portion of said amino acid residue. "Main chain" refers to the structure that is formed by the consecutive connection of at least a first amino acid and a second amino acid, whereby the α-nitrogen atom of an α-amino acid being the second amino acid, the β-nitrogen atom of a β-amino acid being the second amino acid, the γ-nitrogen of a γ-amino acid being the second amino acid, the δ-nitrogen atom of a δ-amino acid being the second amino acid, the ε-nitrogen of an ε-amino acid being the second amino acid or the ω-nitrogen of an ω-amino acid being the second amino acid is connected or covalently linked to the C-1 carbonyl group of a preceding amino acid being the first amino acid.

In an embodiment and as preferably used herein, the term "linking", "linked" or "link" refers to or describes a scenario, where two or more moieties are connected either directly or indirectly with an interspersed group. Preferably, such moiety is a chelator, an amino acid residue or a bio-distribution modifier. In a more preferred embodiment and as preferably used herein, such interspersed group is a linker.

In an embodiment and as preferably used herein, the term "interspersed" refers to or describes a scenario in which a group, preferably the group is a linker, is inserted between a first moiety and a second moiety. Such moiety for example is an amino acid residue, a chelator or a bio-distribution modifier. The inserted group is covalently attached to both of the moieties.

Compounds of the invention typically contain amino acid sequences as provided herein. Conventional amino acids, also referred to as natural amino acids are identified according to their standard three-letter codes, as set forth in Table 3.

**Table 3: Conventional amino acids and their abbreviations**

| **3-letter codes** | **Amino acid** |
|---|---|
| Ala | Alanine |
| Cys | Cysteine |
| Asp | Aspartic acid |
| Glu | Glutamic acid |
| Phe | Phenylalanine |
| Gly | Glycine |
| His | Histidine |
| Ile | Isoleucine |
| Lys | Lysine |
| Leu | Leucine |
| Met | Methionine |
| Asn | Asparagine |
| Pro | Proline |
| Gln | Glutamine |
| Arg | Arginine |
| Ser | Serine |
| Thr | Threonine |
| Val | Valine |
| Trp | Tryptophan |
| Tyr | Tyrosine |

Non-conventional amino acids, also referred to as non-natural amino acids, are any kind of non-oligomeric compound, which comprises an amino group and a carboxylic group and is not a conventional amino acid.

Examples of conventional amino acids, non-conventional amino acids and other building blocks as used for the construction compounds of the invention are identified according to their abbreviation or name found in Table 4. The structures of some building blocks are depicted as resiudes of the corresponding amino acids (amino acid residues) or these building blocks are shown as residue, which is completely attached to another structure like a peptide or amino acid residue. Structures of amino acids are shown as building blocks and as residues of the corresponding amino acids how they are present after implementation in the peptide sequence. Some larger chemical moieties consisting of more than one moiety are also shown for the reason of clarity (e.g. Apc(DOTA)).

**Table 4: Abbreviation, name and structure of non-natural amino-acid and other building blocks and chemical moieties**

| **Abbr.** | **Name** | **Residue** | **Building Block** |
|---|---|---|---|
| 1Ni | 3-(1-naphthyl)-L-alanine | 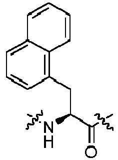 | 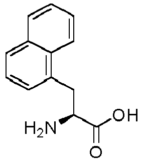 |
| 2Lut | 2,6-lutidylidene (derived from 2,6-lutidine) | 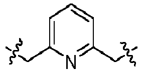 | |
| 2MeBn | 2-Methylbenzylidene (derived from 1,2-Xylene) | 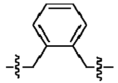 | |
| 2Ni | 3-(2-naphthyl)-L-alanine | 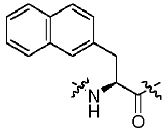 | 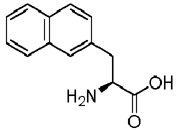 |
| 3CbBn | 3-Carboxybenzyl (derived from meta-methyl benzoic acid) | 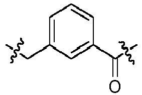 | |
| 3Lut | 3,5-lutidylidene (derived from 3,5-lutidine) | 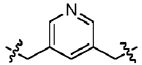 | |
| 3MeBn | 3-Methylbenzylidene (derived from 1,3-Xylene) | 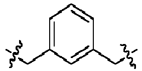 | |
| 3MeBz | 3-methylbenzoyl (derived from meta-methyl benzoic acid) | 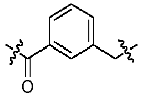 | |
| 3Mh | 3-Methyl-L-histidine | 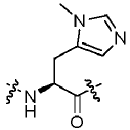 | 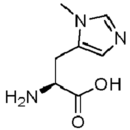 |
| 4Ap | 4-(*S*)-Amino-L-proline | 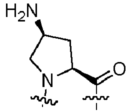 | 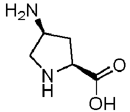 |
| 4ClPhp | 4-chlorophenyl propionic acid | 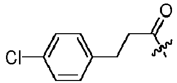 | 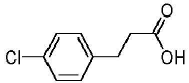 |
| 4Dfp | 4,4-Difluoro-L-proline | 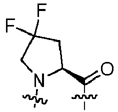 | 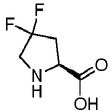 |
| 4FPhp | 4-fluorophenyl propionic acid | 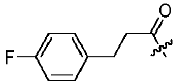 | 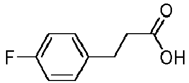 |
| 4MeBn | 4-Methylbenzylidene (derived from 1,4-Xylene) | 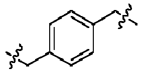 | |
| 4OHPhp | 4-hydroxyphenyl propionic acid | 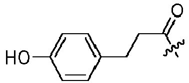 | 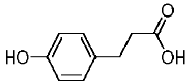 |
| 4Tfp | 4-(*R*)-Fluoro-L-proline | 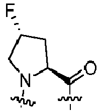 | 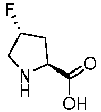 |
| 5Brw | 5-bromo-L-tryptophan | 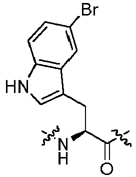 | 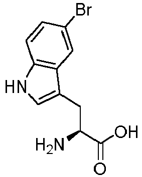 |
| 5Clw | 5-chloro-L-tryptophan | 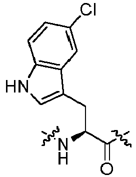 | 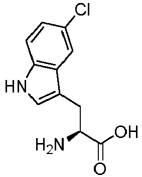 |
| 5Fw | 5-fluoro-L-tryptophan | 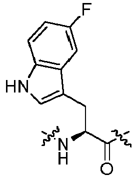 | 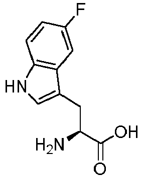 |
| 6Clw | 6-chloro-L-tryptophan | 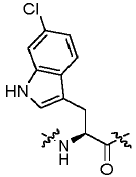 | 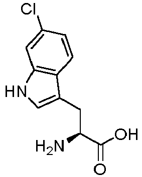 |
| 6Fw | 6-fluoro-L-tryptophan | 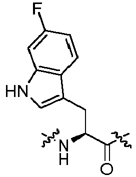 | 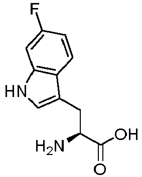 |
| 7Fw | 7-fluoro-L-tryptophan | 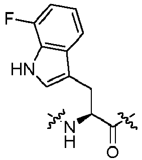 | 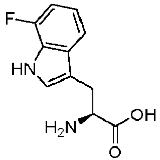 |
| 7Nw | 7-aza-L-tryptophan | 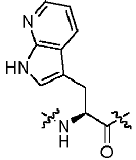 | 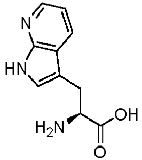 |
| Ac | Acetyl | 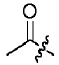 | 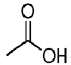 |
| Abu | 2*S*-aminobutyric acid | 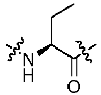 | 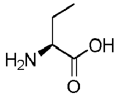 |
| Ahx | 6-aminohexanoic acid | 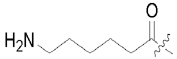 | 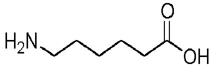 |
| Aib | 1-amino-isobutyric acid | 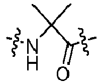 | 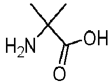 |
| Aic | 2-Aminoindane-2-carboxylic acid | 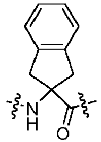 | 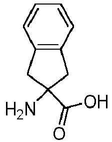 |
| ala | D-alanine | 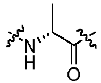 | 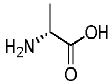 |
| Ala | L-alanine | 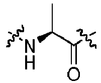 | 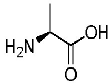 |
| alloThr | L-allo-threonine | 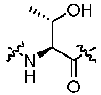 | 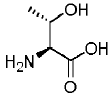 |
| Aml | □ -methyl-L-leucine | 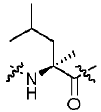 | 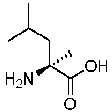 |
| Ams | □ -methyl-L-serine | 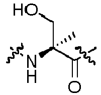 | 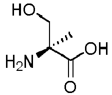 |
| APAc | 4-amino-1-carboxymethylpipe ridine | 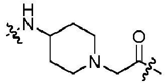 | 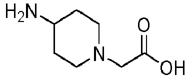 |
| Apc | 4-aminopiperidine-4-carboxylic acid | 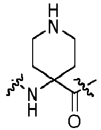 | 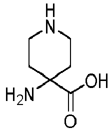 |
| Apc(DOTA) | 4-aminopiperidine-4-carboxylic acid DOTA conjugate | 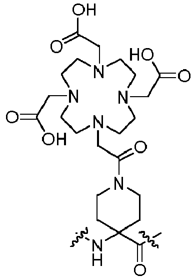 | |
| Ape | 1,5-diaminepentane | 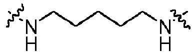 | 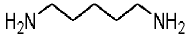 |
| Arg | L-arginine | 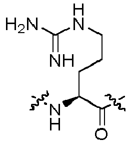 | 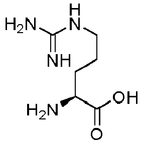 |
| Asn | L-asparagine | 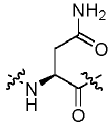 | 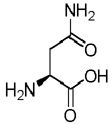 |
| asp | D-aspartic acid | 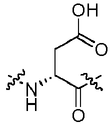 | 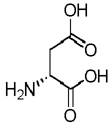 |
| Asp | L-aspartic acid | 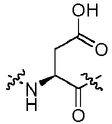 | 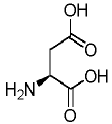 |
| Aze | (*S*)-Azetidine-2-carboxylic acid | 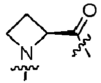 | 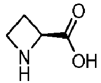 |
| Bhf | (*S*)-β-Homophenyl alanine | 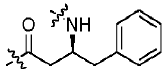 | 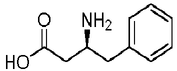 |
| Bio | D-(+)-Biotin | 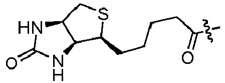 | 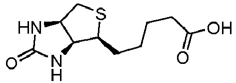 |
| Bta | 3-(3-benzothienyl)-L-alanine | 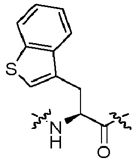 | 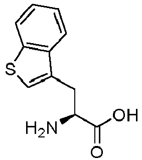 |
| Bzl | Benzyl | 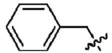 | |
| Cha | L-cyclo-hexyl-alanine | 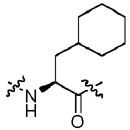 | 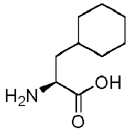 |
| Chg | L-cyclo-hexyl-glycine | 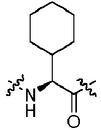 | 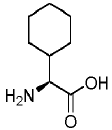 |
| Chy | 4-(*S*)-hydroxy-L-proline | 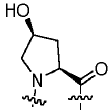 | 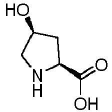 |
| Cfp | (2*S*,4*S*)-4-fluoro-pyrrolidine-2-carboxylic acid | 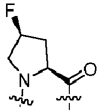 | 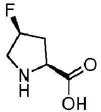 |
| Cit | L-Citrulline | 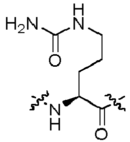 | 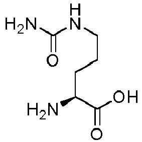 |
| Cpp | trans-3-Azabicyclo[3.1.0]h exane-2-carboxylic acid | 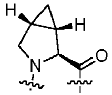 | 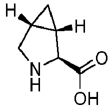 |
| Cy5SO3 | Cy5 dye (mono SO3) | 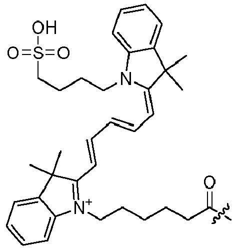 | 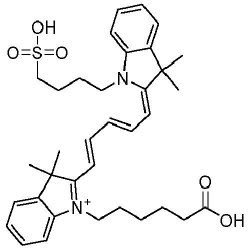 |
| Cya | L-cysteic acid | 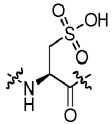 | 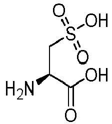 |
| cys | D-cysteine | 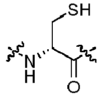 | 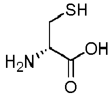 |
| Cys | L-cysteine | 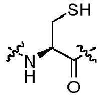 | 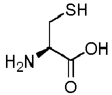 |
| Cys(2Lut) | L-cysteine bound to 2,6-lutidylidene | 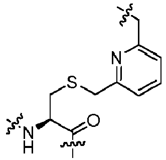 | |
| Cys(3Lut) | L-cysteine bound to 3,5-lutidylidene | 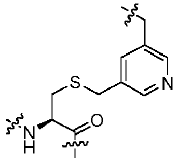 | |
| Cys(3MeBn) | L-cysteine bound to 3-methylbenzylidene | 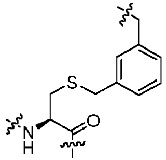 | |
| Cys(mli) | L-cysteine bound to maleimide | 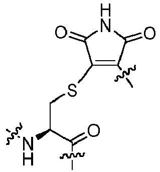 | |
| Cys(tMeBn (DOTA-AET)) | L-cysteine conjugated to mesitylene conjugated to DOTA via 2-aminoethane-1-thiol | 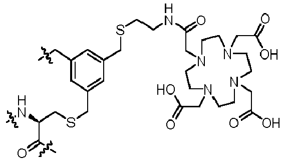 | |
| Cys(tMeBn (H-AET)) | L-cysteine conjugated to mesitylene conjugated with 2-aminoethane-1-thiol linker | 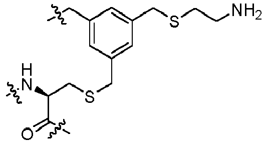 | |
| Cys(tMeBn (DOTA-PP)) | L-cysteine conjugated to mesitylene conjugated to DOTA via 2-aminoethane-1-thiol | 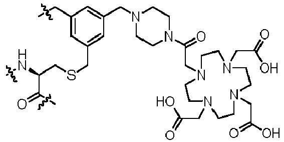 | |
| Cys(tMeBn (H-PP)) | L-cysteine conjugated to mesitylene conjugated with piperazine linker | 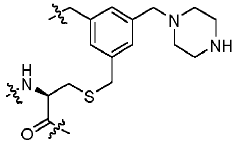 | |
| Dab | (*S*)-2,4-Diaminobutyric acid | 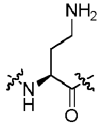 | 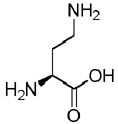 |
| Dap | (*S*)-2,3-Diaminopropionic acid | 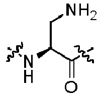 | 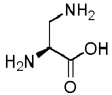 |
| dap | (*R*)-2,3-Diaminopropionic acid | 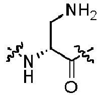 | 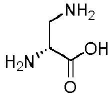 |
| Deg | Diethylgylcine | 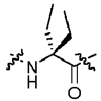 | 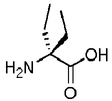 |
| Dfp | 4,4-Difluoro-L-proline | 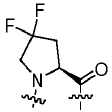 | 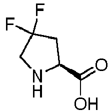 |
| Dmp | (*S*)-5,5-Dimethyl-proline | 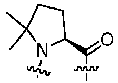 | 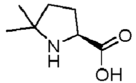 |
| DOTA | 2,2',2",2"'-(1,4,7,10-Tetraazacyclododec ane-1,4,7,10-tetrayl)tetraacetic acid | 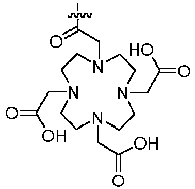 | 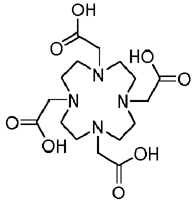 |
| Dtc | (*R*)-5,5-dimethyl-1,3-thiazolidine-4-carboxylic acid | 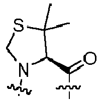 | 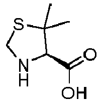 |
| Eaa | 3,4-Dichlor-L-phenylalanine | 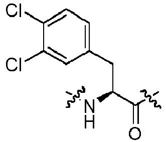 | 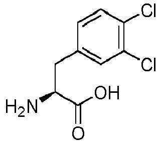 |
| Eaz | L-thiazolidine-4-carboxylic acid | 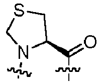 | 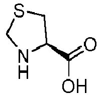 |
| Eca | 1-amino-1-cyclopentane carboxylic acid | 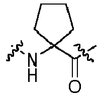 | 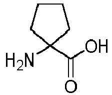 |
| Egd | (*S*)-ω,ω-Dimethyl-arginine | 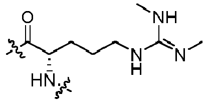 | 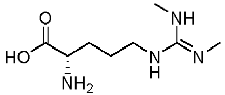 |
| Egm | 2,4-dichloro-L-phenylalanine | 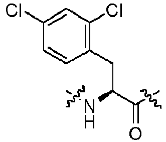 | 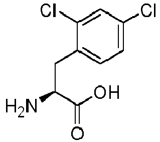 |
| Egn | 3,5-dichloro-L-phenylalanine | 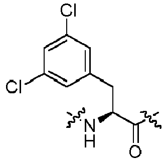 | 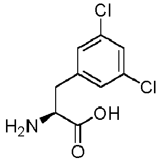 |
| Egp | 2,3-dichloro-L-phenylalanine | 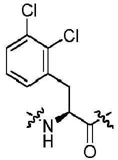 | 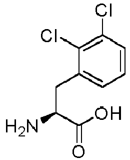 |
| Egz | 1-Amino-1-cyclohexan-carboxylic acid | 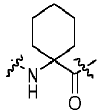 | 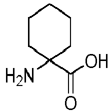 |
| Ghg | γ-Hydroxy-L-glutamic acid | 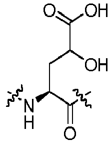 | 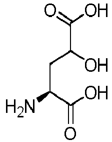 |
| gln | D-glutamine | 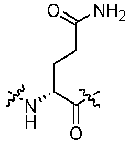 | 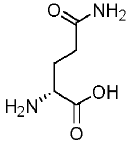 |
| Gln | L-glutamine | 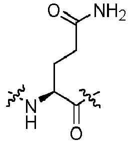 | 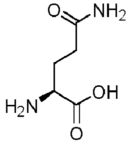 |
| glu | D-glutamic aicd | 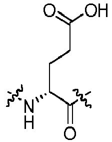 | 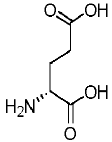 |
| Glu | L-glutamic aicd | 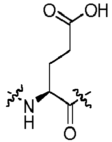 | 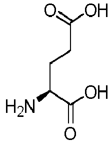 |
| Glutar | glutaric acid as linker | 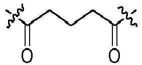 | 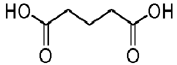 |
| Glutar | glutaric acid when present in the position of Xaa1 | 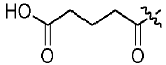 | 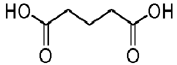 |
| Gly | glycine | 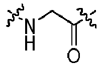 | 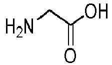 |
| H3p | 3-(S)-hydroxy-L-proline | 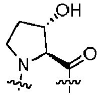 | 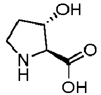 |
| HAC: Ahx | 6-((1,3-dihydroxypropan-2-yl)amino)hexanoic acid | 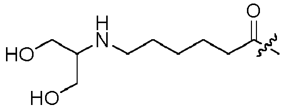 | 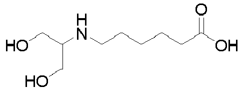 |
| HAC: Aur | 6-(3-(1,3-dihydroxypropan-2-yl)ureido)hexanoic acid | 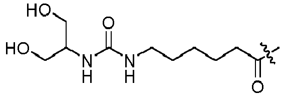 | 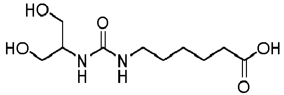 |
| HAC: Hyx | 4-(1-(1,3-dihydroxypropan-2-yl)-1H-1,2,3-triazol-4-yl)butanoic acid | 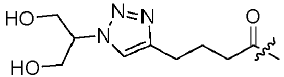 | 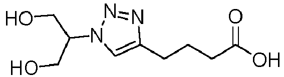 |
| HAC: PEO: Aur | 6-(3-(3-((1,3-dihydroxypropan-2-yl)oxy)-2,2-bis(hydroxymethyl) propyl)ureido)hexa noic acid | 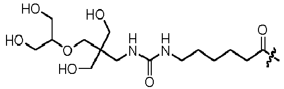 | 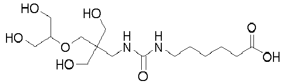 |
| HAC: PEO: Hyx | 4-(1-(3-((1,3-dihydroxypropan-2-yl)oxy)-2,2-bis(hydroxymethyl) propyl)-1H-1,2,3-triazol-4-yl)butanoic acid | 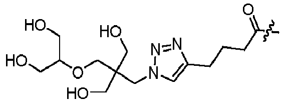 | 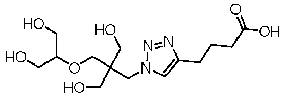 |
| HAC: TRIS: Aur | 6-(3-(1-((1,3-dihydroxypropan-2-yl)oxy)-3-hydroxy-2-(hydroxymethyl)pr opan-2-yl)ureido)hexanoic acid | 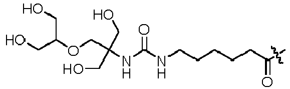 | 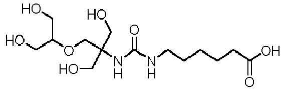 |
| HAC: TRIS: Hyx | 4-(1-(1-((1,3-dihydroxypropan-2-yl)oxy)-3-hydroxy-2-(hydroxymethyl)pr opan-2-yl)-1H-1,2,3-triazol-4-yl)butanoic acid | 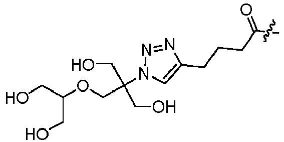 | 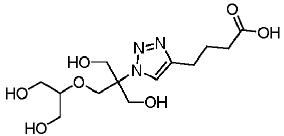 |
| HAC2: HAC: Ahx | 6-((1,3-bis((1,3-dihydroxypropan-2-yl)oxy)propan-2-yl)amino)hexanoic acid | 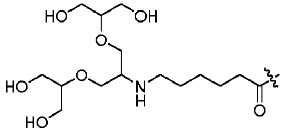 | 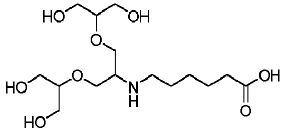 |
| HAC2: HAC: Aur | 6-(3-(1,3-bis((1,3-dihydroxypropan-2-yl)oxy)propan-2-yl)ureido)hexanoic acid | 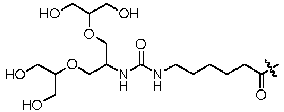 | 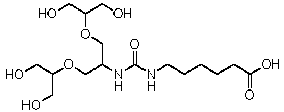 |
| HAC2: HAC: Hyx | 4-(1-(1,3-bis((1,3-dihydroxypropan-2-yl)oxy)propan-2-yl)-1H-1,2,3-triazol-4-yl)butanoic acid | 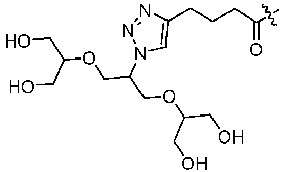 | 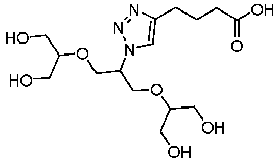 |
| HAC2: HAC: Tzx | 6-(4-(((1,3-bis((1,3-dihydroxypropan-2-yl)oxy)propan-2-yl)oxy)methyl)-1H-1,2,3-triazol-1-yl)hexanoic acid | 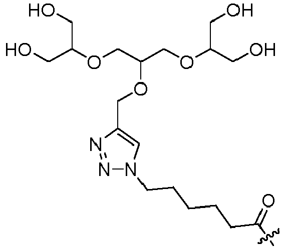 | 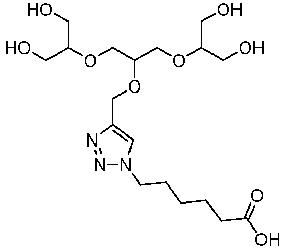 |
| HAC2: HPD: Aur | 6-(3-(3-((1,3-dihydroxypropan-2-yl)oxy)-2-(((1,3-dihydroxypropan-2-yl)oxy)methyl)prop yl)ureido)hexanoic acid | 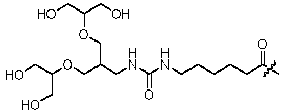 | 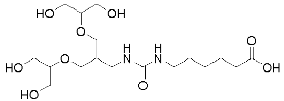 |
| HAC2: HPD: Hyx | 4-(1-(3-((1,3-dihydroxypropan-2-yl)oxy)-2-(((1,3-dihydroxypropan-2-yl)oxy)methyl)prop yl)-1H-1,2,3-triazol-4-yl)butanoic acid | 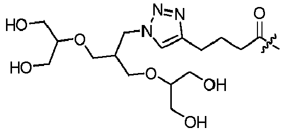 | 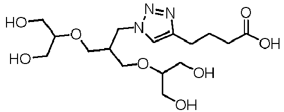 |
| HAC2: HPD: Tzx | 6-(4-((3-((1,3-dihydroxypropan-2-yl)oxy)-2-(((1,3-dihydroxypropan-2-yl)oxy)methyl)prop oxy)methyl)-1H-1,2,3-triazol-1-yl)hexanoic acid | 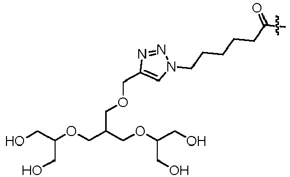 | 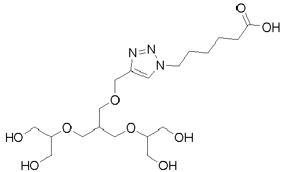 |
| HAC2: PEO: Aur | 6-(3-(3-((1,3-dihydroxypropan-2-yl)oxy)-2-(((1,3-dihydroxypropan-2-yl)oxy)methyl)-2-(hydroxymethyl)pr opyl)ureido)hexano ic acid | 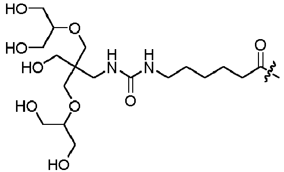 | 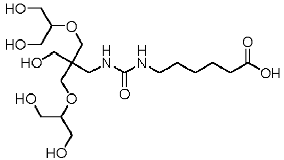 |
| HAC2: PEO: Hyx | 4-(1-(3-((1,3-dihydroxypropan-2-yl)oxy)-2-(((1,3-dihydroxypropan-2-yl)oxy)methyl)-2-(hydroxymethyl)pr opyl)-1H-1,2,3-triazol-4-yl)butanoic acid | 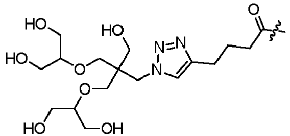 | 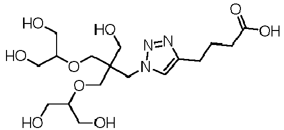 |
| HAC2: TRIS: Aur | 6-(3-(1,3-bis((1,3-dihydroxypropan-2-yl)oxy)-2-(hydroxymethyl)pr opan-2-yl)ureido)hexanoic acid | 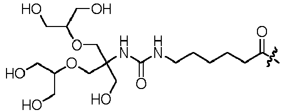 | 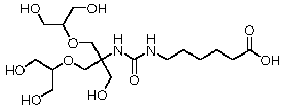 |
| HAC2: TRIS: Hyx | 4-(1-(1,3-bis((1,3-dihydroxypropan-2-yl)oxy)-2-(hydroxymethyl)pr opan-2-yl)-1H-1,2,3-triazol-4-yl)butanoic acid | 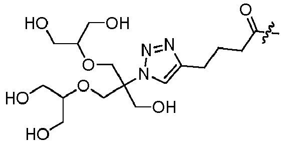 | 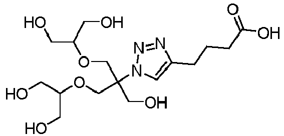 |
| HAC3: PEO: Aur | 6-(3-(3-((1,3-dihydroxypropan-2-yl)oxy)-2,2-bis(((1,3-dihydroxypropan-2-yl)oxy)methyl)prop yl)ureido)hexanoic acid | 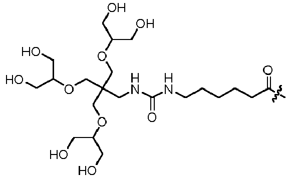 | 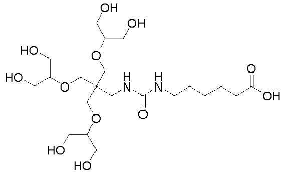 |
| HAC3: PEO: Hyx | 4-(1-(3-((1,3-dihydroxypropan-2-yl)oxy)-2,2-bis(((1,3-dihydroxypropan-2-yl)oxy)methyl)prop yl)-1H-1,2,3-triazol-4-yl)butanoic acid | 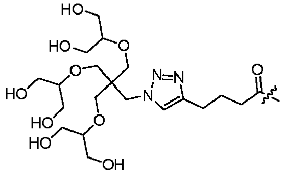 | 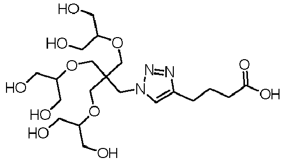 |
| HAC3: TRIS: Aur | 6-(3-(1,3-bis((1,3-dihydroxypropan-2-yl)oxy)-2-(((1,3-dihydroxypropan-2-yl)oxy)methyl)prop an-2-yl)ureido)hexanoic acid | 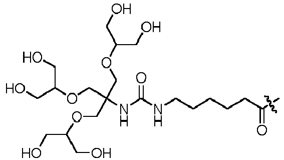 | 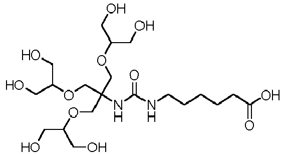 |
| HAC3: TRIS: Hyx | 4-(1-(1,3-bis((1,3-dihydroxypropan-2-yl)oxy)-2-(((1,3-dihydroxypropan-2-yl)oxy)methyl)prop an-2-yl)-1H-1,2,3-triazol-4-yl)butanoic acid | 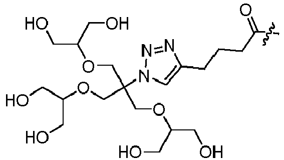 | 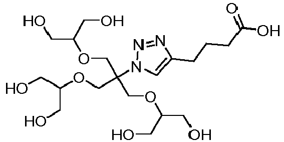 |
| HAC4: HPD2: Ahx | 6-(bis(3-((1,3-dihydroxypropan-2-yl)oxy)-2-(((1,3-dihydroxypropan-2-yl)oxy)methyl)prop yl)amino)hexanoic acid | 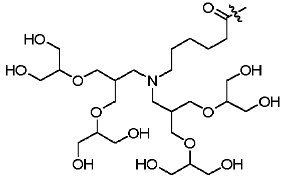 | 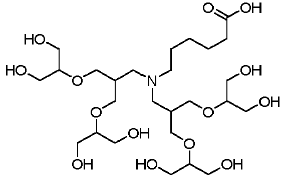 |
| Har | L-Homoarginine | 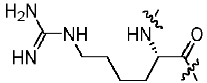 | 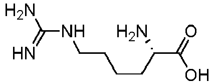 |
| Hci | L-Homocitrulline | 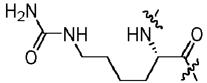 | 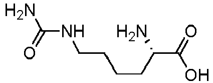 |
| Hey | L-homocysteine | 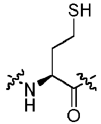 | 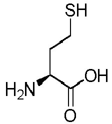 |
| Hga | L-homoglutamic acid | 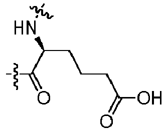 | 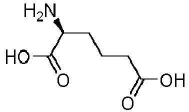 |
| Hfe | L-homophenylalanine | 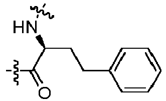 | 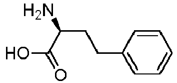 |
| Hle | L-homoleucine | 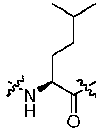 | 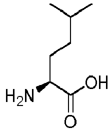 |
| HPD: Aur | 6-(3-(3-hydroxy-2-(hydroxymethyl) propyl)ureido) hexanoic acid | 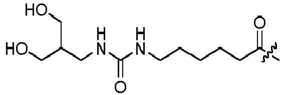 | 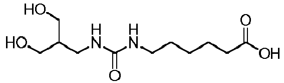 |
| HPD: Hyx | 4-(1-(3-hydroxy-2-(hydroxymethyl) propyl)-1H-1,2,3-triazol-4-yl)butanoic acid | 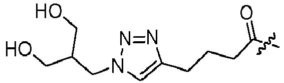 | 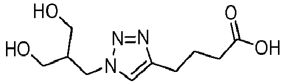 |
| HPD: PEO: Aur | 6-(3-(3-hydroxy-2-((3-hydroxy-2-(hydroxymethyl)pr opoxy)methyl)-2-(hydroxymethyl)pr opyl)ureido)hexano ic acid | 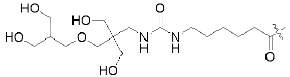 | 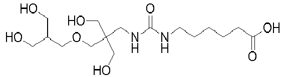 |
| HPD: PEO: Hyx | 4-(1-(3-hydroxy-2-((3-hydroxy-2-(hydroxymethyl)pr opoxy)methyl)-2-(hydroxymethyl)pr opyl)-1H-1,2,3-triazol-4-yl)butanoic acid | 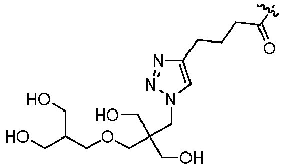 | 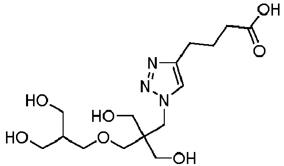 |
| HPD: TRIS: Aur | 6-(3-(1,3-dihydroxy-2-((3-hydroxy-2-(hydroxymethyl)pr opoxy)methyl)prop an-2-yl)ureido)hexanoic acid | 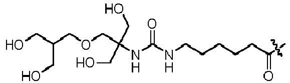 | 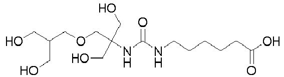 |
| HPD: TRIS: Hyx | 4-(1-(1,3-dihydroxy-2-((3-hydroxy-2-(hydroxymethyl)pr opoxy)methyl)prop an-2-yl)-1H-1,2,3-triazol-4-yl)butanoic acid | 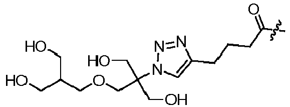 | 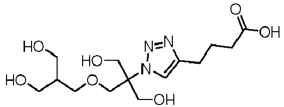 |
| HPD2: Ahx | 6-(bis(3-hydroxy-2-(hydroxymethyl) propyl)amino) hexanoic acid | 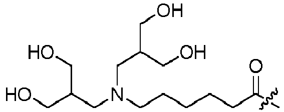 | 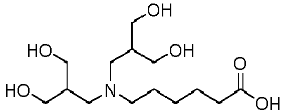 |
| HPD2: HAC: Ahx | 6-((1,3-bis(3-hydroxy-2-(hydroxymethyl) propoxy)propan-2-yl)amino)hexanoic acid | 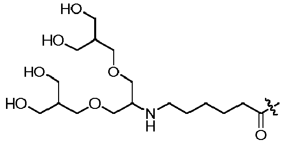 | 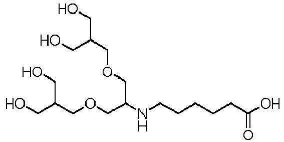 |
| HPD2: HAC: Aur | 6-(3-(1,3-bis(3-hydroxy-2-(hydroxymethyl) propoxy)propan-2-yl)ureido)hexanoic acid | 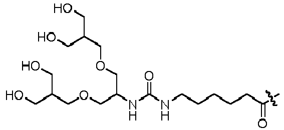 | 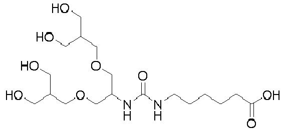 |
| HPD2: HAC: Hyx | 4-(1-(1,3-bis(3-hydroxy-2-(hydroxymethyl) propoxy)propan-2-yl)-1H-1,2,3-triazol-4-yl)butanoic acid | 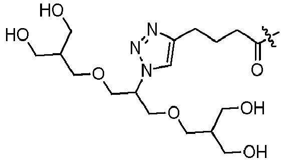 | 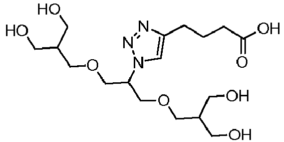 |
| HPD2: HAC: Tzx | 6-(4-(((1,3-bis(3-hydroxy-2-(hydroxymethyl)pr opoxy)propan-2-yl)oxy)methyl)-1H-1,2,3-triazol-1-yl)hexanoic acid | 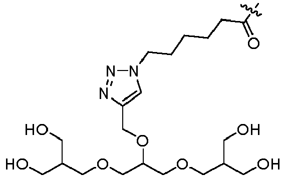 | 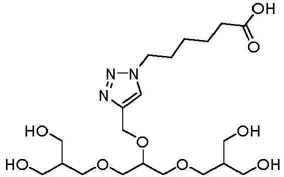 |
| HPD2: HPD: Ahx | 6-((3-(3-hydroxy-2-(hydroxymethyl)pr opoxy)-2-((3-hydroxy-2-(hydroxymethyl)propoxy)methyl)prop yl)amino)hexanoic acid | 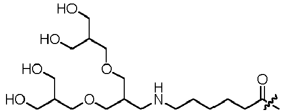 | 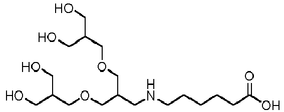 |
| HPD2: HPD: Aur | 6-(3-(3-(3-hydroxy-2-(hydroxymethyl)pr opoxy)-2-((3-hydroxy-2-(hydroxymethyl)pr opoxy)methyl)prop yl)ureido)hexanoic acid | 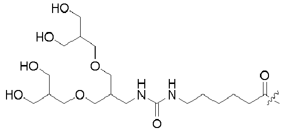 | 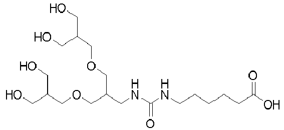 |
| HPD2: HPD: Hyx | 4-(1-(3-(3-hydroxy-2-(hydroxymethyl)pr opoxy)-2-((3-hydroxy-2-(hydroxymethyl)pr opoxy)methyl)prop yl)-1H-1,2,3-triazol-4-yl)butanoic acid | 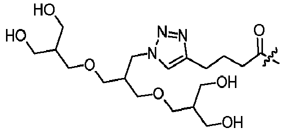 | 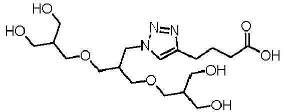 |
| HPD2: HPD: Tzx | 6-(4-((3-(3-hydroxy-2-(hydroxymethyl)pr opoxy)-2-((3-hydroxy-2-(hydroxymethyl)pr opoxy)methyl)prop oxy)methyl)- 1H-1,2,3-triazol-1-yl)hexanoic acid | 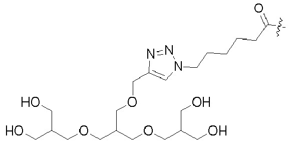 | 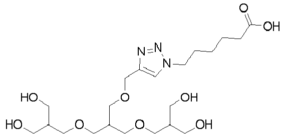 |
| HPD2: PEO: Aur | 6-(3-(3-hydroxy-2,2-bis((3-hydroxy-2-(hydroxymethyl)pr opoxy)methyl)prop yl)ureido)hexanoic acid | 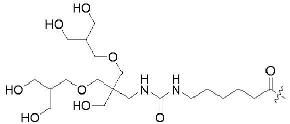 | 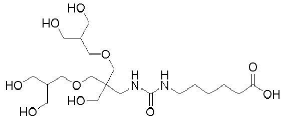 |
| HPD2: PEO: Hyx | 4-(1-(3-hydroxy-2,2-bis((3-hydroxy-2-(hydroxymethyl)pr opoxy)methyl)prop yl)-1H-1,2,3-triazol-4-yl)butanoic acid | 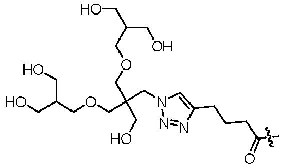 | 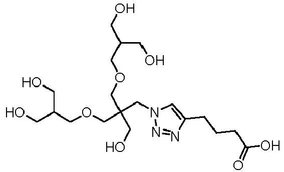 |
| HPD2: TRIS: Aur | 6-(3-(1-hydroxy-3-(3-hydroxy-2-(hydroxymethyl)pr opoxy)-2-((3-hydroxy-2-(hydroxymethyl)pr opoxy)methyl)propan-2-yl)ureido)hexanoic acid | 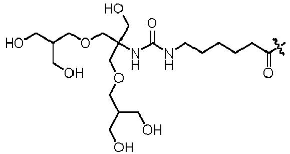 | 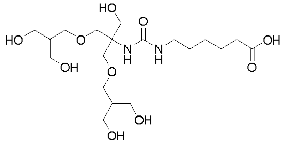 |
| HPD2: TRIS: Hyx | 4-(1-(1-hydroxy-3-(3-hydroxy-2-(hydroxymethyl)pr opoxy)-2-((3-hydroxy-2-(hydroxymethyl)pr opoxy)methyl)prop an-2-yl)-1H-1,2,3-triazol-4-yl)butanoic acid | 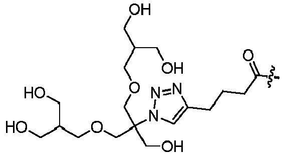 | 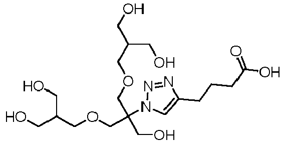 |
| HPD3: PEO: Aur | 6-(3-(3-(3-hydroxy-2-(hydroxymethyl)pr opoxy)-2,2-bis((3-hydroxy-2-(hydroxymethyl)pr opoxy)methyl)prop yl)ureido)hexanoic acid | 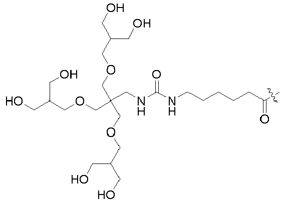 | 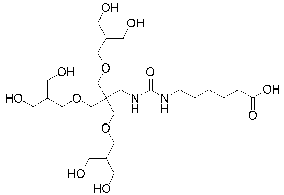 |
| HPD3: PEO: Hyx | 4-(1-(3-(3-hydroxy-2-(hydroxymethyl)pr opoxy)-2,2-bis((3-hydroxy-2-(hydroxymethyl)pr opoxy)methyl)prop yl)-1H-1,2,3-triazol-4-yl)butanoic acid | 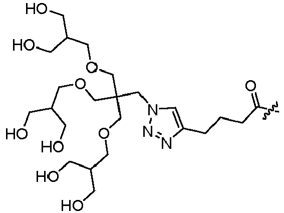 | 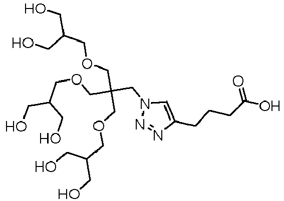 |
| HPD3: TRIS: Aur | 6-(3-(1,3-bis(3-hydroxy-2-(hydroxymethyl)pr opoxy)-2-((3-hydroxy-2-(hydroxymethyl)pr opoxy)methyl)prop an-2-yl)ureido)hexanoic acid | 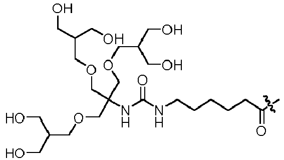 | 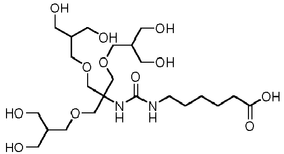 |
| HPD3: TRIS: Hyx | 4-(1-(1,3-bis(3-hydroxy-2-(hydroxymethyl)pr opoxy)-2-((3-hydroxy-2-(hydroxymethyl)pr opoxy)methyl)prop an-2-yl)-1H-1,2,3-triazol-4-yl)butanoic acid | 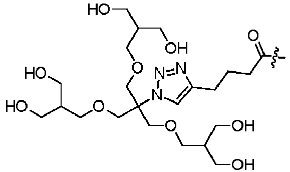 | 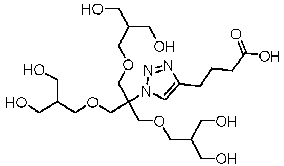 |
| HPD4: HPD2: Ahx | 6-(bis(3-(3-hydroxy-2-(hydroxymethyl)pr opoxy)-2-((3-hydroxy-2-(hydroxymethyl)pr opoxy)methyl)prop yl)amino)hexanoic acid | 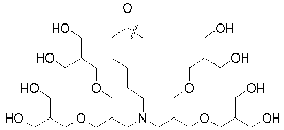 | 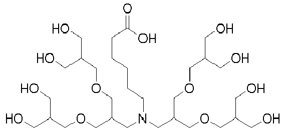 |
| Hse | L-homoserine | 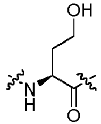 | 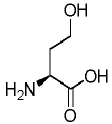 |
| Hth | L-homothreonine | 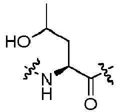 | 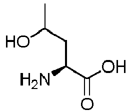 |
| Hym | (2*S*,4*R*)-4-Methyoxy-pyrrolidine-2-carboxylic acid | 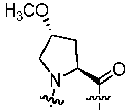 | 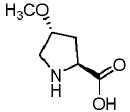 |
| Hyn | hex-5-ynoic acid | 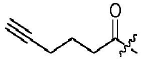 | 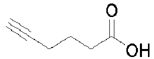 |
| Hyp | 4-(*R*)-hydroxy-L-proline | 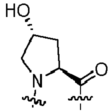 | 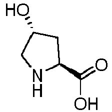 |
| Hyw | 5-hydroxy-L-tryptophane | 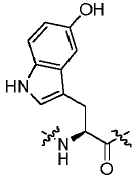 | 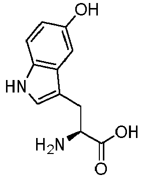 |
| Ile | L-isoleucine | 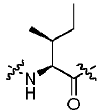 | 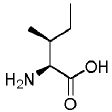 |
| kur: HAC | (*R*)-2-amino-6-(3-(1,3-dihydroxypropan-2-yl)ureido)hexanami de | 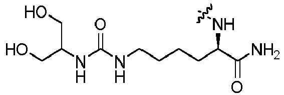 | 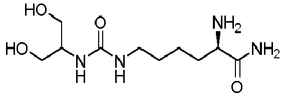 |
| kur: HAC: HAC2 | (*R*)-2-amino-6-(3-(1,3-bis((1,3-dihydroxypropan-2-yl)oxy)propan-2-yl)ureido)hexanami de | 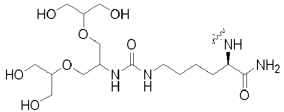 | 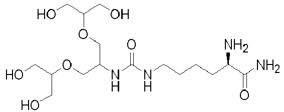 |
| kur: HAC: HPD2 | (*R*)-2-amino-6-(3-(1,3-bis(3-hydroxy-2-(hydroxymethyl)pr opoxy)propan-2-yl)ureido)hexanami de | 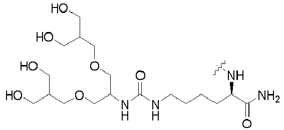 | 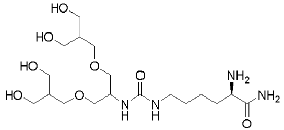 |
| kur: HPD | (*R*)-2-amino-6-(3-(3-hydroxy-2-(hydroxymethyl)pr opyl)ureido)hexana mide | 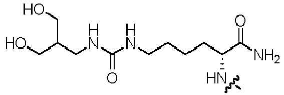 | 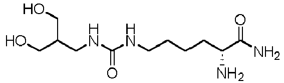 |
| kur: HPD: HAC2 | (*R*)-2-amino-6-(3-(3-((1,3-dihydroxypropan-2-yl)oxy)-2-(((1,3-dihydroxypropan-2-yl)oxy)methyl)prop yl)ureido)hexanami de | 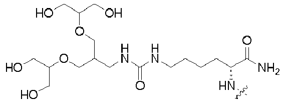 | 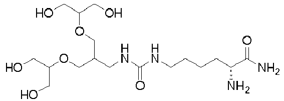 |
| kur: HPD: HPD2 | (*R*)-2-amino-6-(3-(3-(3-hydroxy-2-(hydroxymethyl)pr opoxy)-2-((3-hydroxy-2-(hydroxymethyl)pr opoxy)methyl)prop yl)ureido)hexanami de | 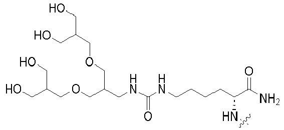 | 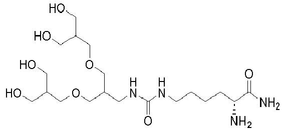 |
| kur: PEO | (*R*)-2-amino-6-(3-(3-hydroxy-2,2-bis(hydroxymethyl) propyl)ureido)hexa namide | 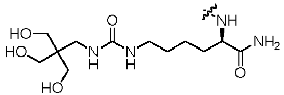 | 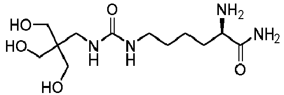 |
| kur: PEO: HAC | (*R*)-2-amino-6-(3-(3-((1,3-dihydroxypropan-2-yl)oxy)-2,2-bis(hydroxymethyl) propyl)ureido)hexa namide | 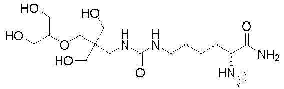 | 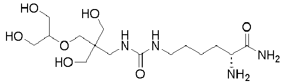 |
| kur: PEO: HAC2 | (*R*)-2-amino-6-(3-(3-((1,3-dihydroxypropan-2-yl)oxy)-2-(((1,3-dihydroxypropan-2-yl)oxy)methyl)-2-(hydroxymethyl)pr opyl)ureido)hexana mide | 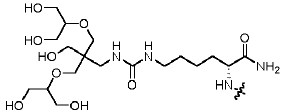 | 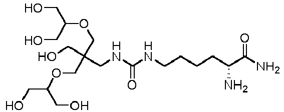 |
| kur: PEO: HAC3 | (*R*)-2-amino-6-(3-(3-((1,3-dihydroxypropan-2-yl)oxy)-2,2-bis(((1,3-dihydroxypropan-2-yl)oxy)methyl)prop yl)ureido)hexanami de | 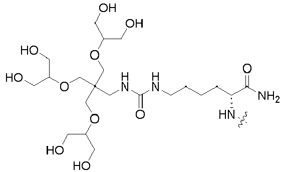 | 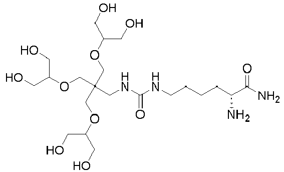 |
| kur: PEO: HPD | (*R*)-2-amino-6-(3-(3-hydroxy-2-((3-hydroxy-2-(hydroxymethyl)pr opoxy)methyl)-2-(hydroxymethyl)pr opyl)ureido)hexana mide | 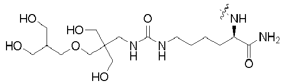 | 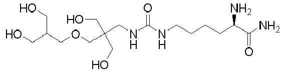 |
| kur: PEO: HPD2 | (*R*)-2-amino-6-(3-(3-hydroxy-2,2-bis((3-hydroxy-2-(hydroxymethyl)pr opoxy)methyl)prop yl)ureido)hexanami de | 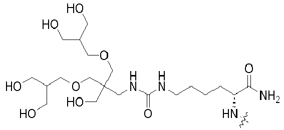 | 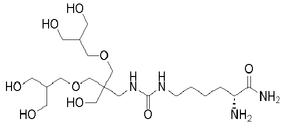 |
| kur: PEO: HPD3 | (*R*)-2-amino-6-(3-(3-(3-hydroxy-2-(hydroxymethyl)pr opoxy)-2,2-bis((3-hydroxy-2-(hydroxymethyl)pr opoxy)methyl)prop yl)ureido)hexanami de | 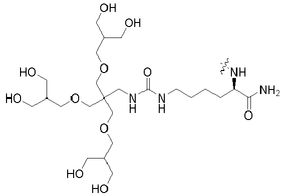 | 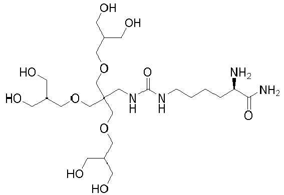 |
| kur: TRIS | (*R*)-2-amino-6-(3-(1,3-dihydroxy-2-(hydroxymethyl)pr opan-2-yl)ureido)hexanami de | 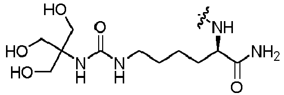 | 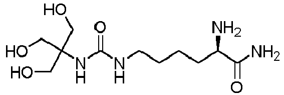 |
| kur: TRIS: HAC | (*R*)-2-amino-6-(3-(1-((1,3-dihydroxypropan-2-yl)oxy)-3-hydroxy-2-(hydroxymethyl)pr opan-2-yl)ureido)hexanami de | 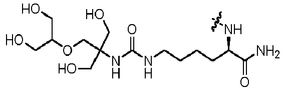 | 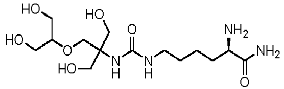 |
| kur: TRIS: HAC2 | (*R*)-2-amino-6-(3-(1,3-bis((1,3-dihydroxypropan-2-yl)oxy)-2-(hydroxymethyl)propan-2-yl)ureido)hexanami de | 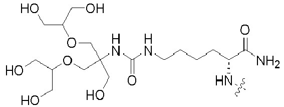 | 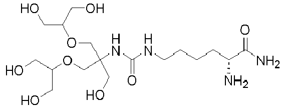 |
| kur: TRIS: HAC3 | (*R*)-2-amino-6-(3-(1,3-bis((1,3-dihydroxypropan-2-yl)oxy)-2-(((1,3-dihydroxypropan-2-yl)oxy)methyl)prop an-2-yl)ureido)hexanami de | 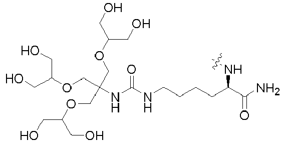 | 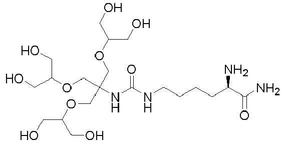 |
| kur: TRIS: HPD | (*R*)-2-amino-6-(3-(1,3-dihydroxy-2-((3-hydroxy-2-(hydroxymethyl)pr opoxy)methyl)prop an-2-yl)ureido)hexanami de | 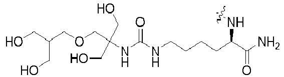 | 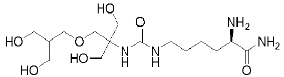 |
| kur: TRIS: HPD2 | (*R*)-2-amino-6-(3-(1-hydroxy-3-(3-hydroxy-2-(hydroxymethyl)pr opoxy)-2-((3-hydroxy-2-(hydroxymethyl)pr opoxy)methyl)prop an-2-yl)ureido)hexanami de | 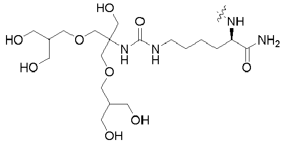 | 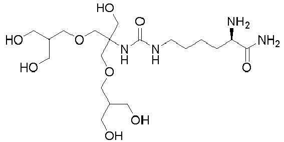 |
| kur: TRIS: HPD3 | (*R*)-2-amino-6-(3-(1,3-bis(3-hydroxy-2-(hydroxymethyl)pr opoxy)-2-((3-hydroxy-2-(hydroxymethyl)pr opoxy)methyl)prop an-2-yl)ureido)hexanami de | 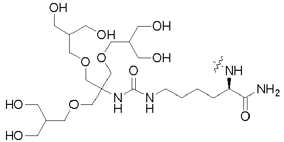 | 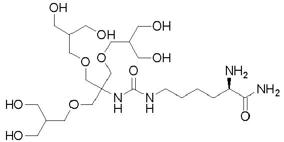 |
| leu | D-leucine | 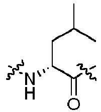 | 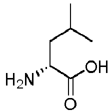 |
| Leu | L-leucine | 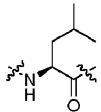 | 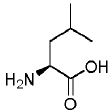 |
| lys | D-lysine | 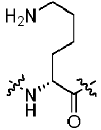 | 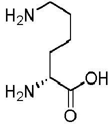 |
| Lys | L-lysine | 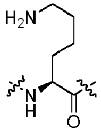 | 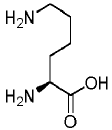 |
| lys: HAC | (*R*)-2-amino-6-((1,3-dihydroxypropan-2-yl)amino)hexanami de | 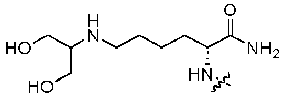 | 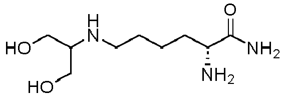 |
| lys: HAC: HAC2 | (*R*)-2-amino-6-((1,3-bis((1,3-dihydroxypropan-2-yl)oxy)propan-2-yl)amino)hexanami de | 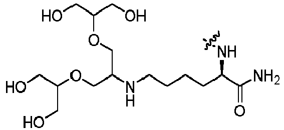 | 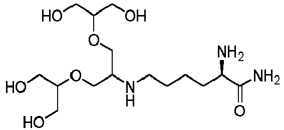 |
| lys: HAC: HPD2 | (2*R*)-2-amino-6-((1,3-bis(3-hydroxy-2-(hydroxymethyl)pr opoxy)propan-2-yl)amino)hexanami de | 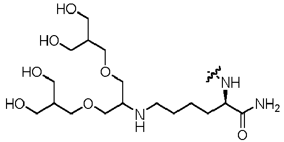 | 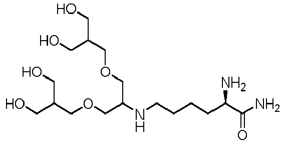 |
| lys: HPD2 | (*R*)-2-amino-6-(bis(3-hydroxy-2-(hydroxymethyl)pr opyl)amino)hexana mide | 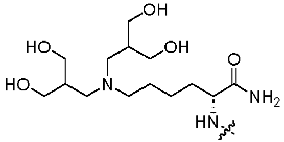 | 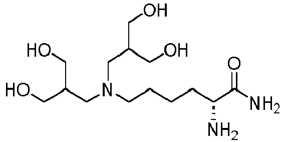 |
| lys: HPD2: HAC4 | (*R*)-2-amino-6-(bis(3-((1,3-dihydroxypropan-2-yl)oxy)-2-(((1,3-dihydroxypropan-2-yl)oxy)methyl)prop yl)amino)hexanami de | 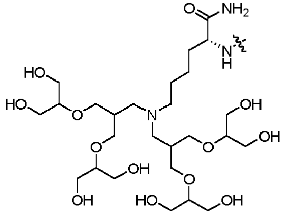 | 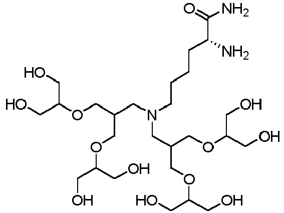 |
| lys: HPD2: HPD4 | (*R*)-2-amino-6-(bis(3-(3-hydroxy-2-(hydroxymethyl)pr opoxy)-2-((3-hydroxy-2-(hydroxymethyl)pr opoxy)methyl)prop yl)amino)hexanami de | 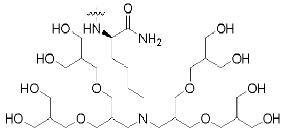 | 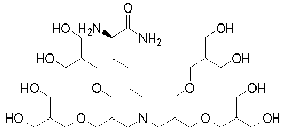 |
| lys: PEO2 | (*R*)-2-amino-6-(bis(3-hydroxy-2,2-bis(hydroxymethyl) propyl)amino) hexanamide | 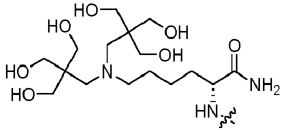 | 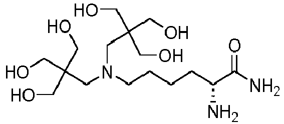 |
| Mcf | 3-chloro-L-phenylalanine | 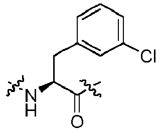 | 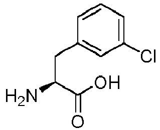 |
| Met | L-methionine | 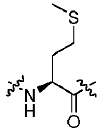 | 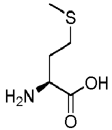 |
| mli | maleimide | 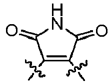 | |
| Moo | (*S*)-Methionine sulfone | 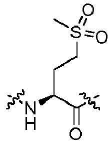 | 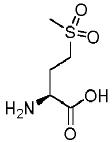 |
| N3Ahx | 6-azidohexanoic acid | 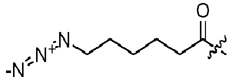 | 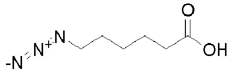 |
| n3lys | N⁶-diazo-D-lysine | 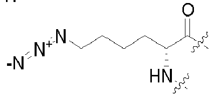 | 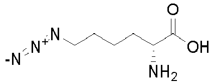 |
| N3Tap | (2*S*,4*R*)-4-azido-1-pyrrolidin-2-carboxylic acid | 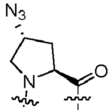 | 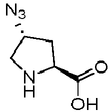 |
| N4Ac | Bis-(2-amino-ethylamine)-methylpropionic acid | 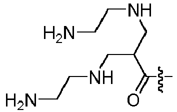 | 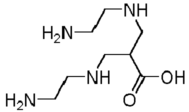 |
| Nglu | N-glutamic acid, N-carboxyethyl glycine | 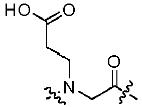 | 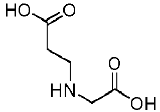 |
| NH₂ | usually represents a C-terminus of a peptide sequence | 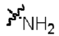 | |
| Nle | L-norleucine | 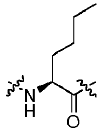 | 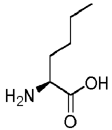 |
| Nleu | N-leucine, N-isobutyl glycine | 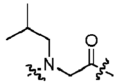 | 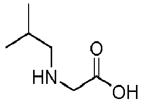 |
| Nlys | N-lysine, N-(4-aminobutyl) glycine | 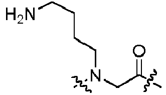 | 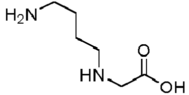 |
| nma | N-methyl-D-alanine | 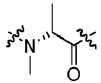 | 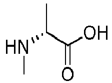 |
| Nma | N-methyl-L-alanine | 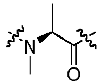 | 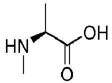 |
| Nmc | N-methyl-L-cysteine | 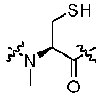 | 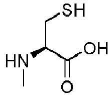 |
| Nmd | N-methyl-L-aspartic acid | 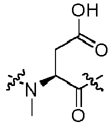 | 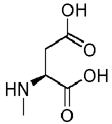 |
| Nme | N-methyl-L-glutamic acid | 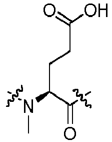 | 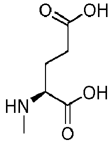 |
| Nmg | N-methyl-glycine | 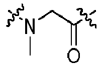 | 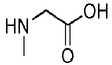 |
| Nml | N-methyl-L-leucine | 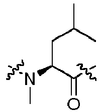 | 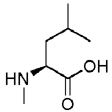 |
| Nmw | N-methyl-L-tryptophan | 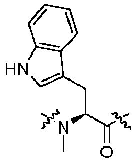 | 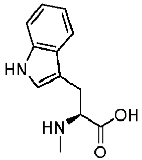 |
| NODAGA | (*R*)-1,4,7-triazacyclononane,1 -glutaric acid-4,7-acetic acid | 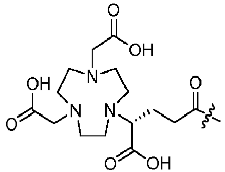 | 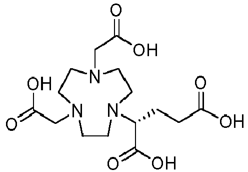 |
| NOPO | 1,4,7-triazacyclononane-1,4-bis [methylene (hydroxymethyl)ph osphinic acid]-7-[methylene (2-carboxyethyl)phosp hinic acid] | 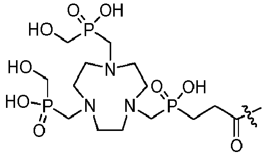 | 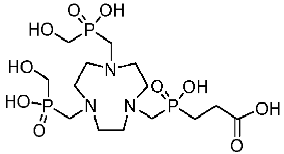 |
| Npg | L-neopentyl-glycine | 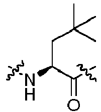 | 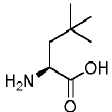 |
| Nphe | N-phenylalanine, N-benzyl-glycine | 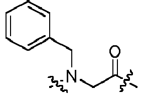 | 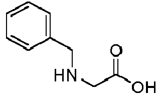 |
| Nta | nitrilotriacetic acid | 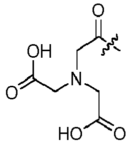 | 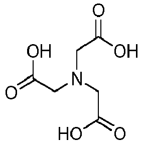 |
| Nva | L-norvaline | 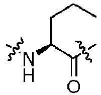 | 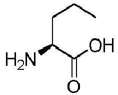 |
| O2Oc | 8-amino-3,6-dioxaoctanoic acid | 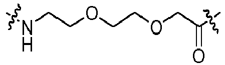 | 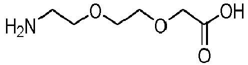 |
| Ocf | ortho-chloro-L-phenylalanine | 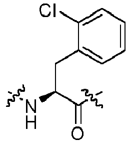 | 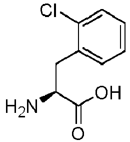 |
| Oic | (2*S*,3a*S*,7a*S*)-octahydroindolecar boxylic acid | 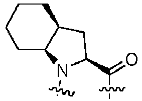 | 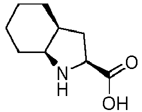 |
| Omr | ω-Methyl-L-arginine | 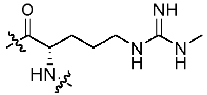 | 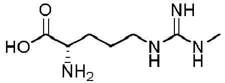 |
| Oxa | (*S*)-oxazolidine-4-carboxylic acid | 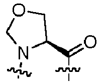 | 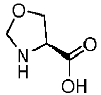 |
| Pcf | para-chloro-L-phenylalanine | 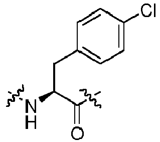 | 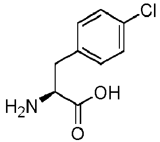 |
| PEG6 | 21-amino-4,7,10,13,16,19-hexaoxaheneicosan oic acid | 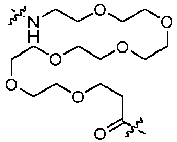 | 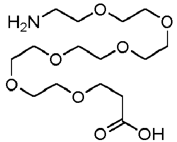 |
| Pen | L-penicillamine | 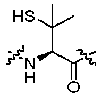 | 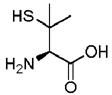 |
| PEO: Aur | 6-(3-(3-hydroxy-2,2-bis(hydroxymethyl) propyl)ureido)hexa noic acid | 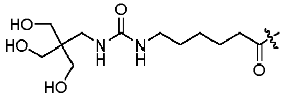 | 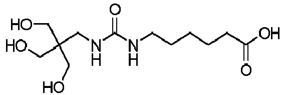 |
| PEO: Hyx | 4-(1-(3-hydroxy-2,2-bis(hydroxymethyl) proppl)-1H-1,2,3-triazol-4-yl)butanoic acid | 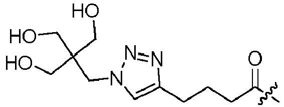 | 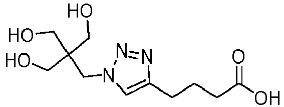 |
| PEO2: Ahx | 6-(bis(3-hydroxy-2,2-bis(hydroxymethyl) propyl)amino)hexa noic acid | 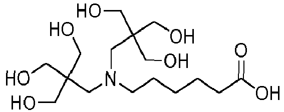 | 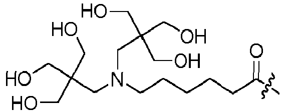 |
| Phe | L-phenylalanine | 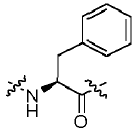 | 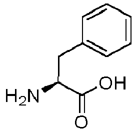 |
| Phg | L-phenylglycine | 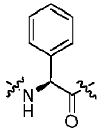 | 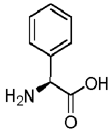 |
| Php | 3 -phenyl-propionic acid | 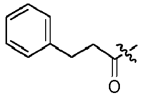 | 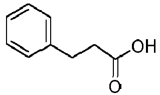 |
| Pip | (S)-Pipecolic Acid | 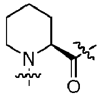 | 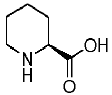 |
| PP | Piperazinyl/ Piperazine | 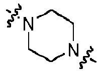 | 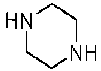 |
| prg | (*R*)-2-aminohex-5-ynoic acid | 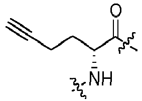 | 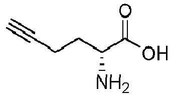 |
| pro | D-proline | 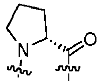 | 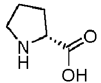 |
| Pro | L-proline | 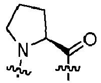 | 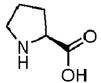 |
| prx: HAC | (*R*)-2-amino-4-(1-(1,3-dihydroxypropan-2-yl)-1H-1,2,3-triazol-4-yl)butanamide | 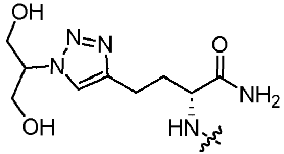 | 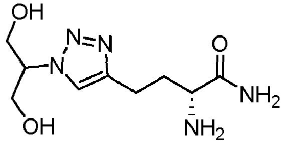 |
| prx: HAC: HAC2 | (*R*)-2-amino-4-(1-(1,3-bis((1,3-dihydroxypropan-2-yl)oxy)propan-2-yl)-1H-1,2,3-triazol-4-yl)butanamide | 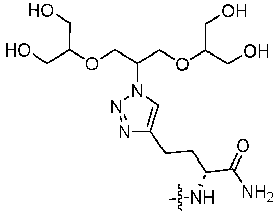 | 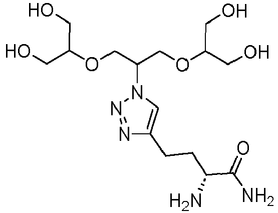 |
| prx: HAC: HPD2 | (*R*)-2-amino-4-(1-(1,3-bis(3-hydroxy-2-(hydroxymethyl)pr opoxy)propan-2-yl)-1H-1,2,3-triazol-4-yl)butanamide | 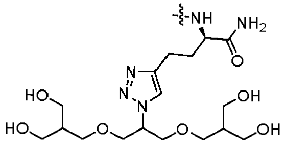 | 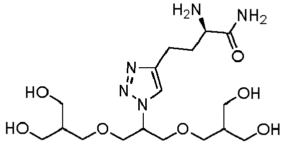 |
| prx: HPD | (*R*)-2-amino-4-(1-(3-hydroxy-2-(hydroxymethyl)pr opyl)-1H-1,2,3-triazol-4-yl)butanamide | 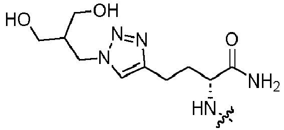 | 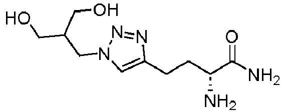 |
| prx: HPD: HAC2 | (*R*)-2-amino-4-(1-(3-((1,3-dihydroxypropan-2-yl)oxy)-2-(((1,3-dihydroxypropan-2-yl)oxy)methyl)prop yl)-1H-1,2,3-triazol-4-yl)butanamide | 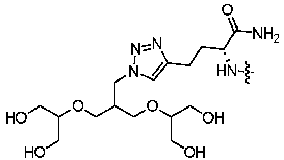 | 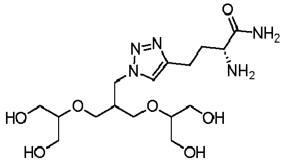 |
| prx: HPD: HPD2 | (*R*)-2-amino-4-(1-(3-(3-hydroxy-2-(hydroxymethyl)pr opoxy)-2-((3-hydroxy-2-(hydroxymethyl)pr opoxy)methyl)prop yl)-1H-1,2,3-triazol-4-yl)butanamide | 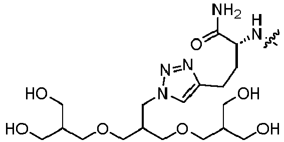 | 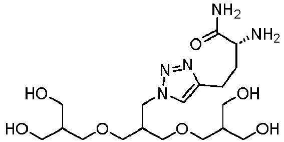 |
| prx: PEO | (*R*)-2-amino-4-(1-(3-hydroxy-2,2-bis(hydroxymethyl) propyl)-1H-1,2,3-triazol-4-yl)butanamide | 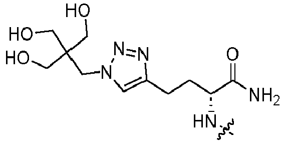 | 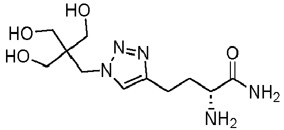 |
| prx: PEO: HAC | (*R*)-2-amino-4-(1-(3-((1,3-dihydroxypropan-2-yl)oxy)-2,2-bis(hydroxymethyl) propyl)-1H-1,2,3-triazol-4-yl)butanamide | 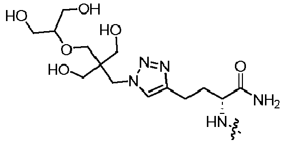 | 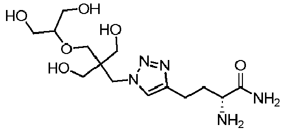 |
| prx: PEO: HAC2 | (*R*)-2-amino-4-(1-(3-((1,3-dihydroxypropan-2-yl)oxy)-2-(((1,3-dihydroxypropan-2-yl)oxy)methyl)-2-(hydroxymethyl)pr opyl)-1H-1,2,3-triazol-4-yl)butanamide | 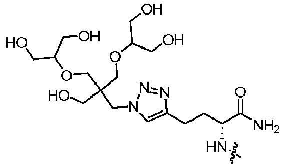 | 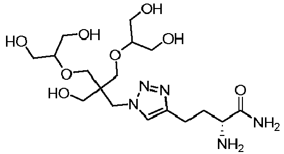 |
| prx: PEO: HAC3 | (*R*)-2-amino-4-(1-(3-((1,3-dihydroxypropan-2-yl)oxy)-2,2-bis(((1,3-dihydroxypropan-2-yl)oxy)methyl)prop yl)-1H-1,2,3-triazol-4-yl)butanamide | 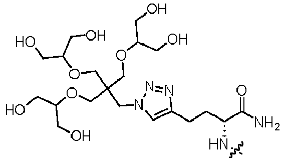 | 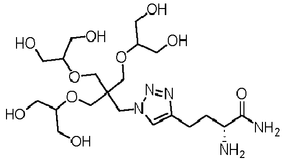 |
| prx: PEO: HPD | (*R*)-2-amino-4-(1-(3-hydroxy-2-((3-hydroxy-2-(hydroxymethyl)pr opoxy)methyl)-2-(hydroxymethyl)pr opyl)-1H-1,2,3-triazol-4-yl)butanamide | 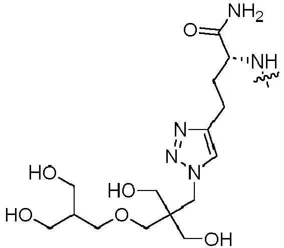 | 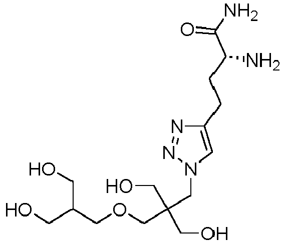 |
| prx: PEO: HPD2 | (*R*)-2-amino-4-(1-(3-hydroxy-2,2-bis((3-hydroxy-2-(hydroxymethyl)pr opoxy)methyl)prop yl)-1H-1,2,3-triazol-4-yl)butanamide | 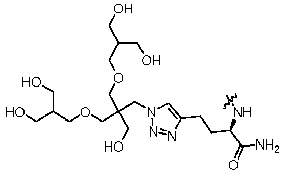 | 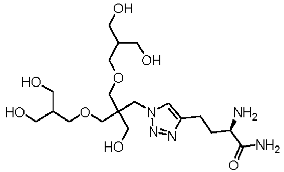 |
| prx: PEO: HPD3 | (*R*)-2-amino-4-(1-(3-(3-hydroxy-2-(hydroxymethyl)pr opoxy)-2,2-bis((3-hydroxy-2-(hydroxymethyl)pr opoxy)methyl)prop yl)-1H-1,2,3-triazol-4-yl)butanamide | 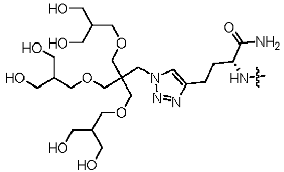 | 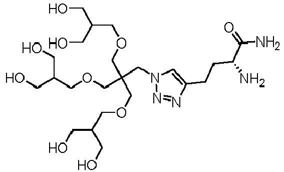 |
| prx: TRIS | (*R*)-2-amino-4-(1-(1,3-dihydroxy-2-(hydroxymethyl)pr opan-2-yl)-1H-1,2,3-triazol-4-yl)butanamide | 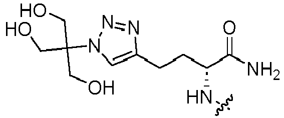 | 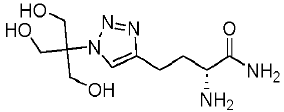 |
| prx: TRIS: HAC | (*R*)-2-amino-4-(1-(1-((1,3-dihydroxypropan-2-yl)oxy)-3-hydroxy-2-(hydroxymethyl)pr opan-2-yl)-1H-1,2,3-triazol-4-yl)butanamide | 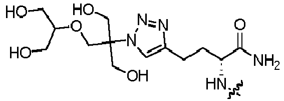 | 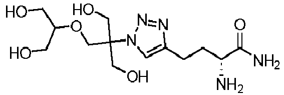 |
| prx: TRIS: HAC2 | (*R*)-2-amino-4-(1-(1,3-bis((1,3-dihydroxypropan-2-yl)oxy)-2-(hydroxymethyl)pr opan-2-yl)-1H-1,2,3-triazol-4-yl)butanamide | 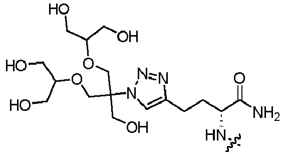 | 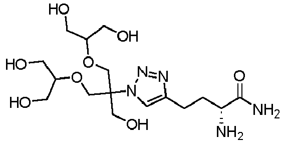 |
| prx: TRIS: HAC3 | (*R*)-2-amino-4-(1-(1,3-bis((1,3-dihydroxypropan-2-yl)oxy)-2-(((1,3-dihydroxypropan-2-yl)oxy)methyl)prop an-2-yl)-1H-1,2,3-triazol-4-yl)butanamide | 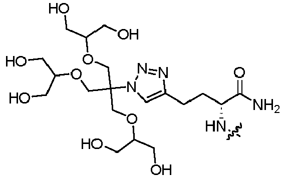 | 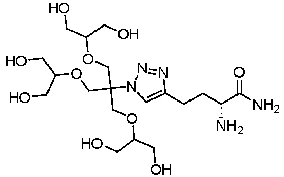 |
| prx: TRIS: HPD | (*R*)-2-amino-4-(1-(1,3-dihydroxy-2-((3-hydroxy-2-(hydroxymethyl)pr opoxy)methyl)prop an-2-yl)-1H-1,2,3-triazol-4-yl)butanamide | 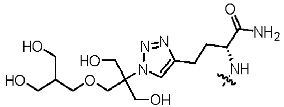 | 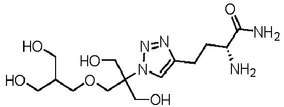 |
| prx: TRIS: HPD2 | (*R*)-2-amino-4-(1-(1-hydroxy-3-(3-hydroxy-2-(hydroxymethyl)pr opoxy)-2-((3-hydroxy-2-(hydroxymethyl)pr opoxy)methyl)prop an-2-yl)-1H-1,2,3-triazol-4-yl)butanamide | 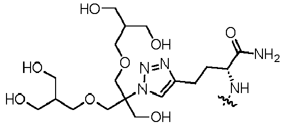 | 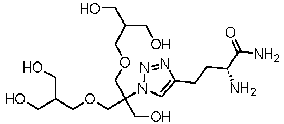 |
| prx: TRIS: HPD3 | (*R*)-2-amino-4-(1-(1,3-bis(3-hydroxy-2-(hydroxymethyl)pr opoxy)-2-((3-hydroxy-2-(hydroxymethyl)pr opoxy)methyl)prop an-2-yl)-1H-1,2,3-triazol-4-yl)butanamide | 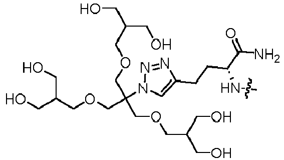 | 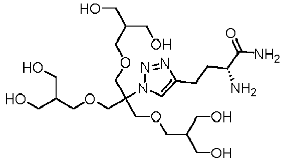 |
| Pse | L-phosphoserine | 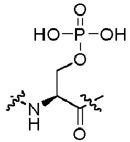 | 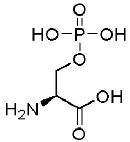 |
| Rni | (*R*)-nipecotic acid | 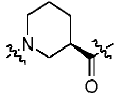 | 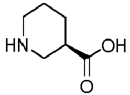 |
| Ser | L-serine | 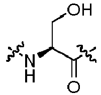 | 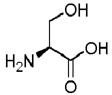 |
| Serol | serinol | 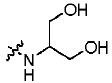 | 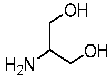 |
| Smc | S-methyl-L-cysteine | 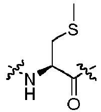 | 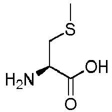 |
| Succinyl | succinic acid as linker | 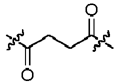 | 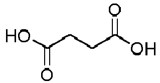 |
| Succinyl | succinic acid when present in the position of Xaa1 | 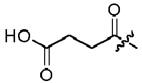 | 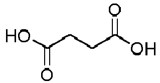 |
| Tap | (2*S*,4*R*)-4-amino-1-pyrrolidin-2-carboxylic acid | 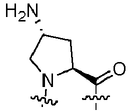 | 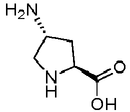 |
| Tip | (2*S*,4*R*)-4-fluoro-pyrrolidine-2-carboxylic acid | 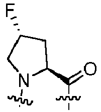 | 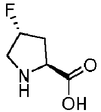 |
| Thi | L-thienylalanine | 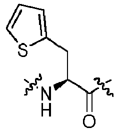 | 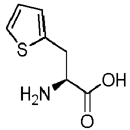 |
| Thp | 4-amino-tetrahydropyran-4-carboxylic acid | 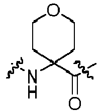 | 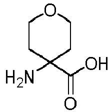 |
| Thr | L-threonine | 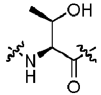 | 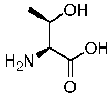 |
| Tie | (*S*)-α-t-butylglycine | 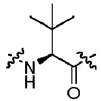 | 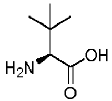 |
| tMeBn | tris-methylbenzyl, mesitylene | 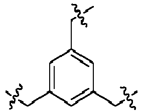 | |
| tMeBn (DOTA-AET) | DOTA attached to mesitylene via 2-aminoethane-1-thiol | 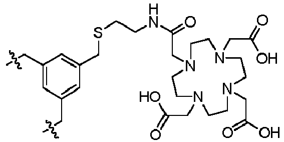 | |
| tMeBn (DOTA-PP) | DOTA attached to mesitylene via piperazine | 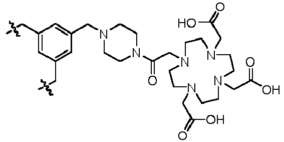 | |
| TRIS: Aur | 6-(3-(1,3-dihydroxy-2-(hydroxymethyl)pr opan-2-yl)ureido)hexanoic acid | 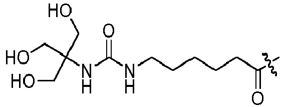 | 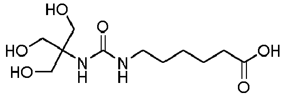 |
| TRIS: Hyx | 4-(1-(1,3-dihydroxy-2-(hydroxymethyl)pr opan-2-yl)-1H-1,2,3-triazol-4-yl)butanoic acid | 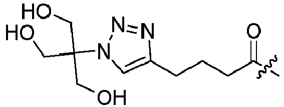 | 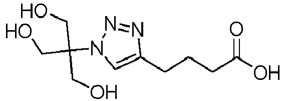 |
| trp | D-tryptophan | 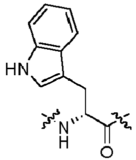 | 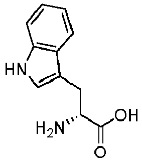 |
| Trp | L-tryptophan | 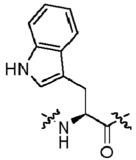 | 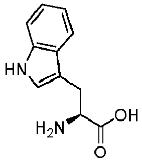 |
| Ttds | 1,13-diamino-4,7,10-trioxatridecan-succinamic acid | 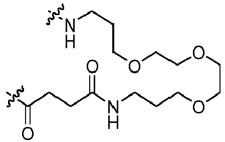 | 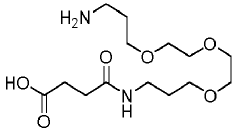 |
| tyr | D-tyrosine | 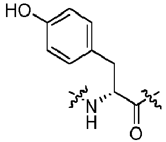 | 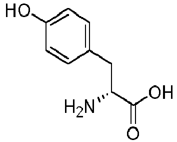 |
| Tyr | L-tyrosine | 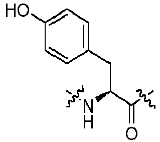 | 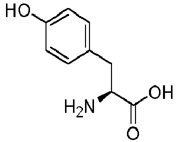 |
| tzk: HAC: HAC2 | (*R*)-2-amino-6-(4-(((1,3-bis((1,3-dihydroxypropan-2-yl)oxy)propan-2-yl)oxy)methyl)-1H-1,2,3-triazol-1-yl)hexanoic acid | 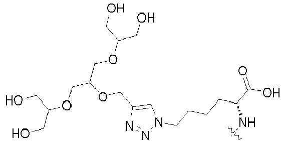 | 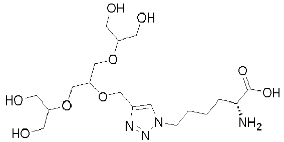 |
| tzk: HAC: HPD2 | (*R*)-2-amino-6-(4-(((1,3-bis(3-hydroxy-2-(hydroxymethyl)pr opoxy)propan-2-yl)oxy)methyl)-1H-1,2,3-triazol-1-yl)hexanoic acid | 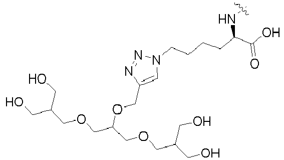 | 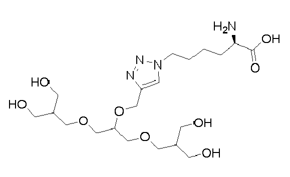 |
| tzk: HPD: HAC2 | (*R*)-2-amino-6-(4-((3-((1,3-dihydroxypropan-2-yl)oxy)-2-(((1,3-dihydroxypropan-2-yl)oxy)methyl)prop oxy)methyl)- 1H-1,2,3-triazol-1-yl)hexanoic acid | 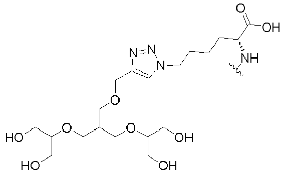 | 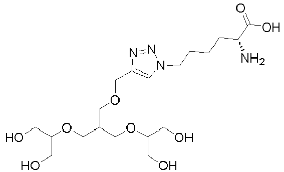 |
| tzk: HPD: HPD2 | (*R*)-2-amino-6-(4-((3-(3-hydroxy-2-(hydroxymethyl)pr opoxy)-2-((3-hydroxy-2-(hydroxymethyl)pr opoxy)methyl)prop oxy)methyl)- 1H-1,2,3-triazol-1-yl)hexanoic acid | 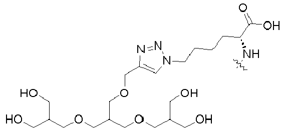 | 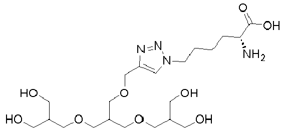 |
| val | D-valine | 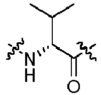 | 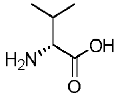 |
| Val | L-valine | 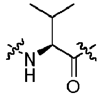 | 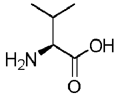 |

The amino acid sequences of the peptides disclosed herein are depicted in typical peptide sequence format, as will be understood by a person skilled in the art. For example, the three-letter code for a residue of a conventional amino acid, the code for a residue of a non-conventional amino acid or the abbreviation for a residue of a building block other than a conventional or non-conventional amino acid indicates the presence of the residue, of the amino acid residue or the presence of the residue of the building block at a specified position within the peptide sequence.The code for a residue of an amino acid and the abbreviation for a residue of a building block, respectively, is connected to the code for a subsequent residue of an amino acid and/or to the code for a preceding residue of an amino acid and, respectively, is connected to the abbreviation for a subsequent residue of a building block and/or to the abbreviation for a preceding residue of a building block, respectively, in the peptide sequence by a hyphen which typically represents an amide linkage. For example, if the hyphen is located before the code for a residue of an amino acid, this usually symbolizes that the amino group (as indicated in the corresponding entry in Table 4) of the residue of the amino acid is covalently attached to a carbonyl group of a preceding residue of an amino acid, and if the hyphen is located after the code for a residue of an amino acid, this usually symbolizes that the carbonyl group (as indicated in the corresponding entry in Table 4) of the residue of the amino acid is covalently attached to an amino group of a subsequent residue of an amino acid.

Depending on the spacing between the amino- and the carboxy group in amino acids they are classified into α-, β-, γ-, δ-, ε-, (and so forth) -amino acids, which means that these groups are typically are spaded apart by 1, 2, 3, 4, and 5 heavy-atoms (typically carbon), respectively.

For amino acids, in their abbreviations the first letter indicates the stereochemistry of the C-α-atom if applicable. For example, a capital first letter indicates that the L-form of the amino acid is present in the peptide sequence, while a lower case first letter indicating that the D-form of the correspondent amino acid is present in the peptide sequence. If the abbreviation starts with a number, the first letter in the abbreviation will be characteristic for the stereochemistry, if applicable. However, for enhanced clarity it is at any abbreviation an option to further clearly specify the stereochemistry of an amino acid abreviation by adding for instance the prefix "D-". As example "lys", "D-Lys" or "D-lys" describe all a D-configured Lys.

For someone skilled in the art it is evident that many amino acids can be N-methylated at their amino group. These N-methyl amino acid feature can occur in combination with some other attributes like L-α- or D-α-N-methyl amino acids which are N-methylated L-α- or D-α-amino acids.

The term "α,α-dialkylamino acid" refers to amino acid which comprise indepently two alkyl groups at the α-carbon atom which maybe in some cases form a ring-structure with each other.

The term "cyclic" amino acid means that the amino acid comprises a cyclic structure wherein in some cases the amino nitrogen of the amino acid is part of a heterocyclic structure usually consisting of 4 to 7 ring members. Cyclic maybe combined with other amino acid attribute as for instance in cyclic D-α-amino acid or cyclic α,α-dialkylamino acid.

The term "aromatic amino acid" refers to an amino acid, which comprise an aromatic structure and this includes a heteroaromatic structure whereas the term "non-aromatic amino acid" refers to amino acids which are devoid of any aromatic structure.

The term "heteroaromatic amino acid" refers to an amino acid, which comprise any kind of heteroaromatic structure.

An "aliphatic amino acid" is a non-aromatic amino acid, which consists of only C and H atoms apart from the amino and carboxy group.

A "polar amino acid" is any kind of amino acid, which comprises apart from the amino and carboxy group functional groups or atoms selected from the group consisting of O, N, S, P, CONH₂, COOH, CN and tetrazole.

A "charged amino acid" is any kind of amino acid which comprises, apart from the amino and carboxy group, functional groups which can be charged at a certain pH, esp. in the range of 4-8, preferably selected from the group of COOH, phosphate, phosphonate, sulfonate, ammonium and amino nitrogen.

A neutral amino acid is any kind of amino acid, which comprises apart from the amino and carboxy group, no further functional groups which can be charged at a certain pH, esp. in the range of 4-8.

If an amino acid contains more than one amino and/or carboxy group all orientations of this amino acid are in principle possible for formation of a covalent bond, but in α-amino acid the utilization of the α-amino and the α-carboxy group is preferred for the attachment to the neighbouring moieties and if other orientations are preferred they are explicitly specified.

Those skilled in the art will recognize if a stereocenter exists in the compounds disclosed herein irrespective thereof whether such stereocenter is part of an amino acid moiety or any other part or moiety of the compound of the invention. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley-lnterscience, 1994).

In an embodiment and as preferably used herein, an aromatic L-α-amino acid is any kind of L-α-amino acid, which comprises an aryl group.

In an embodiment and as preferably used herein, a heteroaromatic L-α-amino acid is any kind of L-α-amino acid which comprises a heteroaryl group.

Unless indicated to the contrary, the amino acid sequences are presented herein in N- to C-terminus direction.

Derivatives of the amino acids constituting the peptides of the invention may be as set forth in Table 5. In any embodiment, one or more amino acids of the compounds of the invention are substituted with a derivative of the corresponding preferred amino acids.

**Table 5: Exemplary derivatives of preferred amino acids contained in the compound of the invention**

| **Amino Acid** | **Exemplary derivatives** |
|---|---|
| Ala | Aib, Bal, Abu, Gly, Nva, Nle |
| Cys | Hcy, Nmc |
| Asp | Glu, Asn, Gln, Cya |
| Glu | Asp, Asn, Gln, Cya, Homoglutamic acid, γ-Hydroxy-glutamic acid, |
| Phe | Hfe, Phg, Bhf, Thi, Bta, Bromophenylalanine, Iodophenylalanine, Chlorophenylalanine, Methylphenylalanine, Nitrophenylalanine, Tyr, Trp, Naphthylalanine, Trifluoromethylphenylalanine |
| Gly | Ala, ala, Nmg |
| Nmg | Pro, Ala, ala, Gly, Nma, nma |
| Ile | Leu, Val, Hle, Nva, Nle, Chg |
| Lys | Arg, Dab, Dap, Har, Egd, Omr, Hci, Cit |
| Leu | Ile, Val, Hle, Nle, Nva, Moo |
| Met | Ile, Val, Hle, Nle, Nva, Moo |
| Nle | Ile, Val, Hle, Met, Nva, Moo |
| Asn | Asp, Glu, Gln, Cya, Thr |
| Pro | Aze, Pip, Hyp, Tfp, Cfp, Dmp, Tap, H3p, 4Ap, Cpp, Hym, Chy, Dfp |
| Gln | Asp, Asn, Glu, Cya, Thr, Hse |
| Arg | Arg, Dab, Dap, Har, Egd, Omr, Hci, Cit |
| Ser | Thr, Hse, *allo*-Threonine |
| Thr | Ser, Homothreonine, allo-Threonine |
| Val | Leu, Ile, Hle, Nva, Nle |
| Trp | Hfe, Phg, Bhf, Thi, Bta, Bromophenylalanine, Iodophenylalanine, Chlorophenylalanine, Methylphenylalanine, Nitrophenylalanine, Tyr, Trp, Naphthylalanine, Trifluoromethylphenylalanine |
| Tyr | Hfe, Phg, Bhf, Thi, Bta, Bromophenylalanine, Iodophenylalanine, Chlorophenylalanine, Methylphenylalanine, Nitrophenylalanine, Tyr, Trp, Naphthylalanine, Trifluoromethylphenylalanine |

### Linear Peptides

A general linear peptide is typically written from the N-to C-terminal direction as shown below:

A-Xaa1-Xaa2-Xaa3 -Xaa4-....... Xaan-C-term;

Therein
1. ,Xaax' is the abbreviation, descriptor or symbol for amino acids or building blocks at specific sequence position x as shown in Table 4,
2. ,A' is the N-terminal modification group A, which is typically is a Z group, wherein the Z group is a bio-distribution modifier optionally comprising a linker, linked to the N-terminal amino acid code (Xaa1) via a hyphen and
3. ,C-term' is the C-terminal group C-term, which is typically is a Z group, wherein the Z group is a bio-distribution modifier optionally comprising a linker, linked to the C-terminal amino acid code (Xaan) via a hyphen.

### Branched peptides with side chains modified by specific building blocks or peptides

Linear, branched peptides are written from the N-to C-terminal direction as shown below:

1. A-Xaa1-Xaa2-Xaa3(*NT-Xab1-Xab2-... .....Xabn*)-*........*Xaan-C-term

Therein, the statements 1. - 3. of the description of linear peptides for the specification of ,Xaax', ,A' and ,C-term' in the main chain of the branched peptide apply. An individual branch is specified by an expression in parentheses, wherein the positioning of the branch within the peptide sequence/structure is indicated by the positioning of the parenthesized expression right next to an 'Xaax' abbreviation (e.g. `Xaa3'). Branches typically either occur at lysine (Lys) residues or 4-aminopiperidine-4-carboxylic acid (Apc) (or similar), which means that the branch is attached to side chain ε-amino function of the lysine or to the nitrogen atom within the piperidine ring of 4-aminopiperidine-4-carboxylic acid via an amide bond.

The content of the parentheses describes the sequence/structure of the peptide branch *'NT-Xab1-Xab2-... ....Xabn' .* Herein
1. *,Xabx'* is the abbreviation, descriptor or symbol for amino acids or building blocks at specific sequence position x of the branch as shown in Table 4, whereby the linear connection of individual Xabx is indicated by a hyphen,
2. *,NT'* is an N-terminal group, e.g. an abbreviation for a specific terminating carboxylic acid like 'Ac' for acetyl or other chemical group or structural formula of chemical groups, more specifically *'NT'* is a Z group, linked to the N-terminal amino acid code (*Xab1*) via a hyphen and
3. the last building block of the branch ,*Xabn'*, which connects the branch with the main chain by forming an amide bond with its own carboxyl function with the side chain amino function of this lysine or 4-aminopiperidine-4-carboxylic acid (or similar residue).

### Cyclic peptides

An exemplary general cyclic peptide written from the N- to C-terminal direction is shown below:

A-Xaa1-[Xaa2-Xaa3-Xaa4-.......Xaan]-C-term;

Therein the statements 1. - 3. of the description of linear peptides for the specification of ,Xaax', ,A' and ,C-term' in the main chain of the cyclic peptide apply. The characteristics of the peptide cycle are specified by square brackets.
1. The opening square bracket indicates the building block at whose side chain the cycle is initiated and
2. the closing square bracket indicates the building block at whose side chain the cycle is terminated.

The chemical nature of the connection between these two residues within the peptide macro cycle is
a disulphide bond in case that those indicated residues/amino acids contain sulfhydryl moieties (e.g., Cys).

### Cyclic peptides containing an additional cyclization element (Yc)

A general extended cyclic peptide written from the N-to C-terminal direction is shown below:

A-Xaa1-[Xaa2(Yc)-Xaa3-Xaa4-.......Xaan]-C-term;

Therein the statements 1. - 3. of the description of linear peptides for the specification of,Xaax', ,A' and ,C-term' in the main chain of the cyclic peptide apply. In addition, ,Yc' is the cyclization element. As in case of cyclic peptides, the characteristics of the cycle are specified by square brackets which indicate the building block at whose side chain the cycle is initiated and the building block at whose side chain the cycle is terminated (statements 1. and 2. of cyclic peptides).

The abbreviation of the structure representing the Yc element of the extended cycle is the content of the parentheses left adjacent to the building block at whose side chain the cycle is initiated. The Yc element is covalently attached to the side chain of said building block. Furthermore, the Yc element is covalently attached to the side chain of the building block at whose side chain the cycle is terminated. The chemical nature of the linkages between either of these residues the Yc element depend on side chain functionality of both corresponding amino acids Xaan. The linkage is a thioether if the side chain of Xaan contains a sulfhydryl group (e.g., Cys).

As non-limiting example for a branched, cyclic peptide containing an additional cyclization element peptide the structure of 
HPD2:Ahx-Asp-Trp-5Clw-Tap-Glu-leu-[Cys(2Lut)-Apc(DOTA)-Leu-Ile-Cys]-APAc-
lys:HPD2
 is depicted below.

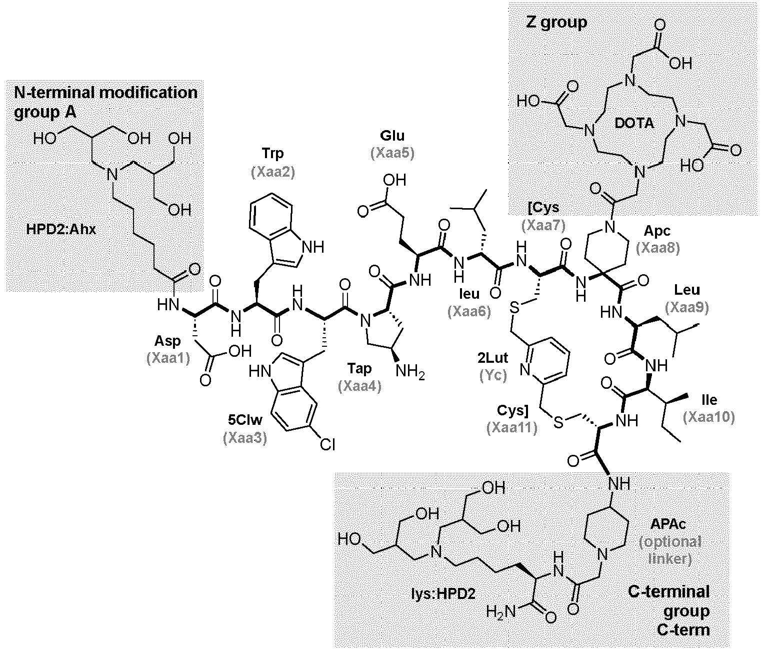

Therein
1. HPD2:Ahx is a bio-distribution modifier bound to the N-terminal Asp (Xaa1) and corresponds to the N-terminal modification group A' in the general formula.
2. Asp, Trp, 5Clw, Hyp, Glu, leu, Cys, Apc, Leu, Ile, Cys correspond to ,Xaa1' to ,Xaa11' in the general formula. For clarity the main chain is represented with bold line thickness.
3. APAc as an optional linker and lys:HPD2, which is a bio-distribution modifier correspond to the C-terminal group C-term' in the general formula. APAc and lys:HPD2 are linked to Cys (Xaa11) as depicted.
4. The opening square bracket (,[`) adjacent to the N-terminal cysteine (Xaa7) in the sequence indicates that at this residue the cycle is initiated.
5. The closing square bracket (,]`) adjacent to the N-terminal cysteine (Xaa11) in the sequence indicates that at this residue the cycle is terminated.
6. 2Lut within the parentheses adjacent to the Cys (Xaa7) indicates the initiation of the peptide macrocycle as initiation residue specifies the cyclization element ,Yc'. It is further bound to the Cys (Xaa11), which indicates the termination of the cycle. The Yc element is connected to said residues via thioether linkages.
7. To the amino group of the Apc residue a Z group is attached, which in the given example is chelator, specifically the chelator is DOTA.

In accordance with the present invention, the compound of the present invention may comprise one or more of a Z group. The Z group comprises a bio-distribution modifier with an optional linker, a chelator with an optional linker. As preferably used, a linker is an element, moiety, or structure, which separates two parts of a molecule. In the present invention, the linker forms a covalent bond with the chelator or the bio-distribution modifier or both and the respective part of the compounds of invention where the Z group is attached. A first functional group of the linker forming the bond with the chelator or the bio-distribution modifier may in principle, be any chemical group, which is capable of forming a bond, preferably a covalent bond with the chelator or the bio-distribution modifier group. In addition, the linker comprises a second functional group forming the bond, preferably a covalent bond with the part of the compounds of invention at the specified positions, where the Z group is attached. Such second functional group may in principle, be any chemical group, which is capable of forming such bond with said part of the compound of invention at the specific positions where the Z group is attached.

An important property or feature of a linker is that it spaces apart the chelator or the bio-distribution modifier from the peptide part of the compound of invention. This is especially important in cases where the target binding ability of the peptide is compromised by the close proximity of the chelator or the bio-distribution modifier. However, the overall linker length in its most extended conformer should not exceed 200 Å, preferably not more than 150 Å and most preferably not more than 100 Å.

A further important feature or property of a linker is that it enables the attachment of the chelator or the bio-distribution modifier to suitable positions of the peptide part of the compound of invention.

In an embodiment, a residue of a dicarboxylic acid represents a building block for a linker. Dicarboxylic acid-based linkers are used to enable the attachment of a chelator or a bio-distribution modifier to the N-terminal amine of the peptide of invention or an amino function of the side chain of an amino acid residue of the peptide of invention. Such N-terminal amine or amino side chain provides an amino function as conjugation group to which one of the at least two carboxyl groups provided by the dicarboxylic acid building block is covalently attached forming an amide linkage. The bio-distribution modifier or chelator provides an amino function as conjugation group to which another of the at least two carboxyl groups provided by the dicarboxylic acid building block is covalently attached forming an amide linkage, too. The very kind of dicarboxylic acid used as building block for a linker is dependent on the nature of the bio-distribution modifier or chelator and the attachment site on the peptide of invention and more specifically any functional group available at or provided by the bio-distribution modifier and chelator, respectively, which is reacted with one of the at least two carboxyl groups of the dicarboxylic acid building block.

Preferred building blocks are dicarboxylic acids, which are selected from formulae (Lin1), (Lin2) or (Lin3)

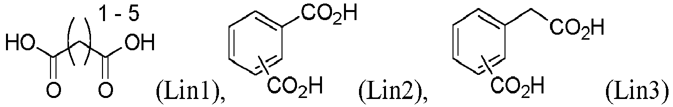

More preferred dicarboxylic acid building blocks such as formula (Lin1) are malonic acid, succinic acid, glutaric acid, adipic acid, wherein the two carboxylic acid groups are attached to ends of the aliphatic chain; such as formula (Lin2) phthalic acid, terephthalic acid, isophthalic acid and such as formula (Lin3) 2-, 3- or 4-carboxy-phenyl acetic acid. The most preferred dicarboxylic acid building block is glutaric acid (Glutar).

In an embodiment, a residue of a diamine represents a building block for a linker. Diamine based linkers are used to enable the attachment of a chelator or a bio-distribution modifier to the C-terminal carboxylic acid moiety of the peptide of invention or a carboxylic acid function of the side chain of an amino acid residue of the peptide of invention. Such C-terminal carboxylic acid or carboxylic acid side chain provides a carboxylic acid function as conjugation group to which one of the at least two amino groups provided by the diamine is covalently attached forming an amide linkage. The bio-distribution modifier or chelator provides a carboxylic acid as conjugation group to which another of the at least two amino groups provided by the diamine building block is covalently attached forming an amide linkage, too. The very kind of diamine used as building block for a linker is dependent on the nature of the bio-distribution modifier or chelator and the attachment site on the peptide of invention and more specifically any functional group available at or provided by the bio-distribution modifier and chelator, respectively, which is reacted with one of the at least two amino groups of the diamine building block.

Preferred building blocks are diamines, which are selected from formulae (Lin4), (Lin5), (Lin6) or (Lin7)

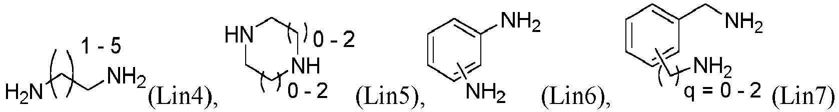

More preferred diamine building blocks are ethylenediamine (en), 1,4-diaminobutane, 1,5-diaminopentane (Ape), 1,6-diaminohexane, 1,3-dazetine, piperazine (PP), 1,3-diazinane, o-, m- and p-phenylenediamine, 4-aminobenzylamine, 1,4-bis(aminomethyl)benzene. The most preferred diamino building blocks are 1,5-diaminepentane (Ape), ethylenediamine (en) and piperazine (PP).

In an embodiment, a residue of an amino acid represents the building block for a linker. Amino acid-based linkers are used to enable the attachment of a chelator or a bio-distribution modifier to N-terminal amine, an amino function of the side chain of an amino acid residue or the C-terminal carboxylic acid moiety of the peptide of invention. Such N-terminal amine or amino side chain of the peptide of invention provides an amino function to which the carboxylic acid moiety of the amino acid linker is attached covalently by formation of an amide bond. The bio-distribution modifier or chelator provides a carboxylic acid as conjugation group, to which the amino function of the amino acid linker is attached covalently by formation of an amide bond. Furthermore, to the amino function of the linker, the carboxylic acid moiety of a further linker can be attached covalently by an amide bond, which preferably is an α-amino acid linker. As an alternative such C-terminal carboxylic acid moiety of the peptide of invention provides a carboxylic acid group to which the amino function of the amino acid linker is attached covalently by formation of an amide bond. In this case the bio-distribution modifier or chelator provides an amine as conjugation group to which the carboxylic acid moiety of the linker is attached covalently by formation of an amide bond. Furthermore, to the carboxylic acid moiety of the linker, the amino function of a further linker can be attached covalently by an amide bond, which preferably is an α-amino acid linker.

Preferred building blocks are amino acids, which are selected from formulae (Lin8), (Lin9), (Lin10), Lin(11) or Lin(12).

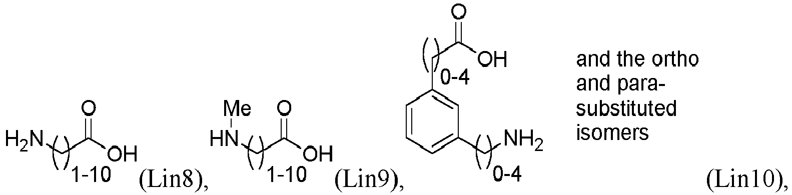

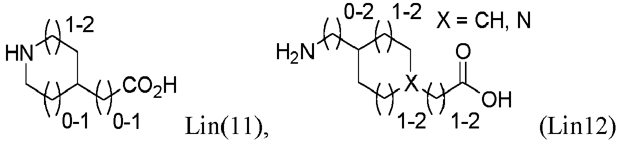

It is within the present invention that such amino acid linker is not further substituted. It is, however, also within the present invention that such amino acid linker is further substituted; preferably such substitution is CO-NH₂ and/or Ac-NH-.

Representatives of this kind of amino acid (structure Lin8), which can be used as a linker building block are glycine (Gly), beta-alanine (Bal), gamma-aminobutyric acid (GABA), 5-amino-pentanoic acid, 6-amino-hexanoic acid and homologs with up to 10 CH₂ groups.

Further representatives of this kind of amino acid (structure Lin9), which can be used as a linker building block are N-methylglycine (Nmg), N-methyl-beta-alanine, N-Methyl-gamma-aminobutyric acid, N-methyl-5-amino-pentanoic acid, N-methyl-6-amino-hexanoic acid and homologs with up to 10 CH₂ groups.

Further representatives of this kind of amino acid (structure Lin10), which can be used as a linker building block are 3-aminomethyl-benzoic acid, 4-aminomethyl-benzoic acid, anthranilic acid, 3-amino-benzoic acid and 4-amino-benzoic acid.

Further representatives of this kind of amino acid (structure Lin11), which can be used as a linker building block are 4-carboxymethyl-piperidine (Cmp), 3-piperidinecarboxylic acid (Nipecotic acid, Rni/Sni) and 4-piperidinecarboxylic acid (Isonipecotic acid, Inp).

Further representatives of this kind of amino acid (structure Lin12), which can be used as a linker building block are (4-aminopiperidin-1-yl)acetic acid (APAc), 2-(piperazin-1-yl)-acetic acid (PPAc) and 4-(aminomethyl)cyclohexanecarboxylic acid (4Amc).

It will be appreciated by a person skilled in the art that in case of amino acids with stereogenic centers all stereoisomeric forms may be used as such a linker.

In a further embodiment, the amino acid is an amino acid which contains, preferably as a backbone, a polyether. Preferably such polyether is polyethylene glycol and consists of up to 30 monomer units. Preferably, an amino acid comprising such polyether shows an increase in hydrophilicity compared to an amino acid not comprising such polyether. If incorporated as a linker the result is typically an increase in hydrophilicity. A preferred embodiment of this kind of amino acid is depicted in the following, wherein it will be acknowledged that such amino acid may comprise 0, 1, 2, 3, 4, 5, 6, 7, 8, 9, 10, 11 or 12 ethylene oxide moieties:

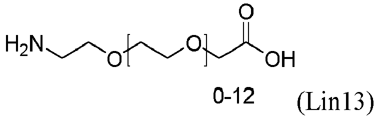

Preferred ethylene glycol containing amino acids are Ttds, O2Oc and PEG6.

In a further embodiment, a residue of an alpha amino acid represents a building block for a linker. Alpha amino acid-based linkers are used to enable the attachment of a chelator and/or a bio-distribution modifier to N-terminal amine, an amino function of the side chain of an amino acid residue of the peptide of invention or the C-terminal carboxylic acid moiety of the peptide of invention. Thereby simultaneous a chelator and a bio-distribution modifier or two bio-distribution modifiers can attached to one site of the peptide of the invention. Such N-terminal amine or amino side chain provides an amino function to which one of the one or more carboxy groups provided by the alpha amino acid building block is covalently attached forming an amide linkage. Such C-terminal carboxylic acid moiety provides a carboxylic acid function as conjugation group to which one of the one or more amino groups provided by the alpha amino acid covalently attached forming an amide linkage. In such cases wherein the alpha amino acid has more than one amino function the bio-distribution modifier or chelator provides a carboxylic acid moiety as conjugation group, which is attached covalently to said amino function of the alpha amino acid forming an amide linkage, while the other one of the amines is used as linkage to peptide of invention. In such cases wherein the alpha amino acid has more than one carboxylic acid moieties the bio-distribution modifier or chelator provides an amine as conjugation group, which is attached covalently to said carboxylic acid moiety of the alpha amino acid forming an amide linkage, while the other one of the carboxylic acid moieties is used as linkage to peptide of invention.

Preferred building blocks are alpha amino acids, which are selected from formula (Lin14) or (Lin 15)

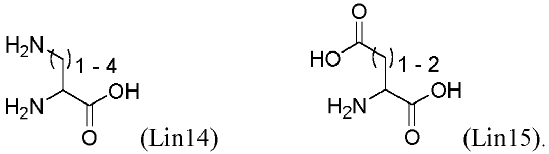

More preferred alpha amino acids building blocks are di-amino-propionic acid (Dap), di-aminobutyric acid (Dab), ornithine, lysine (Lys), aspartic acid and glutamic acid. The most preferred alpha amino acid building blocks are lysine and glutamic acid. It will be appreciated by a person skilled in the art that in case of alpha amino acids with stereogenic centers all stereoisomeric forms may be used as such a linker.

However, the use of linkers usually follows a purpose. In some circumstances it is necessary to space a larger moiety apart from a bioactive molecule in order to retain high bioactivity. In other circumstances introduction of a linker opens the chance to tune physicochemical properties of the molecule by introduction of polarity or multiple charges. In certain circumstances the site of attachment within the sequence of the peptide of invention and the chemical nature of the bio-distribution modifier or chelator necessitate the implementation of a linker. Further, in certain circumstances it might be a strength and achievement if one can combine the chelator with a bioactive compound without the need for such linkers. Compounds comprising C terminal bio-distribution modifiers typically comprise amino acids for the linkage of the bio-distribution modifier to the bioactive compound. Compounds of the present invention where the chelator is directly attached to the side chain of a residue of the bioactive compound under the formation of an amide bond typically perform excellently without the use of any dedicated linker.

It will be acknowledged by a person skilled in the art that the radioactive nuclide which is or which is to be attached to the compound of the invention, is selected taking into consideration the disease to be treated and/or the disease to be diagnosed, respectively, and/or the particularities of the patient and patient group, respectively, to be treated and to be diagnosed, respectively.

In an embodiment of the present invention, the radioactive nuclide is also referred to as radionuclide. Radioactive decay is the process by which an atomic nucleus of an unstable atom loses energy by emitting ionizing particles (ionizing radiation). There are different types of radioactive decay. A decay, or loss of energy, results when an atom with one type of nucleus, called the parent radionuclide, transforms to an atom with a nucleus in a different state, or to a different nucleus containing different numbers of protons and neutrons. Either of these products is named the daughter nuclide. In some decays the parent and daughter are different chemical elements, and thus the decay process results in nuclear transmutation (creation of an atom of a new element). For example, the radioactive decay can be alpha decay, beta decay, and gamma decay. Alpha decay occurs when the nucleus ejects an alpha particle (helium nucleus). This is the most common process of emitting nucleons, but in rarer types of decays, nuclei can eject protons, or specific nuclei of other elements (in the process called cluster decay). Beta decay occurs when the nucleus emits an electron (β'-decay) or positron (β⁺-decay) and a type of neutrino, in a process that changes a proton to a neutron or the other way around. By contrast, there exist radioactive decay processes that do not result in transmutation. The energy of an excited nucleus may be emitted as a gamma ray in gamma decay, or used to eject an orbital electron by interaction with the excited nucleus in a process called internal conversion, or used to absorb an inner atomic electron from the electron shell whereby the change of a nuclear proton to neutron causes the emission of an electron neutrino in a process called electron capture (EC), or may be emitted without changing its number of proton and neutrons in a process called isomeric transition (IT). Another form of radioactive decay, the spontaneous fission (SF), is found only in very heavy chemical elements resulting in a spontaneous breakdown into smaller nuclei and a few isolated nuclear particles.

In a preferred embodiment of the present invention, the radionuclide can be used for labeling of the compound of the invention.

In an embodiment of the present invention, the radionuclide is suitable for complexing with a chelator, leading to a radionuclide chelate complex.

In a further embodiment one or more atoms of the compound of the invention are of non-natural isotopic composition, preferably these atoms are radionuclides; more preferably radionuclides of carbon, oxygen, nitrogen, sulfur, phosphorus and halogens: These radioactive atoms are typically part of amino acids, in some case halogen containing amino acids, and/or building blocks and in some cases halogenated building blocks each of the compound of the invention.

In a preferred embodiment of the present invention, the radionuclide has a half-life that allows for diagnostic and/or therapeutic medical use. Specifically, the half-life is between 1 min and 100 days.

In a preferred embodiment of the present invention, the radionuclide has a decay energy that allows for diagnostic and/or therapeutic medical use. Specifically, for γ-emitting isotopes, the decay energy is between 0.01 and 4 MeV, preferably between 0.08 and 0.4 MeV, for diagnostic use. For positron-emitting isotopes, the mean decay energy is between 0.2 and 3 MeV, preferably between 0.2 and 1 MeV, for diagnostic use. For β⁻-emitting isotopes, the mean decay energy is between 0.02 and 3 MeV, preferably between 0.1 and 1 MeV, for therapeutic use. For α-emitting isotopes, the decay energy is between 3 and 8 MeV, preferably between 3.9 and 6.4 MeV, for therapeutic use.

In a preferred embodiment of the present invention, the radionuclide is industrially produced for medical use. Specifically, the radionuclide is available in GMP quality.

In a preferred embodiment of the present invention, the daughter nuclide(s) after radioactive decay of the radionuclide are compatible with the diagnostic and/or therapeutic medical use. Furthermore, the daughter nuclides are either stable or further decay in a way that does not interfere with or even support the diagnostic and/or therapeutic medical use. Representative radionuclides which may be used in connection with the present invention are summarized in Table 6.

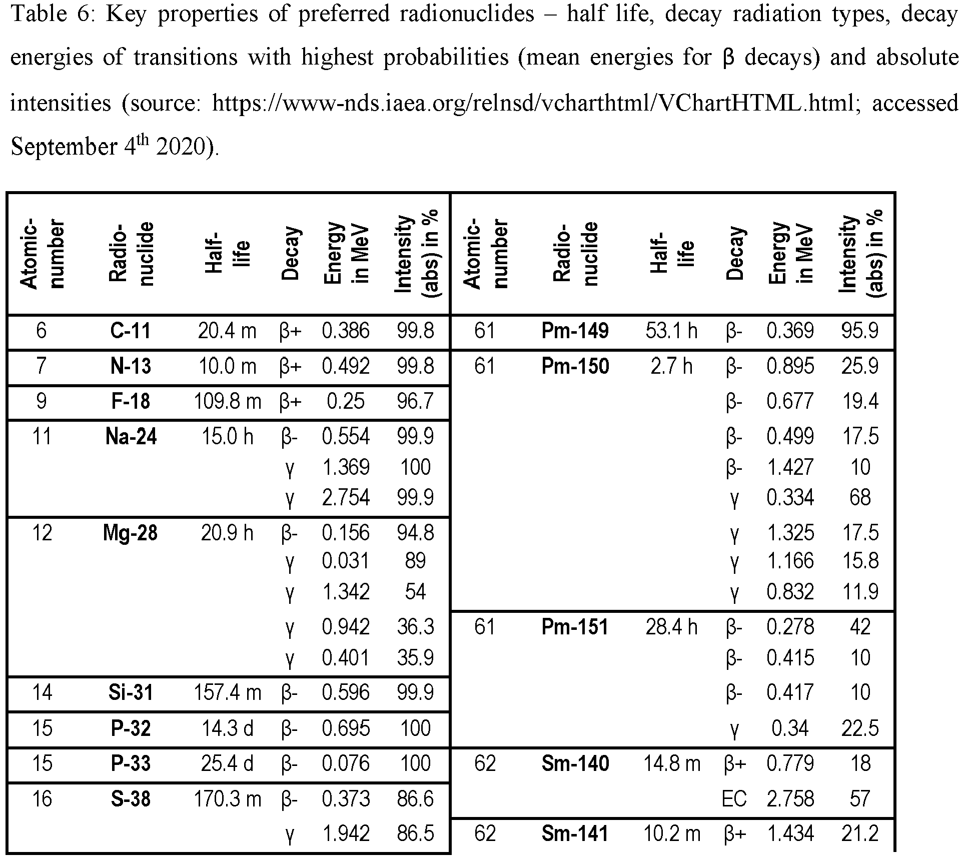

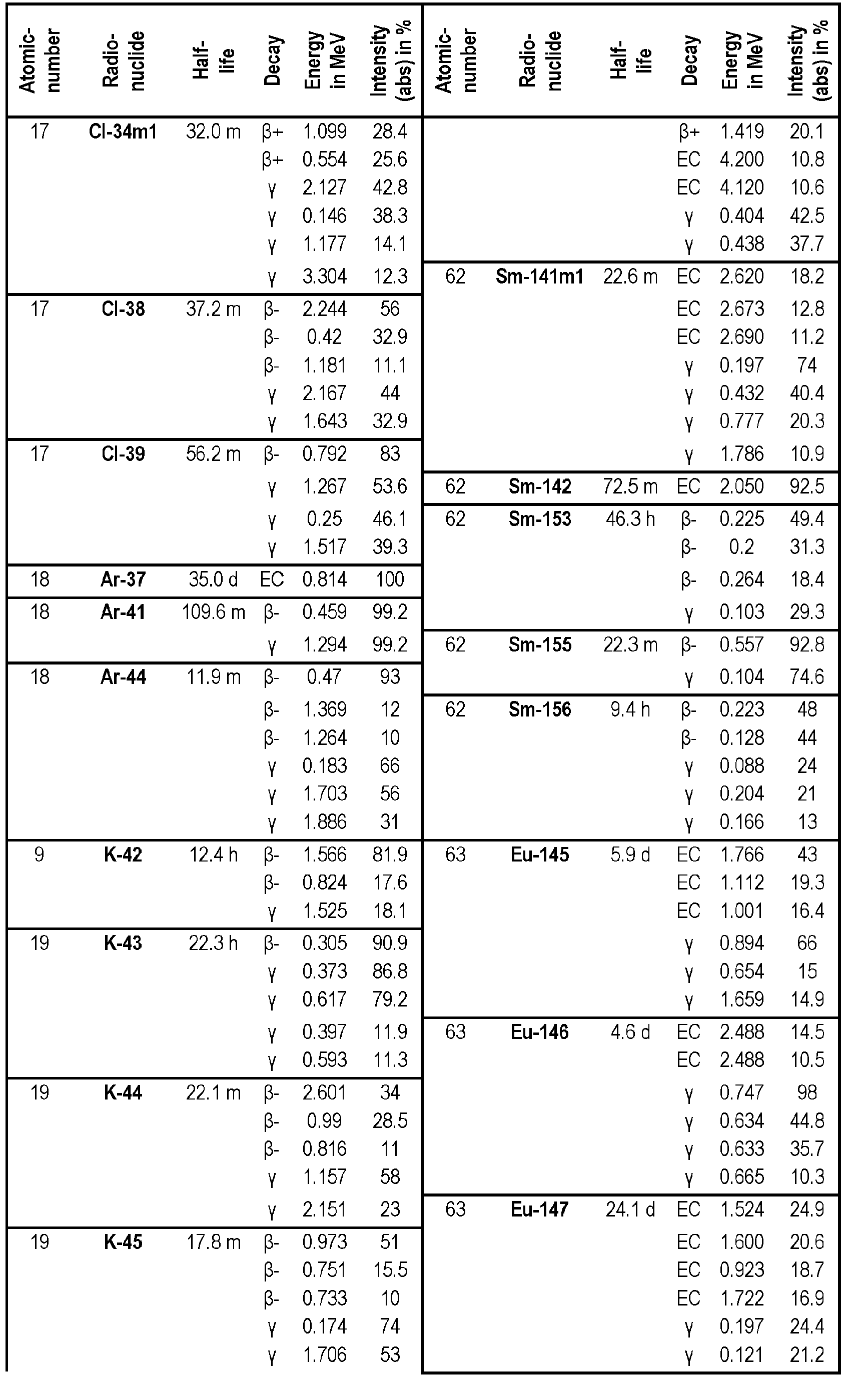

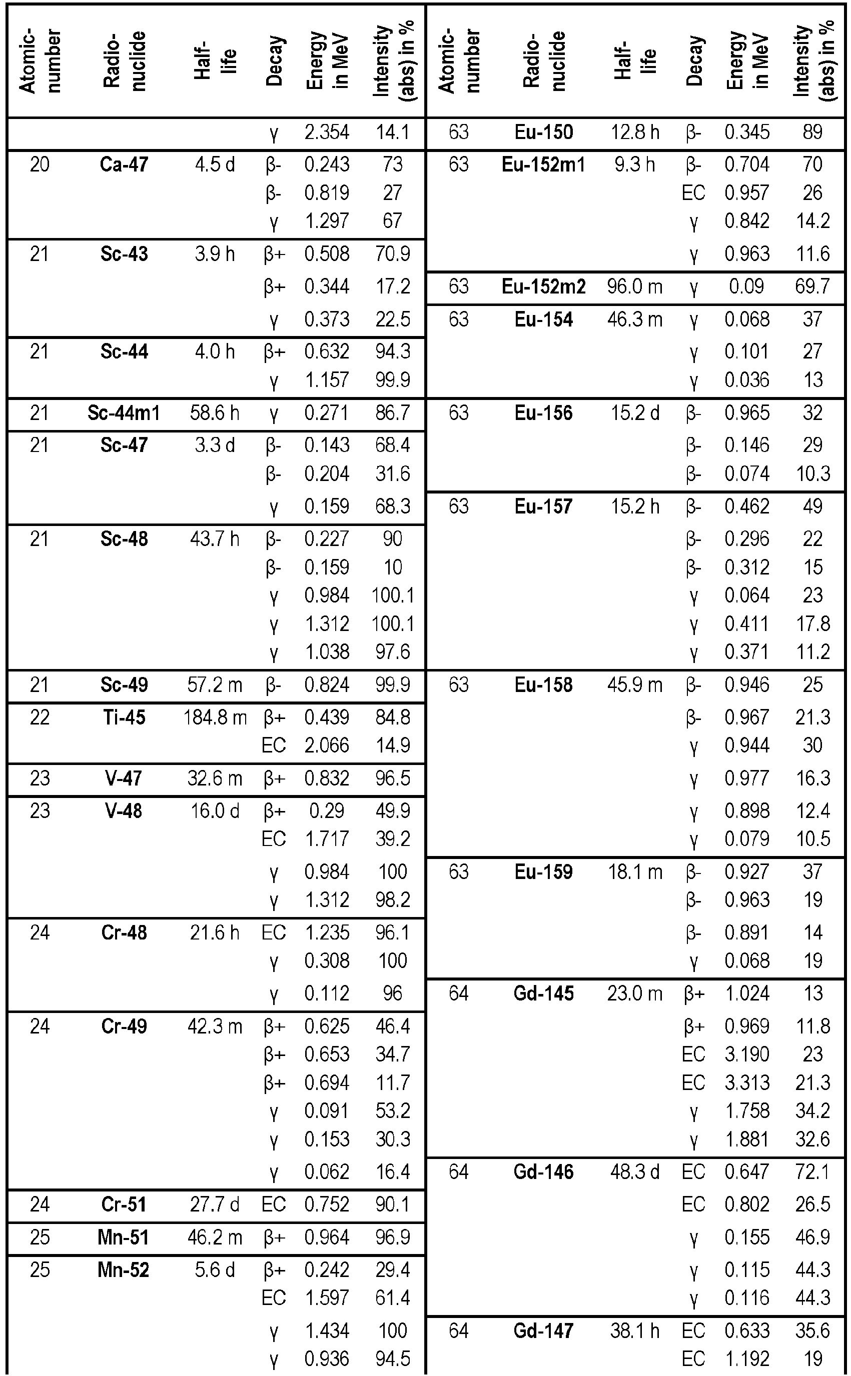

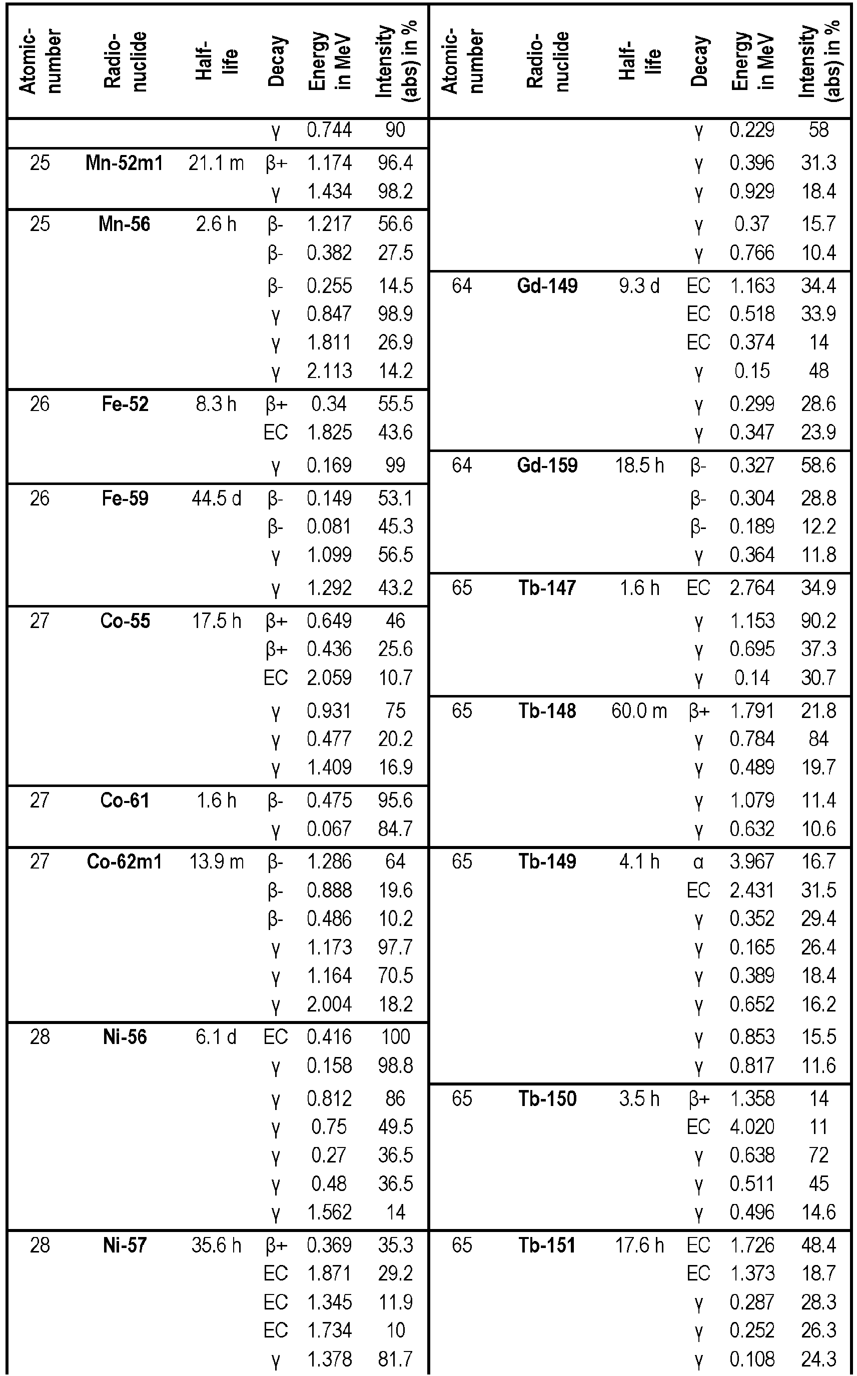

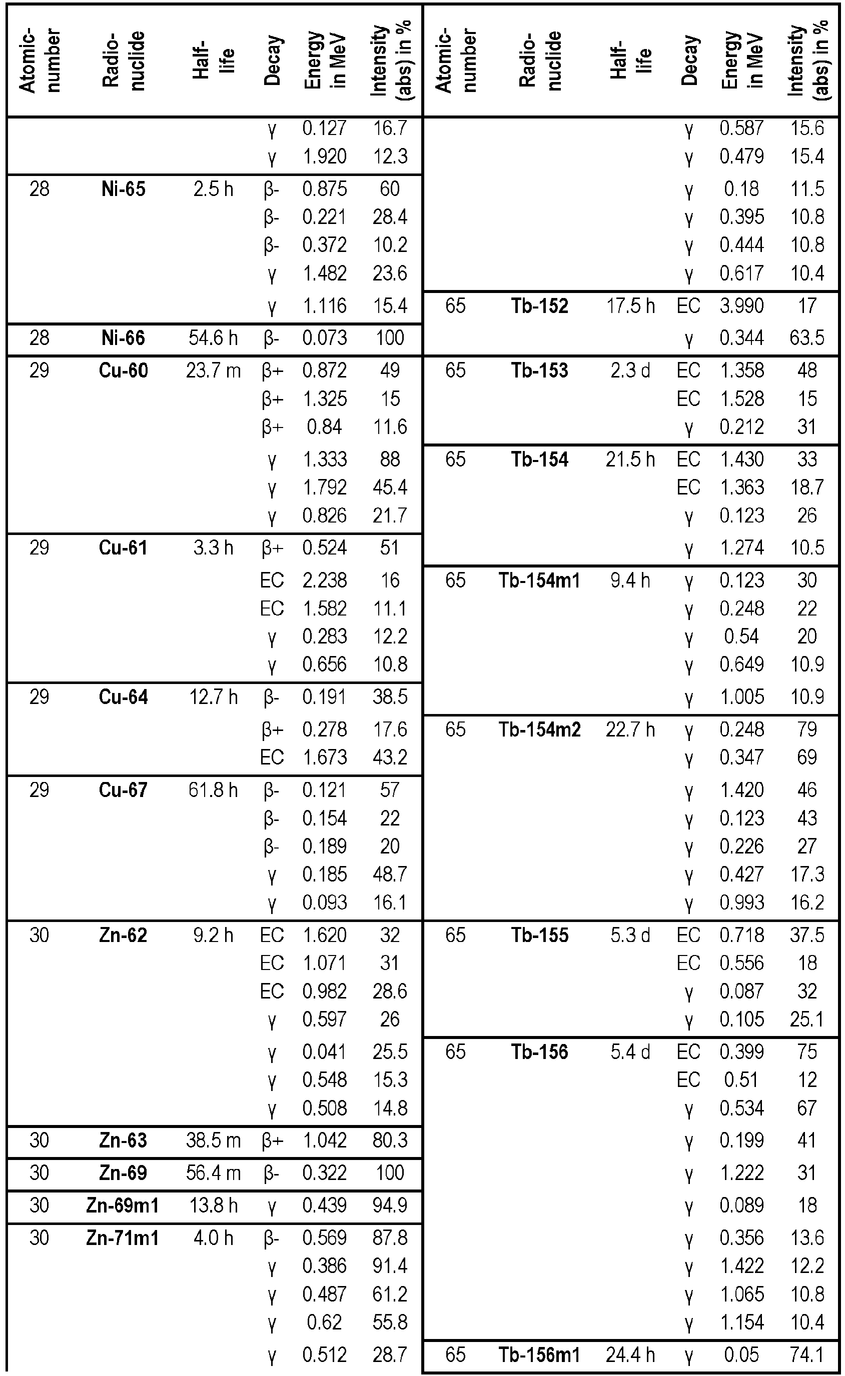

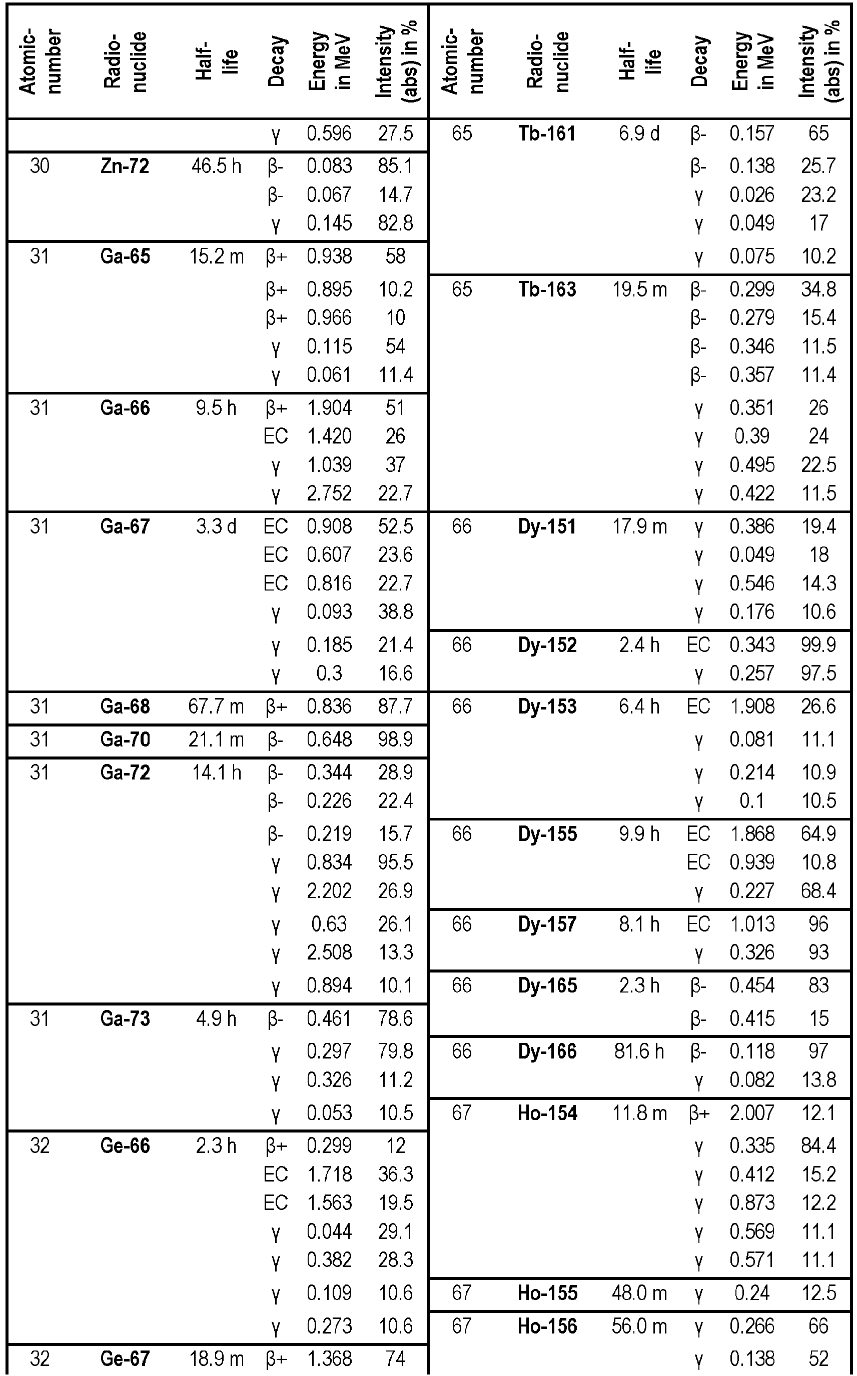

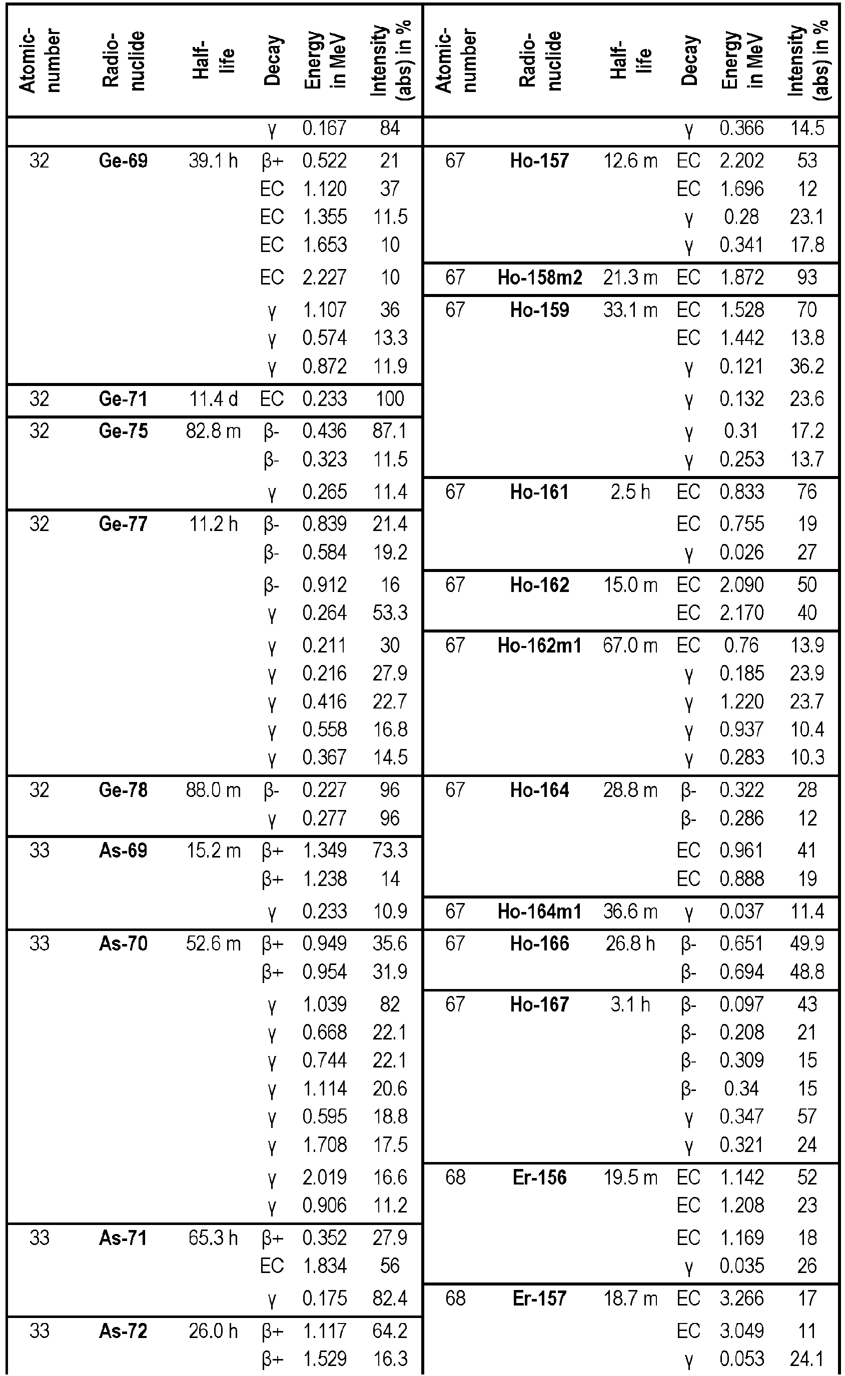

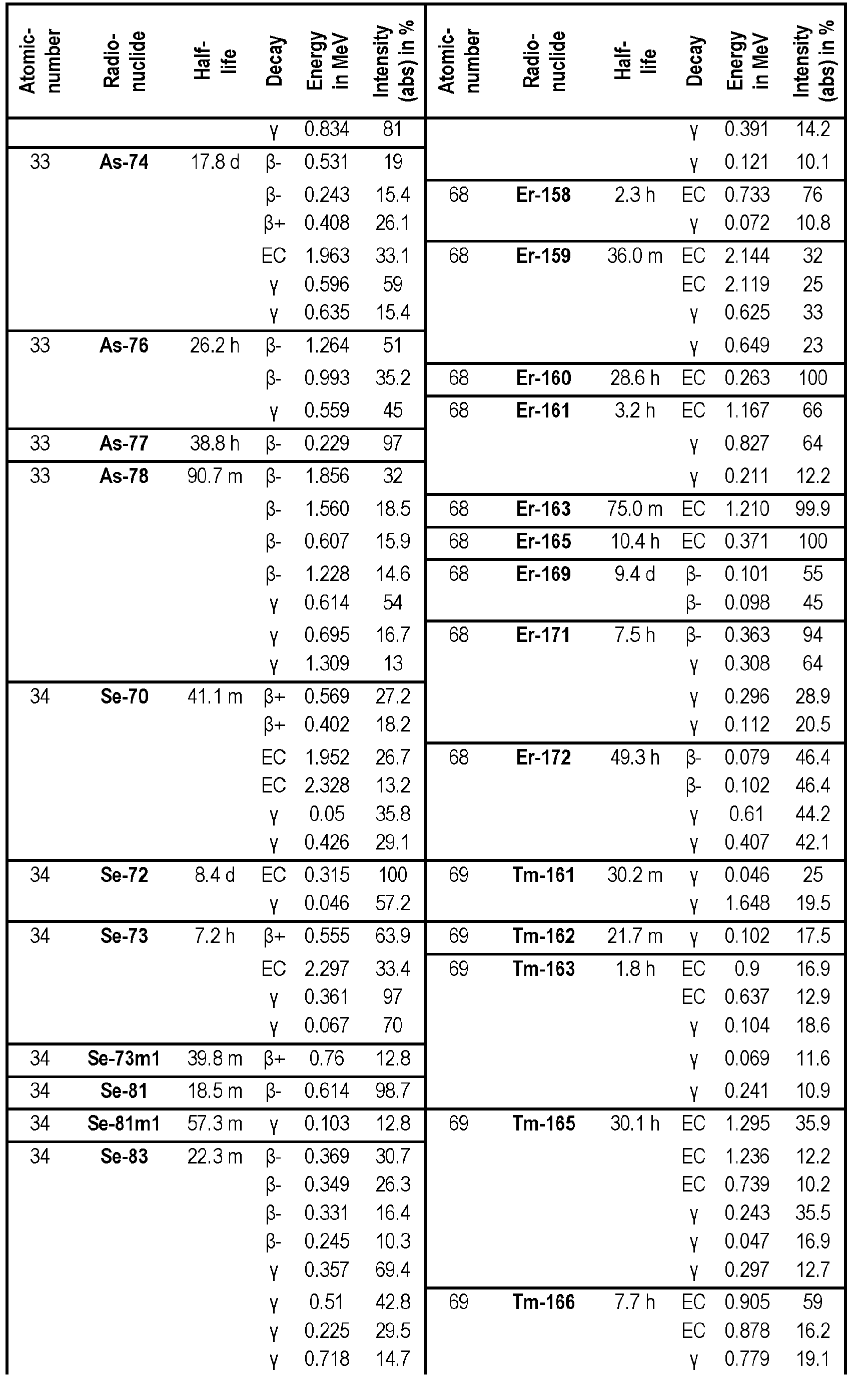

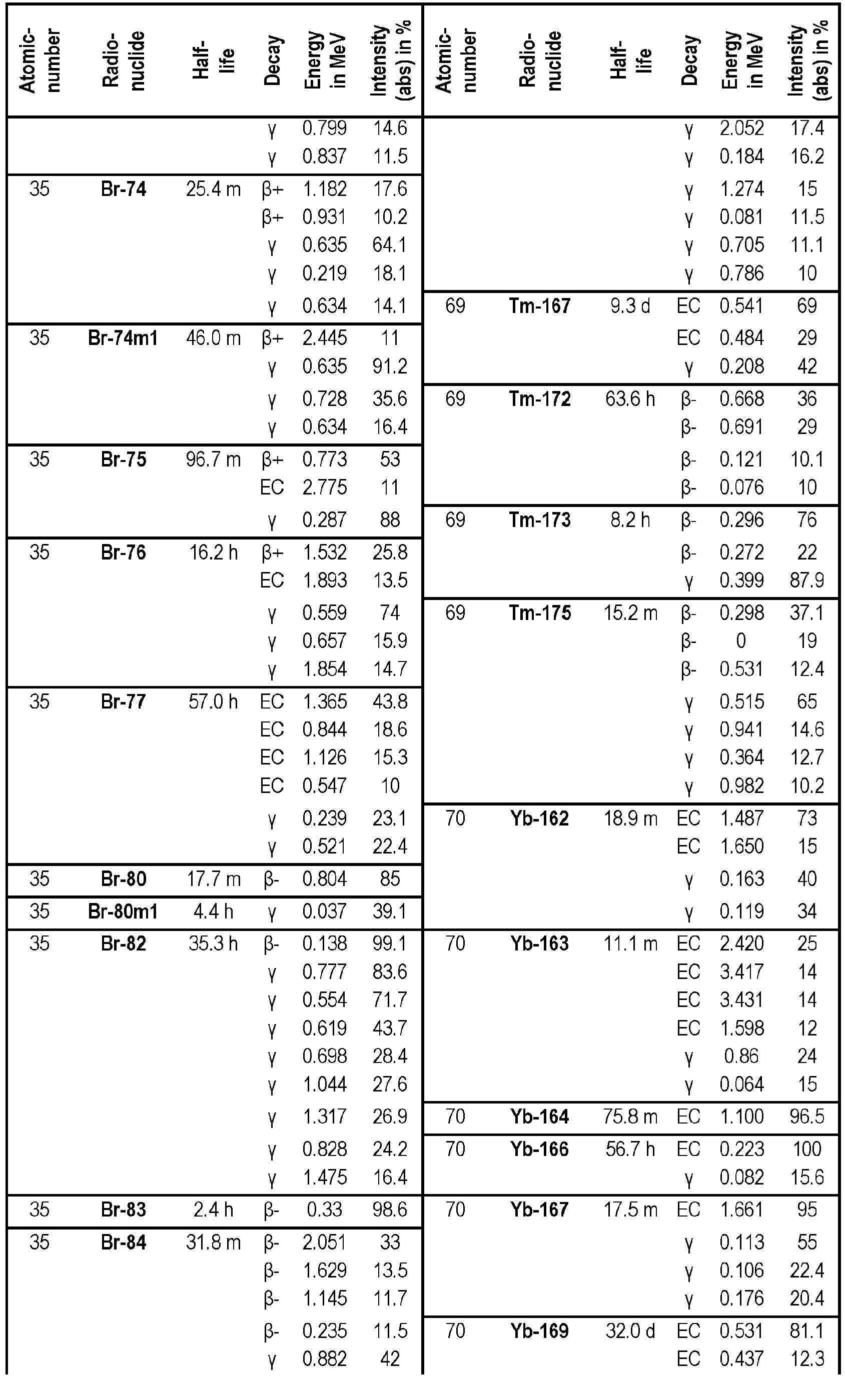

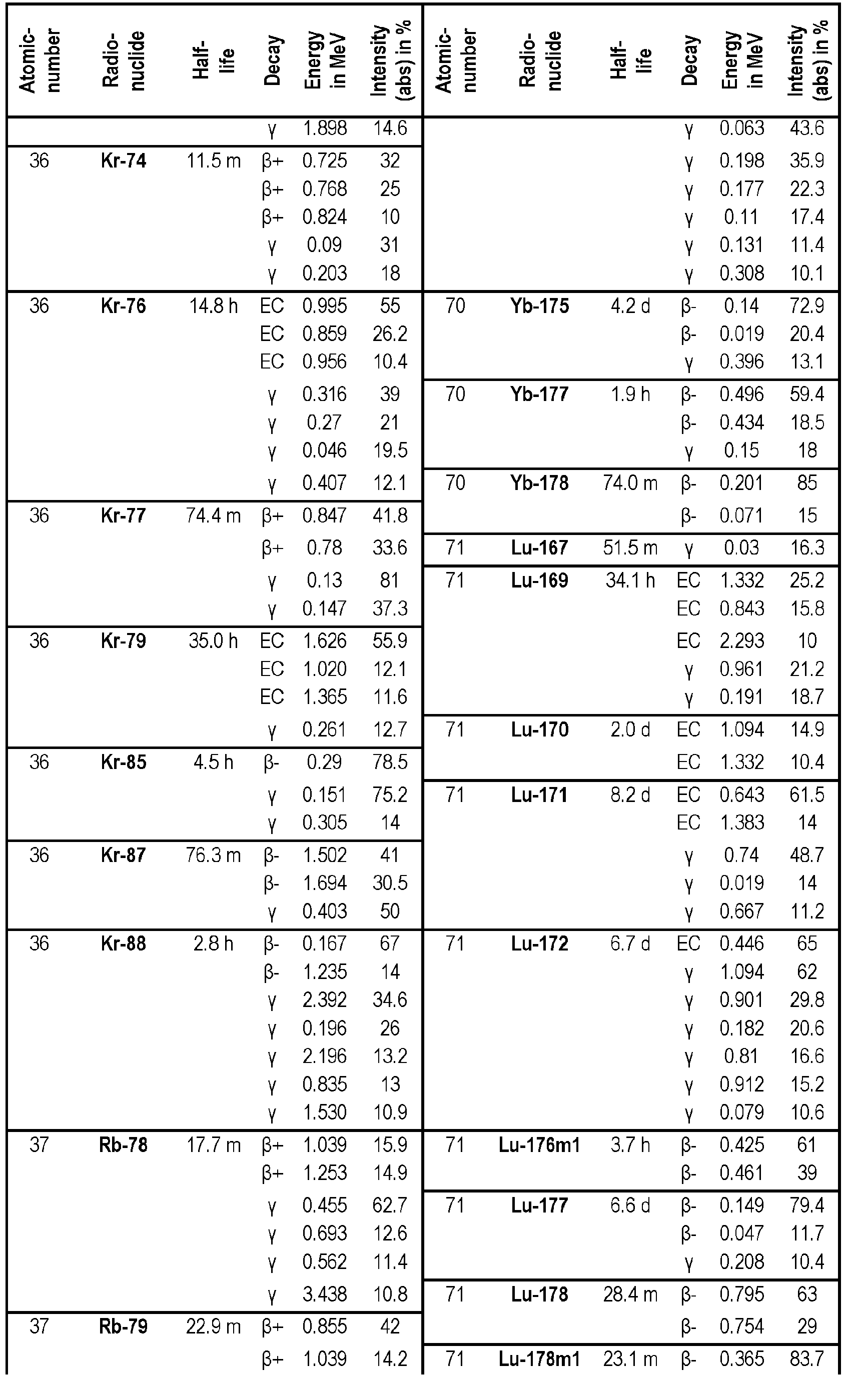

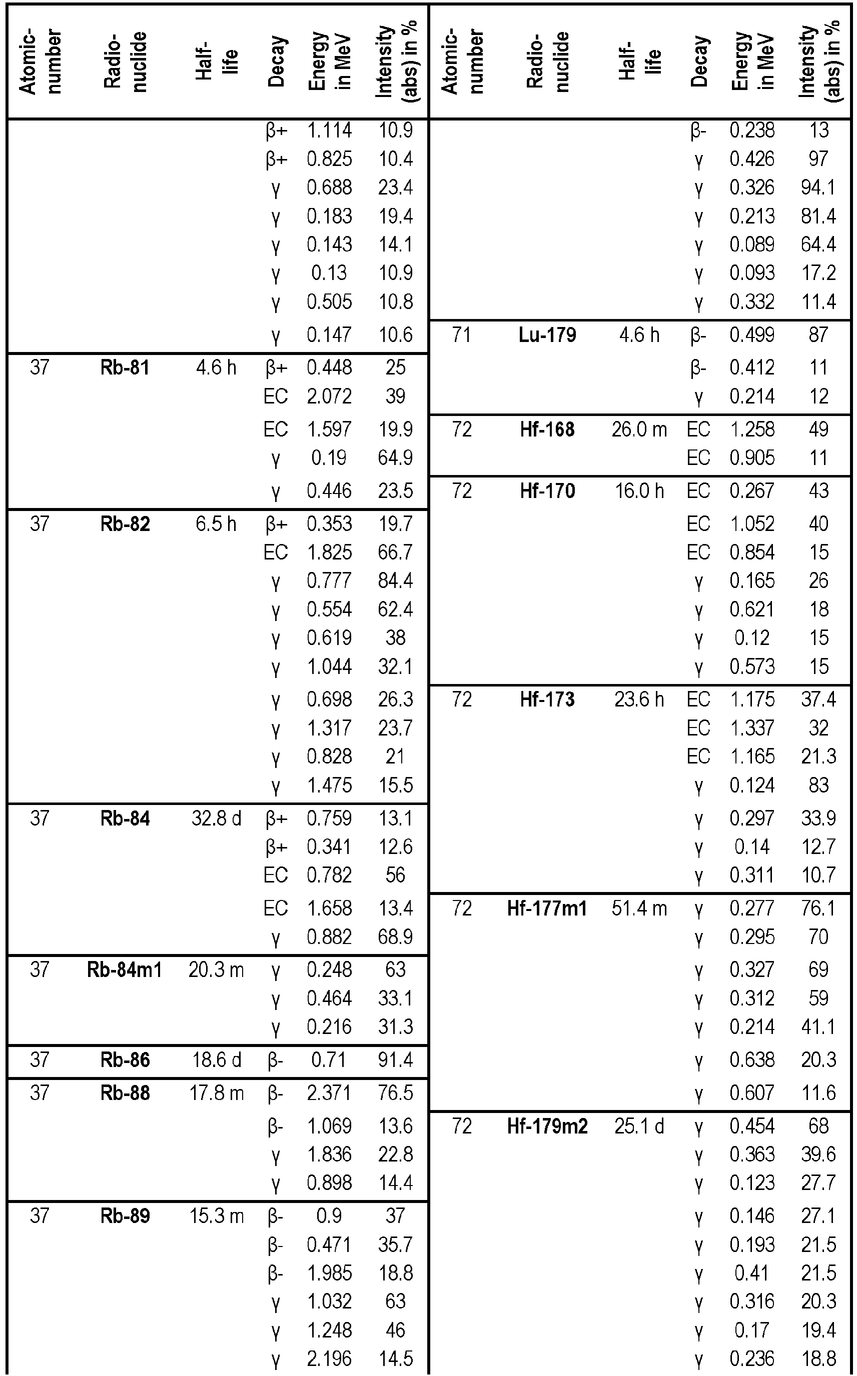

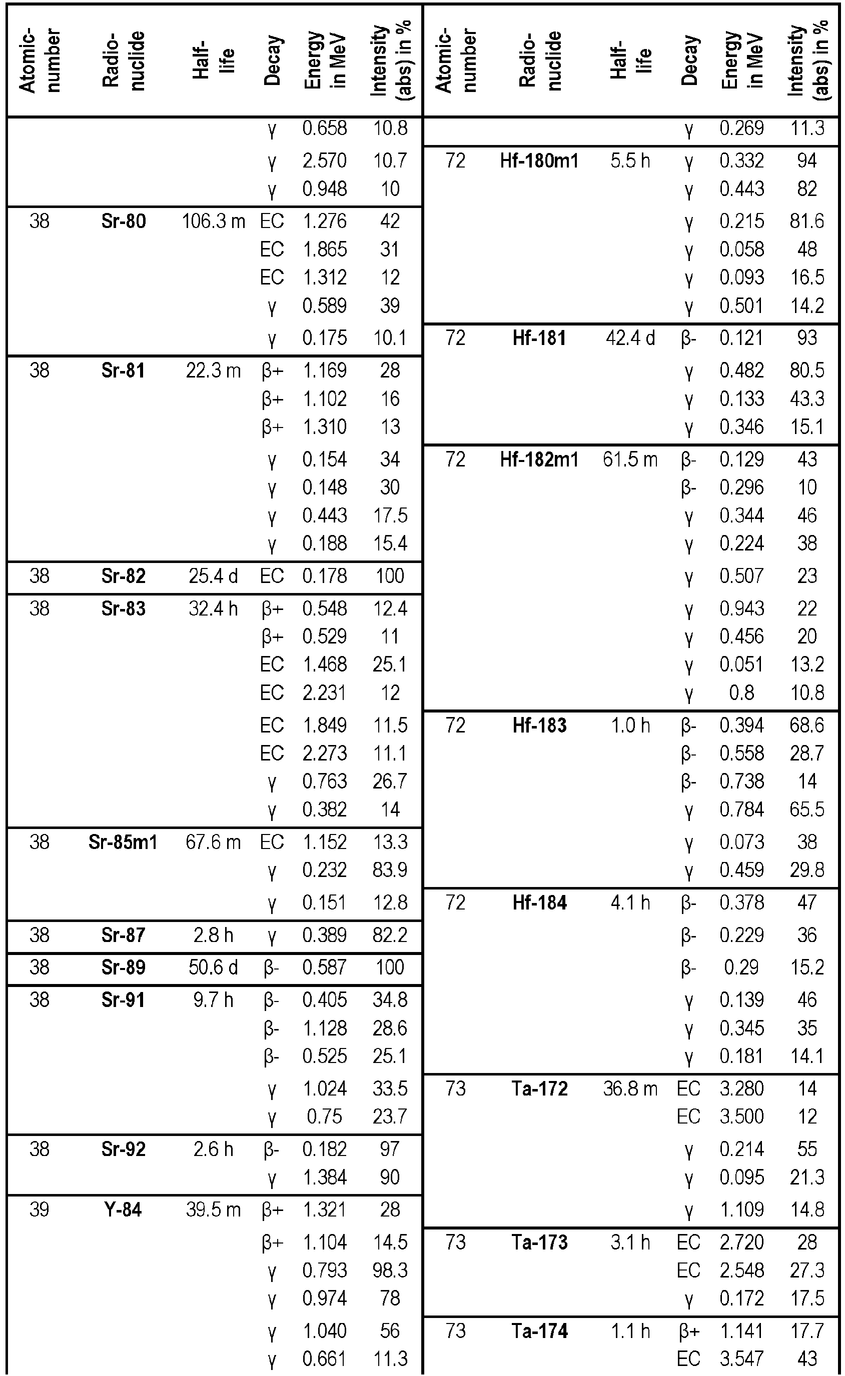

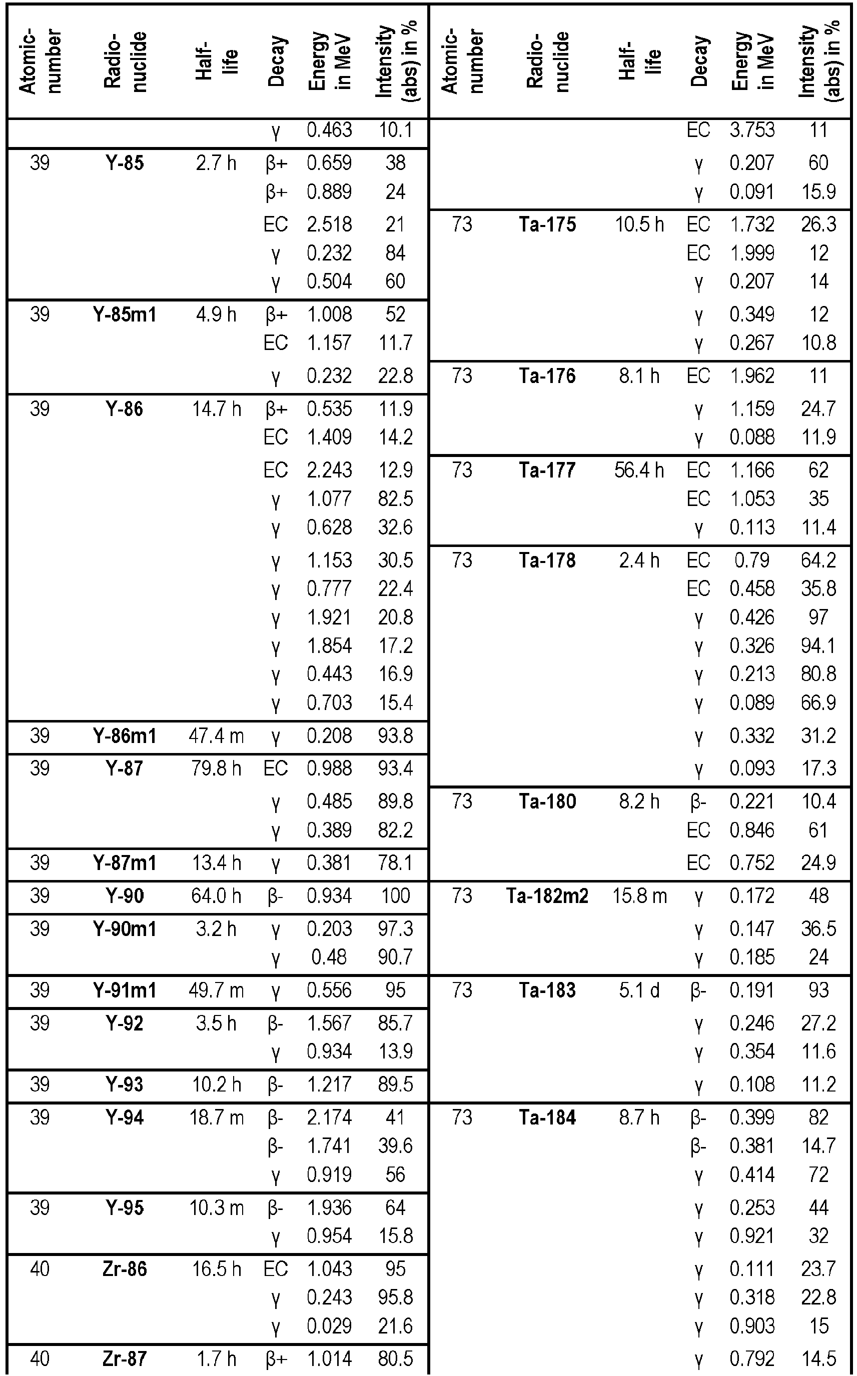

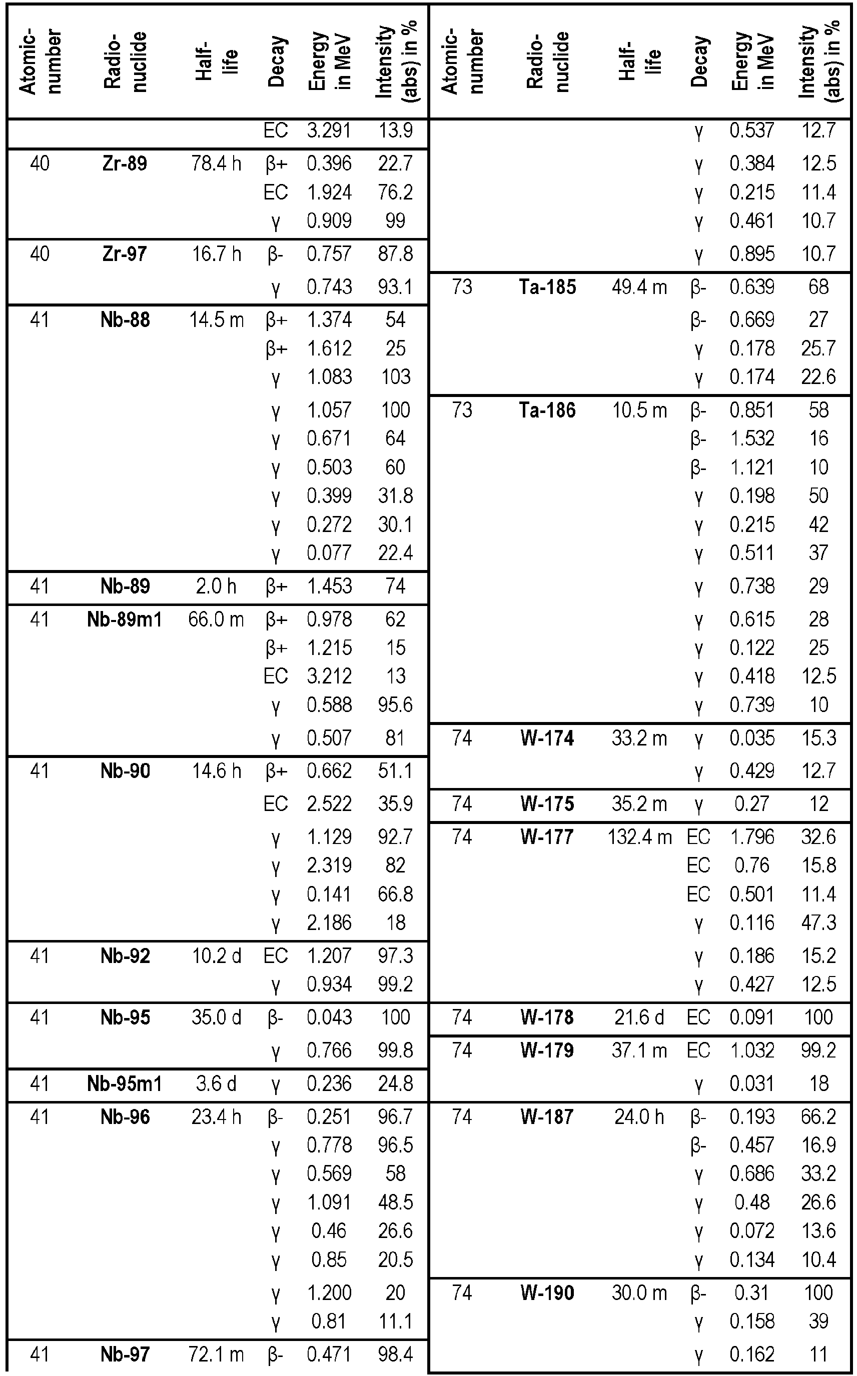

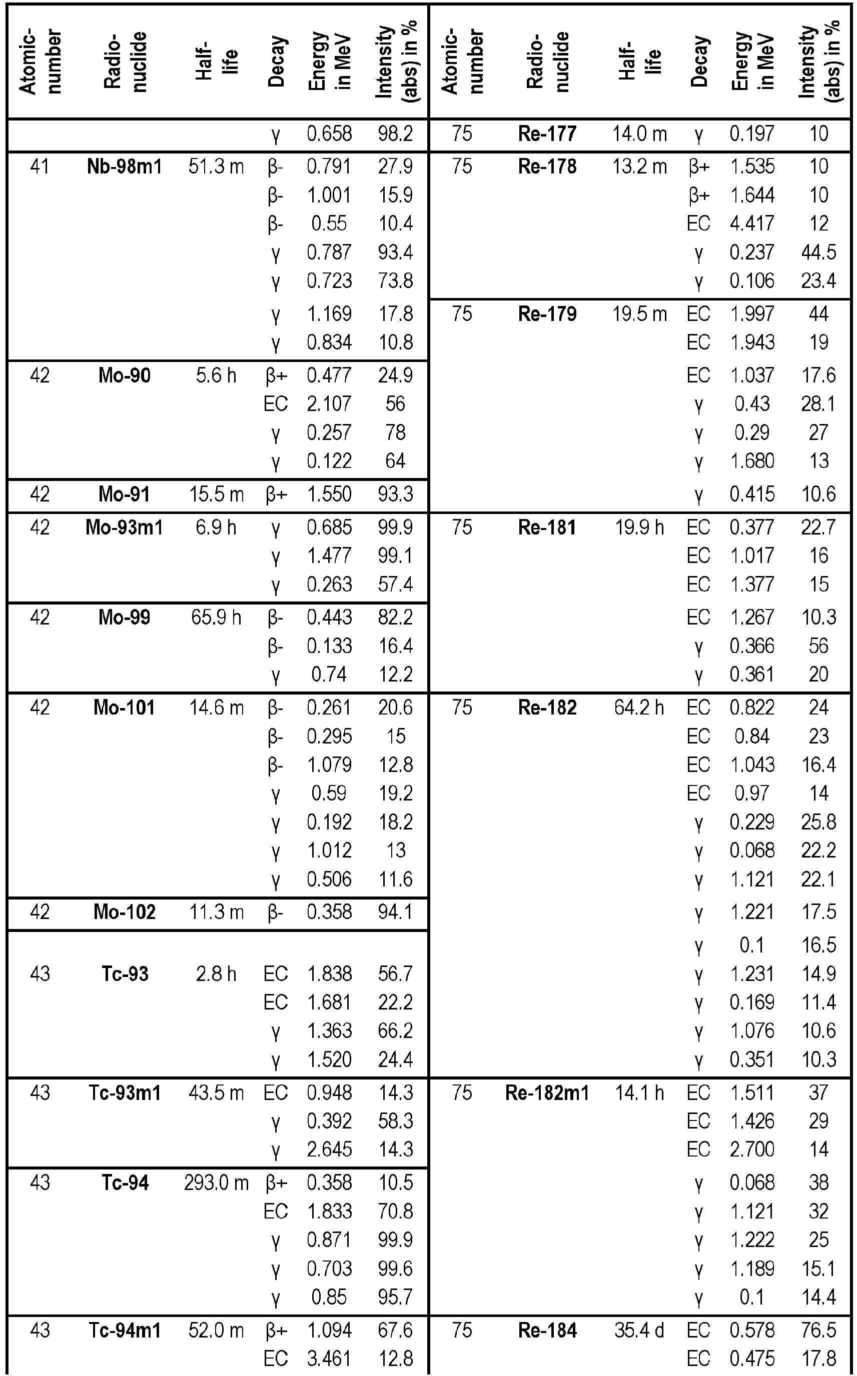

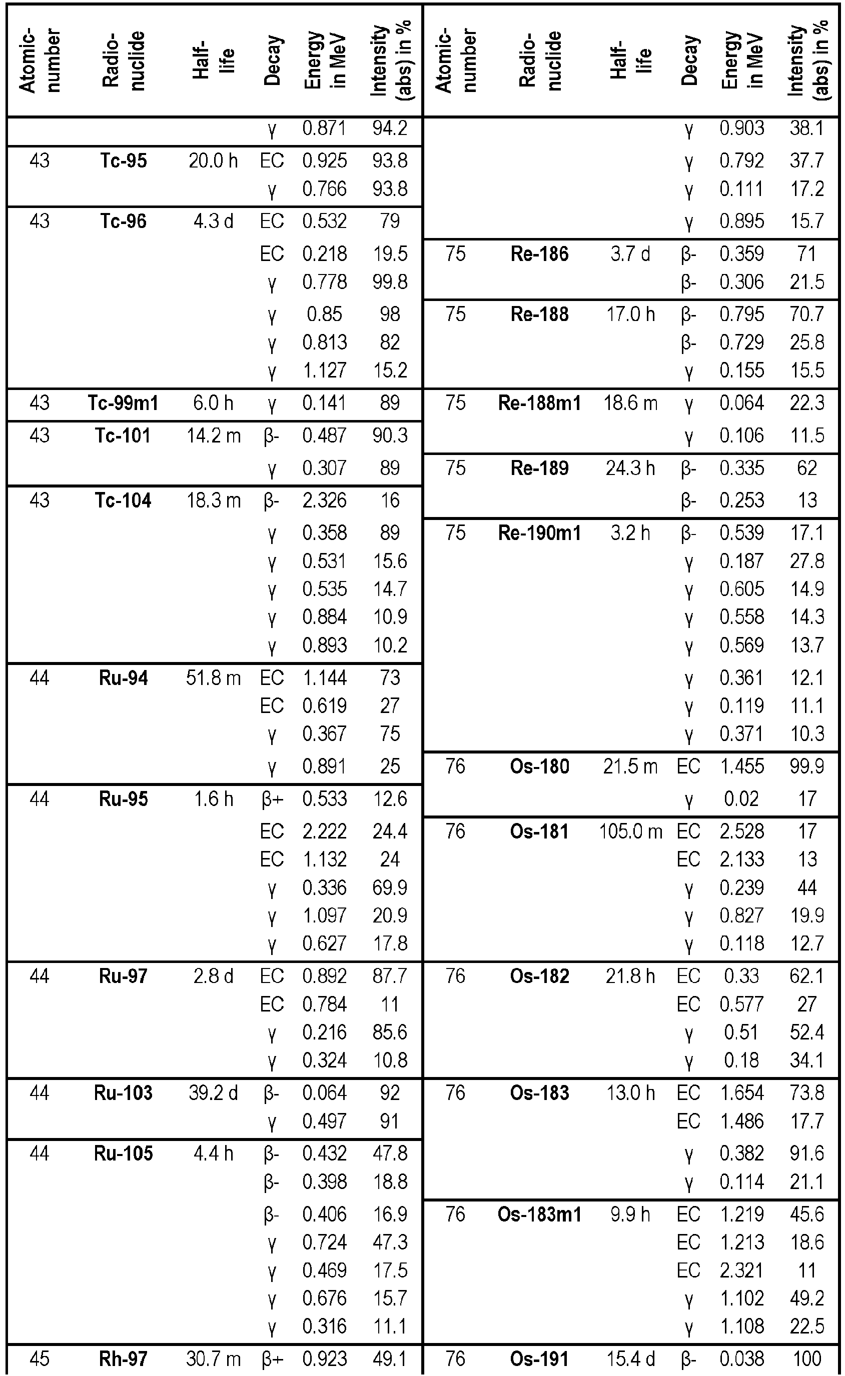

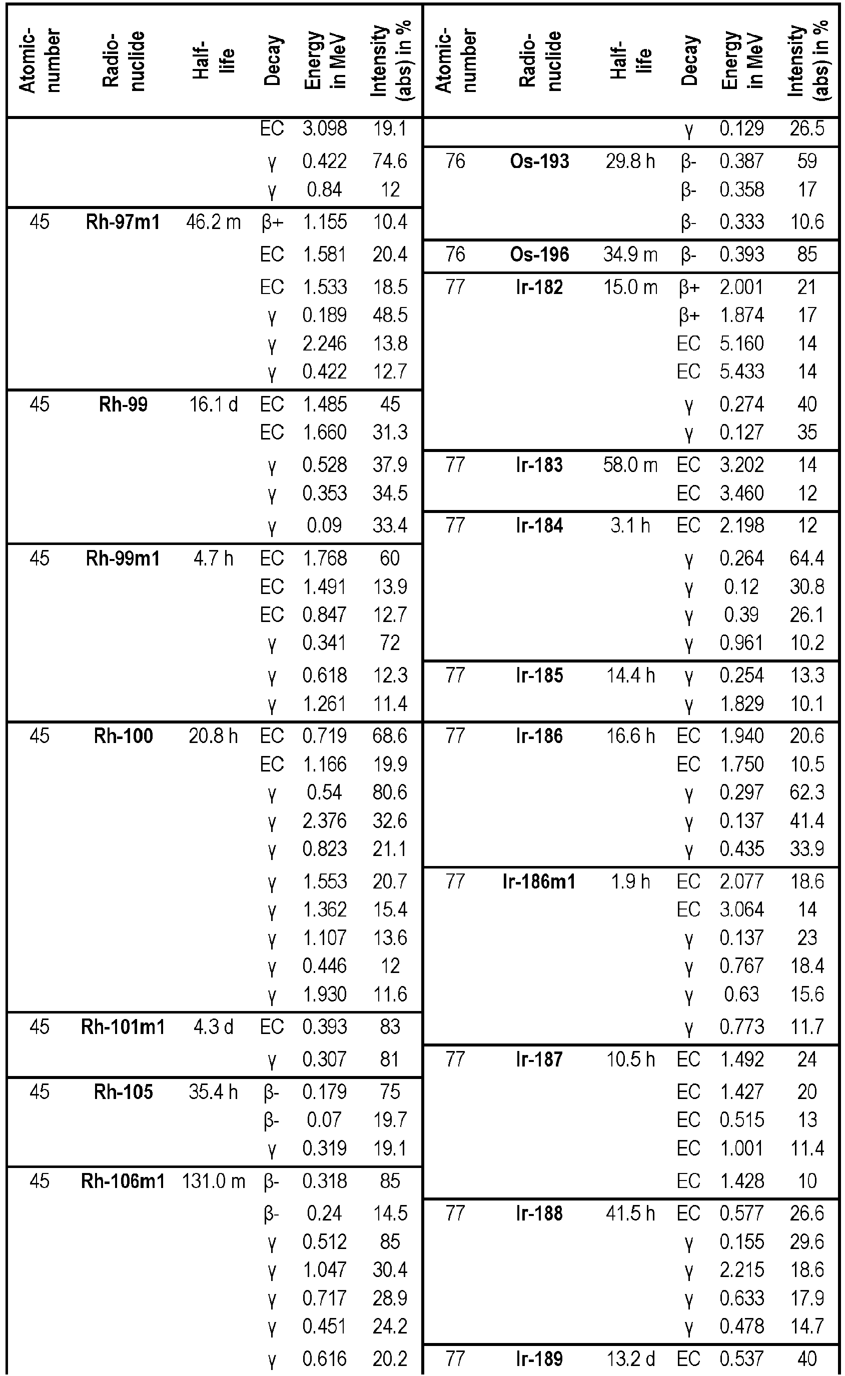

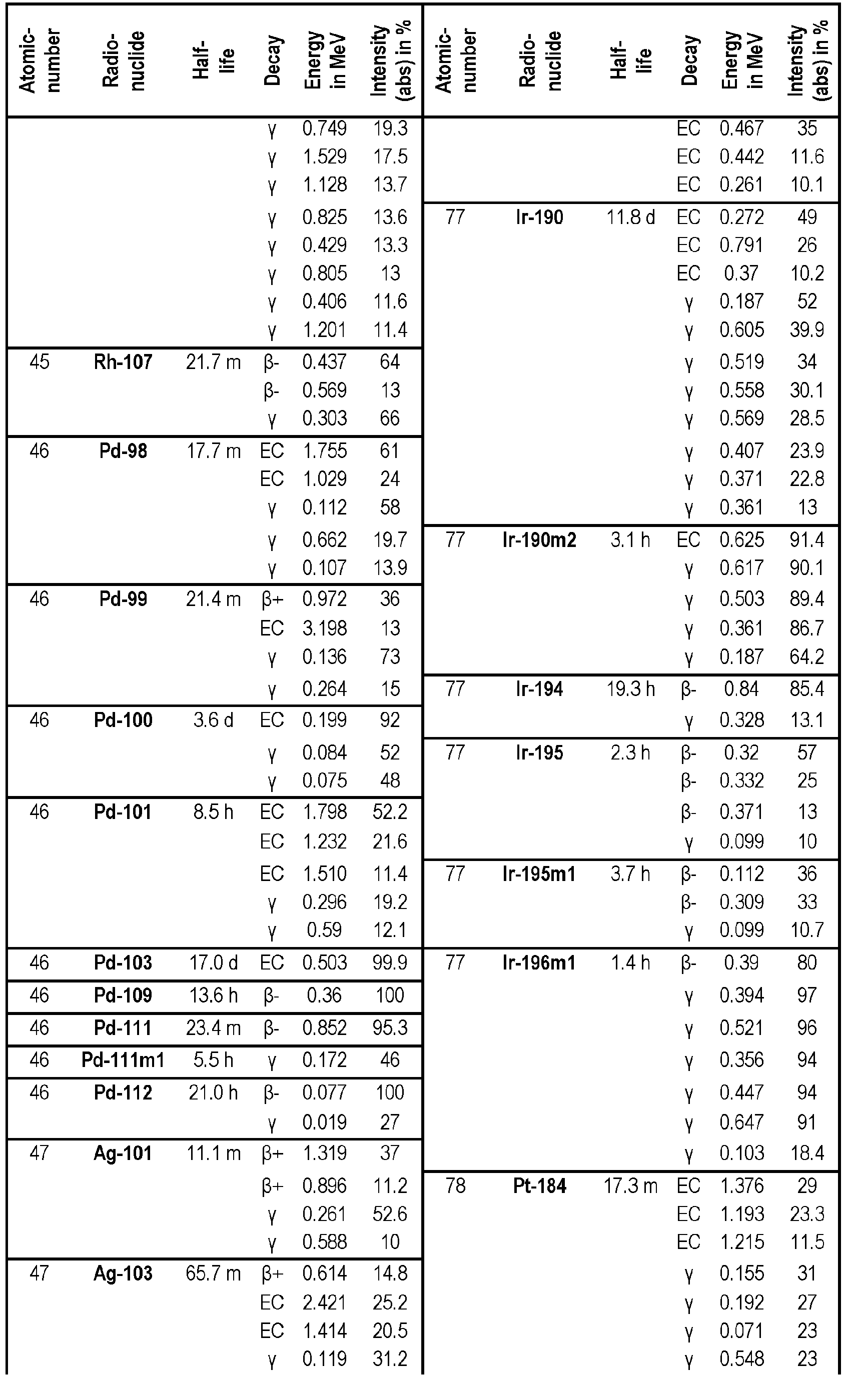

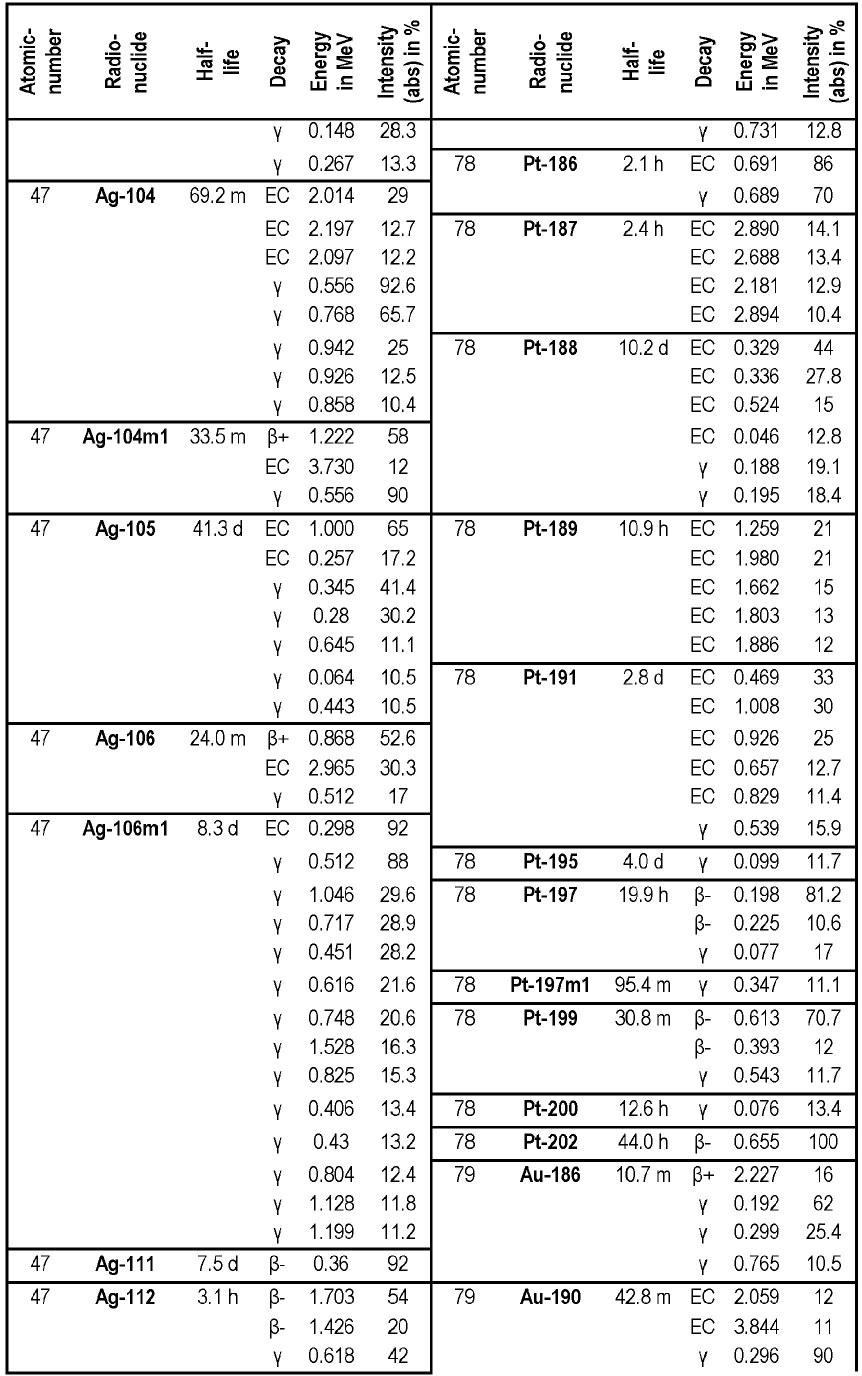

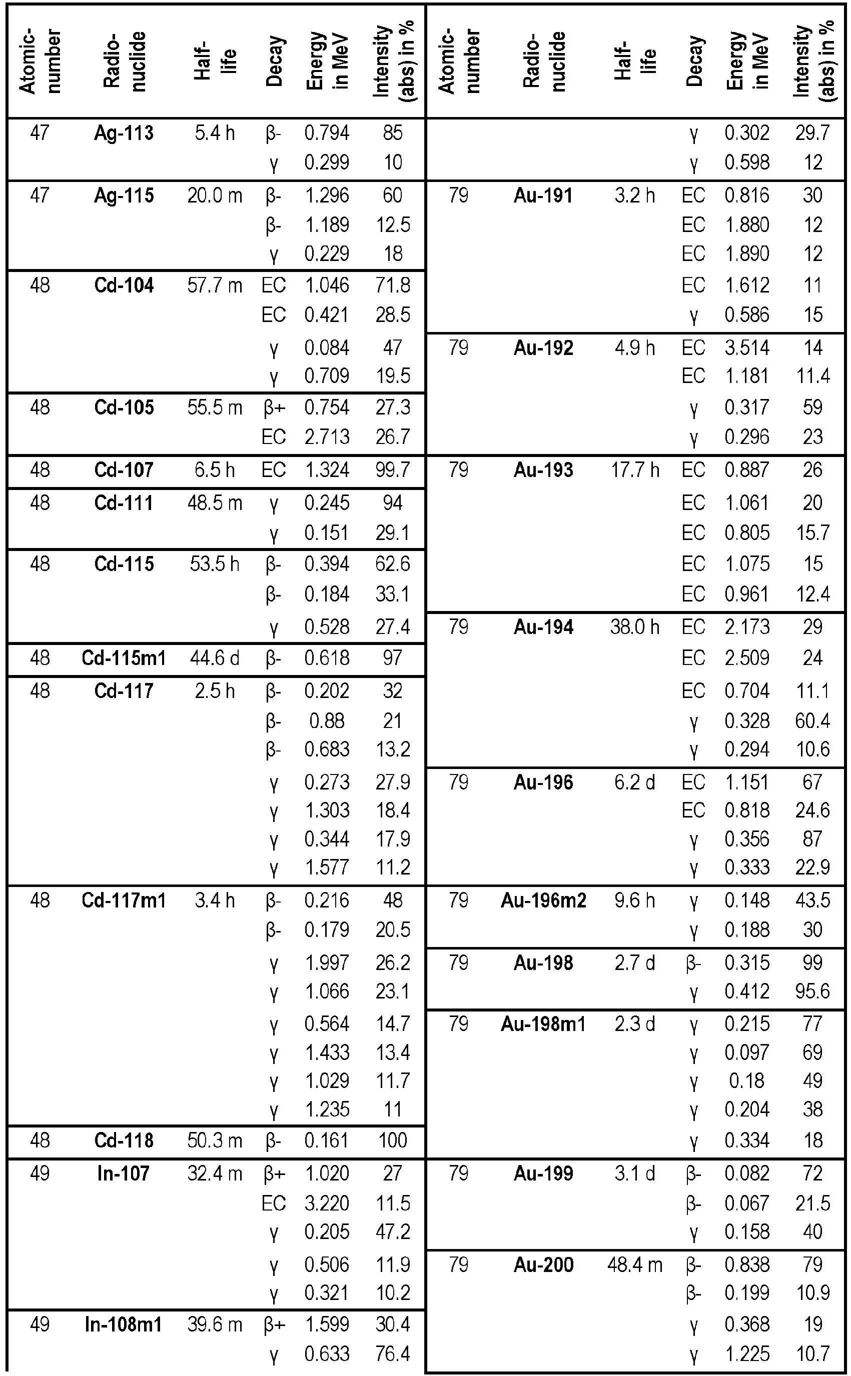

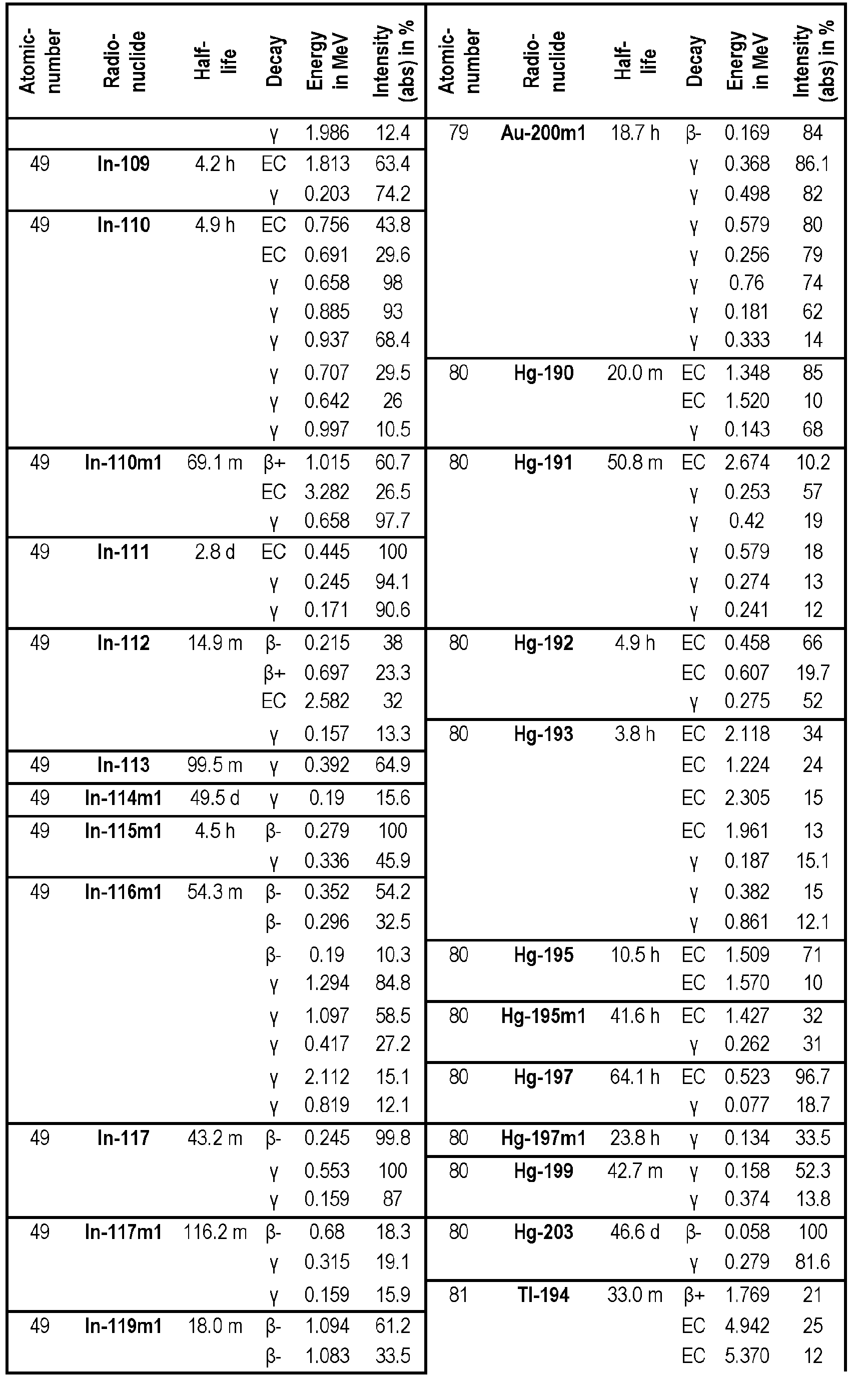

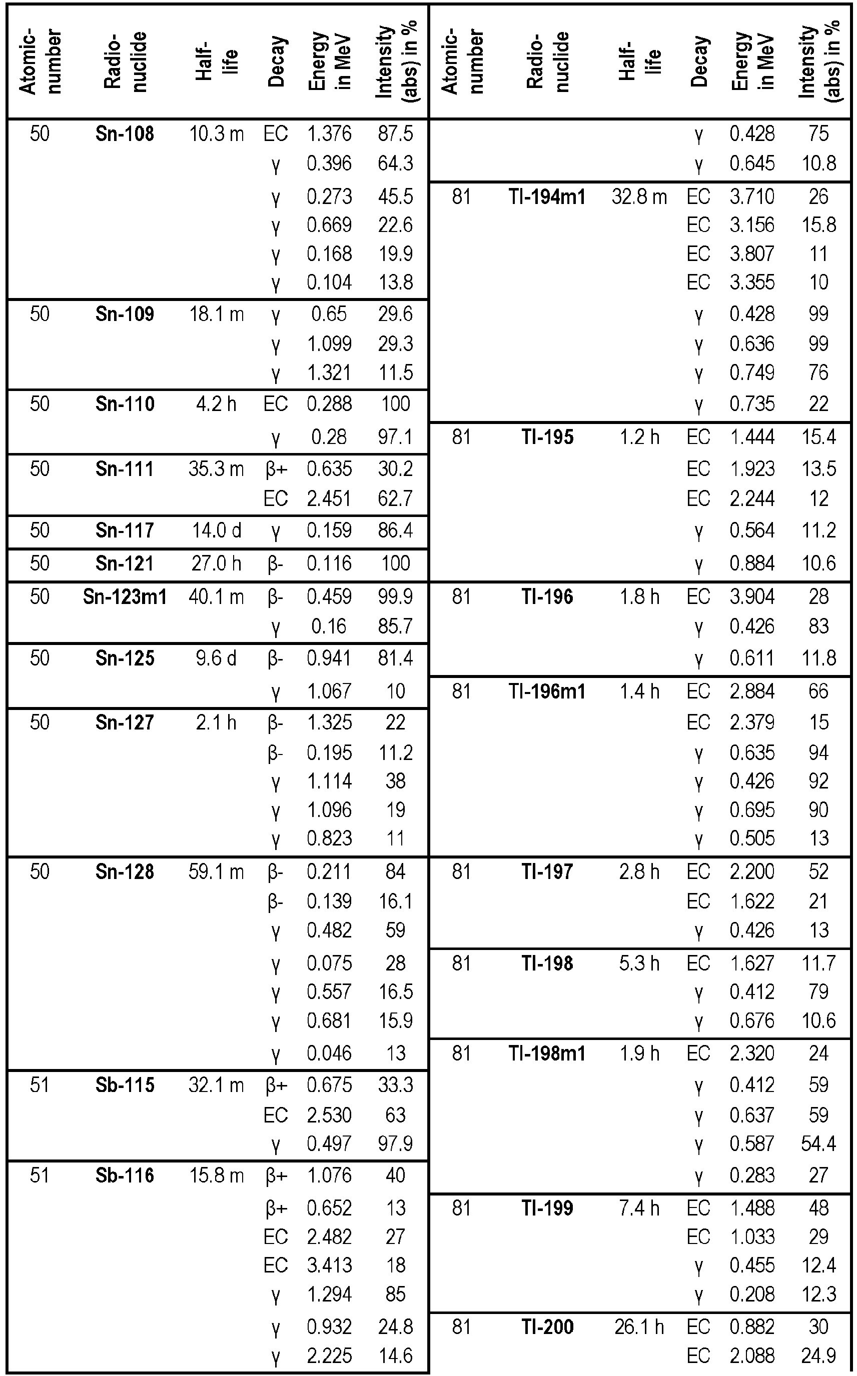

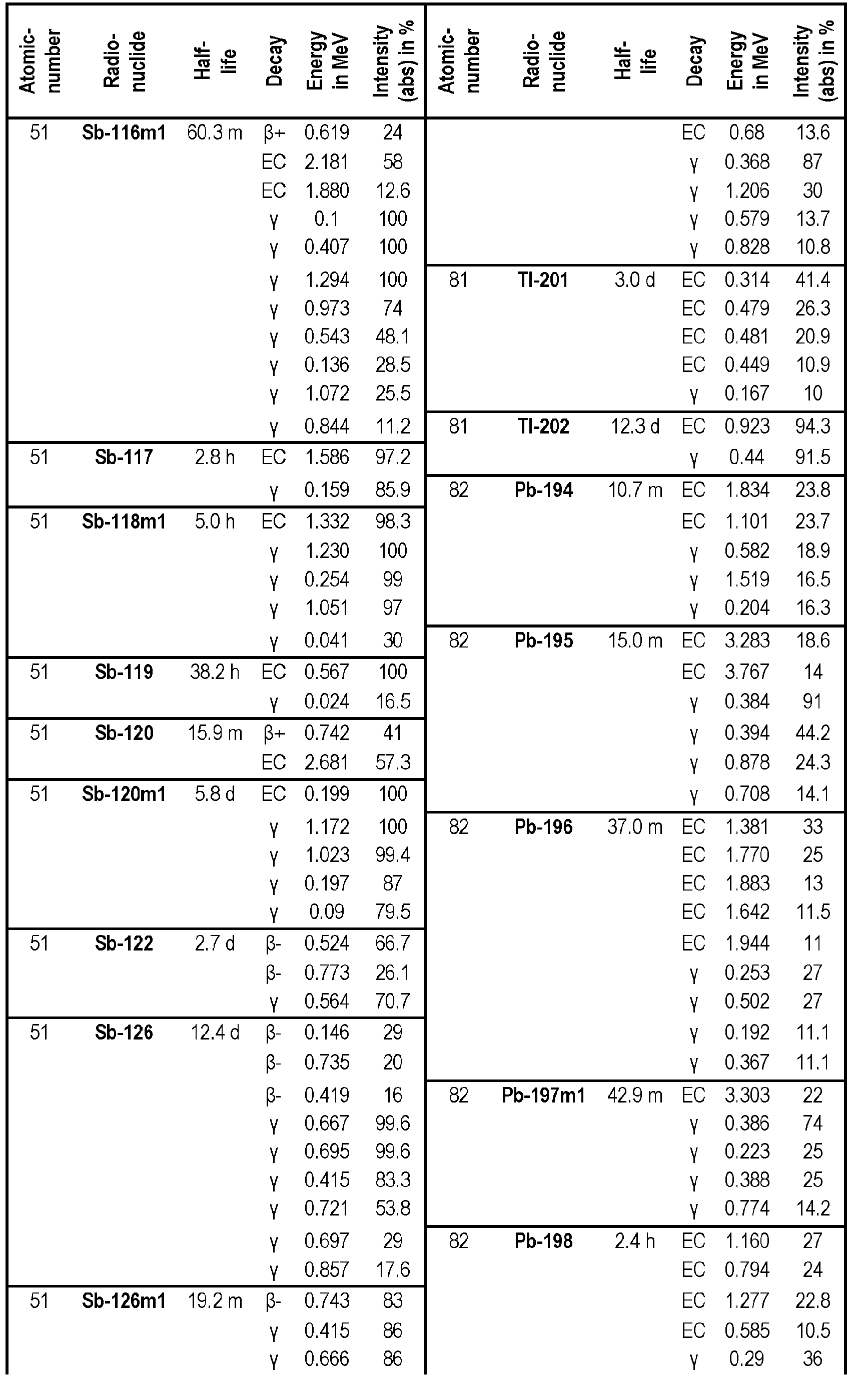

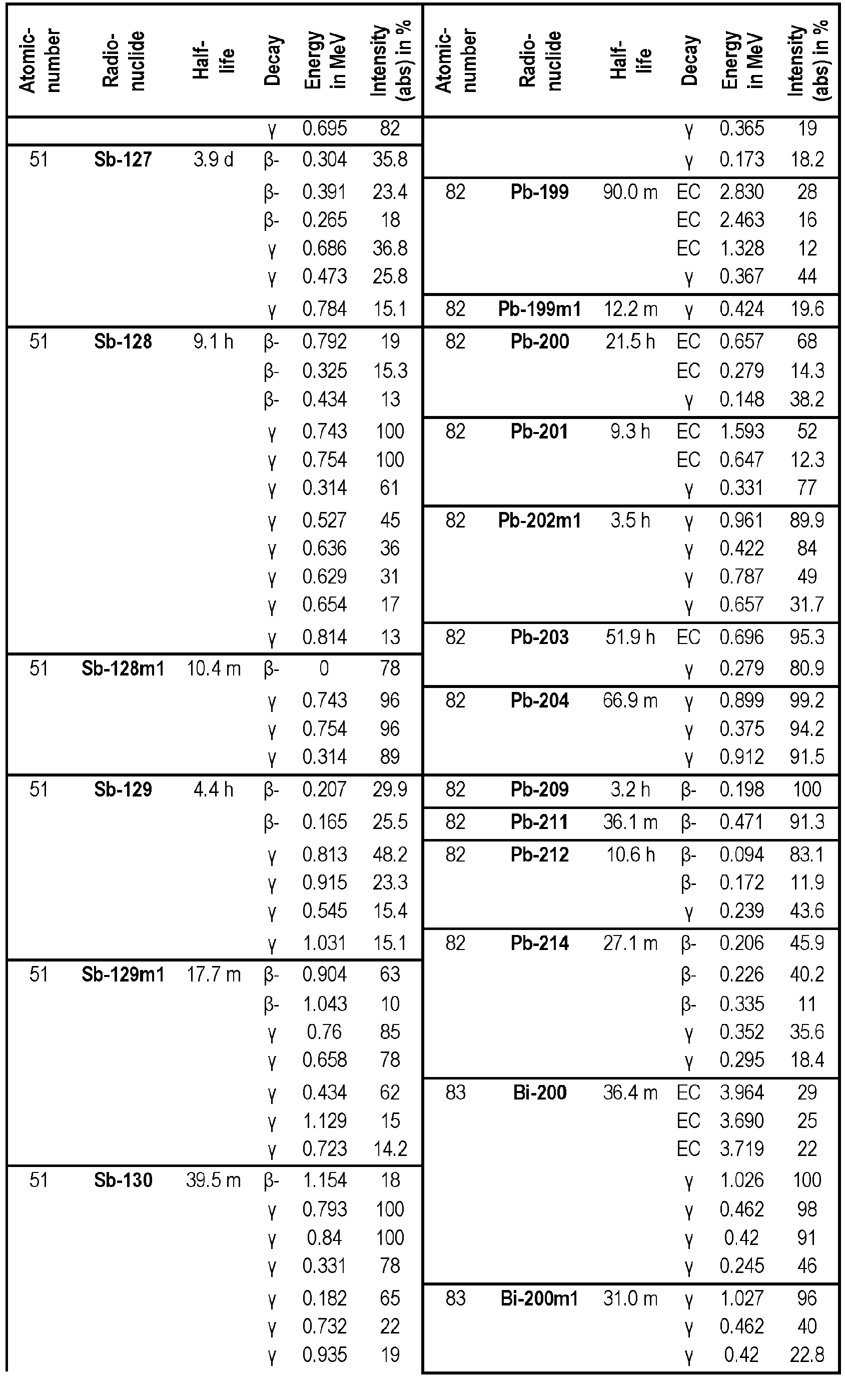

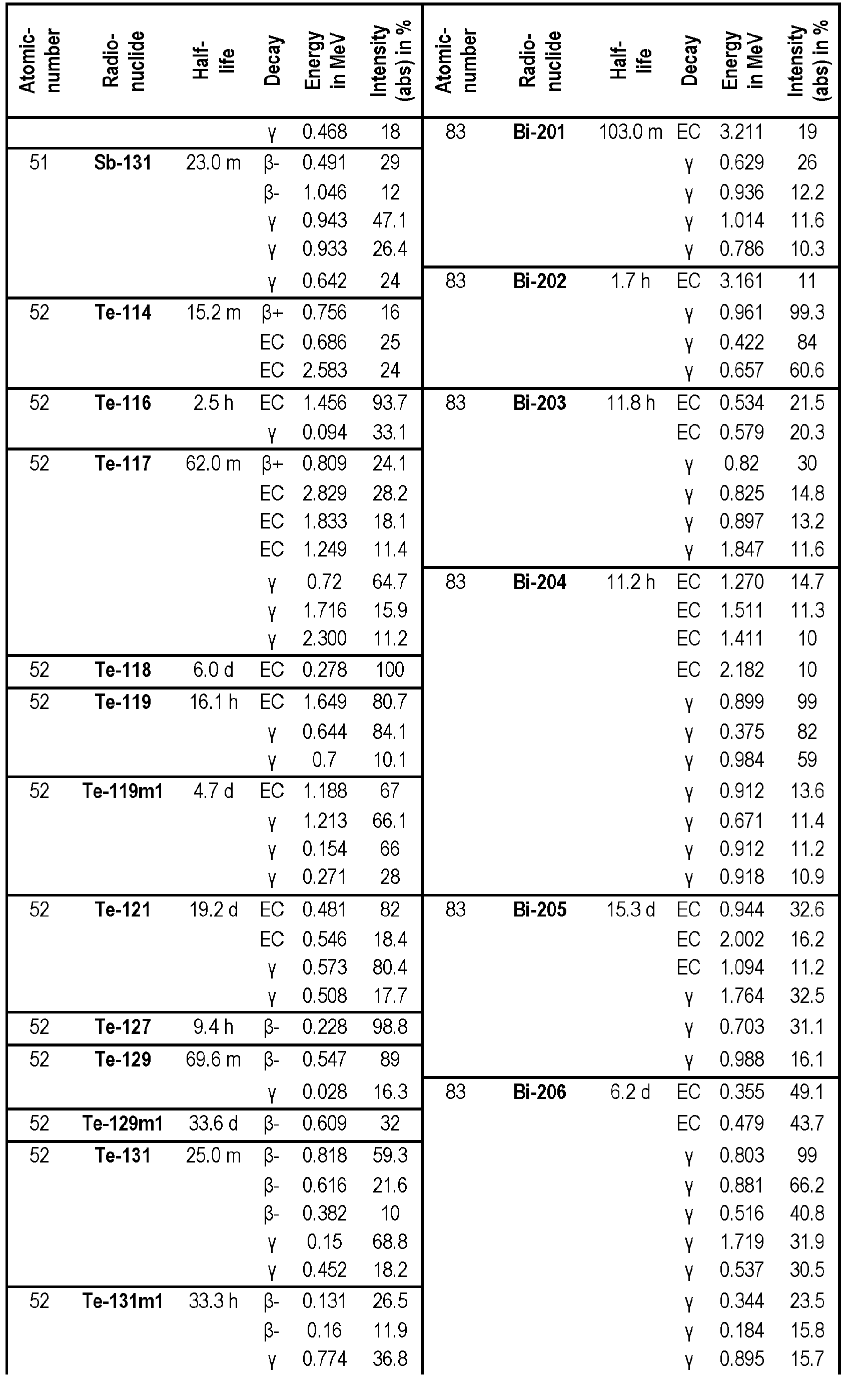

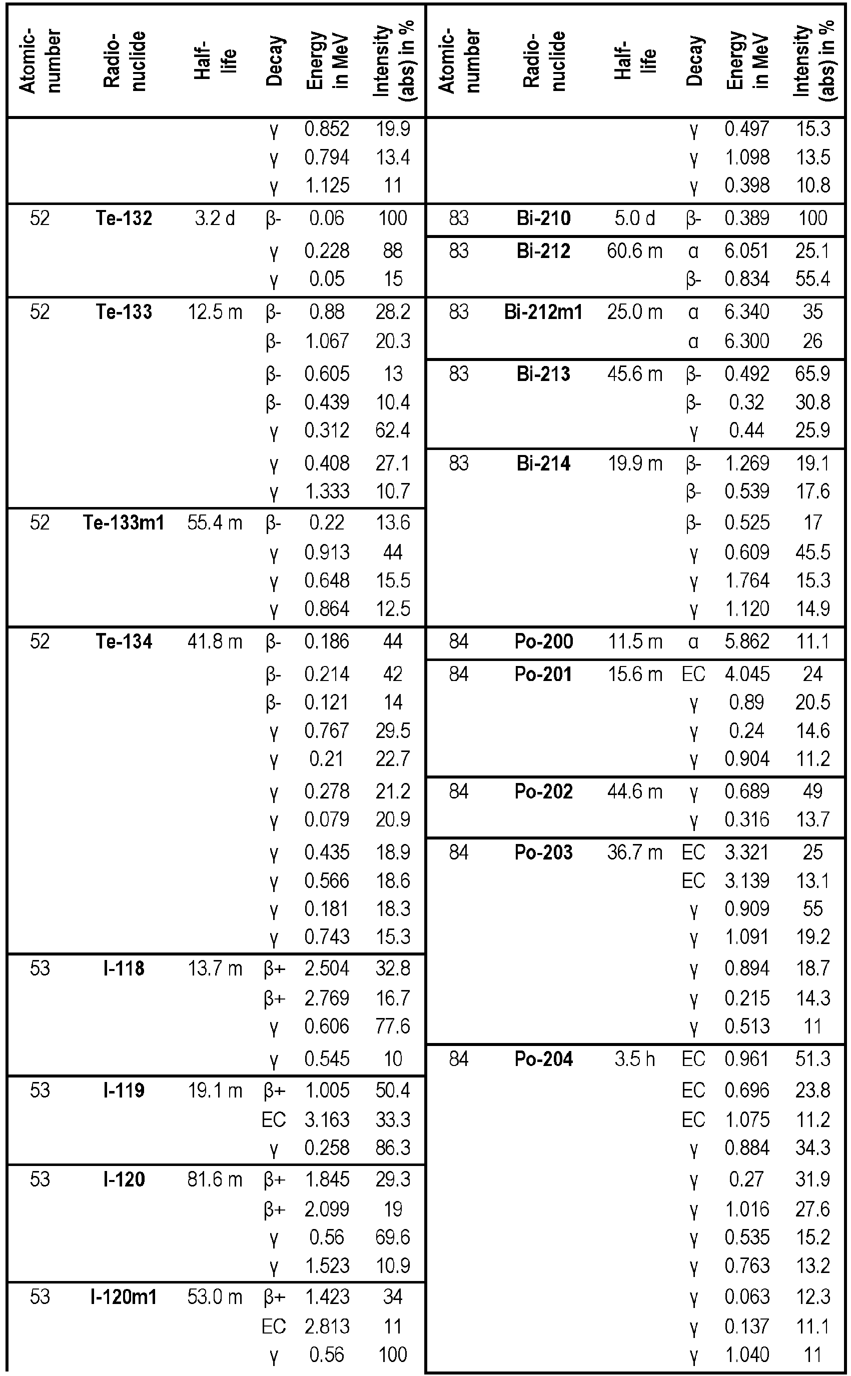

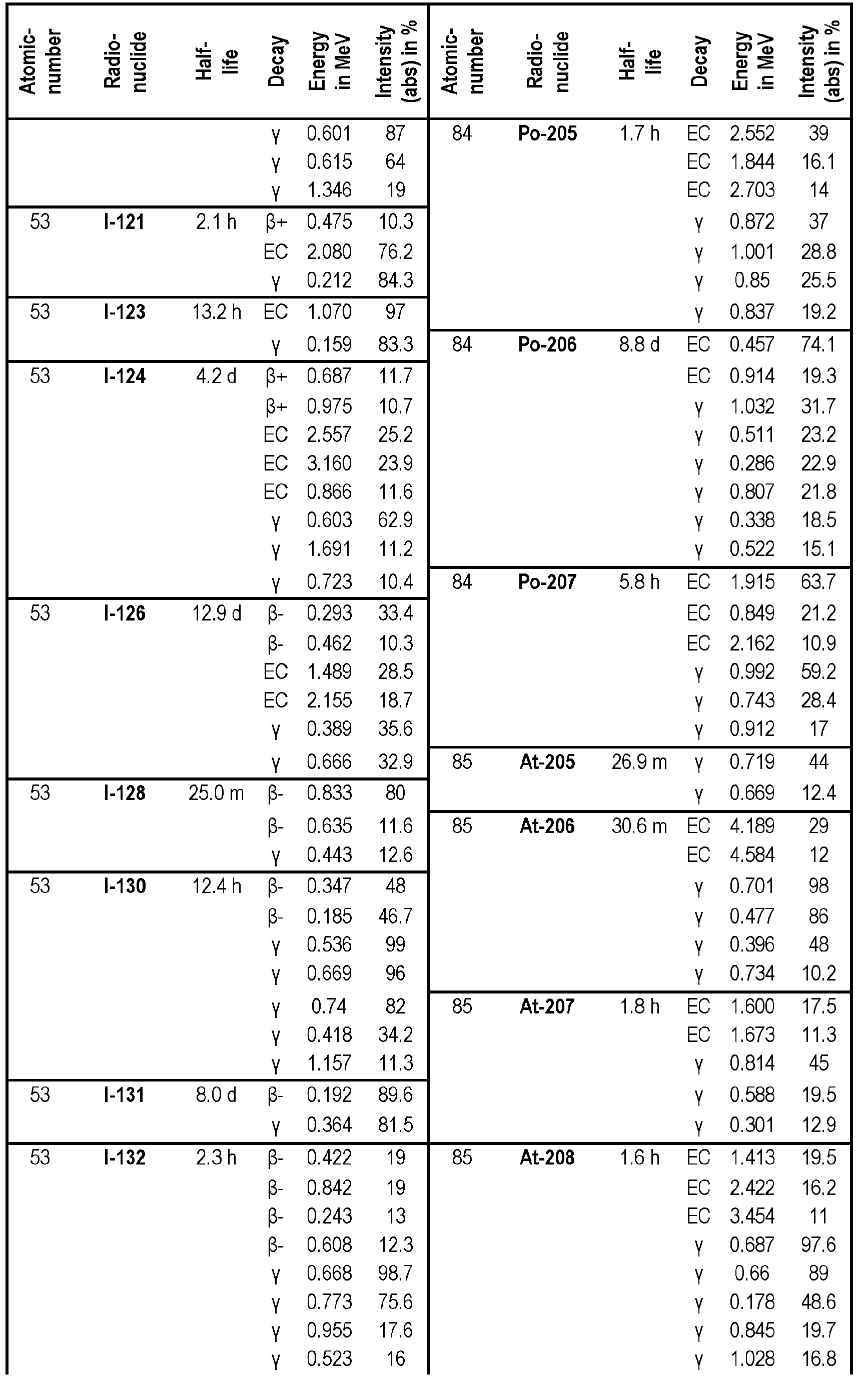

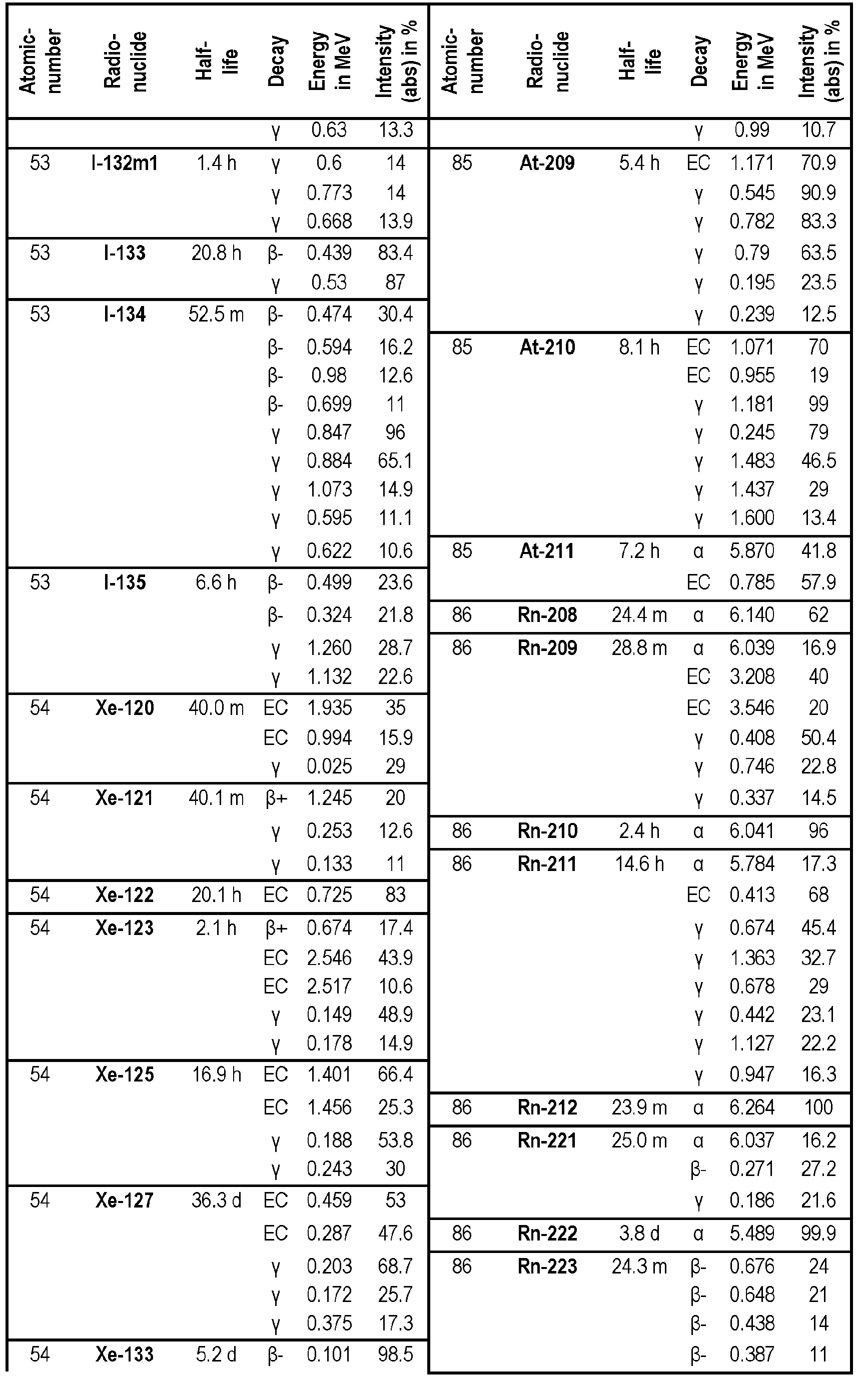

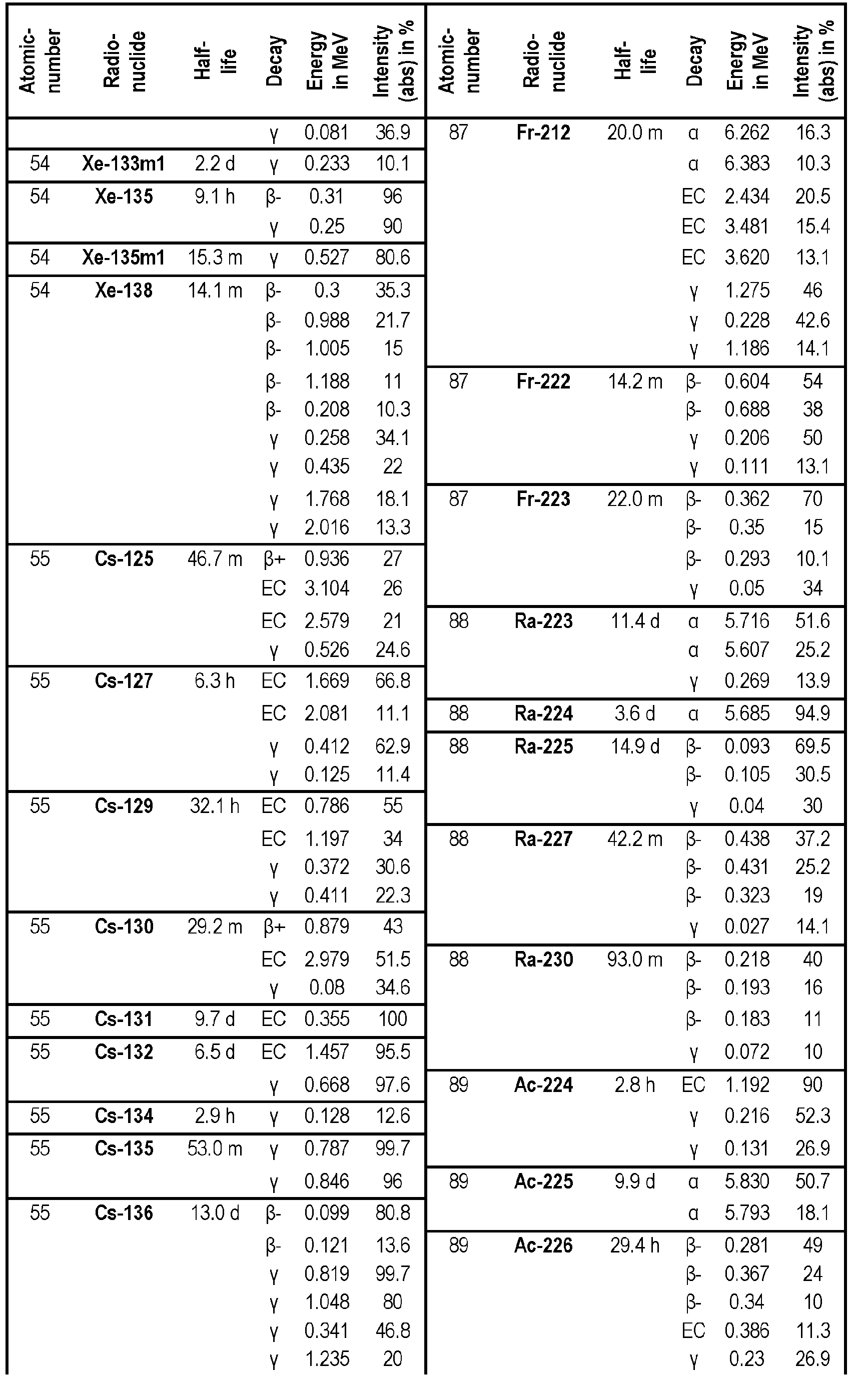

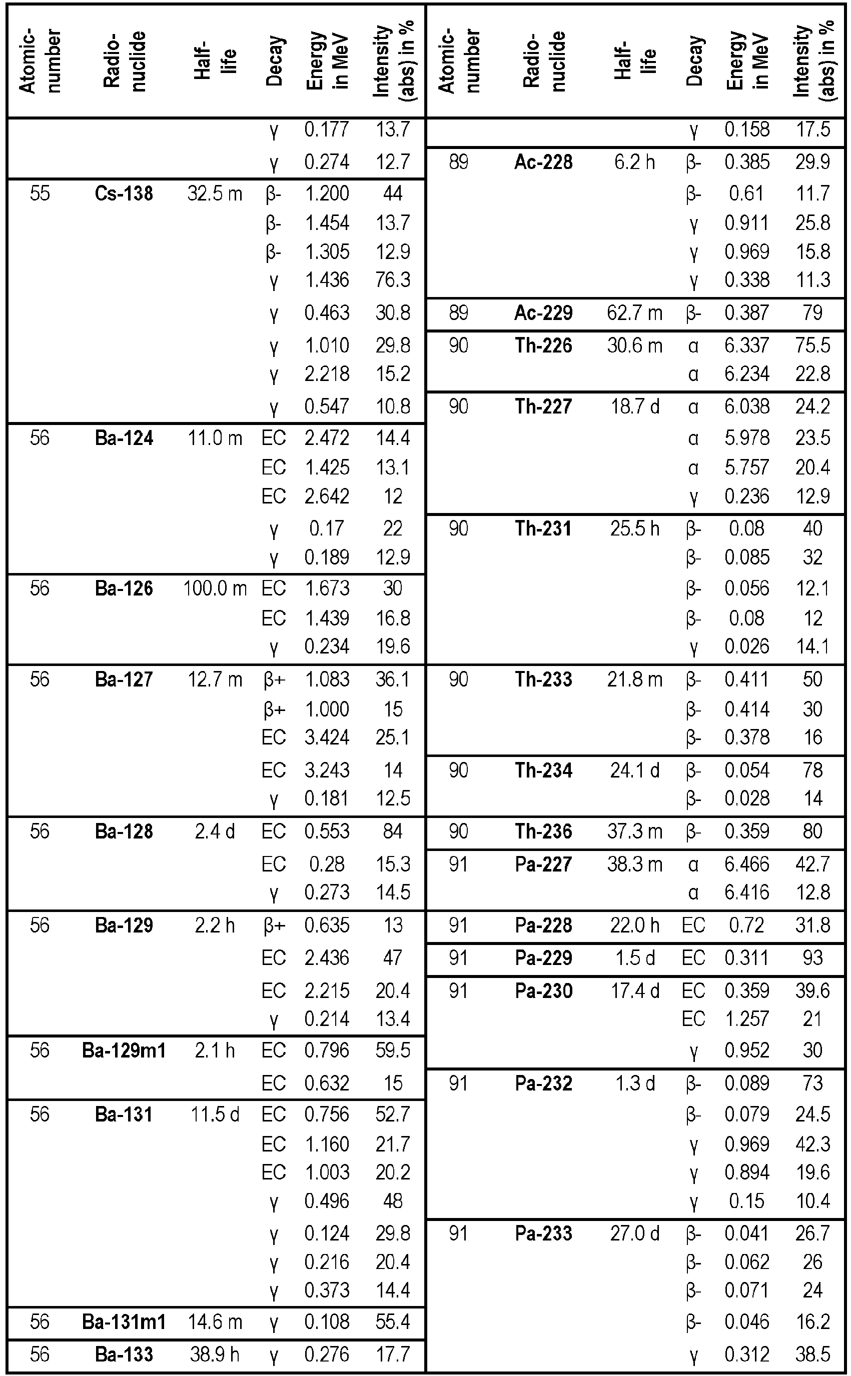

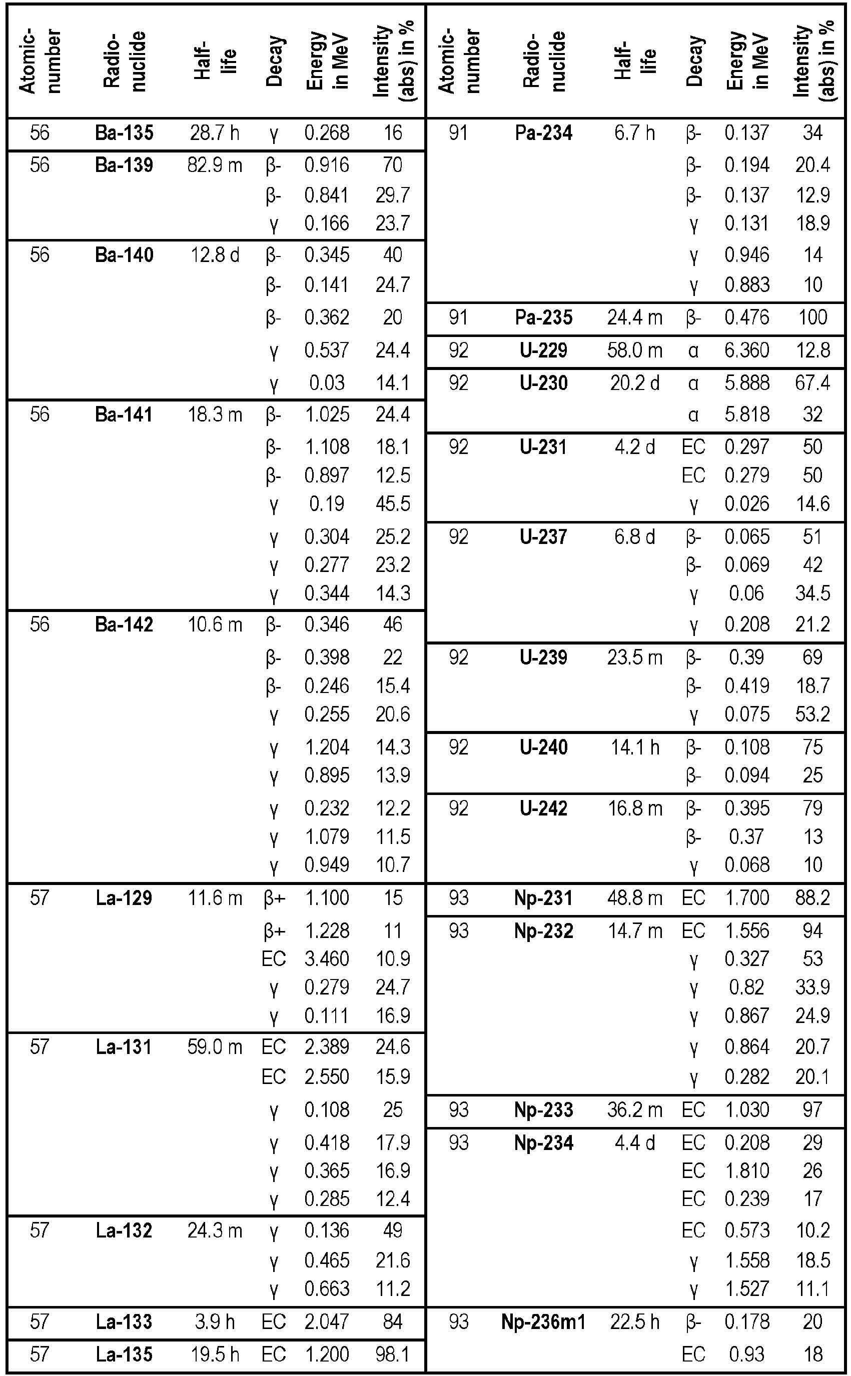

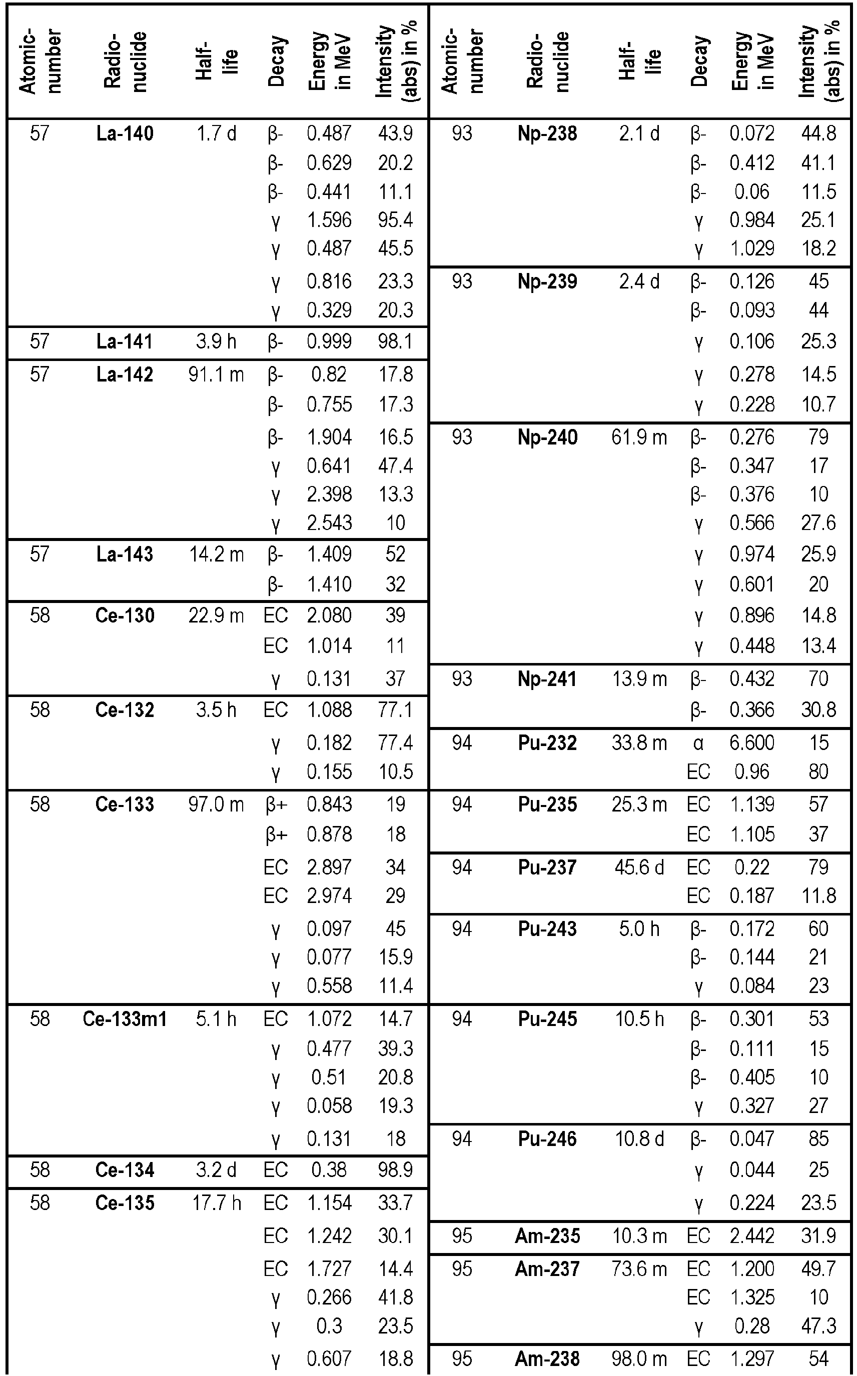

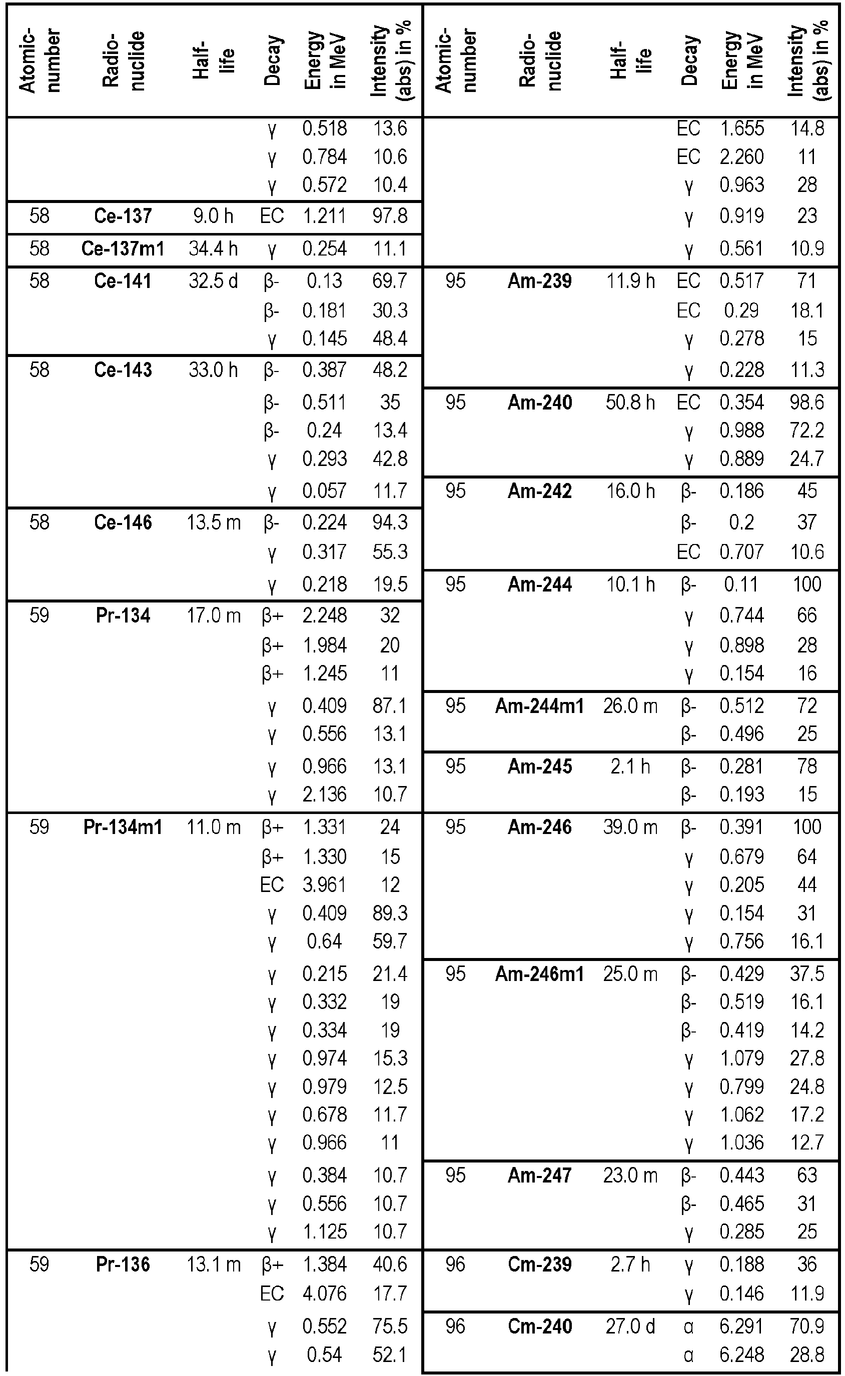

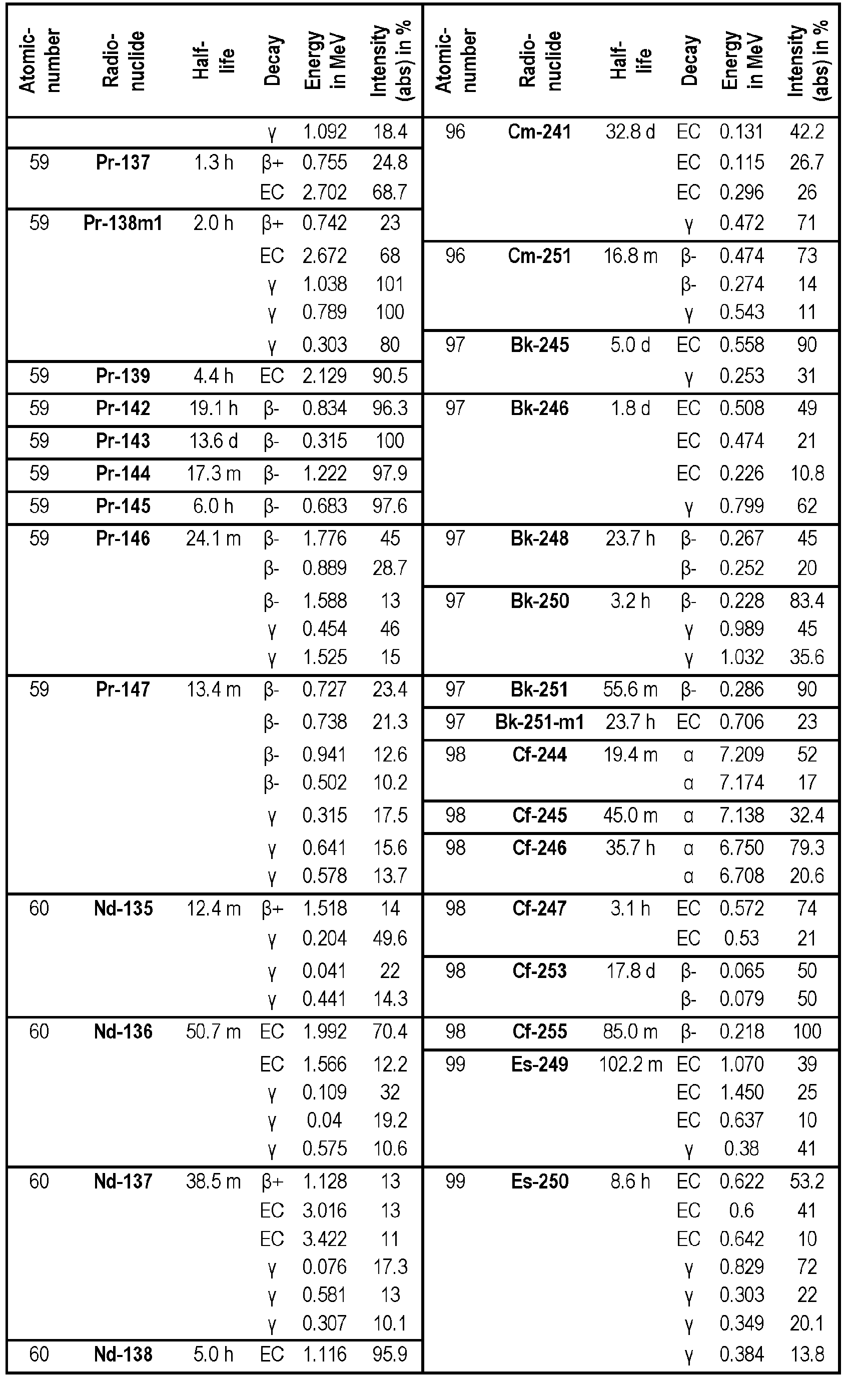

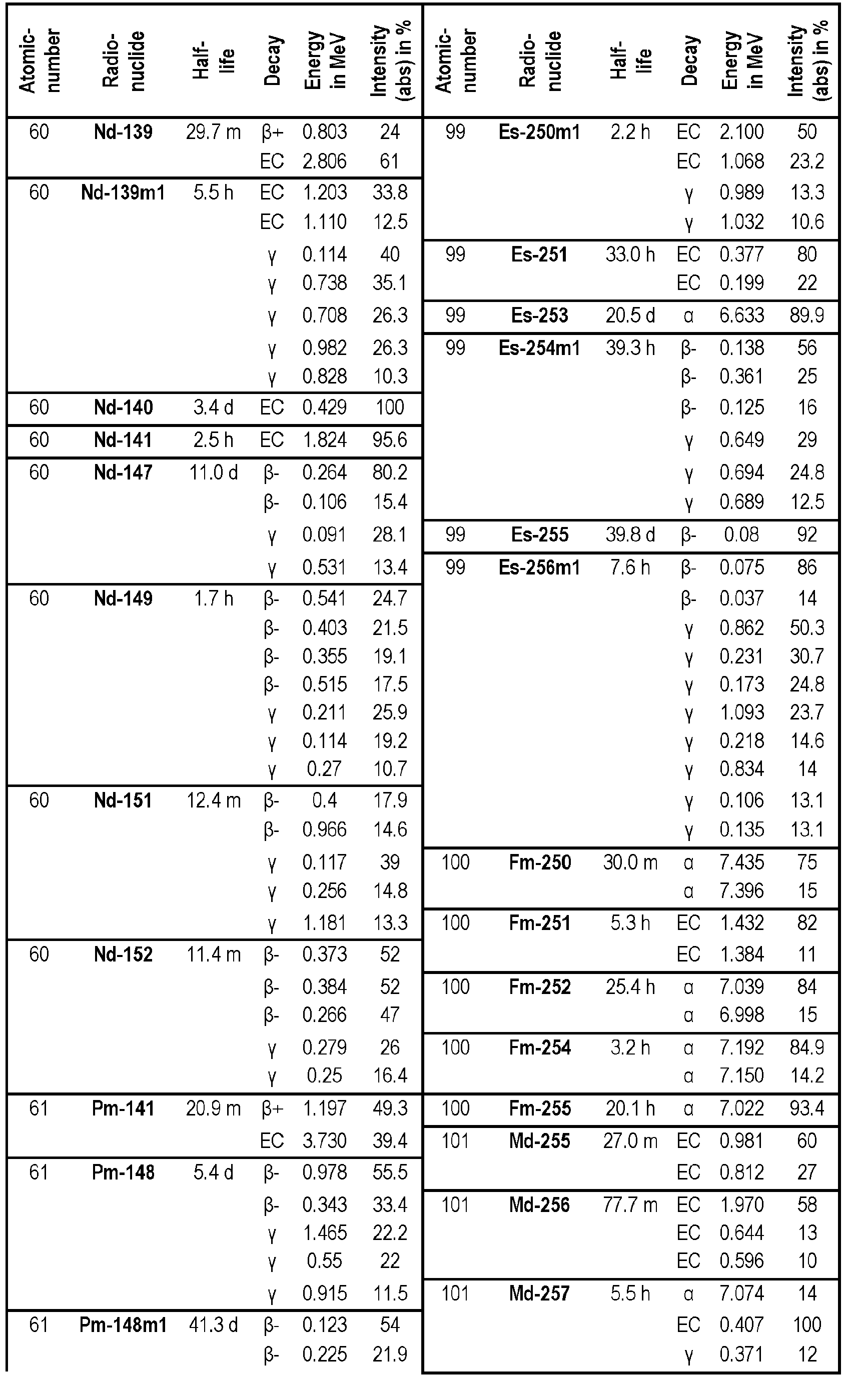

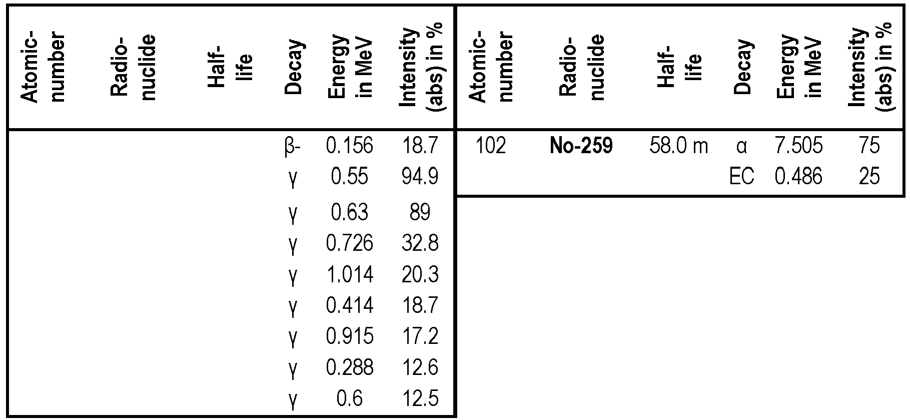

In an embodiment of the present invention, the radionuclide is used for diagnosis. Preferably, the radioactive isotope is selected from the group, but not limited to, comprising ⁴³Sc, ⁴⁴Sc, ⁵¹Mn , ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb , ¹⁵⁵Tb , ¹⁷⁷Lu, ²⁰¹Tl , ²⁰³Pb. More preferably, the radionuclide is selected from the group comprising ⁴³Sc, ⁴⁴Sc, ⁶⁴Cu, ⁶⁷Ga, ⁶⁸Ga, ⁸⁶Y,⁸⁹Zr, ¹¹¹In, ¹⁵²Tb, ¹⁵⁵Tb, ²⁰³Pb. Even more preferably, the radionuclide is ⁶⁴Cu, ⁶⁸Ga and ¹¹¹In. It will, however, also be acknowledged by a person skilled in the art that the use of said radionuclide is not limited to diagnostic purposes, but encompasses their use in therapy and theragnostics when conjugated to the compound of the invention.

In an embodiment of the present invention, the radionuclide is used for therapy. Preferably, the radioactive isotope is selected from the group comprising ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th. More preferably, the radioactive isotope is selected from the group comprising ⁴⁷Sc, ⁶⁷Cu, ⁹⁰Y, ¹⁷⁷Lu, ²¹²Pb, ²¹³Bi, ²²⁵Ac, ²²⁷Th. Even more preferably, the radionuclide is selected from the group comprising ⁹⁰Y, ¹⁷⁷Lu, ²²⁵Ac and ²²⁷Th. It will, however, also be acknowledged by a person skilled in the art that the use of said radionuclide is not limited to therapeutic purposes, but encompasses their use in diagnostic and theragnostics when conjugated to the compound of the invention.

In an embodiment, the compound of the invention comprises a chelator. Preferably, the chelator is part of the compound of the invention, whereby the chelator is either directly or indirectly such as by a linker attached to the compound of the invention. A preferred chelator is a chelator which forms metal chelates preferably comprising at least one radioactive metal. The at least one radioactive metal is preferably useful in or suitable for diagnostic and/or therapeutic and/or theragnostic use and is more preferably useful in or suitable for imaging and/or radiotherapy.

Chelators in principle useful in and/or suitable for the practicing of the instant invention including diagnosis and/or therapy of a disease are known to the person skilled in the art. A wide variety of respective chelators is available and has been reviewed, e.g. by Banerjee et al. (Banerjee, et al., Dalton Trans, 2005, 24: 3886), and references therein (Price, et al., Chem Soc Rev, 2014, 43: 260; Wadas, et al., Chem Rev, 2010, 110: 2858). Such chelators include, but are not limited to linear, cyclic, macrocyclic, tetrapyridine, N3S, N2S2 and N4 chelators as disclosed in US 5,367,080 A, US 5,364,613 A, US, 5,021,556 A, US 5,075,099 A and US 5,886,142 A.

Representative chelators and derivatives thereof include, but are not limited to the examples listed in Table 7.

**Table 7: Examples of chelators with their corresponding chemical names.**

| | |
|---|---|
| 2,3-HOPO | stands for 3-hydroxypyridin-2-one |
| 3,4,3-(Li-1,2-HOPO) | stands for N,N'-1,4-Butanediylbis(N-(3-(((1,6-dihydro-1-hydroxy-6-oxo-2-pyridinyl)carbonyl)amino)propyl)-1,6-dihydro-1-hydroxy-6-oxo-2-pyridinecarboxamide |
| 3p-C-DEPA | stands for 2,2',2"-(10-(2-(bis(carboxymethyl)amino)-5-(4-nitrophenyl)pentyl)-1,4,7,10-tetraazacyclododecane-1,4,7-triyl)triacetic acid |
| 3p-C-DEPA-NCS | stands for 2-[4-[2-[bis(carboxymethyl)amino]-5-(4-thiocyanatophenyl)pentyl]-7,10-bis(carboxymethyl)-1,4,7,10-tetrazacyclododec-1-yl]acetic acid |
| 3p-C-NETA | stands for 2-[4-[2-[bis(carboxymethyl)amino]-5-(4-nitrophenyl)pentyl]-7-(carboxymethyl)-1,4,7-triazonan-1-yl]acetic acid |
| AAZTA | stands for 1,4-bis(carboxymethyl)-6-[bis(carboxymethyl)]amino-6-methylperhydro-1,4-diazepine |
| AmBaSar | stands for 4-((8-amino-3,6,10,13,16,19-hexaazabicyclo [6.6.6] icosane-1-ylamino)methyl)benzoic acid |
| BF-HEHA | stands for 2,2',2",2‴,2ʺʺ,2‴ʺ-(2-(4-isothiocyanatobenzyl)-1,4,7,10,13,16-hexaazacyclooctadecane-1,4,7,10,13,16-hexayl)hexaacetic acid |
| BF-PEPA | stands for 2,2',2",2‴,2ʺʺ-(2-(4-isothiocyanatobenzyl)-1,4,7,10,13-pentaazacyclopentadecane-1,4,7,10,13-pentayl)pentaacetic acid |
| BPCA | stands for 2,2',2",2‴-(([2,2'-bipyridine]-6,6'-diylbis(methylene))bis(azanetriyl))tetraacetic acid |
| C3B-DO2A | stands for 2,2'-(1,4,7,10-tetraazabicyclo[5.5.3]pentadecane-4,10-diyl)diacetic acid |
| CB-DO2A | stands for 4,10-bis(carboxymethyl)-1,4,7,10-tetraazabicyclo[5.5.2]tetradecane |
| CB-TE1A1P | stands for 2-(11-(phosphonomethyl)-1,4,8,11-tetraazabicyclo[6.6.2]hexadecan-4-yl)acetic acid |
| CB-TE1K1P | stands for 6-amino-2-(11-phosphonomethyl-1,4,8,11-tetraaza-bicyclo[6.6.2]hexadec-4-yl)-hexanoic acid |
| CB-TE2A | stands for 4,11-bis-(carboxymethyl)-1,4,8,11-tetraazabicyclo[6.6.2]-hexadecane |
| CB-TE2P | stands for ((1,4,7,10-tetraazabicyclo[5.5.2]tetradecane-4,10-diyl)bis(methylene))bis(phosphonic acid) |
| CDTA | stands for trans-1,2-diaminocyclohexane-N,N,N',N'-tetraacetic acid |
| C-NE3TA-NCS | stands for 2,2'-(7-(2-((carboxymethyl)amino)-3-(4-thiocyanatophenyl)propyl)-1,4,7-triazonane-1,4-diyl)diacetic acid |
| C-NETA-NCS | stands for 2,2'-((1-(4,7-bis(carboxymethyl)-1,4,7-triazonan-1-yl)-4-(4-isothiocyanatophenyl)butan-2-yl)azanediyl)diacetic acid |
| C-NOTA | stands for 2,2',2"-(2-(4-isothiocyanatobenzyl)-1,4,7-triazonane-1,4,7-triyl)triacetic acid |
| CPTA | stands for 4-((1,4,8,11-tetraazacyclotetradecan-1-yl)methyl)benzoic acid |
| cyclam | stands for 1,4,8,11-tetraazacyclotetradecane |
| cyclen | stands for 1,4,7,10-tetraazacyclododecane |
| CY-DTA | stands for trans-1,2-diaminocyclohexane-N,N,N',N'-tetraacetic acid |
| DATA | stands for [4-carboxymethyl-6-(carboxymethyl-methyl-amino)-6-methyl-[1,4]diazepan-1-yl]-acetic acid |
| DATA(M) | stands for 2,2'-(6-((carboxymethyl)(methyl)amino)-6-methyl-1,4-diazepane-1,4-diyl)diacetic acid |
| DATA(P) | stands for (25,2'S)-2,2'-(6-(((S)-1-carboxyethyl)amino)-6-methyl-1,4-diazepane-1,4-diyl)dipropionic acid |
| DATA(Ph) | stands for d2,2'-(5-((carboxymethyl)amino)-5-phenyldihydropyrimidine-1,3(2H,4H)-diyl)diacetic acid |
| DATA(PPh) | stands for (25,2'S)-2,2'-(5-(((S)-1-carboxyethyl)amino)-5-phenyldihydropyrimidine-1,3(2H,4H)-diyl)dipropionic acid |
| deferiprone | stands for 3-hydroxy-1,2-dimethyl-4(1H)-pyridone |
| DEPA | stands for 7-[2-(bis-carboxymethylamino)-ethyl]-4,10-bis-carboxymethyl-1,4,7, 10-tetraaza-cyclododec-1-yl-acetic acid |
| DFO | stands for desferrioxamine - N1-(5-aminopentyl)-N1-hydroxy-N4-(5-(N-hydroxy-4-((5-(N-hydroxyacetamido)pentyl)amino)-4-oxobutanamido)pentyl)succinamide |
| DFO-HOPO | stands for N1-hydroxy-N1-(5-(4-(hydroxy(5-(1-hydroxy-6-oxo-1,6-dihydropyridine-2- carboxamido)pentyl)amino)-4-oxobutanamido)pentyl)-N4-(5-(N-hydroxyacetamido)pentyl)succinamide |
| DiAmSar | stands for 1,8-diamino-3,6,10,13,16,19-hexaazabicyclo[6.6.6]icosane |
| diphosphine | stands for 2,3-bis(diphenylphosphaneyl)maleic acid |
| DM-TE2A | stands for 2,2'-(4,11-dimethyl-1,4,8,11-tetraazacyclotetradecane-1,8-diyl)diacetic acid |
| DO2A | stands for 1,4,7,10-tetraazacyclododecane-1,7-diacetic acid |
| DO2a2p | stands for 2,2'-(4,10-bis(phosphonomethyl)-1,4,7,10-tetraazacyclododecane-1,7-diyl)diacetic acid |
| DO2AP | stands for 2,2'-(4-(phosphonomethyl)-1,4,7,10-tetraazacyclododecane-1,7-diyl)diacetic acid |
| DO3A | stands for 1,4,7,10-tetraazacyclododecane-1,4,7-triacetic acid |
| DO3AM-acetic acid | stands for 2-(4,7,10-tris(2-amino-2-oxoethyl)-1,4,7,10-tetraazacyclododecan-1-yl)acetic acid |
| DOA3P | stands for 2-(4,7,10-tris(phosphonomethyl)-1,4,7,10-tetraazacyclododecan-1-yl)acetic acid |
| DOTA | stands for 1,4,7,10-tetrazacyclododecane-1,4,7,10-tetraacetic acid |
| DOTAGA | stands for 2-(4,7,10-tris(carboxymethyl)-1,4,7,10-tetraazacyclododecan-1-yl)pentanedioic acid |
| DOTMA | stands for (2R,2'R,2"R,2‴R)-2,2',2",2‴-(1,4,7,10-tetraazacyclododecane-1,4,7,10-tetrayl)tetrapropionic acid |
| DOTP | stands for ((1,4,7,10-tetraazacyclododecane-1,4,7,10-tetrayl)tetrakis(methylene))tetrakis(phosphonic acid) |
| Dp44mT | stands for 2-(di(pyridin-2-yl)methylene)-N,N-dimethylhydrazine-1-carbothioamide |
| DpC | stands for N-cyclohexyl-2-(di(pyridin-2-yl)methylene)-N-methylhydrazine-1-carbothioamide |
| DTC | stands for diethyldithiocarbamate |
| DTCBP | stands for (3-hydroxy-3,3-diphosphonopropyl)(methyl)carbamodithioic acid |
| DTPA | stands for diethylenetriaminepentaacetic acid |
| H2ATSM | stands for (2E,2'E)-2,2'-(butane-2,3-diylidene)bis(N-methylhydrazine-1-carbothioamide) |
| H2azapa | stands for 6,6'-((ethane-1,2-diylbis(((1-benzyl-1H-1,2,3-triazol-4-yl)methyl)azanediyl))bis(methylene))dipicolinic acid |
| H2CB-TE2A | stands for 2,2'-(1,4,8,11-tetraazabicyclo[6.6.2]hexadecane-4,11-diyl)diacetic acid |
| H2CHX-DEDPA | stands for 6,6'-((((1R,2R)-cyclohexane-1,2-diyl)bis(azanediyl))bis(methylene))dipicolinic acid |
| H2dedpa | stands for 6,6'-((ethane-1,2-diylbis(azanediyl))bis(methylene))dipicolinic acid |
| H2DO2A | stands for 2,2'-(1,4,7,10-tetraazacyclododecane-1,4-diyl)diacetic acid |
| H2ODO2A | stands for 2,2'-(1-oxa-4,7,10-triazacyclododecane-4,10-diyl)diacetic acid |
| H2PTSM | stands for (2E,2'E)-2,2'-(propane-1,2-diylidene)bis(N-methylhydrazine-1-carbothioamide) |
| H3 THP | stands for 4-amino-N1,N7-bis((3-hydroxy-1,6-dimethyl-4-oxo-1,4-dihydropyridin-2-yl)methyl)-4-(3-(((3-hydroxy-1,6-dimethyl-4-oxo-1,4-dihydropyridin-2-yl)methyl)amino)-3-oxopropyl)heptanediamide |
| H4octapa | stands for 6,6'-((ethane-1,2-diylbis((carboxymethyl)azanediyl))bis(methylene))dipicolinic acid |
| H5 decapa | stands for 6,6'-(((((carboxymethyl)azanediyl)bis(ethane-2,1-diyl))bis((carboxymethyl)azanediyl))bis(methylene))dipicolinic acid |
| HBED | stands for 2,2'-(ethane-1,2-diylbis((2-hydroxybenzyl)azanediyl))diacetic acid |
| HBED-CC | stands for 3,3'-(((ethane-1,2-diylbis((carboxymethyl)azanediyl))bis(methylene))bis(4-hydroxy-3,1-phenylene))dipropionic acid |
| HEHA | stands for 2,2',2",2‴,2ʺʺ,2‴ʺ-(1,4,7,10,13,16-hexaazacyclooctadecane-1,4,7,10,13,16-hexayl)hexaacetic acid |
| HP-DO3A | stands for (2R,2'R,2"R)-2,2',2"-(10-((R)-2-hydroxypropyl)-1,4,7,10-tetraazacyclododecane-1,4,7-triyl)tripropionic acid |
| HYNIC | stands for 6-hydrazineylnicotinic acid |
| macropa | stands for 6,6'-((1,4,10,13-tetraoxa-7,16-diazacyclooctadecane-7,16-diyl)bis(methylene))dipicolinic acid |
| macropaquin | stands for 6-((16-((8-hydroxyquinolin-2-yl)methyl)-1,4,10,13-tetraoxa-7,16-diazacyclooctadecan-7-yl)methyl)picolinic acid |
| macroquin-SO3 | stands for 8-hydroxy-2-((16-((1-hydroxy-4-sulfoisoquinolin-3-yl)methyl)-1,4,10,13-tetraoxa-7,16-diazacyclooctadecan-7-yl)methyl)quinoline-5-sulfonic acid |
| MAG3 | stands for (2-mercaptoacetyl)glycylglycylglycine |
| MAMA | stands for N-(2-mercaptoethyl)-2-((2-mercaptoethyl)amino)acetamide |
| MA-NOTMP | stands for ((1,4,7-triazonane-1,4,7-triyl)tris(methylene))tris(methylphosphinic acid) |
| MM-TE2A | stands for 2,2'-(4-methyl-1,4,8,11-tetraazacyclotetradecane-1,8-diyl)diacetic acid |
| N2S2 | stands for 2,2'-(ethane-1,2-diylbis(azanediyl))bis(ethane-1-thiol) |
| N4 | stands for N,N'-bis-(2-amino-ethyl)-propane-1,3-diamine |
| NETA | stands for 2,2'-((2-(4,7-bis(carboxymethyl)-1,4,7-triazonan-1-yl)ethyl)azanediyl)diacetic acid |
| NO2A | stands for 2,2'-(1,4,7-triazonane-1,4-diyl)diacetic acid |
| NO2AP | stands for 2,2'-(7-(((2-carboxyethyl)(hydroxy)phosphoryl)methyl)-1,4,7-triazonane-1,4-diyl)diacetic acid |
| NO2AP | stands for 2,2'-(7-(((2-carboxyethyl)(hydroxy)phosphoryl)methyl)-1,4,7-triazonane-1,4-diyl)diacetic acid |
| NODA | stands for 2,2'-(7-ethyl-1,4,7-triazonane-1,4-diyl)diacetic acid |
| NODAGA | stands for 2-(4,7-bis(carboxymethyl)-1,4,7-triazonan-1-yl)pentanedioic acid |
| NOPO | stands for 3-(((4,7-bis((hydroxy(hydroxymethyl)phosphoryl)methyl)-1,4,7-triazonan-1-yl)methyl)(hydroxy)phosphoryl)propanoic acid |
| NOTA | stands for 1,4,7-triazacyclononanetriacetic acid |
| NOTAM | stands for 2,2',2"-(1,4,7-triazonane-1,4,7-triyl)triacetamide |
| NOTP | stands for ((1,4,7-triazonane-1,4,7-triyl)tris(methylene))tris(phosphonic acid) |
| NTP | stands for (nitrilotris(methylene))tris(phosphonic acid) |
| NxS4-x | stands for a group of tetradentate chelators with N-atoms (basic amine or non-basic amide) and thiols as donors stabilizing Tc-complexes, especially Tc(V)-oxo complexes |
| oxo-DO3A | stands for 2,2',2"-(1-oxa-4,7,10-triazacyclododecane-4,7,10-triyl)triacetic acid |
| PCBA | stands for 2-((1,4,7,10,13-pentaazacyclopentadecan-1-yl)methyl)benzoic acid |
| PCB-TE1A1P | stands for 2-(11-(phosphonomethyl)-1,4,8,11-tetraazabicyclo[6.6.2]hexadecan-4-yl)acetic acid |
| PCTA | stands for 2,2',2"-(3,6,9-triaza-1(2,6)-pyridinacyclodecaphane-3,6,9-triyl)triacetic acid |
| p-EDDHA | stands for 2,2'-(ethane-1,2-diylbis(azanediyl))bis(2-(4-hydroxyphenyl)acetic acid) |
| PEPA | stands for 2,2',2",2‴,2ʺʺ-(1,4,7,10,13-pentaazacyclopentadecane-1,4,7,10,13-pentayl)pentaacetic acid |
| PIH | stands for (E)-N'-((3-hydroxy-5-(hydroxymethyl)-2-methylpyridin-4-yl)methylene)isonicotinohydrazide |
| PSC | stands for 2,2',2"-(10-(2-amino-2-oxoethyl)-1,4,7,10-tetraazacyclododecane-1,4,7-triyl)triacetic acid |
| pycup | stands for 1,8-(2,6-pyridinedimethylene)-1,4,8,11-tetraazacyclo-tetradecane |
| Sar | stands for 3,6,10,13,16,19-hexazabicyclo[6.6.6]icosane |
| SHBED | stands for 2,2'-(ethane-1,2-diylbis((2-hydroxy-5-sulfobenzyl)azanediyl))diacetic acid |
| Tachpyr | stands for N1,N3,N5-tris(pyridin-2-ylmethyl)cyclohexane-1,3,5-triamine |
| TACN | stands for 1,4,7-triazonane |
| TAM | stands for 2,3-dihydroxyterephthalamide |
| TAME | stands for 2-(aminomethyl)-2-methylpropane-1,3-diamine |
| TAME-Hex | stands for (1,8-N,N'-bis(carboxymethyl)-1,4,8,11-tetraazacyclotetradecane |
| TCE-DOTA | stands for (2R,2'R,2"R,2‴R)-2,2',2",2‴-(1,4,7,10-tetraazacyclododecane-1,4,7,10-tetrayl)tetraglutaric acid |
| TCMC | stands for 2,2',2",2"'-(1,4,7,10-tetraazacyclododecane-1,4,7,10-tetrayl)tetraacetamide |
| TE2A | stands for 2,2'-(1,4,8,11-tetraazabicyclo[6.6.2]hexadecane-4,11-diyl)diacetic acid |
| TE2P | stands for ((1,4,8,11-tetraazabicyclo[6.6.2]hexadecane-4,11-diyl)bis(methylene))bis(phosphonic acid) |
| TEAMA | stands for 2-(11-(2-amino-2-oxoethyl)-1,4,8,11-tetraazabicyclo[6.6.2]hexadecan-4-yl)acetic acid |
| TETA | stands for 2,2',2",2‴-(1,4,8,11-tetraazacyclotetradecane-1,4,8,11-tetrayl)tetraacetic acid |
| TMT-amine | stands for 2,2',2",2‴-(((4'-(3-amino-4-methoxyphenyl)-[2,2':6',2"-terpyridine]-6,6"-diyl)bis(methylene))bis(azanetriyl))tetraacetic acid |
| TRAP | stands for 3,3',3"-(((1,4,7-triazonane-1,4,7-triyl)tris(methylene))tris(hydroxyphosphoryl))tripropanoic acid |
| TREN(Me-3, 2-HOPO) | stands for N,N'-(((2-(3-hydroxy-2-oxo-1,2-dihydropyridine-4-carboxamido)ethyl)azanediyl)bis(ethane-2,1-diyl))bis(3-hydroxy-1-methyl-2-oxo-1,2-dihydropyridine-4-carboxamide) |
| TRITA | stands for 2,2',2",2‴-(1,4,7,10-tetraazacyclotridecane-1,4,7,10-tetrayl)tetraacetic acid |
| TSC | stands for (E)-2-(3-hydroxybenzylidene)hydrazine-1-carbothioamide |

HYNIC, DTPA, EDTA, DOTA, TETA, bisamino bisthiol (BAT)-based chelators as disclosed in US 5,720,934; desferrioxamine (DFO) as disclosed in Doulias et al. (Doulias, et al., Free Radic Biol Med, 2003, 35: 719), tetrapyridine and N3S, N2S2 and N4 chelators as disclosed in US 5,367,080 A, US 5,364,613 A, US 5,021,556 A, US 5,075,099 A, US 5,886,142 A, whereby all of the references are included herein by reference in their entirety. 6-amino-6-methylperhydro-1,4-diazepine-*N*,*N*',*N*",*N*"-tetraacetic acid (AAZTA) is disclosed in Pfister et al. (Pfister, et al., EJNMMI Res, 2015, 5: 74), deferiprone, a 1,2-dimethyl-3,4-hydroxypyridinone and hexadentate tris(3,4-hydroxypyridinone) (THP) are disclosed in Cusnir et al. (Cusnir, et al., Int J Mol Sci, 2017, 18*),* monoamine-monoamide dithiol (MAMA)-based chelators are disclosed in Demoin et al. (Demoin, et al., NuclMedBiol, 2016, 43: 802), macropa and analogs are disclosed in Thiele et al. (Thiele, et al., Angew Chem Int Ed Engl, 2017, 56: 14712), 1,4,7,10,13,16-hexaazacyclohexadecane-N,N*'*,N*"*,N‴,Nʺʺ,N‴ʺ-hexaacetic acid (HEHA) and PEPA analogs are disclosed in Price and Orvig (Price, et al., Chem Soc Rev, 2014, 43: 260), pycup and analogs are disclosed in Boros et al. (Boros, et al., Mol Pharm, 2014, 11: 617), N, N-bis(2-hydroxybenzyl)ethylenediamine-N,N-diacetic acid (HBED), 1,4,7,10-tetrakis (carbamoylmethyl)-1,4,7,10-tetraazacyclododecane (TCM), 2-[(carboxymethyl)]-[5-(4-nitrophenyl-1-[4,7,10-tris-(carboxymethyl)-1,4,7,10-tetraazacyclododecan-1-yl]pentan-2-yl)-amino]acetic acid (3p-C-DEPA), CB-TE2A, TE2A, TE1A1P, DiAmSar, 1-N-(4-aminobenzyl)-3,6,10,13,16,19-hexaazabicyclo[6.6.6]-eicosane-1,8-diamine (SarAr), NETA,, tris(2-mercaptoethyl)-1,4,7-triazacyclononane (TACN-TM), f 4-[2-(bis-carboxymethyl-amino)-ethyl]-7-carboxymethyl-[1,4,7]triazonan-1-yl}-acetic acid (NETA), diethylenetriaminepentaacetic acid (DTP), 3-({4,7-bis-[(2-carboxy-ethyl)-hydroxy-phosphinoylmethyl]-[1,4,7]triazonan-1-ylmethyl}-hydroxy-phosphinoyl)-propionic acid (TRAP), NOPO, H4octapa, SHBED, BPCA, 3,6,9,15-tetraazabicyclo[9.3.1]-pentadeca-1(15),11,13-triene-3,6,9,-triacetic acid (PCTA), and 1,4,7,10,13-pentaazacyclopentadecane-N,N',N",N‴,Nʺʺ-pentaacetic acid (PEPA) are disclosed in Price and Orvig (Price, et al., Chem Soc Rev, 2014, 43: 260), 1-hydroxy-2-pyridone ligand (HOPO) is disclosed in Allott et al. (Allott, et al., Chem Commun (Camb), 2017, 53: 8529), [4-carboxymethyl-6-(carboxymethyl-methyl-amino)-6-methyl-[1,4]diazepan-1-yl]-acetic acid (DATA) is disclosed in Tomesello et al. (Tomesello, et al., Molecules, 2017, 22: 1282), tetrakis(aminomethyl)methane (TAM) and analogs are disclosed in McAuley 1988 (McAuley, et al., Canadian Journal of Chemistry, 1989, 67: 1657), hexadentate tris(3,4-hydroxypyridinone) (THP) and analogs are disclosed in Ma et al. (Ma, et al., Dalton Trans, 2015, 44: 4884).

The diagnostic and/or therapeutic use of some of the above chelators is described in the prior art. For example, 2-hydrazino nicotinamide (HYNIC) has been widely used in the presence of a coligand for incorporation of ^{99m}Tc and ^{186,188}Re (Schwartz, et al., Bioconjug Chem, 1991, 2: 333; Babich, et al., J Nucl Med, 1993, 34: 1964; Babich, et al., Nucl MedBiol, 1995, 22: 25); DTPA is used in Octreoscan^{®} for complexing ¹¹¹In and several modifications are described in the literature (Li, et al., Nucl Med Biol, 2001, 28: 145; Brechbiel, et al., Bioconjug Chem, 1991, 2: 187); DOTA-type chelators for radiotherapy applications are described by Tweedle et al. (US Pat 4,885,363); other polyaza macrocycles for chelating trivalent isotopes metals are described by Eisenwiener *et al.* (Eisenwiener, et al., Bioconjug Chem, 2002, 13: 530); and N4-chelators such as a ^{99m}Tc-N4-chelator have been used for peptide labeling in the case of minigastrin for targeting CCK-2 receptors (Nock, et al., J Nucl Med, 2005, 46: 1727).

In an embodiment the chelator is a metal chelator is selected from the group, but not limited to, comprising DOTA, DOTAGA, DOTAM, DOTP, NOTA, NODAGA, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)₃-chelators and their analogs. Preferably, the chelator additionally comprises one or more functional groups or functionalities allowing attachment to the compound of the invention.

The chemical structures thereof being as follows:

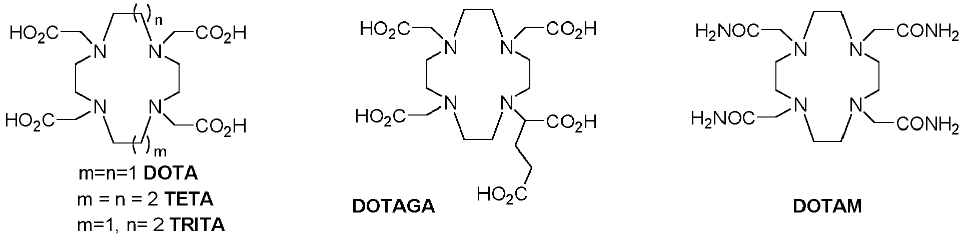

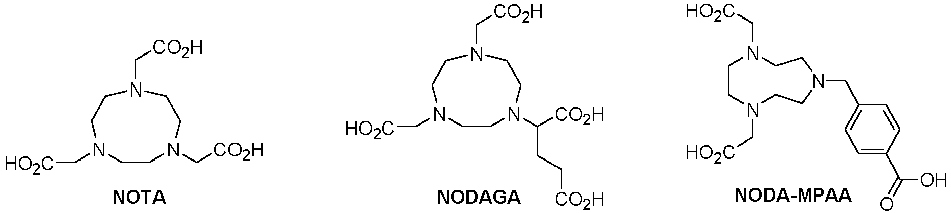

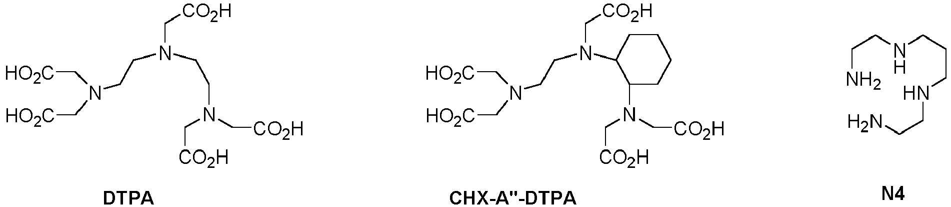

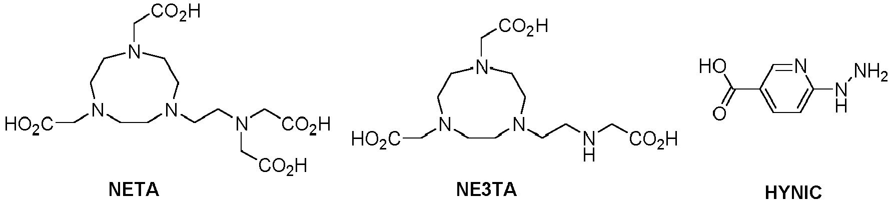

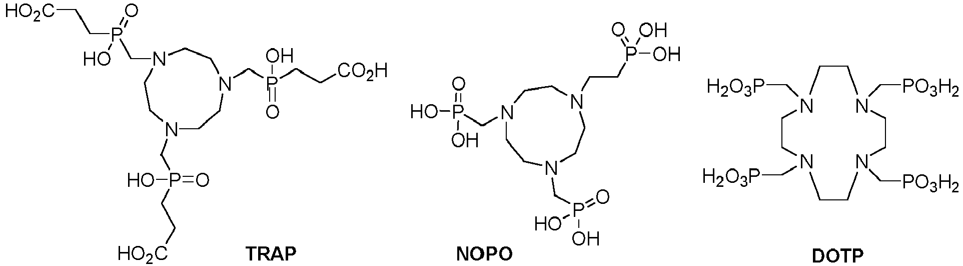

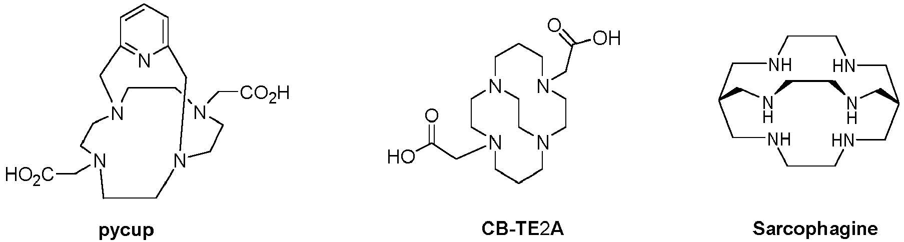

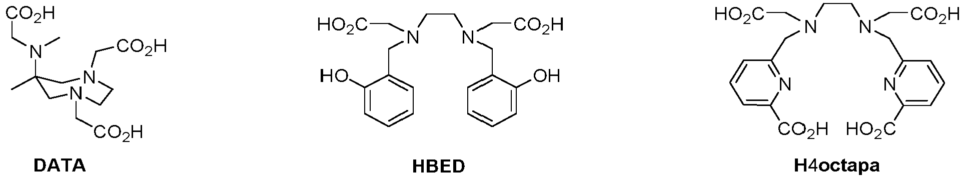

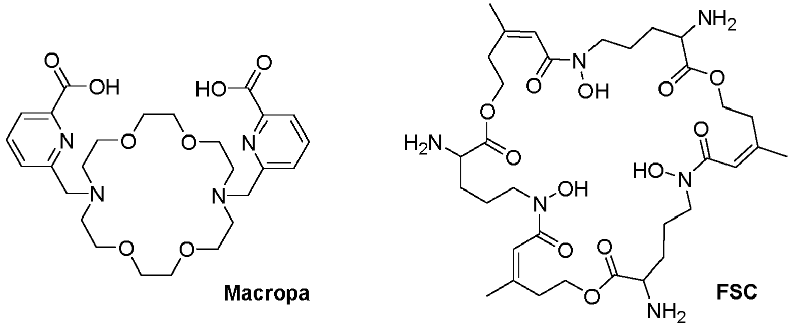

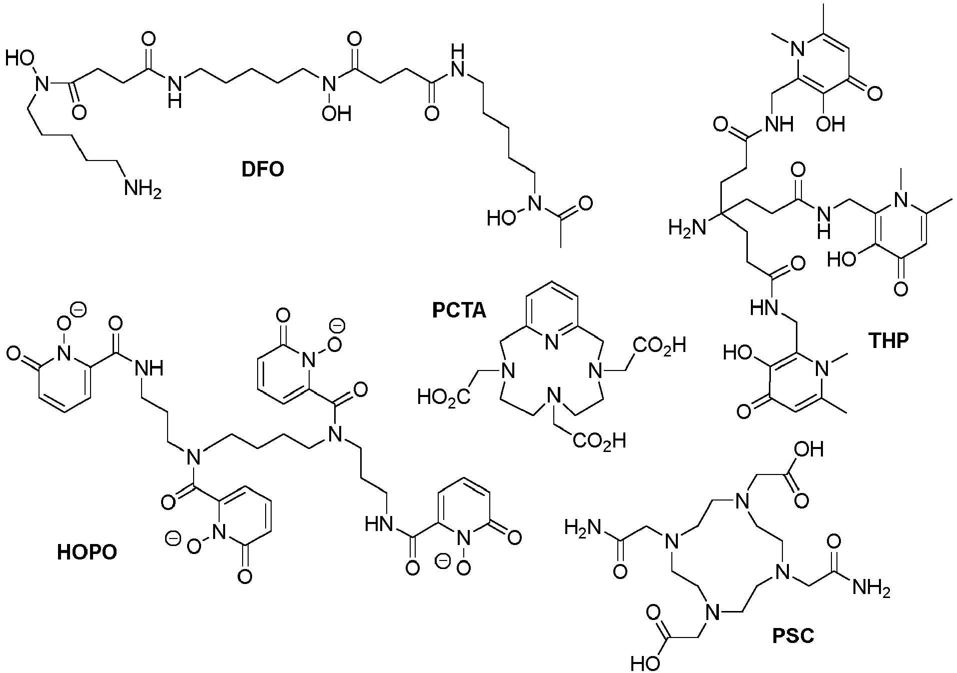

In a preferred embodiment, the metal chelator is selected from the group consisting of DOTA, DOTAGA, DOTAM, NOTA, NODAGA, NODA-MPAA, NOPO, HBED, DTPA, CHX-A"-DTPA, CB-TE2A, Macropa, PCTA, PSC, N4, and analogs thereof. Preferably, the chelator additionally comprises one or more functional groups or functionalities allowing attachment to the compounds of the invention.

In a more preferred embodiment, the metal chelator is selected from the group consisting of DOTA, DOTAGA, NODAGA, and macropa and their analogs thereof. Preferably, the chelator additionally comprises one or more functional groups or functionalities allowing attachment to the compounds of the invention.

It will be acknowledged by the persons skilled in the art that the chelator, in principle, may be used regardless whether the compound of the invention is used in or suitable for diagnosis or therapy. Such principle is, among others, outlined in international patent application WO 2009/109332 A1.

It will be further acknowledged by the persons skilled in the art that the presence of a chelator in the compound of the invention includes, if not stated otherwise, the possibility that the chelator is complexed to any metal complex partner, i.e. any metal which, in principle, can be complexed by the chelator. An explicitly mentioned chelator of a compound of the invention or the general term chelator in connection with the compound of the invention refers either to the uncomplexed chelator as such or to the chelator to which any metal complex partner is bound, wherein the metal complex partner is any radioactive or non-radioactive metal complex partner. Preferably the chelator-metal complex, i.e. the chelator to which the metal complex partner is bound, is a stable chelator-metal complex.

Non-radioactive chelator-metal complexes have several applications, e.g., for assessing properties like stability or activity which are otherwise difficult to determine. One aspect is that cold variants of the radioactive versions of the metal complex partner (e.g., non-radioactive indium complexes es described in the examples) can act as surrogates of the radioactive compounds. Furthermore, they are valuable tools for identifying metabolites *in vitro* or *in vivo,* as well as for assessing toxicity properties of the compounds of invention. Additionally, chelator-metal complexes can be used in binding assays utilizing the fluorescence properties of some metal complexes with distinct ligands (e.g., Europium salts).

Chelators can be synthesized or are commercially available with a wide variety of (possibly already activated) groups for the conjugation to peptides or amino acids.

Direct conjugation of a chelator to an amino-nitrogen of the respective compound of invention is well possible for chelators selected from the group consisting of DTPA, DOTA, DOTAGA, NOTA, NODAGA, NODA-MPAA, HBED, TETA, CB-TE2A, DFO, DATA, sarcophagine and N4, preferably DTPA, DOTA, DOTAGA, NOTA, NODAGA, NODA-MPAA, CB-TE2A, and N4. The preferred linkage in this respect is an amide linkage.

Direct conjugation of an isothiocyanate-functionalized chelator to an amino-nitrogen of the respective compound of invention is well possible for chelators selected from the group consisting of DOTA, DOTAGA, NOTA, NODAGA, DTPA, CHX-A"-DTPA, DFO, and THP, preferably DOTA, DOTAGA, NOTA, NODAGA, DTPA, and CHX-A"-DTPA. The preferred linkage in this respect is a thiourea linkage.

Functional groups at a chelator, which are preferred precursors for the direct conjugation of a chelator to an amino-nitrogen are known to the person skilled in the art and include but are not limited to carboxylic acid, activated carboxylic acid, e.g., active ester like for instance NHS-ester, pentafluorophenol-ester, HOBt-ester, HOAt-ester, and isothiocyanate.

Functional groups at a chelator, which are preferred precursors for the direct conjugation of a chelator to a carboxylic group are known to the person skilled in the art and include but are not limited to alkylamino and arylamino nitrogens. Respective chelator reagents are commercially available for some chelators, e.g., for DOTA with either alkylamino or arylamino nitrogen.

Functional groups at a chelator, which are preferred precursors for the direct conjugation of a chelator to a thiol group are known to the person skilled in the art and include but are not limited to maleimide nitrogens. Respective chelator reagents are commercially available for some chelators, e.g., for DOTA with maleimide nitrogen.

Functional groups at a chelator, which are preferred precursors for the direct conjugation of a chelator to an azide group are known to the person skilled in the art and include but are not limited to acyclic and cyclic alkynes. Respective chelator reagents are commercially available for some chelators, e.g., for DOTA with propargyl or butynyl.

Functional groups at a chelator, which are preferred precursors for the direct conjugation of a chelator to an alkyne group are known to the person skilled in the art and include but are not limited to alkyl and aryl azines. Respective chelator reagents are commercially available for some chelators, e.g., for DOTA with azidopropyl.

In an embodiment, the compound of the invention comprises a bio-distribution modifier. Preferably a bio-distribution modifier is part of the compound of the invention, whereby the bio-distribution modifier is either directly or indirectly such as by a linker attached to the compound of the invention. More preferably a second bio-distribution modifier is part of the compound of the invention, whereby the second bio-distribution modifier is either directly or indirectly such as by a linker attached to the compound of the invention.

The function of a bio-distribution can be the attenuation of the unintended or undesired bio-distribution of the compound of the invention by altering physio-chemical properties of the compound of the invention. To that end, elements with a multitude of hydroxyl groups are implemented within or are attached to the peptide of the invention.

The function of a bio-distribution can be the alteration of serum half-life of the compound by modulation of the renal clearance (Du, et al., Bioconjugate Chem, 2020, 31: 241).

The function of a bio-distribution can be the modulation of serum half-life of the compound of the invention by binding to serum components. (Lorenz, et al., Bioorganic Med Chem Lett, 2013, 23: 4011).

In an embodiment, the bio-distribution modifier is a molecular structure comprising at least one alkyl group comprising two or more primary hydroxyl groups, a linking chemical moiety and an attachment moiety, whereby the attachment moiety is, depending on the position of the bio-distribution modifier within a compound of the invention, either a carboxylic acid or an amino acid comprising a side chain. The attachment moiety covalently links the bio-distribution modifier to the intended position within a compound of the invention; the attachment moiety is covalently linked to the linking chemical moiety; and the linking chemical moiety is covalently linked to the at least one alkyl group comprising two or more primary hydroxyl groups. Without wishing to be bound by any theory, the function of the primary hydroxyl groups is the increased interaction with water molecules in the surrounding of a compound of the invention thereby attenuating hydrophobic properties of the compound. It is within the present invention, that there are at least two types of alkyl groups comprising two or more primary hydroxyl groups, which are categorized depending on their position and/or topology of attachment within the bio-distribution modifier: A first type is a first level alkyl group, and a second type is a second level alky group. A first level alkyl group is covalently attached to the linking chemical moiety of the bio-distribution modifier. A second level alkyl group is covalently attached to a first level alkyl group via an ether linkage.

In a preferred embodiment, a first bio-distribution modifier is located at the N-terminus of the compound of the invention, thereby constituting the N-terminal modification group A and second bio-distribution modifier is located at the C-terminus of the compound of the invention, thereby constituting the C-terminal modification group C-term, while the chelator preferably is located and thus comprised by Xaa8.

In an embodiment, a third bio-distribution modifier is located at and thus comprised by Xaa4.

In an embodiment, a third bio-distribution modifier is located at and thus comprised by Xaa5.

In an embodiment, a third bio-distribution modifier is located at and thus comprised by Xaa8, while the chelator preferably is located and thus comprised by Xaa4.

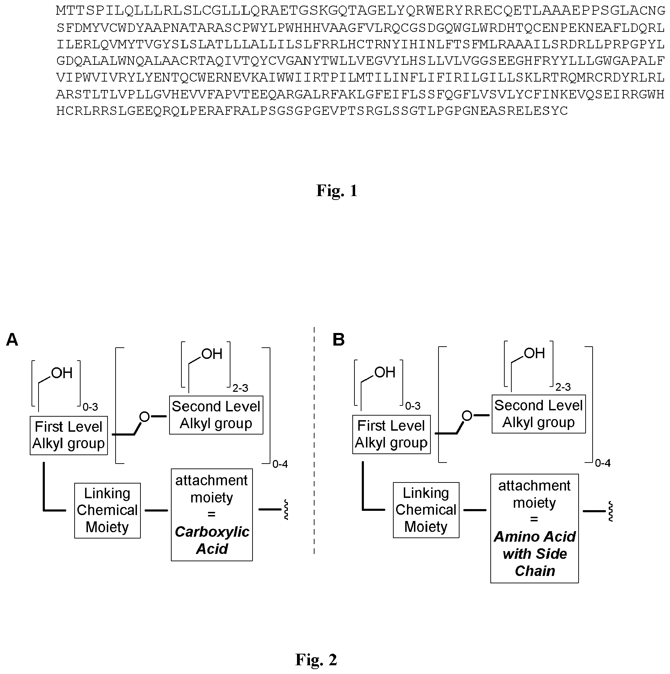

In an embodiment, wherein the bio-distribution modifier is located at the N-terminus of the compound of the invention, thereby constituting the N-terminal modification group A or is located at and thus comprised by Xaa4, Xaa5 or Xaa8, the attachment moiety is a carboxylic acid as depicted in Fig. 2A. Preferably the carboxylic acid is an aliphatic carboxylic acid.

In an embodiment, wherein the bio-distribution modifier is located at the C-terminus of the compound of the invention, thereby constituting the C-terminal modification group C-term, the attachment moiety is an amino acid with a side chain as depicted in Fig. 2B. Preferably the amino acid with a side chain is an α-amino acid with an aliphatic side chain.

In an embodiment the linking chemical moiety, which is covalently attached to the attachment moiety, which is either a carboxylic acid or an amino acid with a side chain is preferably selected from the group comprising a nitrogen atom, a triazolyl moiety, a hydroxymethyl-triazolyl moiety and a urea moiety. The linking chemical moiety, depicted in Fig. 2, also serves to form a linkage of the carboxylic acid to one or two first level alkyl groups. The number of first level alkyl groups depends on the chemical nature of the linking chemical moiety. To a nitrogen atom one or two first level alkyl groups can be attached. To a triazolyl moiety, a hydroxymethyl-triazolyl moiety or a urea moiety one first level alkyl group can be attached. A first level alkyl group is a (C₃-C₅)alkyl group, preferably a first level alkyl group is a structure selected from the group comprising Alkyl-1, Alkyl-2, Alkyl-3 and Alkyl-4, with Alkyl-1 being as follows 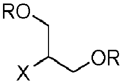 (Alkyl-1), Alkyl-2 being as follows 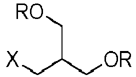 (Alkyl-2), Alkyl-3 being as follows 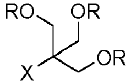 (Alkyl-3) and Alkyl-4 being as follows 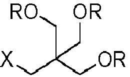 (Alkyl-4). In each and any of first level alkyl groups Alkyl-1, Alkyl-2, Alkyl-3 and Alkyl-4, 'X' indicates the linking chemical moiety. In each and any of Alkyl-1 and Alkyl-2, each and any R is either a hydrogen atom, thereby forming a primary hydroxyl group, or a second level alkyl group. The second level alkyl group is a (C₃-C₅)alkyl group, preferably the second level alkyl group is selected from the group comprising Alkyl-5, Alkyl-6, Alkyl-7 and Alkyl-8, more preferably the second level alkyl group is selected from Alkyl-5 and Alkyl-6, with Akyl-5 being as follows 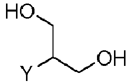 (Alkyl-5), Akyl-6 being as follows 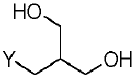 (Alkyl-6), Akyl-7 being as follows 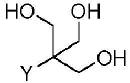 (Alkyl-7) and Akyl-8 being as follows 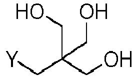 (Alkyl-8). If in Alkyl-1 and Alkyl-2 R is a second level alkyl group, both R in Alkyl-1 and Alkyl-2, respectively, are preferably identical. In each and any of Alkyl-3 and Alkyl-4, each and any R is a hydrogen atom, thereby forming a primary hydroxyl group, and/or a second level alkyl group. The second level alkyl group is a (C₃-C₅)alkyl group, preferably the second level alkyl group is selected from the group comprising Alkyl-5, Alkyl-6, Alkyl-7 and Alkyl-8, more preferably the second level alkyl group is selected from Alkyl-5 and Alkyl-6. If more than one second level alkyl group is attached to a first level alkyl group Alkyl-3 or Alkyl-4, the second level alkyl groups are preferably identical.

In an embodiment of the present invention, preferably an embodiment of the first aspect of the present invention, a bio-distribution modifier is comprised by one of Xaa4, Xaa5 and Xaa8. In this embodiment, the bio-distribution modifier is preferably a bio-distribution modifier disclosed herein comprised by the N-terminal modification group A, more preferably a bio-distribution modified as disclosed in any one of Embodiments 120 to 160.

For a bio-distribution modifier which is comprised by the N-terminal modification group A, Xaa4, Xaa5 and/or Xaa8 and which comprises an attachment moiety, a linking chemical moiety and one or two first level alkyl groups, a summary denominator such as 'HPD2:Ahx' may be used so as to describe the topology of the bio-distribution modifier. Therein, the first part ("HPD2" in this example) refers to the first level alkyl group ("HPD" in this example) and the number of first level alkyl groups ("2" in this example) comprised by the bio-distribution modifier. The second part of the denominator ("Ahx" in this example) refers to the combination of the attachment moiety and the linking chemical moiety.

For a bio-distribution modifier which is comprised by the N-terminal modification group A, Xaa4, Xaa5 and/or Xaa8 and which comprises an attachment moiety, a linking chemical moiety, one or two first level alkyl groups and one, two, three or four second level alkyl groups, a summary denominator such as 'HAC4:HPD2:Ahx' may be used so as to describe the topology of the bio-distribution modifier. Therein, the first part ("HAC4" in this example) refers to the second level alkyl group ("HAC" in this example) and the number of second level alkyl groups ("4" in this example) comprised by the bio-distribution modifier. The second part of the denominator ("HPD2" in this example) refers to the first level alkyl group ("HPD" in this example) and the number of first level alkyl groups ("2" in this example) comprised by the bio-distribution modifier. The third part of the denominator ("Ahx" in this example) refers to the combination of the attachment moiety and the linking chemical moiety.

For a bio-distribution modifier which is comprised by the C-terminal group C-term and which comprises an attachment moiety, a linking chemical moiety and one or two first level alkyl groups, a summary denominator such as 'lys:HPD2' may be used so as to describe the topology of the bio-distribution modifier. Therein, the first part ("lys" in this example) refers to the combination of the attachment moiety and the linking chemical moiety. The second part of the denominator ("HPD2" in this example) refers to the first level alkyl group ("HPD" in this example) and the number of first level alkyl groups ("2" in this example) comprised by the bio-distribution modifier.

For a bio-distribution modifier which is comprised by the C-terminal group C-term and which comprises an attachment moiety, a linking chemical moiety and one or two first level alkyl groups and one, two, three or four second level alkyl groups, a summary denominator such as 'lys:HPD2:HAC4' may be used so as to describe the topology of the bio-distribution modifier. Therein the first part ("lys" in this example) refers to the combination of the attachment moiety and the linking chemical moiety. The second part of the denominator ("HPD2" in this example) refers to the first level alkyl group ("HPD" in this example) and the number of first level alkyl groups ("2" in this example) comprised by the bio-distribution modifier. The third part of the denominator ("HAC4" in this example) refers to the second level alkyl group ("HAC" in this example) and the number of second level alkyl groups ("4" in this example) comprised by the bio-distribution modifier.

In an embodiment, the compound of the invention is present as a pharmaceutically acceptable salt.

A "pharmaceutically acceptable salt" of the compound of the present invention is preferably an acid salt or a base salt that is generally considered in the art to be suitable for use in contact with the tissues of human beings or animals without excessive toxicity or carcinogenicity, and preferably without irritation, allergic response, or other problem or complication. Such salts include mineral and organic acid salts of basic residues such as amines, as well as alkali or organic salts of acidic residues such as carboxylic acids. Compounds of the invention are capable of forming internal salts which are also pharmaceutically acceptable salts.

Suitable pharmaceutically acceptable salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH₂)ₙ-COOH where n is any integer from 0 to 4, i.e., 0, 1, 2, 3, or 4, and the like. Similarly, pharmaceutically acceptable cations include, but are not limited to sodium, potassium, calcium, aluminum, lithium and ammonium. Those of ordinary skill in the art will recognize further pharmaceutically acceptable salts for the compounds provided herein. In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in water or in an organic solvent, or in a mixture of the two. Generally, the use of non-aqueous media, such as ether, ethyl acetate, ethanol, isopropanol or acetonitrile, is preferred.

A "pharmaceutically acceptable solvate" of the compound of the invention is preferably a solvate of the compound of the invention formed by association of one or more solvent molecules to one or more molecules of a compound of the invention. Preferably, the solvent is one which is generally considered in the art to be suitable for use in contact with the tissues of human beings or animals without excessive toxicity or carcinogenicity, and preferably without irritation, allergic response, or other problem or complication. Such solvent includes an organic solvent such as alcohols, ethers, esters and amines.

A "hydrate" of the compound of the invention is formed by association of one or more water molecules to one or more molecules of a compound of the invention. Such hydrate includes but is not limited to a hemi-hydrate, mono-hydrate, dihydrate, trihydrate and tetrahydrate.

Independent of the hydrate composition all hydrates are generally considered as pharmaceutically acceptable.

The compound of the invention has a high binding affinity to the GIPR. The affinity is determined as described in example 7 by the FACS binding assay. Because of a high binding affinity, the compound of the invention is effective as, useful as and/or suitable as a targeting agent and, if conjugated to another moiety, as a targeting moiety. As preferably used herein a targeting agent is an agent which interacts with the target molecule which is in the instant case said GIPR. In terms of cells and tissues thus targeted by the compound of the invention any cell and tissue, respectively, expressing said GIPR is or may be targeted.

In an embodiment, the compound interacts with a gastric inhibitory polypeptide receptor (GIPR), preferably with human GIPR having an amino acid sequence of SEQ ID NO: 1 or a homolog thereof, wherein the amino acid sequence of the homolog has an identity of GIPR that is at least 85% to the amino acid sequence of SEQ ID NO: 1. In preferred embodiments, the identity is 90%, preferably 95 %, 96 %, 97 %, 98 % or 99%.

The identity between two nucleic acid molecules can be determined as known to the person skilled in the art. More specifically, a sequence comparison algorithm may be used for calculating the percent sequence homology for the test sequence(s) relative to the reference sequence, based on the designated program parameters. The test sequence is preferably the sequence or protein or polypeptide which is said to be identical or to be tested whether it is identical, and if so, to what extent, to a different protein or polypeptide, whereby such different protein or polypepetide is also referred to as the reference sequence and is preferably the protein or polypeptide of wild type, more preferably the human GIPR of SEQ ID NO: 1.

Optimal alignment of sequences for comparison can be conducted, e.g., by the local homology algorithm of Smith & Waterman (Smith, et al., Advances in Applied Mathematics, 1981, 2: 482), by the homology alignment algorithm of Needleman & Wunsch (Needleman, et al., J Mol Biol, 1970, 48: 443), by the search for similarity method of Pearson & Lipman (Pearson, et al., Proc Natl Acad Sci U S A, 1988, 85: 2444), by computerized implementations of these algorithms (GAP, BESTFIT, FASTA, and TFASTA in the Wisconsin Genetics Software Package, Genetics Computer Group, 575 Science Dr., Madison, Wis.), or by visual inspection.

One example of an algorithm that is suitable for determining percent sequence identity is the algorithm used in the basic local alignment search tool (hereinafter "BLAST "), see, e.g. Altschul et al., 1990 (Altschul, et al., J Mol Biol, 1990, 215: 403) and Altschul et al., 1997 (Altschul, et al., Nucleic Acids Res, 1997, 25: 3389). Software for performing BLAST analyses is publicly available through the National Center for Biotechnology Information (hereinafter "NCBI"). The default parameters used in determining sequence identity using the software available from NCBI, e.g., BLASTN (for nucleotide sequences) and BLASTP (for amino acid sequences) are described in McGinnis et al. (McGinnis, et al., Nucleic Acids Res, 2004, 32: W20).

It is within the present invention that the compound of the invention is used or is for use in a method for the treatment of a disease as disclosed herein. Such method, preferably, comprises the step of administering to a subject in need thereof a therapeutically effective amount of the compound of the invention. Such method includes, but is not limited to, curative or adjuvant cancer treatment. It is used as palliative treatment where cure is not possible and the aim is for local disease control or symptomatic relief or as therapeutic treatment where the therapy has survival benefit and it can be curative.

The method for the treatment of a disease as disclosed herein includes the treatment of the disease disclosed herein, including tumors and cancer, and may be used either as the primary therapy or as second, third, fourth or last line therapy. It is also within the present invention to combine the compound of the invention with further therapeutic approaches. It is well known to the person skilled in the art that the precise treatment intent including curative, adjuvant, neoadjuvant, therapeutic, or palliative treatment intent will depend on the tumor type, location, and stage, as well as the general health of the patient.

In an embodiment, the diseases which may be treated by the compound of the invention is selected from the group comprising medullary thyroid cancer, gastrointestinal neuroendocrine neoplasms, ileal neuroendocrine neoplasms, pancreatic neuroendocrine neoplasms, nonfunctioning neuroendocrine neoplasms, Insulinomas, Gastrinomas, Glucagonomas, VIPomas, Somatostatinoma, ACTHoma, lung neuroendocrine neoplasms, typical carcinoid tumors, atypical carcinoid tumors, small cell carcinoma of the lung, large cell carcinoma of the lung, thymic neuroendocrine tumors, Merkel cell carcinoma, Pheochromocytoma of the adrenal gland, adrenal cancer, parathyroid cancer, paraganglioma, pituitary gland tumors, neuroendocrine tumors of the ovaries and neuroendocrine tumors of the testicles.

In a further embodiment, the disease which may be treated by the compound of the invention is a metabolic disease. Preferably, the metabolic disease is one where GIPR is expressed, more preferably where the GIPR expression is increased in pancreatic beta cells. In a still further embodiment, the disease is selected from the group comprising metabolic diseases and disorders, preferably type 2 diabetes, type 1 diabetes, metabolic syndrome, insulin resistance, dyslipidemia, impaired fasting glucose, and impaired glucose tolerance.

In an embodiment, the compounds of the present invention are used to detect cells and tissues overexpressing the GIPR, whereby such detection is achieved by conjugating a detectable label to the compounds of the invention, preferably a detectable radionuclide. In a preferred embodiment, the cells and tissues detected are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g., neoplasms, tumors, and cancers) or a non-oncology indication (e.g., metabolic diseases, preferably type 2 diabetes, insulin restistance, impaired glucose tolerance).

In another embodiment, the compounds of the present invention are used to treat cells and tissues overexpressing the GIPR. In a preferred embodiment, the cells and tissues treated are diseased cells and tissues and/or are either a or the cause for the disease and/or the symptoms of the disease, or are part of the pathology underlying the disease. In a further preferred embodiment, the diseased cells and tissues are causing and/or are part of an oncology indication (e.g. neoplasms, tumors, and cancers) and the therapeutic activity is achieved by conjugating therapeutically active effector to the compounds of the present invention, preferably a therapeutically active radionuclide. In a further preferred embodiment, the diseased cells and tissues are causing and/or are part of a non-oncology indication (e.g., metabolic diseases, insulin resistance, type 1 and type 2 diabetes) and the therapeutic activity is either achieved by targeting of the GIPR or it is monitored by GIPR imaging.

In a further embodiment, particularly if the disease is a non-oncology disease or a non-oncology indication (e.g. metabolic diseases, insulin resistance, type 1 and type 2 diabetes), the compounds of the present invention are administered in therapeutically effective amounts; preferably the compound of the present invention does not comprise a therapeutically active nuclide such as a therapeutically active nuclide An effective amount is a dosage of the compound sufficient to provide a therapeutically or medically desirable result or effect in the subject to which the compound is administered. The effective amount will vary with the particular condition being treated, the age and physical condition of the subject being treated, the severity of the condition, the duration of the treatment, the nature of the concurrent or combination therapy (if any), the specific route of administration and like factors within the knowledge and expertise of the health practitioner. For example, in connection with methods directed towards treating subjects having a condition characterized by abnormal cell proliferation, an effective amount to inhibit proliferation would be an amount sufficient to reduce or halt altogether the abnormal cell proliferation so as to slow or halt the development of or the progression of a cell mass such as, for example, a tumor. As used in the embodiments, "inhibit" embraces all of the foregoing.

In other embodiments, a therapeutically effective amount will be an amount necessary to extend the dormancy of micrometastases or to stabilize any residual primary tumor cells following surgical or drug therapy.

Generally, when using an unconjugated compound without a therapeutically active radionuclide, a therapeutically effective amount will vary with the subject's age, condition, and sex, as well as the nature and extent of the disease in the subject, all of which can be determined by one of ordinary skill in the art. The dosage may be adjusted by the individual physician or veterinarian, particularly in the event of any complication. A therapeutically effective amount is typically, but not limited to, an amount in a range from 0.1 µg/kg to about 2000 mg/kg, or from 1.0 µg/kg to about 1000 mg/kg, or from about 0.1 mg/kg to about 500 mg/kg, or from about 1.0 mg/kg to about 100 mg/kg, in one or more dose administrations daily, for one or more days. If desired, the effective daily dose of the active compound may be administered as two, three, four, five, six, or more sub-doses for example administered separately at appropriate intervals throughout the day, optionally, in unit dosage forms. In some embodiments, the compounds are administered for more than 7 days, more than 10 days, more than 14 days and more than 20 days. In still other embodiments, the compound is administered over a period of weeks, or months. In still other embodiments, the compound is delivered on alternate days. For example, the agent is delivered every two days, or every three days, or every four days, or every five days, or every six days, or every week, or every month.

In a preferred embodiment, the compound of the present invention is for use in the treatment and/or prevention of a disease, whereby such treatment is radionuclide therapy.

Preferably, radionuclide therapy makes use of or is based on different forms of radiation emitted by a radionuclide. Such radiation can, for example, be any one of radiation of photons, radiation of electrons including but not limited to β'-particles and Auger-electrons, radiation of protons, radiation of neutrons, radiation of positrons, radiation of α-particles or an ion beam. Depending on the kind of particle or radiation emitted by said radionuclide, radionuclide therapy can, for example, be distinguished as photon radionuclide therapy, electron radionuclide therapy, proton radionuclide therapy, neutron radionuclide therapy, positron radionuclide therapy, α-particle radionuclide therapy or ion beam radionuclide therapy. All of these forms of radionuclide therapy are encompassed by the present invention, and all of these forms of radionuclide therapy can be realized by the compound of the invention, preferably under the proviso that the radionuclide attached to the compound of the invention, more preferably as an effector, is providing for this kind of radiation.

Radionuclide therapy preferably works by damaging the DNA of cells. The damage is caused by a photon, electron, proton, neutron, positron, α-particle or ion beam directly or indirectly ionizing the atoms which make up the DNA chain. Indirect ionization happens as a result of the ionization of water, forming free radicals, notably hydroxyl radicals, which then damage the DNA.

In the most common forms of radionuclide therapy, most of the radiation effect is through free radicals. Because cells have mechanisms for repairing DNA damage, breaking the DNA on both strands proves to be the most significant technique in modifying cell characteristics. Because cancer cells generally are undifferentiated and stem cell-like, they reproduce more, and have a diminished ability to repair sub-lethal damage compared to most healthy differentiated cells. The DNA damage is inherited through cell division, accumulating damage to the cancer cells, causing them to die or reproduce more slowly.

Oxygen is a potent radiosensitizer, increasing the effectiveness of a given dose of radiation by forming DNA-damaging free radicals. Therefore, use of high-pressure oxygen tanks, blood substitutes that carry increased oxygen, hypoxic cell radiosensitizers such as misonidazole and metronidazole, and hypoxic cytotoxins, such as tirapazamine may be applied.

Other factors that are considered when selecting a radioactive dose include whether the patient is receiving chemotherapy, whether radiation therapy is being administered before or after surgery, and the degree of success of surgery.

The total radioactive dose may be fractionated, i.e. spread out over time in one or more treatments for several important reasons. Fractionation allows normal cells time to recover, while tumor cells are generally less efficient in repair between fractions. Fractionation also allows tumor cells that were in a relatively radio-resistant phase of the cell cycle during one treatment to cycle into a sensitive phase of the cycle before the next fraction is given. Similarly, tumor cells that were chronically or acutely hypoxic and, therefore, more radioresistant, may reoxygenate between fractions, improving the tumor cell kill.

It is generally known that different cancers respond differently to radiation therapy. The response of a cancer to radiation is described by its radiosensitivity. Highly radiosensitive cancer cells are rapidly killed by modest doses of radiation. These include leukemias, most lymphomas, and germ cell tumors.

It is important to distinguish radiosensitivity of a particular tumor, which to some extent is a laboratory measure, from "curability" of a cancer by an internally delivered radioactive dose in actual clinical practice. For example, leukemias are not generally curable with radiotherapy, because they are disseminated through the body. Lymphoma may be radically curable if it is localized to one area of the body. Similarly, many of the common, moderately radioresponsive tumors can be treated with curative doses of radioactivity if they are at an early stage. This applies, for example, to non-melanoma skin cancer, head and neck cancer, non-small cell lung cancer, cervical cancer, anal cancer, prostate cancer.

The response of a tumor to radiotherapy is also related to its size. For complex reasons, very large tumors respond less well to radiation than smaller tumors or microscopic disease. Various strategies are used to overcome this effect. The most common technique is surgical resection prior to radiotherapy. This is most commonly seen in the treatment of breast cancer with wide local excision or mastectomy followed by adjuvant radiotherapy. Another method is to shrink the tumor with neoadjuvant chemotherapy prior to radical radionuclide therapy. A third technique is to enhance the radiosensitivity of the cancer by giving certain drugs during a course of radiotherapy. Examples of radiosensiting drugs include, but are not limited to Cisplatin, Nimorazole, and Cetuximab.

Introperative radiotherapy is a special type of radiotherapy that is delivered immediately after surgical removal of the cancer. This method has been employed in breast cancer (TARGeted Introperative radioTherapy), brain tumors and rectal cancers.

Radionuclide therapy is in itself painless. Many low-dose palliative treatments cause minimal or no side effects. Treatment to higher doses may cause varying side effects during treatment (acute side effects), in the months or years following treatment (long-term side effects), or after re-treatment (cumulative side effects). The nature, severity, and longevity of side effects depends on the organs that receive the radiation, the treatment itself (type of radionuclide, dose, fractionation, concurrent chemotherapy), and the patient.

It is within the present inventions that the method for the treatment of a disease of the invention may realize each and any of the above strategies which are as such known in the art, and which insofar constitute further embodiments of the invention.

It is also within the present invention that the compound of the invention is used in a method for the diagnosis of a disease as disclosed herein. Such method, preferably, comprises the step of administering to a subject in need thereof a diagnostically effective amount of the compound of the invention.

In accordance with the present invention, an imaging method is selected from the group consisting of scintigraphy, Single Photon Emission Computed Tomography (SPECT) and Positron Emission Tomography (PET).

Scintigraphy is a form of diagnostic test or method used in nuclear medicine, wherein radiopharmaceuticals are internalized by cells, tissues and/or organs, preferably internalized in vivo, and radiation emitted by said internalized radiopharmaceuticals is captured by external detectors (gamma cameras) to form and display two-dimensional images. In contrast thereto, SPECT and PET forms and displays three-dimensional images. Because of this, SPECT and PET are classified as separate techniques to scintigraphy, although they also use gamma cameras to detect internal radiation. Scintigraphy is unlike a diagnostic X-ray where external radiation is passed through the body to form an image.

Single Photon Emission Tomography (SPECT) scans are a type of nuclear imaging technique using gamma rays. They are very similar to conventional nuclear medicine planar imaging using a gamma camera. Before the SPECT scan, the patient is injected with a radiolabeled chemical emitting gamma rays that can be detected by the scanner. A computer collects the information from the gamma camera and translates this into two-dimensional cross-sections. These cross-sections can be added back together to form a three-dimensional image of an organ or a tissue. SPECT involves detection of gamma rays emitted singly, and sequentially, by the radionuclide provided by the radiolabeled chemical. To acquire SPECT images, the gamma camera is rotated around the patient. Projections are acquired at defined points during the rotation, typically every 3 - 6 degrees. In most cases, a full 360 degree rotation is used to obtain an optimal reconstruction. The time taken to obtain each projection is also variable, but 15 - 20 seconds is typical. This gives a total scan time of 15 - 20 minutes. Multi-headed gamma cameras are faster. Since SPECT acquisition is very similar to planar gamma camera imaging, the same radiopharmaceuticals may be used.

Positron Emitting Tomography (PET) is a non-invasive, diagnostic imaging technique for measuring the biochemical status or metabolic activity of cells within the human body. PET is unique since it produces images of the body's basic biochemistry or functions. Traditional diagnostic techniques, such as X-rays, CT scans, or MRI, produce images of the body's anatomy or structure. The premise with these techniques is that any changes in structure or anatomy associated with a disease can be seen. Biochemical processes are also altered by a disease, and may occur before any gross changes in anatomy. PET is an imaging technique that can visualize some of these early biochemical changes. PET scanners rely on radiation emitted from the patient to create the images. Each patient is given a minute amount of a radioactive pharmaceutical that either closely resembles a natural substance used by the body or binds specifically to a receptor or molecular structure. As the radioisotope undergoes positron emission decay (also known as positive beta decay), it emits a positron, the antiparticle counterpart of an electron. After traveling up to a few millimeters, the positron encounters an electron and annihilates, producing a pair of annihilation (gamma) photons moving in opposite directions. These are detected when they reach a scintillation material in the scanning device, creating a burst of light, which is detected by photomultiplier tubes or silicon avalanche photodiodes. The technique depends on simultaneous or coincident detection of the pair of photons. Photons that do not arrive in pairs, i.e., within a few nanoseconds, are ignored. All coincidences are forwarded to the image processing unit where the final image data is produced using image reconstruction procedures.

SPECT/CT and PET/CT is the combination of SPECT and PET with computed tomography (CT). The key benefits of combining these modalities are improving the reader's confidence and accuracy. With traditional PET and SPECT, the limited number of photons emitted from the area of abnormality produces a very low-level background that makes it difficult to anatomically localize the area. Adding CT helps determine the location of the abnormal area from an anatomic perspective and categorize the likelihood that this represents a disease.

It is within the present inventions that the method for the diagnosis of a disease of the invention may realize each and any of the above strategies which are as such known in the art, and which insofar constitute further embodiments of the invention.

Compounds of the present invention are useful to stratify patients, i.e., to create subsets within a patient population that provide more detailed information about how the patient will respond to a given drug. Stratification can be a critical component to transforming a clinical trial from a negative or neutral outcome to one with a positive outcome by identifying the subset of the population most likely to respond to a novel therapy.

Stratification includes the identification of a group of patients with shared "biological" characteristics to select the optimal management for the patients and achieve the best possible outcome in terms of risk assessment, risk prevention and achievement of the optimal treatment outcome.

A compound of the present invention may be used to assess or detect, a specific disease as early as possible (which is a diagnostic use), the risk of developing a disease (which is a susceptibility/risk use), the evolution of a disease including indolent vs. aggressive (which is a prognostic use) and it may be used to predict the response and the toxicity to a given treatment (which is a predictive use).

It is also within the present invention that the compound of the invention is used in a theragnostic method. The concept of theragnostics is to combine a therapeutic agent with a corresponding diagnostic test that can increase the clinical use of the therapeutic drug. The concept of theragnostics is becoming increasingly attractive and is widely considered the key to improving the efficiency of drug treatment by helping doctors identify patients who might profit from a given therapy and hence avoid unnecessary treatments.

The concept of theragnostics is to combine a therapeutic agent with a diagnostic test that allows doctors to identify those patients who will benefit most from a given therapy. In an embodiment and as preferably used herein, a compound of the present invention is used for the diagnosis of a patient, i.e. identification and localization of the primary tumor mass as well as potential local and distant metastases. Furthermore, the tumor volume can be determined, especially utilizing three-dimensional diagnostic modalities such as SPECT or PET. Only those patients having GIPR-positive tumor masses and who, therefore, might profit from a given therapy are selected for a particular therapy and hence unnecessary treatments are avoided. Preferably, such therapy is a GIPR-targeted therapy using a compound of the present invention. In one particular embodiment, chemically identical tumor-targeted diagnostics, preferably imaging diagnostics for scintigraphy, PET or SPECT and radiotherapeutics are applied. Such compounds only differ in the radionuclide and therefore usually have a very similar if not identical pharmacokinetic profile. This can be realized using a chelator and a diagnostic or therapeutic radiometal. Alternatively, this can be realized using a precursor for radiolabeling and radiolabeling with either a diagnostic or a therapeutic radionuclide. In one embodiment diagnostic imaging is used preferably by means of quantification of the radiation of the diagnostic radionuclide and subsequent dosimetry which is known to those skilled in the art and the prediction of drug concentrations in the tumor compared to vulnerable side effect organs. Thus, a truly individualized drug dosing therapy for the patient is achieved.

In an embodiment and as preferably used herein, the theragnostic method is realized with only one theragnostically active compound such as a compound of the present invention labeled with a radionuclide emitting diagnostically detectable radiation (e.g. positrons or gamma rays) as well as therapeutically effective radiation (e.g. electrons or alpha particles).

The invention also contemplates a method of intraoperatively identifying/disclosing diseased tissues expressing GIPR in a subject. Such method uses a compound of the invention, whereby such compound of the invention preferably comprises as Effector a diagnostically active agent.

According to a further embodiment of the invention, the compound of the invention, particularly if complexed with a radionuclide, may be employed as adjunct or adjuvant to any other tumor treatment including, surgery as the primary method of treatment of most isolated solid cancers, radiation therapy involving the use of ionizing radiation in an attempt to either cure or improve the symptoms of cancer using either sealed internal sources in the form of brachytherapy or external sources, chemotherapy such as alkylating agents, antimetabolites, anthracyclines, plant alkaloids, topoisomerase inhibitors, and other antitumor agents, hormone treatments that modulate tumor cell behavior without directly attacking those cells, targeted agents which directly target a molecular abnormality in certain types of cancer including monoclonal antibodies and tyrosine kinase inhibitors, angiogenesis inhibitors, immunotherapy, cancer vaccination, palliative care including actions to reduce the physical, emotional, spiritual, and psycho-social distress to improve the patient's quality of life and alternative treatments including a diverse group of health care systems, practices, and products that are not part of conventional medicine.

In an embodiment of the methods of the invention, the subject is a patient. In an embodiment, a patient is a subject which has been diagnosed as suffering from or which is suspected of suffering from which or which is at risk of suffering from or developing a disease, whereby the disease is a disease as described herein and preferably a disease involving the GIPR.

Dosages employed in practicing the methods for treatment and diagnosis, respectively, where a radionuclide is used and more specifically attached to or part of the compound of the invention will vary depending e.g., on the particular condition to be treated, for example the known radiosensitivity of the tumor type, the volume of the tumor and the therapy desired. In general, the dose is calculated on the basis of radioactivity distribution to each organ and on observed target uptake. A γ-emitting complex may be administered once or at several times for diagnostic imaging. In animals, an indicated dose range may be from 0.1 µg/kg to 5 mg/kg of the compound of the invention complexed e.g., with 1 to 200 MBq of ¹¹¹In or ⁸⁹Zr. A β-emitting complex of the compound of the invention may be administered at several time points e.g., over a period of 1 to 3 weeks or longer. In animals, an indicated dosage range may be of from 0.1 µg/kg to 5 mg/kg of the compound of the invention complexed e.g., with 1 to 200 MBq ⁹⁰Y or ¹⁷⁷Lu. In larger mammals, for example humans, an indicated dosage range is from 0.1 to 100 µg/kg of the compound of the invention complexed with e.g., 10 to 400 MBq ¹¹¹In or ⁸⁹Zr. In larger mammals, for example humans, an indicated dosage range is of from 0.1 to 100 µg/kg of the compound of the invention complexed with e.g., 10 to 5000 MBq ⁹⁰Y or ¹⁷⁷Lu.

In a further aspect, the instant invention is related to a composition and a pharmaceutical composition in particular, comprising the compound of the invention.

The pharmaceutical composition of the present invention comprises at least one compound of the invention and, optionally, one or more carrier substances, excipients and/or adjuvants. The pharmaceutical composition may additionally comprise, for example, one or more of water, buffers such as, e.g., neutral buffered saline or phosphate buffered saline, ethanol, mineral oil, vegetable oil, dimethylsulfoxide, carbohydrates such as e.g., glucose, mannose, sucrose or dextrans, mannitol, proteins, adjuvants, polypeptides or amino acids such as glycine, antioxidants, chelating agents such as EDTA or glutathione and/or preservatives. Furthermore, one or more other active ingredients may, but need not, be included in the pharmaceutical composition of the invention.

The pharmaceutical composition of the invention may be formulated for any appropriate route of administration, including, for example, topical such as, e.g., transdermal or ocular, oral, buccal, nasal, vaginal, rectal or parenteral administration. The term parenteral as used herein includes subcutaneous, intradermal, intravascular such as, e.g., intravenous, intramuscular, intrathecal and intraperitoneal injection, as well as any similar injection or infusion technique. A preferred route of administration is intravenous administration.

In an embodiment of the invention the compound of the invention comprising a radionuclide is administered by any conventional route, in particular intravenously, e.g., in the form of injectable solutions or suspensions. The compound of the invention may also be administered advantageously by infusion, e.g., by an infusion of 30 to 60 min.

Depending on the site of the tumor, the compound of the invention may be administered as close as possible to the tumor site, e.g., by means of a catheter. Such administration may be carried out directly into the tumor tissue or into the surrounding tissue or into the afferent blood vessels. The compound of the invention may also be administered repeatedly in doses, preferably in divided doses.

According to a preferred embodiment of the invention, a pharmaceutical composition of the invention comprises a stabilizer, e.g., a free radical scavenger, which inhibits autoradiolysis of the compound of the invention. Suitable stabilizers include, e.g., serum albumin, ascorbic acid, retinol, gentisic acid or a derivative thereof, or an amino acid infusion solution such, e.g., used for parenteral protein feeding, preferably free from electrolyte and glucose, for example a commercially available amino acid infusion such as Proteinsteril^{®} KE Nephro. Ascorbic acid and gentisic acid are preferred.

A pharmaceutical composition of the invention may comprise further additives, e.g., an agent to adjust the pH between 7.2 and 7.4, e.g., sodium or ammonium acetate or Na2HPO4. Preferably, the stabilizer is added to the non-radioactive compound of the invention and introduction of the radionuclide, for instance the complexation with the radionuclide, is performed in the presence of the stabilizer, either at room temperature or, preferably, at a temperature of from 40 to 120° C. The complexation may conveniently be performed under air free conditions, e.g., under N2 or Ar. Further stabilizer may be added to the composition after complexation.

Excretion of the compound of the invention, particularly if the Effector is a radionuclide, essentially takes place through the kidneys. Further protection of the kidneys from radioactivity accumulation may be achieved by administration of lysine or arginine or an amino acid solution having a high content of lysine and/or arginine, e.g., a commercially available amino acid solution such as Synthamin^{®}-14 or -10, prior to the injection of or together with the compound of the invention, particularly if the Effector is a radionuclide. Protection of the kidneys may also be achieved by administration of plasma expanders such as e.g., gelofusine, either instead of or in addition to amino acid infusion. Protection of the kidneys may also be achieved by administration of diuretics providing a means of forced diuresis which elevates the rate of urination. Such diuretics include high ceiling loop diuretics, thiazides, carbonic anhydrase inhibitors, potassium-sparing diuretics, calcium-sparing diuretics, osmotic diuretics and low ceiling diuretics. A pharmaceutical composition of the invention may contain, apart from a compound of the invention, at least one of these further compounds intended for or suitable for kidney protection, preferably kidney protection of the subject to which the compound of the invention is administered.

It will be understood by a person skilled in the art that the compound of the invention is disclosed herein for use in various methods. It will be further understood by a person skilled in the art that the composition of the invention and the pharmaceutical composition of the invention can be equally used in said various methods. It will also be understood by a person skilled in the art that the composition of the invention and the pharmaceutical composition are disclosed herein for use in various methods. It will be equally understood by a person skilled in the art that the compound of the invention can be equally used in said various methods.

It will be acknowledged by a person skilled in the art that the composition of the invention and the pharmaceutical composition of the invention contain one or more further compounds in addition to the compound of the invention. To the extent that such one or more further compounds are disclosed herein as being part of the composition of the invention and/or of the pharmaceutical composition of the invention, it will be understood that such one or more further compounds can be administered separately from the compound of the invention to the subject which is exposed to or the subject of a method of the invention. Such administration of the one or more further compounds can be performed prior, concurrently with or after the administration of the compound of the invention. It will also be acknowledged by a person skilled in the art that in a method of the invention, apart from a compound of the invention, one or more further compound may be administered to a subject. Such administration of the one or more further compounds can be performed prior, concurrently with or after the administration of the compound of the invention. To the extent that such one or more further compounds are disclosed herein as being administered as part of a method of the invention, it will be understood that such one or more further compounds are part of a composition of the invention and/or of a pharmaceutical composition of the invention. It is within the present invention that the compound of the invention and the one or more further compounds may be contained in the same or a different formulation. It is also within the present invention that the compound of the invention and the one or more further compounds are not contained in the same formulation, but are contained in the same package containing a first formulation comprising a compound of the invention, and a second formulation comprising the one or more further compounds, whereby the type of formulation may be the same or may be different.

It is within the present invention that more than one type of a compound of the invention is contained in the composition of the invention and/or the pharmaceutical composition of the invention. It is also within the present invention that more than one type of a compound of the invention is used, preferably administered, in a method of the invention.

It will be acknowledged that a composition of the invention and a pharmaceutical composition of the invention may be manufactured in conventional manner.

Radiopharmaceuticals have decreasing content of radioactivity with time, as a consequence of the radioactive decay. The physical half-life of the radionuclide is often short for radiopharmaceutical diagnostics. In these cases, the final preparation has to be done shortly before administration to the patient. This is in particular the case for positron emitting radiopharmaceuticals for tomography (PET radiopharmaceuticals). It often leads to the use of semi-manufactured products such as radionuclide generators, radioactive precursors and kits.

Preferably, a kit of the invention comprises apart from one or more than one compounds of the invention typically at least one of the followings: instructions for use, final preparation and/or quality control, one or more optional excipient(s), one or more optional reagents for the labeling procedure, optionally one or more radionuclide(s) with or without shielded containers, and optionally one or more device(s), whereby the device(s) is/are selected from the group comprising a labeling device, a purification device, an analytical device, a handling device, a radioprotection device or an administration device.

Shielded containers known as "pigs" for general handling and transport of radiopharmaceutical containers come in various configurations for holding radiopharmaceutical containers such as bottles, vials, syringes, etc. One form often includes a removable cover that allows access to the held radiopharmaceutical container. When the pig cover is in place, the radiation exposure is acceptable.

A labeling device is selected from the group of open reactors, closed reactors, microfluidic systems, nanoreactors, cartridges, pressure vessels, vials, temperature controllable reactors, mixing or shaking reactors and combinations thereof.

A purification device is preferably selected from the group of ion exchange chromatography columns or devices, size-exclusion chromatography columns or devices, affinity chromatography columns or devices, gas or liquid chromatography columns or devices, solid phase extraction columns or devices, filtering devices, centrifugations vials columns or devices.

An analytical device is preferably selected from the group of tests or test devices to determine the identity, radiochemical purity, radionuclidic purity, content of radioactivity and specific radioactivity of the radiolabelled compound.

A handling device is preferably selected from the group consisting of devices for mixing, diluting, dispensing, labeling, injecting and administering radiopharmaceuticals to a subject.

A radioprotection device is used in order to protect doctors and other personnel from radiation when using therapeutic or diagnostic radionuclides. The radioprotection device is preferably selected from the group consisting of devices with protective barriers of radiation-absorbing material selected from the group consisting of aluminium, plastics, wood, lead, iron, lead glass, water, rubber, plastic, cloth, devices ensuring adequate distances from the radiation sources, devices reducing exposure time to the radionuclide, devices restricting inhalation, ingestion, or other modes of entry of radioactive material into the body and devices providing combinations of these measures.

An administration device is preferably selected from the group of syringes, shielded syringes, needles, pumps, and infusion devices. Syringe shields are commonly hollow cylindrical structures that accommodate the cylindrical body of the syringe and are constructed of lead or tungsten with a lead glass window that allows the handler to view the syringe plunger and liquid volume within the syringe.

It is within the presen invention that the term "in an embodiment" or "in an embodiment of the invention" refers to each and any aspect of the invention, including any embodiment thereof.

The present invention is now further illustrated by reference to the following figures and examples from which further features, embodiments and advantages, may be taken, wherein
- Fig. 1: shows the amino acid sequence of human GIPR (SEQ ID NO: 1); and
- Fig. 2: shows a scheme illustrating the topology of a bio-distribution modifier comprised by the N-terminal modification group A (Fig. 2A) and a bio-distribution modifier comprised by the C-terminal group C-term (Fig. 2B); in case of the bio-distribution modifier comprised by the N-terminal modification group, the bio-distribution modifier comprises a carboxylic acid to which a linking chemical moiety is connected which links one or two first level alkyl groups which bear primary hydroxyl groups or to which second level alkyl groups bearing primary hydroxyl groups are connected; and in case of the bio-distribution modifier comprised by the C-terminal group C-term, the bio-distribution modifier comprises an amino acid with a side chain to which a linking chemical moiety is connected which links one or two first level alkyl groups which bear primary hydroxyl groups or to which second level alkyl groups bearing primary hydroxyl groups are connected.

The following Examples have been included to provide guidance to one of ordinary skill in the art for practicing representative embodiments of the presently disclosed subject matter. In light of the present disclosure and the general level of skill in the art, those of skill can appreciate that the following examples are intended to be exemplary only and that numerous changes, modifications, and alterations can be employed without departing from the scope of the presently disclosed subject matter. The synthetic descriptions and specific examples that follow are only intended for the purposes of illustration, and are not to be construed as limiting in any manner to make compounds of the disclosure by other methods.

### EXAMPLES

Abbreviations used in the instant application and the following examples in particular are as follows:
- °C: means degree celsius
- ¹¹¹InCl₃: means Indium-111 chloride
- ¹⁷⁷LuCl₃: means Lutetium-177 chloride
- 4PL: means four parameter logistic
- ACN: means acetonitrile
- ADCC: means antibody-dependent cellular cytotoxicity
- Alloc: means allyloxycarbonyl
- amu: means atomic mass unit
- aq.: means aqueous
- AUC: means area under the curve
- Bio: means D-Biotin
- BSA: means bovine serum albumin
- Calc: means Calculated
- cAMP: means cyclic adenosine monophosphate
- CAR-T: means chimeric antigen receptor T
- cDNA: means complementary DNA
- CHO: means chinese hamster ovarian
- CM: means ChemmatrixTM
- CMV: means cytomegalovirus
- CO₂: means carbon dioxide
- CT: means computed tomography
- Da: means Dalton
- DAD: means diode array detector
- DCM: means dichloromethane
- Dde: means N-(1-(4,4-dimethyl-2,6-dioxocyclohexylidene)ethyl)
- DEG: means di(ethylene glycol)
- DIC: means N,N'-Diisopropylcarbodiimide
- DICOM: means digital Imaging and Communications in Medicine
- DIPEA: means N,N-Diisopropylethylamine
- DMEM: means Dulbecco's Modified Eagle Medium
- DMF: means dimethylformamide
- DMSO: means dimethyl sulfoxide
- DOTA: means dodecane tetraacetic acid - 2,2*'*,2*"*,2‴-(1,4,7,10-Tetraazacyclododecane-1,4,7,10-tetrayl)tetraacetic acid
- DOTA(OtBu)₃-OH: means DOTA-tris(tert-butyl ester), Tri-tert-butyl 1,4,7,10-tetraazacyclododecane-1,4,7,10-tetraacetate
- DOTA-NHS: means DOTA N-hydroxysuccinimide ester
- DTPA: means Diethylenetriamine pentaacetate
- DTT: means Dithiothreitol
- e.g.: means for example (exempli gratia)
- EC₅₀: means half-maximal excitatory concentration
- EDT: means 1,2-ethanedithiol
- EDTA: means ethylenediaminetetraacetic acid
- ELSD: means Evaporative Light Scattering Detector
- eq.: means equivalent
- ESI: means electrospray ionization
- Et2O: means diethylether
- EtOAc: means ethylacetate
- FACS: means fluorescence-activated cell sorting
- FCS: means fetal calf serum
- Fmoc: means 9-Fluorenyloxycarbonyl
- GBq: means gigabecquerel
- GHRH: means Growth hormone-releasing hormone
- GIP: means glucose-dependent insulinotropic polypeptide
- GIPR: means glucose-dependent insulinotropic polypeptide receptor
- GLP-1: means Glucagon-like peptide-1
- GLP-2: means Glucagon-like peptide-2
- G-PCR: means G-protein coupled receptor
- h: means hour(s)
- H₂O: means water
- HATU: means Hexafluorophosphate Azabenzotriazole Tetramethyl Uronium-1-[Bis(dimethylamino)methylene]-1H-1,2,3-triazolo[4,5-b]pyridinium 3-oxide hexafluorophosphate
- HCl: means Hydrogen chloride
- HEK: means Human embryonic kindey
- HEPES: means 4-(2-hydroxyethyl)-1-piperazineethanesulfonic acid
- HFIP: means hexafluoro isopropanol
- HOAc: means acetic acid
- HPLC: means high performance liquid chromatography
- i.e.: means that is (id est)
- IC50: means half-maximal inhibitory concentration
- ID: means injected dose
- ID/g: means injected dose per gram
- IDBS: means ID Business Solutions
- K_{D}: means dissociation constant
- kDa: means 1000 Dalton or Kilo
- kDa: means kilo Dalton
- Kᵢ: means inhibitory constant
- k_{off}: means dissociation rate
- kₒₙ: means association rate
- kVp: means peak kilovoltage
- LC: means Liquid chromatography
- LC/TOF-MS: means liquid chromatography time-of-flight mass spectrometry
- LC-MS: means Liquid chromatography mass spectrometry
- M: means molar or mol per Liter
- m/z: means mass divided by charge
- MAPK: means mitogen-activated protein kinase
- max.: means maximum
- MBq: means megabecquerel
- MeCN: means acetonetrile
- MeOH: means methanol
- MeV: means mega electron volt
- MFI: means Median flourescence intensities
- min: means minute(s)
- MTBE: means methyl-tert-butyl ether
- Mtt: means 4-Methyltrityl
- MW: means molecular weight
- n.d.: means not determined
- N3Tap: means L-(4R)-Azidoproline
- NaCl: means sodium chloride
- NaHCO₃: means sodium hydrogencarbonate
- NEP: means neutral endopeptidase
- NET: means neuroendocrine tumor
- NHS: means N-hydroxysuccinimide
- NMP: means N-Methyl-2-pyrrolidone
- NMR: means Nuclear Magnetic Resonance
- OtBu: means tert Butyl ester as protecting group
- p.a.: means for analytical purpose (quality grade)
- PARP: means Poly(ADP-ribose) polymerases
- PBS: means phosphate buffered saline
- PBST: means phosphate buffered saline containing Tween
- pEC₅₀: means the negative log of the EC50 value when converted to molar
- PET: means positron emission tomography
- pH: means potential of hydrogen
- ₚIC₅₀: means the negative log of the IC50 value when converted to molar
- pK_{D}: means negative decadic logarithm of dissociation constant
- ppm: means parts per million
- prep.: means preparative
- PRRT: means peptide receptor radionuclide therapy
- PS: means Polystyrene
- Q-TOF: means quadrupole time-of-flight
- RFU: means relative fluorescence unit
- RLU: means relative luminescence values
- RP: means reversed phase
- RPM: means rounds per minute
- RPMI: means Roswell Park Memorial Institute
- RT: means room temperature
- Rₜ: means retention time
- RU: means resonance units
- SCID: means severe combined immunodeficiency
- SCK: means Single cycle kinetic
- sec: means second
- SPECT: means single photon emission computed tomography
- SPPS: means solid phase peptide synthesis
- SPR: means surface plasmon resonance
- t_{1/2}: means Dissociation half-life, equals ln 2/k_{off} for 1^{st} order dissociation process
- TBDMS: means tert Butyl-dimethyl-silyl
- tBu: means tert Butyl ether as protecting group
- tBuO: means tert Butyl ester as protecting group
- TFA: means trifluoroacetate or trifluoroacetic acid
- TG: means tentagel
- THF: means tetrahydrofurane
- TIPS: means triisopropyl silane
- TLC: means thin layer chromatography
- TOF-MS: means tof of flight mass spectrometry
- UHPLC: means ultrahigh performance liquid chromatography
- UV: means ultraviolet
- VGT: means vertical gene transfer

### Example 1: Material and Methods

The materials and methods as well as general methods are further illustrated by the following examples.

### Solvents:

Solvents are used in the specified quality without further purification. Acetonitrile (Super Gradient, HPLC, VWR - for analytical purposes; PrepSolv, Merck - for preparative purposes); dichloromethane (synthesis, Roth); ethyl acetate (synthesis grade, Roth); N,N-dimethylformamide (peptide synthesis grade, Biosolve); 1-methyl-2-pyrolidone (peptide grade, IRIS BioTech) 1,4-dioxane (reinst, Roth); methanol (p. a., Merck).

Water: Milli-Q Plus, Millipore, demineralized.

### Chemicals:

Chemicals are synthesized according to or in analogy to literature procedures or purchased from Sigma-Aldrich-Merck (Deisenhofen, Germany), Bachem (Bubendorf, Switzerland), VWR (Darmstadt, Germany), Novabiochem (Merck Group, Darmstadt, Germany), Acros Organics (distribution company Fisher Scientific GmbH, Schwerte, Germany), Iris Biotech (Marktredwitz, Germany), Amatek Chemical (Jiangsu, China), Roth (Karlsruhe, Deutschland), Molecular Devices (Chicago, USA), Biochrom (Berlin, Germany), Peptech (Cambridge, MA, USA), Synthetech (Albany, OR, USA), Pharmacore (High Point, NC, USA), PCAS Biomatrix Inc (Saint-Jean-sur-Richelieu, Quebec, Canada), Alfa Aesar (Karlsruhe, Germany), Tianjin Nankai Hecheng S&T Co., Ltd (Tianjin, China), CheMatech (Dijon, France), JenKem Technology (Plano, TX, USA), Trimen Chemicals (Lodz, Poland), BLD Pharmatech GmbH and Anaspec (San Jose, CA, USA) or other companies and used in the assigned quality without further purification.

### 1,3-bis((2,2-dimethyl-1,3-dioxan-5-yl)oxy)propan-2-one (Isopropylidene-HAC2:HAC-one)

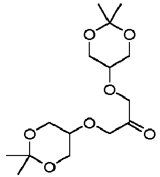

is synthesized as follows:
To a solution of 2,2-dimethyl-1,3-dioxan-5-ol (0.52 g, 4.0 mmol) in DMF (10 mL) a suspension of NaH (60% in mineral oil, 0.24 g, 6.0 mmol) in THF (2 mL) is added at 0 °C and the mixture stirred for 20 minutes. Then 1,3-dibromoacetone (0.46 g, 2.0 mmol) is added and the mixture allowed to warm to 10 °C within 3 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₅H₂₆O₇ (MW = 318.363).

### 1,3 -bis((2,2-dimethyl-1,3 -dioxan-5 -yl)methoxy)propan-2-one (Isopropylidene-HPD2:HAC-one)

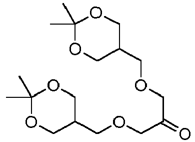

is synthesized as follows:

To a solution of (2,2-dimethyl-1,3-dioxan-5-yl)methanol (0.59 g, 4.0 mmol) in DMF (10 mL) a suspension of NaH (60% in mineral oil, 0.24 g, 6.0 mmol) in THF (2 mL) is added at 0 °C and the mixture stirred for 20 minutes. Then 1,3-dibromoacetone (0.46 g, 2.0 mmol) is added and the mixture allowed to warm to 10 °C within 3 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₇H₃₀O₇ (MW = 346.417).

### 3-bromo-2-(bromomethyl)-N-methoxy-N-methylpropanamide

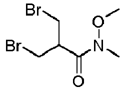

is synthesized as follows:
To a solution of 3-bromo-2-(bromomethyl)propanoyl chloride (0.93 g, 3.5 mmol) and N,O-dimethylhydroxylamine hydrochloride (0.34 g, 3.5 mmol) in DCM (10mL) at 0 °C, DIPEA (1.17 mL, 7 mmol) is added. The mixture is allowed to warm to room temperature and then stirred for 2 hours. The organic phase is successively washed with 0.1 N HCl (2x 20 mL) and saturated sodium chloride solution (2x 20 mL), dried over anhydrous Na₂SO₄, and filtered. The filtrate is concentrated under reduced pressure. The crude material is used in the following steps without further purification.

### 3-((2,2-dimethyl-1,3-dioxan-5-yl)oxy)-2-(((2,2-dimethyl-1,3-dioxan-5-yl)oxy)methyl)-N-methoxy-N-methylpropanamide

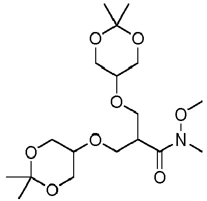

is synthesized as follows:
To a solution of 2,2-dimethyl-1,3-dioxan-5-ol (0.52 g, 4.0 mmol) in DMF (10 mL) a suspension of NaH (60% in mineral oil, 0.24 g, 6.0 mmol) in THF (2 mL) is added at 0 °C and the mixture stirred for 20 minutes. Then 3-bromo-2-(bromomethyl)-N-methoxy-N-methylpropanamide (0.58 g, 2 mmol) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₈H₃₃NO₈ (MW = 391.457).

### 3-((2,2-dimethyl-1,3-dioxan-5-yl)oxy)-2-(((2,2-dimethyl-1,3-dioxan-5-yl)oxy)methyl)propanal (Isopropylidene-HAC2:HPD-aldehyde)

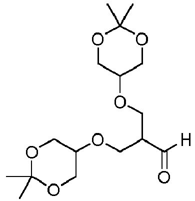

is synthesized as follows:
3-((2,2-dimethyl-1,3-dioxan-5-yl)oxy)-2-(((2,2-dimethyl-1,3-dioxan-5-yl)oxy)methyl)-*N-*methoxy-*N-*methylpropanamide (0.22 g, 0.56 mmol) is dissolved in THF (5 mL, dry) in an inert atmosphere. The solution is cooled to -15 °C and the solution of diisobutylaluminiumhydride in THF (1M, 1 mL, 1 mmol) is added dropwise. The solution is allowed to warm to room temperature and subsequently stirred for 4 hours. Then the solution is cooled to 0 °C and water added dropwise. After dissolution of the precipitate by addition of 0.1 N HCl the mixture is extracted with ethyl acetate (10 mL, 2x). The combined organic layers are washed with saturated sodium chloride solution (2x 20 mL). After phase separation the organic phase is dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₆H₂₈O₇ (MW = 332.390).

### 3-((2,2-dimethyl-1,3-dioxan-5-yl)methoxy)-2-(((2,2-dimethyl-1,3-dioxan-5-yl)methoxy)methyl)-N-methoxy-N-methylpropanamide

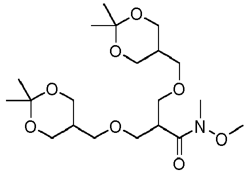

is synthesized as follows:
To a solution of (2,2-dimethyl-1,3-dioxan-5-yl)methanol (0.59 g, 4.0 mmol) in DMF (10 mL) a suspension of NaH (60% in mineral oil, 0.24 g, 6.0 mmol) in THF (2 mL) is added at 0 °C and the mixture stirred for 20 minutes. Then 3-bromo-2-(bromomethyl)-N-methoxy-N-methylpropanamide (0.58 g, 2 mmol) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₀H₃₇NO₈ (MW = 419.510).

### 3-((2,2-dimethyl-1,3-dioxan-5-yl)methoxy)-2-(((2,2-dimethyl-1,3-dioxan-5-yl)methoxy)methyl)propanal (Isopropylidene-HPD2:HPD-aldehyde)

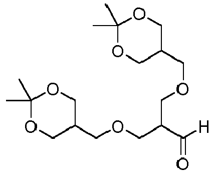

is synthesized as follows:
3-((2,2-dimethyl-1,3-dioxan-5-yl)methoxy)-2-(((2,2-dimethyl-1,3-dioxan-5-yl)methoxy)methyl)-*N-*methoxy-*N*-methylpropanamide (0.64 g, 1.5 mmol) is dissolved in THF (10 mL, dry) in an inert atmosphere. The solution is cooled to -15 °C and the solution of diisobutylaluminiumhydride in THF (1M, 2 mL, 2 mmol) is added dropwise. The solution is allowed to warm to room temperature and subsequently stirred for 4 hours. Then the solution is cooled to 0 °C and water added dropwise. After dissolution of the precipitate by addition of 0.1 N HCl the mixture is extracted with ethyl acetate (10 mL, 2x). The combined organic layers are washed with saturated sodium chloride solution (2x 20 mL). After phase separation the organic phase is dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₈H₃₂O₇ (MW = 360.443).

### 3 -(tert-butoxy)-2_{.}2-bis(tert-butoxymethyl)propanoic acid

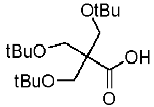

is synthesized as follows:
Ethyl 3-hydroxy-2,2-bis(hydroxymethyl)propanoate (1 g, 5.6 mmol) is filled into a 250 mL Schott bottle, which is topped with a septum and closed with a Schott screw cap with a hole. The bottle is place into liquid nitrogen and gaseous isobutylene is introduced via a cannula and condensed into bottle. Sulfuric acid (0.2 mL) is added to the frozen mixture. The bottle is allowed to warm to 0 °C and the mixture stirred for 4 hours. Then a potassium hydroxid solution (2 M, 10 mL) and subsequently methanol (10 mL) is added to the mixture. The excess isobutylene is carefully allowed to evaporate and the solution stirred overnight. After the mixture is neutralized by addition of 0.1 N HCl, the volume reduced to a minimum and then extracted with DCM (2x 20 mL). The combined organic layers are washed with saturated sodium chloride solution (2x 20 mL). After phase separation the organic phase is dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₇H₃₄O₅ (MW = 318.450).

### 3-(tert-butoxy)-2,2-bis(tert-butoxymethyl)-N-methoxy-N-methylpropanamide

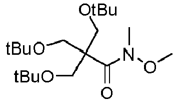

is synthesized as follows:
To the solution of 3-(tert-butoxy)-2,2-bis(tert-butoxymethyl)propanoic acid (1 g, 3.14 mmol) and DIPEA (1.56 mL, 9.43 mmol) in DMF (5 mL), HATU (1.2 g, 3.14 mmol) is added. The solution is left to stir for 5 min, before addition of *N*,*O*-dimethylhydroxylamine hydrochloride (0.3 g, 3.14 mmol). After 1 hour the mixture is diluted with water (5 mL) and then extracted with ethyl acetate (10 mL, 2x). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₉H₃₉NO₅ (MW = 361.517).

### 3-(tert-butoxy)-2,2-bis(tert-butoxymethyl)propanal (tBu3-PEO-aldehyde)

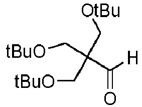

is synthesized as follows:
3-(tert-butoxy)-2,2-bis(tert-butoxymethyl)-N-methoxy-N-methylpropanamide (1 g, 2.77 mmol) is dissolved in THF (20 mL, dry) in an inert atmosphere. The solution is cooled to -15 °C and the solution of diisobutylaluminiumhydride in THF (1 M, 3.5 mL, 3.5 mmol) is added dropwise. The solution is allowed to warm to room temperature and subsequently stirred for 4 hours. Then the solution is cooled to 0 °C and water added dropwise. After dissolution of the precipitate by addition of 0.1 N HCl the mixture is extracted with ethyl acetate (10 mL, 2x). The combined organic layers are washed with saturated sodium chloride solution (2x 20 mL). After phase separation the organic phase is dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₇H₃₄O₄ (MW = 302.450).

### 2,2-dimethyl-1,3-dioxan-5-yl 4-methylbenzenesulfonate

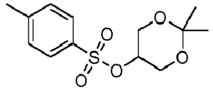

is synthesized as follows:
2,2-Dimethyl-1,3-dioxan-5-ol (200 mg, 1.51 mmol) is dissolved in DCM (30 mL). To the solution p-toluenesulfonyl chloride (420 mg, 2.20 mmol) and triethylamine (420 µL, 2.97 mmol) are added. The reaction solution is stirred for 3 hours and then successively washed with 0.1 N HCl (2x 20 mL) and saturated sodium chloride solution (2x 20 mL). After phase separation the organic phase is dried over anhydrous Na₂SO₄, and filtered. The filtrate is concentrated under reduced pressure and the residue purified by silica gel column chromatography with DCM/MeOH (99:1). ¹H NMR confirms identity of the target compound. C₁₃H₁₈O₅S (MW = 286.346).

### (2,2-dimethyl-1,3-dioxan-5-yl)methyl4-methylbenzenesulfonate

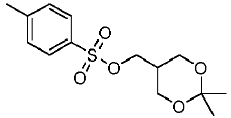

is synthesized as follows:
The solution of (2,2-dimethyl-1,3-dioxan-5-yl)methanol (1.5 g, 10.3 mmol) and triethylamine (1.71 mL, 12.3 mmol) in DCM (15 mL) is cooled to 0 °C. Then p-toluenesulfonyl chloride (2.15 g, 11.3 mmol) is added. After allowing the solution to warm to room temperature, stirring is continued for 1h. The solution is then diluted with DCM (100 mL) and successively washed with 0.1 N HCl (2x 20 mL) and saturated sodium chloride solution (2x 20 mL). After phase separation the organic phase is dried over anhydrous Na₂SO₄, and filtered. The filtrate is concentrated under reduced pressure and the residue purified by silica gel column chromatography with DCM/MeOH (99:1). ¹H NMR confirms identity of the target compound. C₁₄H₂₀O₅S (MW = 300.372).

### 5,5'-((2-((benzyloxy)methyl)propane-1,3-diyl)bis(oxy))bis(2,2-dimethyl-1,3-dioxane)

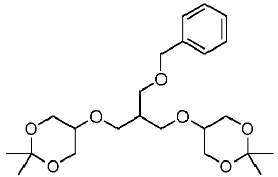

is synthesized as follows:
To the solution of 2-((benzyloxy)methyl)propane-1,3-diol (1 g, 5.1 mmol) in DMF (10 mL) a suspension of NaH (60% in mineral oil, 0.48 g, 12.0 mmol) in THF (4 mL) is added at 0 °C and the mixture stirred for 20 minutes. Then the solution of 2,2-dimethyl-1,3-dioxan-5-yl 4-methylbenzenesulfonate (2.9 g, 10.2 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₃H₃₆O₇ (MW = 424.534)

### 5,5'-(((2-((benzyloxy)methyl)propane-1,3-diyl)bis(oxy))bis(methylene))bis(2,2-dimethyl-1,3-dioxane)

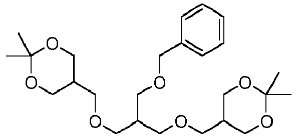

is synthesized as follows:
To the solution of 2-((benzyloxy)methyl)propane-1,3-diol (1 g, 5.1 mmol) in DMF (10 mL) a suspension of NaH (60% in mineral oil, 0.48 g, 12.0 mmol) in THF (4 mL) is added at 0 °C and the mixture stirred for 20 minutes. Then the solution of (2,2-dimethyl-1,3-dioxan-5-yl)methyl 4-methylbenzenesulfonate (3.1 g, 10.2 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₄H₂₀O₅S (MW = 300.369).

### 5,5'-((2-(benzyloxy)propane-1,3-diyl)bis(oxy))bis(2,2-dimethyl-1,3-dioxane)

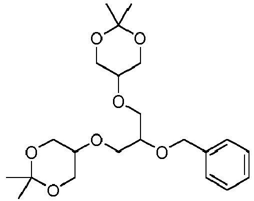

is synthesized as follows:
To the solution of 2-(benzyloxy)propane-1,3-diol (1g, 5.5 mmol) in DMF (10 mL) a suspension of NaH (60% in mineral oil, 0.48 g, 12.0 mmol) in THF (4 mL) is added at 0 °C and the mixture stirred for 20 minutes. Then the solution of 2,2-dimethyl-1,3-dioxan-5-yl 4-methylbenzenesulfonate (3.1 g, 11 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₂H₃₄O₇ (MW = 410.507).

### 5,5'-(((2-(benzyloxy)propane-1,3-diyl)bis(oxy))bis(methylene))bis(2,2-dimethyl-1,3-dioxane)

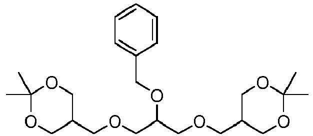

is synthesized as follows:
To the solution of 2-(benzyloxy)propane-1,3-diol (1g, 5.5 mmol) in DMF (10 mL) a suspension of NaH (60% in mineral oil, 0.48 g, 12.0 mmol) in THF (4 mL) is added at 0 °C and the mixture stirred for 20 minutes. Then the solution of (2,2-dimethyl-1,3-dioxan-5-yl)methyl 4-methylbenzenesulfonate (3.3 g, 11 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₄H₃₈O₇ (MW = 438.561).

### 1,3-bis((2,2-dimethyl-1,3-dioxan-5-yl)methoxy)propan-2-ol

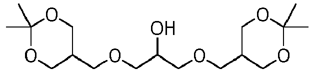

is synthesized as follows:
5,5'-(((2-(benzyloxy)propane-1,3-diyl)bis(oxy))bis(methylene))bis(2,2-dimethyl-1,3-dioxane) (1 g, 2.3 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₁₇H₃₂O₇ (MW = 348.436).

### 1,3-bis((2,2-dimethyl-1,3-dioxan-5-yl)oxy)propan-2-ol

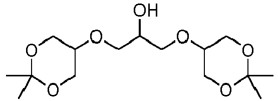

is synthesized as follows:
5,5'-((2-(benzyloxy)propane-1,3-diyl)bis(oxy))bis(2,2-dimethyl-1,3-dioxane) (1 g, 2.4 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₁₅H₂₈O₇ (MW = 320.382).

### 3-((2,2-dimethyl-1,3-dioxan-5-yl)methoxy)-2-(((2,2-dimethyl-1,3-dioxan-5-yl)methoxy)methyl)propan-1 -ol

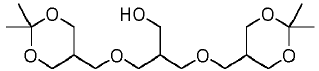

is synthesized as follows:
5,5'-(((2-((benzyloxy)methyl)propane-1,3-diyl)bis(oxy))bis(methylene))bis(2,2-dimethyl-1,3-dioxane) (1 g, 2.2 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₁₈H₃₄O₇ (MW = 362.463).

### 3-((2,2-dimethyl-1,3-dioxan-5-yl)oxy)-2-(((2,2-dimethyl-1,3-dioxan-5-yl)oxy)methyl)propan-1-ol

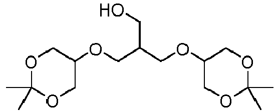

is synthesized as follows:
5,5'-((2-((benzyloxy)methyl)propane-1,3-diyl)bis(oxy))bis(2,2-dimethyl-1,3-dioxane) (1 g, 2.4 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₁₆H₃₀O₇ (MW = 334.409).

### 5,5'-(((2-azidopropane-1,3-diyl)bis(oxy))bis(methylene))bis(2,2-dimethyl-1,3-dioxane)

### (Isopropylidene-HPD2:HAC-azide)

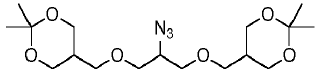

is synthesized as follows:
1,3-bis((2,2-dimethyl-1,3-dioxan-5-yl)methoxy)propan-2-ol (1 g, 2.3 mmol) and 2-azido-1,3-dimethylimidazolinium hexafluorophosphate (ADMP, 1 g, 3.4 mmol) are dissolved in THF (5 mL). At 0 °C 1,8 diazabicyclo(5.4.0)undec-7-ene (DBU, 1 mL, 6.8 mmol) is added to the solution and the solution is stirred for 90 minutes. The combined organic layer is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₇H₃₁N₃O₆ (MW = 373.45).

### 5,5'-((2-azidopropane-1,3-diyl)bis(oxy))bis(2,2-dimethyl-1,3-dioxane)

### (Isopropylidene-HAC2:HAC-azide)

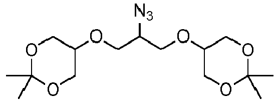

is synthesized as follows:
1,3-bis((2,2-dimethyl-1,3-dioxan-5-yl)oxy)propan-2-ol (1 g, 2.4 mmol) and 2-azido-1,3-dimethylimidazolinium hexafluorophosphate (ADMP, 1 g, 3.7 mmol) are dissolved in THF (5 mL). At 0 °C 1,8 diazabicyclo(5.4.0)undec-7-ene (DBU, 1.1 mL, 7.3 mmol) is added to the solution and the solution is stirred for 90 minutes. Then water (10 mL) is added to the solution and the mixture is extracted with ethyl acetate (2x 20 mL). The combined organic layer is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₅H₂₇N₃O₆ (MW = 345.396).

### 5,5'-(((2-(azidomethyl)propane-1,3-diyl)bis(oxy))bis(methylene))bis(2,2-dimethyl-1,3-dioxane)

### (Isopropylidene-HPD2:HPD-azide)

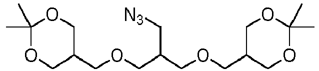

is synthesized as follows:
3-((2,2-dimethyl-1,3-dioxan-5-yl)methoxy)-2-(((2,2-dimethyl-1,3-dioxan-5-yl)methoxy)methyl)propan-1-ol (1 g, 2.2 mmol) and 2-azido-1,3-dimethylimidazolinium hexafluorophosphate (ADMP, 0.9 g, 3.3 mmol) are dissolved in THF (5 mL). At 0 °C 1,8 diazabicyclo(5.4.0)undec-7-ene (DBU, 1 mL, 6.6 mmol) is added to the solution and the solution is stirred for 90 minutes. The combined organic layer is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₈H₃₃N₃O₆ (MW = 387.477).

### 5,5'-((2-(azidomethyl)propane-1,3-diyl)bis(oxy))bis(2,2-dimethyl-1,3-dioxane)

### (Isopropylidene-HAC2:HPD-azide)

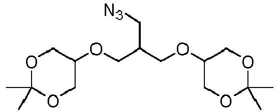

is synthesized as follows:
3-((2,2-dimethyl-1,3-dioxan-5-yl)oxy)-2-(((2,2-dimethyl-1,3-dioxan-5-yl)oxy)methyl)propan-1-ol (1 g, 2.4 mmol) and 2-azido-1,3-dimethylimidazolinium hexafluorophosphate (ADMP, 1 g, 3.5 mmol) are dissolved in THF (5 mL). At 0 °C 1,8 diazabicyclo(5.4.0)undec-7-ene (DBU, 1.1 mL, 7.1 mmol) is added to the solution and the solution is stirred for 90 minutes. The combined organic layer is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₆H₂₉N₃O₆ (MW = 359.423).

### 5,5'-(((2-(azidomethyl)-2-(((2,2-dimethyl-1,3-dioxan-5-yl)methoxy)methyl)propane-1,3 diyl)bis(oxy))bis(methylene))bis(2,2-dimethyl-1,3-dioxane)

### (Isopropylidene-HPD3:PEO-azide)

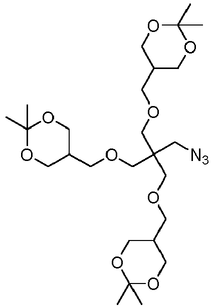

is synthesized as follows:
To the solution of 2-(azidomethyl)-2-(hydroxymethyl)propane-1,3-diol (1g, 6.2 mmol) a suspension of NaH (60% in mineral oil, 1 g, 24.8 mmol) in THF (6 mL) is added at 0 °C and the mixture stirred for 20 minutes. Then the solution of (2,2-dimethyl-1,3-dioxan-5-yl)methyl 4-methylbenzenesulfonate (5.6 g, 18.6 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₆H₄₇N₃O₉ (MW = 545.674)

### 5,5'-((2-(azidomethyl)-2-(((2,2-dimethyl-1,3-dioxan-5-yl)oxy)methyl)propane-1,3-diyl)bis(oxy))bis(2,2-dimethyl-1,3-dioxane)

### (Isopropylidene-HAC3:PEO-azide)

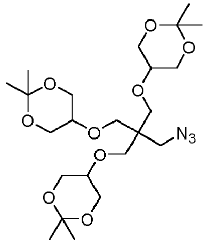

is synthesized as follows:
To the solution of 2-(azidomethyl)-2-(hydroxymethyl)propane-1,3-diol (1g, 6.2 mmol) a suspension of NaH (60% in mineral oil, 1 g, 24.8 mmol) in THF (6 mL) is added at 0 °C and the mixture stirred for 20 minutes. Then the solution of 2,2-dimethyl-1,3-dioxan-5-yl 4-methylbenzenesulfonate (5.3 g, 18.6 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₃H₄₁N₃O₉ (MW = 503.593)

### 5,5'-(((2-azido-2-(((2,2-dimethyl-1,3-dioxan-5-yl)methoxy)methyl)propane-1,3-diyl)bis(oxy))bis(methylene))bis(2,2-dimethyl-1,3-dioxane)

### (Isopropylidene-HPD3:TRIS-azide)

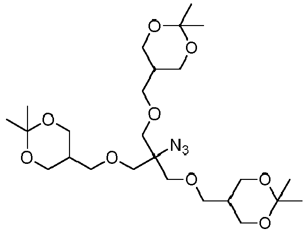

is synthesized as follows:
To the solution of 2-azido-2-(hydroxymethyl)propane-1,3-diol (1g, 6.8 mmol) a suspension of NaH (60% in mineral oil, 1.1 g, 27.2 mmol) in THF (6 mL) is added at 0 °C and the mixture stirred for 20 minutes. Then the solution of (2,2-dimethyl-1,3-dioxan-5-yl)methyl 4-methylbenzenesulfonate (6.1 g, 20.4 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₅H₄₅N₃O₉ (MW = 531.647)

### 5,5'-((2-azido-2-(((2,2-dimethyl-1,3-dioxan-5-yl)oxy)methyl)propane-1,3-diyl)bis(oxy))bis(2,2-dimethyl-1,3-dioxane)

### (Isopropylidene-HAC3:TRIS-azide)

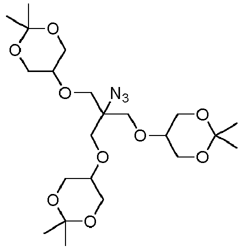

is synthesized as follows:
To the solution of 2-azido-2-(hydroxymethyl)propane-1,3-diol (1g, 6.8 mmol) a suspension of NaH (60% in mineral oil, 1.1 g, 27.2 mmol) in THF (6 mL) is added at 0 °C and the mixture stirred for 20 minutes. Then the solution of 2,2-dimethyl-1,3-dioxan-5-yl 4-methylbenzenesulfonate (5.8 g, 20.4 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₂H₃₉N₃O₉ (MW = 489.566).

### benzyl (1,3-bis((2,2-dimethyl-1,3-dioxan-5-yl)methoxy)propan-2-yl)carbamate

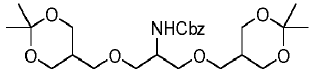

is synthesized as follows:
To the solution of benzyl (1,3-dihydroxypropan-2-yl)carbamate (1g, 4.4 mmol) a suspension of NaH (60% in mineral oil, 0.4 g, 11.1 mmol) in THF (4 mL) is added at 0 °C and the mixture stirred for 20 minutes. Then the solution of (2,2-dimethyl-1,3-dioxan-5-yl)methyl 4-methylbenzenesulfonate (2.7 g, 8.9 mmol) in DMF (5 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₅H₃₉NO₈ (MW = 481.586).

### benzyl (1,3-bis((2,2-dimethyl-1,3-dioxan-5-yl)oxy)propan-2-yl)carbamate

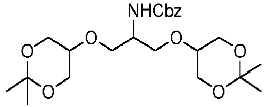

is synthesized as follows:
To the solution of benzyl (1,3-dihydroxypropan-2-yl)carbamate (1g, 4.4 mmol) a suspension of NaH (60% in mineral oil, 0.4 g, 11.1 mmol) in THF (4 mL) is added at 0 °C and the mixture stirred for 20 minutes. Then the solution of 2,2-dimethyl-1,3-dioxan-5-yl 4-methylbenzenesulfonate (2.5 g, 8.9 mmol) in DMF (5 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₃H₃₅NO₈ (MW = 453.532).

### benzyl(3-((2,2-dimethyl-1,3-dioxan-5-yl)methoxy)-2-(((2,2-dimethyl-1,3-dioxan-5-yl)methoxy) methyl)propyl)carbamate

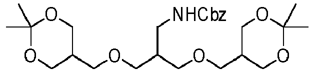

is synthesized as follows:
To the solution of benzyl (3-hydroxy-2-(hydroxymethyl)propyl)carbamate (1g, 4.2 mmol) a suspension of NaH (60% in mineral oil, 0.4 g, 10.4 mmol) in THF (4 mL) is added at 0 °C and the mixture stirred for 20 minutes. Then the solution of (2,2-dimethyl-1,3-dioxan-5-yl)methyl 4-methylbenzenesulfonate (2.5 g, 8.4 mmol) in DMF (5 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₆H₄₁NO₈ (MW = 495.613).

### benzyl(3-((2,2-dimethyl-1,3-dioxan-5-yl)oxy)-2-(((2,2-dimethyl-1,3-dioxan-5-yl)oxy)methyl) propyl)carbamate

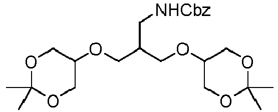

is synthesized as follows:
To the solution of benzyl (3-hydroxy-2-(hydroxymethyl)propyl)carbamate (1g, 4.2 mmol) a suspension of NaH (60% in mineral oil, 0.4 g, 10.4 mmol) in THF (4 mL) is added at 0 °C and the mixture stirred for 20 minutes. Then the solution of 2,2-dimethyl-1,3-dioxan-5-yl 4-methylbenzenesulfonate (2.4 g, 8.4 mmol) in DMF (5 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₄H₃₇NO₈ (MW = 467.559).

### benzyl (1,3-bis((2,2-dimethyl-1,3-dioxan-5-yl)methoxy)-2-(((2,2-dimethyl-1,3-dioxan-5-yl)methoxy) methyl)propan-2-yl)carbamate

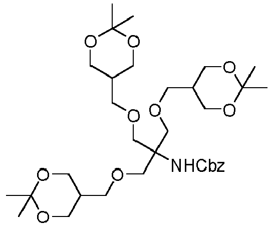

is synthesized as follows:
To the solution of benzyl (1,3-dihydroxy-2-(hydroxymethyl)propan-2-yl)carbamate (1g, 3.9 mmol) a suspension of NaH (60% in mineral oil, 0.6 g, 15.7 mmol) in THF (6 mL) is added at 0 °C and the mixture stirred for 20 minutes. Then the solution of (2,2-dimethyl-1,3-dioxan-5-yl)methyl 4-methylbenzenesulfonate (3.5 g, 11.8 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₃₃H₅₃NO₁₁ (MW = 639.783).

### benzyl (1,3-bis((2,2-dimethyl-1,3-dioxan-5-yl)oxy)-2-(((2,2-dimethyl-1,3-dioxan-5-yl)oxy)methyl) propan-2-yl)carbamate

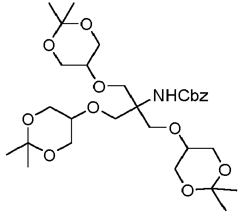

is synthesized as follows:
To the solution of benzyl (1,3-dihydroxy-2-(hydroxymethyl)propan-2-yl)carbamate (1g, 3.9 mmol) a suspension of NaH (60% in mineral oil, 0.6 g, 15.7 mmol) in THF (6 mL) is added at 0 °C and the mixture stirred for 20 minutes. Then the solution of 2,2-dimethyl-1,3-dioxan-5-yl 4-methylbenzenesulfonate (3.4 g, 11.8 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₃₀H₄₇NO₁₁ (MW = 597.702).

### benzyl (3-((2,2-dimethyl-1,3-dioxan-5-yl)methoxy)-2,2-bis(((2,2-dimethyl-1,3-dioxan-5-yl)methoxy) methyl)propyl)carbamate

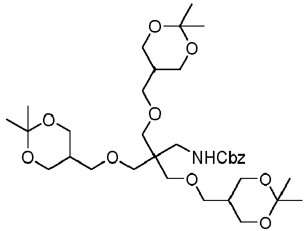

is synthesized as follows:
To the solution of benzyl (3-hydroxy-2,2-bis(hydroxymethyl)propyl)carbamate (1g, 3.7 mmol) a suspension of NaH (60% in mineral oil, 0.6 g, 14.9 mmol) in THF (6 mL) is added at 0 °C and the mixture stirred for 20 minutes. Then the solution of (2,2-dimethyl-1,3-dioxan-5-yl)methyl 4-methylbenzenesulfonate (3.3 g, 11.1 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₃₄H₅₅NO₁₁ (MW = 653.81).

### benzyl (3-((2,2-dimethyl-1,3-dioxan-5-yl)oxy)-2,2-bis(((2,2-dimethyl-1,3-dioxan-5-yl)oxy)methyl) propyl)carbamate

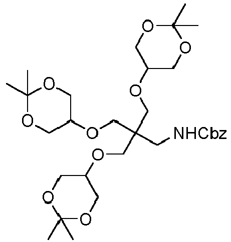

is synthesized as follows:
To the solution of benzyl (3-hydroxy-2,2-bis(hydroxymethyl)propyl)carbamate (1g, 3.7 mmol) a suspension of NaH (60% in mineral oil, 0.6 g, 14.9 mmol) in THF (6 mL) is added at 0 °C and the mixture stirred for 20 minutes. Then the solution of 2,2-dimethyl-1,3-dioxan-5-yl 4-methylbenzenesulfonate (3.2 g, 11.1 mmol) in DMF (10 mL) is added and the mixture allowed to warm to 10 °C within 2 hours. The solution is stirred overnight, then poured into ice-water and the extracted with ethyl acetate (60 mL). The organic phase is successively washed with 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₃₁H₄₉NO₁₁ (MW = 611.729).

### L3-bis((2.2-dimethyl-1.3-dioxan-5-yl)methoxy)propan-2-amine

### (Isopropylidene-HPD2:HAC-amine)

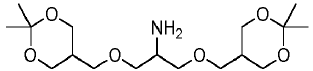

is synthesized as follows:
Benzyl (1,3-bis((2,2-dimethyl-1,3-dioxan-5-yl)methoxy)propan-2-yl)carbamate (1 g, 2.1 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₁₇H₃₃NO₆ (MW = 347.452).

### 1,3-bis((2,2-dimethyl-1,3-dioxan-5-yl)oxy)propan-2-amine

### (Isopropylidene-HAC2:HAC-amine)

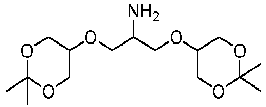

is synthesized as follows:
Benzyl (1,3-bis((2,2-dimethyl-1,3-dioxan-5-yl)oxy)propan-2-yl)carbamate (1 g, 2.2 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum.. ¹H NMR confirms identity of the target compound. C₁₅H₂₉NO₆ (MW = 319.398).

### 3-((2,2-dimethyl-1,3-dioxan-5-yl)methoxy)-2-(((2,2-dimethyl-1,3-dioxan-5-yl)methoxy)methyl) propan-1-amine

### (Isopropylidene-HPD2:HPD-amine)

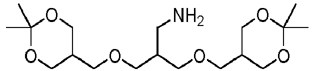

is synthesized as follows:
Benzyl (3-((2,2-dimethyl-1,3-dioxan-5-yl)methoxy)-2-(((2,2-dimethyl-1,3-dioxan-5-yl)methoxy) methyl)propyl)carbamate (1 g, 2 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₁₈H₃₅NO₆ (MW = 361.479).

### 3-((2,2-dimethyl-1,3-dioxan-5-yl)oxy)-2-(((2,2-dimethyl-1,3-dioxan-5-yl)oxy)methyl)propan-1-amine

### (Isopropylidene-HAC2:HPD-amine)

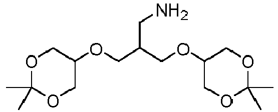

is synthesized as follows:
Benzyl (3-((2,2-dimethyl-1,3-dioxan-5-yl)oxy)-2-(((2,2-dimethyl-1,3-dioxan-5-yl)oxy)methyl)propyl) carbamate (1 g, 2.1 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₁₆H₃₁NO₆ (MW = 333.425).

### 1,3-bis((2,2-dimethyl-1,3-dioxan-5-yl)methoxy)-2-(((2,2-dimethyl-1,3-dioxan-5-yl)methoxy)methyl) propan-2-amine

### (Isopropylidene-HPD3:TRIS-amine)

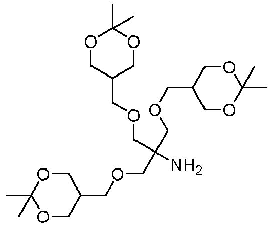

is synthesized as follows:
Benzyl (1,3-bis((2,2-dimethyl-1,3-dioxan-5-yl)methoxy)-2-(((2,2-dimethyl-1,3-dioxan-5-yl)methoxy) methyl)propan-2-yl)carbamate (1 g, 1.6 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₂₅H₄₇NO₉ (MW = 505.649).

### 1,3-bis((2,2-dimethyl-1,3-dioxan-5-yl)oxy)-2-(((2,2-dimethyl-1,3-dioxan-5-yl)oxy)methyl)propan-2-amine

### (Isopropylidene-HAC3:TRIS-amine)

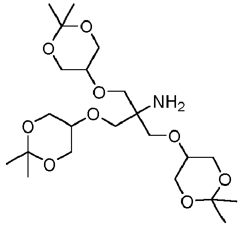

is synthesized as follows:
Benzyl (1,3 -bis((2,2-dimethyl-1,3 -dioxan-5 -yl)oxy)-2-(((2,2-dimethyl-1,3 -dioxan-5 -yl)oxy)methyl) propan-2-yl)carbamate (1 g, 1.7 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₂₂H₄₁NO₉ (MW = 463.568).

### 3-((2,2-dimethyl-1,3-dioxan-5-yl)methoxy)-2,2-bis(((2,2-dimethyl-1,3-dioxan-5-yl)methoxy)methyl) propan-1-amine

### (Isopropylidene-HPD3:PEO-amine)

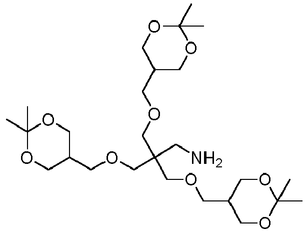

is synthesized as follows:
Benzyl (3-((2,2-dimethyl-1,3-dioxan-5-yl)methoxy)-2,2-bis(((2,2-dimethyl-1,3-dioxan-5-yl)methoxy) methyl)propyl)carbamate (1 g, 1.5 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₂₆H₄₉NO₉ (MW = 519.676).

### 3-((2,2-dimethyl-1,3-dioxan-5-yl)oxy)-2,2-bis(((2,2-dimethyl-1,3-dioxan-5-yl)oxy)methyl)propan-1-amine

### (Isopropylidene-HAC3:PEO-amine)

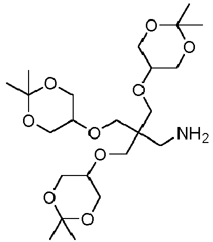

is synthesized as follows:
Benzyl (3-((2,2-dimethyl-1,3-dioxan-5-yl)oxy)-2,2-bis(((2,2-dimethyl-1,3-dioxan-5-yl)oxy)methyl) propyl)carbamate (1 g, 1.6 mmol) and Pd/C (10 wt. %, 0.3 g) are dissolved/suspended in ethanol (10 mL). The mixture is vigorously stirred in a hydrogen atmosphere for 4 hours. Then the catalyst is filtered and the solvent removed in the vaccum. ¹H NMR confirms identity of the target compound. C₂₃H₄₃NO₉ (MW = 477.595).

### 3-((tert-butyldimethylsilyl)oxy)-2,2-bis(((tert-butyldimethylsilyl)oxy)methyl)propan-1-amine

### (TBDMS-PEO-amine)

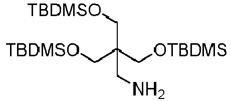

is synthesized as follows:
The solution of 2-(aminomethyl)-2-(hydroxymethyl)propane-1,3-diol (1 g, 7.4 mmol), TBDMS-Cl (5.8 mL, 33.3 mmol) and imidazole (3.0 g, 44.4 mmol) in DMF (10 mL) is stirred for 6 hours at room temperature. Then water (10 mL) is added and the stirring continued for another 2 hours. The mixture is extracted with ethyl acetate (2x 60 mL). The organic phase is washed water (3x 20 mL) and saturated sodium chloride solution (2x 20 mL), dried over anhydrous Na₂SO₄, and filtered. The filtrate is concentrated under reduced pressure and the residue purified by silica gel column chromatography with DCM/methanol (99:1). ¹H NMR confirms identity of the target compound. C₂₃H₅₅NO₃Si₃ (MW = 477.952).

### 5,5'-(((2-(prop-2-yn-1-yloxy)propane-1,3-diyl)bis(oxy))bis(methylene))bis(2,2-dimethyl-1,3-dioxane)

### (Isopropylidene-HPD2:HAC-alkyne)

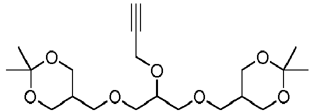

is synthesized as follows:
1,3-bis((2,2-dimethyl-1,3-dioxan-5-yl)methoxy)propan-2-ol (1 g, 2.9 mmol), the solution of propargyl bromide in toluene (80 % solution, 0.64 mL, 4.3 mmol), K₂CO₃ (0.8 g, 5.7 mmol) are dissolved/suspended in acetone (7.5 mL). The mixture is heated to 60 °C for a period of 6 hours. The solid components of the mixture are filtered off, the solvent is removed in the vacuum and the residue dissolved in DCM. The organic phase is successively washed 0.1 N NaOH, 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₀H₃₄O₇ (MW = 386.485).

### 5,5'-((2-(prop-2-yn-1-yloxy)propane-1,3-diyl)bis(oxy))bis(2,2-dimethyl-1,3 -dioxane)

### (Isopropylidene-HAC2:HAC-alkyne)

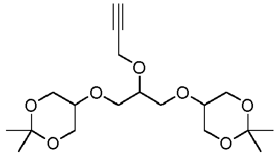

is synthesized as follows:
1,3-bis((2,2-dimethyl-1,3-dioxan-5-yl)oxy)propan-2-ol (1 g, 3.1 mmol), the solution of propargyl bromide in toluene (80 % solution, 0.7 mL, 4.7 mmol), K₂CO₃ (0.9 g, 6.2 mmol) are dissolved/suspended in acetone (7.5 mL). The mixture is heated to 60 °C for a period of 6 hours. The solid components of the mixture are filtered off, the solvent is removed in the vacuum and the residue dissolved in DCM. The organic phase is successively washed 0.1 N NaOH, 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₈H₃₀O₇ (MW = 358.431).

### 5,5'-(((2-((prop-2-yn-1-yloxy)methyl)propane-1,3-diyl)bis(oxy))bis(methylene))bis(2,2-dimethyl-1,3 dioxane)

### (Isopropylidene-HPD2:HPD-alkyne)

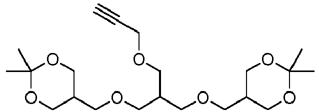

is synthesized as follows:
3-((2,2-dimethyl-1,3-dioxan-5-yl)methoxy)-2-(((2,2-dimethyl-1,3-dioxan-5-yl)methoxy)methyl) propan-1-ol (1 g, 2.8 mmol), the solution of propargyl bromide in toluene (80 % solution, 0.62 mL, 4.1 mmol), K₂CO₃ (0.8 g, 5.5 mmol) are dissolved/suspended in acetone (7.5 mL). The mixture is heated to 60 °C for a period of 6 hours. The solid components of the mixture are filtered off, the solvent is removed in the vacuum and the residue dissolved in DCM. The organic phase is successively washed 0.1 N NaOH, 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₂₁H₃₆O₇ (MW = 400.512).

### 5,5'-((2-((prop-2-yn-1-yloxy)methyl)propane-1,3-diyl)bis(oxy))bis(2,2-dimethyl-1,3-dioxane)

### (Isopropylidene-HAC2:HPD-alkyne)

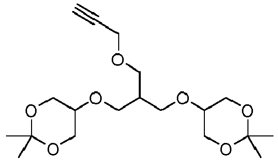

is synthesized as follows:
3-((2,2-dimethyl-1,3-dioxan-5-yl)oxy)-2-(((2,2-dimethyl-1,3-dioxan-5-yl)oxy)methyl)propan-1 -ol (1 g, 3 mmol), the solution of propargyl bromide in toluene (80 % solution, 0.67 mL, 4.5 mmol), K₂CO₃ (0.8 g, 6 mmol) are dissolved/suspended in acetone (7.5 mL). The mixture is heated to 60 °C for a period of 6 hours. The solid components of the mixture are filtered off, the solvent is removed in the vacuum and the residue dissolved in DCM. The organic phase is successively washed 0.1 N NaOH, 0.1 N HCl (2x 20 mL) and saturated sodium chloride solution (2x 20 mL), dried over anhydrous Na₂SO₄, and filtered. The filtrate is concentrated under reduced pressure and the residue purified by silica gel column chromatography with ethyl acetate and cyclohexane. ¹H NMR confirms identity of the target compound. C₁₉H₃₂O₇ (MW = 372.458).

### HPLC/MS analyses

HPLC/MS analyses are performed by injection of 5 µL of a solution of the sample, using a 2 step gradient for all chromatograms (5-65% B in 12 min, followed by 65-90% in 0.5 min, A: 0.1% TFA in water and B: 0.1% TFA in ACN). RP columns are from Agilent (Type Poroshell 120, 2.7µm, EC-C18, 50 x 3.00 mm, flow 0.8 mL, HPLC at room temperature); Mass spectrometer: Agilent 6230 LC/TOF-MS, ESI ionization. MassHunter Qualitative Analysis B.07.00 SP2 is used as software. UV detection is done at λ = 230 nm. Retention times (Rₜ) are indicated in the decimal system (e.g. 1.9 min = 1 min 54 s) and are referring to detection in the UV spectrometer. For the evaluation of observed compound masses the `Find Compounds by Formula'-feature is used. In particular, the individual `neutral mass of a compound (in units of Daltons)'-values and the corresponding isotope distribution pattern is used to confirm compound identity. The accuracy of the mass spectrometer is approx. ± 5 ppm.

### Preparative HPLC:

Preparative HPLC separations are done with reversed phase columns (General: Kinetex 5µ XB-C18 100 Å, 150 x 30 mm from Phenomenex. As mobile phase 0.1% TFA in water (A) and 0.1% TFA in ACN (B) are used, which are mixed in linear binary gradients. The gradients are described as: "10 to 40% B in 30 min", which means a linear gradient from 10% B (and correspondingly 90% A) to 40% B (and correspondingly 60% A) is run within 30min. Flow-rates are within the range of 30 to 50 mL/min. A typical gradient for the purification of the compounds of the invention starts at 5 25% B and ends after 30 min at 35-50% B and the difference between the percentage B at end and start is at least 10%. A commonly used gradient is "15 to 40% B in 30 min". Samples are dissolved preferably in mixtures of HOAc and water or DMSO.

### General procedures for Automated/Semi-automated Solid-Phase Synthesis:

Automated solid-phase of peptides and polyamides is performed on a Tetras Peptide Synthesizer (Advanced ChemTech) in 50 µmol and 100 µmol scales. Manual steps are performed in plastic syringes equipped with frits (material PE, Roland Vetter Laborbedarf OHG, Ammerbuch, Germany). The amount of reagents in the protocols described corresponds to the 100 µmol scale, unless stated otherwise.

Solid-phase synthesis is performed on polystyrene cross-linked di-(ethylene-glycol)-dimethacrylate (DEG). The used resin linker is rink amide. The resin loading is performed as a plain *Coupling of building blocks*/*amino acids* step as described below.

### Alloc/Allyl-deprotection:

After swelling in DMF, the resin is washed with DMF and DCM. DCM is de-oxygenated by passing a stream of nitrogen through the stirred solvent. The oxygen-free solvent is used to wash the resin trice. Then 2 mL of a 2 M solution of barbituric acid in oxygen-free DCM and 1 mL of a 25 µM solution of Tetrakis(triphenylphosphine)palladium(0) in oxygen-free DCM are added to the resin. The resin is agitated for 1 hour and then washed with DCM, MeOH, DMF, 0.5% DIPEA in DMF, 0.5% dithiocarbamate in DMF, DMF and DCM (each washing step is repeated 3 times with 3 mL, 1 min).

### Fmoc-deprotection:

After swelling in DMF, the resin is washed with DMF and then treated with piperidine/DMF (1:4, 3 mL, 2 and 20 min) and subsequently washed with DMF (3 mL, 5x 1 min).

### Dde-deprotection:

After swelling in DMF, the resin is washed with DMF and then treated with hydrazine-hydrate/DMF (2/98, 3 mL 2x 10 min) and subsequently washed with DMF (3 mL, 5x 1 min).

### Mtt-deprotection:

After swelling in DCM, the resin is washed with DCM and then treated with HFIP/DCM (7/3, 4 - 6 mL, 4 hours) and subsequently washed with DCM (3 mL, 3x 1 min), DMF (3 mL, 3x 1 mL) and DIPEA (0.9 M in DMF, 3 mL, 1 min).

### Solutions of reagents:

Building Blocks (0.3 M in DMF or NMP), DIPEA (0.9 M in DMF), HATU (0.4 M in DMF), Acetic anhydride (0.75 M in DMF), DIC (3.2 M in DMF)

### Coupling: Coupling of building blocks/amino acids (chain assembly):

Unless otherwise stated, coupling of building blocks is performed as follows: After subsequent addition of solutions of the corresponding building block (1.7 mL, 5eq.), DIPEA solution (1.15 mL, 10 eq.) and HATU solution (1.3 mL, 5 eq.) the resin is shaken for 45 min. If necessary, the resin is washed with DMF (3 mL, 1 min) and the coupling step is repeated.

In case DOTA(OtBu)₃-OH is coupled as a building block, the coupling is performed for 90 min. After this timespan DIC solution is added (0.2 mL, 12.5 eq.) and the reaction mixture is agitated for further 90 min.

### Copper(I)-catalyzed Azide-Alkyne Cycloaddition (CuAAC)

For the conversion of 0.1 mmol of resin the conditions are as follows: Water is de-oxygenated by a freezing and thawing under reduced pressure (3 repetitions). DMSO is de-oxygenated by passing a stream of nitrogen through the stirred solvent. The resin is swollen in DMSO, then the resin is washed with DMSO (3x) and finally with de-oxygenated DMSO (3x). CuBr (30 mg, 0.2 mmol), sodium-ascorbate (40 mg, 0.2 mmol), 2,6-lutidine (235 µL, 2 mmol) and DIPEA (360 µL, 2 mmol) are dissolved in de-oxygenated DMSO (5 mL) and de-oxygenated water (1 mL). For the cycloaddition of resin bound azides, 10 eq. of the corresponding alkyne is added to this mixture. For the cycoaddition of resin bound alkynes, 10 eq. the corresponding azide is added to the mixture. Then the solution is added to resin, which is the agitated for 24 hours. The resin is the successively washed with Isopropanol:DMSO (5:3), DMF (3x) and DCM (3x).

### Reductive amination of resin bound amines

The resin (0.1 mmol) is swollen in DMF and washed with DMF (3x). To the resin a solution of the sodium cyanoborohydride (125 mg, 2mmol) and the corresponding aldehyde (12 eq.) or ketone (4 eq) is added. The resin is agitated overnight and subsequently washed with DMF (3x) and DCM (3x).

### Urea moiety synthesis

The resin (0.1 mmol) is swollen in DCM. Then trimethylamine (2.5 mmol, 350 µL) in DCM (5 mL) are added to resin. Then N,N'-disuccinimidyl carbonate (1 mmol, 260 mg) is added and the resin agitated for 4 hours. The resin is washed with DCM (3x) and DMF (3x). A solution of the corresponding amine (5 eq.) and DIPEA (90 µL, 0.5 mmol) in DCM or DMF is added and the resin is agitated overnight. Finally the resin is washed with DMF (3x) and DCM (3x).

### Cleavage of unprotected fragments (complete resin cleavage):

After the completion of the assembly of the sequence the resin is finally washed with DCM (3 mL, 4x 1 min), dried in the vacuum overnight and treated with TFA, EDT, water and TIPS (94/2.5/2.5/1) for 4 h (unless otherwise stated). Afterwards the cleavage solution is poured into a chilled mixture of MTBE and cyclohexane (1/1, 10-fold excess compared to the volume of cleavage solution), centrifuged at 4 °C for 5 min and the precipitate collected and dried in the vacuum. The residue is lyophilized from water/acetonitrile prior to purification or further modification.

More relevant Fmoc-solid-phase-peptide synthesis methods are described in detail in "Fmoc Solid Phase Peptide Synthesis" Editors W. Chan, P. White, Oxford University Press, USA, 2000. Compounds were named using ChemDraw 22 by Perkin Elmer Informatics, Inc.

### Solution Cyclization: bis-(bromomethyl)-pyridin cyclization

The crude peptide material is dissolved in a 1:1 mixture of ammonium bicarbonate solution (50 mM, pH = 8.5) and acetonitrile. To the resulting mixture a solution of 2,6-bis(bromomethyl)pyridine in acetonitrile is added. Upon completition of the cyclization reaction, which is judged by analytical LC-MS, DTT is added and the solution stirred for a further hour. Then TFA is added and the reaction solution subjected to lyophilization. The volume of solvent, amount of 2,6-bis(bromomethyl)pyridine, DTT and volume of TFA used in the reaction depend on the amount of resin used for the synthesis of the linear peptide precursor - per 50 µmol of initially used 60 mL of the solvent mixture, 14.5 mg (55 µmol) of 2,6-bis(bromomethyl)pyridine, 15.4 mg (100 µmol) of DTT and 50 µL of TFA are used.

### Preparation of compounds:

Specific embodiments for the preparation of compounds of the invention are prepared by using the preferred general methods disclosed above and other published or other methods which are known to persons skilled in the art. Unless otherwise specified, all starting materials and reagents are of standard commercial grade, and are used without further purification, or are readily prepared from such materials by routine methods. Those skilled in the art of organic synthesis will recognize in light of the instant disclosure that starting materials and reaction conditions may be varied including additional steps employed to produce compounds encompassed by the present invention.

### Example 2: Synthesis of HPD2:Ahx-Asp-Trp-5Clw-Tap-Glu-leu-[Cys(2Lut)-Apc(DOTA)-Leu-Ile-Cys]-APAc-lys:HPD2 (GIP-1001)

The linear sequence (Ahx-Asp-Trp-5Clw-Tap-Glu-leu-Cys-Apc(Alloc)-Leu-Ile-Cys-APAc-lys(Dde)-NH₂) of the peptide is assembled according to the `General procedures for Automated/Semi-automated Solid-Phase Synthesis' in a 100 µmol scale on a Rink amide DEG resin. The Dde protecting group is removed by performing a *'Dde-deprotection'.* A *'Reductive amination of resin bound amines"* with 2,2-dimethyl-1,3-dioxane-5-carbaldehyde (CAS: 102573-84-2) as aldehyde reagent is performed. The Alloc protection group is then removed from the Apc side chain by performing the steps of the *'Alloc*/*Allyl-deprotection'* and DOTA(OtBu)₃-OH is coupled. Thereafter the steps of *'Cleavage of unprotected fragments (complete resin cleavage)'* are performed to cleave the peptide from the resin. Subsequently the lyophilized crude peptide residue is subjected to *'Solution Cyclization: bis-(bromomethyl)-pyridin cyclization'.* After lyophilization the crude product is purified by preparative HPLC. LC/TOF-MS confirms the identity of the target compound. C₁₂₄H₁₉₃ClN₂₆O₃₃S₂ (MW = 2675.627).

### Example 3: Synthesis of HPD3:PEO:Hyx-Asp-Trp-5Clw-Tap-Glu-leu-[Cys(2Lut)-Apc(DOTA)-Leu-Ile-Cys]-APAc-prx:PEO:HPD3 (GIP-1002)

The linear sequence (Hyn-Asp-Trp-5Clw-Tap-Glu-leu-Cys-Apc(Alloc)-Leu-Ile-Cys-APAc-prg-NH₂) of the peptide is assembled according to the `General procedures for Automated/Semi-automated Solid-Phase Synthesis' in a 100 µmol scale on a Rink amide DEG resin. Next the resin is subjected to a *Copper(I)-catalyzed Azide-Alkyne Cycloaddition (CuAAC)* with Isopropylidene-HPD3:PEO-azide as azide reagent. The Alloc protection group is then removed from the Apc side chain by performing the steps of the *'Alloc*/*Allyl-deprotection'* and DOTA(OtBu)₃-OH is coupled. Thereafter the steps of *'Cleavage of unprotected fragments (complete resin cleavage)'* are performed to cleave the peptide from the resin. Subsequently the lyophilized crude peptide residue is subjected to *'Solution Cyclization: bis-(bromomethyl)-pyridin cyclization".* After lyophilization the crude product is purified by preparative HPLC. LC/TOF-MS confirms the identity of the target compound. C₁₄₂H₂₂₁ClN₃₀O₄₃S₂ (MW = 3136.067).

### Example 4: Synthesis of PEO:Aur-Asp-Trp-5Clw-Tap-Glu-leu-[Cys(2Lut)-Apc(DOTA)-Leu-Ile-Cys]-APAc-kur:PEO (GIP-1003)

The linear sequence (Ahx-Asp-Trp-5Clw-Tap-Glu-leu-Cys-Apc(Alloc)-Leu-Ile-Cys-APAc-lys(Dde)-NH₂) of the peptide is assembled according to the `General procedures for Automated/Semi-automated Solid-Phase Synthesis' in a 100 µmol scale on a Rink amide DEG resin. The Dde protecting group is removed by performing a *'Dde-deprotection'.* The steps of the *'Urea moiety synthesis"* are performed with TBDMS3-PEO-amine as amine reagent. The Alloc protection group is then removed from the Apc side chain by performing the steps of the *'Alloc*/*Allyl-deprotection'* and DOTA(OtBu)₃-OH is coupled. Thereafter the steps of *'Cleavage of unprotected fragments (complete resin cleavage)'* are performed to cleave the peptide from the resin. Subsequently the lyophilized crude peptide residue is subjected to *'Solution Cyclization: bis-(bromomethyl)-pyridin cyclization".* After lyophilization the crude product is purified by preparative HPLC. LC/TOF-MS confirms the identity of the target compound. C₁₂₀H₁₈₃ClN₂₈O₃₃S₂ (MW = 2645.517).

### Example 5: Synthesis of HAC2:HAC:Ahx-Asp-Trp-5Clw-Tap-Glu-leu-[Cys(2Lut)-Apc(DOTA)-Leu-Ile-Cys]-APAc-lys:HAC:HAC2 (GIP-1089)

The linear sequence (Ahx-Asp-Trp-5Clw-Tap-Glu-leu-Cys-Apc(Alloc)-Leu-Ile-Cys-APAc-lys(Dde)-NH₂) of the peptide is assembled according to the `General procedures for Automated/Semi-automated Solid-Phase Synthesis' in a 100 µmol scale on a Rink amide DEG resin. The Dde protecting group is removed by performing a *'Dde-deprotection'.* A *'Reductive amination of resin bound amines"* with Isopropylidene-HAC2:HAC-one as ketone reagent is performed. The Alloc protection group is then removed from the Apc side chain by performing the steps of the *'Alloc*/*Allyl-deprotection'* and DOTA(OtBu)₃-OH is coupled. Thereafter the steps of *'Cleavage of unprotected fragments (complete resin cleavage)'* are performed to cleave the peptide from the resin. Subsequently the lyophilized crude peptide residue is subjected to *'Solution Cyclization: bis-(bromomethyl)-pyridin cyclization'.* After lyophilization the crude product is purified by preparative HPLC. LC/TOF-MS confirms the identity of the target compound. C₁₂₆H₁₉₇ClN₂₆O₃₇S₂ (MW = 2767.677).

### Example 6: Synthesis of HAC2:HAC:Tzx-Asp-Trp-5Clw-Tap-Glu-leu-[Cys(2Lut)-Apc(DOTA)-Leu-Ile-Cys]-APAc-tzk:HAC:HAC2 (GIP-1090)

The linear sequence (N3Ahx-Asp-Trp-5Clw-Tap-Glu-leu-Cys-Apc(Alloc)-Leu-Ile-Cys-APAc-n3lys-NH₂) of the peptide is assembled according to the `General procedures for Automated/Semi-automated Solid-Phase Synthesis' in a 100 µmol scale on a Rink amide DEG resin. Next the resin is subjected to a *Copper(I)-catalyzed Azide-Alkyne Cycloaddition (CuAAC)* with Isopropylidene-HAC2:HAC-alkyne as alykne reagent. The Alloc protection group is then removed from the Apc side chain by performing the steps of the *'AlloclAllyl-deprotection'* and DOTA(OtBu)₃-OH is coupled. Thereafter the steps of *'Cleavage of unprotected fragments (complete resin cleavage)'* are performed to cleave the peptide from the resin. Subsequently the lyophilized crude peptide residue is subjected to *'Solution Cyclization: bis-(bromomethyl)-pyridin cyclization'.* After lyophilization the crude product is purified by preparative HPLC. LC/TOF-MS confirms the identity of the target compound. C₁₃₂H₂₀₁ClN₃₀O₃₉S₂ (MW = 2931.801).

Table 8 summarises the synthetic approaches of the compounds GIP-1000 to GIP-1132 with respect to the synthesis procedure according to Example 2 to 6 (Synt. acc. Ex.) and the particular reagent used in the synthesis. Either the particular reagent is synthesized as described above or stems from a commercial source.

**Table 8: Compound summary with ID, Sequence, Synthesis approach as described in Examples 2 to 6 and specific reagent, which is used in its synthesis**

| Comp. ID | Sequence | Synt. acc. Ex. | Reagent |
|---|---|---|---|
| GIP-1001 | HPD2:Ahx-Asp-Trp-5Clw-Tap-Glu-leu-[Cys(2Lut)-Apc(DOTA)-Leu-Ile-Cys]-APAc-lys:HPD2 | 2 | 2,2-dimethyl-1,3-dioxane-5-carbaldehyde (CAS: 102573-84-2) |
| GIP-1002 | HPD3:PEO:Hyx-Asp-Trp-5Clw-Tap-Glu-leu-[Cys(2Lut)-Apc(DOTA)-Leu-Ile-Cys] -APAc-prx: PEO :HPD3 | 3 | Isopropylidene-HPD3:PEO-azide |
| GIP-1003 | PEO:Aur-Asp-Trp-5 Clw-Tap-Glu-leu-[Cys(2Lut)-Apc(DOTA)-Leu-Ile-Cys]-APAc-kur:PEO | 4 | TBDMS3-PEO-amine |
| GIP-1004 | PEO:Hyx-Asp-Trp-5 Clw-Tap-Glu-leu-[Cys(2Lut)-Apc(DOTA)-Leu-Ile-Cys]-APAc-prx:PEO | 3 | 2-(azidomethyl)-2-(hydroxymethyl)propane-1,3-diol (PubChem ID: 89443549) |
| GIP-1005 | PEO2:Ahx-Asp-Trp-5Clw-Tap-Glu-leu-[Cys(2Lut)-Apc(DOTA)-Leu-Ile-Cys]-APAc-lys:PEO2 | 2 | tBu3-PEO-aldehyde |
| GIP-1006 | TRIS:Hyx-Asp-Trp-5Clw-Tap-Glu-leu-[Cys(2Lut)-Apc(DOTA)-Leu-Ile-Cys]-APAc-prx:TRIS | 3 | 2-azido-2-(hydroxymethyl)propane-1,3-diol (CAS: 885318-72-9) |
| GIP-1007 | HPD3:TRIS:Hyx-Asp-Trp-5Clw-Tap-Glu-leu-[Cys(2Lut)-Apc(DOTA)-Leu-Ile-Cys]-APAc-prx:TRIS:HPD3 | 3 | Isopropylidene-HPD3:TRIS-azide |
| GIP-1008 | HAC3:TRIS:Hyx-Asp-Trp-5Clw-Tap-Glu-leu-[Cys(2Lut)-Apc(DOTA)-Leu-Ile-Cys]-APAc-prx:TRIS:HAC3 | 3 | Isopropylidene-HAC3:TRIS-azide |
| GIP-1009 | HAC3:PEO:Hyx-Asp-Trp-5Clw-Tap-Glu-leu-[Cys(2Lut)-Apc(DOTA)-Leu-Ile-Cys]-APAc-prx:PEO:HAC3 | 3 | Isopropylidene-HAC3:PEO-azide |
| GIP-1010 | HAC3:PEO:Aur-Asp-Trp-5Clw-Tap-Glu-leu-[Cys(2Lut)-Apc(DOTA)-Leu-Ile-Cys]-APAc-kur:PEO:HAC3 | 4 | Isopropylidene-HAC3:PEO-amine |
| GIP-1011 | HAC3:TRIS:Aur-Asp-Trp-5Clw-Tap-Glu-leu-[Cys(2Lut)-Apc(DOTA)-Leu-Ile-Cys]-APAc-kur:TRIS:HAC3 | 4 | Isopropylidene-HAC3:TRIS-amine |
| GIP-1012 | HPD3:PEO:Aur-Asp-Trp-5Clw-Tap-Glu-leu-[Cys(2Lut)-Apc(DOTA)-Leu-Ile-Cys]-APAc-kur:PEO:HPD3 | 4 | Isopropylidene-HPD3:PEO-amine |
| GIP-1013 | HPD3:TRIS:Aur-Asp-Trp-5Clw-Tap-Glu-leu-[Cys(2Lut)-Apc(DOTA)-Leu-Ile-Cys]-APAc-kur:TRIS:HPD3 | 4 | Isopropylidene-HPD3:TRIS-amine |
| GIP-1014 | TRIS:Aur-Asp-Trp-5Clw-Tap-Glu-leu-[Cys(2Lut)-Apc(DOTA)-Leu-Ile-Cys]-APAc-kur:TRIS | 4 | 6-(((tert-butyldimethylsilyl)oxy)methyl)-2,2,3,3,9,9,10,10-octamethyl-4,8-dioxa-3,9-disilaundecan-6-amine (CAS: 102522-47-4) |
| GIP-1015 | HPD2:Ahx-Asp-Trp-5Clw-Tap-Glu-leu-[Cys(2Lut)-Amk(DOTA)-Leu-Ile-Cys]-APAc-lys:HPD2 | 2 | 2,2-dimethyl-1,3-dioxane-5-carbaldehyde (CAS: 102573-84-2) |
| GIP-1016 | HPD3:PEO:Hyx-Asp-Trp-5Clw-Tap-Glu-leu-[Cys(2Lut)-Amk(DOTA)-Leu-Ile-Cys]-APAc-prxPEO:HPD3 | 3 | Isopropylidene-HPD3:PEO-azide |
| GIP-1017 | PEO:Aur-Asp-Trp-5Clw-Tap-Glu-leu-[Cys(2Lut)-Amk(DOTA)-Leu-Ile-Cys]-APAc-kur:PEO | 4 | TBDMS3-PEO-amine |
| GIP-1018 | PEO:Hyx-Asp-Trp-5Clw-Tap-Glu-leu-[Cys(2Lut)-Amk(DOTA)-Leu-Ile-Cys]-APAc-prx:PEO | 3 | 2-(azidomethyl)-2-(hydroxymethyl)propane-1,3-diol (PubChem ID: 89443549) |
| GIP-1019 | PEO2:Ahx-Asp-Trp-5Clw-Tap-Glu-leu-[Cys(2Lut)-Amk(DOTA)-Leu-Ile-Cys]-APAc-lys:PEO2 | 2 | tBu3-PEO-aldehyde |
| GIP-1020 | TRIS:Hyx-Asp-Trp-5Clw-Tap-Glu-leu-[Cys(2Lut)-Amk(DOTA)-Leu-Ile-Cys]-APAc-prx:TRIS | 3 | 2-azido-2-(hydroxymethyl)propane-1,3-diol (CAS: 885318-72-9) |
| GIP-1021 | HPD3:TRIS:Hyx-Asp-Trp-5Clw-Tap-Glu-leu-[Cys(2Lut)-Amk(DOTA)-Leu-Ile-Cys]-APAc-prx:TRIS:HPD3 | 3 | Isopropylidene-HPD3:TRIS-azide |
| GIP-1022 | HAC3:TRIS:Hyx-Asp-Trp-5Clw-Tap-Glu-leu-[Cys(2Lut)-Amk(DOTA)-Leu-Ile-Cys]-APAc-prx:TRIS:HAC3 | 3 | Isopropylidene-HAC3:TRIS-azide |
| GIP-1023 | HAC3:PEO:Hyx-Asp-Trp-5Clw-Tap-Glu-leu-[Cys(2Lut)-Amk(DOTA)-Leu-Ile-Cys]-APAc-prx:PEO:HAC3 | 3 | Isopropylidene-HAC3:PEO-azide |
| GIP-1024 | HAC3:PEO:Aur-Asp-Trp-5Clw-Tap-Glu-leu-[Cys(2Lut)-Amk(DOTA)-Leu-Ile-Cys]-APAc-kur:PEO:HAC3 | 4 | Isopropylidene-HAC3:PEO-amine |
| GIP-1025 | HAC3:TRIS:Aur-Asp-Trp-5Clw-Tap-Glu-leu-[Cys(2Lut)-Amk(DOTA)-Leu-Ile-Cys]-APAc-kur:TRIS:HAC3 | 4 | Isopropylidene-HAC3:TRIS-amine |
| GIP-1026 | HPD3:PEO:Aur-Asp-Trp-5Clw-Tap-Glu-leu-[Cys(2Lut)-Amk(DOTA)-Leu-Ile-Cys]-APAc-kur:PEO:HPD3 | 4 | Isopropylidene-HPD3:PEO-amine |
| GIP-1027 | HPD3:TRIS:Aur-Asp-Trp-5Clw-Tap-Glu-leu-[Cys(2Lut)-Amk(DOTA)-Leu-Ile-Cys]-APAc-kur:TRIS:HPD3 | 4 | Isopropylidene-HPD3:TRIS-amine |
| GIP-1028 | TRIS:Aur-Asp-Trp-5Clw-Tap-Glu-leu-[Cys(2Lut)-Amk(DOTA)-Leu-Ile-Cys]-APAc-kur:TRIS | 4 | 6-(((tert-butyldimethylsilyl)oxy)methyl)-2,2,3,3,9,9,10,10-octamethyl-4,8-dioxa-3,9-disilaundecan-6-amine (CAS: 102522-47-4) |
| GIP-1029 | HPD2:Ahx-Asp-Trp-5Clw-Hyp-Glu-leu-[Cys(2Lut)-Apc(DOTA)-Leu-Ile-Cys]-APAc-lys:HPD2 | 2 | 2,2-dimethyl-1,3-dioxane-5-carbaldehyde (CAS: 102573-84-2) |
| GIP-1030 | HPD3:PEO:Hyx-Asp-Trp-5Clw-Hyp-Glu-leu-[Cys(2Lut)-Apc(DOTA)-Leu-Ile-Cys]-APAc-prx:PEO:HPD3 | 3 | Isopropylidene-HPD3:PEO-azide |
| GIP-1031 | PEO: Aur-Asp-Trp-5Clw-Hyp-Glu-leu-[Cys(2Lut)-Apc(DOTA)-Leu-Ile-Cys]-APAc-kur:PEO | 4 | TBDMS3-PEO-amine |
| GIP-1032 | PEO:Hyx-Asp-Trp-5Clw-Hyp-Glu-leu-[Cys(2Lut)-Apc(DOTA)-Leu-Ile-Cys]-APAc-prx:PEO | 3 | 2-(azidomethyl)-2-(hydroxymethyl)propane-1,3-diol (PubChem ID: 89443549) |
| GIP-1033 | PEO2:Ahx-Asp-Trp-5Clw-Hyp-Glu-leu-[Cys(2Lut)-Apc(DOTA)-Leu-Ile-Cys]-APAc-lys:PEO2 | 2 | tBu3-PEO-aldehyde |
| GIP-1034 | TRIS:Hyx-Asp-Trp-5Clw-Hyp-Glu-leu-[Cys(2Lut)-Apc(DOTA)-Leu-Ile-Cys]-APAc-prx:TRIS | 3 | 2-azido-2-(hydroxymethyl)propane-1,3-diol (CAS: 885318-72-9) |
| GIP-1035 | HPD3 :TRIS:Hyx-Asp-Trp-5Clw-Hyp-Glu-leu-[Cys(2Lut)-Apc(DOTA)-Leu-Ile-Cys]-APAc-prx:TRIS:HPD3 | 3 | Isopropylidene-HPD3:TRIS-azide |
| GIP-1036 | HAC3:TRIS:Hyx-Asp-Trp-5Clw-Hyp-Glu-leu-[Cys(2Lut)-Apc(DOTA)-Leu-Ile-Cys]-APAc-prx:TRIS:HAC3 | 3 | Isopropylidene-HAC3:TRIS-azide |
| GIP-1037 | HAC3:PEO:Hyx-Asp-Trp-5Clw-Hyp-Glu-leu-[Cys(2Lut)-Apc(DOTA)-Leu-Ile-Cys]-APAc-prx:PEG:HAC3 | 3 | Isopropylidene-HAC3:PEO-azide |
| GIP-1038 | HAC3:PEO:Aur-Asp-Trp-5Clw-Hyp-Glu-leu-[Cys(2Lut)-Apc(DOTA)-Leu-Ile-Cys]-APAc-kur:PEO:HAC3 | 4 | Isopropylidene-HAC3:PEO-amine |
| GIP-1039 | HAC3:TRIS:Aur-Asp-Trp-5Clw-Hyp-Glu-leu-[Cys(2Lut)-Apc(DOTA)-Leu-Ile-Cys]-APAc-kur:TRIS:HAC3 | 4 | Isopropylidene-HAC3:TRIS-amine |
| GIP-1040 | HPD3:PEO:Aur-Asp-Trp-5Clw-Hyp-Glu-leu-[Cys(2Lut)-Apc(DOTA)-Leu-Ile-Cys]-APAc-kur:PEO:HPD3 | 4 | Isopropylidene-HPD3:PEO-amine |
| GIP-1041 | HPD3:TRIS:Aur-Asp-Trp-5Clw-Hyp-Glu-leu-[Cys(2Lut)-Apc(DOTA)-Leu-Ile-Cys]-APAc-kur:TRIS:HPD3 | 4 | Isopropylidene-HPD3:TRIS-amine |
| GIP-1042 | TRIS:Aur-Asp-Trp-5Clw-Hyp-Glu-leu-[Cys(2Lut)-Apc(DOTA)-Leu-Ile-Cys]-APAc-kur:TRIS | 4 | 6-(((tert-butyldimethylsilyl)oxy)methyl)-2,2,3,3,9,9,10,10-octamethyl-4,8-dioxa-3,9-disilaundecan-6-amine (CAS: 102522-47-4) |
| GIP-1043 | HPD2:Ahx-Asp-Trp-5Clw-Hyp-Glu-leu-[Cys(2Lut)-Amk(DOTA)-Leu-Ile-Cys]-APAc-lys:HPD2 | 2 | 2,2-dimethyl-1,3-dioxane-5-carbaldehyde (CAS: 102573-84-2) |
| GIP-1044 | HPD3:PEO:Hyx-Asp-Trp-5Clw-Hyp-Glu-leu-[Cys(2Lut)-Amk(DOTA)-Leu-Ile-Cys]-APAc-prx:PEO:HPD3 | 3 | Isopropylidene-HPD3:PEO-azide |
| GIP-1045 | PEO:Aur-Asp-Trp-5Clw-Hyp-Glu-leu-[Cys(2Lut)-Amk(DOTA)-Leu-Ile-Cys]-APAc-kur:PEO | 4 | TBDMS3-PEO-amine |
| GIP-1046 | PEO:Hyx-Asp-Trp-5Clw-Hyp-Glu-leu-[Cys(2Lut)-Amk(DOTA)-Leu-Ile-Cys]-APAc-prx:PEO | 3 | 2-(azidomethyl)-2-(hydroxymethyl)propane-1,3-diol (PubChem ID: 89443549) |
| GIP-1047 | PEO2:Ahx-Asp-Trp-5Clw-Hyp-Glu-leu-[Cys(2Lut)-Amk(DOTA)-Leu-Ile-Cys]-APAc-lys:PEO2 | 2 | tBu3-PEO-aldehyde |
| GIP-1048 | TRI S:Hyx-Asp-Trp-5Clw-Hyp-Glu-leu-[Cys(2Lut)-Amk(DOTA)-Leu-Ile-Cys]-APAc-prx:TRIS | 3 | 2-azido-2-(hydroxymethyl)propane-1,3-diol (CAS: 885318-72-9) |
| GIP-1049 | HPD3:TRIS:Hyx-Asp-Trp-5Clw-Hyp-Glu-leu-[Cys(2Lut)-Amk(DOTA)-Leu-Ile-Cys]-APAc-prx:TRIS:HPD3 | 3 | Isopropylidene-HPD3:TRIS-azide |
| GIP-1050 | HAC3:TRIS:Hyx-Asp-Trp-5Clw-Hyp-Glu-leu-[Cys(2Lut)-Amk(DOTA)-Leu-Ile-Cys]-APAc-prx:TRIS:HAC3 | 3 | Isopropylidene-HAC3:TRIS-azide |
| GIP-1051 | HAC3:PEO:Hyx-Asp-Trp-5Clw-Hyp-Glu-leu-[Cys(2Lut)-Amk(DOTA)-Leu-Ile-Cys]-APAc-prx:PEG:HAC3 | 3 | Isopropylidene-HAC3:PEO-azide |
| GIP-1052 | HAC3:PEO:Aur-Asp-Trp-5Clw-Hyp-Glu-leu-[Cys(2Lut)-Amk(DOTA)-Leu-Ile-Cys]-APAc-kur:PEG:HAC3 | 4 | Isopropylidene-HAC3:PEO-amine |
| GIP-1053 | HAC3:TRIS:Aur-Asp-Trp-5Clw-Hyp-Glu-leu-[Cys(2Lut)-Amk(DOTA)-Leu-Ile-Cys]-APAc-kur:TRIS:HAC3 | 4 | Isopropylidene-HAC3:TRIS-amine |
| GIP-1054 | HPD3:PEO:Aur-Asp-Trp-5Clw-Hyp-Glu-leu-[Cys(2Lut)-Amk(DOTA)-Leu-Ile-Cys]-APAc-kur:PEO:HPD3 | 4 | Isopropylidene-HPD3:PEO-amine |
| GIP-1055 | HPD3:TRIS:Aur-Asp-Trp-5Clw-Hyp-Glu-leu-[Cys(2Lut)-Amk(DOTA)-Leu-Ile-Cys]-APAc-kur:TRIS:HPD3 | 4 | Isopropylidene-HPD3:TRIS-amine |
| GIP-1056 | TRIS:Aur-Asp-Trp-5Clw-Hyp-Glu-leu-[Cys(2Lut)-Amk(DOTA)-Leu-Ile-Cys]-APAc-kur:TRIS | 4 | 6-(((tert-butyldimethylsilyl)oxy)methyl)-2,2,3,3,9,9,10,10-octamethyl-4,8-dioxa-3,9-disilaundecan-6-amine (CAS: 102522-47-4) |
| GIP-1057 | HAC2:HPD:Aur-Asp-Trp-5Clw-Tap-Glu-leu-[Cys(2Lut)-Apc(DOTA)-Leu-Ile-Cys]-APAc-kur:HPD:HAC2 | 4 | Isopropylidene-HAC2:HPD-amine |
| GIP-1058 | HAC2:HPD:Hyx-Asp-Trp-5Clw-Tap-Glu-leu-[Cys(2Lut)-Apc(DOTA)-Leu-Ile-Cys]-APAc-prx:HPD:HAC2 | 3 | Isopropylidene-HAC2:HPD-azide |
| GIP-1059 | HAC4:HPD2:Ahx-Asp-Trp-5Clw-Tap-Glu-leu-[Cys(2Lut)-Apc(DOTA)-Leu-Ile-Cys]-APAc-lys:HPD2:HAC4 | 2 | Isopropylidene-HAC2:HPD-aldehyde |
| GIP-1060 | HPD2:HPD:Aur-Asp-Trp-5Clw-Tap-Glu-leu-[Cys(2Lut)-Apc(DOTA)-Leu-Ile-Cys]-APAc-kur:HPD:HPD2 | 4 | Isopropylidene-HPD2:HPD-amine |
| GIP-1061 | HPD2:HPD:Hyx-Asp-Trp-5Clw-Tap-Glu-leu-[Cys(2Lut)-Apc(DOTA)-Leu-Ile-Cys]-APAc-prx:HPD:HPD2 | 3 | Isopropylidene-HPD2:HPD-azide |
| GIP-1062 | HPD4:HPD2:Ahx-Asp-Trp-5Clw-Tap-Glu-leu-[Cys(2Lut)-Apc(DOTA)-Leu-Ile-Cys]-APAc-lys:HPD2:HPD4 | 2 | Isopropylidene-HPD2:HPD-aldehyde |
| GIP-1063 | HAC2:HAC:Aur-Asp-Trp-5Clw-Tap-Glu-leu-[Cys(2Lut)-Apc(DOTA)-Leu-Ile-Cys]-APAc-kur:HAC:HAC2 | 4 | Isopropylidene-HAC2:HAC-amine |
| GIP-1064 | HPD2:HAC:Aur-Asp-Trp-5Clw-Tap-Glu-leu-[Cys(2Lut)-Apc(DOTA)-Leu-Ile-Cys]-APAc-kur:HAC:HPD2 | 4 | Isopropylidene-HPD2:HAC-amine |
| GIP-1065 | HAC2:HPD:Aur-Asp-Trp-5Clw-Tap-Glu-leu-[Cys(2Lut)-Amk(DOTA)-Leu-Ile-Cys]-APAc-kur:HPD:HAC2 | 4 | Isopropylidene-HAC2:HPD-amine |
| GIP-1066 | HAC2:HPD:Hyx-Asp-Trp-5Clw-Tap-Glu-leu-[Cys(2Lut)-Amk(DOTA)-Leu-Ile-Cys]-APAc-prx:HPD:HAC2 | 3 | Isopropylidene-HAC2:HPD-azide |
| GIP-1067 | HAC4:HPD2:Ahx-Asp-Trp-5Clw-Tap-Glu-leu-[Cys(2Lut)-Amk(DOTA)-Leu-Ile-Cys]-APAc-lys:HPD2:HAC4 | 2 | Isopropylidene-HAC2:HPD-aldehyde |
| GIP-1068 | HPD2:HPD:Aur-Asp-Trp-5Clw-Tap-Glu-leu-[Cys(2Lut)-Amk(DOTA)-Leu-Ile-Cys]-APAc-kur:HPD:HPD2 | 4 | Isopropylidene-HPD2:HPD-amine |
| GIP-1069 | HPD2:HPD:Hyx-Asp-Trp-5Clw-Tap-Glu-leu-[Cys(2Lut)-Amk(DOTA)-Leu-Ile-Cys]-APAc-prx:HPD:HPD2 | 3 | Isopropylidene-HPD2:HPD-azide |
| GIP-1070 | HPD4:HPD2:Ahx-Asp-Trp-5Clw-Tap-Glu-leu-[Cys(2Lut)-Amk(DOTA)-Leu-Ile-Cys]-APAc-lys:HPD2:HPD4 | 2 | Isopropylidene-HPD2:HPD-aldehyde |
| GIP-1071 | HAC2:HAC:Aur-Asp-Trp-5Clw-Tap-Glu-leu-[Cys(2Lut)-Amk(DOTA)-Leu-Ile-Cys]-APAc-kur:HAC:HAC2 | 4 | Isopropylidene-HAC2:HAC-amine |
| GIP-1072 | HPD2:HAC:Aur-Asp-Trp-5Clw-Tap-Glu-leu-[Cys(2Lut)-Amk(DOTA)-Leu-Ile-Cys]-APAc-kur:HAC:HPD2 | 4 | Isopropylidene-HPD2:HAC-amine |
| GIP-1073 | HAC2:HPD:Aur-Asp-Trp-5Clw-Hyp-Glu-leu-[Cys(2Lut)-Apc(DOTA)-Leu-Ile-Cys]-APAc-kur:HPD:HAC2 | 4 | Isopropylidene-HAC2:HPD-amine |
| GIP-1074 | HAC2:HPD:Hyx-Asp-Trp5Clw-Hyp-Glu-leu-[Cys(2Lut)-Apc(DOTA)-Leu-Ile-Cys]-APAc-prx:HPD:HAC2 | 3 | Isopropylidene-HAC2:HPD-azide |
| GIP-1075 | HAC4:HPD2:Ahx-Asp-Trp-5Clw-Hyp-Glu-leu-[Cys(2Lut)-Apc(DOTA)-Leu-Ile-Cys]-APAc-lys:HPD2:HAC4 | 2 | Isopropylidene-HAC2:HPD-aldehyde |
| GIP-1076 | HPD2:HPD:Aur-Asp-Trp-5Clw-Hyp-Glu-leu-[Cys(2Lut)-Apc(DOTA)-Leu-Ile-Cys]-APAc-kur:HPD:HPD2 | 4 | Isopropylidene-HPD2:HPD-amine |
| GIP-1077 | HPD2:HPD:Hyx-Asp-Trp-5Clw-Hyp-Glu-leu-[Cys(2Lut)-Apc(DOTA)-Leu-Ile-Cys]-APAc-prx:HPD:HPD2 | 3 | Isopropylidene-HPD2:HPD-azide |
| GIP-1078 | HPD4:HPD2:Ahx-Asp-Trp-5Clw-Hyp-Glu-leu-[Cys(2Lut)-Apc(DOTA)-Leu-Ile-Cys]-APAc-lys:HPD2:HPD4 | 2 | Isopropylidene-HPD2:HPD-aldehyde |
| GIP-1079 | HAC2:HAC:Aur-Asp-Trp-5Clw-Hyp-Glu-leu-[Cys(2Lut)-Apc(DOTA)-Leu-Ile-Cys]-APAc-kur:HAC:HAC2 | 4 | Isopropylidene-HAC2:HAC-amine |
| GIP-1080 | HPD2:HAC:Aur-Asp-Trp-5Clw-Hyp-Glu-leu-[Cys(2Lut)-Apc(DOTA)-Leu-Ile-Cys]-APAc-kur:HAC:HPD2 | 4 | Isopropylidene-HPD2:HAC-amine |
| GIP-1081 | HAC2:HPD:Aur-Asp-Trp-5Clw-Hyp-Glu-leu-[Cys(2Lut)-Amk(DOTA)-Leu-Ile-Cys]-APAc-kur:HPD:HAC2 | 4 | Isopropylidene-HAC2:HPD-amine |
| GIP-1082 | HAC2:HPD:Hyx-Asp-Trp-5Clw-Hyp-Glu-leu-[Cys(2Lut)-Amk(DOTA)-Leu-Ile-Cys]-APAc-prx:HPD:HAC2 | 3 | Isopropylidene-HAC2:HPD-azide |
| GIP-1083 | HAC4:HPD2:Ahx-Asp-Trp-5Clw-Hyp-Glu-leu-[Cys(2Lut)-Amk(DOTA)-Leu-Ile-Cys]-APAc-lys:HPD2:HAC4 | 2 | Isopropylidene-HAC2:HPD-aldehyde |
| GIP-1084 | HPD2:HPD:Aur-Asp-Trp-5Clw-Hyp-Glu-leu-[Cys(2Lut)-Amk(DOTA)-Leu-Ile-Cys]-APAc-kur:HPD:HPD2 | 4 | Isopropylidene-HPD2:HPD-amine |
| GIP-1085 | HPD2:HPD:Hyx-Asp-Trp-5Clw-Hyp-Glu-leu-[Cys(2Lut)-Amk(DOTA)-Leu-Ile-Cys]-APAc-prx:HPD:HPD2 | 3 | Isopropylidene-HPD2:HPD-azide |
| GIP-1086 | HPD4:HPD2:Ahx-Asp-Trp-5Clw-Hyp-Glu-leu-[Cys(2Lut)-Amk(DOTA)-Leu-Ile-Cys]-APAc-lys:HPD2:HPD4 | 2 | Isopropylidene-HPD2:HPD-aldehyde |
| GIP-1087 | HAC2:HAC:Aur-Asp-Trp-5Clw-Hyp-Glu-leu-[Cys(2Lut)-Amk(DOTA)-Leu-Ile-Cys]-APAc-kur:HAC:HAC2 | 4 | Isopropylidene-HAC2:HAC-amine |
| GIP-1088 | HPD2:HAC:Aur-Asp-Trp-5Clw-Hyp-Glu-leu-[Cys(2Lut)-Amk(DOTA)-Leu-Ile-Cys]-APAc-kur:HAC:HPD2 | 4 | Isopropylidene-HPD2:HAC-amine |
| GIP-1089 | HAC2:HAC:Ahx-Asp-Trp-5Clw-Tap-Glu-leu-[Cys(2Lut)-Apc(DOTA)-Leu-Ile-Cys]-APAc-lys:HAC:HAC2 | 5 | Isopropylidene-HAC2:HAC-one |
| GIP-1090 | HAC2:HAC:Tzx-Asp-Trp-5Clw-Tap-Glu-leu-[Cys(2Lut)-Apc(DOTA)-Leu-Ile-Cys]-APAc-tzk:HAC:HAC2 | 6 | Isopropylidene-HAC2:HAC-alkyne |
| GIP-1091 | HAC2:HPD:Tzx-Asp-Trp-5Clw-Tap-Glu-leu-[Cys(2Lut)-Apc(DOTA)-Leu-Ile-Cys]-APAc-tzk:HPD:HAC2 | 6 | Isopropylidene-HAC2:HPD-alkyne |
| GIP-1092 | HPD:Aur-Asp-Trp-5Clw-Tap-Glu-leu-[Cys(2Lut)-Apc(DOTA)-Leu-Ile-Cys]-APAc-kur:HPD | 4 | 1 -(2,2-dimethyl-1,3-dioxan-5-yl)methanamine (CAS: 52774-72-8) |
| GIP-1093 | HPD:Hyx-Asp-Trp-5Clw-Tap-Glu-leu-[Cys(2Lut)-Apc(DOTA)-Leu-Ile-Cys]-APAc-prx:HPD | 3 | 2-(azidomethyl)propane-1,3-diol (CAS: 882516-48-5) |
| GIP-1094 | HPD2:HAC:Ahx-Asp-Trp-5Clw-Tap-Glu-leu-[Cys(2Lut)-Apc(DOTA)-Leu-Ile-Cys]-APAc-lys:HAC:HPD2 | 5 | Isopropylidene-HPD2:HAC-one |
| GIP-1095 | HPD2:HPD:Tzx-Asp-Trp-5Clw-Tap-Glu-leu-[Cys(2Lut)-Apc(DOTA)-Leu-Ile-Cys]-APAc-tzk:HPD:HPD2 | 6 | Isopropylidene-HPD2:HPD-alkyne |
| GIP-1096 | HAC:Aur-Asp-Trp-5Clw-Tap-Glu-leu-[Cys(2Lut)-Apc(DOTA)-Leu-Ile-Cys]-APAc-kur:HAC | 4 | 2,2-dimethyl-1,3-dioxan-5-amine (CAS: 40137-24-4) |
| GIP-1097 | HPD2:HAC:Tzx-Asp-Trp-5Clw-Tap-Glu-leu-[Cys(2Lut)-Apc(DOTA)-Leu-Ile-Cys]-APAc-tzk:HAC:HPD2 | 6 | Isopropylidene-HPD2:HAC-alkyne |
| GIP-1098 | HAC2:HAC:Ahx-Asp-Trp-5Clw-Tap-Glu-leu-[Cys(2Lut)-Amk(DOTA)-Leu-Ile-Cys]-APAc-lys:HAC:HAC2 | 5 | Isopropylidene-HAC2:HAC-one |
| GIP-1099 | HAC2:HAC:Tzx-Asp-Trp-5Clw-Tap-Glu-leu-[Cys(2Lut)-Amk(DOTA)-Leu-Ile-Cys]-APAc-tzk:HAC:HAC2 | 6 | Isopropylidene-HAC2:HAC-alkyne |
| GIP-1100 | HAC2:HPD:Tzx-Asp-Trp-5Clw-Tap-Glu-leu-[Cys(2Lut)-Amk(DOTA)-Leu-Ile-Cys]-APAc-tzk:HPD:HAC2 | 6 | Isopropylidene-HAC2:HPD-alkyne |
| GIP-1101 | HPD:Aur-Asp-Trp-5Clw-Tap-Glu-leu-[Cys(2Lut)-Amk(DOTA)-Leu-Ile-Cys]-APAc-kur:HPD | 4 | 1-(2,2-dimethyl-1,3-dioxan-5-yl)methanamine (CAS: 52774-72-8) |
| GIP-1102 | HPD:Hyx-Asp-Trp-5Clw-Tap-Glu-leu-[Cys(2Lut)-Amk(DOTA)-Leu-Ile-Cys]-APAc-prx:HPD | 3 | 2-(azidomethyl)propane-1,3-diol (CAS: 882516-48-5) |
| GIP-1103 | HPD2:HAC:Ahx-Asp-Trp-5Clw-Tap-Glu-leu-[Cys(2Lut)-Amk(DOTA)-Leu-Ile-Cys]-APAc-lys:HAC:HPD2 | 5 | Isopropylidene-HPD2:HAC-one |
| GIP-1104 | HPD2:HPD:Tzx-Asp-Trp-5Clw-Tap-Glu-leu-[Cys(2Lut)-Amk(DOTA)-Leu-Ile-Cys]-APAc-tzk:HPD:HPD2 | 6 | Isopropylidene-HPD2:HPD-alkyne |
| GIP-1105 | HAC:Aur-Asp-Trp-5Clw-Tap-Glu-leu-[Cys(2Lut)-Amk(DOTA)-Leu-Ile-Cys]-APAc-kur:HAC | 4 | 2,2-dimethyl-1,3-dioxan-5-amine (CAS: 40137-24-4) |
| GIP-1106 | HPD2:HAC:Tzx-Asp-Trp-5Clw-Tap-Glu-leu-[Cys(2Lut)-Amk(DOTA)-Leu-Ile-Cys]-APAc-tzk:HAC:HPD2 | 6 | Isopropylidene-HPD2:HAC-alkyne |
| GIP-1107 | HAC2:HAC:Ahx-Asp-Trp-5Clw-Hyp-Glu-leu-[Cys(2Lut)-Apc(DOTA)-Leu-Ile-Cys]-APAc-lys:HAC:HAC2 | 5 | Isopropylidene-HAC2:HAC-one |
| GIP-1108 | HAC2:HAC:Tzx-Asp-Trp-5Clw-Hyp-Glu-leu-[Cys(2Lut)-Apc(DOTA)-Leu-Ile-Cys]-APAc-tzk:HAC:HAC2 | 6 | Isopropylidene-HAC2:HAC-alkyne |
| GIP-1109 | HAC2:HPD:Tzx-Asp-Trp-5Clw-Hyp-Glu-leu-[Cys(2Lut)-Apc(DOTA)-Leu-Ile-Cys]-APAc-tzk:HPD:HAC2 | 6 | Isopropylidene-HAC2:HPD-alkyne |
| GIP-1110 | HPD:Aur-Asp-Trp-5Clw-Hyp-Glu-leu-[Cys(2Lut)-Apc(DOTA)-Leu-Ile-Cys]-APAc-kur:HPD | 4 | 1-(2,2-dimethyl-1,3-dioxan-5-yl)methanamine (CAS: 52774-72-8) |
| GIP-1111 | HPD:Hyx-Asp-Trp-5Clw-Hyp-Glu-leu-[Cys(2Lut)-Apc(DOTA)-Leu-Ile-Cys]-APAc-prx:HPD | 3 | 2-(azidomethyl)propane-1,3-diol (CAS: 882516-48-5) |
| GIP-1112 | HPD2:HAC:Ahx-Asp-Trp-5Clw-Hyp-Glu-leu-[Cys(2Lut)-Apc(DOTA)-Leu-Ile-Cys]-APAc-lys:HAC:HPD2 | 5 | Isopropylidene-HPD2:HAC-one |
| GIP-1113 | HPD2:HPD:Tzx-Asp-Trp-5Clw-Hyp-Glu-leu-[Cys(2Lut)-Apc(DOTA)-Leu-Ile-Cys]-APAc-tzk:HPD:HPD2 | 6 | Isopropylidene-HPD2:HPD-alkyne |
| GIP-1114 | HAC:Aur-Asp-Trp-5Clw-Hyp-Glu-leu-[Cys(2Lut)-Apc(DOTA)-Leu-Ile-Cys]-APAc-kur:HAC | 4 | 2,2-dimethyl-1,3-dioxan-5-amine (CAS: 40137-24-4) |
| GIP-1115 | HPD2:HAC:Tzx-Asp-Trp-5Clw-Hyp-Glu-leu-[Cys(2Lut)-Apc(DOTA)-Leu-Ile-Cys]-APAc-tzk:HAC:HPD2 | 6 | Isopropylidene-HPD2:HAC-alkyne |
| GIP-1116 | HAC2:HAC:Ahx-Asp-Trp-5Clw-Hyp-Glu-leu-[Cys(2Lut)-Amk(DOTA)-Leu-Ile-Cys]-APAc-lys:HAC:HAC2 | 5 | Isopropylidene-HAC2:HAC-one |
| GIP-1117 | HAC2:HAC:Tzx-Asp-Trp-5Clw-Hyp-Glu-leu-[Cys(2Lut)-Amk(DOTA)-Leu-Ile-Cys]-APAc-tzk:HAC:HAC2 | 6 | Isopropylidene-HAC2:HAC-alkyne |
| GIP-1118 | HAC2:HPD:Tzx-Asp-Trp-5Clw-Hyp-Glu-leu-[Cys(2Lut)-Amk(DOTA)-Leu-Ile-Cys]-APAc-tzk:HPD:HAC2 | 6 | Isopropylidene-HAC2:HPD-alkyne |
| GIP-1119 | HPD:Aur-Asp-Trp-5Clw-Hyp-Glu-leu-[Cys(2Lut)-Amk(DOTA)-Leu-Ile-Cys]-APAc-kur:HPD | 4 | 1-(2,2-dimethyl-1,3-dioxan-5-yl)methanamine (CAS: 52774-72-8) |
| GIP-1120 | HPD:Hyx-Asp-Trp-5Clw-Hyp-Glu-leu-[Cys(2Lut)-Amk(DOTA)-Leu-Ile-Cys]-APAc-prx:HPD | 3 | 2-(azidomethyl)propane-1,3-diol (CAS: 882516-48-5) |
| GIP-1121 | HPD2:HAC:Ahx-Asp-Trp-5Clw-Hyp-Glu-leu-[Cys(2Lut)-Amk(DOTA)-Leu-Ile-Cys]-APAc-lys:HAC:HPD2 | 5 | Isopropylidene-HPD2:HAC-one |
| GIP-1122 | HPD2:HPD:Tzx-Asp-Trp-5Clw-Hyp-Glu-leu-[Cys(2Lut)-Amk(DOTA)-Leu-Ile-Cys]-APAc-tzk:HPD:HPD2 | 6 | Isopropylidene-HPD2:HPD-alkyne |
| GIP-1123 | HAC:Aur-Asp-Trp-5Clw-Hyp-Glu-leu-[Cys(2Lut)-Amk(DOTA)-Leu-Ile-Cys]-APAc-kur:HAC | 4 | 2,2-dimethyl-1,3-dioxan-5-amine (CAS: 40137-24-4) |
| GIP-1124 | HPD2:HAC:Tzx-Asp-Trp-5Clw-Hyp-Glu-leu-[Cys(2Lut)-Amk(DOTA)-Leu-Ile-Cys]-APAc-tzk:HAC:HPD2 | 6 | Isopropylidene-HPD2:HAC-alkyne |
| GIP-1125 | HAC:Ahx-Asp-Trp-5Clw-Tap-Glu-leu-[Cys(2Lut)-Apc(DOTA)-Leu-Ile-Cys]-APAc-lys:HAC | 5 | 2,2-Dimethyl-1,3-dioxan-5-one (CAS: 74181-34-3) |
| GIP-1126 | HAC:Hyx-Asp- Trp-5Clw- Tap-Glu-leu-[Cys(2Lut)-Apc(DOTA)-Leu-Ile-Cys]-APAc-prx:HAC | 3 | 2-azidopropane-1,3 -diol (CAS: 92948-99-7) |
| GIP-1127 | HAC:Ahx-Asp-Trp-5Clw-Tap-Glu-leu-[Cys(2Lut)-Amk(DOTA)-Leu-Ile-Cys]-APAc-lys:HAC | 5 | 2,2-Dimethyl-1,3-dioxan-5-one (CAS: 74181-34-3) |
| GIP-1128 | HAC:Hyx-Asp-Trp-5Clw-Tap-Glu-leu-[Cys(2Lut)-Amk(DOTA)-Leu-Ile-Cys]-APAc-prx:HAC | 3 | 2-azidopropane-1,3-diol (CAS: 92948-99-7) |
| GIP-1129 | HAC:Ahx-Asp-Trp-5Clw-Hyp-Glu-leu-[Cys(2Lut)-Apc(DOTA)-Leu-Ile-Cys]-APAc-lys:HAC | 5 | 2,2-Dimethyl-1,3-dioxan-5-one (CAS: 74181-34-3) |
| GIP-1130 | HAC:Hyx-Asp-Trp-5Clw-Hyp-Glu-leu-[Cys(2Lut)-Apc(DOTA)-Leu-Ile-Cys]-APAc-prx:HAC | 3 | 2-azidopropane-1,3-diol (CAS: 92948-99-7) |
| GIP-1131 | HAC:Ahx-Asp-Trp-5Clw-Hyp-Glu-leu-[Cys(2Lut)-Amk(DOTA)-Leu-Ile-Cys]-APAc-lys:HAC | 5 | 2,2-Dimethyl-1,3-dioxan-5-one (CAS: 74181-34-3) |
| GIP-1132 | HAC:Hyx-Asp-Trp-5Clw-Hyp-Glu-leu-[Cys(2Lut)-Amk(DOTA)-Leu-Ile-Cys]-APAc-prx:HAC | 3 | 2-azidopropane-1,3-diol (CAS: 92948-99-7) |

### Example 7: FACS Binding Assay

In order to determine binding of compounds according to the present invention to GIPR-expressing cells, a FACS binding assay is established.

CHO-K1-hGIPR cells (GenScript Biotech, #M00486) are cultured in Ham's F12 medium (Sigma-Aldrich, #N4888) including 10% fetal calf serum (FCS), 2 mM L-glutamine, 100 U/mL penicillin, 100 µg/mL streptomycin, 200µg/mL Zeocin and 100µg/mL HygromycinB. Cells are detached with Accutase (Biolegend, #BLD-423201) and washed in FACS buffer (PBS including 1% FCS). Cells are diluted in FACS buffer to a final concentration of 500.000 cells per mL. 200 µL of the cell suspension is transferred to a u-shaped non-binding 96-well plate (Greiner) and cells are washed in ice-cold FACS buffer.

For EC₅₀ determination, cells are incubated with various concentrations of biotinylated compound (H-Met-Tyr-Asp-Trp-Trp-Pro-Glu-Leu-[Cys(3MeBn)-Ala-Leu-Ile-Cys]-Ttds-Lys(Bio)-NH₂) (SEQ ID NO: 3) at 4°C for 1 hour. The determined pEC₅₀ value of the biotinylated compound was 8.5. For IC₅₀ determination, cells are incubated with 20 nM of the biotinylated compound in the presence of increasing concentrations of non-labeled test compounds at 4°C for 1 hour. Cells are washed twice in FACS buffer. An additional incubation step with 1 µg/mL APC-Streptavidin (Miltenyi; #130-106-791) in 50 µL FACS buffer for 30 min on ice is performed followed by two washes with FACS buffer.

Cells are analyzed in an Attune NxT flow cytometer. Median fluorescence intensities (MFI) of the APC/Cy5-channel are calculated by Attune NxT software. MFI values are plotted against peptide concentration and four parameter logistic (4PL) curve fitting and EC₅₀/pEC₅₀ or IC₅₀/pIC₅₀ calculations are performed using ActivityBase software. Compounds GIP-1001 to GIP-1132 perform excellent in the FACS binding assay.

### Example 8: Surface Plasmon Resonance (SPR) based Binding Assay

Surface plasmon resonance (SPR) studies are performed using a Biacore^{™} T200 SPR system. Briefly, polarized light is directed towards a gold-labeled sensor surface, and minimum intensity reflected light is detected. The angle of reflected light changes as molecules bind and dissociate.

Biotinylate extracellular of the human GIP receptor (bioGIPR, Proteros biostructures GmbH, lot#02/2b) is captured on a biotin capture chip (Biotin CAPture Kit, Series S, GE Healthcare, Cat#28-9202-34). bioGIPR is diluted in Running Buffer (HBST from Xantec Analytics; 150 nM NaCl, 0.05% Tween20, 0.1% DMSO) to a final concentration 100 nM and than flushed over the Fc-capture chip to immobilized ~3500 RUs.

Stock solutions of test compounds are prepared by dissolving each compound in DMSO. DMSO stock solution are diluted 1: 1000 in Running Buffer without DMSO. Further sequential dilutions are made with Running Buffer containing 0.1% DMSO. SPR binding analyses are performed in Single Cycle Kinetic (SCK) mode at 25°C. Flow cell without immobilized bioGIPR serves as reference flowcell. After each SCK run, bioGIRP and the Biotin Capture Reagent are removed with Regeneration Solution (6 M Guanidium, 0.75 M NaOH).

In between every three SCK measurements, a blank run with Running Buffer instead of test compound is included to correct for baseline drifts (double blanking method).

Table 9 describes the protocol steps for bioGIPR capturing and assessment of the binding kinetics.

**Table 9: SPR protocol steps with bioGIPR.**

| **Step** | **Injected solution** | **Contact time** | **Flow rate** |
|---|---|---|---|
| Startup cycle (3x): | Running Buffer | 60 s | 30 µL/min |
| Washing & surface regeneration | 6 M Guanidin-HCl | 5 s | |
| Immobilize Biotin Capture Reagent (Streptavidin-oligo fusion) | Biotin Capture Reagent | 300 s | 2 µL/min |
| Wash | Running Buffer | 60 s | 30 µL/min |
| Capture bioGIPR | bioGIPR (100 nM) | 300 s | 10 µL/min |
| 1. Binding kinetics of test compound | Dilution no. 5 (0.78 nM) | 120 s | 30 µL/min |
| 2. Binding kinetics of test compound | Dilution no. 4 (3.125 nM) | 120 s | 30 µL/min |
| 3. Binding kinetics of test compound | Dilution no. 3 (12.5 nM) | 120 s | 30 µL/min |
| 4. Binding kinetics of test compound | Dilution no. 2 (50 nM) | 120 s | 30 µL/min |
| 5. Binding kinetics of test compound | Dilution no. 1 (100 nM) | 120 s | 30 µL/min |
| Dissociation cycle | Running Buffer | 2400 s | 30 µL/min |
| Regeneration (2x) | Regeneration solution | 120 s | 5 µL/min |

For each test compound, SPR raw data in the form of resonance units (RU) is plotted as sensorgram using the Biacore^{™} T200 control software. The signal from the blank sensorgram is subtracted from that of the test compound sensorgram (blank corrected). The blank corrected sensorgram is corrected for baseline drift by subtracting the sensorgram of a SCK run without the test compound (running buffer only). The association rate (kₒₙ), dissociation rate (k_{off}), dissociation constant (K_{D}), and t_{1/2} are calculated from blank corrected SPR data using the 1: 1 Langmuir binding model from the Biacore^{™} T200 evaluation software. Raw data and fit results are imported as text files in IDBS. The pK_{D} value (negative decadic logarithm of dissociation constant) is calculated in the IDBS excel template.

Compounds GIP-1001 to GIP-1132 perform excellent in the Surface Plasmon Resonance (SPR) based Binding Assay with respect to pK_{D} and t_{1/2} values.

### Example 9: Plasma stability assay

In order to determine the stability of selected compounds of the invention in human and mouse plasma, a plasma stability assay is carried out. Such plasma stability assay measures degradation of compounds of the present invention in blood plasma. This is an important characteristic of a compound as compounds, with the exception of pro-drugs, which rapidly degrade in plasma, generally show poor *in vivo* efficacy.

The plasma stability samples are prepared by spiking 50 µL plasma aliquots (all K₂EDTA) with 1 µL of a 0.5 mM compound stock solution in DMSO. After vortexing the samples are incubated in a Thermomixer at 37°C for 0, 4 and 24 hours. After incubation the samples are stored on ice until further treatment. All samples are prepared in duplicates.

A suitable internal standard is added to each sample (1 µL of a 0.5 mM stock solution in DMSO). Protein precipitation is performed by addition of 250 µL of acetonitrile containing 1% trifluoroacetic acid. After incubation at room temperature for 30 min the precipitate is separated by centrifugation and 150 µL of the supernatant is diluted with 150 µL of 1% aqueous formic acid.

The determination of the analyte in the clean sample solutions is performed on an Agilent 1290 UHPLC system coupled to an Agilent 6530 Q-TOF mass spectrometer. The chromatographic separation is carried out on a Phenomenex BioZen XB-C18 HPLC column (50 x 2 mm, 1.7 µm particle size) with gradient elution using a mixture of 0.1% formic acid in water as eluent A and acetonitrile as eluent B (2% B to 41% in 7 min, 800 µL/min, 40°C). Mass spectrometric detection is performed in positive ion ESI mode by scanning the mass range from m/z 50 to 3000 with a sampling rate of 2 / sec.

From the mass spectrometric raw data, the ion currents for the double or triple charged monoisotopic signal is extracted for both, the compound and the internal standard.

Quantitation is performed by external matrix calibration with internal standard using the integrated analyte signals.

Additionally, recovery is determined by spiking a pure plasma sample that only contains the internal standard after treatment with a certain amount of the compound.

Carry-over is evaluated by analysis of a blank sample (20% acetonitrile) after the highest calibration sample.

The results show that compounds selected from the compounds GIP-1001 to GIP-1132 are highly stable in human and mouse plasma. The stability is sufficient for the diagnostic, therapeutic and theragnostic use of these compounds according to the present invention.

### Example 9: Proteolytic stability assay against NEP

Peptides are often sensitive to proteolytic cleavage in blood (Werle et al., Amino Acids, 2006, 30, 351-367). If peptides degrade rapidly, in vivo elimination could be dominated by metabolism. Since metabolites often demonstrate poor binding to the target, performance of the compound during radiopharmaceutical use (diagnostic or therapeutic) can be significantly decreased. Therefore, evaluating the stability of the compound against proteases in the early stages of compound development is essential.

Two classes of proteases and peptidases can be found in the blood circulation: soluble and membrane-bound proteases. The conduction of a plasma stability study only covers the impact of the soluble fraction on compound stability, since all membrane-bound proteases are separated during blood collection (proteins expressed on the surface of blood vessels) or during plasma generation (proteins expressed on blood cells). Thus, the result of a plasma stability assay is not always predictive for in vivo behaviour.

Membrane-bound peptidases are mainly responsible for the activation and deactivation of bioactive peptides, such as hormones, cytokines, and neurohormones (Antczak et al., Bioessays, 2001, 23, 251-260). They activate hormones by cleavage of a pre-hormone and deactivate them through another cleavage step. Activation is usually very specific, as well as the activation peptidases, including ACE, ECE, and DPP-IV. With a specific activation, signaling remains specific even if deactivation is more unspecific. Thus, several peptidases that are mainly responsible for hormone deactivation reveal a broad substrate pattern (e.g. neutral endopeptidase, meprines). It is not possible to assess all peptidases during early compound development. Therefore, more importance should be focused on those peptidases with broad substrate patterns, since it is more likely that the peptide sequence fits into this pattern.

NEP (neutral endopeptidase, EC 3.4.24.11, CD10, CALLA, endopeptidase 24.11, enkephalinase, neprilysin, membrane metallopeptidase A) is a membrane-bound metallopeptidase. It is expressed on neutrophils and highly active in blood (Antczak et al., Bioessays, 2001, 23, 251-260). In addition, the cleavage pattern of NEP is very broad, covering many different peptide hormones, with more than 50 different natural substrates already described (Bayes-Genis et al., Journal of the American College of Cardiology, 2016, 68, 639-653). NEP preferably cleaves amide bonds between a hydrophilic and hydrophobic amino acid (preferably leucine or phenylalanine), even in small cyclic peptides such as CNP. Plamboeck et al. (2005) showed a significant impact of NEP caused cleavage on the pharmacokinetic behavior of peptides (Plamboeck et al., Diabetologia, 2005, 48, 1882-1890).

To assess the stability of compounds against NEP, the compounds are subjected to an in vitro assay based on the protocol described by Edelson et al. (Edelson et al., Pulmonary pharmacology & therapeutics, 2013, 26, 229-238).

Recombinant soluble human NEP (BioTechne, Wiesbaden, Germany) at a concentration of 100 ng/mL is mixed with the compound (10 µM) and a stable internal standard (10 µM) and incubated at 37°C. After several time points, samples are taken and analyzed with LC-MS.

The determination of the analyte in the clean sample solutions is performed on an Agilent 1290 UHPLC system coupled to an Agilent 6530 Q-TOF mass spectrometer. The chromatographic separation is carried out on a Phenomenex BioZen XB-C18 HPLC column (50 x 2 mm, 1.7 µm particle size) with gradient elution using a mixture of 0.1% formic acid in water as eluent A and acetonitrile as eluent B (2% B to 41% in 7 min, 800 µL/min, 40°C). Mass spectrometric detection is performed in positive ion ESI mode by scanning the mass range from m/z 50 to 3000 with a sampling rate of 2 / sec.

From the mass spectrometric raw data the ion currents for the double or triple charged monoisotopic signal are extracted for both, the compound and the internal standard.

Quantitation is performed by external matrix calibration with internal standard using the integrated analyte signals.

The activity of NEP is assured using a commercially available chromogenic substrate of NEP.

The results show that compounds selected from the compounds GIP-1001 to GIP-1132 are stable against NEP degradation and are considered as stable enough for diagnostic and therapeutic applications.

### Example 10: ¹¹¹In- and ¹⁷⁷Lu-labeling of selected compounds

In order to serve as a diagnostically, therapeutically, or theragnostically active agent, a compound needs to be labeled with a radioactive isotope. The labeling procedure needs to be appropriate to ensure a high radiochemical yield and purity of the radiolabeled compound of the invention.

30-105 MBq of ¹¹¹InCl₃ (in 0.02 M HCl) are mixed with 1 nmol of compound (200 µM stock solution in 0.1 M HEPES pH 7) per 30 MBq and buffer (A: 1 M sodium acetate buffer pH 5, containing 20 mg/mL gentisic acid, B: 1 M sodium acetate / ascorbic acid buffer pH 5 containing 25 mg/mL methionine or C: 1 M sodium acetate buffer pH 5 containing 25 mg/mL methionine) at a final buffer concentration of 0.1 M. The mixture is heated to 80 °C for 25 min. After cooling down, DTPA and TWEEN-20 are added at a final concentration of 0.2 mM and 0.1%, respectively (Formulation A). In some instances, 20 µL of a 200 mg/mL ascorbic acid solution per 100 µL reaction mixture are additionally added at the end of synthesis (Formulation B).

2.0 ± 0.1 GBq ¹⁷⁷LuCl₃ (in 0.04 M HCl) are mixed with 1 nmol of compound (200 µM stock solution in 0.1 M HEPES pH 7) per 60 - 90 MBq and buffer (1 M sodium acetate / ascorbic acid buffer pH 5 containing 25 mg/mL methionine) at a final buffer concentration of ~0.4 M.

The mixture is heated for 20 min at 90 °C. After cooling down, DTPA and TWEEN-20 are added at a final concentration of 0.2 mM and 0.1%, respectively (Formulation A). In some instances, 9 volumes of a 100 mg/mL ascorbic acid solution containing 5 mg/mL methionine and 0.8 mM DTPA are added instead (Formulation C).

Radiochemical purity is analyzed by HPLC. 5 µL of diluted labeling solution are analyzed with a Poroshell SB-C18 2.7 µm (Agilent). Eluent A: H₂O, 0.1 % TFA, eluent B: MeCN; gradient from 5 % B to 70 % B within 15 min, flow rate 0.5 mL/min; detector: Nal (Raytest), DAD 230 nm. The peak eluting with the dead volume represents free radionuclide, the peak eluting with the peptide-specific retention time as determined with an unlabeled sample represents radiolabeled compound.

### REFERENCES

The disclosure of each and any references recited herein is incorporated herein by reference.

## Claims

1. A compound comprising a cyclic peptide of formula (Ia) 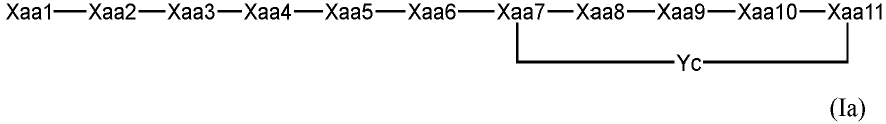
or a cyclic peptide of formula (Ib) 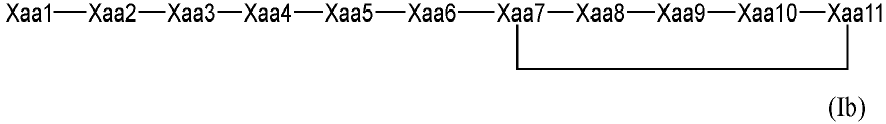
each comprising an N-terminal modification group A attached to Xaa1,
and each comprising a C-terminal group C-term attached to Xaa11,
wherein
the peptide sequence is drawn from left to right in N- to C-terminal direction,
the N-terminal modification group A comprises a Z group, wherein a linker is optionally interspersed between the Z group and Xaa1, wherein the Z group comprises a bio-distribution modifier,
Xaa1 is a residue of an α-amino acid comprising a side chain, wherein the side chain comprises an acidic or polar group,
wherein the carbonyl group of Xaa1 is covalently attached to the α-nitrogen atom of Xaa2,
Xaa2 is a residue of an α-amino acid comprising a side chain, wherein the side chain comprises a heteroaryl ring or an aryl ring, preferably Xaa2 is a residue of an α-amino acid with a side chain comprising a heteroaryl ring,
wherein the carbonyl group of Xaa2 is covalently attached to the α-nitrogen atom of Xaa3,
Xaa3 is a residue of an α-amino acid comprising a side chain, wherein the side chain comprises a heteroaryl ring or an aryl ring, preferably Xaa3 is a residue of an α-amino acid comprising a side chain, wherein the side chain comprises a heteroaryl ring,
wherein the carbonyl group of Xaa3 is covalently attached to the α-nitrogen atom of Xaa4,
Xaa4 is a residue of an α-amino acid optionally comprising a Z group or is a residue of an N-alkylated α-amino acid optionally comprising a Z group, wherein the Z group is a chelator or a bio-distribution modifier,
wherein the carbonyl group of Xaa4 is covalently attached to the α-nitrogen atom of Xaa5,
Xaa5 is a residue of an α-amino acid optionally comprising a Z group, wherein the Z group is a chelator or a bio-distribution modifier,
wherein the carbonyl group of Xaa5 is covalently attached to the α-nitrogen atom of Xaa6,
Xaa6 is a residue of an α-amino acid comprising a side chain, wherein the side chain comprises a branched aliphatic group,
wherein the carbonyl group of Xaa6 is covalently attached to the α-nitrogen atom of Xaa7,
Xaa7 is a residue of an α-amino acid comprising a side chain, wherein the side chain comprises a nucleophilic group, wherein the nucleophilic group comprises a heteroatom, wherein the heteroatom is selected from the group comprising a sulfur atom and a nitrogen atom,
wherein the carbonyl group of Xaa7 is covalently attached to the α-nitrogen atom of Xaa8,
Xaa8 is a residue of an α-amino acid optionally comprising a Z group, wherein the Z group is a chelator or a bio-distribution modifier,
wherein the carbonyl group of Xaa8 is covalently attached to the α-nitrogen atom of Xaa9,
Xaa9 is a residue of an α-amino acid,
wherein the carbonyl group of Xaa9 is covalently attached to the α-nitrogen atom of XaalO,
XaalO is a residue of an α-amino acid comprising a side chain, wherein the side chain comprises a branched aliphatic group or an unsubstituted aromatic ring,
wherein the carbonyl group of XaalO is covalently attached to the α-nitrogen atom of Xaa11,
Xaa11 is a residue of an α-amino acid comprising a side chain, wherein the side chain comprises a nucleophilic group, wherein the nucleophilic group comprises a heteroatom, wherein the heteroatom is selected from the group comprising a sulfur atom and a nitrogen atom,
the C-terminal modification C-term comprises a Z group, wherein a linker is optionally interspersed between the Xaa11 and the Z group, wherein the Z group comprises a bio-distribution modifier,
wherein Xaa7 and Xaa11 are indirectly linked or directly covalently linked to each other forming a macrocyclic ring,
wherein if Xaa7 and Xaa11 are indirectly linked forming a cyclic peptide of formula (Ia), the heteroatom of Xaa7 and the heteroatom of Xaa11 are linked through a ring interspersed between the heteroatom of Xaa7 and the heteroatom of Xaa11 forming Yc, wherein the ring is a heteroaryl ring, an aryl ring optionally comprising a Z group or a heterocyclic ring, wherein the heteroatom is each and individually selected from the group consisting of a sulfur atom and a nitrogen atom, wherein if the heteroatom of Xaa7 is a sulfur atom, a thioether bond covalently links Xaa7 to the ring, if the heteroatom of Xaa7 is a nitrogen atom, an amine bond or an amide bond covalently links Xaa7 to the ring, if the heteroatom of Xaa11 is a sulfur atom, a thioether bond covalently links Xaa11 to the ring, and if the heteroatom of Xaa11 is a nitrogen atom, an amine bond or an amide bond covalently links Xaa11 to the ring,
or wherein if Xaa7 and Xaa11 are directly covalently linked forming a cyclic peptide of formula (Ib), the heteroatom of Xaa7 is a sulfur atom and the heteroatom of Xaa11 is a sulfur atom forming a disulfide bond,
and wherein each and any of the bio-distribution modifiers of the N-terminal modification group A, Xaa4, Xaa5 and Xaa8 comprises a residue of an aliphatic carboxylic acid,
wherein to an ω-carbon atom of the aliphatic carboxylic acid a nitrogen atom, a triazolyl moiety, a hydroxymethyl-triazolyl moiety or an urea moiety is covalently attached,
wherein to the nitrogen atom, to the triazolyl moiety, to the hydroxymethyl triazolyl moiety or to the urea moiety
(a) 1 or 2 first level alkyl groups are covalently attached, wherein each and any first level alkyl group individually and independently comprises 2 or more hydroxyl groups, wherein each and any hydroxyl group is a primary hydroxyl group,
or (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group
wherein, if the bio-distribution modifier is comprised by the N-terminal modification group A,
(a) the carbonyl group of the aliphatic carboxylic acid of the bio-distribution modifier is covalently attached to the α-nitrogen atom of Xaa1,
or (b) if a linker comprising an amino group and a carbonyl group is interspersed between the bio-distribution modifier and Xaa1 the carbonyl group of the aliphatic carboxylic acid of the bio-distribution modifier is covalently attached to the amino group of the linker and the carbonyl group of the linker is covalently attached to the α-nitrogen atom of Xaa1,
or wherein, if the bio-distribution modifier is comprised by Xaa4 comprising an amino group in the side chain, the carbonyl group of the aliphatic carboxylic acid of the bio-distribution modifier is covalently attached to the amino group of the side chain of Xaa4,
or wherein, if the bio-distribution modifier is comprised by Xaa5 comprising an amino group in the side chain, the carbonyl group of the aliphatic carboxylic acid of the bio-distribution modifier is covalently attached to the amino group of the side chain of Xaa5,
or wherein, if the bio-distribution modifier is comprised by Xaa8 comprising an amino group in the side chain, the carbonyl group of the aliphatic carboxylic acid of the bio-distribution modifier is covalently attached to the amino group of the side chain of Xaa8, and
wherein the bio-distribution modifier comprised by the C-terminal modification group C-term comprises a residue of an α-amino acid with an aliphatic side chain,
wherein to an ω-carbon atom of the side chain of the α-amino acid a nitrogen atom, a triazolyl moiety, a hydroxymethyl-triazolyl moiety or an urea moiety is covalently attached,
wherein to the nitrogen atom, to the triazolyl moiety, to the hydroxymethyl triazolyl moiety or to the urea moiety
(a) 1 or 2 first level alkyl groups are covalently attached, wherein each and any first level alkyl group individually and independently comprises 2 or more hydroxyl groups, wherein each and any hydroxyl group is a primary hydroxyl group,
or (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
and wherein
(a) the carbonyl group of Xaa11 is covalently attached to the α-nitrogen atom of the α-amino acid of the bio-distribution modifier,
or (b) if a linker comprising an amino group and a carbonyl group is interspersed between the Xaa11 and the bio-distribution modifier, the carbonyl group of Xaa11 is covalently attached to the amino group of the linker and the carbonyl group of the linker is covalently attached to the α-nitrogen atom of the α-amino acid,
and wherein to the carbonyl group of the α-amino acid of the bio-distribution modifier an amino group is covalently attached thereby forming an amide.

2. The compound of claim 1,
wherein
Xaa1 is a residue of an α-amino acid comprising a side chain, wherein the side chain comprises an acidic or polar group, preferably Xaa1 is a residue of an α-amino acid of formulae (IIa), (IIb) or (IIc), more preferably Xaa1 is a residue of an α-amino acid of formula (IIa), 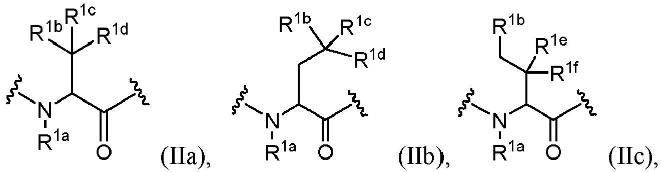
wherein
the carbonyl group of the aliphatic carboxylic acid comprised by the bio-distribution modifier comprised by the N-terminal modification group A is covalently attached to the α-nitrogen atom of Xaa1, or if a linker, comprising an amino group and a carbonyl group is interspersed between the bio-distribution modifier comprised by the N-terminal modification group A and Xaa1, the carbonyl group of the linker is covalently attached to the α-nitrogen atom of Xaa1,
R^{1a} is selected from the group consisting of H and (C₁-C₄)alkyl,
R^{1b} is selected from the group consisting of CO₂H, SO₃H, OPO₃H₂, CONH₂ and OH,
R^{1c} is selected from the group consisting of H, OH and (C₁-C₂)alkyl under the proviso that R^{1b} is selected from the group consisting of CO₂H, SO₃H and CONH₂,
or is selected from the group consisting of H and (C₁-C₂)alkyl under the proviso that R^{1b} is selected from the group consisting of OH and OPO₃H₂,
R^{1d} is selected from the group consisting of H and (C₁-C₂)alkyl, preferably if R^{1c} is (C₁-C₂)alkyl, R^{1d} is the same (C₁-C₂)alkyl,
R^{1e} is selected from the group consisting of H, OH and (C₁-C₂)alkyl,
R^{1f} is selected from the group consisting of H and (C₁-C₂)alkyl, preferably if R^{1e} is (C₁-C₂)alkyl, R^{1f} is the same (C₁-C₂)alkyl,
and the carbonyl group of Xaa1 is covalently attached to the α-nitrogen atom of Xaa2,
Xaa2 is (a) a residue of an α-amino acid comprising a side chain, wherein the side chain comprises a heteroaryl ring or an aryl ring, preferably Xaa2 is a residue of an α-amino acid of formulae (IIIa) or (IIIb), more preferably a residue of an α-amino acid of formula (IIIa), 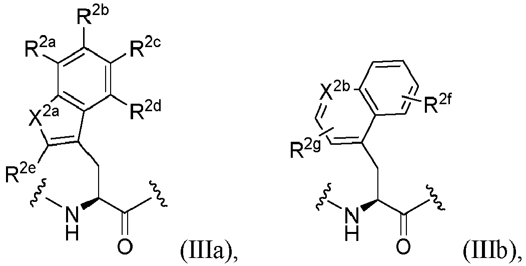
wherein
X^{2a} is selected from the group consisting of NH, NCH₃ and S, preferably selected from the group consisting of NH and S,
X^{2b} is selected from the group consisting of N, C-H, C-F and C-CH₃, preferably selected from the group consisting of N and C-H,
R^{2a} is selected from the group consisting of H, a halogen, methyl and OCH₃, preferably R^{2a} is selected from the group consisting of H and F,
R^{2b} is selected from the group consisting of H, a halogen, methyl and OCH₃, preferably the halogen is selected from the group consisting of Cl and Br, more preferably R^{2b} is selected from the group consisting of H, Cl and Br,
R^{2c} is selected from the group consisting of H, OH, a halogen, methyl and OCH₃, preferably the halogen is selected from the group consisting of F, Cl and Br, more preferably R^{2c} is selected from the group consisting of H, F and OH,
R^{2d} is selected from the group consisting of H, a halogen, methyl and OCH₃, wherein the halogen is selected from the group consisting of F, Cl and Br, preferably R^{2d} is H,
R^{2e} is selected from the group consisting of H, a halogen and methyl, preferably the halogen is Cl, more preferably R^{2e} is H,
R^{2f} is selected from the group consisting of H and OCH₃, preferably R^{2f} is H,
R^{2g} is selected from the group consisting of H, OH, methyl and OCH₃, preferably R^{2g} is H,
or (b), under the proviso that Xaa3 is of formulae (IVa) or (IVb), preferably under the proviso that Xaa3 is of formula (IVa), Xaa2 is a residue of an α-amino acid comprising a side chain, wherein the side chain comprises an aryl ring, preferably Xaa2 is a residue of an α-amino acid of formula (IIIc) 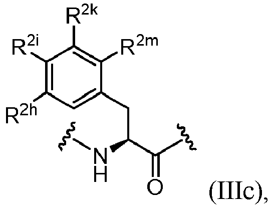
wherein
R^{2h} is selected from the group consisting of H, methyl, CF₃, OH, OCH₃, OCF₃, CONH₂ and a halogen, preferably the halogen is selected from the group consisting of Cl and Br, more preferably R^{2h} is H, Cl or Br,
R²ⁱ is selected from the group consisting of H, methyl, C(CH₃)₃, CF₃, OH, OCH₃, OCH₂CH₃, OCF₃, CONH₂, CO₂H and a halogen, preferably the halogen is selected from the group consisting of Cl and Br, more preferably R²ⁱ is selected from the group consisting of H, Cl and Br,
R^{2k} is selected from the group consisting of H, methyl, CF₃, OH, OCH₃, OCF₃, CONH₂ and a halogen, preferably the halogen is selected from the group consisting of Cl and Br, more preferably R^{2k} is selected from the group consisting of H, Cl and Br,
R^{2m} is selected from the group consisting of H, methyl, CF₃, OH, OCH₃, OCF₃, CONH₂ and a halogen, preferably the halogen is selected from the group consisting of Cl and Br, more preferably R^{2m} is selected from the group consisting of H, Cl and Br,
and the carbonyl group of Xaa2 is covalently attached to the α-nitrogen atom of Xaa3,
Xaa3 is (a) a residue of an α-amino acid comprising a side chain, wherein the side chain comprises a heteroaryl ring or an aryl ring, preferably Xaa3 is a residue of an α-amino acid of formulae (IVa) or (IVb), more preferably Xaa3 is a residue of an α-amino acid of formula (IVa), 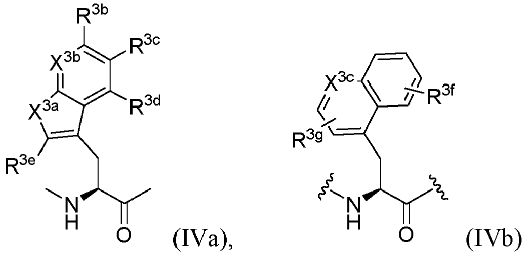
wherein
X^{3a} is selected from the group consisting of NH, NCH₃ and S, preferably X^{3a} is selected from the group consisting of NH and S,
X^{3b} is selected from the group consisting of C-H, C-F, C-Cl, C-Br, C-CH₃, C-OCH₃ and N, preferably X^{3b} is selected from the group consisting of C-H and C-F,
X^{3c} is selected from the group consisting of C-H and N, preferably X^{3c} is C-H,
R^{3b} is selected from the group consisting of H, a halogen, methyl and OCH₃, preferably the halogen is selected from the group consisting of F and Cl, more preferably R^{3b} is selected from the group consisting of H and F,
R^{3c} is selected from the group consisting of H, a halogen, methyl and OCH₃, preferably the halogen is selected from the group consisting of Cl, Br and F, more preferably R^{3c} is selected from the group consisting of Cl and Br,
R^{3d} is selected from the group consisting of H, a halogen, methyl and OCH₃, preferably the halogen is F,
R^{3e} is selected from the group consisting of H, a halogen and methyl, preferably the halogen is Cl,
R^{3f} is selected from the group consisting of H and OCH₃,
R^{3g} is selected from the group consisting of H, F, OH, methyl and OCH₃,
or (b) under the proviso that Xaa2 is an amino acid residue of formula (IIIa), Xaa3 is a residue of an α-amino acid comprising a side chain, wherein the side chain comprises an aryl ring, preferably Xaa3 is a residue of an α-amino acid of formulae (IVc) or (IVd) 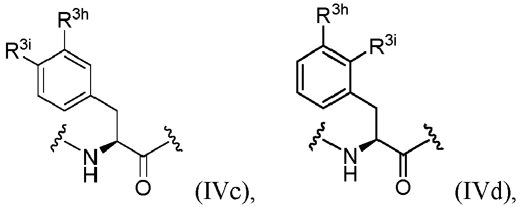
wherein
R^{3h} is selected from the group consisting of a halogen, OH, methyl, CF₃, OCH₃, OCF₃ and CONH₂, preferably the halogen is selected from the group consisting of Cl and Br,
R³¹ is selected from the group consisting of a halogen, OH, methyl, CF₃, OCH₃, OCF₃ and CONH₂, preferably the halogen is selected from the group consisting of Cl and Br,
and the carbonyl group of Xaa3 is covalently attached to the α-nitrogen atom of Xaa4,
Xaa4 is (a) a residue of a cyclic non-aromatic α-amino acid, preferably Xaa4 is a residue of an α-amino acid of formula (Va) 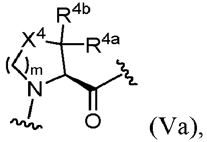
wherein
R^{4a} is selected from the group consisting of H, OH, methyl and CF₃,
R^{4b} is selected from the group consisting of H and methyl, preferably R^{4b} is methyl if R^{4a} is methyl,
X⁴ is selected from the group consisting of CHR^{4c}, S and O,
wherein R^{4c} is selected from the group consisting of NH₂, OH, H, NHR^{4d}, methyl and F, preferably R^{4c} is NH₂ or OH,
wherein R^{4d} is selected from the group consisting of Ac and a Z group,
m is 1 or 2, preferably m is 1,
or (b) a residue of an N-alkylated α-amino acid, preferably Xaa4 is a residue of an N-alkylated α-amino acid of formula (Vb) 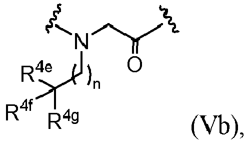
wherein
n is 0, 1, 2, 3, 4, or 5, preferably n is 0, 1 or 3,
R^{4e} is, if n is 0, selected from the group consisting of H, methyl, an aryl ring, COOH, CONH₂ and C(=O)R⁴ⁿ,
or is, if n is 1, 2, 3, 4 or 5, selected from the group consisting of H, methyl, an aryl ring, OH, NH₂, COOH, CONH₂ and NHR⁴ⁿ,
R^{4f} is selected from the group consisting of H and (C₁-C₂)alkyl,
R^{4g} is selected from the group consisting of H and methyl,
R^{4h} is a Z group, preferably the Z group is a bio-distribution modifier,
or (c) Xaa4 is a residue of a bicyclic non-aromatic α-amino acid, preferably Xaa4 is a residue of a bicyclic non-aromatic α-amino acid of formula (Vc) 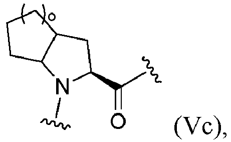
wherein
o is 1 or 2,
and the carbonyl group of Xaa4 is covalently attached to the α-nitrogen atom of Xaa5,
Xaa5 is (a) a residue of an α-amino acid optionally comprising a Z group, wherein the α-nitrogen atom of the α-amino acid is optionally substituted by (C₁-C₄)alkyl or (b) a cyclic α-amino acid, and the carbonyl group of Xaa5 is covalently attached to the α-nitrogen atom of Xaa6,
Xaa6 is a residue of an α-amino acid comprising a side chain, wherein the side chain comprises a branched aliphatic group, preferably Xaa6 is a residue of an α-amino acid of formula (VIIa) 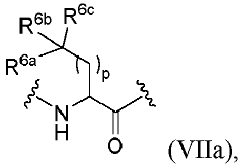
wherein
p is 1, 0 or 2, preferably p is 1,
R^{6a} is (C₁-C₂)alkyl,
R^{6b} is methyl,
R^{6c} is H, if p is 0,
or is selected from the group consisting of H and methyl, if p is 1 or 2,
and the carbonyl group of Xaa6 is covalently attached to the α-nitrogen atom of Xaa7,
Xaa7 is a residue of an α-amino acid comprising a side chain, wherein the side chain comprises a nucleophilic group, wherein the nucleophilic group comprises a heteroatom, wherein the heteroatom is selected from the group comprising a sulfur atom and a nitrogen atom, preferably Xaa7 is a residue of an α-amino acid of formula (Villa) 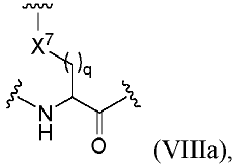
wherein
X⁷ comprises the heteroatom and is selected from the group consisting of-S- and -NH-,
q is 1 or 2, preferably q is 1,
and the carbonyl group of Xaa7 is covalently attached to the α-nitrogen atom of Xaa8,
Xaa8 is (a) a residue of an aliphatic cyclic α-amino acid, of an aliphatic heterocyclic α-amino acid or of an aliphatic cyclic α-amino acid comprising an annulated aromatic ring, preferably Xaa8 is a residue of an α-amino acid of formula (IXa) or (IXb), more preferably Xaa8 is a residue of an α-amino acid of formula (IXa), 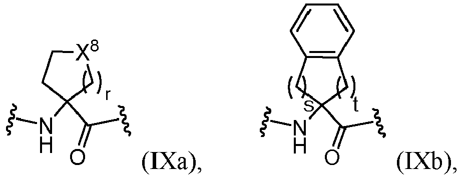
wherein
X⁸ is selected from the group consisting of NR^{8a}, O, NH, and CH₂,
wherein R^{8a} is a Z group or R^{8a} is acetyl,
wherein if R^{8a} is a Z goup, the Z group preferably is a chelator optionally comprising a linker,
r is 2 or 1, preferably r is 2,
s is 1 or 2, preferably s is 1,
t is 1 or 2, preferably t is 1 if s is 1, and t is 2 if s is 2,
or (b) a residue of an α-amino acid or an α-alkyl-α-amino acid, preferably Xaa8 is a residue of an α-amino acid of formula (IXc), 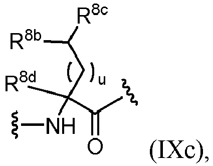
wherein
R^{8b} is selected from the group consisting of H, OH, NH₂, NHR^{8e}, (C₁-C₂)alkyl, COOH, CONH₂, an aryl ring and a heteroaryl ring, preferably the aryl ring is phenyl and the heteroaryl ring is 3-indoyl, wherein R^{8e} is a Z group, preferably the Z group is a chelator optionally comprising a linker,
R^{8c} is selected from the group consisting of H and methyl, if R^{8b} is selected from the group consisting of OH, NH₂ and NHR^{8e},
or is selected from the group consisting of H, methyl and OH, if R^{8b} is selected from the group consisting of H, (C₁-C₂)alkyl, COOH, CONH₂, an aryl ring and a heteroaryl ring,
R^{8d} is selected from the group consisting of H and (C₁-C₂)alkyl,
u is 0, 1, 2, 3, 4, or 5, preferably u is 0, 1 or 3,
and the carbonyl group of Xaa8 is covalently attached to the α-nitrogen atom of Xaa9,
Xaa9 is a residue of an acyclic α-amino acid, wherein the acyclic α-amino acid is in L-configuration and the carbonyl group of Xaa9 is covalently attached to the α-nitrogen atom of XaalO,
XaalO is a residue of an α-amino acid comprising a side chain, wherein the side chain comprises a branched aliphatic group or an unsubstituted aromatic ring, preferably XaalO is a residue of an α-amino acid of formula (XIa), 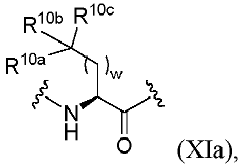
wherein
R^{10a} is selected from the group consisting of (C₁-C₂)alkyl and phenyl,
R^{10b} is selected from the group consisting of methyl and H,
R^{10c} is selected from the group consisting of H and methyl,
w is 0 or 1,
and the carbonyl group of XaalO is covalently attached to the α-nitrogen atom of Xaa11,
Xaa11 is a residue of an α-amino acid comprising a side chain, wherein the side chain comprises a nucleophilic group, wherein the nucleophilic group comprises a heteroatom, wherein the heteroatom is selected from the group comprising a sulfur atom and a nitrogen atom, preferably Xaa11 is an α-amino acid of formula (XIIa), 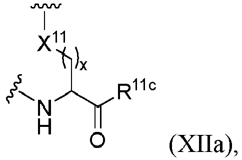
wherein
X¹¹ comprises the heteroatom and is selected from the group consisting of -S- and -NH-,
R^{11c} is the C-terminal group C-term,
x is 1 or 2,
and wherein a linker is optionally interspersed between the bio-distribution modifier comprised by the N-terminal modification group A and Xaa1, wherein the bio-distribution modifier comprises a residue of an aliphatic carboxylic acid,
wherein to the ω-carbon atom of the aliphatic carboxylic acid a nitrogen atom, a triazolyl moiety, a hydroxymethyl-triazolyl moiety or an urea moiety is covalently attached,
wherein to the nitrogen atom, to the triazolyl moiety, to the hydroxymethyl triazolyl moiety or to the urea moiety
(a) 1 or 2 first level alkyl groups are covalently attached, wherein each and any first level alkyl group individually and independently comprises 2 or more hydroxyl groups, wherein each and any hydroxyl group is a primary hydroxyl group,
or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
preferably the bio-distribution modifier is a structure of formula (A-I), 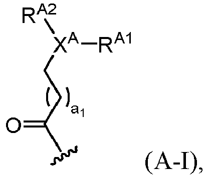
wherein
X^{A} comprises a nitrogen atom, a triazolyl moiety, a hydroxymethyl-triazolyl moiety or an urea moiety,
a₁ is 0, 1, 2, 3, 4, 5, or 6,
if X^{A} is a triazolyl moiety, preferably a₁ is 2,
if X^{A} is selected from the group comprising a nitrogen atom, a hydroxymethyl-triazolyl moiety and an urea moiety, preferably a₁ is 4,
R^{A1} is a first level alkyl group of a structure of formula (A-II), 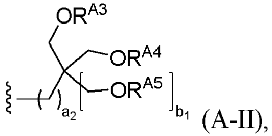
wherein
a₂ is 0 or 1,
b₁ is 0 or 1,
wherein, if b₁ is 0,
R^{A3} and R^{A4} are individually and independently a second level alkyl group of a structure of formula (A-III),
or R^{A3} and R^{A4} are each H,
or wherein, if b₁ is 1,
each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), 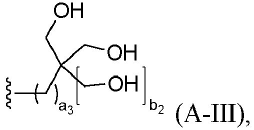
wherein
a₃ is 0 or 1, and
b₂ is 0 or 1,
and
R^{A2} is absent, if X^{A} is selected from the group comprising a triazolyl moiety, a hydroxmethyl-triazolyl moiety and an urea moiety,
or R^{A2} is H or a first level alkyl group of a structure of formula (A-II), if X^{A} is a nitrogen atom, wherein
a₂ is 0 or 1,
b₁ is 0 or 1,
wherein if b₁ is 0,
R^{A3} and R^{A4} are individually and independently a second level alkyl group of a structure of formula (A-III)
or R^{A3} and R^{A4} are H,
or wherein if b₁ is 1
each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), wherein in formula (A-III)
a₃ is 0 or 1, and
b₂ is 0 or 1,
and wherein the carbonyl group of the structure (A-I) is covalently attached to the α-nitrogen atom of Xaa1,
or if a linker, comprising an amino group and a carbonyl group, is interspersed between the bio-distribution modifier comprised by the N-terminal modification group A and Xaa1, the carbonyl group of the structure (A-I) is covalently attached to the amino group of the linker,
and wherein a linker is optionally interspersed between Xaa11 and the bio-distribution modifier comprised by the C-terminal modification group C-term,
wherein the bio-distribution modifier comprises a residue of an α-amino acid with an aliphatic side chain,
wherein to an ω-carbon atom of the aliphatic side chain of the α-amino acid a nitrogen atom, a triazolyl moiety, a hydroxymethyl-triazolyl moiety or an urea moiety is covalently attached,
wherein to the nitrogen atom, to the triazolyl moiety, to the hydroxymethyl triazolyl moiety or to the urea moiety
(a) 1 or 2 first level alkyl groups are covalently attached, wherein each and any first level alkyl group individually and independently comprises 2 or more hydroxyl groups, wherein each and any hydroxyl group is a primary hydroxyl group,
or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
and wherein to the carbonyl group attached to the α-carbon atom of the α-amino acid an amino group is covalently attached thereby forming an amide,
preferably the bio-distribution modifier is a structure of formula (C-I), 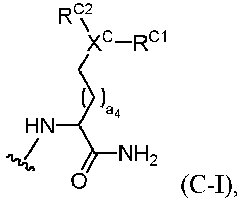 wherein
X^{C} comprises a nitrogen atom, a triazolyl moiety, a hydroxymethyl-triazolyl moiety and an urea moiety,
a₄ is 0, 1, 2, 3, or 4, if X^{C} is a triazolyl moiety, preferably a₄ is 1 or 2, more preferably a₄ is 1 if X^{C} is selected from the group comprising a nitrogen atom, a hydroxymethyl-triazolyl moiety and an urea moiety, preferably a₄ is 3,
R^{C1} is a first level alkyl group of a structure of formula (C-II), 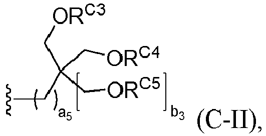 wherein
a₅ is 0 or 1,
b₃ is 0 or 1,
wherein, if b₃ is 0,
R^{C3} and R^{C4} are individually and independently a second level alkyl group of a structure of formula (C-III) or R^{C3} and R^{C4} are each H,
or wherein, if b₃ is 1, each and any of R^{C3}, R^{C4} and R^{C5} is selected from
a second level alkyl group of a structure of formula (C-III) and H, preferably each and any of R^{C3}, R^{C4} and R^{C5} is a second level alkyl group of a structure of formula (C-III), 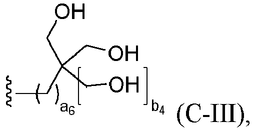
wherein
a₆ is 0 or 1, and
b₄ is 0 or 1
and R^{C2} is absent, if X^{C} is selected from the group comprising a triazolyl moiety, a hydroxmethyl-triazolyl moiety and an urea moiety,
or R^{C2} is H or is a first level alkyl group of a structure of formula (C-II), if X^{C} is a nitrogen atom, wherein
a₅ is 0 or 1,
b₃ is 0 or 1,
wherein, if b₃ is 0,
R^{C3} and R^{C4} are individually and independently a second level alkyl group of a structure of formula (C-III)
or R^{C3} and R^{C4} are each H,
or wherein, if b₃ is 1,
each and any of R^{C3}, R^{C4} and R^{C5} is individually and independently selected from a second level alkyl group of a structure of formula (C-III) and H, preferably each and any of R^{C3}, R^{C4} and R^{C5} is individually and independently a second level alkyl group of a structure of formula (C-III), wherein in formula (C-III)
a₆ is 0 or 1, and
b₄ is 0 or 1,
and wherein the α-nitrogen atom of the structure (C-I) is covalently attached to the carbonyl group of Xaa11, or if a linker comprising a carbonyl group and an amino group is interspersed between Xaa11 and the bio-distribution modifier comprised by the C-terminal modification group C-term, the α-nitrogen atom of the structure (C-I) is covalently attached to the carbonyl group of the linker, more preferably a linker is interspersed between Xaa11 and the bio-distribution comprised by the C-terminal modification group C-term.

3. The compound of any one of claims 1 and 2,
wherein
(a)
Xaa7 is a residue of formula (VIIIa) 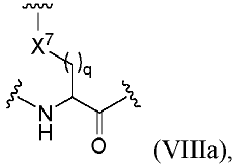 wherein
X⁷ is -S- or -NH-,
q is 1 or 2, preferably q is 1,
Yc is a structure of formula (XIIIa) 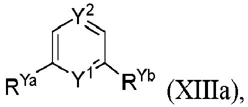 wherein
R^{Ya} is -CH₂-,
R^{Yb} is -CH₂-,
Y¹ is selected from the group consisting of N and CH,
Y² is selected from the group consisting of CH and N,
and Xaa11 is a residue of formula (XIIa) 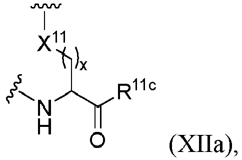 wherein
X¹¹ is -S- or -NH-,
R^{11c} is the C-terminal group C-term,
x is 1 or 2, preferably x is 1,
preferably the compound comprises a cyclic peptide of formula (Ic), 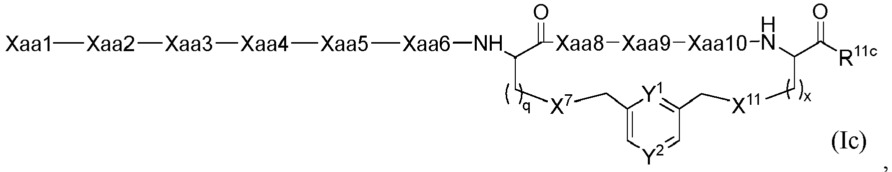
more preferably the compound comprises a cyclic peptide of formula (Ic),
wherein X⁷ is -S-, X¹¹ is -S-, Y¹ is N or CH, Y² is CH, q is 1, and x is 1,
most preferably the compound comprises a cyclic peptide of formula (Ic),
wherein X⁷ is -S-, X¹¹ is -S-, Y¹ is N, Y² is CH, q is 1, and x is 1,
or (b)
Xaa7 is a residue of formula (VIIIa) 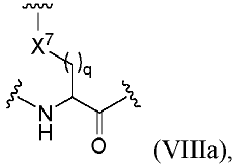 wherein
X⁷ is -S- or -NH-,
q is 1 or 2, preferably q is 1,
Yc is a structure of formula (XIIIh) 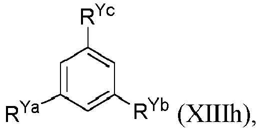 wherein
R^{Ya} is -CH₂-,
R^{Yb} is -CH₂-, and
R^{Yc} is -CH₂-R^{Yd}, and
R^{Yd} is a structure of formulae (XIIIc), (XIIId) or (XIIIe), 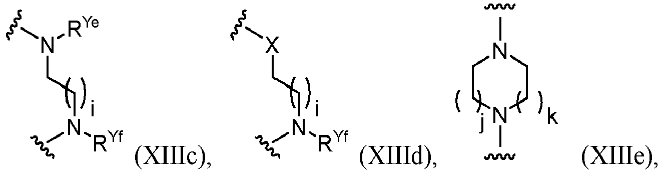
wherein
R^{Ye} and R^{Yf} are each and independently selected from the group consisting of H and (C₁-C₄)alkyl,
i is each and independently 1, 2, 3, 4, 5 or 6, preferably i is 1 or 2,
j and k are each and independently 1, 2 or 3, and
X is O or S, preferably X is S,
wherein
in formulae (XIIIc) and (XIIIe)
one of the two nitrogen atoms is covalently attached to -CH₂- of RYC
and in formula (XIIId)
-X- is covalently attached to -CH₂- of R^{Yc} while to the remaining nitrogen atom optionally a Z group is covalently attached, preferably the Z group comprises a chelator and optionally a linker,
and Xaa11 is a residue of formula (XIIa) 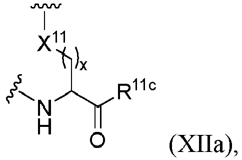 wherein
X¹¹ is -S- or -NH-,
R^{11c} is the C-terminal group C-term,
x is 1 or 2, preferably x is 1,
preferably the compound comprises a cyclic peptide of formula (Ii), 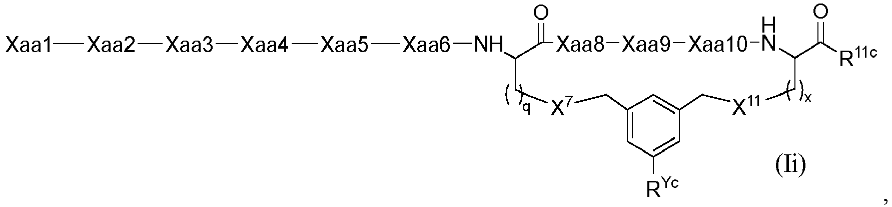
more preferably the compound comprises a cyclic peptide of formula (Ii),
wherein
X⁷ is -S- and X¹¹ is -S-,
R^{Yc} is -CH₂-R^{Yd}, and
R^{Yd} is a structure of formulae (XIIId) or (XIIIe),
wherein R^{Yf} is H, i is 1, j is 1, and k is 1,
wherein in formula (XIIId) -X- is S and is attached to -CH₂- of R^{Yc}, and in formula (XIIIe) one of the two nitrogen atoms is attached to -CH₂- of RYC,
while to the remaining nitrogen atom in either (XIIId) or (XIIIe) optionally a Z group is covalently attached, preferably the Z group comprises a chelator and optionally a linker,
q is 1, and
x is 1,
most preferably the compound comprises a cyclic peptide of formula (Ii), wherein R^{Yd} is a structure of formula (XIIId), wherein -X- is S and R^{Yf} is H, and i is 1,
preferably Yc is selected from the group comprising 2Lut, 3Lut, 3MeBn, tMeBn(DOTA-AET) and tMeBn(DOTA-PP), Xaa7 is selected from the group comprising Cys, Hey and Dap, Xaa11 is selected from the group comprising Cys, Hey and Dap,
more preferably Yc is selected from the group comprising 2Lut and 3MeBn, Xaa7 is Cys and Xaa11 is Cys
most preferably Yc is 2Lut, Xaa7 is Cys and Xaa11 is Cys.

4. The compound of any one of claims 1 to 3, wherein Xaa1 is a residue of an α-amino acid comprising a side chain, wherein the side chain comprises an acidic or polar group within the side chain, preferably Xaa1 is a residue of an α-amino acid of formulae (IId) or (IIe), more preferably Xaa1 is a residue of an α-amino acid formula (IId), 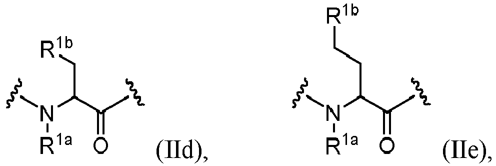 wherein
R^{1a} is selected from the group consisting of H and methyl,
R^{1b} is selected from the group consisting of CO₂H, CONH₂, OH, SO₃H and OPO₃H₂,
and wherein the carbonyl group of the aliphatic carboxylic acid comprised by the bio-distribution modifier comprised by the N-terminal modification group A is covalently attached to the α-nitrogen atom Xaa1, or if a linker comprising an amino group and a carbonyl group is interspersed between the bio-distribution modifier comprised by the N-terminal modification group A and Xaa1, a carbonyl group of the linker is covalently attached to the α-nitrogen atom of Xaa1,
preferably Xaa1 is a residue of an α-L-amino acid selected from the group consisting of Asp, Asn, Glu, Gln, Hse, Cya, Pse, Nmd and Ser,
more preferably Xaa1 is a residue of an α-L-amino acid selected from the group consisting of Asp, Asn, Glu, and Gln,
most preferably Xaa1 is an Asp residue.

5. The compound of any one of claims 1 to 4, wherein Xaa2 is a residue of an α-L-amino acid of formula (IIId), 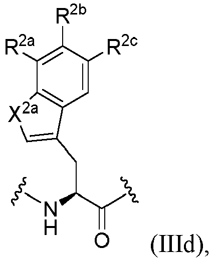 wherein
X^{2a} is NH or S, preferably is NH,
R^{2a} is H or F,
R^{2b} is selected from the group consisiting of H, F and Cl,
R^{2c} is selected from the group consisting of H, F, Cl, Br and OH,
preferably Xaa2 is a residue an α-L-amino acid selected from the group consisting of Trp, Hyw, 5Fw, 6Clw, 7Fw, 5Clw, 5Brw and 6Fw,
more preferably Xaa2 is a residue an α-L-amino acid selected from the group consisting of Trp, Hyw, 5Fw and 7Fw,
most preferably Xaa2 is a Trp residue.

6. The compound of any one of claims 1 to 5, wherein Xaa3 is a residue of an amino acid of formula (IVe) or (IVf), 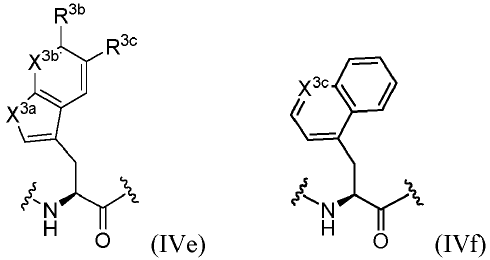 wherein
R^{3b} is H or F,
R^{3c} is selected from the group consisting of Cl, Br, H and F,
X^{3a} is NH or S,
X^{3b} is selected from the group consisting of C-H, C-F and N, and
X^{3c} is C-H or N, preferably X^{3c} is C-H,
preferably Xaa3 is a residue of an α-L-amino acid selected from the group consisting of 5Clw, 5Brw, Trp, 1Ni, Bta, 5Fw, 6Fw and 7Fw,
more preferably Xaa3 is a residue of an α-L-amino acid selected from the group consisting of 5Clw and 5Brw, and
most preferably Xaa3 is a 5Clw residue.

7. The compound of any one of claims 1 to 6, wherein
(a)
Xaa4 is a residue of an α-L-amino acid of formula (Va) 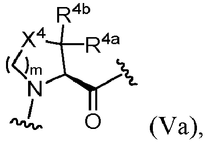 wherein
m is 1 or 2, preferably m is 1,
R^{4a} is selected from the group consisting of H, OH and methyl,
R^{4b} is either H or methyl, preferably R^{4b} is methyl if R^{4a} is methyl,
X⁴ is selected from the group consisting of CHR^{4c}, CH₂, S and O,
wherein R^{4c} is selected from the group consisting of NHR^{4d}, NH₂, H, OH, methyl and F,
wherein R^{4d} is Ac or is a Z group, preferably the Z group is a chelator optionally comprising a linker, more preferably the chelator is DOTA,
preferably Xaa4 is a residue of an α-L-amino acid selected from the group consisting of Tap, Hyp, Pro, 4Ap, 4Ap(Ac), Tap(DOTA), 4Tfp and H3p,
more preferably Xaa4 is a residue of an α-L-amino acid selected from the group consisting of Tap, Hyp, Pro and 4Ap, and
most preferably Xaa4 is a Tap residue,
or
(b)
Xaa4 is a residue of an N-alkylated α-amino acid of formula (Vf) 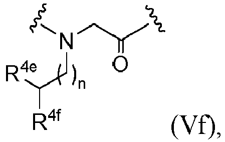 wherein
n is 0, 1 or 3,
R^{4e} if n = 0, is selected from the group consisting of H and phenyl,
or if n = 1, is selected from the group consisting of methyl, CONH₂ and COOH,
or if n = 3, is selected from the group consisting of NH₂ and NHR^{4h}, wherein R^{4h} is a Z group, preferably the Z group is a bio-distribution modifier,
R^{4f} is selected from the group consisting of H and methyl,
preferably Xaa4 is a residue of an N-alkylated α-amino acid selected from the group consisting of Nmg, Nlys, Nleu, Nphe and Nglu,
or
(c)
Xaa4 is an Oic residue.

8. The compound of any one of claims 1 to 7, wherein Xaa5 is a residue of an α amino acid optionally comprising a Z group, wherein the α-nitrogen atom of the α-amino acid is optionally substituted by a methyl group,
preferably Xaa5 is a residue of an α-L-amino acid selected from the group consisiting of Glu, Gln, Asp, Asn, Ser, Hse, Trp, Lys, Arg, His, Hly, and Har,
more preferably Xaa5 is an α-L-amino acid selected from the group consisiting of Glu, Gln, Asp, Asn, Ser, and Hse,
most preferably Xaa5 is a Glu residue.

9. The compound of any one of claims 1 to 8, wherein Xaa6 is a residue of an amino acid of formula (VIIa) 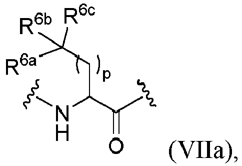 wherein
p is 1 or 0, preferably p is 1,
R^{6a} is (C₁-C₂)alkyl,
R^{6b} is methyl, and
R^{6c} is H if p is 0, and is H or methyl, if p is 1,
preferably Xaa6 is a residue of an α-amino acid selected from the group consisting of leu, Leu, Npg, Val, Ile and Hle,
more preferably Xaa6 is a residue of an α-amino acid selected from the group consisting of leu, Leu, Npg, Val and Ile, and
most preferably Xaa6 is a leu residue.

10. The compound of any one of claims 1 to 9, wherein
(a)
Xaa8 is a residue of a cyclic α-amino acid of formula (IXa), 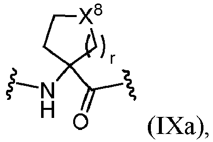 wherein
r is 2 or 1, preferably r is 2,
X⁸ is selected from the group consisting of NR^{8a}, O, NH, and CH₂,
wherein R^{8a} is a Z group or R^{8a} is acetyl, preferably R^{8a} is a Z group, wherein the Z group is a chelator optionally comprising a linker, wherein more preferably the chelator is DOTA,
preferably Xaa8 is a residue of a cyclic α-amino acid residue selected from the group consisting of Apc(R^{8a}), Apc, and Thp, wherein R^{8a} is a Z group, wherein if the Z group is a chelator optionally comprising a linker, preferably the chelator is DOTA, or R^{8a} is acetyl,
more preferably Xaa8 is Apc(R^{8a}), wherein R^{8a} is a Z group, wherein the Z group is a chelator optionally comprising a linker , wherein the chelator
a) preferably is selected from the group consisting of DOTA, DOTAGA, DOTAM, DOTP, NOTA, NODAGA, PSC, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
b) more preferably is selected from the group consisting of DOTA, DOTAGA, DOTAM, NOTA, NODAGA, PSC, NODA-MPAA, NOPO, HBED, DTPA, CHX-A"-DTPA, CB-TE2A, Macropa, PCTA, N4, and analogs thereof,
and
c) most preferably is selected from the group consisting of DOTA, DOTAGA, DOTAM, NOTA, NODAGA, PSC, NOPO, Macropa, PCTA and analogs thereof,
(b) Xaa8 is an Aic residue,
(c)
Xaa8 is a residue of an α-amino acid selected from the group consisting of Lys, Lys(R^{8e}), lys(R^{8e}), Amk(R^{8e}), Dab(R^{8e}) and dab(R^{8e}), wherein R^{8e} is a Z group, preferably the Z group is a chelator optionally comprising a linker,
preferably Xaa8 is a Amk(R^{8e}) residue, wherein R^{8e} is a Z group, wherein the Z group is a chelator optionally comprising a linker, wherein the chelator
a) preferably is selected from the group consisting of DOTA, DOTAGA, DOTAM, DOTP, NOTA, NODAGA, PSC, NODA-MPAA, HBED, TETA, CB-TE2A, DTPA, CHX-A"-DTPA, DFO, Macropa, HOPO, TRAP, THP, DATA, NOPO, NOTP, PCTA, sarcophagine, FSC, NETA, NE3TA, H4octapa, pycup, HYNIC, NxS4-x (N4, N2S2, N3S), ^{99m}Tc(CO)3-chelators and their analogs,
b) more preferably is selected from the group consisting of DOTA, DOTAGA, DOTAM, NOTA, NODAGA, PSC, NODA-MPAA, NOPO, HBED, DTPA, CHX-A"-DTPA, CB-TE2A, Macropa, PCTA, N4, and analogs thereof,
and
c) most preferably is selected from the group consisting of DOTA, DOTAGA, DOTAM, NOTA, NODAGA, PSC, NOPO, Macropa, PCTA and analogs thereof,
or
(d) Xaa8 is a residue of an α-amino acid residue selected from the group consisting of Aib, Deg, Ams, ala, Ala, Gln, Thr, Trp, Phe and Glu.

11. The compound of any one of claims 1 to 10, wherein Xaa9 is a residue of an amino acid of formula (Xa) 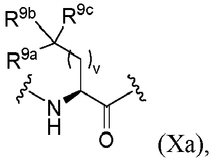 wherein
R^{9a} is selected from the group consisting of (C₁-C₄)alkyl, COOH, CONH₂, OH, NH₂, NHC(=NH)NH₂, an aryl ring and a heteroaryl ring, preferably the aryl ring is phenyl and the heteroaryl ring is 3-indoyl, and cyclo-hexyl,
R^{9b} is methyl or H,
R^{9c} is H or methyl, and
v is 0, 1, 2, preferably v is 1,
preferably Xaa9 is a residue of an α-L-amino acid selected from the group consisting of Leu, Hle, Ile, Arg, Trp, Glu, Phe, Ser, Cha, Npg and Tle,
more preferably Xaa9 is a residue of an α-L-amino acid selected from the group consisting of Leu, Hle, Ile, Arg, Trp, Glu, Phe and Ser, and
most preferably Xaa9 is a Leu residue.

12. The compound of any one of claims 1 to 11, wherein Xaa10 is a residue of an amino acid of formula (XIa) 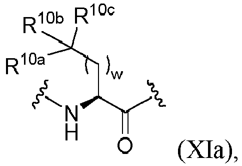 wherein
R^{10a} is selected from the group consisting of (C₁-C₂)alkyl and phenyl, preferably R^{10a} is (C₁-C₂)alkyl, more preferably (C₁-C₂)alkyl is ethyl,
R^{10b} is selected from the group consisting of methyl and H, preferably R^{10b} is methyl,
R^{10c} is selected from the group consisting of H and methyl, R^{10C} is H, if w is 1, preferably R^{10c} is H, and
w is 0 or 1, preferably w is 0,
preferably XaalO is a residue of an α-L-amino acid selected from the group consisting of Ile, Leu, Tle, Val, and Phe,
more preferably XaalO is a residue of α-L-amino acid selected from the group consisting of Ile, Leu and Tle,
most preferably XaalO is an Ile residue.

13. The compound of any one of claims 1 to 12, wherein the N-terminal modification group A wherein a linker comprising an amino group and a carbonyl group is optionally interspersed between the bio-distribution modifier comprised by the N-terminal modification group A and Xaa1, wherein the bio-distribution modifier comprises a residue of an aliphatic carboxylic acid,
wherein to an ω-carbon atom of the aliphatic carboxylic acid a nitrogen atom, a triazolyl moiety, a hydroxymethyl-triazolyl moiety or an urea moiety is covalently attached,
wherein to the nitrogen atom, to the triazolyl moiety, to the hydroxymethyl triazolyl moiety or to the urea moiety
(a) 1 or 2 first level alkyl groups are covalently attached, wherein each and any first level alkyl group individually and independently comprises 2 or more hydroxyl groups, wherein each and any hydroxyl group is a primary hydroxyl group,
or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
preferably the bio-distribution modifier is a structure of formula (A-I), 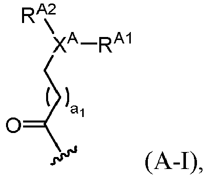
wherein
X^{A} comprises a nitrogen atom, a triazolyl moiety, a hydroxymethyl-triazolyl moiety or an urea moiety,
a₁ is 0, 1, 2, 3, 4, 5, or 6,
if X^{A} is a triazolyl moiety, preferably a₁ is 2,
if X^{A} is selected from the group comprising a nitrogen atom, a hydroxymethyl-triazolyl moiety and an urea moiety, preferably a₁ is 4,
R^{A1} is a first level alkyl group of a structure of formula (A-II), 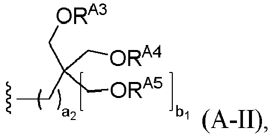 wherein
a₂ is 0 or 1,
b₁ is 0 or 1,
wherein, if b₁ is 0,
R^{A3} and R^{A4} are individually and independently a second level alkyl group of a structure of formula (A-III),
or R^{A3} and R^{A4} are each H,
or wherein, if b₁ is 1,
each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), 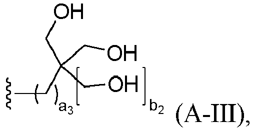
wherein
a₃ is 0 or 1, and
b₂ is 0 or 1,
and wherein
R^{A2} is absent, if X^{A} is selected from the group comprising a triazolyl moiety, a hydroxmethyl-triazolyl moiety and an urea moiety,
or R^{A2} is H or a first level alkyl group of a structure of formula (A-II), if X^{A} is a nitrogen atom, wherein
a₂ is 0 or 1,
b₁ is 0 or 1,
wherein if b₁ is 0,
R^{A3} and R^{A4} are individually and independently a second level alkyl group of a structure of formula (A-III)
or R^{A3} and R^{A4} are each H,
or wherein if b₁ is 1,
each and any of R^{A3}, R^{A4} and R^{A5} is individually and independently selected from a second level alkyl group of a structure of formula (A-III) and H, preferably each and any of R^{A3}, R^{A4} and R^{A5} is individually and independently a second level alkyl group of a structure of formula (A-III), wherein in formula (A-III)
as is 0 or 1, and
b₂ is 0 or 1,
and wherein the carbonyl group of the structure (A-I) is covalently attached to the α-nitrogen atom of Xaa1,
or if a linker, comprising an amino group and a carbonyl group, is interspersed between the bio-distribution modifier comprised by the N-terminal modification group A and Xaa1, the carbonyl group of the structure (A-I) is covalently attached to the amino group of the linker,
wherein if to the ω-carbon atom of the aliphatic carboxylic acid
a) a nitrogen atom is covalently attached, the N-terminal modification group A
is selected from the group consisting of HPD2:Ahx, PEO2:Ahx, HAC4:HPD2:Ahx, HPD4:HPD2:Ahx, HAC2:HAC:Ahx, HPD2:HAC:Ahx and HAC:Ahx,
preferably is selected from the group consisting of HPD2:Ahx, PEO2:Ahx, HAC4:HPD2:Ahx and HPD4:HPD2:Ahx,
more preferably is HPD2:Ahx or PEO2:Ahx,
b) a triazolyl moiety is covalently attached, the N-terminal modification group A
is selected from the group consisting of HPD:Hyx, PEO:Hyx, TRIS:Hyx, HAC3:TRIS:Hyx, HPD3:PEO:Hyx, HPD3:TRIS:Hyx, HAC3:PEO:Hyx, HAC2:HPD:Hyx, HPD2:HPD:Hyx, HAC2:HAC:Hyx, HPD2:HAC:Hyx and HAC:Hyx,
preferably is selected from the group consisting of PEO:Hyx, TRIS:Hyx, HAC3:TRIS:Hyx, HPD3:PEO:Hyx, HPD3:TRIS:Hyx, HAC3:PEO:Hyx, HAC2:HPD:Hyx and HPD2:HPD:Hyx,
more preferably is selected from the group consisting of PEO:Hyx, TRIS:Hyx, HAC3:TRIS:Hyx, HPD3:PEO:Hyx, HPD3:TRIS:Hyx and HAC3:PEO:Hyx,
c) a hydroxymethyl-triazolyl moiety is covalently attached, the N-terminal modification group A
is selected from the group consisting of HAC2:HAC:Tzx, HAC2:HPD:Tzx, HPD2:HPD:Tzx and HPD2:HAC:Tzx,
or d) an urea moiety is covalently attached, the N-terminal modification group A
is selected from the group consisting of PEO:Aur, TRIS:Aur, HAC3:PEO:Aur, HAC3:TRIS:Aur, HPD3:PEO:Aur, HPD3:TRIS:Aur, HAC2:HPD:Aur, HPD2:HPD:Aur, HAC2:HAC:Aur, HPD2:HAC:Aur, HPD:Aur and HAC:Aur,
preferably is selected from the group consisting of PEO:Aur, TRIS:Aur, HAC3:PEO:Aur, HAC3:TRIS:Aur, HPD3:PEO:Aur, HPD3:TRIS:Aur, HAC2:HPD:Aur, HPD2:HPD:Aur, HAC2:HAC:Aur and HPD2:HAC:Aur,
more preferably is selected from the group consisting of PEO:Aur, TRIS:Aur, HAC3:PEO:Aur, HAC3:TRIS:Aur, HPD3:PEO:Aur and HPD3:TRIS:Aur; and/or
wherein a linker comprising an amino group and a carbonyl group is optionally interspersed between Xaa11 and the bio-distribution modifier comprised by the C-terminal modification group C-term,
wherein the bio-distribution modifier comprises a residue of an α-amino acid with an aliphatic side chain,
wherein to an ω-carbon atom of the aliphatic side chain of the α-amino acid a nitrogen atom, a triazolyl moiety, a hydroxymethyl-triazolyl moiety or an urea moiety is covalently attached,
wherein to the nitrogen atom, to the triazolyl moiety, to the hydroxymethyl triazolyl moiety or to the urea moiety
(a) 1 or 2 first level alkyl groups are covalently attached, wherein each and any first level alkyl group individually and independently comprises 2 or more hydroxyl groups, wherein each and any hydroxyl group is a primary hydroxyl group,
or, (b) 1 or 2 first level alkyl groups are covalently attached, wherein each and any first level alkyl group is covalently attached via an ether linkage to 1 or more second level alkyl groups, wherein each and any second level alkyl group individually and independently comprises 2 or more hydroxyl groups, wherein each and any hydroxyl group is a primary hydroxyl group, and wherein each and any first level alkyl group individually and independently comprises 0, 1 or 2 hydroxyl groups, wherein each and any hydroxyl group is a primary hydroxyl group,
and wherein to the carbonyl group of the α-amino acid an amino group is covalently attached thereby forming an amide,
preferably the bio-distribution modifier is a structure of formula (C-I), 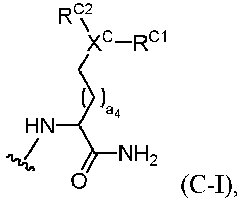
wherein
X^{C} comprises a nitrogen atom, a triazolyl moiety, a hydroxymethyl-triazolyl moiety and an urea moiety,
a₄ is 0, 1, 2, 3, or 4,
if X^{C} is a triazolyl moiety, preferably a₄ is 1 or 2, more preferably a₄ is 1
if X^{C} is selected from the group comprising a nitrogen atom, a hydroxymethyl-triazolyl moiety and an urea moiety, preferably a₄ is 3,
R^{C1} is a first level alkyl group of a structure
of formula (C-II), 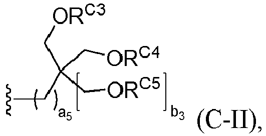 wherein
as is 0 or 1,
b₃ is 0 or 1,
wherein, if b₃ is 0,
R^{C3} and R^{C4} are individually and independently a second level alkyl group of a structure of formula (C-III) or R^{C3} and R^{C4} are each H,
or wherein, if b₃ is 1, each and any of R^{C3}, R^{C4} and R^{C5} is selected from a second level alkyl group of a structure of formula (C-III) and H, preferably each and any of R^{C3}, R^{C4} and R^{C5} is individually and independently a second level alkyl group of a structure of formula (C-III), 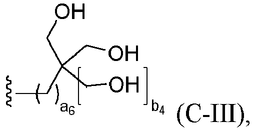 wherein
a₆ is 0 or 1, and
b₄ is 0 or 1
and R^{C2} is absent, if X^{C} is selected from the group comprising a triazolyl moiety, a hydroxmethyl-triazolyl moiety and an urea moiety,
or R^{C2} is H or is a first level alkyl group of a structure of formula (C-II), if X^{C} is a nitrogen atom, wherein
as is 0 or 1,
b₃ is 0 or 1,
wherein, if b₃ is 0,
R^{C3} and R^{C4} are individually and independently a second level alkyl group of a structure of formula (C-III) or R^{C3} and R^{C4} are each H,
or wherein, if b₃ is 1,
each and any of R^{C3}, R^{C4} and R^{C5} is individually and independently selected from a second level alkyl group of a structure of formula (C-III) and H, preferably each and any of R^{C3}, R^{C4} and R^{C5} is individually and independently a second level alkyl group of a structure of formula (C-III), wherein in formula (C-III)
a₆ is 0 or 1, and
b₄ is 0 or 1,
and wherein the α-nitrogen atom of the structure (C-I) is covalently attached to the carbonyl group of Xaa11, or if a linker comprising an amino group and a carbonyl group is interspersed between Xaa11 and the bio-distribution modifier comprised by the C-terminal modification group C-term, the α-nitrogen atom of the structure (C-I) is covalently attached to the carbonyl group of the linker, more preferably the linker is interspersed between Xaa11 and the bio-distribution comprised by the C-terminal modification group C-term, wherein if to the ω-carbon atom of the aliphatic side chain of the α-amino acid
a) a nitrogen atom is covalently attached, the C-terminal modification group C-term
is selected from the group consisting of lys:HPD2, lys:PEO2, lys:HPD2:HAC4, lys:HPD2:HPD4, lys:HAC:HAC2, lys:HAC:HPD2 and lys:HAC,
preferably is selected from the group consisting of lys:HPD2, lys:PEO2, lys:HPD2:HAC4 and lys:HPD2:HPD4,
more preferably is lys:HPD2 or lys:PEO2,
b) a triazolyl moiety is covalently attached, the C-terminal modification group C-term
is selected from the group consisting of pry:HPD, prx:PEO, prx:TRIS, prx:TRIS:HAC3, prx:PEO:HPD3, prx:TRIS:HPD3, prx:PEO:HAC3, prx:HPD:HAC2, prx:HPD:HPD2, prx:HAC:HAC2, prx:HAC:HPD2 and prx:HAC,
preferably is selected from the group consisting of prx:PEO, prx:TRIS, prx:TRIS:HAC3, prx:PEO:HPD3, prx:TRIS:HPD3, prx:PEO:HAC3, prx:HPD:HAC2 and prx:HPD:HPD2,
more preferably is selected from the group consisting of prx:PEO, prx:TRIS, prx:TRIS:HAC3, prx:PEO:HPD3, prx:TRIS:HPD3 and prx:PEO:HAC3,
c) a hydroxymethyl-triazolyl moiety is covalently attached, the C-terminal modification group C-term
is selected from the group consisting of tzk:HAC:HAC2, tzk:HPD:HAC2, tzk:HPD:HPD2 and tzk:HAC:HPD2,
or d) an urea moiety is covalently attached, the C-terminal modification group C-term
is selected from the group consisting of kur:PEO, kur:TRIS, kur:PEO:HAC3, kur:TRIS:HAC3, kur:PEO:HPD3, kur:TRIS:HPD3, kur:HPD:HAC2, kur:HPD:HPD2, kur:HAC:HAC2, kur:HAC:HPD2, kur:HPD and kur:HAC,
preferably is selected from the group consisting of kur:PEO, kur:TRIS, kur:PEO:HAC3, kur:TRIS:HAC3, kur:PEO:HPD3, kur:TRIS:HPD3, kur:HPD:HAC2, kur:HPD:HPD2, kur:HAC:HAC2 and kur:HAC:HPD2,
more preferably is selected from the group consisting of kur:PEO, kur:TRIS, kur:PEO:HAC3, kur:TRIS:HAC3, kur:PEO:HPD3 and kur:TRIS:HPD3.

14. The compound of any one of claims 1 to 13, wherein the compound comprises three or four Z groups; preferably the compound comprises three Z-groups, wherein
a first of the three Z groups is the Z group comprised by the N-terminal modification group A, wherein the Z group comprises a bio-distribution modifier optionally comprising a linker,
and a second of the three Z groups is the Z group comprised by the C-terminal modification group C-term, wherein the Z group comprises a bio-distribution modifier optionally comprising a linker,
and a third of the three Z groups is covalently attached to or is part of substituent R^{8a}, under the proviso that Xaa8 is a structure of formula (IXa),
or is covalently attached to or is part of substituent R^{8e}, under the proviso that Xaa8 is selected from the group comprising Lys(R^{8e}), lys(R^{8e}), Amk(R^{8e}), Dab(R^{8e}) and dab(R^{8e}),
preferably each and any linker comprises an amino group and a carbonyl group.

15. The compound of any one of claims 1 to 14,
wherein
Xaa1 is Asp, Asn, Glu, Gln or Cya,
Xaa2 is Trp, Hyw, 5Fw, 6Clw or 7Fw,
Xaa3 is 5Clw, 5Brw, 5Fw, Trp, 1Ni, Bta, 6Fw or Trp,
Xaa4 is Tap, Hyp, 4Tfp, Pro, H3p, Eaz, Oxa, Dtc, Nmg, Nleu, Nlys, Nphe or Nglu,
Xaa5 is Glu, glu, Nme, Gln, Asp, Asn or Hse,
Xaa6 is leu, Leu, Npg, Val or Ile,
Xaa7 is Cys or Hcy, Dap
Xaa8 is Apc(DOTA), Thp, Ape, Aib, Deg, Ams, Amk(DOTA), Egz, Eca, or ala,
Xaa9 is Leu, Hle, Ile, Phe, Glu, Arg, Ser or Trp,
XaalO is Ile, Leu, Val or Tle,
Xaa11 is Cys, Hey, Dap and
Yc is 3MeBn or 3Lut,
or wherein
Xaa1 is Asp or Glu,
Xaa2 is Trp or Hyw,
Xaa3 is 5Clw or 5Brw,
Xaa4 is Tap, Hyp, or Pro,
Xaa5 is Glu,
Xaa6 is leu or Leu,
Xaa7 is Cys,
Xaa8 is Apc(DOTA) or Amk(DOTA),
Xaa9 is Leu,
XaalO is Ile
Xaa11 is Cys and
Yc is 3Lut,
or wherein
Xaa1 is Asp,
Xaa2 is Trp,
Xaa3 is 5Clw,
Xaa4 is Tap or Hyp,
Xaa5 is Glu,
Xaa6 is leu,
Xaa7 is Cys,
Xaa8 is Apc(DOTA) or Amk(DOTA),
Xaa9 is Leu,
XaalO is Ile
Xaa11 is Cys and
Yc is 3Lut,
preferably
the compound comprises three Z groups,
wherein a first of the three Z groups is a bio-distribution modifier,
wherein the bio-distribution modifier forms the N-terminal modification group A,
wherein the bio-distribution modifier is selected from the group comprising HPD2:Ahx, HPD3:PEO:Hyx, PEO:Aur, PEO:Hyx, PEO2:Ahx, TRIS:Hyx, HPD3:TRIS:Hyx, HAC3:TRIS:Hyx, HAC3:PEO:Hyx, HAC3:PEO:Aur, HAC3:TRIS:Aur, HPD3:PEO:Aur, HPD3:TRIS:Aur and TRIS:Aur
and wherein a second of the three Z groups is a bio-distribution modifier optionally comprising
a linker,
wherein the bio-distribution modifier and the linker form the C-terminal group C-term,
wherein the linker is APAc and
wherein the bio-distribution modifier is selected from the group comprising lys:HPD2, prx:PEO:HPD3, kur:PEO, prx:PEO, lys:PEO2, prx:TRIS, prx:TRIS:HPD3, prx:TRIS:HAC3, prx:PEO:HAC3, kur:PEO:HAC3, kur:TRIS:HAC3, kur:PEO:HPD3, kur:TRIS:HPD3 and kur:TRIS,
and wherein a third of the three Z groups is a chelator,
wherein the chelator is covalently attached to Xaa8,
wherein the Xaa8 is Apc or Amk and wherein the chelator is DOTA.

16. The compound of any one of claims 1 to 15, wherein the compound is selected from the group consisting of compound HPD2:Ahx-Asp-Trp-5Clw-Tap-Glu-leu-[Cys(2Lut)-Apc(DOTA)-Leu-Ile-Cys]-APAc-lys:HPD2 (GIP-1001) of the following formula 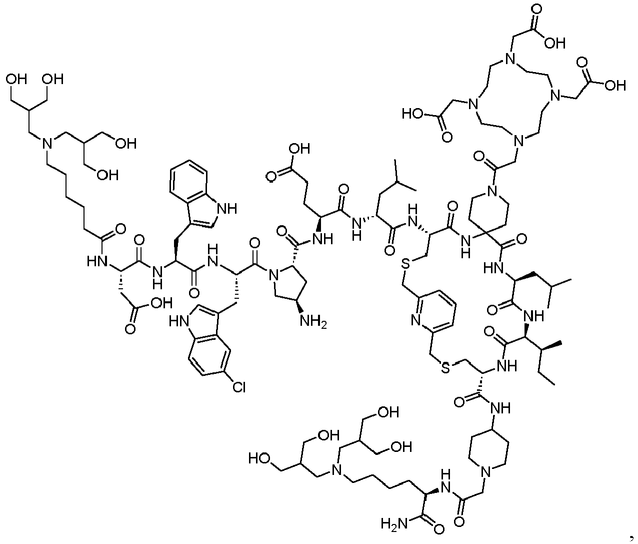
compound HPD3:PEO:Hyx-Asp-Trp-5Clw-Tap-Glu-leu-[Cys(2Lut)-Apc(DOTA)-Leu-Ile-Cys]-APAc-prx:PEO:HPD3 (GIP-1002) of the following formula 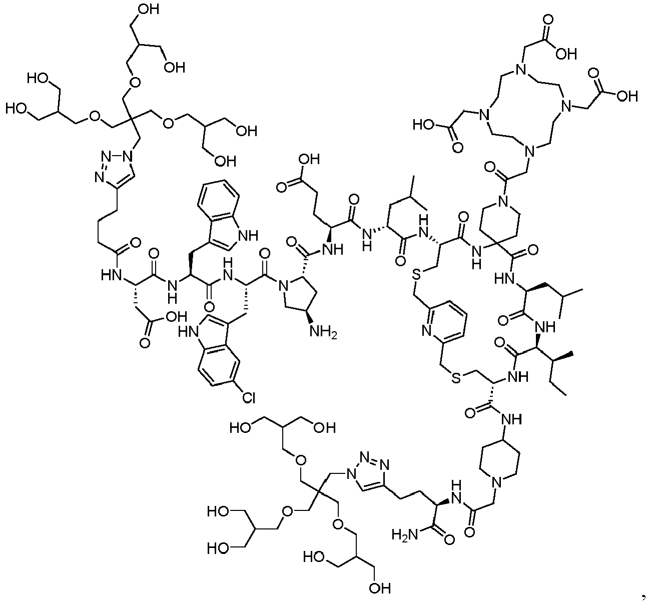
compound PEO:Aur-Asp-Trp-5Clw-Tap-Glu-leu-[Cys(2Lut)-Apc(DOTA)-Leu-Ile-Cys]-APAc-kur:PEO (GIP-1003) of the following formula 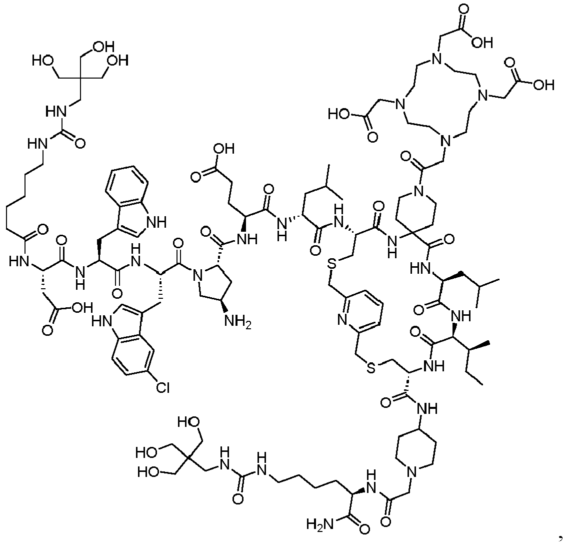
compound PEO:Hyx-Asp-Trp-5Clw-Tap-Glu-leu-[Cys(2Lut)-Apc(DOTA)-Leu-Ile-Cys]-APAc-prx:PEO (GIP-1004) of the following formula 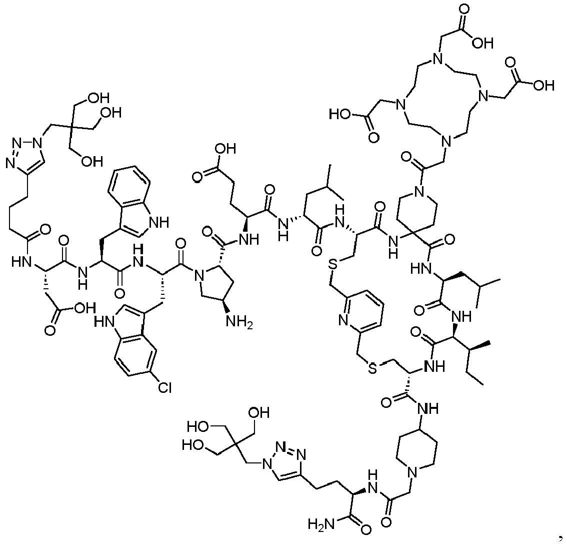
compound PEO2:Ahx-Asp-Trp-5Clw-Tap-Glu-leu-[Cys(2Lut)-Apc(DOTA)-Leu-Ile-Cys]-APAc-lys:PEO2 (GIP-1005) of the following formula 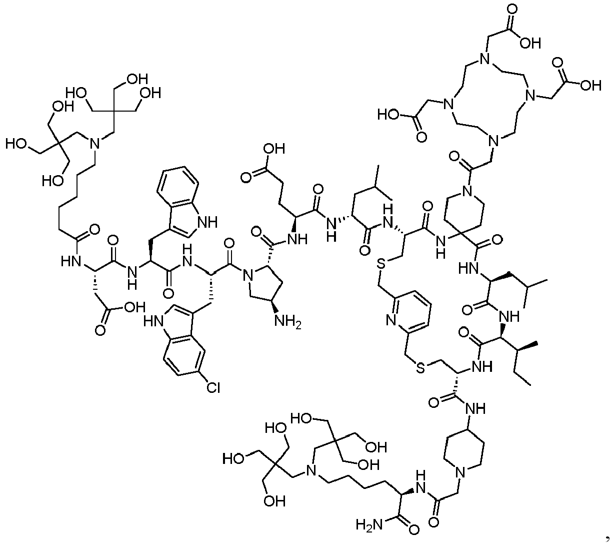
compound TRIS:Hyx-Asp-Trp-5Clw-Tap-Glu-leu-[Cys(2Lut)-Apc(DOTA)-Leu-Ile-Cys]-APAc-prx:TRIS (GIP-1006) of the following formula 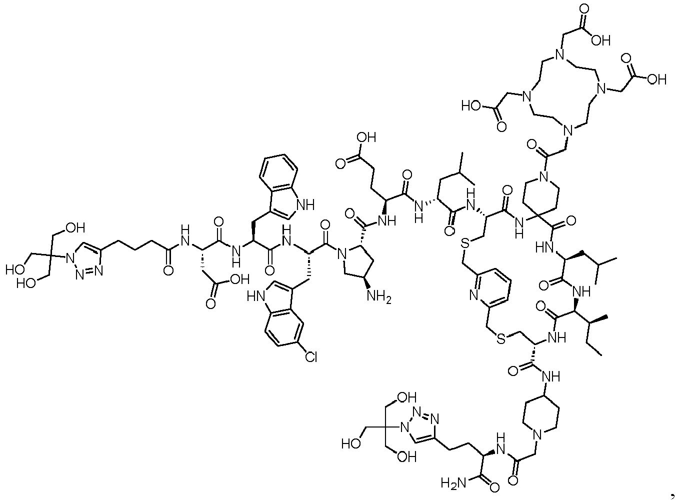
compound HPD3:TRIS:Hyx-Asp-Trp-SClw-Tap-Glu-leu-[Cys(2Lut)-Apc(DOTA)-Leu-Ile-Cys]-APAc-prx:TRIS:HPD3 (GIP-1007) of the following formula 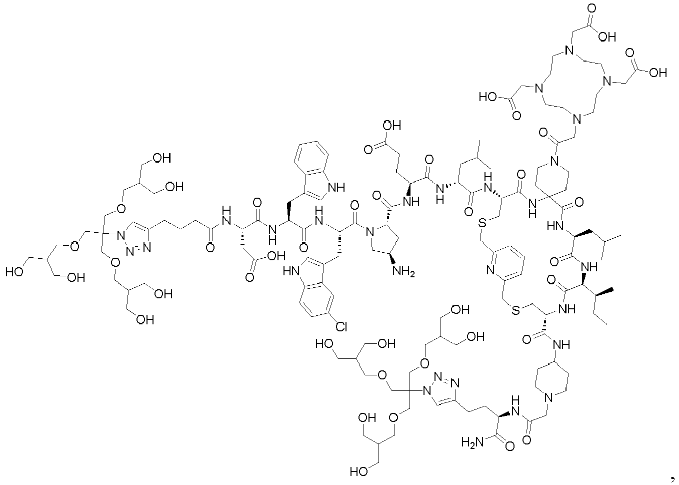
compound HAC3:TRIS:Hyx-Asp-Trp-5Clw-Tap-Glu-leu-[Cys(2Lut)-Apc(DOTA)-Leu-Ile-Cys]-APAc-prx:TRIS:HAC3 (GIP-1008) of the following formula 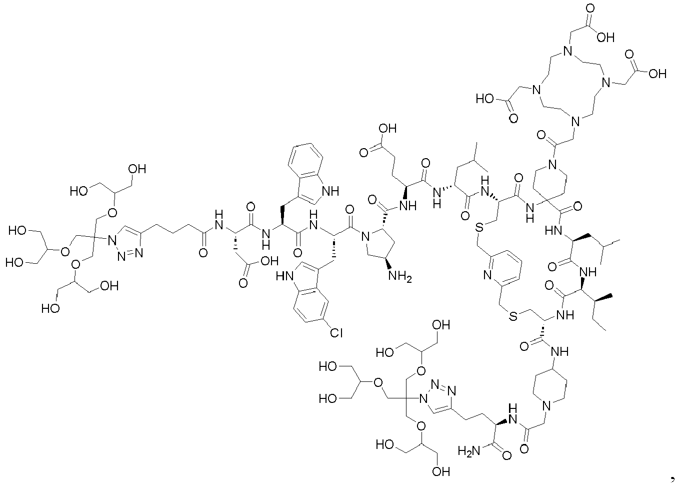
compound HAC3:PEO:Hyx-Asp-Trp-5Clw-Tap-Glu-leu-[Cys(2Lut)-Apc(DOTA)-Leu-Ile-Cys]-APAc-prx:PEO:HAC3 (GIP-1009) of the following formula 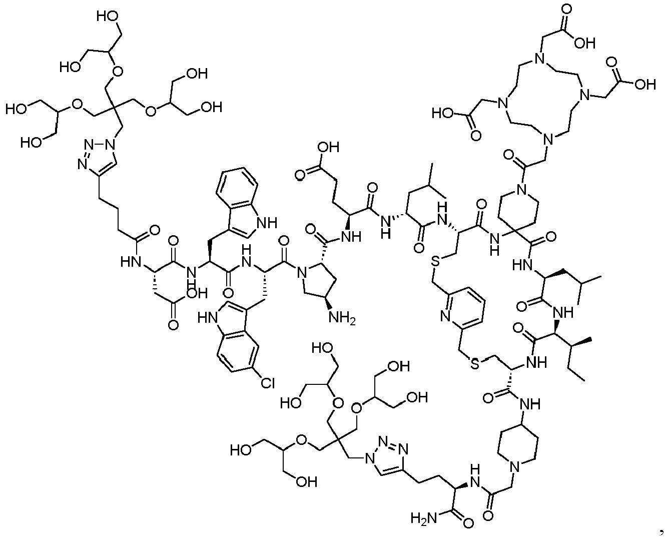
compound HAC3:PEO:Aur-Asp-Trp-5Clw-Tap-Glu-leu-[Cys(2Lut)-Apc(DOTA)-Leu-Ile-Cys]-APAc-kur:PEO:HAC3 (GIP-1010) of the following formula 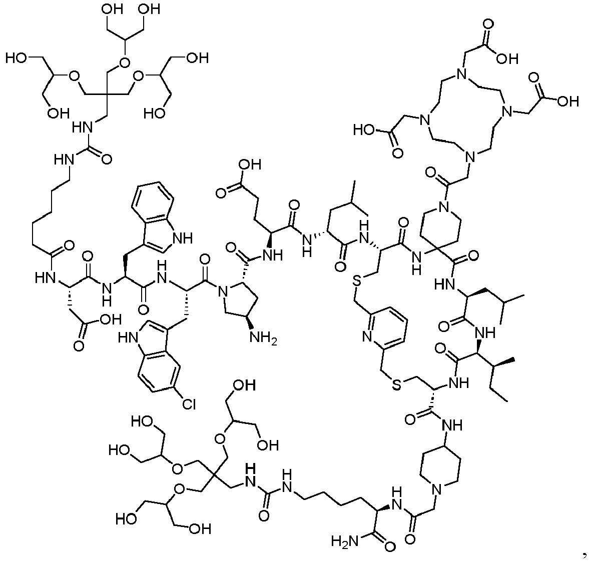
compound HAC3:TRIS:Aur-Asp-Trp-5Clw-Tap-Glu-leu-[Cys(2Lut)-Apc(DOTA)-Leu-Ile-Cys]-APAc-kur:TRIS:HAC3 (GIP-1011) of the following formula 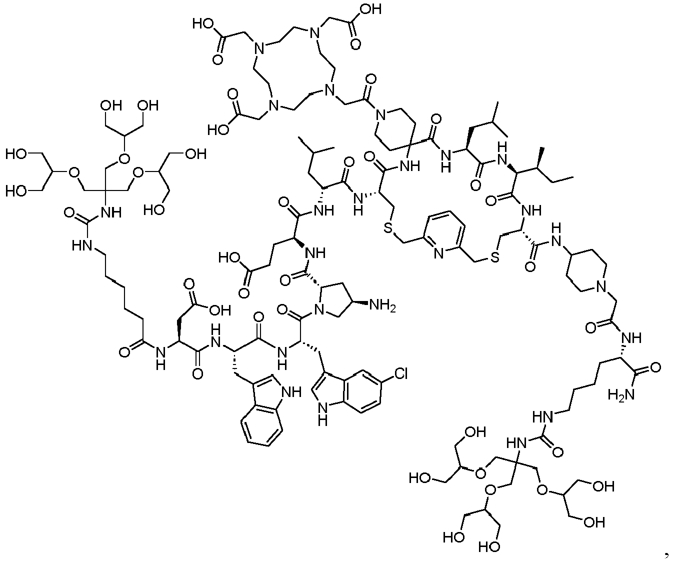
compound HPD3:PEO:Aur-Asp-Trp-5Clw-Tap-Glu-leu-[Cys(2Lut)-Apc(DOTA)-Leu-Ile-Cys]-APAc-kur:PEO:HPD3 (GIP-1012) of the following formula 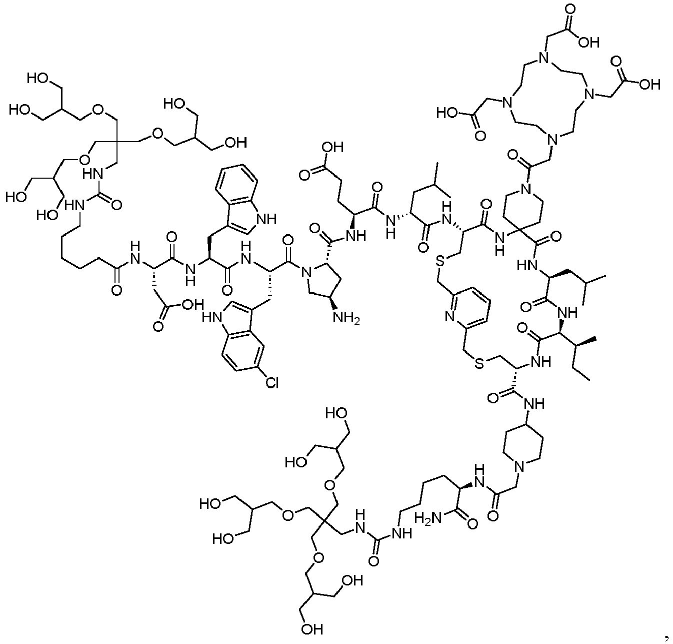
compound HPD3 :TRIS:Aur-Asp- Trp-5Clw- Tap-Glu-leu-[Cys(2Lut)-Apc(DOT A)-Leu-Ile-Cys]-APAc-kur:TRIS:HPD3 (GIP-1013) of the following formula 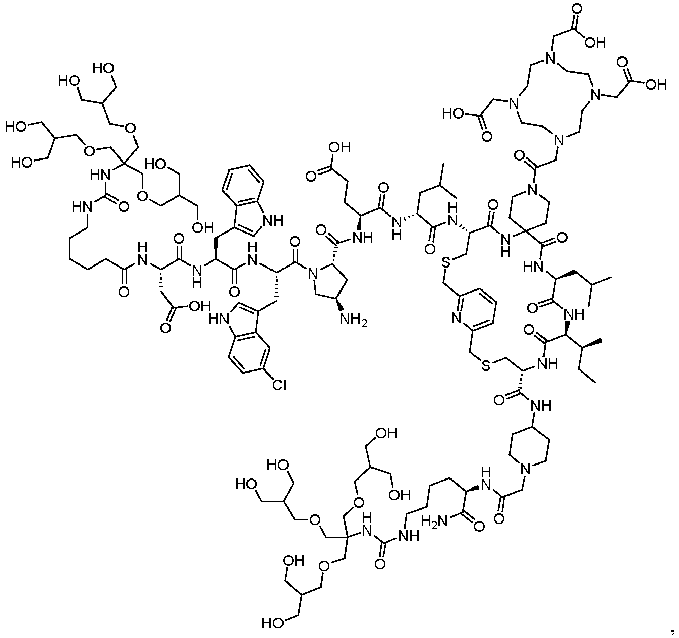
compound TRIS:Aur-Asp-Trp-5Clw-Tap-Glu-leu-[Cys(2Lut)-Apc(DOTA)-Leu-Ile-Cys]-APAc-kur:TRIS (GIP-1014) of the following formula 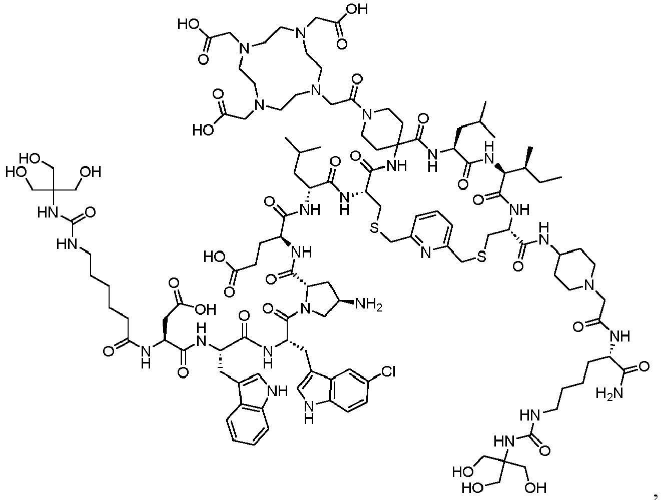
compound HPD2:Ahx-Asp-Trp-5Clw-Tap-Glu-leu-[Cys(2Lut)-Amk(DOTA)-Leu-Ile-Cys]-APAc-lys:HPD2 (GIP-1015) of the following formula 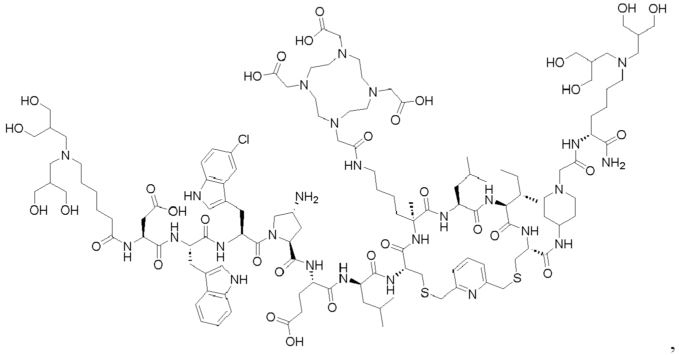
compound HPD3:PEO:Hyx-Asp-Trp-5Clw-Tap-Glu-leu-[Cys(2Lut)-Amk(DOTA)-Leu-Ile-Cys]-APAc-prx:PEO:HPD3 (GIP-1016) of the following formula 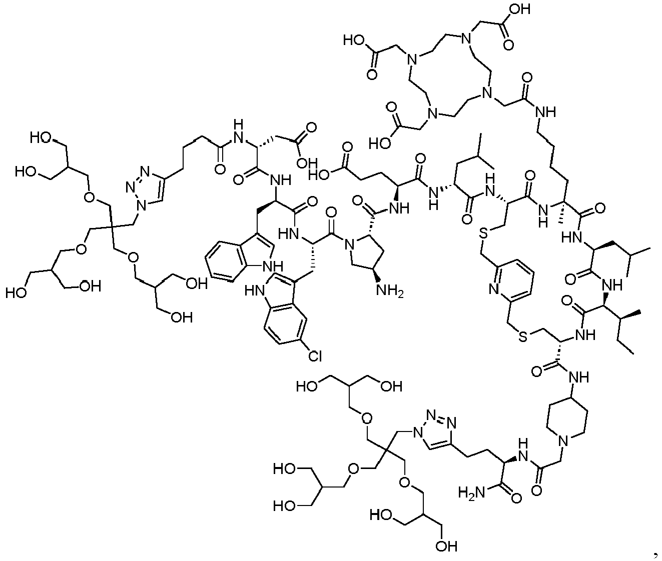
compound PEO:Aur-Asp-Trp-5Clw-Tap-Glu-leu-[Cys(2Lut)-Amk(DOTA)-Leu-Ile-Cys]-APAc-kur:PEO (GIP-1017) of the following formula 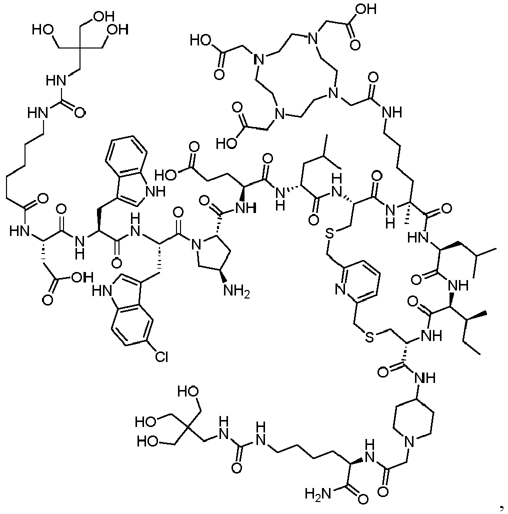
compound PEO:Hyx-Asp-Trp-5Clw-Tap-Glu-leu-[Cys(2Lut)-Amk(DOTA)-Leu-Ile-Cys]-APAc-prx:PEO (GIP-1018) of the following formula 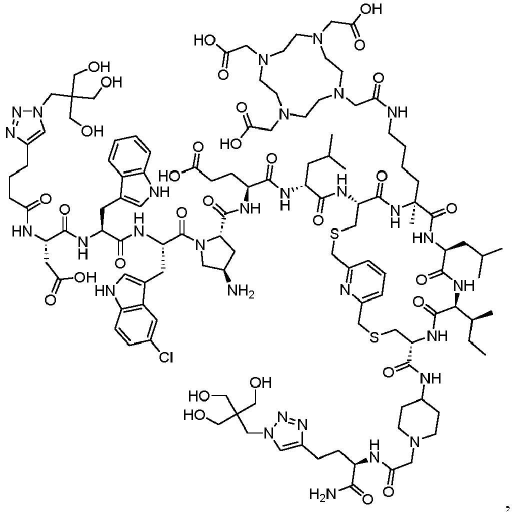
compound PEO2:Ahx-Asp-Trp-5Clw-Tap-Glu-leu-[Cys(2Lut)-Amk(DOTA)-Leu-Ile-Cys]-APAc-lys:PEO2 (GIP-1019) of the following formula 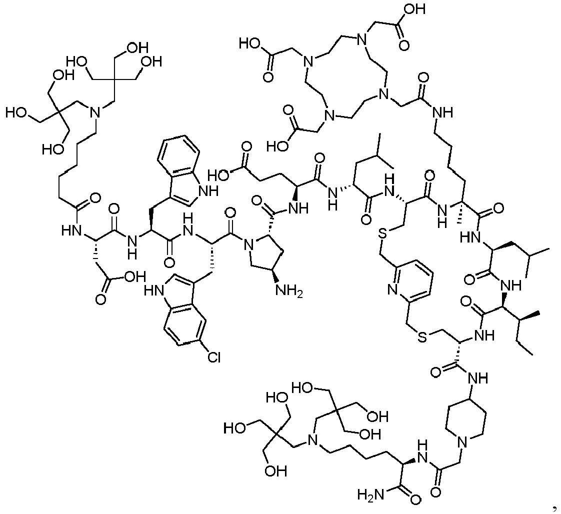
compound TRIS:Hyx-Asp-Trp-5Clw-Tap-Glu-leu-[Cys(2Lut)-Amk(DOTA)-Leu-Ile-Cys]-APAc-prx:TRIS (GIP-1020) of the following formula 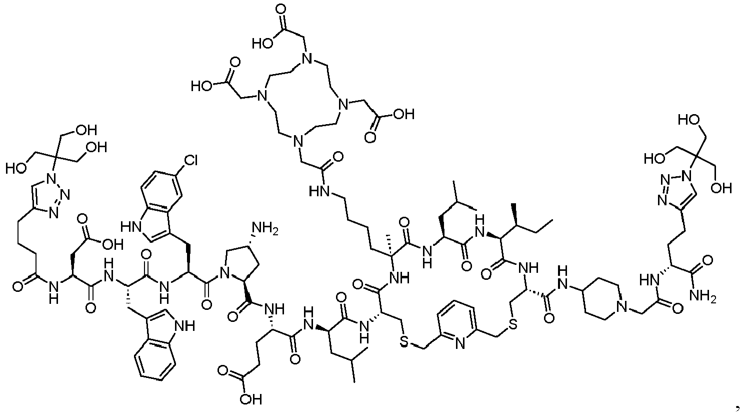
compound HPD3:TRIS:Hyx-Asp-Trp-5Clw-Tap-Glu-leu-[Cys(2Lut)-Amk(DOTA)-Leu-Ile-Cys]-APAc-prx:TRIS:HPD3 (GIP-1021) of the following formula 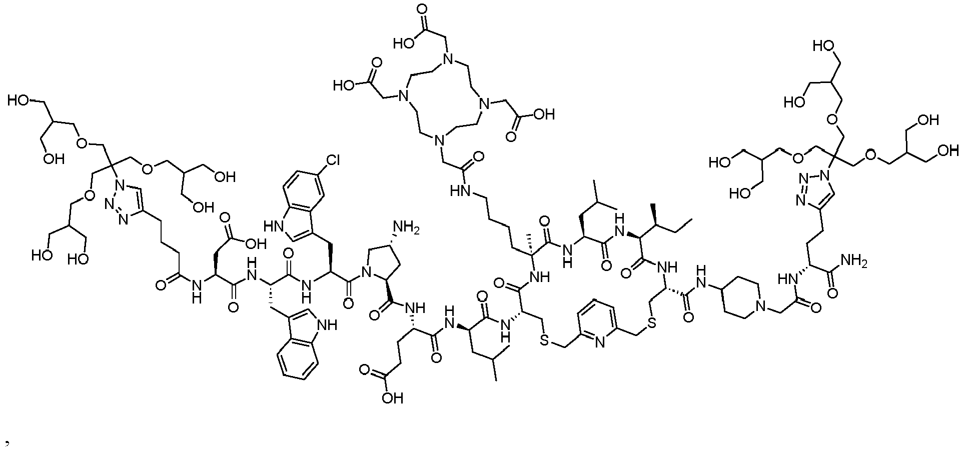
compound HAC3:TRIS:Hyx-Asp-Trp-SClw-Tap-Glu-leu-[Cys(2Lut)-Amk(DOTA)-Leu-Ile-Cys]-APAc-prx:TRIS:HAC3 (GIP-1022) of the following formula 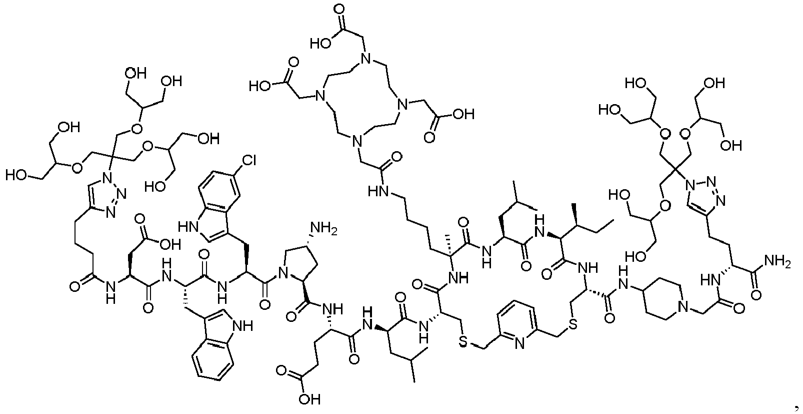
compound HAC3:PEO:Hyx-Asp-Trp-5Clw-Tap-Glu-leu-[Cys(2Lut)-Amk(DOTA)-Leu-Ile-Cys]-APAc-prx:PEO:HAC3 (GIP-1023) of the following formula 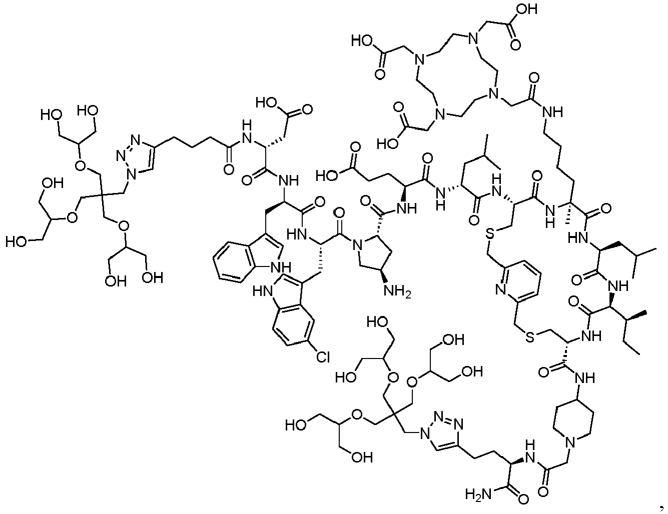
compound HAC3:PEO:Aur-Asp-Trp-5Clw-Tap-Glu-leu-[Cys(2Lut)-Amk(DOTA)-Leu-Ile-Cys]-APAc-kur:PEO:HAC3 (GIP-1024) of the following formula 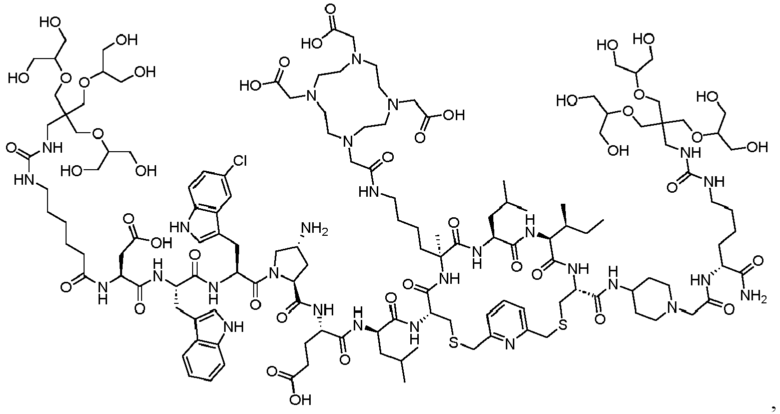
compound HAC3:TRIS:Aur-Asp-Trp-5Clw-Tap-Glu-leu-[Cys(2Lut)-Amk(DOTA)-Leu-Ile-Cys]-APAc-kur:TRIS:HAC3 (GIP-1025) of the following formula 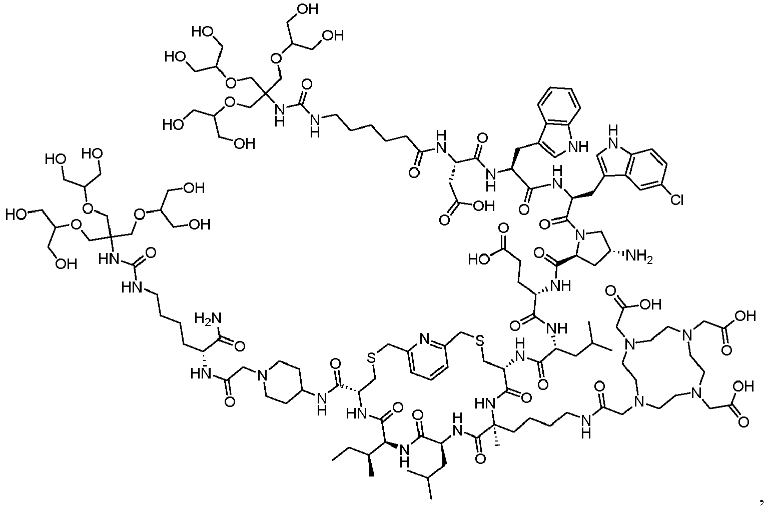
compound HPD3:PEO:Aur-Asp-Trp-5Clw-Tap-Glu-leu-[Cys(2Lut)-Amk(DOTA)-Leu-Ile-Cys]-APAc-kur:PEO:HPD3 (GIP-1026) of the following formula 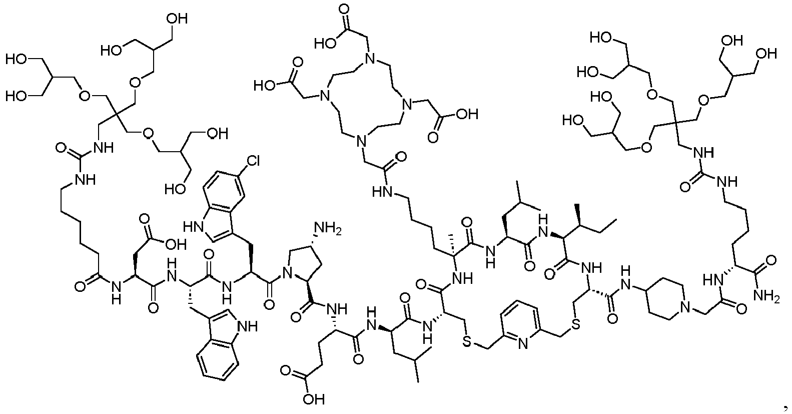
compound HPD3:TRIS:Aur-Asp-Trp-5Clw-Tap-Glu-leu-[Cys(2Lut)-Amk(DOTA)-Leu-Ile-Cys]-APAc-kur:TRIS:HPD3 (GIP-1027) of the following formula 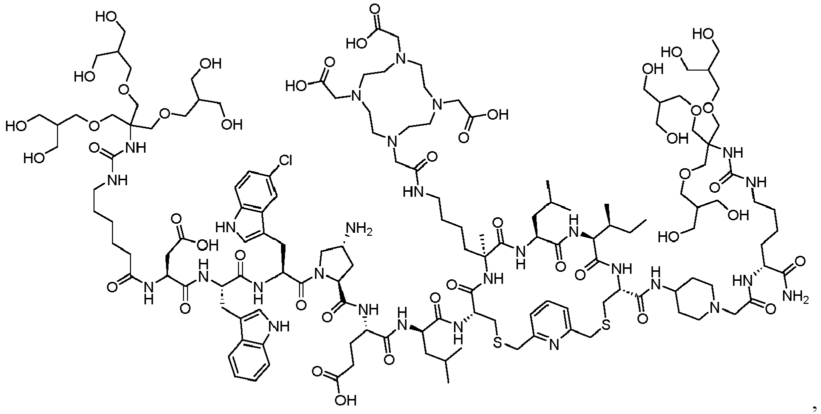
compound TRIS:Aur-Asp-Trp-5Clw-Tap-Glu-leu-[Cys(2Lut)-Amk(DOTA)-Leu-Ile-Cys]-APAc-kur:TRIS (GIP-1028) of the following formula 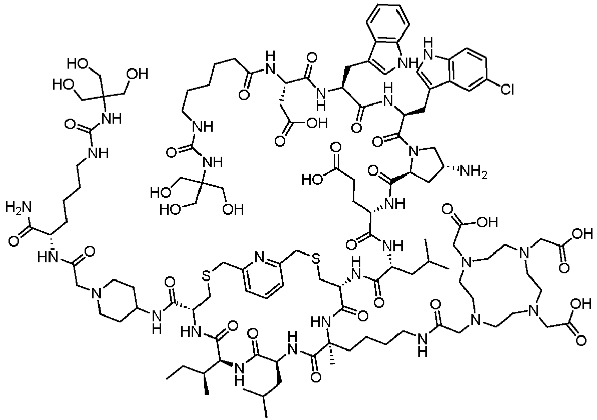
compound HPD2:Ahx-Asp-Trp-5Clw-Hyp-Glu-leu-[Cys(2Lut)-Apc(DOTA)-Leu-Ile-Cys]-APAc-lys:HPD2 (GIP-1029) of the following formula 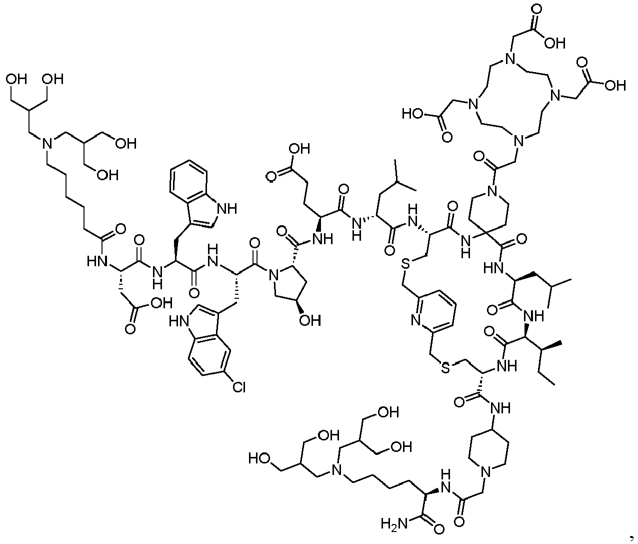
compound HPD3:PEO:Hyx-Asp-Trp-5Clw-Hyp-Glu-leu-[Cys(2Lut)-Apc(DOTA)-Leu-Ile-Cys]-APAc-prx:PEO:HPD3 (GIP-1030) of the following formula 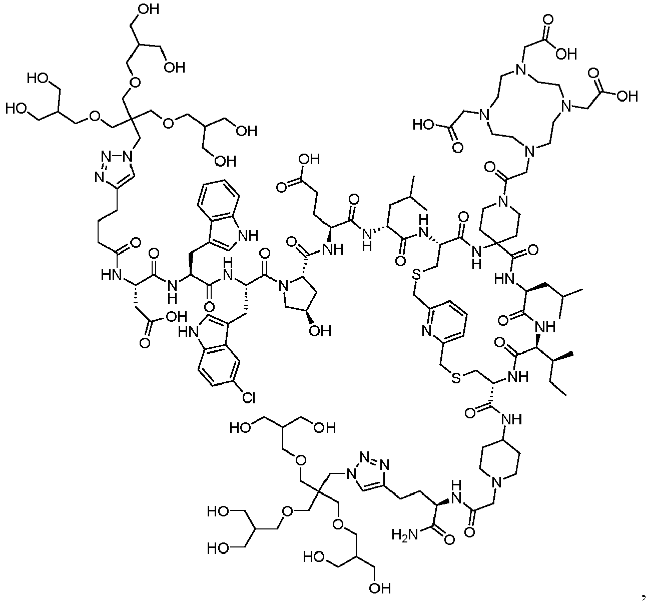
compound PEO:Aur-Asp-Trp-5Clw-Hyp-Glu-leu-[Cys(2Lut)-Apc(DOTA)-Leu-Ile-Cys]-APAc-kur:PEO (GIP-1031) of the following formula 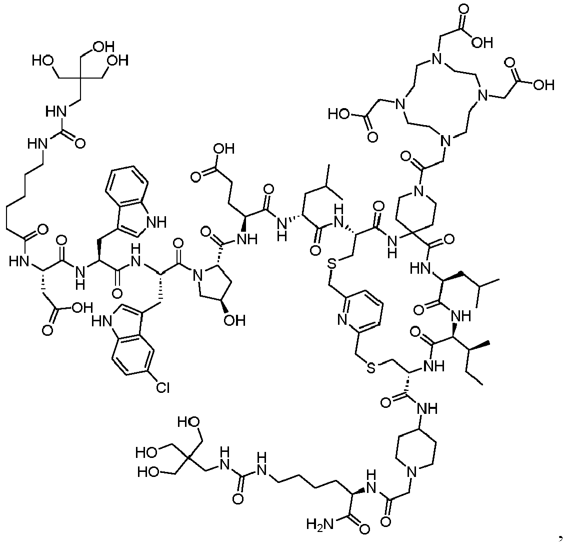
compound PEO:Hyx-Asp-Trp-5Clw-Hyp-Glu-leu-[Cys(2Lut)-Apc(DOTA)-Leu-Ile-Cys]-APAc-prx:PEO (GIP-1032) of the following formula 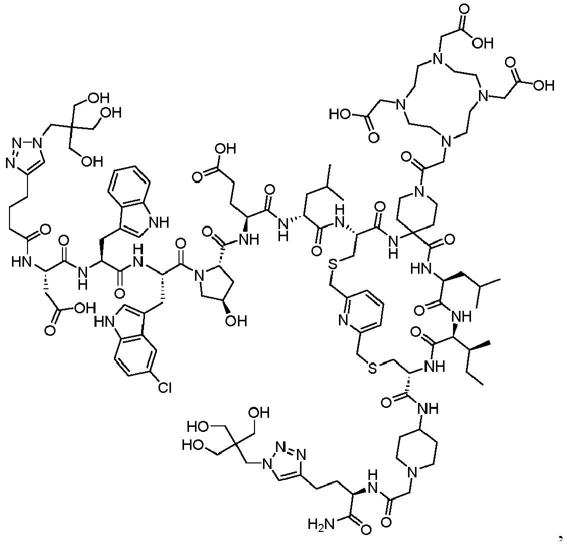
compound PEO2:Ahx-Asp-Trp-5Clw-Hyp-Glu-leu-[Cys(2Lut)-Apc(DOTA)-Leu-Ile-Cys]-APAc-lys:PEO2 (GIP-1033) of the following formula 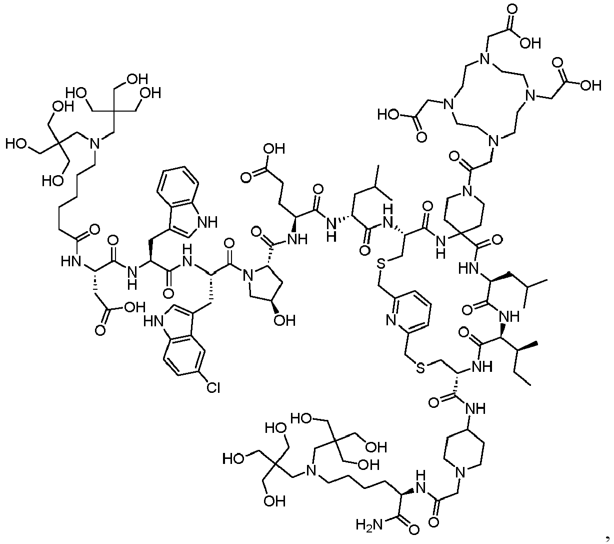
compound TRIS:Hyx-Asp-Trp-5Clw-Hyp-Glu-leu-[Cys(2Lut)-Apc(DOTA)-Leu-Ile-Cys]-APAc-prx:TRIS (GIP-1034) of the following formula 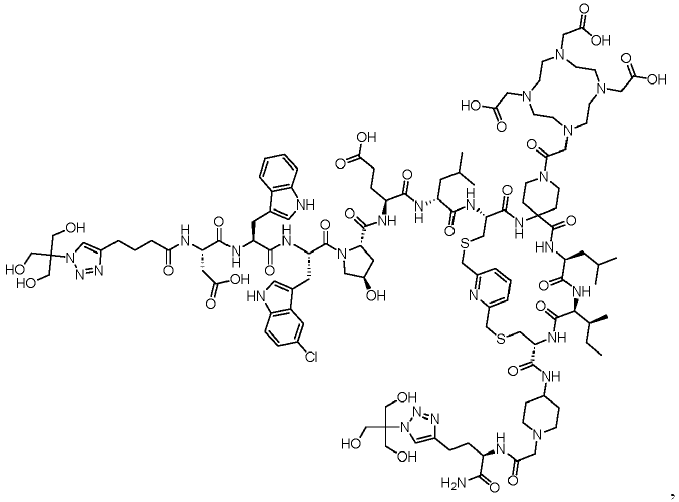
compound HPD3:TRIS:Hyx-Asp-Trp-5Clw-Hyp-Glu-leu-[Cys(2Lut)-Apc(DOTA)-Leu-Ile-Cys]-APAc-prx:TRIS:HPD3 (GIP-1035) of the following formula 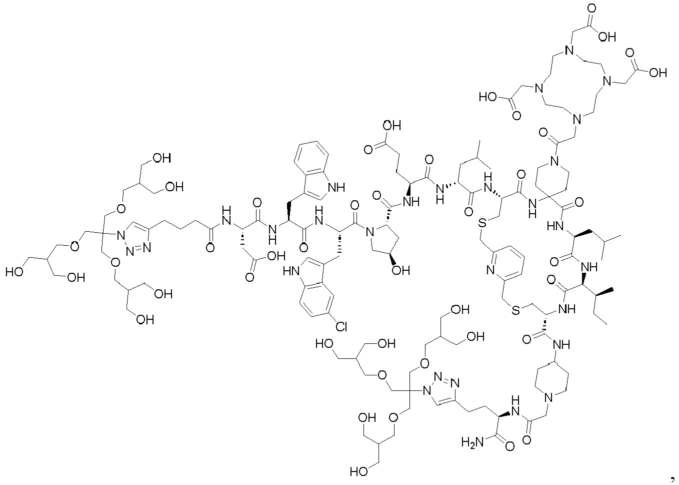
compound HAC3:TRIS:Hyx-Asp-Trp-5Clw-Hyp-Glu-leu-[Cys(2Lut)-Apc(DOTA)-Leu-Ile-Cys]-APAc-prx:TRIS:HAC3 (GIP-1036) of the following formula 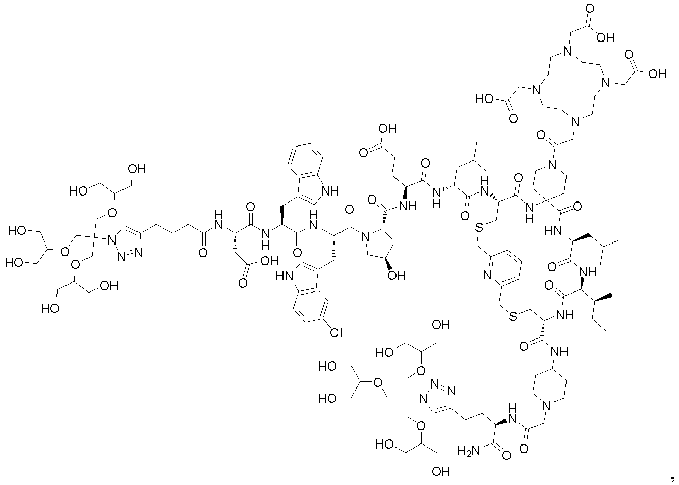
compound HAC3:PEO:Hyx-Asp-Trp-5Clw-Hyp-Glu-leu-[Cys(2Lut)-Apc(DOTA)-Leu-Ile-Cys]-APAc-prx:PEO:HAC3 (GIP-1037) of the following formula 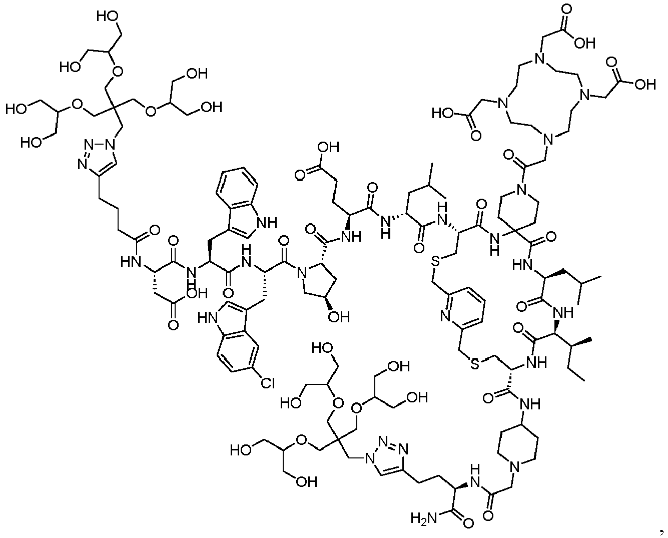
compound HAC3:PEO:Aur-Asp-Trp-5Clw-Hyp-Glu-leu-[Cys(2Lut)-Apc(DOTA)-Leu-Ile-Cys]-APAc-kur:PEO:HAC3 (GIP-1038) of the following formula 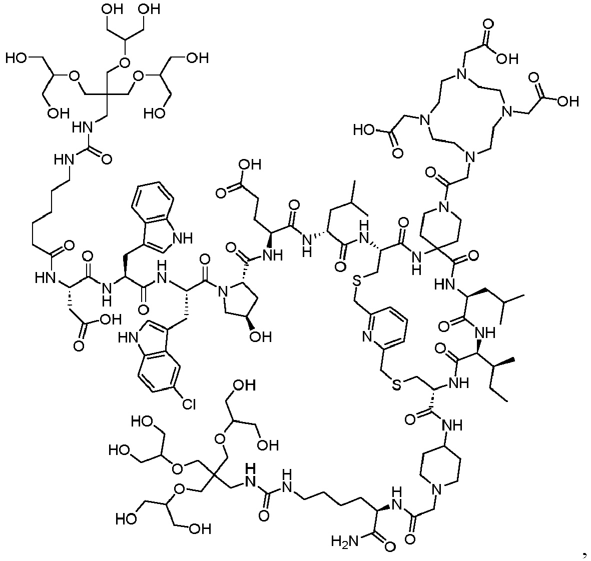
compound HAC3:TRIS:Aur-Asp-Trp-5Clw-Hyp-Glu-leu-[Cys(2Lut)-Apc(DOTA)-Leu-Ile-Cys]-APAc-kur:TRIS:HAC3 (GIP-1039) of the following formula 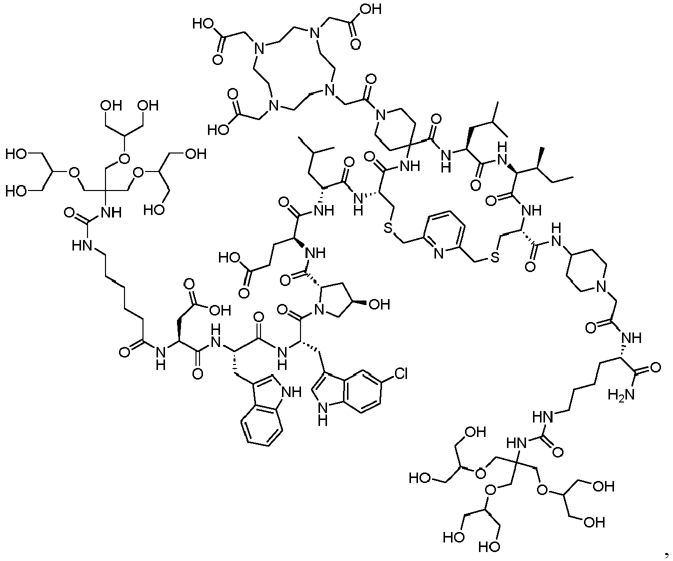
compound HPD3:PEO:Aur-Asp-Trp-5Clw-Hyp-Glu-leu-[Cys(2Lut)-Apc(DOTA)-Leu-Ile-Cys]-APAc-kur:PEO:HPD3 (GIP-1040) of the following formula 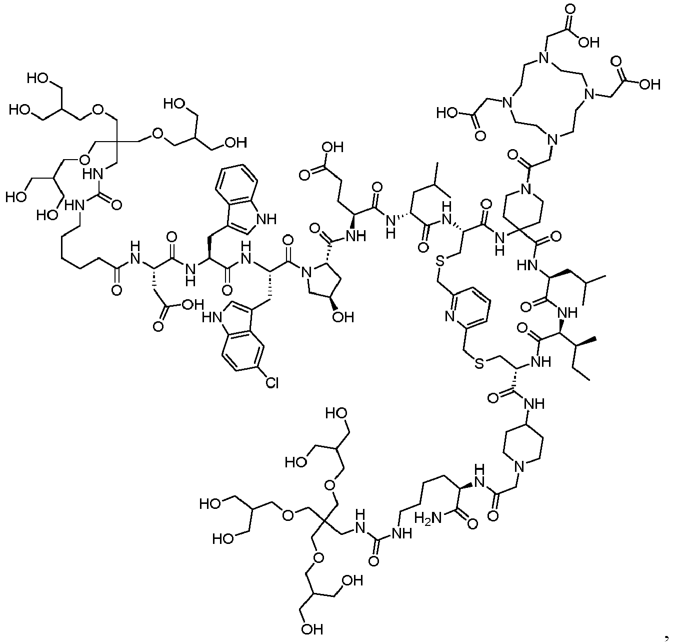
compound HPD3:TRIS:Aur-Asp-Trp-5Clw-Hyp-Glu-leu-[Cys(2Lut)-Apc(DOTA)-Leu-Ile-Cys]-APAc-kur:TRIS:HPD3 (GIP-1041) of the following formula 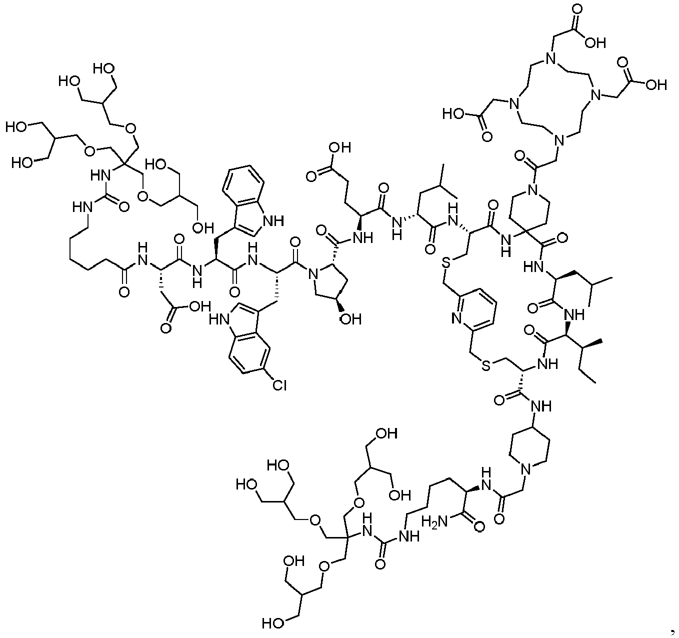
compound TRIS:Aur-Asp-Trp-5Clw-Hyp-Glu-leu-[Cys(2Lut)-Apc(DOTA)-Leu-Ile-Cys]-APAc-kur:TRIS (GIP-1042) of the following formula 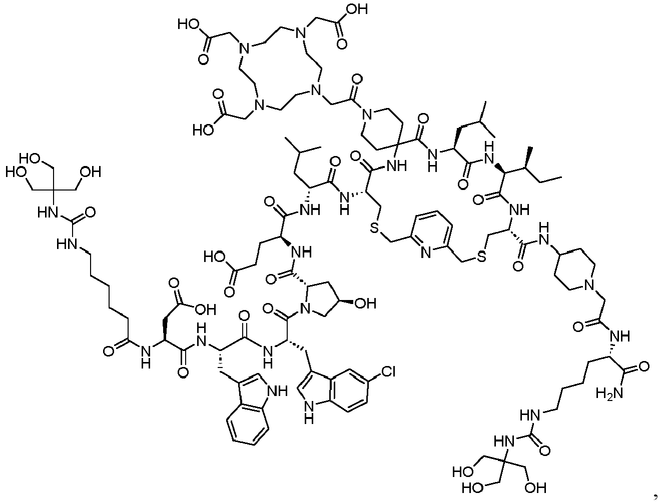
compound HPD2:Ahx-Asp-Trp-5Clw-Hyp-Glu-leu-[Cys(2Lut)-Amk(DOTA)-Leu-Ile-Cys]-APAc-lys:HPD2 (GIP-1043) of the following formula 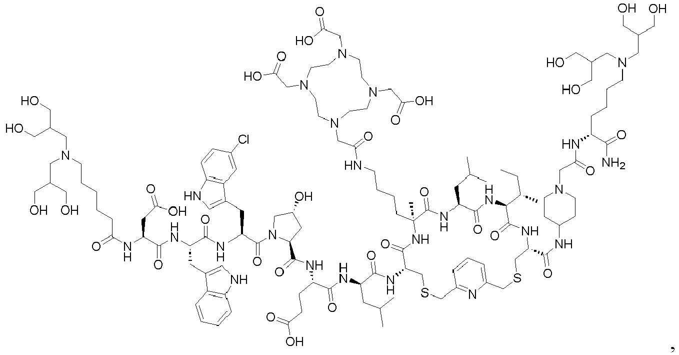
compound HPD3:PEO:Hyx-Asp-Trp-5Clw-Hyp-Glu-leu-[Cys(2Lut)-Amk(DOTA)-Leu-Ile-Cys]-APAc-prx:PEO:HPD3 (GIP-1044) of the following formula 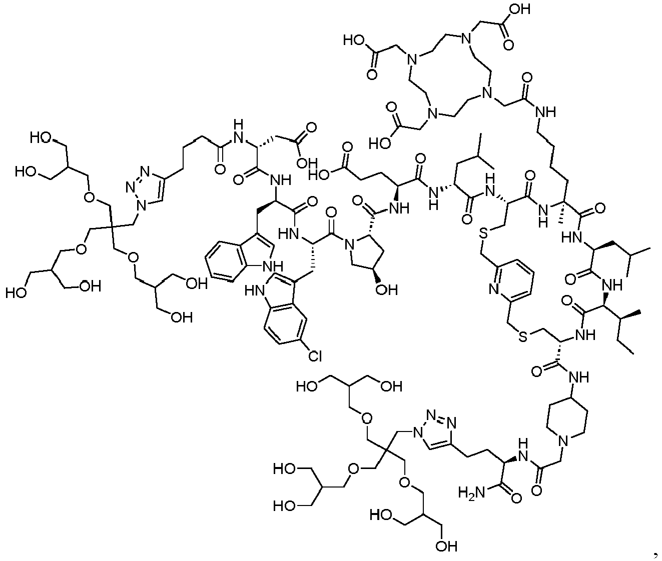
compound PEO:Aur-Asp-Trp-5Clw-Hyp-Glu-leu-[Cys(2Lut)-Amk(DOTA)-Leu-Ile-Cys]-APAc-kur:PEO (GIP-1045) of the following formula 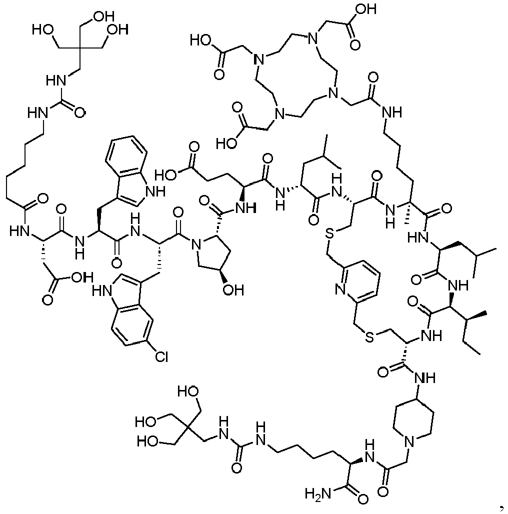
compound PEO:Hyx-Asp-Trp-5Clw-Hyp-Glu-leu-[Cys(2Lut)-Amk(DOTA)-Leu-Ile-Cys]-APAc-prx:PEO (GIP-1046) of the following formula 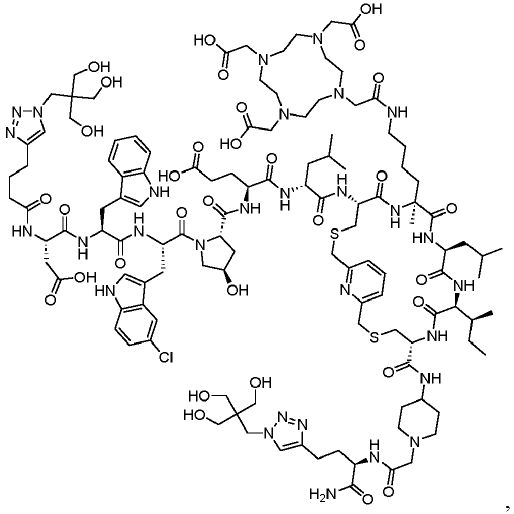
compound PEO2:Ahx-Asp-Trp-5Clw-Hyp-Glu-leu-[Cys(2Lut)-Amk(DOTA)-Leu-Ile-Cys]-APAc-lys:PEO2 (GIP-1047) of the following formula 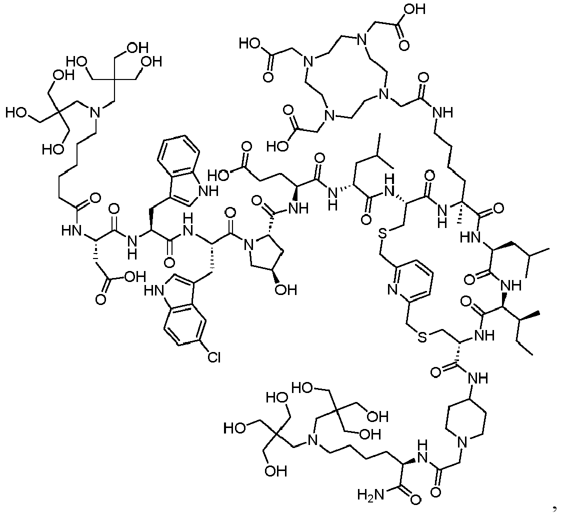
compound TRIS:Hyx-Asp-Trp-5Clw-Hyp-Glu-leu-[Cys(2Lut)-Amk(DOTA)-Leu-Ile-Cys]-APAc-prx:TRIS (GIP-1048) of the following formula 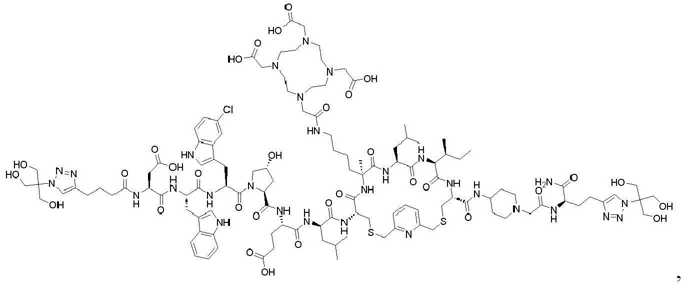
compound HPD3:TRIS:Hyx-Asp-Trp-5Clw-Hyp-Glu-leu-[Cys(2Lut)-Amk(DOTA)-Leu-Ile-Cys]-APAc-prx:TRIS:HPD3 (GIP-1049) of the following formula 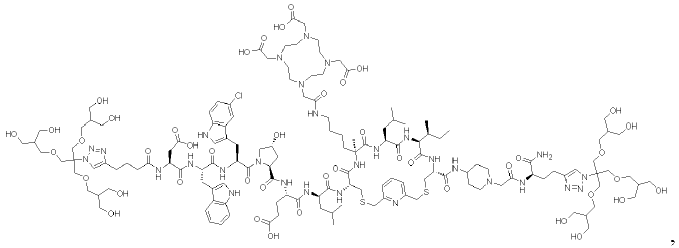
compound HAC3:TRIS:Hyx-Asp-Trp-5Clw-Hyp-Glu-leu-[Cys(2Lut)-Amk(DOTA)-Leu-Ile-Cys]-APAc-prx:TRIS:HAC3 (GIP-1050) of the following formula 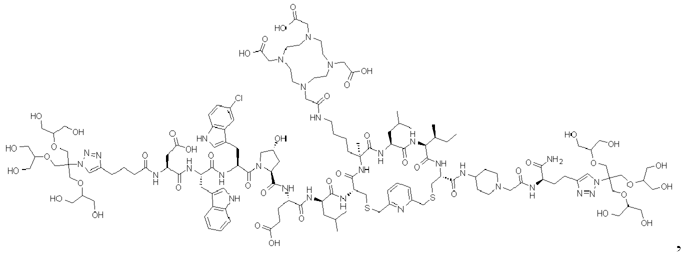
compound HAC3:PEO:Hyx-Asp-Trp-5Clw-Hyp-Glu-leu-[Cys(2Lut)-Amk(DOTA)-Leu-Ile-Cys]-APAc-prx:PEO:HAC3 (GIP-1051) of the following formula 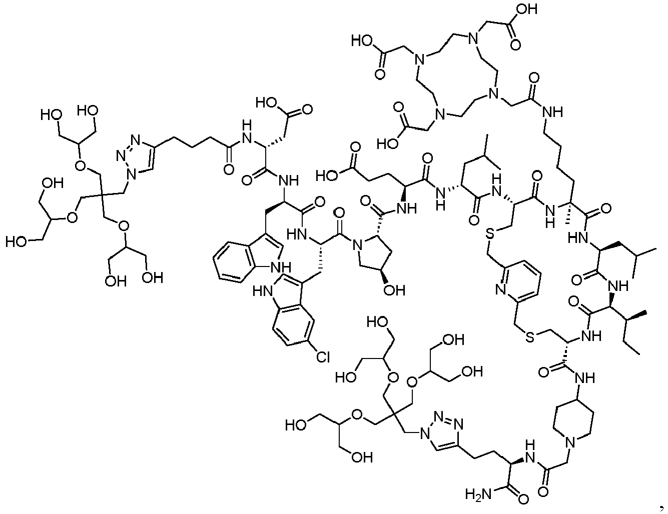
compound HAC3:PEO:Aur-Asp-Trp-5Clw-Hyp-Glu-leu-[Cys(2Lut)-Amk(DOTA)-Leu-Ile-Cys]-APAc-kur:PEO:HAC3 (GIP-1052) of the following formula 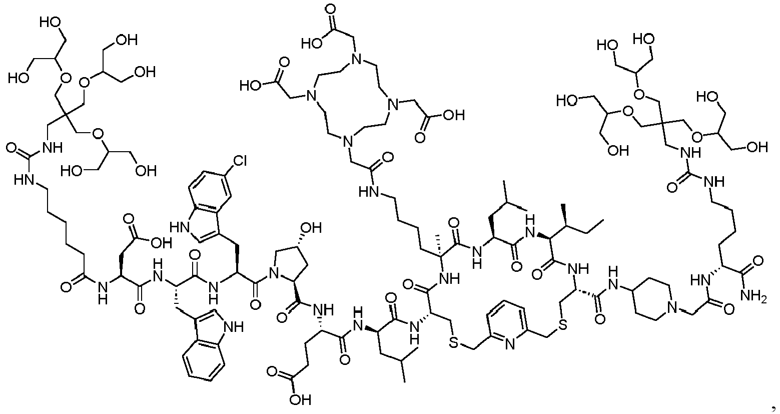
compound HAC3:TRIS:Aur-Asp-Trp-5Clw-Hyp-Glu-leu-[Cys(2Lut)-Amk(DOTA)-Leu-Ile-Cys]-APAc-kur:TRIS:HAC3 (GIP-1053) of the following formula 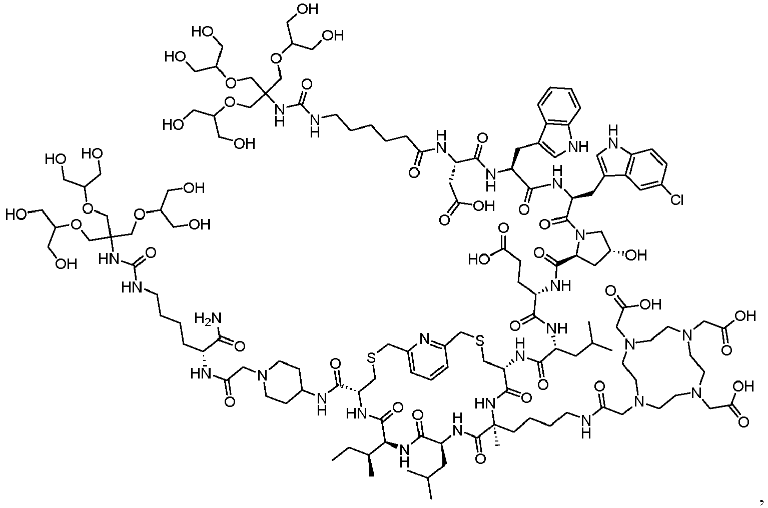
compound HPD3:PEO:Aur-Asp-Trp-5Clw-Hyp-Glu-leu-[Cys(2Lut)-Amk(DOTA)-Leu-Ile-Cys]-APAc-kur:PEO:HPD3 (GIP-1054) of the following formula 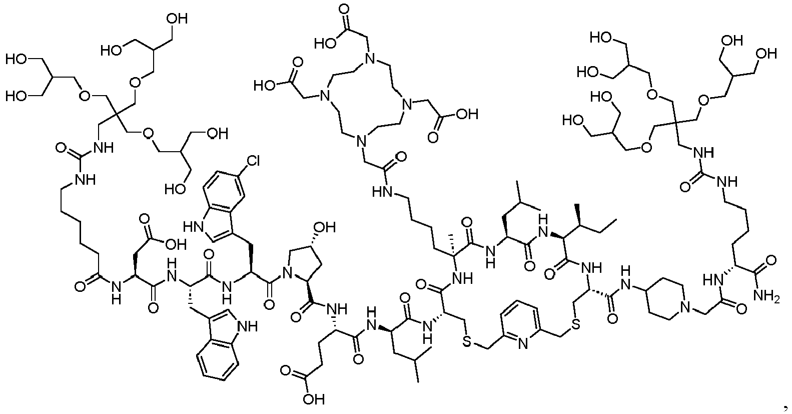
compound HPD3:TRIS:Aur-Asp-Trp-5Clw-Hyp-Glu-leu-[Cys(2Lut)-Amk(DOTA)-Leu-Ile-Cys]-APAc-kur:TRIS:HPD3 (GIP-1055) of the following formula 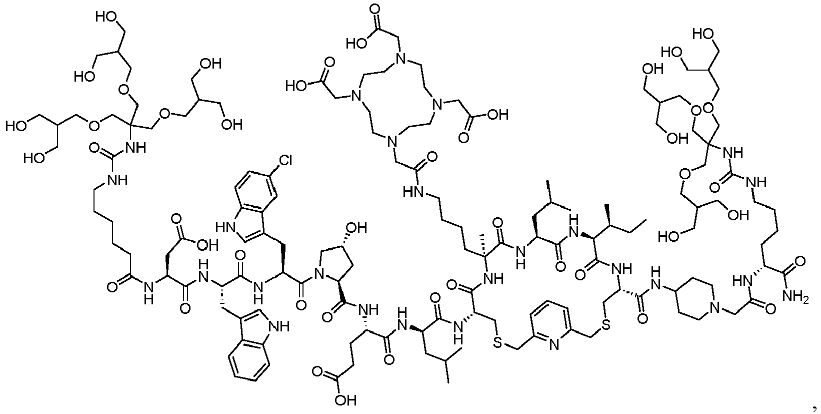
compound TRIS:Aur-Asp-Trp-5Clw-Hyp-Glu-leu-[Cys(2Lut)-Amk(DOTA)-Leu-Ile-Cys]-APAc-kur:TRIS (GIP-1056) of the following formula 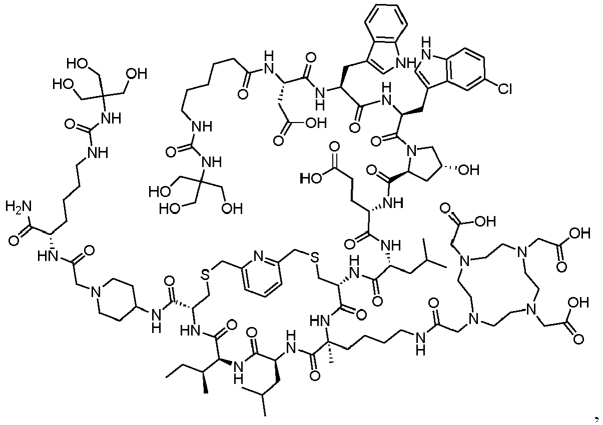
compound HAC2:HPD:Aur-Asp-Trp-5Clw-Tap-Glu-leu-[Cys(2Lut)-Apc(DOTA)-Leu-Ile-Cys]-APAc-kur:HPD:HAC2 (GIP-1057) of the following formula 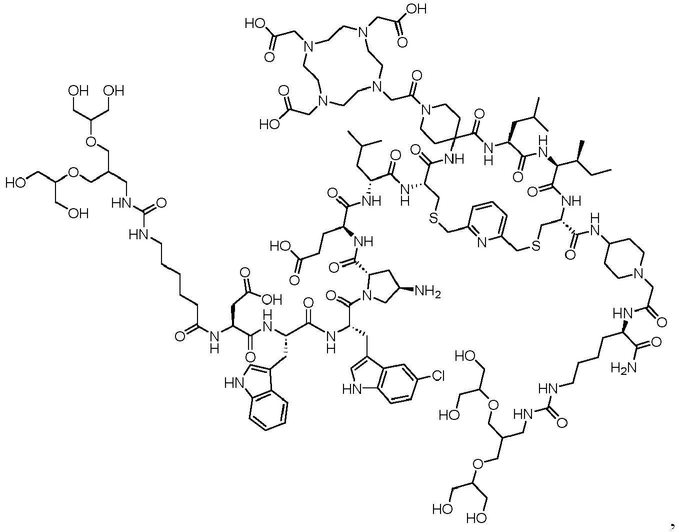
compound HAC2:HPD:Hyx-Asp-Trp-5Clw-Tap-Glu-leu-[Cys(2Lut)-Apc(DOTA)-Leu-Ile-Cys]-APAc-prx:HPD:HAC2 (GIP-1058) of the following formula 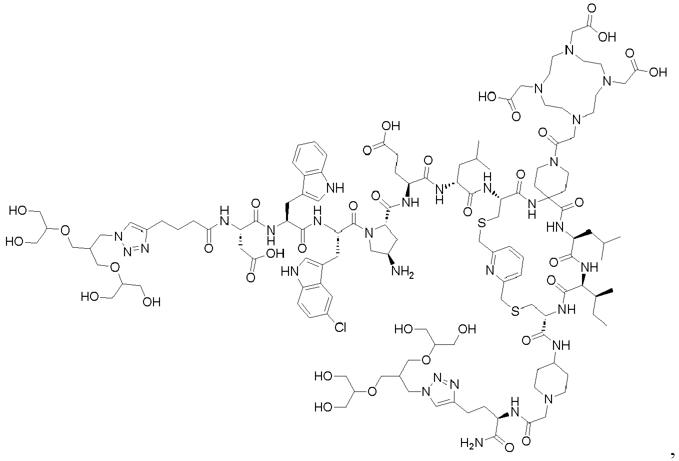
compound HAC4:HPD2:Ahx-Asp-Trp-5Clw-Tap-Glu-leu-[Cys(2Lut)-Apc(DOTA)-Leu-Ile-Cys]-APAc-lys:HPD2:HAC4 (GIP-1059) of the following formula 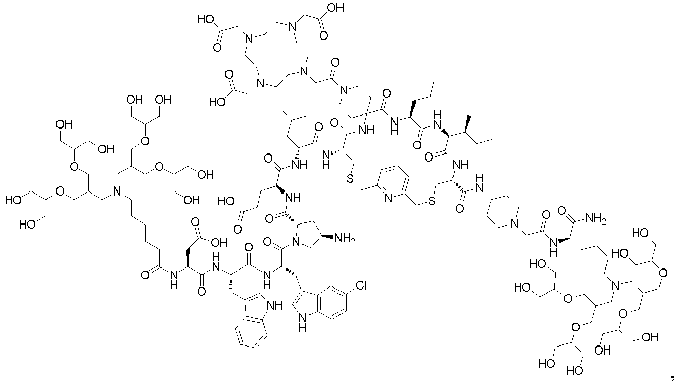
compound HPD2:HPD:Aur-Asp-Trp-5Clw-Tap-Glu-leu-[Cys(2Lut)-Apc(DOTA)-Leu-Ile-Cys]-APAc-kur:HPD:HPD2 (GIP-1060) of the following formula 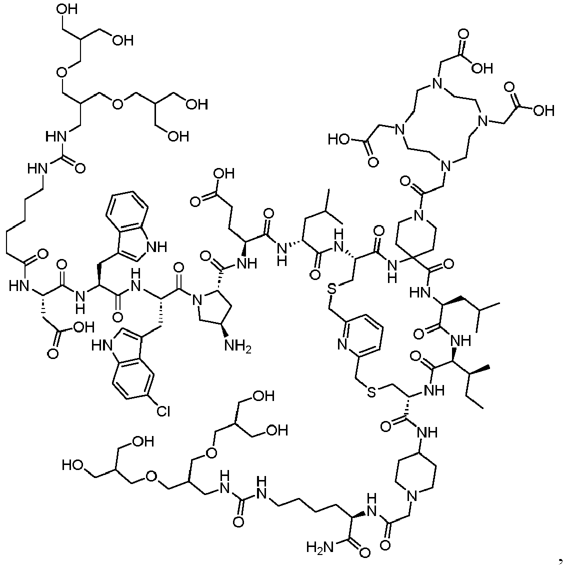
compound HPD2:HPD:Hyx-Asp-Trp-5Clw-Tap-Glu-leu-[Cys(2Lut)-Apc(DOTA)-Leu-Ile-Cys]-APAc-prx:HPD:HPD2 (GIP-1061) of the following formula 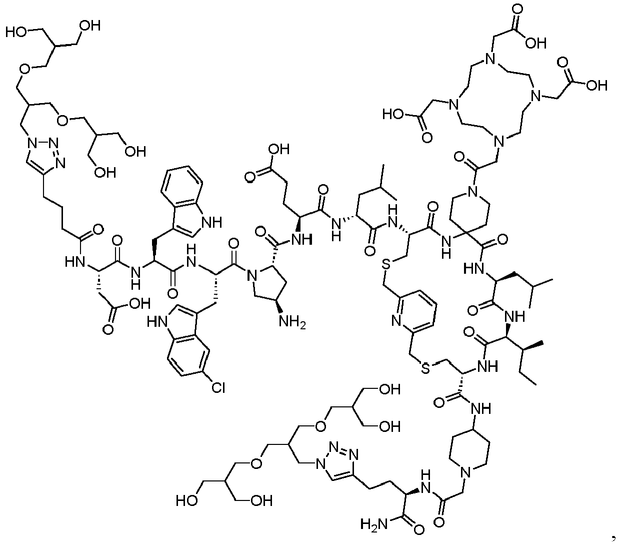
compound HPD4:HPD2:Ahx-Asp-Trp-5Clw-Tap-Glu-leu-[Cys(2Lut)-Apc(DOTA)-Leu-Ile-Cys]-APAc-lys:HPD2:HPD4 (GIP-1062) of the following formula 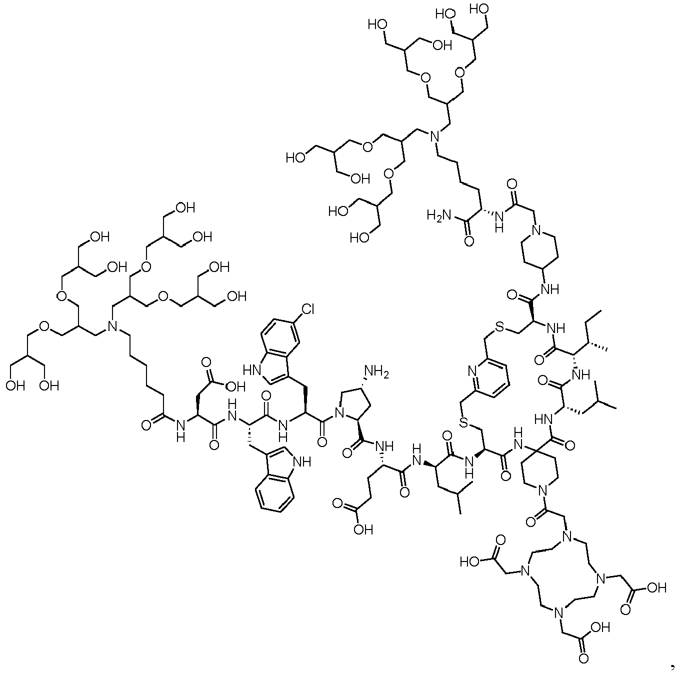
compound HAC2:HAC:Aur-Asp-Trp-5Clw-Tap-Glu-leu-[Cys(2Lut)-Apc(DOTA)-Leu-Ile-Cys]-APAc-kur:HAC:HAC2 (GIP-1063) of the following formula 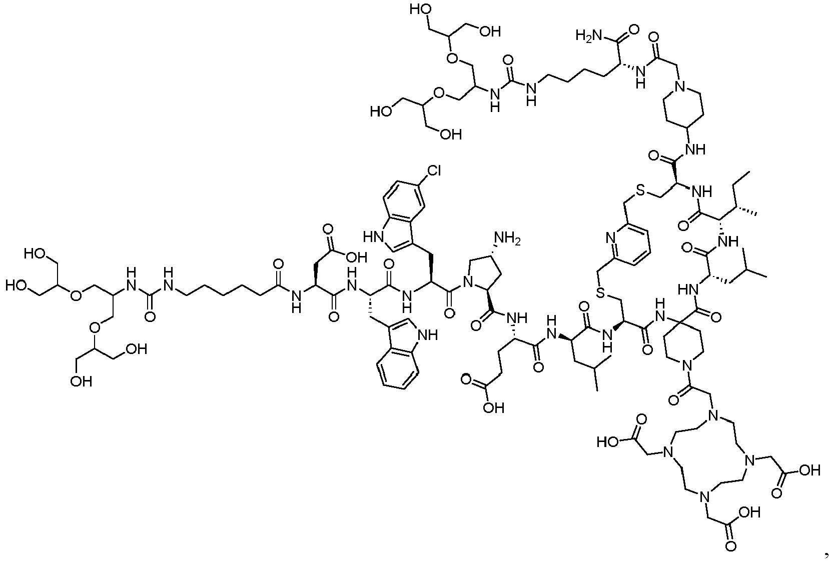
compound HPD2:HAC:Aur-Asp-Trp-5Clw-Tap-Glu-leu-[Cys(2Lut)-Apc(DOTA)-Leu-Ile-Cys]-APAc-kur:HAC:HPD2 (GIP-1064) of the following formula 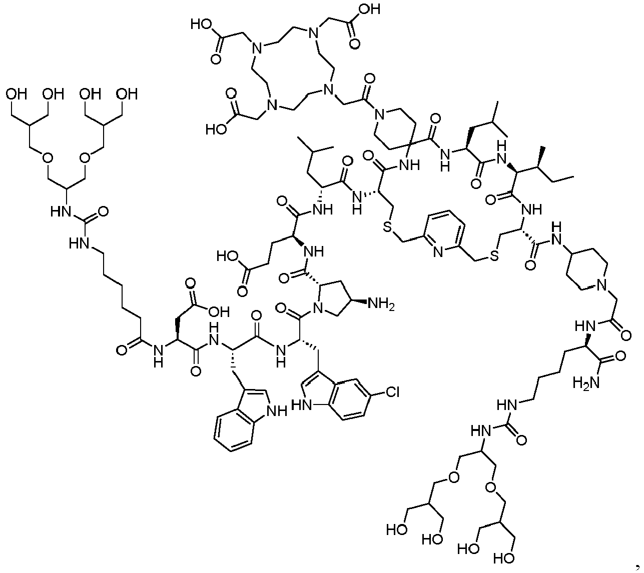
compound HAC2:HPD:Aur-Asp-Trp-5Clw-Tap-Glu-leu-[Cys(2Lut)-Amk(DOTA)-Leu-Ile-Cys]-APAc-kur:HPD:HAC2 (GIP-1065) of the following formula 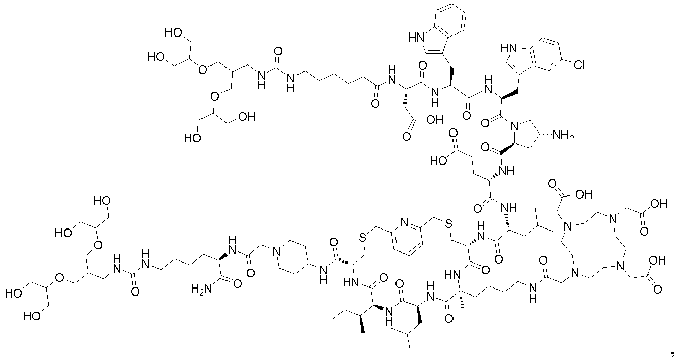
compound HAC2:HPD:Hyx-Asp-Trp-5Clw-Tap-Glu-leu-[Cys(2Lut)-Amk(DOTA)-Leu-Ile-Cys]-APAc-prx:HPD:HAC2 (GIP-1066) of the following formula 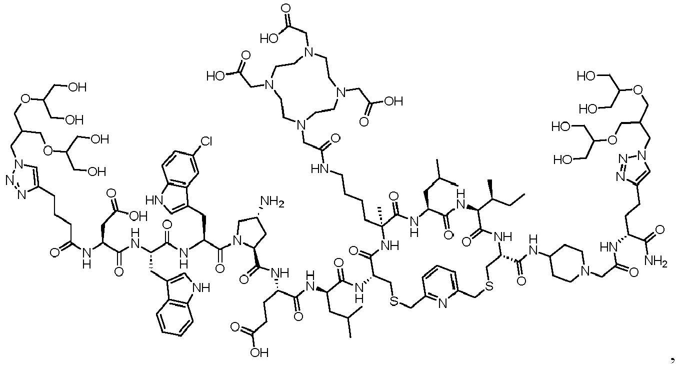
compound HAC4:HPD2:Ahx-Asp-Trp-5Clw-Tap-Glu-leu-[Cys(2Lut)-Amk(DOTA)-Leu-Ile-Cys]-APAc-lys:HPD2:HAC4 (GIP-1067) of the following formula 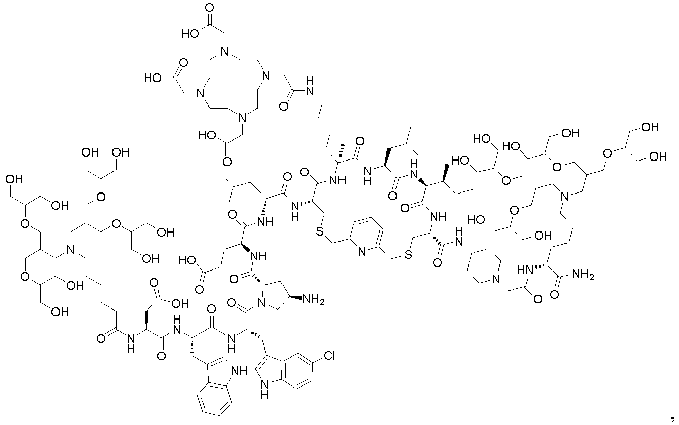
compound HPD2:HPD:Aur-Asp-Trp-5Clw-Tap-Glu-leu-[Cys(2Lut)-Amk(DOTA)-Leu-Ile-Cys]-APAc-kur:HPD:HPD2 (GIP-1068) of the following formula 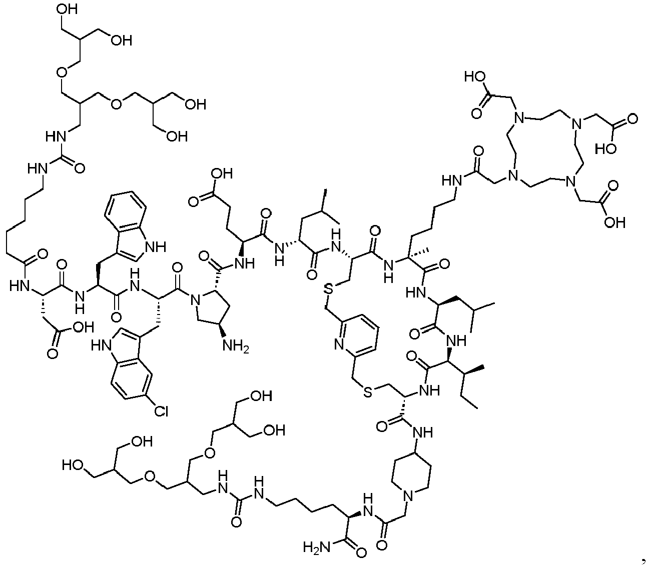
compound HPD2:HPD:Hyx-Asp-Trp-5Clw-Tap-Glu-leu-[Cys(2Lut)-Amk(DOTA)-Leu-Ile-Cys]-APAc-prx:HPD:HPD2 (GIP-1069) of the following formula 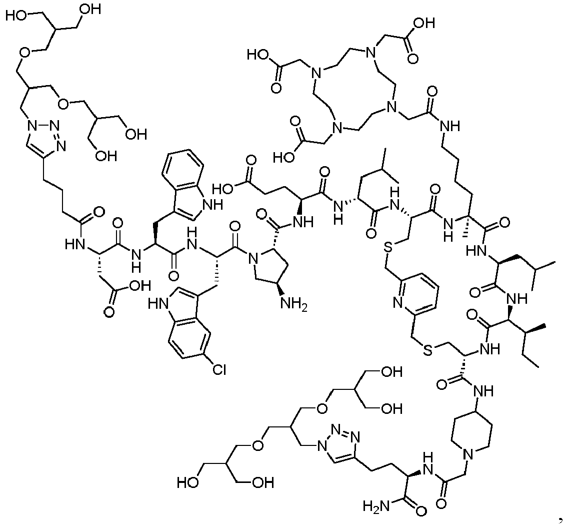
compound HPD4:HPD2:Ahx-Asp-Trp-5Clw-Tap-Glu-leu-[Cys(2Lut)-Amk(DOTA)-Leu-Ile-Cys]-APAc-lys:HPD2:HPD4 (GIP-1070) of the following formula 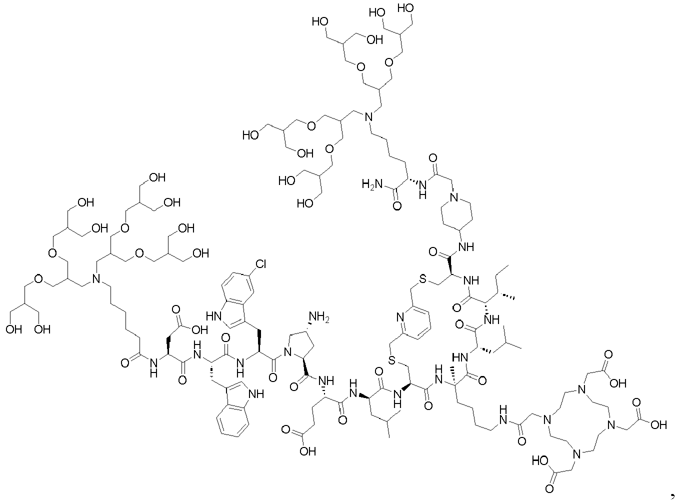
compound HAC2:HAC:Aur-Asp-Trp-5Clw-Tap-Glu-leu-[Cys(2Lut)-Amk(DOTA)-Leu-Ile-Cys]-APAc-kur:HAC:HAC2 (GIP-1071) of the following formula 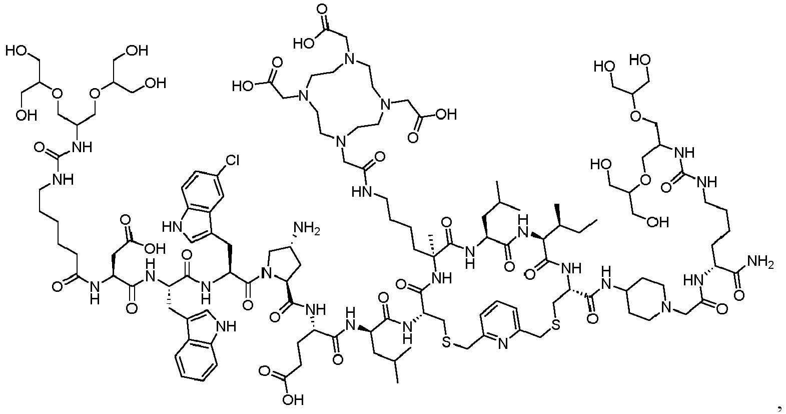
compound HPD2:HAC:Aur-Asp-Trp-5Clw-Tap-Glu-leu-[Cys(2Lut)-Amk(DOTA)-Leu-Ile-Cys]-APAc-kur:HAC:HPD2 (GIP-1072) of the following formula 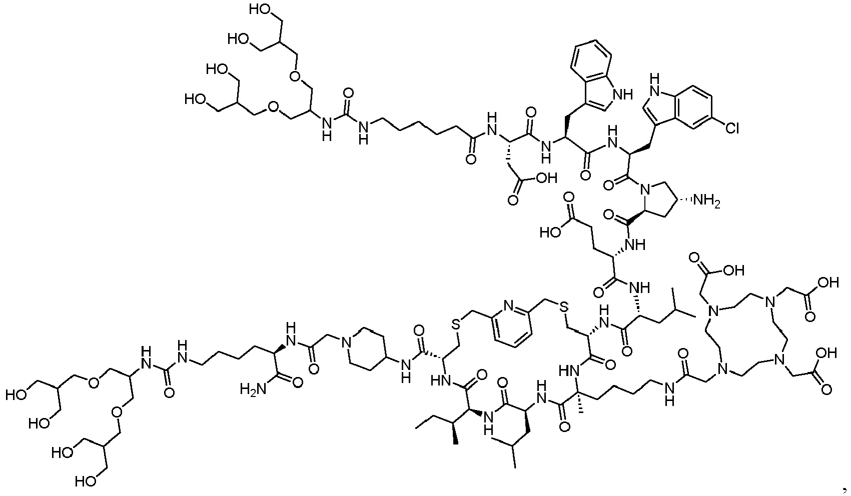
compound HAC2:HPD:Aur-Asp-Trp-5Clw-Hyp-Glu-leu-[Cys(2Lut)-Apc(DOTA)-Leu-Ile-Cys]-APAc-kur:HPD:HAC2 (GIP-1073) of the following formula 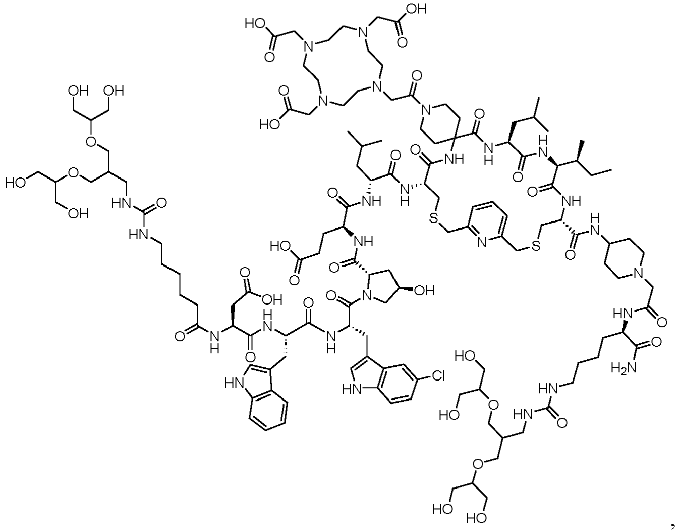
compound HAC2:HPD:Hyx-Asp-Trp-5Clw-Hyp-Glu-leu-[Cys(2Lut)-Apc(DOTA)-Leu-Ile-Cys]-APAc-prx:HPD:HAC2 (GIP-1074) of the following formula 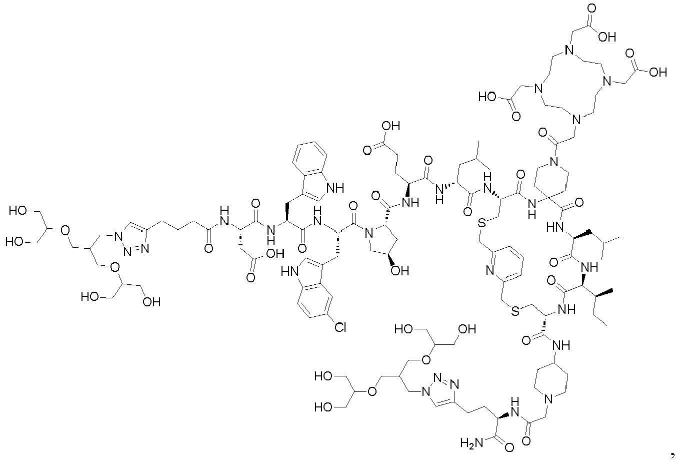
compound HAC4:HPD2:Ahx-Asp-Trp-5Clw-Hyp-Glu-leu-[Cys(2Lut)-Apc(DOTA)-Leu-Ile-Cys]-APAc-lys:HPD2:HAC4 (GIP-1075) of the following formula 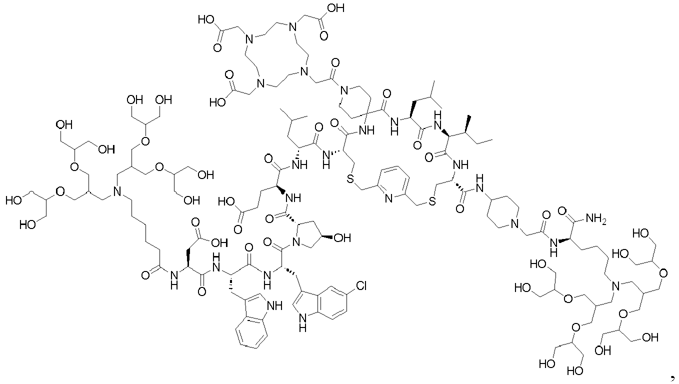
compound HPD2:HPD:Aur-Asp-Trp-5Clw-Hyp-Glu-leu-[Cys(2Lut)-Apc(DOTA)-Leu-Ile-Cys]-APAc-kur:HPD:HPD2 (GIP-1076) of the following formula 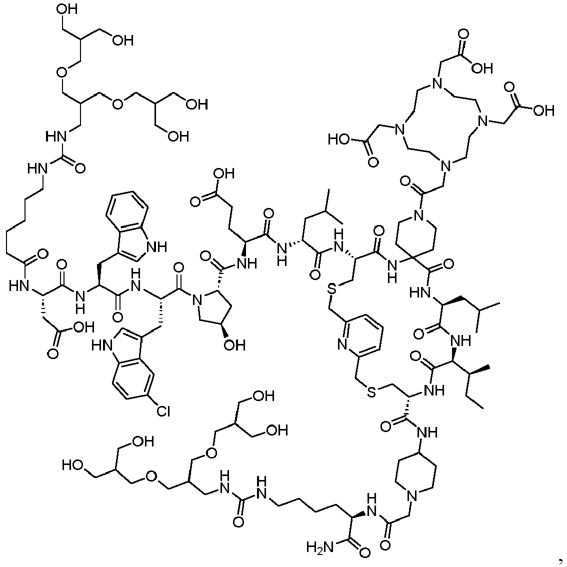
compound HPD2:HPD:Hyx-Asp-Trp-5Clw-Hyp-Glu-leu-[Cys(2Lut)-Apc(DOTA)-Leu-Ile-Cys]-APAc-prx:HPD:HPD2 (GIP-1077) of the following formula 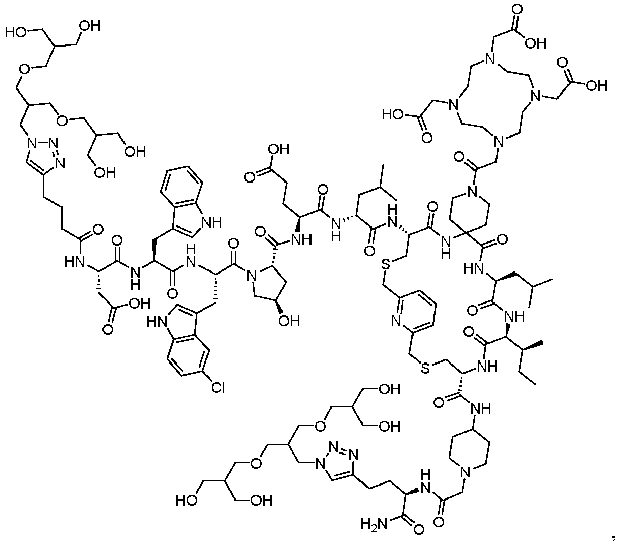
compound HPD4:HPD2:Ahx-Asp-Trp-5Clw-Hyp-Glu-leu-[Cys(2Lut)-Apc(DOTA)-Leu-Ile-Cys]-APAc-lys:HPD2:HPD4 (GIP-1078) of the following formula 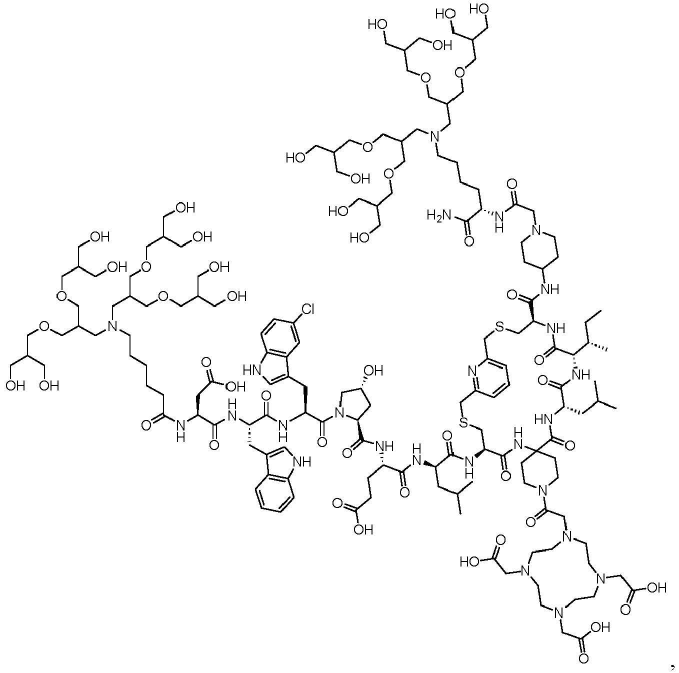
compound HAC2:HAC:Aur-Asp-Trp-5Clw-Hyp-Glu-leu-[Cys(2Lut)-Apc(DOTA)-Leu-Ile-Cys]-APAc-kur:HAC:HAC2 (GIP-1079) of the following formula 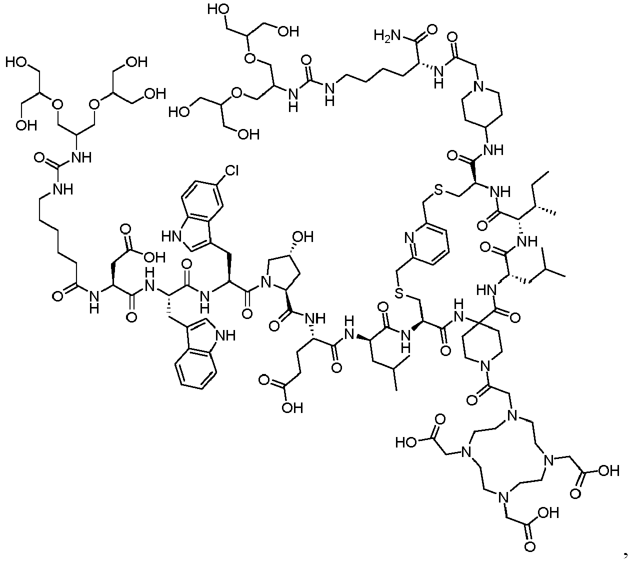
compound HPD2:HAC:Aur-Asp-Trp-5Clw-Hyp-Glu-leu-[Cys(2Lut)-Apc(DOTA)-Leu-Ile-Cys]-APAc-kur:HAC:HPD2 (GIP-1080) of the following formula 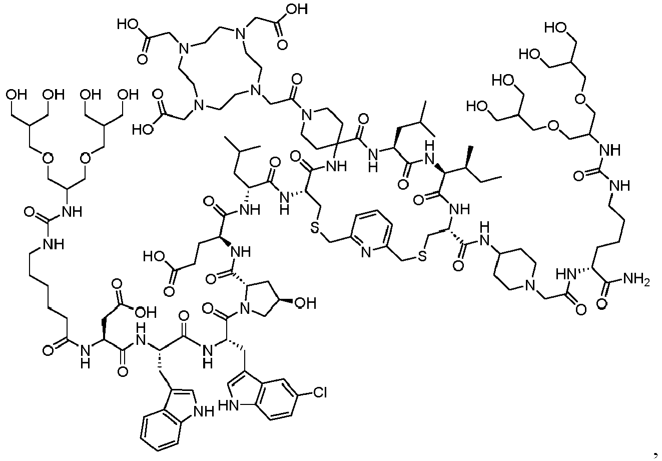
compound HAC2:HPD:Aur-Asp-Trp-5Clw-Hyp-Glu-leu-[Cys(2Lut)-Amk(DOTA)-Leu-Ile-Cys]-APAc-kur:HPD:HAC2 (GIP-1081) of the following formula 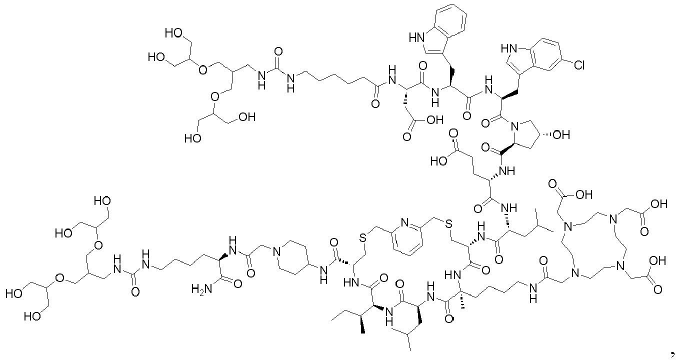
compound HAC2:HPD:Hyx-Asp-Trp-5Clw-Hyp-Glu-leu-[Cys(2Lut)-Amk(DOTA)-Leu-Ile-Cys]-APAc-prx:HPD:HAC2 (GIP-1082) of the following formula 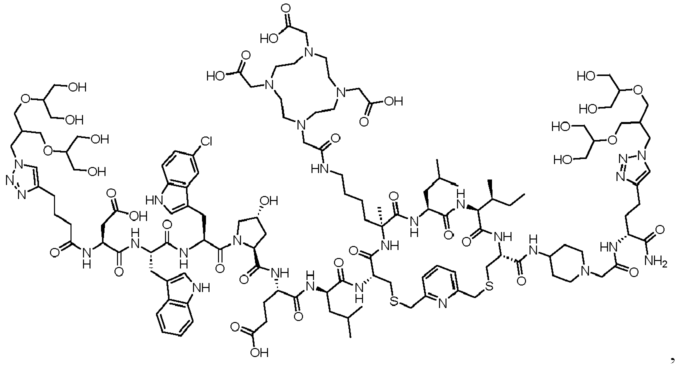
compound HAC4:HPD2:Ahx-Asp-Trp-5Clw-Hyp-Glu-leu-[Cys(2Lut)-Amk(DOTA)-Leu-Ile-Cys]-APAc-lys:HPD2:HAC4 (GIP-1083) of the following formula 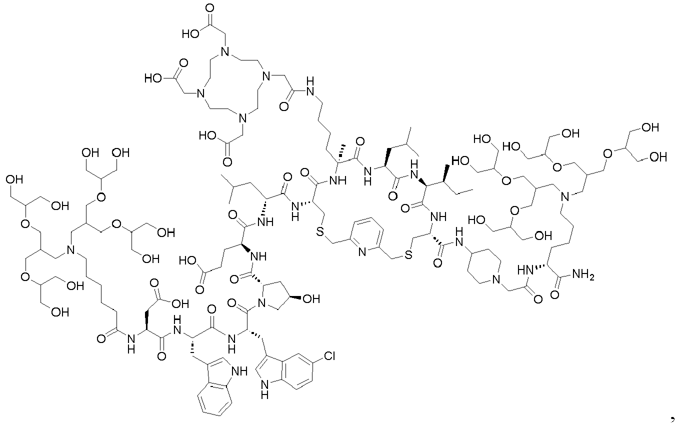
compound HPD2:HPD:Aur-Asp-Trp-5Clw-Hyp-Glu-leu-[Cys(2Lut)-Amk(DOTA)-Leu-Ile-Cys]-APAc-kur:HPD:HPD2 (GIP-1084) of the following formula 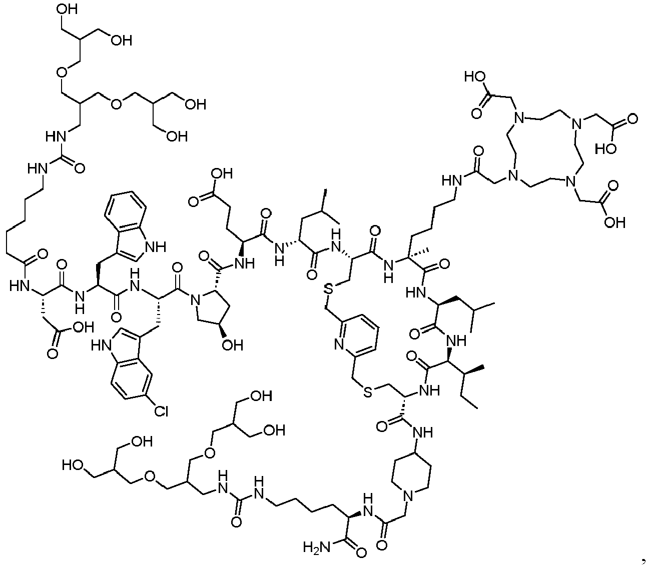
compound HPD2:HPD:Hyx-Asp-Trp-5Clw-Hyp-Glu-leu-[Cys(2Lut)-Amk(DOTA)-Leu-Ile-Cys]-APAc-prx:HPD:HPD2 (GIP-1085) of the following formula 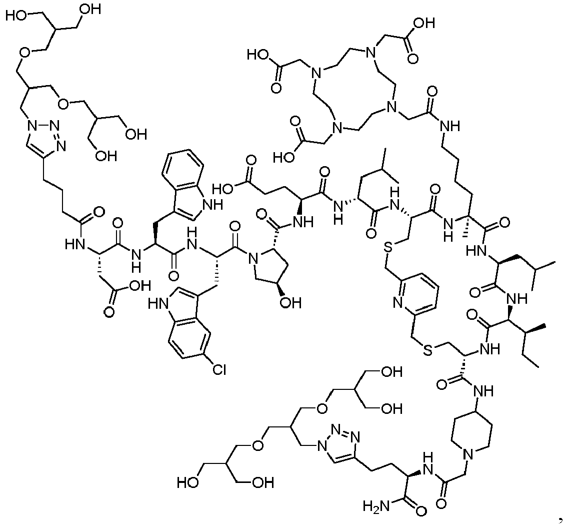
compound HPD4:HPD2:Ahx-Asp-Trp-5Clw-Hyp-Glu-leu-[Cys(2Lut)-Amk(DOTA)-Leu-Ile-Cys]-APAc-lys:HPD2:HPD4 (GIP-1086) of the following formula 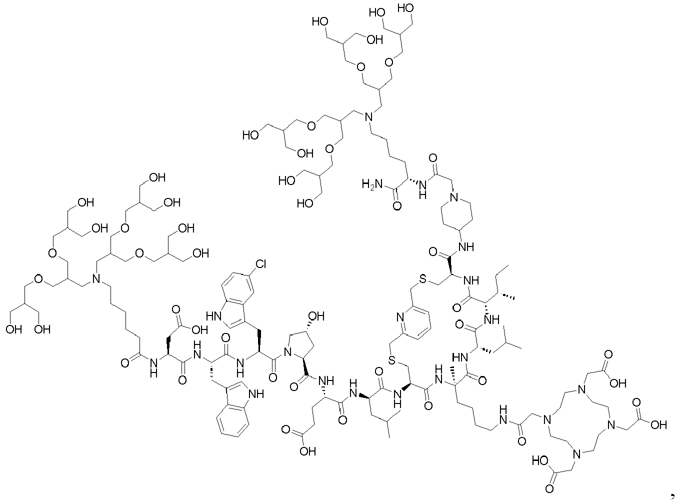
compound HAC2:HAC:Aur-Asp-Trp-5Clw-Hyp-Glu-leu-[Cys(2Lut)-Amk(DOTA)-Leu-Ile-Cys]-APAc-kur:HAC:HAC2 (GIP-1087) of the following formula 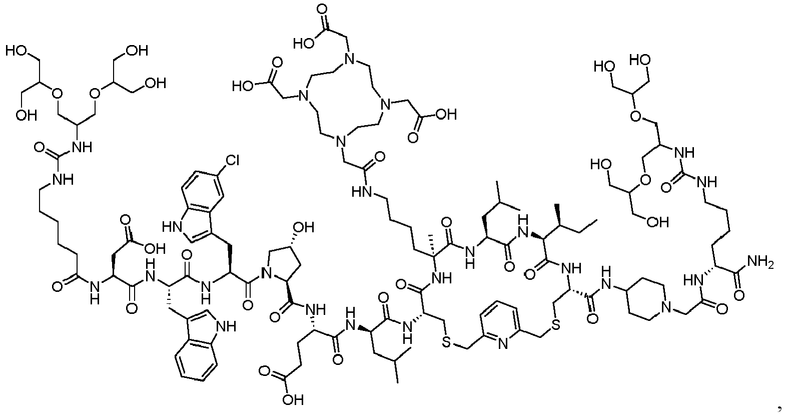
compound HPD2:HAC:Aur-Asp-Trp-5Clw-Hyp-Glu-leu-[Cys(2Lut)-Amk(DOTA)-Leu-Ile-Cys]-APAc-kur:HAC:HPD2 (GIP-1088) of the following formula 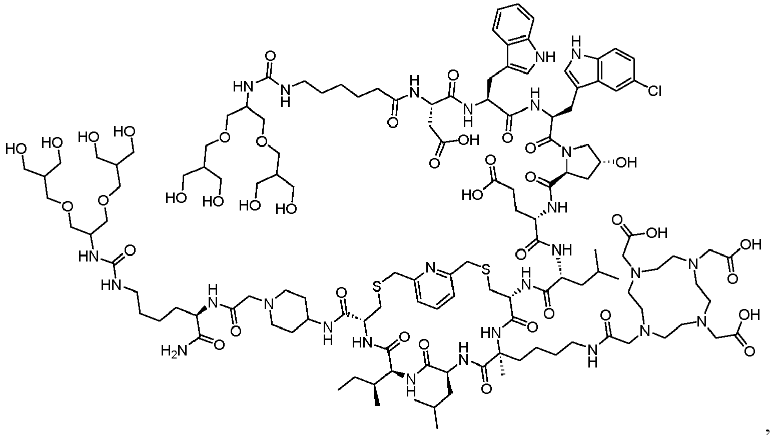
compound HAC2:HAC:Ahx-Asp-Trp-5Clw-Tap-Glu-leu-[Cys(2Lut)-Apc(DOTA)-Leu-Ile-Cys]-APAc-lys:HAC:HAC2 (GIP-1089) of the following formula 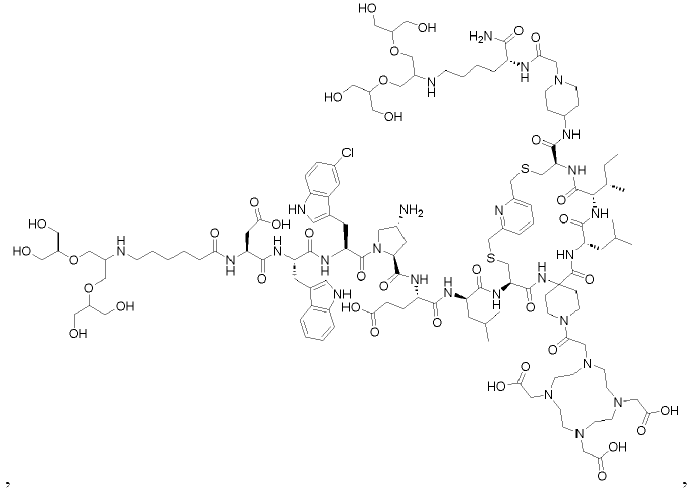
compound HAC2 :HAC: Tzx-Asp-Trp-5 Clw-Tap-Glu-leu-[Cys(2Lut)-Apc(DOTA)-Leu-Ile-Cys] - APAc-tzk:HAC:HAC2 (GIP-1090) of the following formula 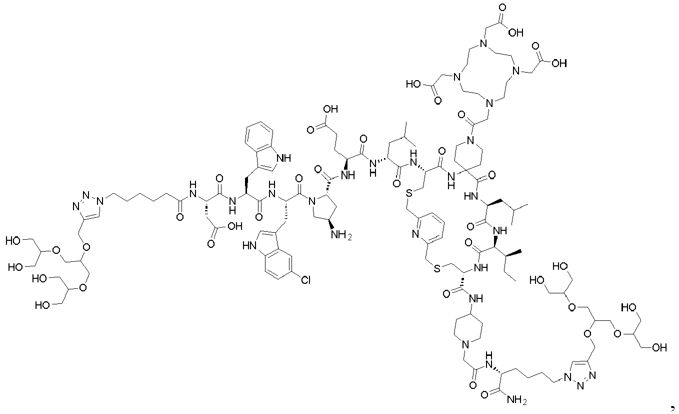
compound HAC2:HPD:Tzx-Asp-Trp-5Clw-Tap-Glu-leu-[Cys(2Lut)-Apc(DOTA)-Leu-Ile-Cys]-APAc-tzk:HPD:HAC2 (GIP-1091) of the following formula 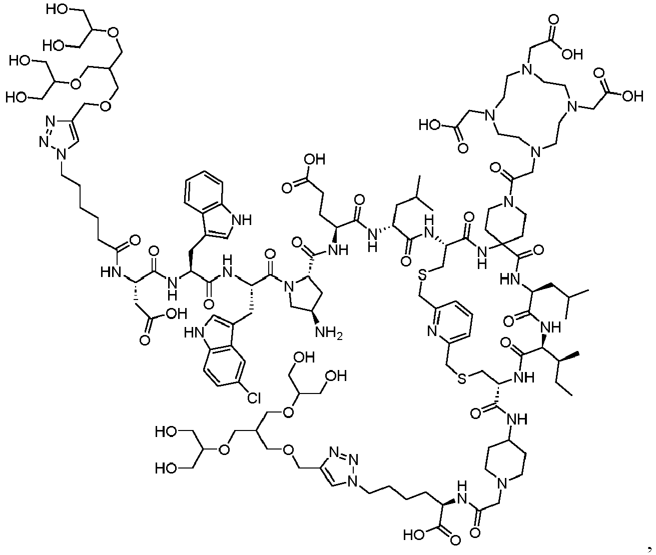
compound HPD:Aur-Asp-Trp-5Clw-Tap-Glu-leu-[Cys(2Lut)-Apc(DOTA)-Leu-Ile-Cys]-APAc-kur:HPD (GIP-1092) of the following formula 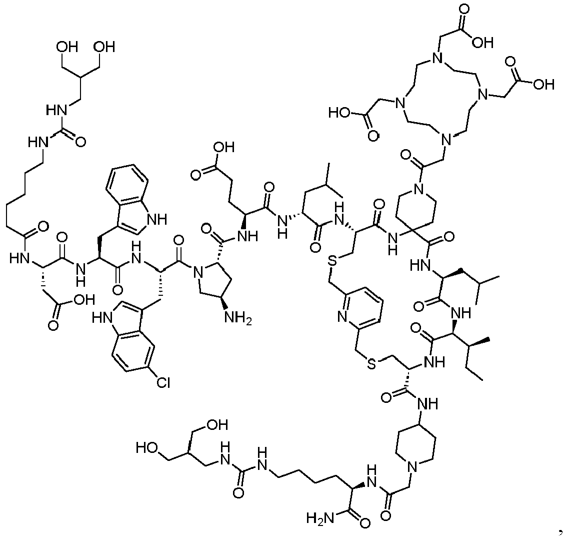
compound HPD:Hyx-Asp-Trp-5Clw-Tap-Glu-leu-[Cys(2Lut)-Apc(DOTA)-Leu-Ile-Cys]-APAc-prx:HPD (GIP-1093) of the following formula 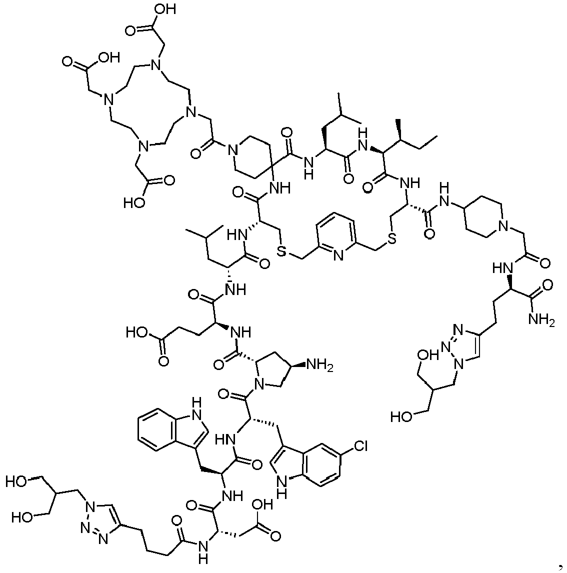
compound HPD2:HAC:Ahx-Asp-Trp-5Clw-Tap-Glu-leu-[Cys(2Lut)-Apc(DOTA)-Leu-Ile-Cys]-APAc-lys:HAC:HPD2 (GIP-1094) of the following formula 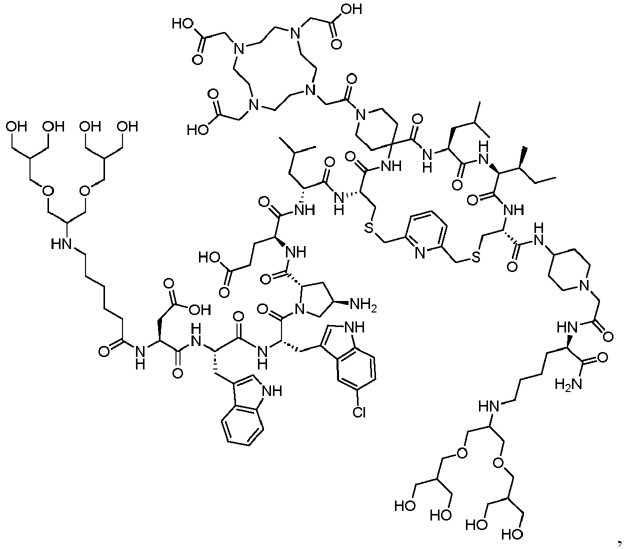
compound HPD2:HPD:Tzx-Asp-Trp-5Clw-Tap-Glu-leu-[Cys(2Lut)-Apc(DOTA)-Leu-Ile-Cys]-APAc-tzk:HPD:HPD2 (GIP-1095) of the following formula 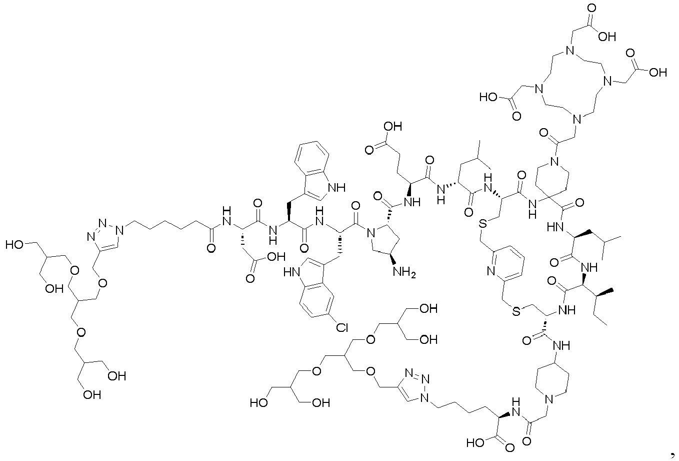
compound HAC:Aur-Asp-Trp-5Clw-Tap-Glu-leu-[Cys(2Lut)-Apc(DOTA)-Leu-Ile-Cys]-APAc-kur:HAC (GIP-1096) of the following formula 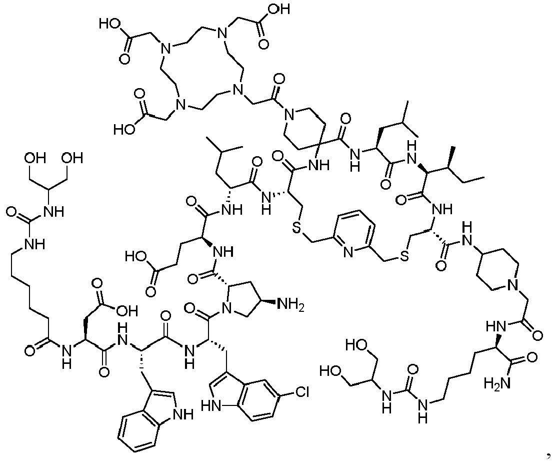
compound HPD2:HAC:Tzx-Asp-Trp-5Clw-Tap-Glu-leu-[Cys(2Lut)-Apc(DOTA)-Leu-Ile-Cys]-APAc-tzk:HAC:HPD2 (GIP-1097) of the following formula 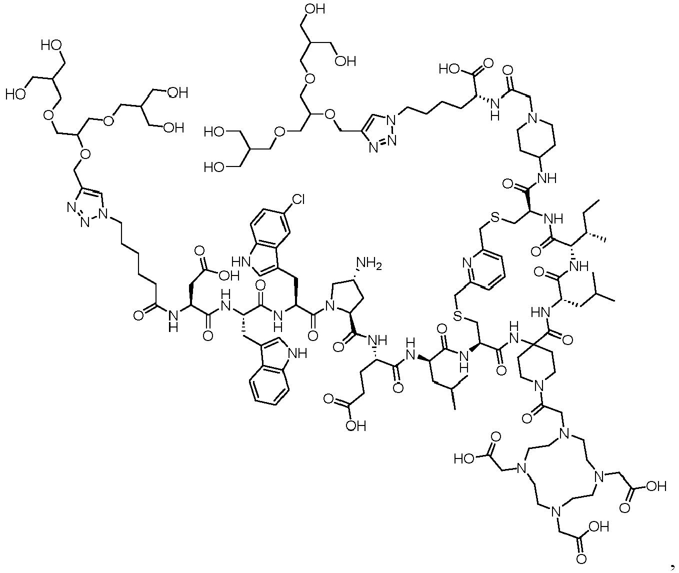
compound HAC2:HAC:Ahx-Asp-Trp-5Clw-Tap-Glu-leu-[Cys(2Lut)-Amk(DOTA)-Leu-Ile-Cys]-APAc-lys:HAC:HAC2 (GIP-1098) of the following formula 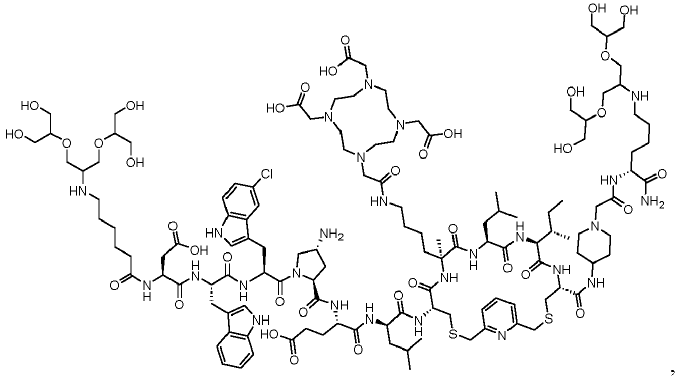
compound HAC2:HAC:Tzx-Asp-Trp-5Clw-Tap-Glu-leu-[Cys(2Lut)-Amk(DOTA)-Leu-Ile-Cys]-APAc-tzk:HAC:HAC2 (GIP-1099) of the following formula 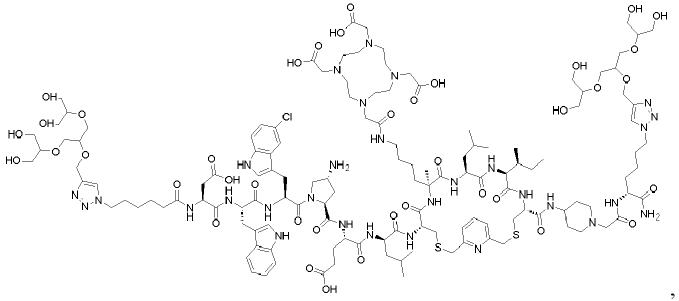
compound HAC2:HPD:Tzx-Asp-Trp-5Clw-Tap-Glu-leu-[Cys(2Lut)-Amk(DOTA)-Leu-Ile-Cys]-APAc-tzk:HPD:HAC2 (GIP-1100) of the following formula 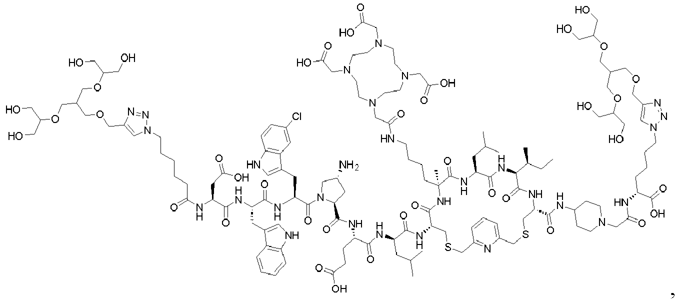
compound HPD:Aur-Asp-Trp-5Clw-Tap-Glu-leu-[Cys(2Lut)-Amk(DOTA)-Leu-Ile-Cys]-APAc-kur:HPD (GIP-1101) of the following formula 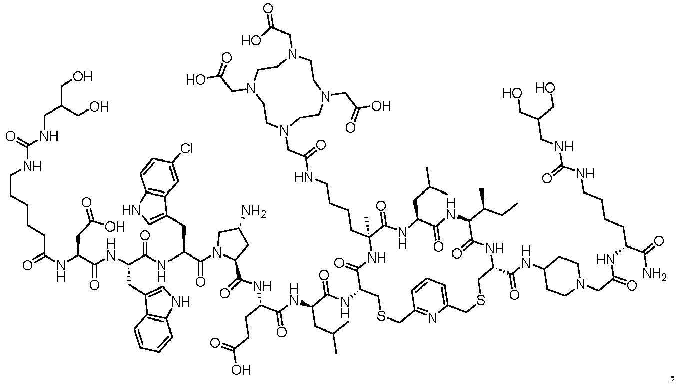
compound HPD:Hyx-Asp-Trp-5Clw-Tap-Glu-leu-[Cys(2Lut)-Amk(DOTA)-Leu-Ile-Cys]-APAc-prx:HPD (GIP-1102) of the following formula 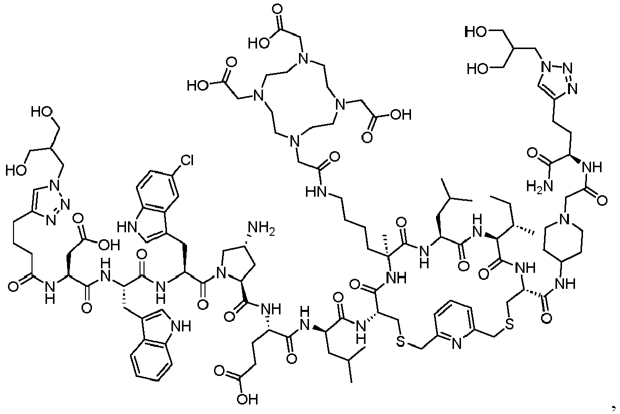
compound HPD2:HAC:Ahx-Asp-Trp-5Clw-Tap-Glu-leu-[Cys(2Lut)-Amk(DOTA)-Leu-Ile-Cys]-APAc-lys:HAC:HPD2 (GIP-1103) of the following formula 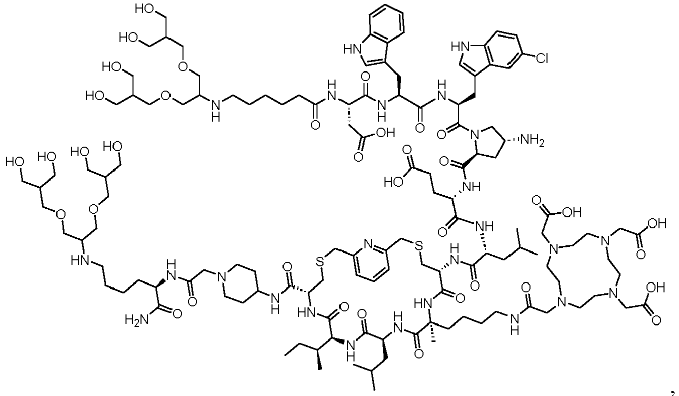
compound HPD2:HPD:Tzx-Asp-Trp-5Clw-Tap-Glu-leu-[Cys(2Lut)-Amk(DOTA)-Leu-Ile-Cys]-APAc-tzk:HPD:HPD2 (GIP-1104) of the following formula 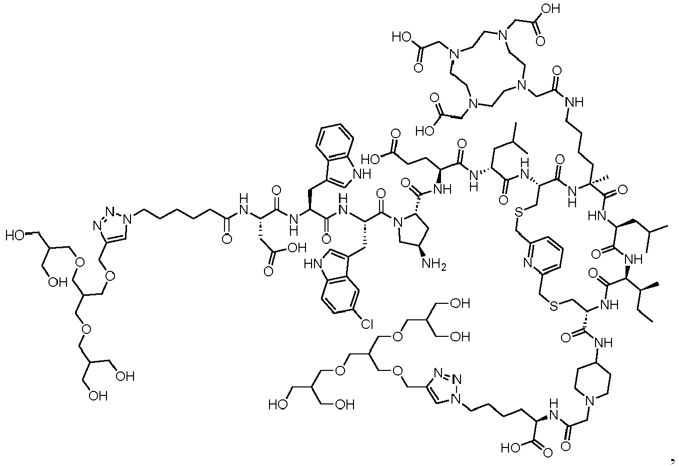
compound HAC:Aur-Asp-Trp-5Clw-Tap-Glu-leu-[Cys(2Lut)-Amk(DOTA)-Leu-Ile-Cys]-APAc-kur:HAC (GIP-1105) of the following formula 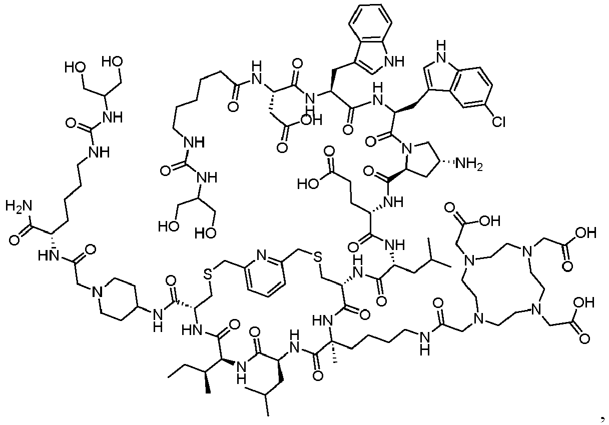
compound HPD2:HAC:Tzx-Asp-Trp-5Clw-Tap-Glu-leu-[Cys(2Lut)-Amk(DOTA)-Leu-Ile-Cys]-APAc-tzk:HAC:HPD2 (GIP-1106) of the following formula 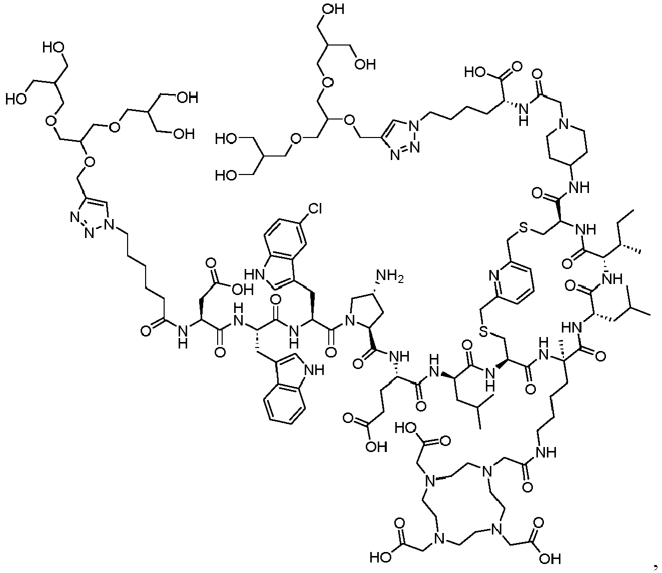
compound HAC2:HAC:Ahx-Asp-Trp-5Clw-Hyp-Glu-leu-[Cys(2Lut)-Apc(DOTA)-Leu-Ile-Cys]-APAc-lys:HAC:HAC2 (GIP-1107) of the following formula 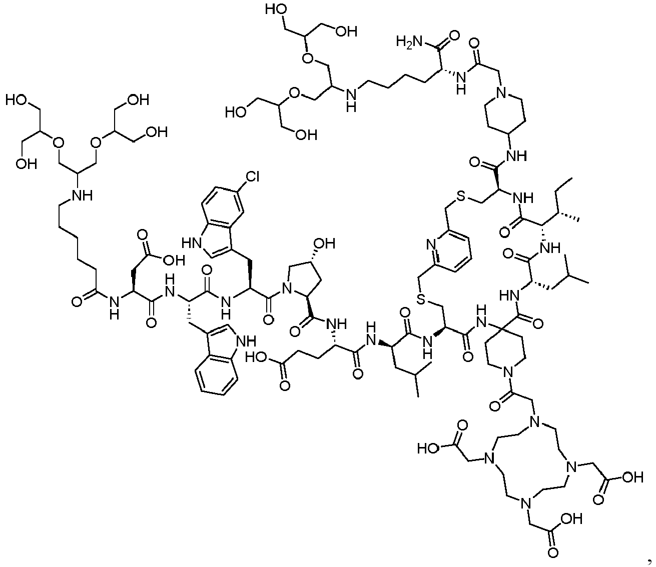
compound HAC2:HAC:Tzx-Asp-Trp-5Clw-Hyp-Glu-leu-[Cys(2Lut)-Apc(DOTA)-Leu-Ile-Cys]-APAc-tzk:HAC:HAC2 (GIP-1108) of the following formula 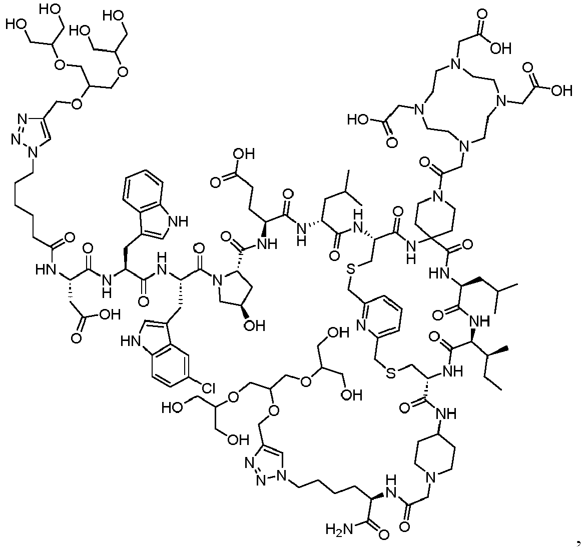
compound HAC2:HPD:Tzx-Asp-Trp-5Clw-Hyp-Glu-leu-[Cys(2Lut)-Apc(DOTA)-Leu-Ile-Cys]-APAc-tzk:HPD:HAC2 (GIP-1109) of the following formula 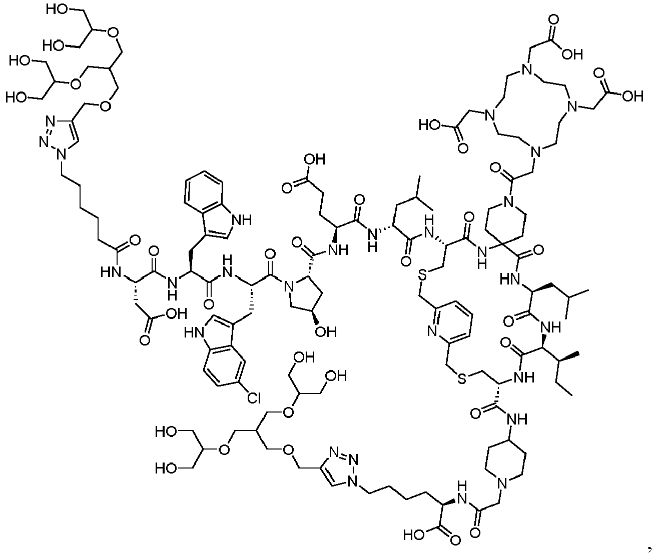
compound HPD:Aur-Asp-Trp-5Clw-Hyp-Glu-leu-[Cys(2Lut)-Apc(DOTA)-Leu-Ile-Cys]-APAc-kur:HPD (GIP-1110) of the following formula 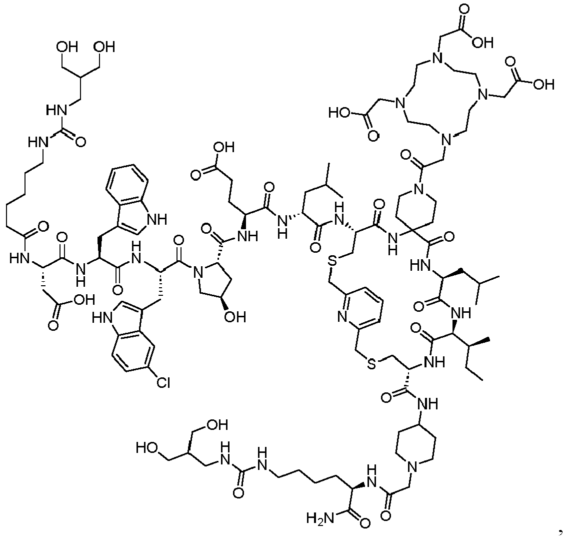
compound HPD:Hyx-Asp-Trp-5Clw-Hyp-Glu-leu-[Cys(2Lut)-Apc(DOTA)-Leu-Ile-Cys]-APAc-prx:HPD (GIP-1111) of the following formula 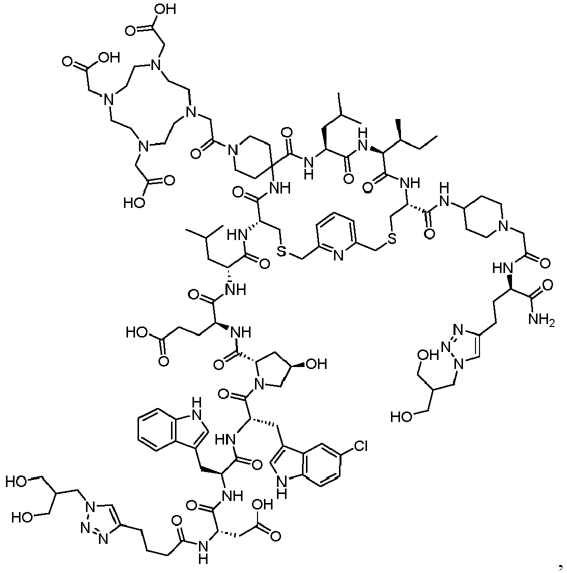
compound HPD2:HAC:Ahx-Asp-Trp-5Clw-Hyp-Glu-leu-[Cys(2Lut)-Apc(DOTA)-Leu-Ile-Cys]-APAc-lys:HAC:HPD2 (GIP-1112) of the following formula 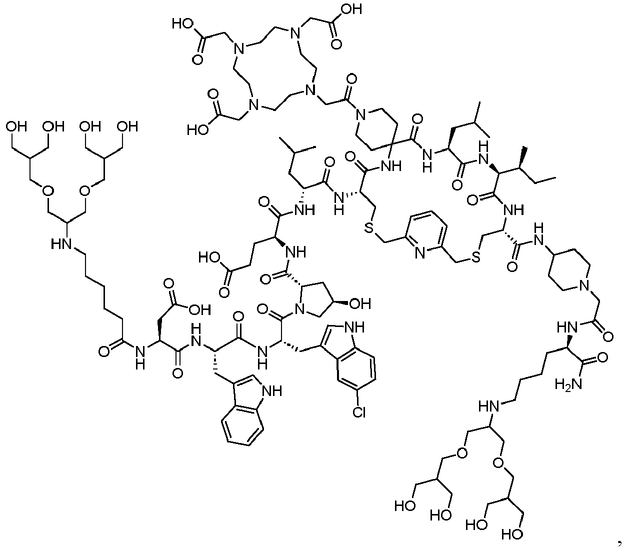
compound HPD2:HPD:Tzx-Asp-Trp-5Clw-Hyp-Glu-leu-[Cys(2Lut)-Apc(DOTA)-Leu-Ile-Cys]-APAc-tzk:HPD:HPD2 (GIP-1113) of the following formula 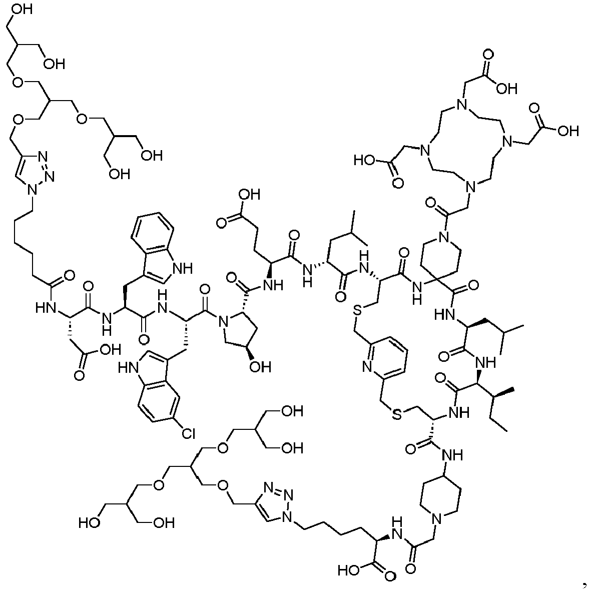
compound HAC:Aur-Asp-Trp-5Clw-Hyp-Glu-leu-[Cys(2Lut)-Apc(DOTA)-Leu-Ile-Cys]-APAc-kur:HAC (GIP-1114) of the following formula 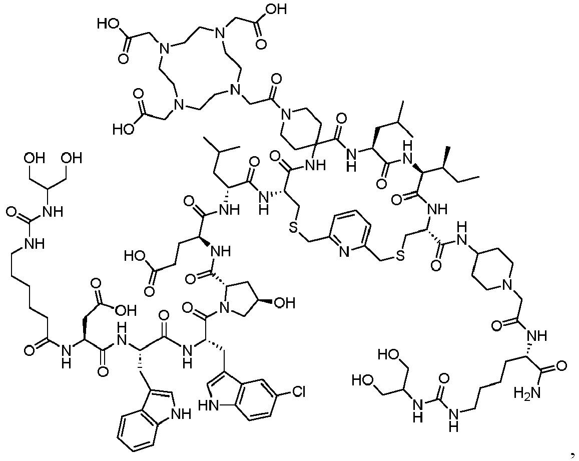
compound HPD2:HAC:Tzx-Asp-Trp-5Clw-Hyp-Glu-leu-[Cys(2Lut)-Apc(DOTA)-Leu-Ile-Cys]-APAc-tzk:HAC:HPD2 (GIP-1115) of the following formula 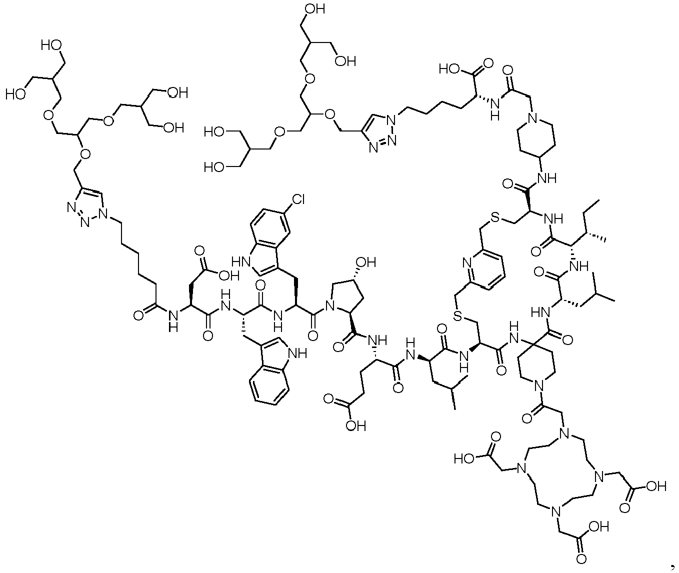
compound HAC2:HAC:Ahx-Asp-Trp-5Clw-Hyp-Glu-leu-[Cys(2Lut)-Amk(DOTA)-Leu-Ile-Cys]-APAc-lys:HAC:HAC2 (GIP-1116) of the following formula 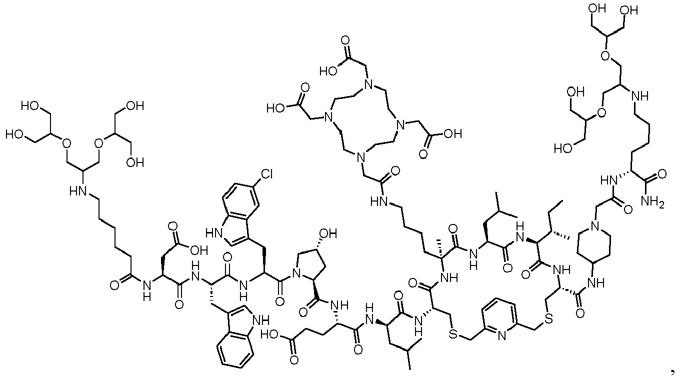
compound HAC2:HAC:Tzx-Asp-Trp-5Clw-Hyp-Glu-leu-[Cys(2Lut)-Amk(DOTA)-Leu-Ile-Cys]-APAc-tzk:HAC:HAC2 (GIP-1117) of the following formula 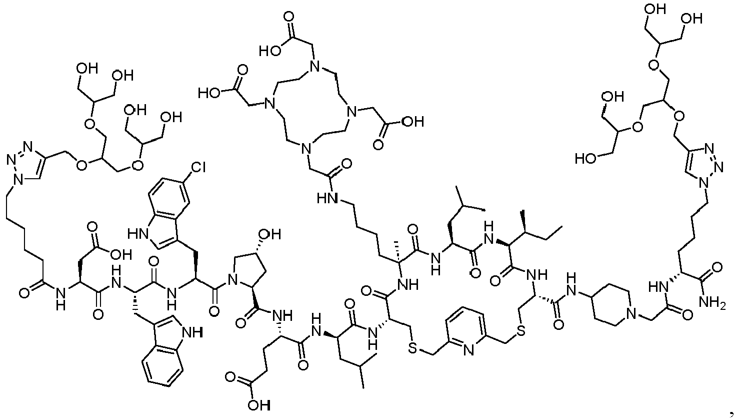
compound HAC2:HPD:Tzx-Asp-Trp-5Clw-Hyp-Glu-leu-[Cys(2Lut)-Amk(DOTA)-Leu-Ile-Cys]-APAc-tzk:HPD:HAC2 (GIP-1118) of the following formula 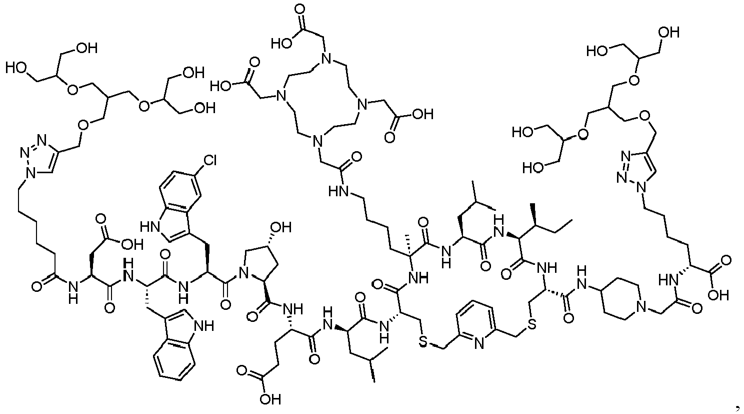
compound HPD:Aur-Asp-Trp-5Clw-Hyp-Glu-leu-[Cys(2Lut)-Amk(DOTA)-Leu-Ile-Cys]-APAc-kur:HPD (GIP-1119) of the following formula 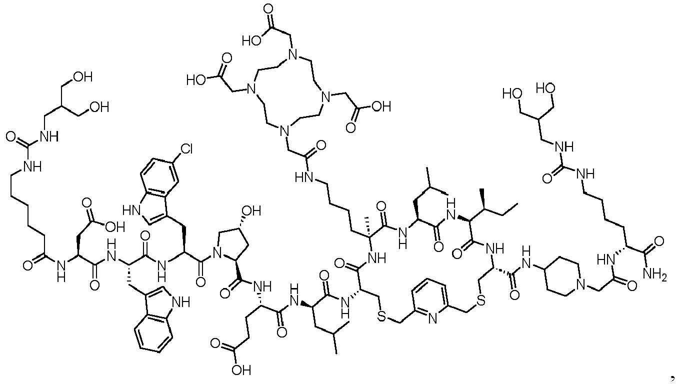
compound HPD:Hyx-Asp-Trp-5Clw-Hyp-Glu-leu-[Cys(2Lut)-Amk(DOTA)-Leu-Ile-Cys]-APAc-prx:HPD (GIP-1120) of the following formula 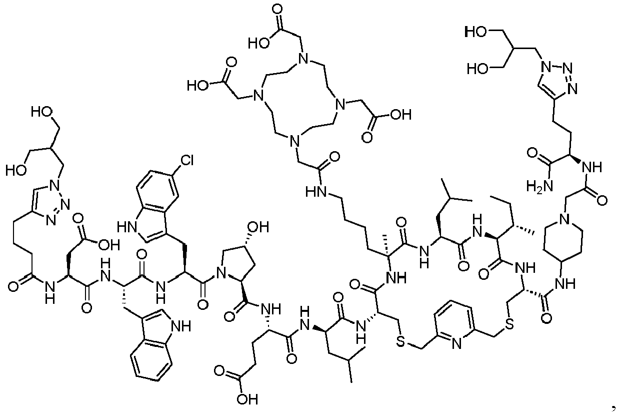
compound HPD2:HAC:Ahx-Asp-Trp-5Clw-Hyp-Glu-leu-[Cys(2Lut)-Amk(DOTA)-Leu-Ile-Cys]-APAc-lys:HAC:HPD2 (GIP-1121) of the following formula 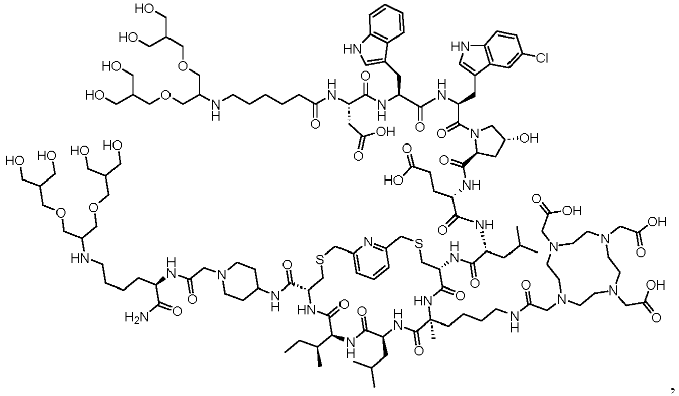
compound HPD2:HPD:Tzx-Asp-Trp-5Clw-Hyp-Glu-leu-[Cys(2Lut)-Amk(DOTA)-Leu-Ile-Cys]-APAc-tzk:HPD:HPD2 (GIP-1122) of the following formula 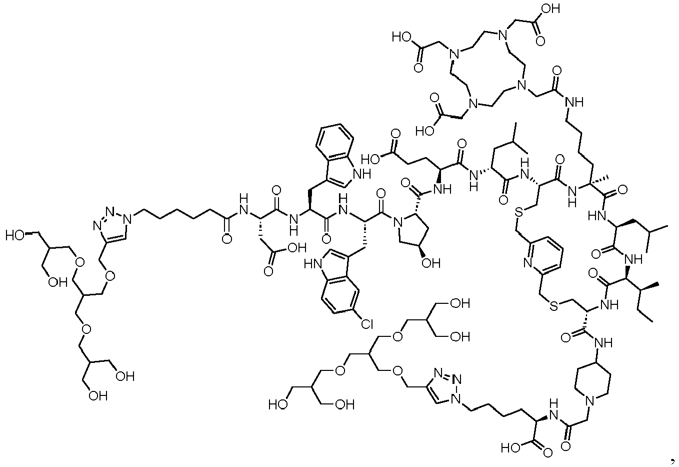
compound HAC:Aur-Asp-Trp-5Clw-Hyp-Glu-leu-[Cys(2Lut)-Amk(DOTA)-Leu-Ile-Cys]-APAc-kur:HAC (GIP-1123) of the following formula 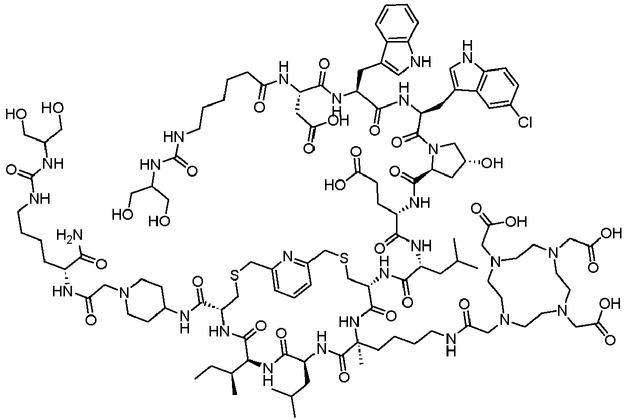
compound HPD2:HAC:Tzx-Asp-Trp-5Clw-Hyp-Glu-leu-[Cys(2Lut)-Amk(DOTA)-Leu-Ile-Cys]-APAc-tzk:HAC:HPD2 (GIP-1124) of the following formula 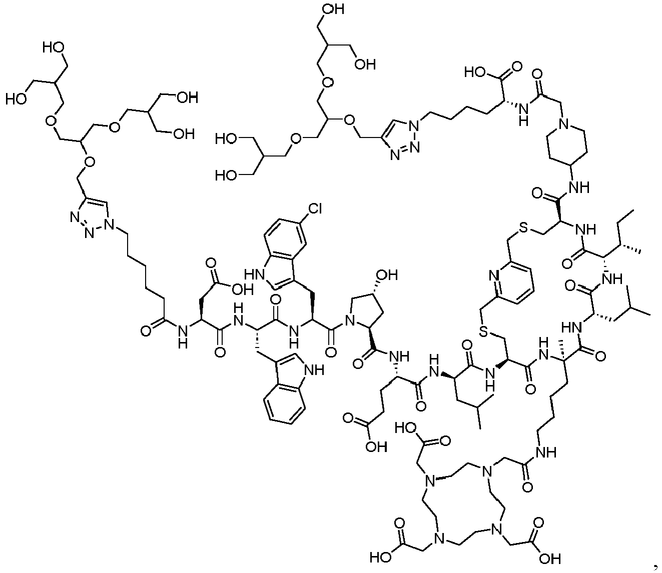
compound HAC:Ahx-Asp-Trp-5Clw-Tap-Glu-leu-[Cys(2Lut)-Apc(DOTA)-Leu-Ile-Cys]-APAc-lys:HAC (GIP-1125) of the following formula 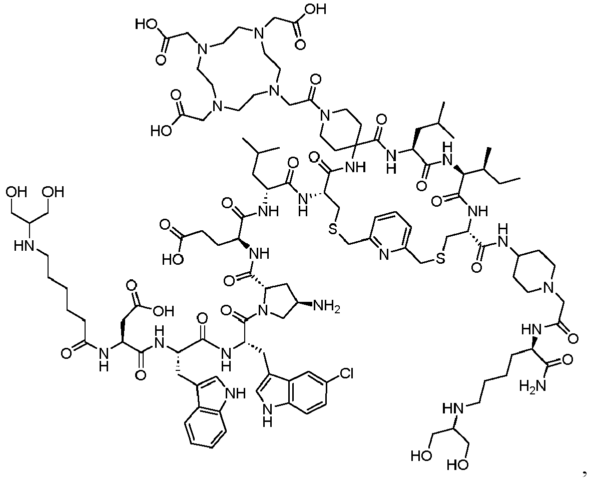
compound HAC:Hyx-Asp-Trp-5Clw-Tap-Glu-leu-[Cys(2Lut)-Apc(DOTA)-Leu-Ile-Cys]-APAc-prx:HAC (GIP-1126) of the following formula 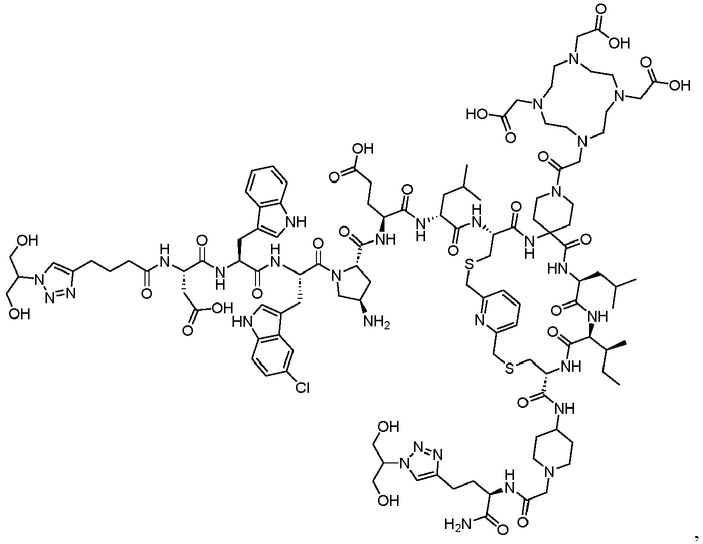
compound HAC:Ahx-Asp-Trp-5Clw-Tap-Glu-leu-[Cys(2Lut)-Amk(DOTA)-Leu-Ile-Cys]-APAc-lys:HAC (GIP-1127) of the following formula 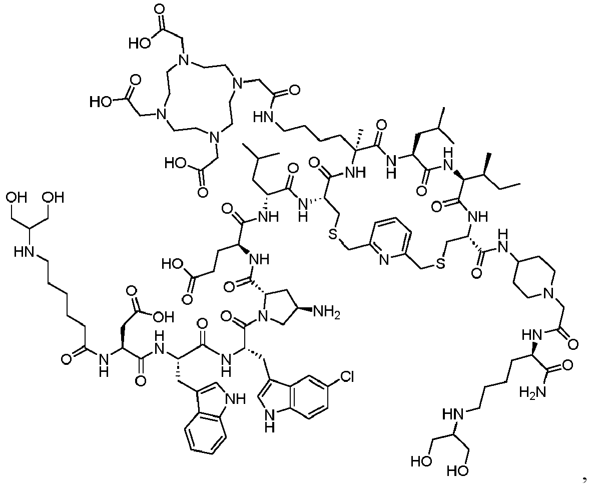
compound HAC:Hyx-Asp-Trp-5Clw-Tap-Glu-leu-[Cys(2Lut)-Amk(DOTA)-Leu-Ile-Cys]-APAc-prx:HAC (GIP-1128) of the following formula 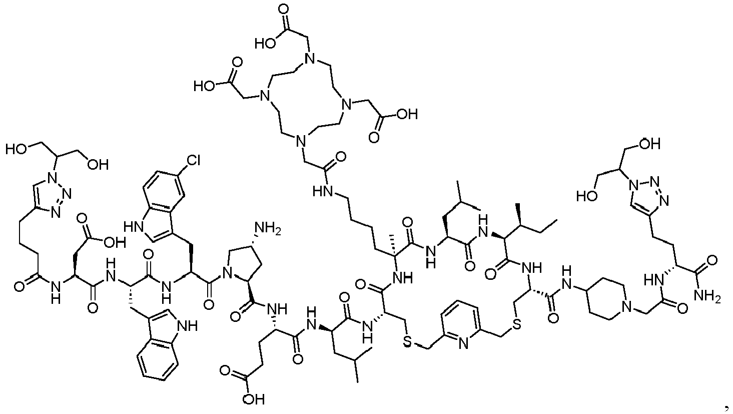
compound HAC:Ahx-Asp-Trp-5Clw-Hyp-Glu-leu-[Cys(2Lut)-Apc(DOTA)-Leu-Ile-Cys]-APAc-lys:HAC (GIP-1129) of the following formula 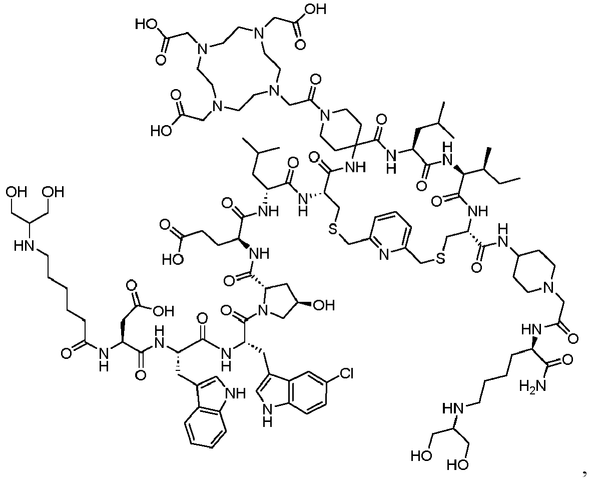
compound HAC:Hyx-Asp-Trp-5Clw-Hyp-Glu-leu-[Cys(2Lut)-Apc(DOTA)-Leu-Ile-Cys]-APAc-prx:HAC (GIP-1130) of the following formula 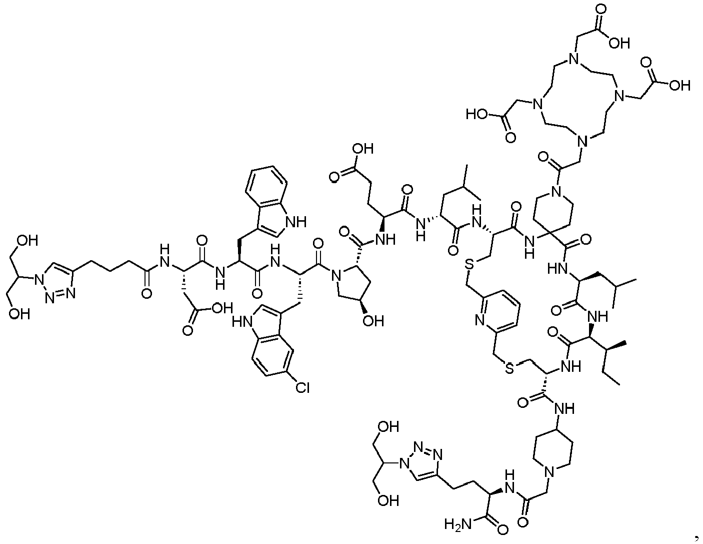
compound HAC:Ahx-Asp-Trp-5Clw-Hyp-Glu-leu-[Cys(2Lut)-Amk(DOTA)-Leu-Ile-Cys]-APAc-lys:HAC (GIP-1131) of the following formula 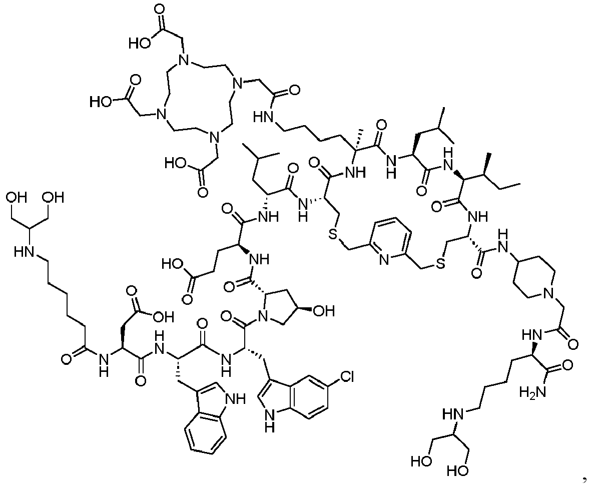
and compound HAC:Hyx-Asp-Trp-5Clw-Hyp-Glu-leu-[Cys(2Lut)-Amk(DOTA)-Leu-Ile-Cys]-APAc-prx:HAC (GIP-1132) of the following formula 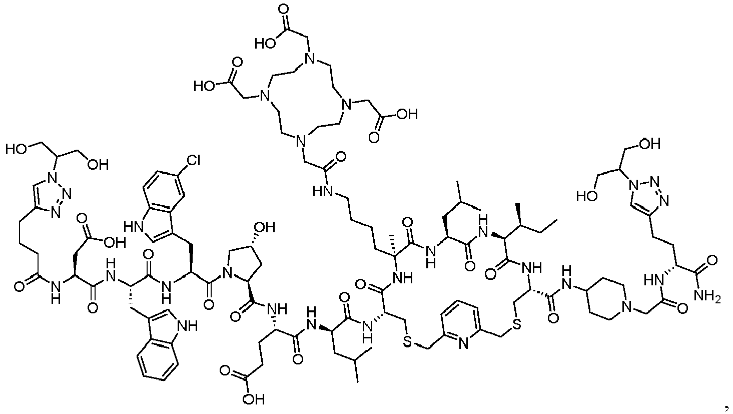
the compound
preferably is selected from the group consisting of GIP-1001 to GIP-1124,
more preferably is selected from the group consisting of GIP-1001 to GIP-1088
and most preferably is selected from the group consisting of GIP-1001 to GIP-1056.

17. The compound of any one of claims 1 to 16, wherein any S-atom, which can be oxidized, preferably any S atom of a thioether group, is present as -S-, -S(O)- or S(O2)- or mixture thereof.

18. The compound of any one of claims 1 to 17, wherein the compound comprises a radionuclide, wherein the radionuclide is a radionuclide suitable for diagnosis of a disease; preferably the radionuclide
is selected from the group comprising, ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ¹⁷⁷Lu, ²⁰¹Tl, ²⁰³Pb, ¹⁸F, ⁷⁶Br, ^{77B}r, ¹²³I, ¹²⁴I, and ¹²⁵I,
more preferably is selected from the group comprising ⁴³Sc, ⁴⁴Sc, ⁶⁴Cu, ⁶⁷Ga, ⁶⁸Ga, ⁸⁶Y,⁸⁹Zr, ¹¹¹In, ¹⁵²Tb, ¹⁵⁵Tb, and ²⁰³Pb,
and
most preferably is selected from the group comprising ⁶⁴Cu, ⁶⁸Ga, ¹¹¹In and ²⁰³Pb.

19. The compound of any one of claims 1 to 17, wherein the compound comprises a radionuclide, wherein the radionuclide is a radionuclide suitable for the treatment of a disease; preferably the radionuclide
is selected from the group comprising ⁴⁷Sc, ⁶⁷Cu, ⁸⁹Sr, ⁹⁰Y, ¹¹¹In, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹³¹I, and ²¹¹At,
more preferably is selected from the group comprising ⁴⁷Sc, ⁶⁷Cu, ⁹⁰Y, ¹⁷⁷Lu, ²¹²Pb, ²¹³Bi, ²²⁵Ac, and ²²⁷Th,
and most preferably is selected from the group comprising ⁹⁰Y, ¹⁷⁷Lu, ²¹²Pb, ²²⁵Ac, and ²²⁷Th.

20. The compound of any one of claims 1 to 18, for use in a method for diagnosing a disease.

21. The compound of any one of claims 1 to 17 and 19, for use in a method for the treatment of a disease.

22. The compound of any one of claims 1 to 18, for use in a method for the identification of a subject, wherein the subject is likely to respond or likely not to respond to a treatment of a disease, wherein the method for the identification of a subject comprises carrying out a method of diagnosing a disease using a compound of any one of claims 1 to 18, or for use in a method for the selection of a subject from a group of subjects, wherein the subject is likely to respond or likely not to respond to a treatment of a disease, wherein the method for the selection of a subject from a group of subjects comprises carrying out a method of diagnosing a disease using a compound of any one of claims 1 to 18 or for use in a method for the stratification of a group of subjects into subjects which are likely to respond to a treatment of a disease, and into subjects which are not likely to respond to a treatment of a disease, wherein the method for the stratification of a group of subjects comprises carrying out a method of diagnosing a disease using a compound of any one of claims 1 to 18.

23. The compound for use of any one of claims 20 to 22, wherein the disease is cancer, preferably the cancer is expressing Gastric Inhibitory Peptide Receptor (GIPR), more preferably the cancer is overexpressing GIPR.

24. The compound for use of claim 23, wherein the cancer is a neuroendocrine cancer or a neuroendocrine neoplasm, preferably the cancer is selected from the group consisting of medullary thyroid cancer, gastrointestinal neuroendocrine neoplasms, ileal neuroendocrine neoplasms, pancreatic neuroendocrine neoplasms, nonfunctioning neuroendocrine neoplasms, Insulinomas, Gastrinomas, Glucagonomas, VIPomas, Somatostatinoma, ACTHoma, lung neuroendocrine neoplasms, typical carcinoid tumors, atypical carcinoid tumors, small cell carcinoma of the lung, large cell carcinoma of the lung, thymic neuroendocrine tumors, Merkel cell carcinoma, Pheochromocytoma of the adrenal gland, adrenal cancer, parathyroid cancer, paraganglioma, pituitary gland tumors, neuroendocrine tumors of the ovaries and neuroendocrine tumors of the testicles.

25. The compound for use of any one of claims 20 to 22, wherein the disease is a metabolic disease, preferably a diseased cell and/or a diseased tissue, preferably a diseased cell and/or a diseased tissue of the metabolic disease expresses GIPR; more preferably the diseased cell and/or the diseased tissue overexpresses GIPR; most preferably the diseased cell is a pancreatic cell.

26. The compound for use of claim 25, wherein the metabolic disease is selected from the group consisting of type 2 diabetes, type 1 diabetes, metabolic syndrome, insulin resistance, dyslipidemia, impaired fasting glucose, and impaired glucose tolerance.

27. A composition comprising a compound of any one of claims 1 to 19 and a pharmaceutically acceptable excipient; preferably the composition is a pharmaceutical composition.

28. A kit comprising a compound of any one of claims 1 to 19 and one or more optional excipient(s) and optionally one or more device(s); preferably the device(s) is/are selected from the group comprising a labelling device, a purification device, a handling device, a radioprotection device, an analytical device or an administration device.
